(19) Europäisches Patentamt
European Patent Office
Office européen des brevets

(11) EP 3 002 332 A2

(12) EUROPEAN PATENT APPLICATION

(43) Date of publication:
**06.04.2016 Bulletin 2016/14**

(21) Application number: **15173330.0**

(22) Date of filing: **30.11.2010**

(51) Int Cl.:
***C12N 15/82*** *(2006.01)* ***C12N 15/113*** *(2010.01)*
***C12N 5/10*** *(2006.01)* ***C12N 5/04*** *(2006.01)*
***A01H 4/00*** *(2006.01)* ***A01H 5/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2009 US 266248 P**

(62) Document number(s) of the earlier application(s) in accordance with Art. 76 EPC:
**10834295.7 / 2 507 375**

(27) Previously filed application:
**30.11.2010 PCT/IB2010/055490**

(71) Applicant: **BASF Plant Science Company GmbH
67056 Ludwigshafen (DE)**

(72) Inventors:
- **Fu, Huihua
Cary, NC 27519 (US)**
- **Brown, Jeffrey A.
Apex, NC 27502 (US)**
- **Francis, Kirk
Cary, NC 27513 (US)**
- **Song, Hee-Sook
Raleigh, NC 27606 (US)**

(74) Representative: **BASF IP Association
BASF SE
ZRX-C6
67056 Ludwigshafen (DE)**

Remarks:
This application was filed on 23-06-2015 as a divisional application to the application mentioned under INID code 62.

# (54) EXPRESSION CASSETTES FOR EMBRYO-SPECIFIC EXPRESSION IN PLANTS

(57) The present invention relates to an expression cassette for regulating embryo-specific expression of a polynucleotide of interest, said expression cassette comprising a transcription regulating nucleotide sequence, to a vector comprising said expression cassette, host cells and transgenic plants comprising the expression cassette, and methods of producing said transgenic plants.

EP 3 002 332 A2

SEQUENCE LISTING

<110> BASF Plant Science Company GmbH

<120> Expression cassettes for embryo-specific expression in plants

<130> PF62833

<150> US61/266248

<151> 2009-12-03

<160> 196

<170> PatentIn version 3.4

<210> 1

<211> 1106

<212> DNA

<213> Zea mays

<400> 1

ttggtttttt gataatttgt ttatctcaca tgatggttct aacaatatgc gcagatctta 60
tacatctctc tcgtagtttt tcataaacta cgagaaagat ataggtttta tgaacaaatt 120
tacttttatt ttgtcatata atgaaatggt caaaatataa attgtacatc atgatgagtt 180
atacaaattt gtagtccaaa atcttttcat ttaaattaat ttactgcttt aaaatgtgat 240
ttttaattgt ctttgcctag tgttgaaaaa aagcactcgg caaagaaact ctttgccgag 300
tgttaaaaaa acactcggca aagaatctct ttgccgagtg tcaaaaataa aatatttggc 360
aaatagcttc tttgctgagt gttttttgc ttagcacttg gtaaagaact tctttgccga 420
gtgtccgaaa aagcactcga taaaatattt ggcactcagc aaagaaccga attccagtag 480
cgtgagtcag aagttggcat ttgaaccctt tgaaataatc aaacaaatca acgcagttgc 540
atatgaattg gctttgccac atggtagtgc agtccatcga gtgttccatg tttcacagct 600
taaactaacc catggttttg gtaacagaac acacaccagt gactccatcg ttaccggatt 660
ttgatactac actgaaggtt ccggaacaaa tactggattc tagattgatt ctagattgct 720
tcgatcaaat ggggatcatc aacctaacac agcttaaatt atacatgcat gcgtacattt 780
ggtccttcaa gaggaggcag gtgatgatca aggtctctct agcggcctgg tgtgcagcca 840
cgtacccatc cacgcgtcgc cgcgggcgac gacaggtgtt ggccaacctg gacatcagac 900
aagcacgaca atgacaggcg ttcgccaagc gtacgtacag gagacgaaca cgacaagtag 960
caccgtcctg gccgagagcc cgtcatgctc atgcatgcat gcatgctgat catgcatact 1020
cgataaatat acagcagaga gctgagctac gtacacaaac aagaagcttg cattgttcgt 1080
tgctcgatcg ttgcaggtaa tggaga 1106

<210> 2

<211> 922

<212> DNA

<213> Zea mays

<400> 2

ttagtagaga ataacacaca tctccctaag tatcacaaaa aaattaattt taagaggcaa 60
ttttagtttc caaagcgatt tattaagatt taagggaggg tatttattag aaggaagtac 120

attgatcaaa cagagaaaat tccatcatta tggtccccca tagaaatttg tcactcgatt 180
gtcgcaaaaa aagttaccag taaatttgat aaattttgga taccggtctg ttttaactca 240
tcaagaatag cacgacgaaa taatagattg cgtacgtttt cattcataac atctctcact 300
aaagcatttt ttacacacgc tattaaaatg gttcggaaac tgatccttaa gcggtctatt 360
catatccatc tatcgttcta agattcttcc tgtacgtaga ttctttccga ttaaattctc 420
atgaaaaatt aaaaacaaac aggtccttaa actagtcgcc ggaagttctc gcttttgctg 480
tagttgtagt gtcgatgcat gggtggaaac gtggacagat agattcatga aatgtggaca 540
acccacctgg tccagagcat catgatacat tgatacacgt ggcacgctgc ccactgatgc 600
cctcagcttt gaagctaacg tgtcaagtgt ggccggcccc tgcattgcgt cgtgccaatc 660
gtccaagtcc catggcaaaa acccagcgct ttgtgccgcc gccgtccgcc ggcccctctg 720
cccttgtacg tgcacctaga cacatcgtca tcgatcatca cacgcaatcg acacaagaag 780
ttaataaaca gcccaaggac gcagagatca gctgatcgag aaggacttgt actactactc 840
agtattgtcg tcacatgcac atatatgtac ataaagagct agctacctga gctctaccca 900
aggtcgcgtt gatcgatcga tc 922

<210> 3
<211> 1945
<212> DNA
<213> Zea mays
<400> 3

gattcagaac atctggtcag cttaaatgat gtggtaaagg gagcattatt cttttatttt 60
gttctttgtt ctgtgcaatg gccatatatt ttcgtattga tatgctctca gtggaacagt 120
ctgaagctaa tgtgaaactt cgatgttcag gtatgataat gtaccaacag agattaaccg 180
gctgagatgc agagttaact atcatgcatt aaagttccta cctgatattg aggaaatggc 240
tgttaagctt gctgcaagaa tgaggaacaa aactggcagc ataaatccat acatgtacgt 300
tataattatc cttttcgctg ggtgatattg tgtacagtag agcttgtatc tacccaagac 360
actatccagt gcatagctga agttgacgct ctcaaagttt atttcacaat caaatattga 420
cacatttttt ttgttcttaa agggctcttc acctgagatt tgaaaaaggg atggtggggc 480
tttctttctg tgattttgcc ggcacacggg aggagaaggc aatgatggcg gcttataggc 540
agaaagaatg gccaaggcgc ttcaaggtga cgcggactgg actagataca tctgttacat 600
tggactgaga aagaatccct aggagaaatg aagcaccgac actaactata cgcgctagtt 660
cgggaacccc gttttctcac gagactttta tttttccaag aaaaattagt tcattttttt 720
taggaaaata taaatctctt aagaaaatgt agttccgaaa ctagccctac aagtctaacg 780
attttctctt ttttgtatct gccacgcaga atggatccca tctatggcca ctggcgctgc 840
agaagagaaa agaagggcgg tgccctcttg agcccggtga gatcgctgtg atcctgcggg 900
cactggggta cacgagcggc acacagatat acgtcgcgtc cgggcaagtg tacggcggca 960
agaaccggat ggctcccctc aggaacatgt tccccaacct ggtaggcagg ctcaaatcca 1020
cttcagattg tgtcaagctg aagccagtga taactgataa gccactgccg tgcattgtgc 1080
atcgctgcag gtgacgaagg aggagctagc gagcgcggag gagctggcgc cgttccggcg 1140
gcacgtgacg agcctggcgg cgctggactt cctggtgtgc ctgcggtcgg acgcgttcgt 1200
gatgacgcac ggcggcaact tcgccaaact catcatcggg gcgcgccgct acgcggggca 1260
ccgcctcaag tcggtgaagc ccgacaaggg cctcatgtcc aagtccctgg gcgaccccga 1320

catgggctgg gcctccttca cggaggacgt cgtcgtcacg caccgcacga ggacgggcct 1380
ccccgagccc accttccccg gctacgatct ctgggagaac ccgctgacac cctgcatgtg 1440
cagggcatga gacgggccat ccctcagatc agagtggccg tctggccgag tcctcgtctt 1500
cgctaggcta cgactacgag tagtctcgtg cccacttcca tcagcgcgca tttcagcgag 1560
catatgcttc tactagttaa acacctagat ttgtatgtat ttggctggcc taatctgtag 1620
tttcacatag atcacttcat gctgaaaatg tcttggacaa ggcagtgaat gaagataaca 1680
tcagtaacga cctgcttttc caaagctcct cttggattta ccaagctgag ccaagaaatg 1740
gctaacctga gttttgactt gcagccttgc aggcatgacg tgggcgacct gaactcgcta 1800
gtcgtagagt ttctttccac gcggcagctc ggcagagcta cgcagcctct ctgcccgcca 1860
cgttctcctg caaggctgca actcgaagca ctgagaagct attaattaat ggattctcga 1920
gcatgcggcc ttgttcgcta aacct 1945

<210> 4

<211> 512

<212> DNA

<213> Zea mays

<400> 4

gccagtgcta atgatattta cactagcggg ctgctaaaga aaaccgccag tgctaaagat 60
atttacacta gcggttggtg aacaactgcc tgtgaaaaaa gccgatttct actagcccct 120
agctagcact ggcgacataa aaaacgtcag tgaaaatagc tctaggatcg tcactataga 180
gcttctatgt acttagtggt tagaactgat attgtagtgc accaagtgcc gattttaatt 240
aaaccaatac taaatactag taaataatac tagtggtctg aattcgattt ctatagtaat 300
gtttgcttgc aagccgcaaa tagagtaaac attcgtcgtc acagaaatcc acattacatc 360
aaggtccatg gcggccggcc acgtacccat cccacgcgtc gctgcggagg acacgtgttg 420
gctgaccgga cagttggccg atcagacagt ggacagaccg gacaatagaa gaagaagacg 480
acgacggcgg cggcaccgcc gagtaggtgc at 512

<210> 5

<211> 698

<212> DNA

<213> Zea mays

<400> 5

gtgattaagt tgactggcaa attgcataga cgataataca ctctatgctt ttgagataac 60
cactgtacac caaccgactt gttgtttgaa ggctgatctt taaccattac tactgtaact 120
aactgttgtt gaataaacta ttcatacgac ttggagactt tgatgtatat tatgttttgt 180
acctatacta ttaactaccg cagatacgta tttacatctc ggtaacatct gccagtcgca 240
caaatgcact aaggtgactg acagactgta gcgaccgata gagcgctgcc ttgttctagt 300
caaaattcaa gcctggtggt cttctcgatc cctctgcgcg cgccctgctc ttttatcctg 360
tgtgtctcct ccacgaggga aggcgtcatg agaggacgac acgacgacca tgcagtgctg 420
cgcaaccgcg cgcgtctgcc tgttcactga gccccaggcg gccggtccaa gctattacca 480
cgtcgccccc ctccagggct gcgggcagca acgtgtcgac cggtccgcct ctgacgtgtc 540
cctcccgtcc tctgcataaa agggtgcggc tggcggggag cctcagcttc atatcgtcgc 600
aagctctccg tgtttctctc gtttcttcac tgctgccaac tgtgctgtgc agtagttgca 660

agagctgatt ggtagctagg tacgctaggc aagtaggc 698

<210> 6

<211> 1355

<212> DNA

<213> Zea mays

<400> 6

tataaattag aacggagggt atgttttttt acgtttccat tatttgagtc tgctcatcac 60
gtttaatttg tttatacagt tcaatgagtt cagttacagc ggatgataat tcgacggcgt 120
ttatttgcat gcacttactt tttacattac gcctagaatg taatcggatg gacgaatatt 180
acttgttttg tgtttatttt catatttttt tctcttcgga tacggatatt atcgagttgt 240
gccgaacaag attttgaatg tatatcgaca tcttaaatat ataactttga atatacggat 300
acgtatatag atcggatatt gaatacccag ctagactcag attaaattgg atcttagtta 360
ttttaggggg tgtttgaatg cactaaaact aatagttagt ggctaaaatt agttgaaaca 420
tccaaacacc ctatctaata gttcagctat tagttatttt tggaaaatta gttaatagtt 480
aggtagttat ctgttagcta gctaattcaa ctaattataa gttctagtgc atttaaatac 540
cccttagacg gatcgaatta gaacaccgat ggataatatc cataacattt tacacctata 600
aatatgtcct atatgtatat agatgtcaaa aacaggttgg gcatgttgga tgacccgtgg 660
cacgatacag ttttaagcag tgccaaacat ggcatggcgg cgagctagac atgatacggt 720
ccggttttat tttgtatttt tttttcgta ataatatctg tattatgctt gttcgaacct 780
cacttgctta tgtggatgtg atagtatcaa ctcgctggcg ttccgtgcat cgtaacatct 840
atcacatccc acagatcatg tcgcttatct tttctgatac tatagcagta gcacacagat 900
gccggtcgaa cccagaagca tcacgccatc accccctccc cttttcccaa tcaaaaccac 960
gtccgtccca atggtaaccc agcaccaatc tcgtgttcgt gtgagacaga gcatgcaaac 1020
aaacagcatg catgcgcgaa ggaaagcgtg cagcgcgcgg gacgtgaacc gcgcgcgaac 1080
cctgccgccc gtccggtctc agctcacgcc cagcgtcctt cccaggccgc gatccggcca 1140
tcctgacgcg cgtttcgtct gggccccaaa accacgtccg ccgcggcggc ggcgcgccca 1200
cgggctcccg tgcccgccga tagtttgccg gaccctgccg cctcctataa atgcagagcc 1260
ctccgccagc gtctcccgca tctcatcttc ttcctccatc cgcactactg agagcgacaa 1320
agctagctct ctccagggat cggattcgga tcacc 1355

<210> 7

<211> 1941

<212> DNA

<213> Zea mays

<400> 7

aagggactcg tggcctacac acgcgcacaa taatcagtta ccatgaatta ttttatatgc 60
gaatatcgac attacacatg aataatgtga atttgaattt ccgcagcatg caaaattccc 120
gtgagtttga agggcgataa aaacgggtgg ccaaaattgg ggcggcaatg caagaagata 180
agcctatcca tcccttgcat tggtattgtc atctggggca gggcagggga gggcaggaga 240
agctatctat tggccatcta tccagcagcc gctctcccca gccccactct ctccatctag 300
agctccctaa ctgcaccggc cgccacacca ccgtaccccg acccctcgac aacaatggcc 360
accgtcctag gcagcccccg cgcgccggcc ttcttcttct cgccgtcctc cctccgtgcc 420

gcgccggcgc ccaccgccgt ggcgctgcct gcggccaagg tgggcatcat gggccgtagc 480
gccagcagca ggggcaggct gcgcgcgcag gccacctaca acgtgaagct gatcacgccg 540
gagggggagg tggagctgca ggtgcccgac gacgtgtaca tcctggacca ggccgaggag 600
gacggcatcg acctgcccta ctcctgccgc gcggggtcct gctcctcctg cgccggcaag 660
gtcgtctccg gctccgtgga ccagtccgac cagagctacc tcgacgacgg ccagatcgcc 720
gccggctggg tgctcacctg ccacgcctac cccacctctg acgtcgtcat cgagacgcac 780
aaggaggagg agctcaccgg cgcataatca ttcatgccca tccatctcta tctctgtggg 840
tcgtcgttgt gtgtaatttg agtacgcgcg tacacgtact gcttttgttt catttgagca 900
ctgttcgtgt aagcgtcgag ttgccctgcc atcgtctctc tctctctcga tctctactat 960
ttctgtgtgt gcgcgcttct atttttccaa tgttgagtta cgcatatttg tccagtaacc 1020
ttgttttgct taatcacaca cagctcccaa attccagtcg ttctgtggtg acggttggtt 1080
gtggggtgtg gcggcgacgt cagtcatggg agaagagatc cgaatgcatg gggtgttgg 1140
cgctttctct gtccctctcc ctctccctgt cttctcgcgc gctcgccgtc gtcatggctc 1200
gacgaacaga cattcactgc cttccccgcg cgcttcagac agaccacgct tgtacgtagc 1260
gccgcttctc tagtcgccgg agcaggcacg agtgggtggc gaagaggcca cggcccgacc 1320
tgctagcgcc agcgaccgca tctcgccacc agccgctgcc cgctggccgc tgccaccgta 1380
tgttgggttg ggctgaagcc tgaagcccgt gaccttttc ttttcttta ttctttcctt 1440
ttcttttgcc ggacaagaca agtgatgttg caaaatccat ataaaatcct agagaaacta 1500
taaagttttt ttttgttgaa ctccttataa caacgtctac cctttaatat atggtatata 1560
atttcacatg ttttagtata aattgtttac tgtatttcta tgtctatatg ctcttggtaa 1620
gattgtttat aaattttaaa tttaaaatct taaaagtcat aaacttatag aaaaaaatta 1680
ggatcctaaa caattttaaa caaaaaaaaa aacttttcaa ttacaaaatc gtatgtgttc 1740
tttatgcatg catgcaactg agaagctgcc caacggccag cgcagcagaa gggcagagcg 1800
gtgggcggcc acgtcgccgc cgcaaaggaa ctgacgtgtc ccggcccgac gacgacgacg 1860
cgaaccttca ccgcgccgcg ctccacctct cttaagctaa atacgctagt gcaggcgcag 1920
gcatccagct gcgacaacgt a 1941

<210> 8

<211> 1188

<212> DNA

<213> Zea mays

<400> 8

gagcgacctc ggactcagcg gctccgcctg tcggcgagcg cacgccgcgt gcctgcccgc 60
ggtctctcgt cgtctccaga agatgcgccc cgatactcgt ctttccaccc ttcttctacc 120
tccttccacc cccttctccc cacgccccat cggatcgcac gcgcaccgca attgttgttc 180
atcacctctc cgaagaagcg tcacagagca ccgcccggag gttccccttc ccgtcgccgt 240
cgcttggcca ggtaagtaat gactaatcgc ctgagtctcc tgcgcctcat tacttcgaac 300
tgtggtttcg ttagtcggac gtcggagcaa tgctatgaac cttgccattc catgttactt 360
ggtgctgcgt gtccacattc gacgccacat ttttttcttg ctagccaacc attgcgcgtc 420
tgtgttacag ttccaccggc gatatacatt atgcgatcat ttgatgtgga tattggtgtt 480
tttttggtta gcggatcacc ttagttttgc actgaacatg gaacaaatgt ggatgttggt 540
gtttttttgt tagtggatca ccttagtttt gcactgaaga tggaacgagg aaatggggaa 600

ggggaactga acgaagcaca tagtttcaga gactatgtgc actgcagaga ctatgcgaat 660
ggtcaaggga atttctcgcc ttggtttcct aatcctcgct tccttcttgt ttcgaattcg 720
gtgtatatat gtggtttatt cggcacgttt aggaatttag ttggggaatg gggcgcagta 780
tgtcagggtt gtgtcatctg aatatataat gcaagatcta ttactagttt aaattgactt 840
attatgcaag tcgattaacc tggctgggct cagtggtgta ccatggaagc tttgtgattg 900
tagccgggca gtagaacgct aaacctgagg tttggtactg tagcactcgg ctagtcagct 960
gtatgccaag atgttactta ctcgatactt tctgccatgt ctcatctgtg ttaaccaggg 1020
tgtgtgccac atagggcttc tttccatgct gtttgttaac ctgttcacac ttcttccata 1080
tatatattca taatagctcg atctacaatt ttcctgtctt gagcttcttt gttcctaaaa 1140
acgaattgta taccagcaat gtatatctga tgcaatattg tttgtagg 1188

<210> 9
<211> 1507
<212> DNA
<213> Zea mays
<400> 9

ctacatttat gttatagagg cgcaagtaga agaatgtgtt ataagttgta catcggaaaa 60
atagcatgta aatctataga atcaattttc atctttcacc ccatgaattt gaggtatgct 120
tatatgataa ctttagaaag tggtggaatg tcatattcta aaaaaaatag cctatttcat 180
tagtaagatt ccaattcctc gaaatgaaag aaaacaaacg gggccttatt agaatggaat 240
tcaattccaa tgatccaaat ggggcgtaag ggatttccac tttcctaaga aaaattagct 300
cattttccct tggtgtttaa taattatgtt ttgttaaaag gtcagcttcc cgagaatgcg 360
ttgtgtaaaa gcccagtata agggtctgtt tggttgggct gtggctgtga aaaaagttgc 420
tgtgggctgt gagctgtgga aaaagctgct gtagactgta agctgttaaa aagctaaaaa 480
ccgtttggtg gaaaccacta aaagtcgtta aaaaatcttc gatatatgtt ttcacagttc 540
catccgaaaa gccactaaaa gcaggtccag aggtgctttc agatttgcac tacgagaaag 600
tcggttttta gaaaaagctg cttcctggat ccagcccttt ggktggcttt tggctttwag 660
gggcacaaaa gccaaagcca aaagtcaaac caaacacacc ctaagtcgcg tgagtgggcc 720
atttatctat tccctatgca aatctcctga aaaattacaa cattagctcg ttaaaaagta 780
aaataatttt aaaaaattag agactaatta tttgataact ttatagttca tcacattttc 840
attgaaaatg ttttattttt tttgccgaac tgatctacac aatatttgaa tctacacttt 900
tggcttgggc tcggagaaaa atagtgcgtg cccagccctt tgctctttcc ccgataacaa 960
cgcggtatgt ggcctaacgc ccaaatccgg cccatttatt ccggtgcgta gatgcagacg 1020
cgggagcagg tgccaagtca ctggctcact gctgcaggtg gagtggtgga cactagtgcc 1080
gcggttcatc tgacacgtgt cgccacgtgc cgccatggca gcacctcagc ccggccggcg 1140
ggccgactga cgtcttgggc aaagcggcga gcgacgcagg cggcgaaagc catccgattt 1200
gacccctcgc tagacctttc aagaacgaac gctgtgctgc tcagatcaga ccgtgtctgc 1260
ctcaaagcga tgccaggacg ccacgtccaa gcaaagcacc cgatgccatt gccacctccc 1320
agcactcacg cgtgagcgtg actataaaaa acgcaccctc tgcatccgcc cccgtctgcc 1380
tgccctaccg aatctttcgc cgtcccatca gcccagcaat tcttcgctgt tcgaggaccc 1440
ctcggtttcg accgaagccc agcaagccga ccacacaccg ctgccgttgg ttccgtccca 1500
agagatg 1507

<210> 10
<211> 910
<212> DNA
<213> Zea mays
<400> 10
catacgattt cctaagcgga atctcgagac gaaccacatt tttcatccgg tctatgagcg 60
cgctcaatcg tcagtgacag agaacactgt aggtccttaa ctggaactaa caattcagaa 120
acaatcctaa atgtttatta cactggatgg attttatttg gatagatgat actaccgttc 180
gtgggctaaa aagcaaggta aaaactggcc taacgcacgt gatcctccca tggtctcaga 240
agaaaaccac tcgcaaaaaa aaaaacgcag caatgaggct gctttcagtt gacggtgtgc 300
acgactcgct gaagccagcg tgattttgtc ccggttcttg gagcccagca aaaagggcgc 360
ctgaaattcc acaggttgaa ggggaaaacg gcaacaaaag gggctccgac atgcggtgca 420
gaaagtgaaa acgcgagaaa agcgcccgtc ctttctcgtt cccttccgtg cagttgcaag 480
tgttgccacg ccgcgccgcg acacaccacg caggaggcag cgagcgcaat aatgcgcacg 540
cagaggagca cagcggcgtc actgctgcgg cagtaatggc cgcctggttc cacttccacc 600
acggtgtgca ctagcgctac accgaggcca ccttttcttg gttccttcaa cacgcctact 660
ccggcctccg gatcgggcac cacgtctgcc ccctgctccc ggaatctttg ccaagcggcg 720
ccgctactgt ccggcaaggc gaccccgtct ctcggccggc gttgatctgg tgatgcgctt 780
ttcacggtgt ttttttttt ttttgtttca ctcccccctc cgtcctgtgt gattgtgctg 840
cagttttgtg ccggtgctaa cggaaaccgg aaagtttgcg tctttatgtt actgtggttg 900
aggcgggcgg 910

<210> 11
<211> 1131
<212> DNA
<213> Zea mays
<400> 11
ccagccatcg tgcttgagtg aatgtttttt tgcgtgtgtg taggtgtgcg tgtgtgttta 60
attgatataa aataacccaa ccaaacatct cctaagattt ccataagaca gagaggtgag 120
gaatagtatt agttctcaaa tgaattcaat caactaaata tcaccatcat atcaacatta 180
ataatagaac aataaaattt tcatgataga gaggctggta aactttattt tttcatttga 240
ttccagtgtg gaaaatagta atataagcag taaatttttt cttaaaaaag agtggaatat 300
tcaccttttt atataaatta gttattcttc gaagcgtcac acacggatgt cctaaagata 360
ttcttcaact gcaacacaag cattctgaaa aggttagtaa caatcagatt aatgttgtaa 420
aaatatgtga cacgaaaatt taaagaacgc gaagtacaat acattggtaa gaaatcaaag 480
acacatatta accaactatt agagaacata tacatgtttc ttctaatcct ggttaggaca 540
taggacgagg gtttcacttc tatcacaatg tgctgcaact ttcagagtta atcaaatctg 600
ttattcagtg ttatgcagcg acaaattcca aatgtataat cccacgatga ttcatgtgac 660
tccatttcgt ctgtaattga tgcgaaactg ccgaattgtt actgctccca taagttaatt 720
gtaccacgct tgatgaagac gttcacgtat cttgtgttcg atttatgtag ccgttattca 780
gaccattgca gcgctgagct tcaaggcaat tagttttgcc ccgcgatgtg aggcaggtga 840
tgacacgcgc ccttcgggcc gatgccgacg cgccaccgtg cagactctac cgaggatgag 900

agcagcgatc gagagacgtg gagcgaggag gagactttag gagaggagag accagagcct 960
taagcggtga cacacccaat gatcaggacc acgcttgcac gtgtcaggtg cctctcctcc 1020
acgcgtcgcg cgcatcacac tgatactgtc atccagctcg acgctatata aagtggtaag 1080
catcttcgtc ctcggggccg ccccaacggc ccttgcgatc gccaccacgt c 1131

<210> 12
<211> 563
<212> DNA
<213> Zea mays
<400> 12

cattgttata catcggtgat gcagatcttc gcttcgcatg ggtggcaatt tagtaacaga 60
attgctctat gagttattgc ttttagtttc tgtctttccc caactgcgtt tgagtttgca 120
ttgtcacatg tggccttttt tggttgtaag acttgtttga tcggttgtgg tgggctgtca 180
tcctttggat gtaggccgga acctcttttc ccattatcta aaaaaataca tcgtcatttc 240
cagtgagacc cagcggtcgt gtcatcagaa aagcggacac gcgtcgcgtc gcgaggccag 300
gtgggttatg cacggacctg ctatgctagc catgctccag gctccagctc cacccgtcca 360
ccgacttttc ttctcaacca ggctgcctcc tcaccatgca cgacgacacg tgcgatgcca 420
tcacgcatcc acactcacct gttgcctcta tatccaccgc cccaccgccg tagcaccaga 480
aagaaatgta cagtctacac agctgaccag agagctagtg caagcgatct agcaagtttt 540
aatcttggaa gaagccagct agg 563

<210> 13
<211> 991
<212> DNA
<213> Zea mays
<400> 13

atcatcaccc taccccgagc tgactcgggc cgcagggaac cagaccggtg tcccatctgg 60
ctagctccgc cagataggca atgaaggcgc cccgcatgct ctgtgacgac ggcggctctc 120
agccccctta cggaagcaag aggacgtcag caaggaccca accgctccga cagctgtccc 180
tccgccaggc tccatcgctc ctccgacggc cacgacatcg caccagctgg gtgccaaaat 240
ctctccggct gccacgacgg catgtactta gggcgctagc tctcccccgc tagacacgta 300
gcactctgct acacccccca ttgtacacct ggatcctctc cttacaccta taaaaggaag 360
gaccagggcc ctctcagaga aggttggccg cgcggggacg aggacgagac aggcgctcgc 420
ttggggccgc tcgctccctc tcccacgtgg acgcttgtaa ccccctactg caagcgcacc 480
cgacctgggc gcgggacgaa cacgaaggcc gcgggattcc cacctctctc acgccggtct 540
ccggccgcct cgctctcccc ccttcgcgct cgccctcgcg ctcgatccat ctgggctggg 600
gcacgcggcg acactcactc gtcggcccaa gggacccccg gtctcgaaac gccgacaata 660
tgatatatag aaactataat gatacacatg caattaacta gtgctgataa tatatttaca 720
ctgtagattt ttccgataag tagaaaccaa atatttcacc gccacaaaag gcttcatccc 780
atcagttacc catgtagcaa aaccaaaata cttgtgtgcg agaggccaag agcccaaacc 840
accgccaagg aaaccccca cccaccaggc gccttcctct ataaataccc taactccggt 900
ggccgaagtt cctcacccat tcactcactc atctcaaggc ctaggtagca ccgtagcagc 960
tactagaagc agctagccag aacaactcgt c 991

<210> 14
<211> 2519
<212> DNA
<213> Zea mays
<400> 14

cttccgataa aaatatttgg aaccgcatcc tgcatattta tttagaaaca ttgcatcgat 60
aaccgttgca gagcaatata cagggtactc accaactaaa tccataccat acagtgacag 120
gtgaaattgg aatacttgat cagcttgaat aacagagagc aaagataaat ctagccctgg 180
taataaccat ggctcacatc ttatgtacaa gagccaattc taaagacaca gctgcgggat 240
gtagcagtta ggcaaggaaa gaaacaaaat taccagctaa ctgctaagtg tcaaaacatt 300
gctttacact aaagggttaa atatttgcca agaaggtagt gggcacctct cctctgcaag 360
cagcagcata atctagggcg aggcttggtg cagcttcctt gtaggggcat atgaacttat 420
ccatgaaggc actctcactc atctggacag cagtatagta gctccggtcc tgctccaaca 480
atccattttc cttcacgcct agtttggatc actgtaattg aattccattc taataatagt 540
aatttagaca tatatcaatt aagataattc actttgtac aaaatatatt tgtatactat 600
tattagcaat atatcctaaa tatttatgtg ctacattttt actatagagg agtagaacga 660
agagtgtcat ataagttata gattagaaac aaattctaat catgcataaa atcatttccc 720
atcctccacc ctatgaattt gagataggct tatatctgaa ctttagaaag tggtggaatg 780
tcaaattcca aactaaataa gttactttat tgagtgaatt ctaattcttt tgatttgaag 840
gaatccaaac gccccgtcag gtaattcaca acatagtgtc ggggcatgag gcggcgttcc 900
aagctatatg taagcagagc aggcctgtga gcaatgtact ctggctccaa cccaaccttg 960
gtgatcagga acgcggatcc gggcgtcgcc gggggcgcga ccctgtgctt gccaaattga 1020
aaatttagca aagtattttt tttaccttgt tgattatgtt ggataaccgc atgtccctaa 1080
tttgaatgat attgctcctt atctctaaac gaccaactgt agcttgttct attgaaaact 1140
catcagaata atccatagag ataacatcac caacaaggac tgaatcatcc agagatgcat 1200
caatggccat tggagctagg gaagtcggcc taactacctt caacccgact tggccaactt 1260
gtacagtaga agacaccatg gaaatcgatt taacggactc taagggagtg tttgaataca 1320
ctaaaactaa tagttttagt ggctaaaatt agttggagac atccaaacac cctagctaat 1380
attttagcta ttaactattt ttagtaaatt agttacaagt tagctagcta tttattagct 1440
agctaattct actaacaaat ttttagctaa ctaacgcatt caaacactgt ctaaacaggt 1500
aatctaggca cgcgaagtct gactggccag cccaaactca gcctttgtag tagagagagc 1560
cgaccttggc ggcctgcacg acatttttga actttttta tcggtttggt cttcattact 1620
ttatattctt tgtcaaccaa atactccctt cctcttaaat tataattcgt ttgatttttt 1680
ttatgtaaag ttttatgtat agtccaccta gttaacttta aaagttggtt agtctagatt 1740
ttatggaggc cgactacaat agcaaatcta acaataaaaa agttagctag atcagatttc 1800
aagaacagtc tgaccgattt tgaagatttg attcaggtta attctacgat tttgaagatc 1860
cgacctaacc taattttacg ttcagactaa agacatgttt gtttaagatt atgatatgtc 1920
tagttatata atttaactta ttttaaacta acacttaatt taaaataagt tagattatat 1980
gatcagaata ttatatttat ataattccaa acaaataact ctaatatcga ttctggttta 2040
tattgtataa tttttatgtt gaatattacg gcgcgccaac aaattgcgat cgttatgtaa 2100
tcatgtatac gaccgactgt actattcata aaatataatt caaaatagga gatgctcgag 2160

atagcctggc aagggaggag gtatcggagg cggcgtcgcc tgccgcgagc gcaccatgcg 2220
tcaacgctca ggacctctcg caggcgcgct gttggatcgt gagcaagcac gcgtggcaaa 2280
tggcatgcat tcctctcgtg caagtgccga gctgagggtc ccgacgcggg gggcaggacg 2340
tggcagagcg ccgcccaggc ggacgccaag tgccgagcta ccagggcacc ttctttattc 2400
cgtccttgct cagtcacacc tcgctctcgc tcactctcgc cgtccgcaca gccgctcatc 2460
gtctcccact gcctgccctc tccctgcgcc ggccggtgcc ccgacgcgcc atgtcgtac 2519

<210> 15
<211> 1264
<212> DNA
<213> Zea mays
<400> 15

tcatcggtac tcgcgatgtc gtgcgcgtcg acgactacta tatcaaggac tatgacgtca 60
tcttgacacc agaggtgaca cagaggagga ccaacaacaa taaccactcg ccaccgtcgg 120
tcggtctgcc tttaattctc ttcgcaaaca catacacttg cttttttgtt taagcaagcg 180
tgtatgatgg tgcgtacctg atgactgaca atgtacgagg gaacttctac cggcaggtgt 240
aacacgtgct cttcaatgat aagatcatgc aaatcgtgtc atatatcgaa gcctgcatga 300
tactgatggt tgaaatatag tagtgaaaca gctaaattaa aataacaaaa tttatgtatg 360
gctaggatta caaataaatt ataaaatatt tttttataac aatataagac acattttgta 420
tataagttat tatattatta tatgtttctg ttgcaacgca cgagtactca cctagtatat 480
ccataggctt ttgatcctct acgtcgagtt tttgcttgca tgagagtcga cctcttctct 540
tttatccctt cttctctatc ttagcatttg ctcgatcaac tgtaaactaa ttattatacg 600
tacttcaagt catattgaaa ccaatggcat ttaaaaaagt aaccatctga atggaaatat 660
caagctaaat aaagaagtta cgggagtatg tgctgagaaa acctagacac attcagagaa 720
gttaccaagc aagccttaaa taacaatttt tggtgcttta ttgcagatag atactcctat 780
aggttggaga gaagggcggc gatgctaatg caccatgcat gtgtgtctgc ccatatactc 840
tatcttatct gaatcctacc aaactcttta atttgactct ttatgaatgg aattcccata 900
tgaattctat aacacaacag tacgacgtct actggagcat actgccaagc acgctagccg 960
acggctcggc gaccgtctga ggctgtccat gcatgcaaat gtgcacacat gtacgcccct 1020
tttagcgctg gcatgcaccc acgaccacga cacggctccc gtggccgtga atctgacatc 1080
ccgggactcc cgcctccggc cgcgcgtgcc gtgtcagcct ccatcgagcg gtgtactcca 1140
tgcaagcctc gacccgggcc acgtaccccc ctcccctccg ctacgtgtca ccgctctcgt 1200
cacctatata tggcggtctt gtccagtctt tccacttaac tactccgtca ctttgtttgc 1260
aaag 1264

<210> 16
<211> 623
<212> DNA
<213> Zea mays
<400> 16

ctcacacaaa tctaaatagt aaagtagatc tggttacaga gcagaaattt tgaagtgtca 60
tagataggct gagaatatgt agtcacgagc aacacattac tacttgaggc catcaatact 120
gttttttctt ctccaaccta tgtaactatg ttattctttt cctccttaaa tgtaaggcaa 180

acctcctatc ctttttcttt caaattcatt gcattaactg agtggttctt acatgtagaa 240
atgaagttgg agcaaagagg cttggtgcgg aaccgaaaat ttgttagtct ataaatcatt 300
agagggcgcc tgaagttcgc caaatgttct agtagatttt agtaaccacc cacgctagct 360
gactcgacca ccgtctgctg gctggcgctg tccatgcaca tgtacagccc ctggcgctgg 420
cacgcaccca cggcatacct cccatgactc tgacagcccg ggactccctc cgctcgtgcc 480
gtgtcagcct cactcgagcg gtgtactcca tgcaagcctc cacctcggcc acgtaccccc 540
tccccttcct tacgtgtcac cgctctcgct ccctatatac ggctgcctgg ccaggccttt 600
ccacttcact actctctctc tct 623

<210> 17

<211> 1950

<212> DNA

<213> Zea mays

<400> 17

ggttcaagat atgtatgtga tgaacataat tttactcaaa agtgatggtt tcattgtact 60
aactagcatc aatacttatg ccagagtgga gcagtgtgtc tatgcactac tccgtacaag 120
agacatgtcc ctcacgctac aaggagtatg ggatcccagt tgactggttg tctgattccg 180
gagtagttgg caaggtgtgc atctctagca tacactccaa tttcgccaaa catgtgttaa 240
aaacatgcat ttttaattcc ttatgaaggt gggtatgatt ttactcagag tatgcaccta 300
aatgaactca ataatttatt tgcatgttgg atatatcaaa ttggcatctg ttcagcttgc 360
gaacaagtat atgaagaggg ttgcatcaga acttgatgcg ttggaaggca ctgagaaaga 420
gcccaataga gagtttttgc ttctccaggg tggcagggtg tgaggttctc tttccgtgtt 480
catcaggtaa aatcctcacg ccaatatttt cagaacccag gtacttcaca ggtgatatga 540
atatttcgaa tcagtaactt attcatctac cttctgcagt ttgctggggg atttgatgca 600
gaaagcatga aagcttttga agagctaaga agcaagatga gtacacataa atctgctcca 660
cagatatctg aaggctgaag catgagttga acacaggatt caacacacat ctggtcttac 720
ttgctggata ctgattttta gttgtgtaca aaagagaagt ttaaattcca gtgtggtgtc 780
aagcaagttg tatatgctaa gagaatacaa gtcatcaggt tatgttcatt tattttttat 840
gaaagtaagg gcaggacagg ttatgttcat gcatataaca gttttagctc aagtgtatac 900
cttttcattt taaaggctgt aactgtaaac aggcagctcc agaaaatgaa tcacttgggc 960
atgtttttgt tgctacgcat atactactaa taaaatgtca aaagcgtcca ttcggttagt 1020
ccggtaccca tggagataat tcgtctgaat gaaccttgcc gtctttgtta aaagaaaaaa 1080
aaattacttt gtgcctcacc gcattcgata ctttgaagaa attgacccgt taccagttac 1140
caccccacgca catacagtgc tagaacagca gatctgactt gaagaagcat atatcaagac 1200
aaaagtccac ctgaacggag acagtcgagg ttgtggcgga gagctccacc tgaacgccga 1260
agtggaaccg aacagttcca gtggttggcc gtggcgagct ggccacctac tagtctccgg 1320
ggaggggggct ggctccagcg cctccgagcg cggtgccagt gtcgccgtcg cgtgggttcc 1380
ggttccggcc gccgccgctt gccggggaat ccaaacggca gacggggtcg ccgtgaccgt 1440
gaatcgtgtt tacttgggcc ttatctaggc cacaacgttt gtgactctgt gagcttattg 1500
tttgggccca tctctttggc gtgtctgaga acgaatttta tcatcaagcc cacgaagcca 1560
aaccaggatc ctacccacct agcacatact agtgaagaag gtaaacaaag tgtattttca 1620
tgttaattca catgcgcgtg gtcgacacca caccacacga cttaaaactc ggtgttatgt 1680

tctacgacca caagagccat gttaatttta agccgtgtct gcgccatgat ccgaagagcg 1740
cgtacagtga gagaagaaac gcacggccgc cagcagctgc cgcgggagcg aggcagcacg 1800
tccgttggtc ggcccgggcg ggagcgaggc acacgtcgcg catgcacgcg cggcgcccgg 1860
acacgtgccg gcgcgggcac gtccctctcc gcgccttata ctcctccccg ccatttccca 1920
gcagccacaa caagacagag agatacccga 1950

<210> 18
<211> 2112
<212> DNA
<213> Zea mays
<400> 18

ctaggttggt gagatcctta ggttttggaa ggctttatgt ggcagctctg aagactccaa 60
catctatggc ctctagctat gcctccatat gtattttgta ataagaaaaa tcatctccct 120
taaacatggg agagggttca tctccatcga acatctttct ttaggtggtg aagcctaaaa 180
taatgagcac gacactttga tactaattga aaggattact atgcccaaga ggtagggtgg 240
attagaccaa tctaaaattt cttgtagcaa taaaatctta tcgaatatcc cacttcacca 300
cttgtgctta agagtgatat atattctatc acaaaaggtt ttacacccta gtttgaaccc 360
tattctagta tgttagttct agtaatgtaa agacaagaat taaattgcta aaatgcaaat 420
gcttaagata aagaagggtt agaaacatga cagcattttt ccaaagtatt aaagagccgg 480
gactctccaa tagtcctcgt tggagcaccc acacaaggat attgctcccc attgaacttc 540
acaaggatca agggctctct atgggtttgt tcctcgccac tctagcatgg tgagtcaccc 600
acagttgttt acaccttgag tggtgatata cactagattt cttaagtgtg tatcatcaga 660
cttatttagg tcaaactatc attcatggcc cctttatagt atagtcaaag gaaaaaacaa 720
aggtgctatc actagtatat atgtctatca actctattaa ctattagaac tagccagtcc 780
ttaacctttt tgctcatcct tctaaaatca ataattgaat accatcaata ggggcacaaa 840
aactatatag ccaattatta ttactcatag ccaataattt agtgttgtca ttagtcacta 900
aaactagatg ctttcatatc tatcatgctc gtgtttgcct gatccaattc ctaggtcatg 960
tttgggctcc acttagagct tgtaatggca tcacaacatg atctgattaa gaaaatgttg 1020
actaaagacc ttctcaccca catataaaat gccatgactt cgctcaaact tcatccccta 1080
gtctcctact acacccatgt ctatgcttcg caacttcaca agtctaccac atgtacttgc 1140
aaagggctga ttctcctagc tcccatgtcg tcgccatctt tcccctttac caccacaaaa 1200
tcacaaatgt gctcacttat tatccccgcg tcttcccttc cctaccctaa acactatggg 1260
caacaacgtc gcatcacttg gtggtccttg tagatctaga cgaggtctcc tccccgtttt 1320
gtgcttgttg atatagtgat cttgtccttg tcttgtcgct gtcctttgga gtcgtcgcag 1380
agaggcggtg aaaattctag ccgcttctag aaggtgcggt cgccggtcgg atgagagtcg 1440
agtgagtgca ggcctataaa aagtagcaat tggataacat tttgaaaaaa atgaaagaaa 1500
taaaaaaact agggttaaat aagattctaa attaaatcta agaaatctat agacattcgg 1560
aaatctattt atcttttgta ctcctttgat tggatttcta gattttgacg tttgtgtaat 1620
tgggcttgac agccccatca atacaggcag gccagctgat ttcgggtgcc gctgatttgg 1680
cccatggagg atagagcagg cctttcaaca ggcaggcagt ccatgacgta gtgcctcggc 1740
ctcaaagccc atgcgattaa tatccactgg tacgtacgcg acagccgagg gagtcactga 1800
gtcagtgccg aacggcctgg aaaccccccg ccgtgtgccg gtgtccacgc gcgccccggt 1860

gcggccgcca cgaccgcgga cacgtgccgg gcctcgcaca cgcgtccccc acggccccct 1920
cgcatcatta aaaccgcggt gcccgcgcca cggatccccg gcacttcatt ccggtttccg 1980
gatagcttac ctcccatcga cggtcggtga gccgttggtg agtggcgaga cagcgggtgc 2040
gctgggcaag aacaggcgac gaagaagatg agcacgccgg acacgcggac gctcaaggac 2100
gagctgacga gc 2112

<210> 19
<211> 456
<212> DNA
<213> Zea mays
<400> 19

atggtgactg tggagggaag aagaggctct agtgatcaga caggcgacat gaggtacgtg 60
agtgacttcc ccgtctacga tggccgtggc tccgacgccg tcctggtggc tcgcgtgcag 120
ggcgtcacaa ccacgttcgg gaactccaac cagttcttca ccgtcgcctt cgaggccggc 180
agcaggctca agggctccac gctccttacc gaaggagtgg tgacggatgg atcagatgaa 240
tgggcaatct acggcgggac cggagagttt gctatggcga gaggtgtcgt caaaagaagg 300
taccttgccg atagggatgg cgccgggaac actgacgagc tcagcatgca ggttttctgc 360
ccggtgtttg gctcatcaca gcaaccaaac aagcaggact ccagcatctc tgtcaccaag 420
attgggttat ggggcgccga ctttgatttg atctga 456

<210> 20
<211> 492
<212> DNA
<213> Zea mays
<400> 20

atggcgcggt tcgtggaccc gctggtggtg gggcgggtga tcggcgaggt ggtggacctg 60
ttcgtgcccg gctccaagga catcagcaac ggctgcctcc tcaagccgtc cgccaccgcc 120
gcgccgccgc tcgtccgcat ctccggccgc cgcaacgacc tctacacgct gatcatgacg 180
gacccccgatg cgcctagccc cagcaacccg accatgagag agtacctcca ctggatagtg 240
attaacatac caggaggaac agatgctact aaaggtgagg aggtggtgga gtacatgggc 300
ccgcggccgc cggtgggcat ccaccgctac gtgctggtgc tgttcgagca gaagacgcgc 360
gtgcacgcgg aggcccccgg cgaccgcgcc aacttcaaga cgcgcgcgtt cgcggcggcg 420
cacgagctcg gcctccccac tgccgtcgtc tacttcaacg cgcagaagga gcccgccagc 480
cgccgccgct ag 492

<210> 21
<211> 1278
<212> DNA
<213> Zea mays
<400> 21

atggcgcagc ttagagcagc cgccgacgag cagcaggagg cggcgatgca cggcgtcgcc 60
tccggcgacg ggaacgagag caacaagaag gcgcgcgcgg ggctctgtgg cctgctccgc 120
gagcgcaagg tggtggacct ggcgcgggcc aagcggcggc tggtggaggt tccctacacc 180
gcgacgctgg cgcacacggc caacgcgctc ctggcagcgc gcgtctctgc cgtggccgtg 240

gctgcgccac cgggccactg gatcggcgcc ggcggctcca tgatcctcga gtccgacccg 300
gccaccggcg ccgtccgcaa gcactacatc ggcatggtga acatgctcga catccttgcc 360
cacatcgccg aggccagcga cgacgacgag gcggccgacc tcgaccgccg gatggccgtg 420
ccggtgtcct ccgtcatcgg acactccctc gagggcctca ctctgtggac gctccacccg 480
agcaccagcg tggtggactg catggagacg ttcagcaagg gtgtgcaccg cgcgctggtg 540
cccctggaga gctcggcgga caacgtggtg gcgctggagc tggtggagtc ggcgccgggg 600
taccggatgc tcacccagat ggacgtggtg aggttcctca gggcgcacgg cgcggagctc 660
aggggcgtgc tgttgcgcac cgtgcgcgag ctcggcgccg tgaacgacac cgtgttcgcc 720
gtctctggcg ttaccaaggt gatcgacgtc atcaaggcga tgcgggcggc gtcgctgacc 780
gccgtgcccg tcgtggacgc cgtcggtagc ggtacagaga cccttcaata cgggagcggg 840
caaagggcgg tcgagacgtt ctcggcgacg gacctgcggg actgcccggt ggcgcggctg 900
caggcttggc tggggatcag cgtgatggag ttcaagagga aggtggctga gtaccgcgcc 960
agcaccaggc ttgtggtccc cggcgccgac gccacggaca ctggcacccc tgtcgccggg 1020
tacgccgaca cccccgcggc tgccgccgcc actgacgaag agcagagcga gcagcaggag 1080
tcggcgctgg tgacgtgctc ccaggagagc accctcggcg aggcgatcga ggcggcgaca 1140
tcgaggcacg tgcaccggct gtgggtggtc gacgaggaag ggctcttgcg tggggtcgtg 1200
tcgctcaccg acatcctccg ggcagtgcgg gaggccgcgc tcggcgagga cctggagctg 1260
cacagcatcg tgccgtga 1278

<210> 22

<211> 975

<212> DNA

<213> Zea mays

<400> 22

atggcggaga accctgctcc gagttccccg ctagtgactt ctcccgacgc cgctgcgccg 60
cctctgccag cggagttcac caagctcacc ttccgtagcc tgtacatccg tcggacaggt 120
ccaggcagcc gggagatgac tgtggacggg aggccgtctg accagctggg acgacggttc 180
gtgagcgact ttcccatata cgatggccgt ggctccggcg cccgcctcgt cgctcgcttg 240
cagggagtca ctgtccaaat cggcagctcg caccagctag tcagcatcgt cttcgaggcc 300
gagaggctta agggctccac gctgctcacc aacggagtga taacggatgg gtcagacgag 360
tgggccatct acggtggcac cggggtgttc gccatggcca ccggtgtcat acgaagaagg 420
tttctcgccg gtagcaacga cggcaactct gatgagctta ccagcatcga agttttctgc 480
ccggcgtttg gctcggcaca acaacagcca aacaagcagg actccgtctc tgtcaccaag 540
attggggtat ggggtggagg aggagggtcg gcgcaggaca tcacgacgac ggagccacca 600
cagcgtctgc acagcatcac cgttcgaact ggttttgccg tcgactccat cgagttcacg 660
tatactgaca gaggtggcca gaggcgcact gctgggcgat ggggtggact tggcggaaac 720
cttcgaacga tcgatcttgg cgacgccgag gttgtcaggg aggtctcagg aacatacggc 780
acgtttgaag gcgccaccac gctgacctcg atcagaattc tcaccagcag cagaacctgg 840
gggccatggg ggatcgagga cgggacacgt ttctgcatca ccgcgccgat cggcagcagc 900
atcgtggggt tctatggacg ctcgaccagc aggctcgtcg ctgcgatcgg tgtttacctg 960
cgccaacaac tctga 975

<210> 23

<211> 573
<212> DNA
<213> Zea mays
<400> 23
atggctccca agcggcgcgg aaacaaggtg gtgggctccg tggtgaagac caaactagtg 60
caggagaccg tggaggtcat cgtcgccgac gacgatgggt tgcacgcgga gaagcagcag 120
gtcccggagg ctctggccct ggcgcacccc accgtggacg tctctggctc cacggtggtg 180
catgtcgtcg aggtcactgc caaaagaggc cgtggtggtg gtggtgggga agaaagcagc 240
aacaccaaca aagaagacga agacgcagca gccgcggcgg cggcggcagc ggctggggca 300
ggccagctcc ggaggcgacg ccgggatggg cggcgtggga gggtacaggc ggtacgtgtg 360
gcgcgtgctg aagcaggtgc acccggacct gggcgtgtcg ggcacgccca tgcaggtgct 420
ggacatgatg atggccgaca tgttcgagcg cctcgcggag gaggcggcgc gcctgtccaa 480
ggccacgggc agggcgacgc tcacctcccg cgaggtgcag agcgccgtca ggctcgtgct 540
ccccggggag ctgggcaggc acgccatctc tga 573
<210> 24
<211> 351
<212> DNA
<213> Zea mays
<400> 24
atgccgtcca ccgtgaactc cgcgtccctc gaccagaacc ttgagctcgc caagcaatgc 60
tcgcgagagg ccgccctcgc agggttcaag gcagcagctg tcgcgaccgt cgcctctgca 120
gttcccaccc tggttagcgt caggatgctg ccatgggcca aggccaacat caaccccgct 180
ggccaggccc tcatcgtctc cacagttgct ggcatggcgt acttcatcgc ggcggacaag 240
aagatcctct cgctggcgag gcagcactcg tacgagaacg cgccggagca cctcaaggac 300
acctccttcc agggcaccgg ccgtccgcac ccagggttct tcaggccttg a 351
<210> 25
<211> 390
<212> DNA
<213> Zea mays
<400> 25
atggcgcata gcaagcgcga aatcaagccc gggagggcgc ataccaaggt ggccggcgac 60
gacgagatgc tgcggaccgg gttcttcgac ggcacgccgc tggagggcgg caagatcgcc 120
gactcccagc ccgtcgacct cttcgccgct gcccgccgcg tcgccgacgc cccgtactca 180
tcgcagcagg aaggaccaca cgaggagggg accaacaaga acgccgtcat cgcggagtcg 240
gagcccgtcg acctgcccgc gagcgcgcgg ggcgtggcgg aggtgaaccg tgctgggccg 300
gaggagcaga caggccgcgg tcggcagggc atgaccgagc ccaccgccgg tggacgtcgc 360
ttgggattag gtcgcccggc cccggcttaa 390
<210> 26
<211> 1737
<212> DNA
<213> Zea mays

<400> 26

atggctgaac aattctatac cgtggcatcg gatagtgaaa ccactggtga agacaaatca 60
cagccttcat tccctgatgt tgctgttggc attgacattg ggacttccaa atgcagtgtt 120
gctatttgga atggccacca agttgagctc ttaaagaata cccgtaatca gaaagggatg 180
aggtcatatg tcatgttcaa agatgatact ctttcagcag gagttactgg aggtgcagcc 240
aaggaaaatg cacacgagga aagagacatc ttgtcaggaa gtgccatatt caacatgaaa 300
cgcctgatag gaagaatgga cactgatgaa gtggttcaag ctagtaagac cctcccattt 360
cttgtgcaga cattgggtat cggtgtgaga ctattcattg cggccctggt gaacaacatg 420
tggcgatcta caactccaga ggaggtcctt gccatctttc tacttgaatt gaaggccttg 480
gtggagatgc accttaagca tcccgttagg aatgctgtgc ttacaatacc agttgcgttc 540
agtcggtttc aacagaccag gattgagaga gcctgtgcaa tggctgggct gcatgtgttg 600
aggttgatgc cggaaccaac tgctgtggcg cttctgtacg ctcagcagca gcagcagctt 660
gtgcatgaca atatgggaag cggcatcgag aaaattgctc tgatatttaa catgggtgct 720
gggtactgtg atgtggccgt ggctgccaca gctggaggtg tttctcagat aagagcatta 780
tcaggatgca cagctggtgg ggaggatata cttcagaaca taatgcgcca tgttcttccc 840
aattttgacg gtatatatgc tggccagaca atggatagaa tcaagtcgat gagcttactg 900
cgaattgcta ctcaagatgc aattcacagg ctcgttagtc aagaatccgt ggagatcaat 960
atagatttgg ggaatggcca catggtgtcc aaagttctag accattcaga gtttgaacaa 1020
gtcaaccggg caattttcga caagtgcgaa aagatcatca accagtgctt ggctgatgcg 1080
aagttggtac cagaggacat caatgatgtc atactggtcg gaggttgttc gaagatcccc 1140
agaatcagaa gccttgtcct gggtttgtgc aagaaggagg tctcctacaa gaacatagat 1200
gctctagaag ctgctgtttc aggtgctgcg ctggaaggag ccatcgcttc gggagtaaat 1260
gatccttcgg ggagcttgga tcttctcacg atccaggcaa ctcccatgaa cctgggaatc 1320
cgtgccgacg gtgatagctt cgctgcgatc atccctagga acacgacggt tccagcaagg 1380
agggacatgc tgttcacaac gacacacgac aagcagaccg aagccctaat tgctgtgtat 1440
gaaggagaag gggaacgtgc tgaagataac catctgttag ggtacttcaa gatcactgga 1500
attccggcag cccctaaggg agcagttgat atcagtgtat gcatggacat cgatgctgcc 1560
aatgtcctca gggtgttcgc aggggtcgtg aagcctcaag gcccagccat tccaccgttc 1620
atcgaggtga ggatgccaac gctggacgat ggccatggct ggtgtgggca agctctagca 1680
aagatgtatg ggaagactct cgaccttgct gttcttccga agaagctgca gccatga 1737

<210> 27
<211> 423
<212> DNA
<213> Zea mays

<400> 27

atgggcaagt cctggtcgct catcagccac ctccacaccg tcgccgggcc aagcatcacc 60
ctgctgtacc ctctgtacgc gtcggtgtgc gcgatggaga gcccgtccaa ggcggacgac 120
gagcagtggc tgtcatactg gatcatacac tccttcgtca ccctcctgga gatgctcgcc 180
gagccgctcc tccactgggt acccgtgtgg tacccggcca aggtgctgtt cgcggcgtgg 240
ctggcgctgc cgcagttcaa gggcgcctcc ttcgtctacg agaagctcgt cagggaacag 300
ctccggaact accgcgccag atacccgcgc aagggcgccg ccgccgccgc cgtcggcggc 360

gacgacgacg accataaggt gcacatagcc aaggctgagg ctgagcacga ccatgtgcag 420
tga 423

<210> 28
<211> 1320
<212> DNA
<213> Zea mays
<400> 28

atgggcatct cggcgaggtg gctcaagtcg ttagttgggt tgaggaaggt ggggcggcag 60
cagcagcagc ggcgaaagga tgatgcagat gttggtcgaa tgaaaaccga tgtcgccgac 120
cagtttcatt ttcagattca gcactctcaa gatgacaata gcattgctgc acaagaaatt 180
ccagaggttt cttatggaaa tgatccacct gaagatgatt ctaatgtacc ttcatgcttc 240
gagcctgctc gtagttcagc tcatatgcca ttttgtcaaa ctgaagaagc ccagaaagag 300
atctgggctg ctacaattat tcagacagca tttagagctt tcctggcaag gagagcccgc 360
cgagctttaa aagggttggt taggcttcaa gcccttgtaa gaggtcatat agtaagaaag 420
cgagcagcta cgacactccg ttgtatgcaa gctttggtca gggttcaggc ccgtgttaga 480
gcaaggcgag ttcgcatggc tttggaaaac cagactgatc ggcaaaatac ttcaccagag 540
cacacgatcg aggcacgtgt cagagaaatc gaggatggct ggtgtgatag tatcggttct 600
gtgggagata tccaagcaaa acttctaaag aggcaggaag cggcagctaa acgtgagcga 660
gccatggcct atgctctagc tcatcagtgg caggcaagtt caagacaacc tacagcattt 720
gaacctgaca agaacagctg ggctggaat tggctagaga gatggatggc tgttcggcct 780
tgggagagtc ggttccttgg cacttacggg acagatggga ttttgtagt taatgaaacc 840
agacaacctg acagaagtgc aaccaagacc ccatacagaa aacctgttaa aaagcatgat 900
tcaaccctc aatcaaacat gttgaaccag aaggtcttcc catcaaactc agagggtggt 960
ggctcctcga caaatcgatc aagtggtttg gtatcaacta aatctagact gaaggtttta 1020
cccagagaag gttctgagga agcctcatct catccttcag taattgtgcg gtccactagt 1080
aatccgaagg agaggatttc cagtattaat tcaaaggaga ggactgggga cttggatttt 1140
caagttcata aaagatcctc cttgcctagc attggtccag aagctggcaa acacctgaca 1200
aagaaaggca tggttaaccg atccctgaag gccacaaaag attcccaaat actggggtca 1260
aggcatcatc ttgccagttc cattgatcca gttcccacca gagttgaact ggagacttga 1320

<210> 29
<211> 498
<212> DNA
<213> Zea mays
<400> 29

atgcacggcc accagcagca gccgtaccca tacgggctgc ggcaagaccc gctgcggcgg 60
atgcgaggtc tggacgtgaa atcggcgctc caggccagcc cgcgcacgtc cacggtggcc 120
acggccgcac tcgtcgtccc gctgggtgcc gcgctgctgt gcgcgtcggg gatcgcgctg 180
gctgcgaccg tgaccggtct cgcgctcgcc acgcctcccc tcgtgatttt cagcccggtg 240
ctcataccct tcgcgctggc cgcggggctc atcgcgtcgg ggctcctcgc gtcgggcgcg 300
ctcggcgtcg cgggcgtctc ggcgctcacg tgggccgtcg ggtacgtctg gcaacggcag 360
ggcgaaggcg gcggcggggt cactgggatg gtggtgcagc cgctggacca ggggtcgaag 420

cgccatggtg tccaaggcgc cgccgcgttc gtggggcacc gccgcctgcc acgggacatc 480
gacgttgctg gtgaatga 498

<210> 30
<211> 1569
<212> DNA
<213> Zea mays
<400> 30

atggatcgcc tcactgtgat ggcgccgccg ctgctcgtcc tcctcctgct gctcctctcg 60
cggtgctcgg cggcggcgtc acggcgcggc gggggctggt gggaggaggg cgagggggag 120
tggaggccga gcgaggagga agagaagggc aaaggcaaag ggagggggct gttcctgctg 180
caccgggtgg agaaggtggt ggagtcggag ggcgggcagg tgcgcgtggt gcgcggccag 240
ccgtggccgc cggcgtcctt cgcctgccgc gaggggctca tgcacatcgg cttcatcacc 300
atggagccca agacgctgtt cgtgccgcag tacctcgact ccagcatcac tctcttcgtg 360
cagcgggggg aagcgaaggt tgggtatatt cacaaggacg agctcgtgga gaggaagctc 420
aagatgggcg acgtcctcca catcgacgcc ggctccacct tctacatggt caaccccggc 480
aaaggccaga ggctgcagat catatgcagc gtggacgcct ccgatagcct tggctttgga 540
cctccttatc aggccttctt cctcggcggc gcaggggacc cggcgtcggt cattgccggt 600
tttgggccga agacgcttac ccgtgccttc aacgccacct acgacgagtt ggcgcgtatc 660
ctgttgccac gaaccggcgg ccccatcgtg tactacaccg cggacgcgga gccagagagc 720
ggtgccgccg aagaggagcg cgggcaggtc gacgggcacg acggtgtgct ggacagggga 780
gcgcgacgcg agggcgccgg cgcgtgggtg ccgggtggca ggggagacgg aggcgacgag 840
tgcggcggca gcgatgacgc gcgtgaggcg acgtggtggt ggacgaagct ggttaacagg 900
gtcgtcggag gggcggctgg cggcggcggc gctgcggagg cgaacaggaa gggcaagaag 960
aagaagggcg gcgcgccgga gccgtacaac ctctacgaca gcgagcccgg gttccggaac 1020
gcgtacggct ggaccgtctc cgtcgacaag caccagtacg agcccctcaa gcacccggac 1080
atcggcgtct acctcgtgaa cctcaccgcg gggtcgatgc tggcgccgca cgtgaaccct 1140
cgggcgacgg agtacggcgt ggtgctgggc ggggaaggca cggtccaggt ggtgttcccg 1200
aacgggtccc tggcgatgag cgaggtggtg cgcccccggcg acgtgttctg gatcccgcgc 1260
tacttcccct tctgccaggt ggcggcgcgg gccgggccct tcgagttctt cggcttcacc 1320
acctcggcgc gccgcaaccg gccgcagttc ctggtcggcg cctcctcggt gctccgtacc 1380
atgctagggc cggagatcgc cgccgcgttc ggcgctcgcg agaaggagtt cagtaagctg 1440
gtgcgtgcgc aacgggaggc cctgattatg ccgtcctctc ctgggaagga ggaggaggag 1500
catgggaaga aagggaggga gaaagaggag tcactgccga tggttgtcga gcaggcggcg 1560
gcggagtga 1569

<210> 31
<211> 1644
<212> DNA
<213> Zea mays
<400> 31

atggcgatgg cctccgcggc ttgctcatgc acggacggca cgtggtgggt gtacgcgctc 60
ccggcgctgc tcggctccga caccctgtgc gcccacccgg ccctcctggc tggcctgatc 120

tttctggcca ccgtctcggt ggctctgctg gcgtgggcca cgtcgccggg cggtccggcg 180
tggacgaacg gccgcggcgc ctcggcgtca ctcctatcgt gggaccccgt ggtctgcccg 240
tgttcggcag catcttcgcg ctgtcccggg gctgccgcac cgcgccctcg ccgagatggc 300
ccgcgccgca ggccccgggc caaggagctc atggcgttct ccgtcggtga cacgcccgcg 360
gtcgtgtcgt cctgcccggc cacggcacgt gaggtgctcg cgcacccgtc attcgccgac 420
cgccctgtga agcggtcggc ccgggagctc atgttcgcgc gtgccatcgg gttcgcgccc 480
aacggcgagt actggcgccg cctccgccgc gtcgcgtcca cgcacctatt ctccccgcgc 540
cgggtcgcct cgcacgagcc gggacgccaa ggtgacgcgg aggccatgct ccgctccatc 600
gccgccgaac agtcggcctc tggcgccgtc gccctccgcc cgcacctcca ggccgccgct 660
ctcaacaaca tcatgggcag cgtcttcggc acgcggtacg acgtcacatc aggcgccggc 720
gccgcggagg ccgagcatct caagagcatg gtgcgcgagg ggttcgagct cctcggcgcc 780
ttcaactggt ccgaccacct cccctggctc gcccacctgt acgacccaag caacgtcacc 840
cgccggtgcg ccgcgctcgt gccgcgcgtc cagaccttcg tccgtggcgt catcgacgag 900
caccggcgcc gccgccaaaa ctccgccgcc ctcaacgaca atgctgactt cgtcgacgtg 960
ctcctctccc tcgagggtga cgagaagctc ggcgacgacg acatggtcgc catcctctgg 1020
gagatggtct tccgcggtac ggacacgacg gcgcttctga ccgagtggtg catggcggag 1080
ctggtgcgcc acccggcggt gcaggcgagg gtgcgcgccg aggtcgacgc ggctgtcggt 1140
gccggaggtt gccccaccga cgccgacgtg gcgcgcatgc cgtacctgca ggcggttgtg 1200
aaggagacgc tgcgcgccca cccgcctggc ccgctgctga gctgggctcg cctcgccacc 1260
gccgacgtgc cactctgcaa cggcatggtg gtcccggctg gcaccacggc gatggtgaat 1320
atgtgggcca taacccacga tgccgccgtg tgggccgacc cggacgcgtt cgcgccggag 1380
cggttcctgc cctccgaggg cggcgccgac gtggacgtcc gcggcgtcga cctccgcctg 1440
gccccgttcg gcgccgggcg tcgcgtctgc cccggcaaga acctgggcct caccaccgtg 1500
ggcctctggg ttgcccgcct cgtgcacgcc ttccagtggg ccctgcctga cggcgcggcg 1560
gccgtttgcc tcgacgaggt cctcaagctc tccctggaga tgaagacgcc gctcgtcgcc 1620
gcagccatcc cccgcaccgc ctga 1644

<210> 32

<211> 1488

<212> DNA

<213> Zea mays

<400> 32

atgtcgtaca acaaggcccc gtccatcacc gccgagacca taaatcccag ggtcaagatc 60
atcagatacg cgccttgcgg ggagatcgtc aagcacgcag cgcggctgga gcaggagata 120
gaggagaacc ctgcttctat ccctttccaa gagataatat actgcaacct cgggaacccc 180
caggtccttg gtcagccgcc actaactttc tttcgtgagg ttctctccct gtgcgacaac 240
ccagccctca tggacagaga tgaagctcgt gccttattca gcccttgtag cataagaagg 300
gccaggagga tcctaaactc gatccccagc aaagacactg gtggatatac tgattgccgg 360
ggaatcaaat gcctgcgtca agtagtagca gatgggatta ccgcaagaga cggttttcca 420
tcgacagcgg acgatatctt tctgacagat ggagccacct cagcgatcaa cttgatgatg 480
cagatactca tcaggtctca cgaagacggc atcttgagcc ctctacccga atacccccttg 540
tactcggcct ccatcatact gcacggtgga acgatggtgc cgtacaatct cactgaagat 600

aacggttggg gcctagagat ctttgaagtc aagaggtgct tggaagaggc tcgttcggct 660
ggcctcactg ttagagctat ggtggtaata aatcccggca accctactgg acaggtgcta 720
tccgtcacaa accaggagga gatagtagag ttttgtcgga aggaaggtct ggttatactt 780
gctgatgagg tataccagga aaatatctac gcagagaaca agaagttcca ttccttcaag 840
aaagtggcca gatcacttgc ttacgacgaa aatgacctca ccctagtatc atttctttcc 900
gtttcaatga gctatgggga gagtggcaga agaggaggct acatggaggt gtctggtgtt 960
gcagctaatg tgaaggatca gatttacaag gtggcttctt taacactctg ccccaacatt 1020
gctggccaga ttctcgtcag ccttgcaatg gatccaccga agctaggaga tgaatctttc 1080
gagtcatttg ataaggattt tgttcagatc cgttcatcct tctgcaagcg agccaagacc 1140
ctggagaaag ctttcagcgg cctggagggc gtgacttgca acaagttaga gggtgcgctg 1200
tacttgttcc cacggctcca cctcccctcc gctgcgatca gggccgccga tttcgaggga 1260
gtttcacctg acatattcta cgctcaccgc ctgcttgatg ccaccgggat tgccgttgtg 1320
ccgggctctg ggttccacca ggcctctggg acgatccata tccgatgcac gatccttcct 1380
gacgaaagaa aaatcgaggc gatggtcgca cgcttgaggg cgttccataa ggcgttcatg 1440
aacgagttcc ggggctctca gcaggtcatg aacgatctcc gtcgctga 1488

<210> 33

<211> 561

<212> DNA

<213> Zea mays

<400> 33

atggcggacc gtgaccgcag cggcatctac ggcggcgccc acgccaccta cgggcagcag 60
cagcagcagg gaggaggcgg gcgcccgatg ggtgagcagg tgaagggcat gctccacgac 120
aaggggccga cggcgtcgca ggcgctgacg gtggcgacgc tgttcccgct gggcgggctg 180
ctgctggtgc tgtcggggct ggcgctgacg gcctccgtgg tggggctggc cgtggccacg 240
ccggtgttcc tgatcttcag ccccgtgctg gtccccgccg cgctgctcat cgggacggcc 300
gtcatggggt tcctcacgtc gggcgcgctg gggctcgggg gcctgtcctc gctcacgtgc 360
ctcgccaaca cggcgcggca ggcgttccag cgcaccccgg actacgtgga ggaggcgcac 420
cgcaggatgg cggaggccgc ggcgcacgcg ggccacaaga ccgcgcaggc aggccaggcc 480
atccagggca gggcgcagga ggccggcgcc gggggaggtg caggtgccgg cgctggcggc 540
ggcggcaggg cttcctcgta a 561

<210> 34

<211> 531

<212> DNA

<213> Zea mays

<400> 34

atggcagatc gcgaccgcag cggcatctac ggcggcggcg cctacgggca gcagcagggg 60
ggcggccgcc cgatgggtga gcaggtgaag ggcatgatcc acgacaaggg gccgacggcg 120
tcccaggcgc tgacggtggc gacgctgttc cctctgggcg ggctgctcct ggtgctgtcg 180
ggcctggcgc tggcggcctc cacggtgggg ctggccctgg ccacgccccgt gttcctgctc 240
ttcagccccg tgctcgtccc cgccgcgctg ctcatcggca cggccgtcgc ggggttcctc 300
acgtcgggcg cgctggggct cggcggcctg tcctcgctca cgtgcctcgc caacacggcg 360

cggcaggcgt tccagcgcac cccggactac gtcgaggagg cgcgccgcag gatggcggag 420
gccgcggcgc acgcgggcca caagaccgcg caggccgggc acggcatcca gagcaaggcg 480
caggaggccg gtgctggcac tggcgccggc ggcggcagga cttcctcgta a 531

<210> 35
<211> 474
<212> DNA
<213> Zea mays
<400> 35

atgggagacc ggcatcagca tgggcctggc agcggcgtgg aggacccggc gacgctgctg 60
cggcgggtgc agacccgcgc gcccaactcg acgcaggtgg tggggttcct gacgctgctc 120
gtctctggcg ccgtgctgct gctgctgacg gggtgacgc tgacgggcgc cgtggtggcg 180
ctcgtcttcc tgggccccat cgcgctgctc accagcccca tctgggtgcc cgtcgccgtc 240
gtgatctccg ccatcgtcgc cgccgcgctc tccgcctgcg ccttcgccgc cgccgcgctc 300
cccgtggcca cctggatgta ccgctacttc acgggccgcc accccgtggg cgccgaccgg 360
gtggactacg cgcgcagccg gatcgccgac accgccagcc acgtcaagga ctacgcgcgc 420
gagtacggcg gatacctgcg cacccgcgcc aaggacgccg cgcccggcgc gtag 474

<210> 36
<211> 480
<212> DNA
<213> Zea mays
<400> 36

atggacagga agtgcctcgt ggacctcggc cacccgctgc tcaaccgcgt cgccgacagc 60
ttcatccgcg ccgctggggt cggggcggcc agggccgtct ccagggaggc ctacgtcgtc 120
accgtcgaag ggctttcagg agactcgtcc gggctagacg ccgacggcgg caagcggagc 180
catttctcca gcatcagagg tgacgacggc cagaggtcgc tcgacgcagt ggtaaaaacg 240
gccggcaagg aggccttcca gtgggggttg gcagccggag tctactcagg gctcacgtac 300
gcgctgcggg aggccagggg atgccacgac tggaagaaca gcgccatcgc aggcgccatc 360
gctggggcgg cggtggcgct cacgggtgac gccggcggcc actccgacaa gctcgtcaac 420
ttcgccatca ccggcgccgc gctctccagc gccgggagct tgctctccgg catattctga 480

<210> 37
<211> 151
<212> PRT
<213> Zea mays
<400> 37

Met Val Thr Val Glu Gly Arg Arg Gly Ser Ser Asp Gln Thr Gly Asp
1 5 10 15
Met Arg Tyr Val Ser Asp Phe Pro Val Tyr Asp Gly Arg Gly Ser Asp
20 25 30
Ala Val Leu Val Ala Arg Val Gln Gly Val Thr Thr Thr Phe Gly Asn
35 40 45
Ser Asn Gln Phe Phe Thr Val Ala Phe Glu Ala Gly Ser Arg Leu Lys

50 55 60

Gly Ser Thr Leu Leu Thr Glu Gly Val Val Thr Asp Gly Ser Asp Glu

65 70 75 80

Trp Ala Ile Tyr Gly Gly Thr Gly Glu Phe Ala Met Ala Arg Gly Val

85 90 95

Val Lys Arg Arg Tyr Leu Ala Asp Arg Asp Gly Ala Gly Asn Thr Asp

100 105 110

Glu Leu Ser Met Gln Val Phe Cys Pro Val Phe Gly Ser Ser Gln Gln

115 120 125

Pro Asn Lys Gln Asp Ser Ser Ile Ser Val Thr Lys Ile Gly Leu Trp

130 135 140

Gly Ala Asp Phe Asp Leu Ile

145 150

<210> 38

<211> 163

<212> PRT

<213> Zea mays

<400> 38

Met Ala Arg Phe Val Asp Pro Leu Val Val Gly Arg Val Ile Gly Glu

1 5 10 15

Val Val Asp Leu Phe Val Pro Gly Ser Lys Asp Ile Ser Asn Gly Cys

20 25 30

Leu Leu Lys Pro Ser Ala Thr Ala Ala Pro Pro Leu Val Arg Ile Ser

35 40 45

Gly Arg Arg Asn Asp Leu Tyr Thr Leu Ile Met Thr Asp Pro Asp Ala

50 55 60

Pro Ser Pro Ser Asn Pro Thr Met Arg Glu Tyr Leu His Trp Ile Val

65 70 75 80

Ile Asn Ile Pro Gly Gly Thr Asp Ala Thr Lys Gly Glu Glu Val Val

85 90 95

Glu Tyr Met Gly Pro Arg Pro Pro Val Gly Ile His Arg Tyr Val Leu

100 105 110

Val Leu Phe Glu Gln Lys Thr Arg Val His Ala Glu Ala Pro Gly Asp

115 120 125

Arg Ala Asn Phe Lys Thr Arg Ala Phe Ala Ala Ala His Glu Leu Gly

130 135 140

Leu Pro Thr Ala Val Val Tyr Phe Asn Ala Gln Lys Glu Pro Ala Ser

145 150 155 160

Arg Arg Arg

<210> 39

<211> 425
<212> PRT
<213> Zea mays
<400> 39

Met Ala Gln Leu Arg Ala Ala Ala Asp Glu Gln Gln Glu Ala Ala Met
1 5 10 15
His Gly Val Ala Ser Gly Asp Gly Asn Glu Ser Asn Lys Lys Ala Arg
20 25 30
Ala Gly Leu Cys Gly Leu Leu Arg Glu Arg Lys Val Val Asp Leu Ala
35 40 45
Arg Ala Lys Arg Arg Leu Val Glu Val Pro Tyr Thr Ala Thr Leu Ala
50 55 60
His Thr Ala Asn Ala Leu Leu Ala Ala Arg Val Ser Ala Val Ala Val
65 70 75 80
Ala Ala Pro Pro Gly His Trp Ile Gly Ala Gly Gly Ser Met Ile Leu
85 90 95
Glu Ser Asp Pro Ala Thr Gly Ala Val Arg Lys His Tyr Ile Gly Met
100 105 110
Val Asn Met Leu Asp Ile Leu Ala His Ile Ala Glu Ala Ser Asp Asp
115 120 125
Asp Glu Ala Ala Asp Leu Asp Arg Arg Met Ala Val Pro Val Ser Ser
130 135 140
Val Ile Gly His Ser Leu Glu Gly Leu Thr Leu Trp Thr Leu His Pro
145 150 155 160
Ser Thr Ser Val Val Asp Cys Met Glu Thr Phe Ser Lys Gly Val His
165 170 175
Arg Ala Leu Val Pro Leu Glu Ser Ser Ala Asp Asn Val Val Ala Leu
180 185 190
Glu Leu Val Glu Ser Ala Pro Gly Tyr Arg Met Leu Thr Gln Met Asp
195 200 205
Val Val Arg Phe Leu Arg Ala His Gly Ala Glu Leu Arg Gly Val Leu
210 215 220
Leu Arg Thr Val Arg Glu Leu Gly Ala Val Asn Asp Thr Val Phe Ala
225 230 235 240
Val Ser Gly Val Thr Lys Val Ile Asp Val Ile Lys Ala Met Arg Ala
245 250 255
Ala Ser Leu Thr Ala Val Pro Val Val Asp Ala Val Gly Ser Gly Thr
260 265 270
Glu Thr Leu Gln Tyr Gly Ser Gly Gln Arg Ala Val Glu Thr Phe Ser
275 280 285
Ala Thr Asp Leu Arg Asp Cys Pro Val Ala Arg Leu Gln Ala Trp Leu

290 295 300
Gly Ile Ser Val Met Glu Phe Lys Arg Lys Val Ala Glu Tyr Arg Ala
305 310 315 320
Ser Thr Arg Leu Val Val Pro Gly Ala Asp Ala Thr Asp Thr Gly Thr
325 330 335
Pro Val Ala Gly Tyr Ala Asp Thr Pro Ala Ala Ala Ala Ala Thr Asp
340 345 350
Glu Glu Gln Ser Glu Gln Gln Glu Ser Ala Leu Val Thr Cys Ser Gln
355 360 365
Glu Ser Thr Leu Gly Glu Ala Ile Glu Ala Ala Thr Ser Arg His Val
370 375 380
His Arg Leu Trp Val Val Asp Glu Glu Gly Leu Leu Arg Gly Val Val
385 390 395 400
Ser Leu Thr Asp Ile Leu Arg Ala Val Arg Glu Ala Ala Leu Gly Glu
405 410 415
Asp Leu Glu Leu His Ser Ile Val Pro
420 425

<210> 40
<211> 324
<212> PRT
<213> Zea mays
<400> 40

Met Ala Glu Asn Pro Ala Pro Ser Ser Pro Leu Val Thr Ser Pro Asp
1 5 10 15
Ala Ala Ala Pro Pro Leu Pro Ala Glu Phe Thr Lys Leu Thr Phe Arg
20 25 30
Ser Leu Tyr Ile Arg Arg Thr Gly Pro Gly Ser Arg Glu Met Thr Val
35 40 45
Asp Gly Arg Pro Ser Asp Gln Leu Gly Arg Arg Phe Val Ser Asp Phe
50 55 60
Pro Ile Tyr Asp Gly Arg Gly Ser Gly Ala Arg Leu Val Ala Arg Leu
65 70 75 80
Gln Gly Val Thr Val Gln Ile Gly Ser Ser His Gln Leu Val Ser Ile
85 90 95
Val Phe Glu Ala Glu Arg Leu Lys Gly Ser Thr Leu Leu Thr Asn Gly
100 105 110
Val Ile Thr Asp Gly Ser Asp Glu Trp Ala Ile Tyr Gly Gly Thr Gly
115 120 125
Val Phe Ala Met Ala Thr Gly Val Ile Arg Arg Arg Phe Leu Ala Gly
130 135 140
Ser Asn Asp Gly Asn Ser Asp Glu Leu Thr Ser Ile Glu Val Phe Cys

145 150 155 160

Pro Ala Phe Gly Ser Ala Gln Gln Gln Pro Asn Lys Gln Asp Ser Val

165 170 175

Ser Val Thr Lys Ile Gly Val Trp Gly Gly Gly Gly Gly Ser Ala Gln

180 185 190

Asp Ile Thr Thr Thr Glu Pro Pro Gln Arg Leu His Ser Ile Thr Val

195 200 205

Arg Thr Gly Phe Ala Val Asp Ser Ile Glu Phe Thr Tyr Thr Asp Arg

210 215 220

Gly Gly Gln Arg Arg Thr Ala Gly Arg Trp Gly Gly Leu Gly Gly Asn

225 230 235 240

Leu Arg Thr Ile Asp Leu Gly Asp Ala Glu Val Val Arg Glu Val Ser

245 250 255

Gly Thr Tyr Gly Thr Phe Glu Gly Ala Thr Thr Leu Thr Ser Ile Arg

260 265 270

Ile Leu Thr Ser Ser Arg Thr Trp Gly Pro Trp Gly Ile Glu Asp Gly

275 280 285

Thr Arg Phe Cys Ile Thr Ala Pro Ile Gly Ser Ser Ile Val Gly Phe

290 295 300

Tyr Gly Arg Ser Thr Ser Arg Leu Val Ala Ala Ile Gly Val Tyr Leu

305 310 315 320

Arg Gln Gln Leu

<210> 41

<211> 190

<212> PRT

<213> Zea mays

<400> 41

Met Ala Pro Lys Arg Arg Gly Asn Lys Val Val Gly Ser Val Val Lys

1 5 10 15

Thr Lys Leu Val Gln Glu Thr Val Glu Val Ile Val Ala Asp Asp Asp

20 25 30

Gly Leu His Ala Glu Lys Gln Gln Val Pro Glu Ala Leu Ala Leu Ala

35 40 45

His Pro Thr Val Asp Val Ser Gly Ser Thr Val Val His Val Val Glu

50 55 60

Val Thr Ala Lys Arg Gly Arg Gly Gly Gly Gly Gly Glu Glu Ser Ser

65 70 75 80

Asn Thr Asn Lys Glu Asp Glu Asp Ala Ala Ala Ala Ala Ala Ala Ala

85 90 95

Ala Ala Gly Ala Gly Gln Leu Arg Arg Arg Arg Arg Asp Gly Arg Arg

100 105 110
Gly Arg Val Gln Ala Val Arg Val Ala Arg Ala Glu Ala Gly Ala Pro
115 120 125
Gly Pro Gly Arg Val Gly Ala Arg His Ala Gly Ala Gly His Asp Asp
130 135 140
Gly Arg His Val Arg Ala Pro Arg Gly Gly Gly Gly Ala Pro Val Gln
145 150 155 160
Gly His Gly Gln Gly Asp Ala His Leu Pro Arg Gly Ala Glu Arg Arg
165 170 175
Gln Ala Arg Ala Pro Arg Gly Ala Gly Gln Ala Arg His Leu
180 185 190

<210> 42
<211> 116
<212> PRT
<213> Zea mays
<400> 42

Met Pro Ser Thr Val Asn Ser Ala Ser Leu Asp Gln Asn Leu Glu Leu
1 5 10 15
Ala Lys Gln Cys Ser Arg Glu Ala Ala Leu Ala Gly Phe Lys Ala Ala
20 25 30
Ala Val Ala Thr Val Ala Ser Ala Val Pro Thr Leu Val Ser Val Arg
35 40 45
Met Leu Pro Trp Ala Lys Ala Asn Ile Asn Pro Ala Gly Gln Ala Leu
50 55 60
Ile Val Ser Thr Val Ala Gly Met Ala Tyr Phe Ile Ala Ala Asp Lys
65 70 75 80
Lys Ile Leu Ser Leu Ala Arg Gln His Ser Tyr Glu Asn Ala Pro Glu
85 90 95
His Leu Lys Asp Thr Ser Phe Gln Gly Thr Gly Arg Pro His Pro Gly
100 105 110
Phe Phe Arg Pro
115

<210> 43
<211> 129
<212> PRT
<213> Zea mays
<400> 43

Met Ala His Ser Lys Arg Glu Ile Lys Pro Gly Arg Ala His Thr Lys
1 5 10 15
Val Ala Gly Asp Asp Glu Met Leu Arg Thr Gly Phe Phe Asp Gly Thr
20 25 30

Pro Leu Glu Gly Gly Lys Ile Ala Asp Ser Gln Pro Val Asp Leu Phe
35 40 45
Ala Ala Ala Arg Arg Val Ala Asp Ala Pro Tyr Ser Ser Gln Gln Glu
50 55 60
Gly Pro His Glu Glu Gly Thr Asn Lys Asn Ala Val Ile Ala Glu Ser
65 70 75 80
Glu Pro Val Asp Leu Pro Ala Ser Ala Arg Gly Val Ala Glu Val Asn
85 90 95
Arg Ala Gly Pro Glu Glu Gln Thr Gly Arg Gly Arg Gln Gly Met Thr
100 105 110
Glu Pro Thr Ala Gly Gly Arg Arg Leu Gly Leu Gly Arg Pro Ala Pro
115 120 125
Ala

<210> 44
<211> 578
<212> PRT
<213> Zea mays
<400> 44
Met Ala Glu Gln Phe Tyr Thr Val Ala Ser Asp Ser Glu Thr Thr Gly
1 5 10 15
Glu Asp Lys Ser Gln Ala Ser Phe Pro Asp Val Ala Val Gly Ile Asp
20 25 30
Ile Gly Thr Ser Lys Cys Ser Val Ala Ile Trp Asn Gly His Gln Val
35 40 45
Glu Leu Leu Lys Asn Thr Arg Asn Gln Lys Gly Met Arg Ser Tyr Val
50 55 60
Met Phe Lys Asp Asp Thr Leu Ser Ala Gly Val Thr Gly Gly Ala Ala
65 70 75 80
Lys Glu Asn Ala His Glu Glu Arg Asp Ile Leu Ser Gly Ser Ala Ile
85 90 95
Phe Asn Met Lys Arg Leu Ile Gly Arg Met Asp Thr Asp Glu Val Val
100 105 110
Gln Ala Ser Lys Thr Leu Pro Phe Leu Val Gln Thr Leu Gly Ile Gly
115 120 125
Val Arg Pro Phe Ile Ala Ala Leu Val Asn Asn Met Trp Arg Ser Thr
130 135 140
Thr Pro Glu Glu Val Leu Ala Ile Phe Leu Leu Glu Leu Lys Ala Leu
145 150 155 160
Val Glu Met His Leu Lys His Pro Val Arg Asn Ala Val Leu Thr Ile
165 170 175

Pro Val Ala Phe Ser Arg Phe Gln Gln Thr Arg Ile Glu Arg Ala Cys
180 185 190

Ala Met Ala Gly Leu His Val Leu Arg Leu Met Pro Glu Pro Thr Ala
195 200 205

Val Ala Leu Leu Tyr Ala Gln Gln Gln Gln Gln Leu Val His Asp Asn
210 215 220

Met Gly Ser Gly Ile Glu Lys Ile Ala Leu Ile Phe Asn Met Gly Ala
225 230 235 240

Gly Tyr Cys Asp Val Ala Val Ala Ala Thr Ala Gly Gly Val Ser Gln
245 250 255

Ile Arg Ala Leu Ser Gly Cys Thr Ala Gly Gly Glu Asp Ile Leu Gln
260 265 270

Asn Ile Met Arg His Val Leu Pro Asn Phe Asp Gly Ile Tyr Ala Gly
275 280 285

Gln Thr Met Asp Arg Ile Lys Ser Met Ser Leu Leu Arg Ile Ala Thr
290 295 300

Gln Asp Ala Ile His Arg Leu Val Ser Gln Glu Ser Val Glu Ile Asn
305 310 315 320

Ile Asp Leu Gly Asn Gly His Met Val Ser Lys Val Leu Asp His Ser
325 330 335

Glu Phe Glu Gln Val Asn Arg Ala Ile Phe Asp Lys Cys Glu Lys Ile
340 345 350

Ile Asn Gln Cys Leu Ala Asp Ala Lys Leu Val Pro Glu Asp Ile Asn
355 360 365

Asp Val Ile Leu Val Gly Gly Cys Ser Lys Ile Pro Arg Ile Arg Ser
370 375 380

Leu Val Leu Gly Leu Cys Lys Lys Glu Val Ser Tyr Lys Asn Ile Asp
385 390 395 400

Ala Leu Glu Ala Ala Val Ser Gly Ala Ala Leu Glu Gly Ala Ile Ala
405 410 415

Ser Gly Val Asn Asp Pro Ser Gly Ser Leu Asp Leu Leu Thr Ile Gln
420 425 430

Ala Thr Pro Met Asn Leu Gly Ile Arg Ala Asp Gly Asp Ser Phe Ala
435 440 445

Ala Ile Ile Pro Arg Asn Thr Thr Val Pro Ala Arg Arg Asp Met Leu
450 455 460

Phe Thr Thr Thr His Asp Lys Gln Thr Glu Ala Leu Ile Ala Val Tyr
465 470 475 480

Glu Gly Glu Gly Glu Arg Ala Glu Asp Asn His Leu Leu Gly Tyr Phe
485 490 495

Lys Ile Thr Gly Ile Pro Ala Ala Pro Lys Gly Ala Val Asp Ile Ser

500 505 510

Val Cys Met Asp Ile Asp Ala Ala Asn Val Leu Arg Val Phe Ala Gly

515 520 525

Val Val Lys Pro Gln Gly Pro Ala Ile Pro Pro Phe Ile Glu Val Arg

530 535 540

Met Pro Thr Leu Asp Asp Gly His Gly Trp Cys Gly Gln Ala Leu Ala

545 550 555 560

Lys Met Tyr Gly Arg Ile Leu Asp Leu Ala Val Leu Pro Lys Lys Leu

565 570 575

Gln Pro

<210> 45

<211> 140

<212> PRT

<213> Zea mays

<400> 45

Met Gly Lys Ser Trp Ser Leu Ile Ser His Leu His Thr Val Ala Gly

1 5 10 15

Pro Ser Ile Thr Leu Leu Tyr Pro Leu Tyr Ala Ser Val Cys Ala Met

20 25 30

Glu Ser Pro Ser Lys Ala Asp Asp Glu Gln Trp Leu Ser Tyr Trp Ile

35 40 45

Ile His Ser Phe Val Thr Leu Leu Glu Met Leu Ala Glu Pro Leu Leu

50 55 60

His Trp Val Pro Val Trp Tyr Pro Ala Lys Val Leu Phe Ala Ala Trp

65 70 75 80

Leu Ala Leu Pro Gln Phe Lys Gly Ala Ser Phe Val Tyr Glu Lys Leu

85 90 95

Val Arg Glu Gln Leu Arg Asn Tyr Arg Ala Arg Tyr Pro Arg Lys Gly

100 105 110

Ala Ala Ala Ala Ala Val Gly Gly Asp Asp Asp Asp His Lys Val His

115 120 125

Ile Ala Lys Ala Glu Ala Glu His Asp His Val Gln

130 135 140

<210> 46

<211> 439

<212> PRT

<213> Zea mays

<400> 46

Met Gly Ile Ser Ala Arg Trp Leu Lys Ser Leu Val Gly Leu Arg Lys

1 5 10 15

Val Gly Arg Gln Gln Gln Gln Arg Arg Lys Asp Asp Ala Asp Val Gly
20 25 30
Arg Met Lys Thr Asp Val Ala Asp Gln Phe His Phe Gln Ile Gln His
35 40 45
Ser Gln Asp Asp Asn Ser Ile Ala Ala Gln Glu Ile Pro Glu Val Ser
50 55 60
Tyr Gly Asn Asp Pro Pro Glu Asp Asp Ser Asn Val Pro Ser Cys Phe
65 70 75 80
Glu Pro Ala Arg Ser Ser Ala His Met Pro Phe Cys Gln Thr Glu Glu
85 90 95
Ala Gln Lys Glu Ile Trp Ala Ala Thr Ile Ile Gln Thr Ala Phe Arg
100 105 110
Ala Phe Leu Ala Arg Arg Ala Arg Arg Ala Leu Lys Gly Leu Val Arg
115 120 125
Leu Gln Ala Leu Val Arg Gly His Ile Val Arg Lys Arg Ala Ala Thr
130 135 140
Thr Leu Arg Cys Met Gln Ala Leu Val Arg Val Gln Ala Arg Val Arg
145 150 155 160
Ala Arg Arg Val Arg Met Ala Leu Glu Asn Gln Thr Asp Arg Gln Asn
165 170 175
Thr Ser Pro Glu His Thr Ile Glu Ala Arg Val Arg Glu Ile Glu Asp
180 185 190
Gly Trp Cys Asp Ser Ile Gly Ser Val Gly Asp Ile Gln Ala Lys Leu
195 200 205
Leu Lys Arg Gln Glu Ala Ala Ala Lys Arg Glu Arg Ala Met Ala Tyr
210 215 220
Ala Leu Ala His Gln Trp Gln Ala Ser Ser Arg Gln Pro Thr Ala Phe
225 230 235 240
Glu Pro Asp Lys Asn Ser Trp Gly Trp Asn Trp Leu Glu Arg Trp Met
245 250 255
Ala Val Arg Pro Trp Glu Ser Arg Phe Leu Gly Thr Tyr Gly Thr Asp
260 265 270
Gly Ile Phe Val Val Asn Glu Thr Arg Gln Pro Asp Arg Ser Ala Thr
275 280 285
Lys Thr Pro Tyr Arg Lys Pro Val Lys Lys His Asp Ser Thr Leu Gln
290 295 300
Ser Asn Met Leu Asn Gln Lys Val Phe Pro Ser Asn Ser Glu Gly Gly
305 310 315 320
Gly Ser Ser Thr Asn Arg Ser Ser Gly Leu Val Ser Thr Lys Ser Arg
325 330 335
Leu Lys Val Leu Pro Arg Glu Gly Ser Glu Glu Ala Ser Ser His Pro

340 345 350

Ser Val Ile Val Arg Ser Thr Ser Asn Pro Lys Glu Arg Ile Ser Ser

355 360 365

Ile Asn Ser Lys Glu Arg Thr Gly Asp Leu Asp Phe Gln Val His Lys

370 375 380

Arg Ser Ser Leu Pro Ser Ile Gly Pro Glu Ala Gly Lys His Leu Thr

385 390 395 400

Lys Lys Gly Met Val Asn Arg Ser Leu Lys Ala Thr Lys Asp Ser Gln

405 410 415

Ile Leu Gly Ser Arg His His Leu Ala Ser Ser Ile Asp Pro Val Pro

420 425 430

Thr Arg Val Glu Leu Glu Thr

435

<210> 47

<211> 165

<212> PRT

<213> Zea mays

<400> 47

Met His Gly His Gln Gln Gln Pro Tyr Pro Tyr Gly Leu Arg Gln Asp

1 5 10 15

Pro Leu Arg Arg Met Arg Gly Leu Asp Val Lys Ser Ala Leu Gln Ala

20 25 30

Ser Pro Arg Thr Ser Thr Val Ala Thr Ala Ala Leu Val Val Pro Leu

35 40 45

Gly Ala Ala Leu Leu Cys Ala Ser Gly Ile Ala Leu Ala Ala Thr Val

50 55 60

Thr Gly Leu Ala Leu Ala Thr Pro Pro Leu Val Ile Phe Ser Pro Val

65 70 75 80

Leu Ile Pro Phe Ala Leu Ala Ala Gly Leu Ile Ala Ser Gly Leu Leu

85 90 95

Ala Ser Gly Ala Leu Gly Val Ala Gly Val Ser Ala Leu Thr Trp Ala

100 105 110

Val Gly Tyr Val Trp Gln Arg Gln Gly Glu Gly Gly Gly Gly Val Thr

115 120 125

Gly Met Val Val Gln Pro Leu Asp Gln Gly Ser Lys Arg His Gly Val

130 135 140

Gln Gly Ala Ala Ala Phe Val Gly His Arg Arg Leu Pro Arg Asp Ile

145 150 155 160

Asp Val Ala Gly Glu

165

<210> 48

<211> 522
<212> PRT
<213> Zea mays
<400> 48
Met Asp Arg Leu Thr Val Met Ala Pro Pro Leu Leu Val Leu Leu Leu
1 5 10 15
Leu Leu Leu Ser Arg Cys Ser Ala Ala Ala Ser Arg Arg Gly Gly Gly
20 25 30
Trp Trp Glu Glu Gly Glu Gly Glu Trp Arg Pro Ser Glu Glu Glu Glu
35 40 45
Lys Gly Lys Gly Lys Gly Arg Gly Leu Phe Leu Leu His Arg Val Glu
50 55 60
Lys Val Val Glu Ser Glu Gly Gly Gln Val Arg Val Val Arg Gly Gln
65 70 75 80
Pro Trp Pro Pro Ala Ser Phe Ala Cys Arg Glu Gly Leu Met His Ile
85 90 95
Gly Phe Ile Thr Met Glu Pro Lys Thr Leu Phe Val Pro Gln Tyr Leu
100 105 110
Asp Ser Ser Ile Thr Leu Phe Val Gln Arg Gly Glu Ala Lys Val Gly
115 120 125
Tyr Ile His Lys Asp Glu Leu Val Glu Arg Lys Leu Lys Met Gly Asp
130 135 140
Val Leu His Ile Asp Ala Gly Ser Thr Phe Tyr Met Val Asn Pro Gly
145 150 155 160
Lys Gly Gln Arg Leu Gln Ile Ile Cys Ser Val Asp Ala Ser Asp Ser
165 170 175
Leu Gly Phe Gly Pro Pro Tyr Gln Ala Phe Phe Leu Gly Gly Ala Gly
180 185 190
Asp Pro Ala Ser Val Ile Ala Gly Phe Gly Pro Lys Thr Leu Thr Arg
195 200 205
Ala Phe Asn Ala Thr Tyr Asp Glu Leu Ala Arg Ile Leu Leu Pro Arg
210 215 220
Thr Gly Gly Pro Ile Val Tyr Tyr Thr Ala Asp Ala Glu Pro Glu Ser
225 230 235 240
Gly Ala Ala Glu Glu Glu Arg Gly Gln Val Asp Gly His Asp Gly Val
245 250 255
Leu Asp Arg Gly Ala Arg Arg Glu Gly Ala Gly Ala Trp Val Pro Gly
260 265 270
Gly Arg Gly Asp Gly Gly Asp Glu Cys Gly Gly Ser Asp Asp Ala Arg
275 280 285
Glu Ala Thr Trp Trp Trp Thr Lys Leu Val Asn Arg Val Val Gly Gly

290 295 300

Ala Ala Gly Gly Gly Gly Ala Ala Glu Ala Asn Arg Lys Gly Lys Lys

305 310 315 320

Lys Lys Gly Gly Ala Pro Glu Pro Tyr Asn Leu Tyr Asp Ser Glu Pro

325 330 335

Gly Phe Arg Asn Ala Tyr Gly Trp Thr Val Ser Val Asp Lys His Gln

340 345 350

Tyr Glu Pro Leu Lys His Pro Asp Ile Gly Val Tyr Leu Val Asn Leu

355 360 365

Thr Ala Gly Ser Met Leu Ala Pro His Val Asn Pro Arg Ala Thr Glu

370 375 380

Tyr Gly Val Val Leu Gly Gly Glu Gly Thr Val Gln Val Val Phe Pro

385 390 395 400

Asn Gly Ser Leu Ala Met Ser Glu Val Val Arg Pro Gly Asp Val Phe

405 410 415

Trp Ile Pro Arg Tyr Phe Pro Phe Cys Gln Val Ala Ala Arg Ala Gly

420 425 430

Pro Phe Glu Phe Phe Gly Phe Thr Thr Ser Ala Arg Arg Asn Arg Pro

435 440 445

Gln Phe Leu Val Gly Ala Ser Ser Val Leu Arg Thr Met Leu Gly Pro

450 455 460

Glu Ile Ala Ala Ala Phe Gly Ala Arg Glu Lys Glu Phe Ser Lys Leu

465 470 475 480

Val Arg Ala Gln Arg Glu Ala Leu Ile Met Pro Ser Ser Pro Gly Lys

485 490 495

Glu Glu Glu Glu His Gly Lys Lys Gly Arg Glu Lys Glu Glu Ser Leu

500 505 510

Pro Met Val Val Glu Gln Ala Ala Ala Glu

515 520

<210> 49

<211> 547

<212> PRT

<213> Zea mays

<400> 49

Met Ala Met Ala Ser Ala Ala Cys Ser Cys Thr Asp Gly Thr Trp Trp

1 5 10 15

Val Tyr Ala Leu Pro Ala Leu Leu Gly Ser Asp Thr Leu Cys Ala His

20 25 30

Pro Ala Leu Leu Ala Gly Leu Ile Phe Leu Ala Thr Val Ser Val Ala

35 40 45

Leu Leu Ala Trp Ala Thr Ser Pro Gly Gly Pro Ala Trp Thr Asn Gly

50 55 60

Arg Gly Ala Ser Ala Ser Leu Leu Ser Trp Asp Pro Val Val Cys Pro

65 70 75 80

Cys Ser Ala Ala Ser Ser Arg Cys Pro Gly Ala Ala Ala Pro Arg Pro

85 90 95

Arg Arg Asp Gly Pro Arg Arg Arg Pro Arg Ala Lys Glu Leu Met Ala

100 105 110

Phe Ser Val Gly Asp Thr Pro Ala Val Val Ser Ser Cys Pro Ala Thr

115 120 125

Ala Arg Glu Val Leu Ala His Pro Ser Phe Ala Asp Arg Pro Val Lys

130 135 140

Arg Ser Ala Arg Glu Leu Met Phe Ala Arg Ala Ile Gly Phe Ala Pro

145 150 155 160

Asn Gly Glu Tyr Trp Arg Arg Leu Arg Arg Val Ala Ser Thr His Leu

165 170 175

Phe Ser Pro Arg Arg Val Ala Ser His Glu Pro Gly Arg Gln Gly Asp

180 185 190

Ala Glu Ala Met Leu Arg Ser Ile Ala Ala Glu Gln Ser Ala Ser Gly

195 200 205

Ala Val Ala Leu Arg Pro His Leu Gln Ala Ala Ala Leu Asn Asn Ile

210 215 220

Met Gly Ser Val Phe Gly Thr Arg Tyr Asp Val Thr Ser Gly Ala Gly

225 230 235 240

Ala Ala Glu Ala Glu His Leu Lys Ser Met Val Arg Glu Gly Phe Glu

245 250 255

Leu Leu Gly Ala Phe Asn Trp Ser Asp His Leu Pro Trp Leu Ala His

260 265 270

Leu Tyr Asp Pro Ser Asn Val Thr Arg Arg Cys Ala Ala Leu Val Pro

275 280 285

Arg Val Gln Thr Phe Val Arg Gly Val Ile Asp Glu His Arg Arg Arg

290 295 300

Arg Gln Asn Ser Ala Ala Leu Asn Asp Asn Ala Asp Phe Val Asp Val

305 310 315 320

Leu Leu Ser Leu Glu Gly Asp Glu Lys Leu Gly Asp Asp Asp Met Val

325 330 335

Ala Ile Leu Trp Glu Met Val Phe Arg Gly Thr Asp Thr Thr Ala Leu

340 345 350

Leu Thr Glu Trp Cys Met Ala Glu Leu Val Arg His Pro Ala Val Gln

355 360 365

Ala Arg Val Arg Ala Glu Val Asp Ala Ala Val Gly Ala Gly Gly Cys

370 375 380

Pro Thr Asp Ala Asp Val Ala Arg Met Pro Tyr Leu Gln Ala Val Val
385 390 395 400
Lys Glu Thr Leu Arg Ala His Pro Pro Gly Pro Leu Leu Ser Trp Ala
405 410 415
Arg Leu Ala Thr Ala Asp Val Pro Leu Cys Asn Gly Met Val Val Pro
420 425 430
Ala Gly Thr Thr Ala Met Val Asn Met Trp Ala Ile Thr His Asp Ala
435 440 445
Ala Val Trp Ala Asp Pro Asp Ala Phe Ala Pro Glu Arg Phe Leu Pro
450 455 460
Ser Glu Gly Gly Ala Asp Val Asp Val Arg Gly Val Asp Leu Arg Leu
465 470 475 480
Ala Pro Phe Gly Ala Gly Arg Arg Val Cys Pro Gly Lys Asn Leu Gly
485 490 495
Leu Thr Thr Val Gly Leu Trp Val Ala Arg Leu Val His Ala Phe Gln
500 505 510
Trp Ala Leu Pro Asp Gly Ala Ala Ala Val Cys Leu Asp Glu Val Leu
515 520 525
Lys Leu Ser Leu Glu Met Lys Thr Pro Leu Val Ala Ala Ala Ile Pro
530 535 540
Arg Thr Ala
545

<210> 50
<211> 495
<212> PRT
<213> Zea mays

<400> 50

Met Ser Tyr Asn Lys Ala Pro Ser Ile Thr Ala Glu Thr Ile Asn Pro
1 5 10 15
Arg Val Lys Ile Ile Arg Tyr Ala Pro Cys Gly Glu Ile Val Lys His
20 25 30
Ala Ala Arg Leu Glu Gln Glu Ile Glu Glu Asn Pro Ala Ser Ile Pro
35 40 45
Phe Gln Glu Ile Ile Tyr Cys Asn Leu Gly Asn Pro Gln Val Leu Gly
50 55 60
Gln Pro Pro Leu Thr Phe Phe Arg Glu Val Leu Ser Leu Cys Asp Asn
65 70 75 80
Pro Ala Leu Met Asp Arg Asp Glu Ala Arg Ala Leu Phe Ser Pro Cys
85 90 95
Ser Ile Arg Arg Ala Arg Arg Ile Leu Asn Ser Ile Pro Ser Lys Asp
100 105 110

Thr Gly Gly Tyr Thr Asp Cys Arg Gly Ile Lys Cys Leu Arg Gln Val
115 120 125
Val Ala Asp Gly Ile Thr Ala Arg Asp Gly Phe Pro Ser Thr Ala Asp
130 135 140
Asp Ile Phe Leu Thr Asp Gly Ala Thr Ser Ala Ile Asn Leu Met Met
145 150 155 160
Gln Ile Leu Ile Arg Ser His Glu Asp Gly Ile Leu Ser Pro Leu Pro
165 170 175
Glu Tyr Pro Leu Tyr Ser Ala Ser Ile Ile Leu His Gly Gly Thr Met
180 185 190
Val Pro Tyr Asn Leu Thr Glu Asp Asn Gly Trp Gly Leu Glu Ile Phe
195 200 205
Glu Val Lys Arg Cys Leu Glu Glu Ala Arg Ser Ala Gly Leu Thr Val
210 215 220
Arg Ala Met Val Val Ile Asn Pro Gly Asn Pro Thr Gly Gln Val Leu
225 230 235 240
Ser Val Thr Asn Gln Glu Glu Ile Val Glu Phe Cys Arg Lys Glu Gly
245 250 255
Leu Val Ile Leu Ala Asp Glu Val Tyr Gln Glu Asn Ile Tyr Ala Glu
260 265 270
Asn Lys Lys Phe His Ser Phe Lys Lys Val Ala Arg Ser Leu Ala Tyr
275 280 285
Asp Glu Asn Asp Leu Thr Leu Val Ser Phe Leu Ser Val Ser Met Ser
290 295 300
Tyr Gly Glu Ser Gly Arg Arg Gly Gly Tyr Met Glu Val Ser Gly Val
305 310 315 320
Ala Ala Asn Val Lys Asp Gln Ile Tyr Lys Val Ala Ser Leu Thr Leu
325 330 335
Cys Pro Asn Ile Ala Gly Gln Ile Leu Val Ser Leu Ala Met Asp Pro
340 345 350
Pro Lys Leu Gly Asp Glu Ser Phe Glu Ser Phe Asp Lys Asp Phe Val
355 360 365
Gln Ile Arg Ser Ser Phe Cys Lys Arg Ala Lys Thr Leu Glu Lys Ala
370 375 380
Phe Ser Gly Leu Glu Gly Val Thr Cys Asn Lys Leu Glu Gly Ala Leu
385 390 395 400
Tyr Leu Phe Pro Arg Leu His Leu Pro Ser Ala Ala Ile Arg Ala Ala
405 410 415
Asp Phe Glu Gly Val Ser Pro Asp Ile Phe Tyr Ala His Arg Leu Leu
420 425 430
Asp Ala Thr Gly Ile Ala Val Val Pro Gly Ser Gly Phe His Gln Ala

435 440 445

Ser Gly Thr Ile His Ile Arg Cys Thr Ile Leu Pro Asp Glu Arg Lys

450 455 460

Ile Glu Ala Met Val Ala Arg Leu Arg Ala Phe His Lys Ala Phe Met

465 470 475 480

Asn Glu Phe Arg Gly Ser Gln Gln Val Met Asn Asp Leu Arg Arg

485 490 495

<210> 51

<211> 186

<212> PRT

<213> Zea mays

<400> 51

Met Ala Asp Arg Asp Arg Ser Gly Ile Tyr Gly Gly Ala His Ala Thr

1 5 10 15

Tyr Gly Gln Gln Gln Gln Gln Gly Gly Gly Gly Arg Pro Met Gly Glu

20 25 30

Gln Val Lys Gly Met Leu His Asp Lys Gly Pro Thr Ala Ser Gln Ala

35 40 45

Leu Thr Val Ala Thr Leu Phe Pro Leu Gly Gly Leu Leu Leu Val Leu

50 55 60

Ser Gly Leu Ala Leu Thr Ala Ser Val Val Gly Leu Ala Val Ala Thr

65 70 75 80

Pro Val Phe Leu Ile Phe Ser Pro Val Leu Val Pro Ala Ala Leu Leu

85 90 95

Ile Gly Thr Ala Val Met Gly Phe Leu Thr Ser Gly Ala Leu Gly Leu

100 105 110

Gly Gly Leu Ser Ser Leu Thr Cys Leu Ala Asn Thr Ala Arg Gln Ala

115 120 125

Phe Gln Arg Thr Pro Asp Tyr Val Glu Glu Ala His Arg Arg Met Ala

130 135 140

Glu Ala Ala Ala His Ala Gly His Lys Thr Ala Gln Ala Gly Gln Ala

145 150 155 160

Ile Gln Gly Arg Ala Gln Glu Ala Gly Ala Gly Gly Gly Ala Gly Ala

165 170 175

Gly Ala Gly Gly Gly Gly Arg Ala Ser Ser

180 185

<210> 52

<211> 176

<212> PRT

<213> Zea mays

<400> 52

Met Ala Asp Arg Asp Arg Ser Gly Ile Tyr Gly Gly Gly Ala Tyr Gly
1 5 10 15
Gln Gln Gln Gly Gly Gly Arg Pro Met Gly Glu Gln Val Lys Gly Met
20 25 30
Ile His Asp Lys Gly Pro Thr Ala Ser Gln Ala Leu Thr Val Ala Thr
35 40 45
Leu Phe Pro Leu Gly Gly Leu Leu Leu Val Leu Ser Gly Leu Ala Leu
50 55 60
Ala Ala Ser Thr Val Gly Leu Ala Leu Ala Thr Pro Val Phe Leu Leu
65 70 75 80
Phe Ser Pro Val Leu Val Pro Ala Ala Leu Leu Ile Gly Thr Ala Val
85 90 95
Ala Gly Phe Leu Thr Ser Gly Ala Leu Gly Leu Gly Gly Leu Ser Ser
100 105 110
Leu Thr Cys Leu Ala Asn Thr Ala Arg Gln Ala Phe Gln Arg Thr Pro
115 120 125
Asp Tyr Val Glu Glu Ala Arg Arg Arg Met Ala Glu Ala Ala Ala His
130 135 140
Ala Gly His Lys Thr Ala Gln Ala Gly His Gly Ile Gln Ser Lys Ala
145 150 155 160
Gln Glu Ala Gly Ala Gly Thr Gly Ala Gly Gly Gly Arg Thr Ser Ser
165 170 175

<210> 53
<211> 157
<212> PRT
<213> Zea mays
<400> 53

Met Gly Asp Arg His Gln His Gly Pro Gly Ser Gly Val Glu Asp Pro
1 5 10 15
Ala Thr Leu Leu Arg Arg Val Gln Thr Arg Ala Pro Asn Ser Thr Gln
20 25 30
Val Val Gly Phe Leu Thr Leu Leu Val Ser Gly Ala Val Leu Leu Leu
35 40 45
Leu Thr Gly Val Thr Leu Thr Gly Ala Val Val Ala Leu Val Phe Leu
50 55 60
Gly Pro Ile Ala Leu Leu Thr Ser Pro Ile Trp Val Pro Val Ala Val
65 70 75 80
Val Ile Ser Ala Ile Val Ala Ala Ala Leu Ser Ala Cys Ala Phe Ala
85 90 95
Ala Ala Ala Leu Pro Val Ala Thr Trp Met Tyr Arg Tyr Phe Thr Gly
100 105 110

Arg His Pro Val Gly Ala Asp Arg Val Asp Tyr Ala Arg Ser Arg Ile
115 120 125

Ala Asp Thr Ala Ser His Val Lys Asp Tyr Ala Arg Glu Tyr Gly Gly
130 135 140

Tyr Leu Arg Thr Arg Ala Lys Asp Ala Ala Pro Gly Ala
145 150 155

<210> 54

<211> 159

<212> PRT

<213> Zea mays

<400> 54

Met Asp Arg Lys Cys Leu Val Asp Leu Gly His Pro Leu Leu Asn Arg
1 5 10 15

Val Ala Asp Ser Phe Ile Arg Ala Ala Gly Val Gly Ala Ala Arg Ala
20 25 30

Val Ser Arg Glu Ala Tyr Val Val Thr Val Glu Gly Leu Ser Gly Asp
35 40 45

Ser Ser Gly Leu Asp Ala Asp Gly Gly Lys Arg Ser His Phe Ser Ser
50 55 60

Ile Arg Gly Asp Asp Gly Gln Arg Ser Leu Asp Ala Val Val Lys Thr
65 70 75 80

Ala Gly Lys Glu Ala Phe Gln Trp Gly Leu Ala Ala Gly Val Tyr Ser
85 90 95

Gly Leu Thr Tyr Ala Leu Arg Glu Ala Arg Gly Cys His Asp Trp Lys
100 105 110

Asn Ser Ala Ile Ala Gly Ala Ile Ala Gly Ala Ala Val Ala Leu Thr
115 120 125

Gly Asp Ala Gly Gly His Ser Asp Lys Leu Val Asn Phe Ala Ile Thr
130 135 140

Gly Ala Ala Leu Ser Ser Ala Gly Ser Leu Leu Ser Gly Ile Phe
145 150 155

<210> 55

<211> 4178

<212> DNA

<213> Artificial

<220>

<223> Vector

<400> 55

ttggtttttt gataatttgt ttatctcaca tgatggttct aacaatatgc gcagatctta 60

tacatctctc tcgtagtttt tcataaacta cgagaaagat ataggtttta tgaacaaatt 120

tacttttatt ttgtcatata atgaaatggt caaaatataa attgtacatc atgatgagtt 180

atacaaattt gtagtccaaa atcttttcat ttaaattaat ttactgcttt aaaatgtgat 240
ttttaattgt ctttgcctag tgttgaaaaa aagcactcgg caaagaaact ctttgccgag 300
tgttaaaaaa acactcggca aagaatctct ttgccgagtg tcaaaaataa aatatttggc 360
aaatagcttc tttgctgagt gtttttttgc ttagcacttg gtaaagaact tctttgccga 420
gtgtccgaaa aagcactcga taaaatattt ggcactcagc aaagaaccga attccagtag 480
cgtgagtcag aagttggcat ttgaaccctt tgaaataatc aaacaaatca acgcagttgc 540
atatgaattg gctttgccac atggtagtgc agtccatcga gtgttccatg tttcacagct 600
taaactaacc catggttttg gtaacagaac acacaccagt gactccatcg ttaccggatt 660
ttgatactac actgaaggtt ccggaacaaa tactggattc tagattgatt ctagattgct 720
tcgatcaaat ggggatcatc aacctaacac agcttaaatt atacatgcat gcgtacattt 780
ggtccttcaa gaggaggcag gtgatgatca aggtctctct agcggcctgg tgtgcagcca 840
cgtacccatc cacgcgtcgc cgcgggcgac gacaggtgtt ggccaacctg gacatcagac 900
aagcacgaca atgacaggcg ttcgccaagc gtacgtacag gagacgaaca cgacaagtag 960
caccgtcctg gccgagagcc cgtcatgctc atgcatgcat gcatgctgat catgcatact 1020
cgataaatat acagcagaga gctgagctac gtacacaaac aagaagcttg cattgttcgt 1080
tgctcgatcg ttgcaggtaa tggagaaccc tgctccgagt atcgtagtgc cccccatcgc 1140
cgcacctgta tcagcgaact tcagcagact cgccttccgc aacctgtaca tccgacggac 1200
gggtccagac agcagggagc gtacggccta ggccttcacc tgcggagggt aagatccgat 1260
caccatcttc tgaatttctg ttcttgatct gtcatgtata ataactgtct agtcttggtg 1320
ttggtgagat ggaaattcgg tggatctcgg aagggatatt gttcgtttgc tggggttttt 1380
tttgtgtgtt gtgatccgta gagaatttgt gtttatccat gttgttgatc ttggtatgta 1440
ttcatgacat attgacatgc atgtgttgta tgtgtcatat gtgtgcctct ccttgggatt 1500
tgttttggat aatagaacat gttatggact caatagtctg tgaacaaatc tttttttaga 1560
tggtggccaa atctgatgat gatctttctt gagaggaaaa agttcatgat agaaaaatct 1620
tttttgagat ggtggcttaa tgtgatgatg atctttcttg agaggaaaaa aaagattcat 1680
tataggagat tttgatttag ctcctttcca ccgatattaa atgaggagca tgcatgctga 1740
ttgctgataa ggatctgatt tttttatccc ctcttctttg aacagacaag aaataggctc 1800
tgaatttctg attgattatt tgtacatgca gaagggcgaa ttcgacctag gccaagtttg 1860
tacaaaaaag caggcttgat aaccaaccat ggtccgtcct gtagaaaccc caacccgtga 1920
aatcaaaaaa ctcgacggcc tgtgggcatt cagtctggat cgcgaaaact gtggaattga 1980
tcagcgttgg tgggaaagcg cgttacaaga aagccgggca attgctgtgc caggcagttt 2040
taacgatcag ttcgccgatg cagatattcg taattatgcg ggcaacgtct ggtatcagcg 2100
cgaagtcttt ataccgaaag gttgggcagg ccagcgtatc gtgctgcgtt tcgatgcggt 2160
cactcattac ggcaaagtgt gggtcaataa tcaggaagtg atggagcatc agggcggcta 2220
tacgccattt gaagccgatg tcacgccgta tgttattgcc gggaaaagtg tacgtaagtt 2280
tctgcttcta cctttgatat atatataata attatcatta attagtagta atataatatt 2340
tcaaatattt ttttcaaaat aaaagaatgt agtatatagc aattgctttt ctgtagttta 2400
taagtgtgta tattttaatt tataactttt ctaatatatg accaaaattt gttgatgtgc 2460
aggtatcacc gtttgtgtga acaacgaact gaactggcag actatcccgc cgggaatggt 2520
gattaccgac gaaaacggca agaaaaagca gtcttacttc catgatttct ttaactatgc 2580
cggaatccat cgcagcgtaa tgctctacac cacgccgaac acctgggtgg acgatatcac 2640

cgtggtgacg catgtcgcgc aagactgtaa ccacgcgtct gttgactggc aggtggtggc 2700
caatggtgat gtcagcgttg aactgcgtga tgcggatcaa caggtggttg caactggaca 2760
aggcactagc gggactttgc aagtggtgaa tccgcacctc tggcaaccgg gtgaaggtta 2820
tctctatgaa ctgtgcgtca cagccaaaag ccagacagag tgtgatatct acccgcttcg 2880
cgtcggcatc cggtcagtgg cagtgaaggg cgaacagttc ctgattaacc acaaaccgtt 2940
ctactttact ggctttggtc gtcatgaaga tgcggacttg cgtggcaaag gattcgataa 3000
cgtgctgatg gtgcacgacc acgcattaat ggactggatt ggggccaact cctaccgtac 3060
ctcgcattac ccttacgctg aagagatgct cgactgggca gatgaacatg gcatcgtggt 3120
gattgatgaa actgctgctg tcggctttaa cctctcttta ggcattggtt tcgaagcggg 3180
caacaagccg aaagaactgt acagcgaaga ggcagtcaac ggggaaactc agcaagcgca 3240
cttacaggcg attaaagagc tgatagcgcg tgacaaaaac cacccaagcg tggtgatgtg 3300
gagtattgcc aacgaaccgg atacccgtcc gcaaggtgca cgggaatatt tcgcgccact 3360
ggcggaagca acgcgtaaac tcgacccgac gcgtccgatc acctgcgtca atgtaatgtt 3420
ctgcgacgct cacaccgata ccatcagcga tctctttgat gtgctgtgcc tgaaccgtta 3480
ttacggatgg tatgtccaaa gcggcgattt ggaaacggca gagaaggtac tggaaaaaga 3540
acttctggcc tggcaggaga aactgcatca gccgattatc atcaccgaat acggcgtgga 3600
tacgttagcc gggctgcact caatgtacac cgacatgtgg agtgaagagt atcagtgtgc 3660
atggctggat atgtatcacc gcgtctttga tcgcgtcagc gccgtcgtcg gtgaacaggt 3720
atggaatttc gccgattttg cgacctcgca aggcatattg cgcgttggcg gtaacaagaa 3780
agggatcttc actcgcgacc gcaaaccgaa gtcggcggct tttctgctgc aaaaacgctg 3840
gactggcatg aacttcggtg aaaaaccgca gcagggaggc aaacaatgaa tcaaacccag 3900
ctttcttgta caaagtggga cctaggatcg ttcaaacatt tggcaataaa gtttcttaag 3960
attgaatcct gttgccggtc ttgcgatgat tatcatataa tttctgttga attacgttaa 4020
gcatgtaata attaacatgt aatgcatgac gttatttatg agatgggttt ttatgattag 4080
agtcccgcaa ttatacattt aatacgcgat agaaaacaaa atatagcgcg caaactagga 4140
taaattatcg cgcgcggtgt catctatgtt actagatc 4178

<210> 56

<211> 3882

<212> DNA

<213> Artificial

<220>

<223> Vector

<400> 56

ttagtagaga ataacacaca tctccctaag tatcacaaaa aaattaattt taagaggcaa 60
ttttagtttc caaagcgatt tattaagatt taagggaggg tatttattag aaggaagtac 120
attgatcaaa cagagaaaat tccatcatta tggtccccca tagaaatttg tcactcgatt 180
gtcgcaaaaa aagttaccag taaatttgat aaattttgga taccggtctg ttttaactca 240
tcaagaatag cacgacgaaa taatagattg cgtacgtttt cattcataac atctctcact 300
aaagcatttt ttacacacgc tattaaaatg gttcggaaac tgatccttaa gcggtctatt 360
catatccatc tatcgttcta agattcttcc tgtacgtaga ttctttccga ttaaattctc 420
atgaaaaatt aaaaacaaac aggtccttaa actagtcgcc ggaagttctc gcttttgctg 480

tagttgtagt gtcgatgcat gggtggaaac gtggacagat agattcatga aatgtggaca 540
acccacctgg tccagagcat catgatacat tgatacacgt ggcacgctgc ccactgatgc 600
cctcagcttt gaagctaacg tgtcaagtgt ggccggcccc tgcattgcgt cgtgccaatc 660
gtccaagtcc catggcaaaa acccagcgct ttgtgccgcc gccgtccgcc ggcccctctg 720
cccttgtacg tgcacctaga cacatcgtca tcgatcatca cacgcaatcg acacaagaag 780
ttaataaaca gcccaaggac gcagagatca gctgatcgag aaggacttgt actactactc 840
agtattgtcg tcacatgcac atatatgtac ataaagagct agctacctga gctctaccca 900
aggtcgcgtt gatcgatcga tcacgtacgg cctaggcctt cacctgcgga gggtaagatc 960
cgatcaccat cttctgaatt tctgttcttg atctgtcatg tataataact gtctagtctt 1020
ggtgttggtg agatggaaat tcggtggatc tcggaaggga tattgttcgt ttgctggggt 1080
tttttttgtg tgttgtgatc cgtagagaat ttgtgtttat ccatgttgtt gatcttggta 1140
tgtattcatg acatattgac atgcatgtgt tgtatgtgtc atatgtgtgc ctctccttgg 1200
gatttgtttt ggataataga acatgttatg gactcaatag tctgtgaaca aatctttttt 1260
tagatggtgg ccaaatctga tgatgatctt tcttgagagg aaaaagttca tgatagaaaa 1320
atcttttttg agatggtggc ttaatgtgat gatgatcttt cttgagagga aaaaaaagat 1380
tcattatagg agattttgat ttagctcctt tccaccgata ttaaatgagg agcatgcatg 1440
ctgattgctg ataaggatct gatttttta tcccctcttc tttgaacaga caagaaatag 1500
gctctgaatt tctgattgat tatttgtaca tgcagaaggg cgaattcgac ctaggccaag 1560
tttgtacaaa aaagcaggct tgataaccaa ccatggtccg tcctgtagaa accccaaccc 1620
gtgaaatcaa aaaactcgac ggcctgtggg cattcagtct ggatcgcgaa aactgtggaa 1680
ttgatcagcg ttggtgggaa agcgcgttac aagaaagccg ggcaattgct gtgccaggca 1740
gttttaacga tcagttcgcc gatgcagata ttcgtaatta tgcgggcaac gtctggtatc 1800
agcgcgaagt ctttataccg aaaggttggg caggccagcg tatcgtgctg cgtttcgatg 1860
cggtcactca ttacggcaaa gtgtgggtca ataatcagga agtgatggag catcagggcg 1920
gctatacgcc atttgaagcc gatgtcacgc cgtatgttat tgccgggaaa agtgtacgta 1980
agtttctgct tctacctttg atatatatat aataattatc attaattagt agtaatataa 2040
tatttcaaat atttttttca aaataaaaga atgtagtata tagcaattgc ttttctgtag 2100
tttataagtg tgtatatttt aatttataac ttttctaata tatgaccaaa atttgttgat 2160
gtgcaggtat caccgtttgt gtgaacaacg aactgaactg gcagactatc ccgccgggaa 2220
tggtgattac cgacgaaaac ggcaagaaaa agcagtctta cttccatgat ttctttaact 2280
atgccggaat ccatcgcagc gtaatgctct acaccacgcc gaacacctgg gtggacgata 2340
tcaccgtggt gacgcatgtc gcgcaagact gtaaccacgc gtctgttgac tggcaggtgg 2400
tggccaatgg tgatgtcagc gttgaactgc gtgatgcgga tcaacaggtg gttgcaactg 2460
gacaaggcac tagcgggact ttgcaagtgg tgaatccgca cctctggcaa ccgggtgaag 2520
gttatctcta tgaactgtgc gtcacagcca aaagccagac agagtgtgat atctacccgc 2580
ttcgcgtcgg catccggtca gtggcagtga agggcgaaca gttcctgatt aaccacaaac 2640
cgttctactt tactggcttt ggtcgtcatg aagatgcgga cttgcgtggc aaaggattcg 2700
ataacgtgct gatggtgcac gaccacgcat taatggactg gattggggcc aactcctacc 2760
gtacctcgca ttacccttac gctgaagaga tgctcgactg ggcagatgaa catggcatcg 2820
tggtgattga tgaaactgct gctgtcggct ttaacctctc tttaggcatt ggtttcgaag 2880
cgggcaacaa gccgaaagaa ctgtacagcg aagaggcagt caacggggaa actcagcaag 2940

cgcacttaca ggcgattaaa gagctgatag cgcgtgacaa aaaccaccca agcgtggtga 3000
tgtggagtat tgccaacgaa ccggataccc gtccgcaagg tgcacgggaa tatttcgcgc 3060
cactggcgga agcaacgcgt aaactcgacc cgacgcgtcc gatcacctgc gtcaatgtaa 3120
tgttctgcga cgctcacacc gataccatca gcgatctctt tgatgtgctg tgcctgaacc 3180
gttattacgg atggtatgtc caaagcggcg atttggaaac ggcagagaag gtactggaaa 3240
aagaacttct ggcctggcag gagaaactgc atcagccgat tatcatcacc gaatacggcg 3300
tggatacgtt agccgggctg cactcaatgt acaccgacat gtggagtgaa gagtatcagt 3360
gtgcatggct ggatatgtat caccgcgtct ttgatcgcgt cagcgccgtc gtcggtgaac 3420
aggtatggaa tttcgccgat tttgcgacct cgcaaggcat attgcgcgtt ggcggtaaca 3480
agaaagggat cttcactcgc gaccgcaaac cgaagtcggc ggcttttctg ctgcaaaaac 3540
gctggactgg catgaacttc ggtgaaaaac cgcagcaggg aggcaaacaa tgaatcaaac 3600
ccagctttct tgtacaaagt gggacctagg atcgttcaaa catttggcaa taaagtttct 3660
taagattgaa tcctgttgcc ggtcttgcga tgattatcat ataatttctg ttgaattacg 3720
ttaagcatgt aataattaac atgtaatgca tgacgttatt tatgagatgg gtttttatga 3780
ttagagtccc gcaattatac atttaatacg cgatagaaaa caaaatatag cgcgcaaact 3840
aggataaatt atcgcgcgcg gtgtcatcta tgttactaga tc 3882

<210> 57

<211> 4949

<212> DNA

<213> Artificial

<220>

<223> Vector

<400> 57

gattcagaac atctggtcag cttaaatgat gtggtaaagg gagcattatt cttttatttt 60
gttctttgtt ctgtgcaatg gccatatatt ttcgtattga tatgctctca gtggaacagt 120
ctgaagctaa tgtgaaactt cgatgttcag gtatgataat gtaccaacag agattaaccg 180
gctgagatgc agagttaact atcatgcatt aaagttccta cctgatattg aggaaatggc 240
tgttaagctt gctgcaagaa tgaggaacaa aactggcagc ataaatccat acatgtacgt 300
tataattatc cttttcgctg ggtgatattg tgtacagtag agcttgtatc tacccaagac 360
actatccagt gcatagctga agttgacgct ctcaaagttt atttcacaat caaatattga 420
cacatttttt ttgttcttaa agggctcttc acctgagatt tgaaaaaggg atggtggggc 480
tttctttctg tgattttgcc ggcacacggg aggagaaggc aatgatggcg gcttataggc 540
agaaagaatg gccaaggcgc ttcaaggtga cgcggactgg actagataca tctgttacat 600
tggactgaga aagaatccct aggagaaatg aagcaccgac actaactata cgcgctagtt 660
cgggaacccc gttttctcac gagactttta tttttccaag aaaaattagt tcattttttt 720
taggaaaata taaatctctt aagaaaatgt agttccgaaa ctagccctac aagtctaacg 780
attttctctt ttttgtatct gccacgcaga atggatccca tctatggcca ctggcgctgc 840
agaagagaaa agaagggcgg tgccctcttg agcccggtga gatcgctgtg atcctgcggg 900
cactggggta cacgagcggc acacagatat acgtcgcgtc cgggcaagtg tacggcggca 960
agaaccggat ggctcccctc aggaacatgt tccccaacct ggtaggcagg ctcaaatcca 1020
cttcagattg tgtcaagctg aagccagtga taactgataa gccactgccg tgcattgtgc 1080

atcgctgcag gtgacgaagg aggagctagc gagcgcggag gagctggcgc cgttccggcg 1140
gcacgtgacg agcctggcgg cgctggactt cctggtgtgc ctgcggtcgg acgcgttcgt 1200
gatgacgcac ggcggcaact tcgccaaact catcatcggg gcgcgccgct acgcggggca 1260
ccgcctcaag tcggtgaagc ccgacaaggg cctcatgtcc aagtccctgg gcgaccccga 1320
catgggctgg gcctccttca cggaggacgt cgtcgtcacg caccgcacga ggacgggcct 1380
ccccgagccc accttccccg gctacgatct ctgggagaac ccgctgacac cctgcatgtg 1440
cagggcatga gacgggccat ccctcagatc agagtggccg tctggccgag tcctcgtctt 1500
cgctaggcta cgactacgag tagtctcgtg cccacttcca tcagcgcgca tttcagcgag 1560
catatgcttc tactagttaa acacctagat ttgtatgtat ttggctggcc taatctgtag 1620
tttcacatag atcacttcat gctgaaaatg tcttggacaa ggcagtgaat gaagataaca 1680
tcagtaacga cctgcttttc caaagctcct cttggattta ccaagctgag ccaagaaatg 1740
gctaacctga gttttgactt gcagccttgc aggcatgacg tgggcgacct gaactcgcta 1800
gtcgtagagt ttctttccac gcggcagctc ggcagagcta cgcagcctct ctgcccgcca 1860
cgttctcctg caaggctgca actcgaagca ctgagaagct attaattaat ggattctcga 1920
gcatgcggcc ttgttcgcta aacctcgcgc acagaaacac aagcgagtac cagagcagct 1980
aggagtggtc acgtacggcc taggccttca cctgcggagg gtaagatccg atcaccatct 2040
tctgaatttc tgttcttgat ctgtcatgta taataactgt ctagtcttgg tgttggtgag 2100
atggaaattc ggtggatctc ggaagggata ttgttcgttt gctggggttt tttttgtgtg 2160
ttgtgatccg tagagaattt gtgtttatcc atgttgttga tcttggtatg tattcatgac 2220
atattgacat gcatgtgttg tatgtgtcat atgtgtgcct ctccttggga tttgttttgg 2280
ataatagaac atgttatgga ctcaatagtc tgtgaacaaa tcttttttta gatggtggcc 2340
aaatctgatg atgatctttc ttgagaggaa aaagttcatg atagaaaaat cttttttgag 2400
atggtggctt aatgtgatga tgatctttct tgagaggaaa aaaaagattc attataggag 2460
attttgattt agctcctttc caccgatatt aaatgaggag catgcatgct gattgctgat 2520
aaggatctga tttttttatc ccctcttctt tgaacagaca agaaataggc tctgaatttc 2580
tgattgatta tttgtacatg cagaagggcg aattcgacct aggccaagtt tgtacaaaaa 2640
agcaggcttg ataaccaacc atggtccgtc ctgtagaaac cccaaccccgt gaaatcaaaa 2700
aactcgacgg cctgtgggca ttcagtctgg atcgcgaaaa ctgtggaatt gatcagcgtt 2760
ggtgggaaag cgcgttacaa gaaagccggg caattgctgt gccaggcagt tttaacgatc 2820
agttcgccga tgcagatatt cgtaattatg cgggcaacgt ctggtatcag cgcgaagtct 2880
ttataccgaa aggttgggca ggccagcgta tcgtgctgcg tttcgatgcg gtcactcatt 2940
acggcaaagt gtgggtcaat aatcaggaag tgatggagca tcagggcggc tatacgccat 3000
ttgaagccga tgtcacgccg tatgttattg ccgggaaaag tgtacgtaag tttctgcttc 3060
tacctttgat atatatataa taattatcat taattagtag taatataata tttcaaatat 3120
ttttttcaaa ataaaagaat gtagtatata gcaattgctt ttctgtagtt tataagtgtg 3180
tatattttaa tttataactt ttctaatata tgaccaaaat ttgttgatgt gcaggtatca 3240
ccgtttgtgt gaacaacgaa ctgaactggc agactatccc gccgggaatg gtgattaccg 3300
acgaaaacgg caagaaaaag cagtcttact tccatgattt ctttaactat gccggaatcc 3360
atcgcagcgt aatgctctac accacgccga acacctgggt ggacgatatc accgtggtga 3420
cgcatgtcgc gcaagactgt aaccacgcgt ctgttgactg gcaggtggtg gccaatggtg 3480
atgtcagcgt tgaactgcgt gatgcggatc aacaggtggt tgcaactgga caaggcacta 3540

gcgggacttt gcaagtggtg aatccgcacc tctggcaacc gggtgaaggt tatctctatg 3600
aactgtgcgt cacagccaaa agccagacag agtgtgatat ctacccgctt cgcgtcggca 3660
tccggtcagt ggcagtgaag ggcgaacagt tcctgattaa ccacaaaccg ttctacttta 3720
ctggctttgg tcgtcatgaa gatgcggact tgcgtggcaa aggattcgat aacgtgctga 3780
tggtgcacga ccacgcatta atggactgga ttggggccaa ctcctaccgt acctcgcatt 3840
acccttacgc tgaagagatg ctcgactggg cagatgaaca tggcatcgtg gtgattgatg 3900
aaactgctgc tgtcggcttt aacctctctt taggcattgg tttcgaagcg ggcaacaagc 3960
cgaaagaact gtacagcgaa gaggcagtca acggggaaac tcagcaagcg cacttacagg 4020
cgattaaaga gctgatagcg cgtgacaaaa accacccaag cgtggtgatg tggagtattg 4080
ccaacgaacc ggatacccgt ccgcaaggtg cacgggaata tttcgcgcca ctggcggaag 4140
caacgcgtaa actcgacccg acgcgtccga tcacctgcgt caatgtaatg ttctgcgacg 4200
ctcacaccga taccatcagc gatctctttg atgtgctgtg cctgaaccgt tattacggat 4260
ggtatgtcca aagcggcgat ttggaaacgg cagagaaggt actggaaaaa gaacttctgg 4320
cctggcagga gaaactgcat cagccgatta tcatcaccga atacggcgtg gatacgttag 4380
ccgggctgca ctcaatgtac accgacatgt ggagtgaaga gtatcagtgt gcatggctgg 4440
atatgtatca ccgcgtcttt gatcgcgtca gcgccgtcgt cggtgaacag gtatggaatt 4500
tcgccgattt tgcgacctcg caaggcatat tgcgcgttgg cggtaacaag aaagggatct 4560
tcactcgcga ccgcaaaccg aagtcggcgg cttttctgct gcaaaaacgc tggactggca 4620
tgaacttcgg tgaaaaaccg cagcagggag gcaaacaatg aatcaaaccc agctttcttg 4680
tacaaagtgg gagctcgatc gttcaaacat ttggcaataa agtttcttaa gattgaatcc 4740
tgttgccggt cttgcgatga ttatcatata atttctgttg aattacgtta agcatgtaat 4800
aattaacatg taatgcatga cgttatttat gagatgggtt tttatgatta gagtcccgca 4860
attatacatt taatacgcga tagaaaacaa aatatagcgc gcaaactagg ataaattatc 4920
gcgcgcggtg tcatctatgt tactagatc 4949

<210> 58
<211> 3638
<212> DNA
<213> Artificial
<220>
<223> Vector
<400> 58

gccagtgcta atgatattta cactagcggg ctgctaaaga aaaccgccag tgctaaagat 60
atttacacta gcggttggtg aacaactgcc tgtgaaaaaa gccgatttct actagcccct 120
agctagcact ggcgacataa aaaacgtcag tgaaaatagc tctaggatcg tcactataga 180
gcttctatgt acttagtggt tagaactgat attgtagtgc accaagtgcc gattttaatt 240
aaaccaatac taaatactag taaataatac tagtggtctg aattcgattt ctatagtaat 300
gtttgcttgc aagccgcaaa tagagtaaac attcgtcgtc acagaaatcc acattacatc 360
aaggtccatg gcggccggcc acgtacccat cccacgcgtc gctgcggagg acacgtgttg 420
gctgaccgga cagttggccg atcagacagt ggacagaccg gacaatagaa gaagaagacg 480
acgacggcgg cggcaccgcc gagtaggtgc atggtcacgc tagctgtagc tttttgcaga 540
gcgtcgtctg taaatacgta gcccttccac aagcgaggca aggggggaga gagtatcgtc 600

agctagcaga gagagtgcgt agcaactagc aatggcggag aaccctgctc catttaaatg 660
ccagctgtac actagttatc gtacggccta ggccttcacc tgcggagggt aagatccgat 720
caccatcttc tgaatttctg ttcttgatct gtcatgtata ataactgtct agtcttggtg 780
ttggtgagat ggaaattcgg tggatctcgg aagggatatt gttcgtttgc tggggttttt 840
tttgtgtgtt gtgatccgta gagaatttgt gtttatccat gttgttgatc ttggtatgta 900
ttcatgacat attgacatgc atgtgttgta tgtgtcatat gtgtgcctct ccttgggatt 960
tgttttggat aatagaacat gttatggact caatagtctg tgaacaaatc tttttttaga 1020
tggtggccaa atctgatgat gatctttctt gagaggaaaa agttcatgat agaaaaatct 1080
tttttgagat ggtggcttaa tgtgatgatg atctttcttg agaggaaaaa aaagattcat 1140
tataggagat tttgatttag ctcctttcca ccgatattaa atgaggagca tgcatgctga 1200
ttgctgataa ggatctgatt tttttatccc ctcttctttg aacagacaag aaataggctc 1260
tgaatttctg attgattatt tgtacatgca gaagggcgaa ttcgacctag gccaagtttg 1320
tacaaaaaag caggcttgat aaccaaccat ggtccgtcct gtagaaaccc caacccgtga 1380
aatcaaaaaa ctcgacggcc tgtgggcatt cagtctggat cgcgaaaact gtggaattga 1440
tcagcgttgg tgggaaagcg cgttacaaga aagccgggca attgctgtgc caggcagttt 1500
taacgatcag ttcgccgatg cagatattcg taattatgcg ggcaacgtct ggtatcagcg 1560
cgaagtcttt ataccgaaag gttgggcagg ccagcgtatc gtgctgcgtt tcgatgcggt 1620
cactcattac ggcaaagtgt gggtcaataa tcaggaagtg atggagcatc agggcggcta 1680
tacgccattt gaagccgatg tcacgccgta tgttattgcc gggaaaagtg tacgtaagtt 1740
tctgcttcta cctttgatat atatataata attatcatta attagtagta atataatatt 1800
tcaaatattt ttttcaaaat aaaagaatgt agtatatagc aattgctttt ctgtagttta 1860
taagtgtgta tattttaatt tataactttt ctaatatatg accaaaattt gttgatgtgc 1920
aggtatcacc gtttgtgtga acaacgaact gaactggcag actatcccgc cgggaatggt 1980
gattaccgac gaaaacggca agaaaaagca gtcttacttc catgatttct ttaactatgc 2040
cggaatccat cgcagcgtaa tgctctacac cacgccgaac acctgggtgg acgatatcac 2100
cgtggtgacg catgtcgcgc aagactgtaa ccacgcgtct gttgactggc aggtggtggc 2160
caatggtgat gtcagcgttg aactgcgtga tgcggatcaa caggtggttg caactggaca 2220
aggcactagc gggactttgc aagtggtgaa tccgcacctc tggcaaccgg gtgaaggtta 2280
tctctatgaa ctgtgcgtca cagccaaaag ccagacagag tgtgatatct acccgcttcg 2340
cgtcggcatc cggtcagtgg cagtgaaggg cgaacagttc ctgattaacc acaaaccgtt 2400
ctactttact ggctttggtc gtcatgaaga tgcggacttg cgtggcaaag gattcgataa 2460
cgtgctgatg gtgcacgacc acgcattaat ggactggatt ggggccaact cctaccgtac 2520
ctcgcattac ccttacgctg aagagatgct cgactgggca gatgaacatg gcatcgtggt 2580
gattgatgaa actgctgctg tcggctttaa cctctcttta ggcattggtt tcgaagcggg 2640
caacaagccg aaagaactgt acagcgaaga ggcagtcaac ggggaaactc agcaagcgca 2700
cttacaggcg attaaagagc tgatagcgcg tgacaaaaac cacccaagcg tggtgatgtg 2760
gagtattgcc aacgaaccgg atacccgtcc gcaaggtgca cgggaatatt tcgcgccact 2820
ggcggaagca acgcgtaaac tcgacccgac gcgtccgatc acctgcgtca atgtaatgtt 2880
ctgcgacgct cacaccgata ccatcagcga tctctttgat gtgctgtgcc tgaaccgtta 2940
ttacggatgg tatgtccaaa gcggcgattt ggaaacggca gagaaggtac tggaaaaaga 3000
acttctggcc tggcaggaga aactgcatca gccgattatc atcaccgaat acggcgtgga 3060

tacgttagcc gggctgcact caatgtacac cgacatgtgg agtgaagagt atcagtgtgc 3120
atggctggat atgtatcacc gcgtctttga tcgcgtcagc gccgtcgtcg gtgaacaggt 3180
atggaatttc gccgattttg cgacctcgca aggcatattg cgcgttggcg gtaacaagaa 3240
agggatcttc actcgcgacc gcaaaccgaa gtcggcggct tttctgctgc aaaaacgctg 3300
gactggcatg aacttcggtg aaaaaccgca gcagggaggc aaacaatgaa tcaaacccag 3360
ctttcttgta caaagtggga cctaggatcg ttcaaacatt tggcaataaa gtttcttaag 3420
attgaatcct gttgccggtc ttgcgatgat tatcatataa tttctgttga attacgttaa 3480
gcatgtaata attaacatgt aatgcatgac gttatttatg agatgggttt ttatgattag 3540
agtcccgcaa ttatacattt aatacgcgat agaaaacaaa atatagcgcg caaactagga 3600
taaattatcg cgcgcggtgt catctatgtt actagatc 3638

<210> 59

<211> 3657

<212> DNA

<213> Artificial

<220>

<223> Vector

<400> 59

gtgattaagt tgactggcaa attgcataga cgataataca ctctatgctt ttgagataac 60
cactgtacac caaccgactt gttgtttgaa ggctgatctt taaccattac tactgtaact 120
aactgttgtt gaataaacta ttcatacgac ttggagactt tgatgtatat tatgttttgt 180
acctatacta ttaactaccg cagatacgta tttacatctc ggtaacatct gccagtcgca 240
caaatgcact aaggtgactg acagactgta gcgaccgata gagcgctgcc ttgttctagt 300
caaaattcaa gcctggtggt cttctcgatc cctctgcgcg cgccctgctc ttttatcctg 360
tgtgtctcct ccacgaggga aggcgtcatg agaggacgac acgacgacca tgcagtgctg 420
cgcaaccgcg cgcgtctgcc tgttcactga gccccaggcg gccggtccaa gctattacca 480
cgtcgccccc ctccagggct gcgggcagca acgtgtcgac cggtccgcct ctgacgtgtc 540
cctcccgtcc tctgcataaa agggtgcggc tggcggggag cctcagcttc atatcgtcgc 600
aagctctccg tgtttctctc gtttcttcac tgctgccaac tgtgctgtgc agtagttgca 660
agagctgatt ggtagctagg tacgctaggc aagtaggccg tacggcctag gccttcacct 720
gcggagggta agatccgatc accatcttct gaatttctgt tcttgatctg tcatgtataa 780
taactgtcta gtcttggtgt tggtgagatg gaaattcggt ggatctcgga agggatattg 840
ttcgtttgct ggggtttttt ttgtgtgttg tgatccgtag agaatttgtg tttatccatg 900
ttgttgatct tggtatgtat tcatgacata ttgacatgca tgtgttgtat gtgtcatatg 960
tgtgcctctc cttgggattt gttttggata atagaacatg ttatggactc aatagtctgt 1020
gaacaaatct ttttttagat ggtggccaaa tctgatgatg atctttcttg agaggaaaaa 1080
gttcatgata gaaaaatctt ttttgagatg gtggcttaat gtgatgatga tctttcttga 1140
gaggaaaaaa aagattcatt ataggagatt ttgatttagc tcctttccac cgatattaaa 1200
tgaggagcat gcatgctgat tgctgataag gatctgattt ttttatcccc tcttctttga 1260
acagacaaga aataggctct gaatttctga ttgattattt gtacatgcag aagggcgaat 1320
tcgacctagg ccaagtttgt acaaaaaagc aggcttgata accaaccatg gtccgtcctg 1380
tagaaacccc aacccgtgaa atcaaaaaac tcgacggcct gtgggcattc agtctggatc 1440

gcgaaaactg tggaattgat cagcgttggt gggaaagcgc gttacaagaa agccgggcaa 1500
ttgctgtgcc aggcagtttt aacgatcagt tcgccgatgc agatattcgt aattatgcgg 1560
gcaacgtctg gtatcagcgc gaagtcttta taccgaaagg ttgggcaggc cagcgtatcg 1620
tgctgcgttt cgatgcggtc actcattacg gcaaagtgtg ggtcaataat caggaagtga 1680
tggagcatca gggcggctat acgccatttg aagccgatgt cacgccgtat gttattgccg 1740
ggaaaagtgt acgtaagttt ctgcttctac ctttgatata tatataataa ttatcattaa 1800
ttagtagtaa tataatattt caaatatttt tttcaaaata aaagaatgta gtatatagca 1860
attgcttttc tgtagtttat aagtgtgtat attttaattt ataactttc taatatatga 1920
ccaaaatttg ttgatgtgca ggtatcaccg tttgtgtgaa caacgaactg aactggcaga 1980
ctatcccgcc gggaatggtg attaccgacg aaaacggcaa gaaaaagcag tcttacttcc 2040
atgatttctt taactatgcc ggaatccatc gcagcgtaat gctctacacc acgccgaaca 2100
cctgggtgga cgatatcacc gtggtgacgc atgtcgcgca agactgtaac cacgcgtctg 2160
ttgactggca ggtggtggcc aatggtgatg tcagcgttga actgcgtgat gcggatcaac 2220
aggtggttgc aactggacaa ggcactagcg ggactttgca agtggtgaat ccgcacctct 2280
ggcaaccggg tgaaggttat ctctatgaac tgtgcgtcac agccaaaagc cagacagagt 2340
gtgatatcta cccgcttcgc gtcggcatcc ggtcagtggc agtgaagggc gaacagttcc 2400
tgattaacca caaaccgttc tactttactg gctttggtcg tcatgaagat gcggacttgc 2460
gtggcaaagg attcgataac gtgctgatgg tgcacgacca cgcattaatg gactggattg 2520
gggccaactc ctaccgtacc tcgcattacc cttacgctga agagatgctc gactgggcag 2580
atgaacatgg catcgtggtg attgatgaaa ctgctgctgt cggctttaac ctctctttag 2640
gcattggttt cgaagcgggc aacaagccga aagaactgta cagcgaagag gcagtcaacg 2700
gggaaactca gcaagcgcac ttacaggcga ttaaagagct gatagcgcgt gacaaaaacc 2760
acccaagcgt ggtgatgtgg agtattgcca acgaaccgga tacccgtccg caaggtgcac 2820
gggaatattt cgcgccactg gcggaagcaa cgcgtaaact cgacccgacg cgtccgatca 2880
cctgcgtcaa tgtaatgttc tgcgacgctc acaccgatac catcagcgat ctctttgatg 2940
tgctgtgcct gaaccgttat tacggatggt atgtccaaag cggcgatttg gaaacggcag 3000
agaaggtact ggaaaaagaa cttctggcct ggcaggagaa actgcatcag ccgattatca 3060
tcaccgaata cggcgtggat acgttagccg ggctgcactc aatgtacacc gacatgtgga 3120
gtgaagagta tcagtgtgca tggctggata tgtatcaccg cgtctttgat cgcgtcagcg 3180
ccgtcgtcgg tgaacaggta tggaatttcg ccgattttgc gacctcgcaa ggcatattgc 3240
gcgttggcgg taacaagaaa gggatcttca ctcgcgaccg caaaccgaag tcggcggctt 3300
ttctgctgca aaaacgctgg actggcatga acttcggtga aaaaccgcag cagggaggca 3360
aacaatgaat caaacccagc tttcttgtac aaagtgggac ctaggatcgt tcaaacattt 3420
ggcaataaag tttcttaaga ttgaatcctg ttgccggtct tgcgatgatt atcatataat 3480
ttctgttgaa ttacgttaag catgtaataa ttaacatgta atgcatgacg ttatttatga 3540
gatgggtttt tatgattaga gtcccgcaat tatacattta atacgcgata gaaaacaaaa 3600
tatagcgcgc aaactaggat aaattatcgc gcgcggtgtc atctatgtta ctagatc 3657

<210> 60

<211> 4315

<212> DNA

<213> Artificial

<220>
<223> Vector
<400> 60
tataaattag aacggagggt atgttttttt acgtttccat tatttgagtc tgctcatcac 60
gtttaatttg tttatacagt tcaatgagtt cagttacagc ggatgataat tcgacggcgt 120
ttatttgcat gcacttactt tttacattac gcctagaatg taatcggatg gacgaatatt 180
acttgttttg tgtttatttt catatttttt tctcttcgga tacggatatt atcgagttgt 240
gccgaacaag attttgaatg tatatcgaca tcttaaatat ataactttga atatacggat 300
acgtatatag atcggatatt gaatacccag ctagactcag attaaattgg atcttagtta 360
ttttaggggg tgtttgaatg cactaaaact aatagttagt ggctaaaatt agttgaaaca 420
tccaaacacc ctatctaata gttcagctat tagttatttt tggaaaatta gttaatagtt 480
aggtagttat ctgttagcta gctaattcaa ctaattataa gttctagtgc atttaaatac 540
cccttagacg gatcgaatta gaacaccgat ggataatatc cataacattt tacacctata 600
aatatgtcct atatgtatat agatgtcaaa aacaggttgg gcatgttgga tgacccgtgg 660
cacgatacag ttttaagcag tgccaaacat ggcatggcgg cgagctagac atgatacggt 720
ccggttttat ttttgtattt ttttttcgta ataatatctg tattatgctt gttcgaacct 780
cacttgctta tgtggatgtg atagtatcaa ctcgctggcg ttccgtgcat cgtaacatct 840
atcacatccc acagatcatg tcgcttatct tttctgatac tatagcagta gcacacagat 900
gccggtcgaa cccagaagca tcacgccatc accccctccc cttttcccaa tcaaaaccac 960
gtccgtccca atggtaaccc agcaccaatc tcgtgttcgt gtgagacaga gcatgcaaac 1020
aaacagcatg catgcgcgaa ggaaagcgtg cagcgcgcgg gacgtgaacc gcgcgcgaac 1080
cctgccgccc gtccggtctc agctcacgcc cagcgtcctt cccaggccgc gatccggcca 1140
tcctgacgcg cgtttcgtct gggccccaaa accacgtccg ccgcggcggc ggcgcgccca 1200
cgggctcccg tgcccgccga tagtttgccg gaccctgccg cctcctataa atgcagagcc 1260
ctccgccagc gtctcccgca tctcatcttc ttcctccatc cgcactactg agagcgacaa 1320
agctagctct ctccagggat cggattcgga tcaccacgta cggcctaggc cttcacctgc 1380
ggagggtaag atccgatcac catcttctga atttctgttc ttgatctgtc atgtataata 1440
actgtctagt cttggtgttg gtgagatgga aattcggtgg atctcggaag ggatattgtt 1500
cgtttgctgg ggtttttttt gtgtgttgtg atccgtagag aatttgtgtt tatccatgtt 1560
gttgatcttg gtatgtattc atgacatatt gacatgcatg tgttgtatgt gtcatatgtg 1620
tgcctctcct tgggatttgt tttggataat agaacatgtt atggactcaa tagtctgtga 1680
acaaatcttt ttttagatgg tggccaaatc tgatgatgat ctttcttgag aggaaaaagt 1740
tcatgataga aaaatctttt ttgagatggt ggcttaatgt gatgatgatc tttcttgaga 1800
ggaaaaaaaa gattcattat aggagatttt gatttagctc ctttccaccg atattaaatg 1860
aggagcatgc atgctgattg ctgataagga tctgattttt ttatcccctc ttctttgaac 1920
agacaagaaa taggctctga atttctgatt gattatttgt acatgcagaa gggcgaattc 1980
gacctaggcc aagtttgtac aaaaaagcag gcttgataac caaccatggt ccgtcctgta 2040
gaaaccccaa cccgtgaaat caaaaaactc gacggcctgt gggcattcag tctggatcgc 2100
gaaaactgtg gaattgatca gcgttggtgg gaaagcgcgt tacaagaaag ccgggcaatt 2160
gctgtgccag gcagttttaa cgatcagttc gccgatgcag atattcgtaa ttatgcgggc 2220
aacgtctggt atcagcgcga agtctttata ccgaaaggtt gggcaggcca gcgtatcgtg 2280

ctgcgtttcg atgcggtcac tcattacggc aaagtgtggg tcaataatca ggaagtgatg 2340
gagcatcagg gcggctatac gccatttgaa gccgatgtca cgccgtatgt tattgccggg 2400
aaaagtgtac gtaagtttct gcttctacct ttgatatata tataataatt atcattaatt 2460
agtagtaata taatatttca aatatttttt tcaaaataaa agaatgtagt atatagcaat 2520
tgcttttctg tagtttataa gtgtgtatat tttaatttat aacttttcta atatatgacc 2580
aaaatttgtt gatgtgcagg tatcaccgtt tgtgtgaaca acgaactgaa ctggcagact 2640
atcccgccgg gaatggtgat taccgacgaa aacggcaaga aaaagcagtc ttacttccat 2700
gatttcttta actatgccgg aatccatcgc agcgtaatgc tctacaccac gccgaacacc 2760
tgggtggacg atatcaccgt ggtgacgcat gtcgcgcaag actgtaacca cgcgtctgtt 2820
gactggcagg tggtggccaa tggtgatgtc agcgttgaac tgcgtgatgc ggatcaacag 2880
gtggttgcaa ctggacaagg cactagcggg actttgcaag tggtgaatcc gcacctctgg 2940
caaccgggtg aaggttatct ctatgaactg tgcgtcacag ccaaaagcca gacagagtgt 3000
gatatctacc cgcttcgcgt cggcatccgg tcagtggcag tgaagggcga acagttcctg 3060
attaaccaca aaccgttcta ctttactggc tttggtcgtc atgaagatgc ggacttgcgt 3120
ggcaaaggat tcgataacgt gctgatggtg cacgaccacg cattaatgga ctggattggg 3180
gccaactcct accgtacctc gcattaccct tacgctgaag agatgctcga ctgggcagat 3240
gaacatggca tcgtggtgat tgatgaaact gctgctgtcg gctttaacct ctctttaggc 3300
attggtttcg aagcgggcaa caagccgaaa gaactgtaca gcgaagaggc agtcaacggg 3360
gaaactcagc aagcgcactt acaggcgatt aaagagctga tagcgcgtga caaaaaccac 3420
ccaagcgtgg tgatgtggag tattgccaac gaaccggata cccgtccgca aggtgcacgg 3480
gaatatttcg cgccactggc ggaagcaacg cgtaaactcg acccgacgcg tccgatcacc 3540
tgcgtcaatg taatgttctg cgacgctcac accgatacca tcagcgatct ctttgatgtg 3600
ctgtgcctga accgttatta cggatggtat gtccaaagcg gcgatttgga aacggcagag 3660
aaggtactgg aaaaagaact tctggcctgg caggagaaac tgcatcagcc gattatcatc 3720
accgaatacg gcgtggatac gttagccggg ctgcactcaa tgtacaccga catgtggagt 3780
gaagagtatc agtgtgcatg gctggatatg tatcaccgcg tctttgatcg cgtcagcgcc 3840
gtcgtcggtg aacaggtatg gaatttcgcc gattttgcga cctcgcaagg catattgcgc 3900
gttggcggta acaagaaagg gatcttcact cgcgaccgca aaccgaagtc ggcggctttt 3960
ctgctgcaaa aacgctggac tggcatgaac ttcggtgaaa aaccgcagca gggaggcaaa 4020
caatgaatca aacccagctt tcttgtacaa agtgggacct aggatcgttc aaacatttgg 4080
caataaagtt tcttaagatt gaatcctgtt gccggtcttg cgatgattat catataattt 4140
ctgttgaatt acgttaagca tgtaataatt aacatgtaat gcatgacgtt atttatgaga 4200
tgggttttta tgattagagt cccgcaatta tacatttaat acgcgataga aaacaaaata 4260
tagcgcgcaa actaggataa attatcgcgc gcggtgtcat ctatgttact agatc 4315

<210> 61
<211> 4960
<212> DNA
<213> Artificial
<220>
<223> Vector
<400> 61

aagggactcg tggcctacac acgcgcacaa taatcagtta ccatgaatta ttttatatgc 60
gaatatcgac attacacatg aataatgtga atttgaattt ccgcagcatg caaaattccc 120
gtgagtttga agggcgataa aaacgggtgg ccaaaattgg ggcggcaatg caagaagata 180
agcctatcca tcccttgcat tggtattgtc atctggggca gggcagggga gggcaggaga 240
agctatctat tggccatcta tccagcagcc gctctcccca gccccactct ctccatctag 300
agctccctaa ctgcaccggc cgccacacca ccgtaccccg acccctcgac aacaatggcc 360
accgtcctag gcagcccccg cgcgccggcc ttcttcttct cgccgtcctc cctccgtgcc 420
gcgccggcgc ccaccgccgt ggcgctgcct gcggccaagg tgggcatcat gggccgtagc 480
gccagcagca ggggcaggct gcgcgcgcag gccacctaca acgtgaagct gatcacgccg 540
gagggggagg tggagctgca ggtgcccgac gacgtgtaca tcctggacca ggccgaggag 600
gacggcatcg acctgcccta ctcctgccgc gcggggtcct gctcctcctg cgccggcaag 660
gtcgtctccg gctccgtgga ccagtccgac cagagctacc tcgacgacgg ccagatcgcc 720
gccggctggg tgctcacctg ccacgcctac cccacctctg acgtcgtcat cgagacgcac 780
aaggaggagg agctcaccgg cgcataatca ttcatgccca tccatctcta tctctgtggg 840
tcgtcgttgt gtgtaatttg agtacgcgcg tacacgtact gcttttgttt catttgagca 900
ctgttcgtgt aagcgtcgag ttgccctgcc atcgtctctc tctctctcga tctctactat 960
ttctgtgtgt gcgcgcttct atttttccaa tgttgagtta cgcatatttg tccagtaacc 1020
ttgttttgct taatcacaca cagctcccaa attccagtcg ttctgtggtg acggttggtt 1080
gtggggtgtg gcggcgacgt cagtcatggg agaagagatc cgaatgcatg gggtgttgg 1140
cgctttctct gtccctctcc ctctccctgt cttctcgcgc gctcgccgtc gtcatggctc 1200
gacgaacaga cattcactgc cttccccgcg cgcttcagac agaccacgct tgtacgtagc 1260
gccgcttctc tagtcgccgg agcaggcacg agtgggtggc gaagaggcca cggcccgacc 1320
tgctagcgcc agcgaccgca tctcgccacc agccgctgcc cgctggccgc tgccaccgta 1380
tgttgggttg ggctgaagcc tgaagcccgt gaccttttc ttttcttta ttctttcctt 1440
ttcttttgcc ggacaagaca agtgatgttg caaaatccat ataaaatcct agagaaacta 1500
taaagttttt ttttgttgaa ctccttataa caacgtctac cctttaatat atggtatata 1560
atttcacatg ttttagtata aattgtttac tgtatttcta tgtctatatg ctcttggtaa 1620
gattgtttat aaattttaaa tttaaaatct taaaagtcat aaacttatag aaaaaaatta 1680
ggatcctaaa caattttaaa caaaaaaaaa aacttttcaa ttacaaaatc gtatgtgttc 1740
tttatgcatg catgcaactg agaagctgcc caacggccag cgcagcagaa gggcagagcg 1800
gtgggcggcc acgtcgccgc cgcaaaggaa ctgacgtgtc ccggcccgac gacgacgacg 1860
cgaaccttca ccgcgccgcg ctccacctct cttaagctaa atacgctagt gcaggcgcag 1920
gcatccagct gcgacaacgt agcatacact agtgcttttg ctagctactt gtgcgagaag 1980
agagagcgca cggcgataga tcgtacggcc taggccttca cctgcggagg gtaagatccg 2040
atcaccatct tctgaatttc tgttcttgat ctgtcatgta taataactgt ctagtcttgg 2100
tgttggtgag atggaaattc ggtggatctc ggaagggata ttgttcgttt gctggggttt 2160
tttttgtgtg ttgtgatccg tagagaattt gtgtttatcc atgttgttga tcttggtatg 2220
tattcatgac atattgacat gcatgtgttg tatgtgtcat atgtgtgcct ctccttggga 2280
tttgttttgg ataatagaac atgttatgga ctcaatagtc tgtgaacaaa tcttttttta 2340
gatggtggcc aaatctgatg atgatctttc ttgagaggaa aaagttcatg atagaaaaat 2400
ctttttgag atggtggctt aatgtgatga tgatctttct tgagaggaaa aaaaagattc 2460

attataggag attttgattt agctcctttc caccgatatt aaatgaggag catgcatgct 2520
gattgctgat aaggatctga tttttttatc ccctcttctt tgaacagaca agaaataggc 2580
tctgaatttc tgattgatta tttgtacatg cagaagggcg aattcgacct aggccaagtt 2640
tgtacaaaaa agcaggcttg ataaccaacc atggtccgtc ctgtagaaac cccaacccgt 2700
gaaatcaaaa aactcgacgg cctgtgggca ttcagtctgg atcgcgaaaa ctgtggaatt 2760
gatcagcgtt ggtgggaaag cgcgttacaa gaaagccggg caattgctgt gccaggcagt 2820
tttaacgatc agttcgccga tgcagatatt cgtaattatg cgggcaacgt ctggtatcag 2880
cgcgaagtct ttataccgaa aggttgggca ggccagcgta tcgtgctgcg tttcgatgcg 2940
gtcactcatt acggcaaagt gtgggtcaat aatcaggaag tgatggagca tcagggcggc 3000
tatacgccat ttgaagccga tgtcacgccg tatgttattg ccgggaaaag tgtacgtaag 3060
tttctgcttc tacctttgat atatatataa taattatcat taattagtag taatataata 3120
tttcaaatat ttttttcaaa ataaaagaat gtagtatata gcaattgctt ttctgtagtt 3180
tataagtgtg tatattttaa tttataactt ttctaatata tgaccaaaat ttgttgatgt 3240
gcaggtatca ccgtttgtgt gaacaacgaa ctgaactggc agactatccc gccgggaatg 3300
gtgattaccg acgaaaacgg caagaaaaag cagtcttact tccatgattt ctttaactat 3360
gccggaatcc atcgcagcgt aatgctctac accacgccga acacctgggt ggacgatatc 3420
accgtggtga cgcatgtcgc gcaagactgt aaccacgcgt ctgttgactg gcaggtggtg 3480
gccaatggtg atgtcagcgt tgaactgcgt gatgcggatc aacaggtggt tgcaactgga 3540
caaggcacta gcgggacttt gcaagtggtg aatccgcacc tctggcaacc gggtgaaggt 3600
tatctctatg aactgtgcgt cacagccaaa agccagacag agtgtgatat ctacccgctt 3660
cgcgtcggca tccggtcagt ggcagtgaag ggcgaacagt tcctgattaa ccacaaaccg 3720
ttctacttta ctggctttgg tcgtcatgaa gatgcggact tgcgtggcaa aggattcgat 3780
aacgtgctga tggtgcacga ccacgcatta atggactgga ttggggccaa ctcctaccgt 3840
acctcgcatt acccttacgc tgaagagatg ctcgactggg cagatgaaca tggcatcgtg 3900
gtgattgatg aaactgctgc tgtcggcttt aacctctctt taggcattgg tttcgaagcg 3960
ggcaacaagc cgaaagaact gtacagcgaa gaggcagtca acggggaaac tcagcaagcg 4020
cacttacagg cgattaaaga gctgatagcg cgtgacaaaa accacccaag cgtggtgatg 4080
tggagtattg ccaacgaacc ggatacccgt ccgcaaggtg cacgggaata tttcgcgcca 4140
ctggcggaag caacgcgtaa actcgacccg acgcgtccga tcacctgcgt caatgtaatg 4200
ttctgcgacg ctcacaccga taccatcagc gatctctttg atgtgctgtg cctgaaccgt 4260
tattacggat ggtatgtcca aagcggcgat ttggaaacgg cagagaaggt actggaaaaa 4320
gaacttctgg cctggcagga gaaactgcat cagccgatta tcatcaccga atacggcgtg 4380
gatacgttag ccgggctgca ctcaatgtac accgacatgt ggagtgaaga gtatcagtgt 4440
gcatggctgg atatgtatca ccgcgtcttt gatcgcgtca gcgccgtcgt cggtgaacag 4500
gtatggaatt tcgccgattt tgcgacctcg caaggcatat tgcgcgttgg cggtaacaag 4560
aaagggatct tcactcgcga ccgcaaaccg aagtcggcgg cttttctgct gcaaaaacgc 4620
tggactggca tgaacttcgg tgaaaaaccg cagcagggag gcaaacaatg aatcaaaccc 4680
agctttcttg tacaaagtgg gacctaggat cgttcaaaca tttggcaata aagtttctta 4740
agattgaatc ctgttgccgg tcttgcgatg attatcatat aatttctgtt gaattacgtt 4800
aagcatgtaa taattaacat gtaatgcatg acgttattta tgagatgggt ttttatgatt 4860
agagtcccgc aattatacat ttaatacgcg atagaaaaca aaatatagcg cgcaaactag 4920

gataaattat cgcgcgcggt gtcatctatg ttactagatc 4960

<210> 62

<211> 4146

<212> DNA

<213> Artificial

<220>

<223> Vector

<400> 62

gagcgacctc ggactcagcg gctccgcctg tcggcgagcg cacgccgcgt gcctgcccgc 60
ggtctctcgt cgtctccaga agatgcgccc cgatactcgt ctttccaccc ttcttctacc 120
tccttccacc cccttctccc cacgccccat cggatcgcac gcgcaccgca attgttgttc 180
atcacctctc cgaagaagcg tcacagagca ccgcccggag gttccccttc ccgtcgccgt 240
cgcttggcca ggtaagtaat gactaatcgc ctgagtctcc tgcgcctcat tacttcgaac 300
tgtggtttcg ttagtcggac gtcggagcaa tgctatgaac cttgccattc catgttactt 360
ggtgctgcgt gtccacattc gacgccacat ttttttcttg ctagccaacc attgcgcgtc 420
tgtgttacag ttccaccggc gatatacatt atgcgatcat ttgatgtgga tattggtgtt 480
tttttggtta gcggatcacc ttagttttgc actgaacatg gaacaaatgt ggatgttggt 540
gtttttttgt tagtggatca ccttagtttt gcactgaaga tggaacgagg aaatggggaa 600
ggggaactga acgaagcaca tagtttcaga gactatgtgc actgcagaga ctatgcgaat 660
ggtcaaggga atttctcgcc ttggtttcct aatcctcgct tccttcttgt ttcgaattcg 720
gtgtatatat gtggtttatt cggcacgttt aggaatttag ttggggaatg gggcgcagta 780
tgtcagggtt gtgtcatctg aatatataat gcaagatcta ttactagttt aaattgactt 840
attatgcaag tcgattaacc tggctgggct cagtggtgta ccatggaagc tttgtgattg 900
tagccgggca gtagaacgct aaacctgagg tttggtactg tagcactcgg ctagtcagct 960
gtatgccaag atgttactta ctcgatactt tctgccatgt ctcatctgtg ttaaccaggg 1020
tgtgtgccac atagggcttc tttccatgct gtttgttaac ctgttcacac ttcttccata 1080
tatatattca taatagctcg atctacaatt ttcctgtctt gagcttcttt gttcctaaaa 1140
acgaattgta taccagcaat gtatatctga tgcaatattg tttgtaggcg tacggcctag 1200
gccttcacct gcggagggta agatccgatc accatcttct gaatttctgt tcttgatctg 1260
tcatgtataa taactgtcta gtcttggtgt tggtgagatg gaaattcggt ggatctcgga 1320
agggatattg ttcgtttgct ggggtttttt ttgtgtgttg tgatccgtag agaatttgtg 1380
tttatccatg ttgttgatct tggtatgtat tcatgacata ttgacatgca tgtgttgtat 1440
gtgtcatatg tgtgcctctc cttgggattt gttttggata atagaacatg ttatggactc 1500
aatagtctgt gaacaaatct ttttttagat ggtggccaaa tctgatgatg atctttcttg 1560
agaggaaaaa gttcatgata gaaaaatctt ttttgagatg gtggcttaat gtgatgatga 1620
tctttcttga gaggaaaaaa aagattcatt ataggagatt ttgatttagc tcctttccac 1680
cgatattaaa tgaggagcat gcatgctgat tgctgataag gatctgattt ttttatcccc 1740
tcttctttga acagacaaga aataggctct gaatttctga ttgattattt gtacatgcag 1800
aagggcgaat tcgacctagg ccaagtttgt acaaaaaagc aggcttgata accaaccatg 1860
gtccgtcctg tagaaacccc aacccgtgaa atcaaaaaac tcgacggcct gtgggcattc 1920
agtctggatc gcgaaaactg tggaattgat cagcgttggt gggaaagcgc gttacaagaa 1980

agccgggcaa ttgctgtgcc aggcagtttt aacgatcagt tcgccgatgc agatattcgt 2040
aattatgcgg gcaacgtctg gtatcagcgc gaagtcttta taccgaaagg ttgggcaggc 2100
cagcgtatcg tgctgcgttt cgatgcggtc actcattacg gcaaagtgtg ggtcaataat 2160
caggaagtga tggagcatca gggcggctat acgccatttg aagccgatgt cacgccgtat 2220
gttattgccg ggaaaagtgt acgtaagttt ctgcttctac ctttgatata tatataataa 2280
ttatcattaa ttagtagtaa tataatattt caaatatttt tttcaaaata aaagaatgta 2340
gtatatagca attgcttttc tgtagtttat aagtgtgtat attttaattt ataactttttc 2400
taatatatga ccaaaatttg ttgatgtgca ggtatcaccg tttgtgtgaa caacgaactg 2460
aactggcaga ctatcccgcc gggaatggtg attaccgacg aaaacggcaa gaaaaagcag 2520
tcttacttcc atgatttctt taactatgcc ggaatccatc gcagcgtaat gctctacacc 2580
acgccgaaca cctgggtgga cgatatcacc gtggtgacgc atgtcgcgca agactgtaac 2640
cacgcgtctg ttgactggca ggtggtggcc aatggtgatg tcagcgttga actgcgtgat 2700
gcggatcaac aggtggttgc aactggacaa ggcactagcg ggactttgca agtggtgaat 2760
ccgcacctct ggcaaccggg tgaaggttat ctctatgaac tgtgcgtcac agccaaaagc 2820
cagacagagt gtgatatcta cccgcttcgc gtcggcatcc ggtcagtggc agtgaagggc 2880
gaacagttcc tgattaacca caaaccgttc tactttactg gctttggtcg tcatgaagat 2940
gcggacttgc gtggcaaagg attcgataac gtgctgatgg tgcacgacca cgcattaatg 3000
gactggattg gggccaactc ctaccgtacc tcgcattacc cttacgctga agagatgctc 3060
gactgggcag atgaacatgg catcgtggtg attgatgaaa ctgctgctgt cggctttaac 3120
ctctctttag gcattggttt cgaagcgggc aacaagccga aagaactgta cagcgaagag 3180
gcagtcaacg gggaaactca gcaagcgcac ttacaggcga ttaaagagct gatagcgcgt 3240
gacaaaaacc acccaagcgt ggtgatgtgg agtattgcca acgaaccgga tacccgtccg 3300
caaggtgcac gggaatattt cgcgccactg gcggaagcaa cgcgtaaact cgacccgacg 3360
cgtccgatca cctgcgtcaa tgtaatgttc tgcgacgctc acaccgatac catcagcgat 3420
ctctttgatg tgctgtgcct gaaccgttat tacggatggt atgtccaaag cggcgatttg 3480
gaaacggcag agaaggtact ggaaaaagaa cttctggcct ggcaggagaa actgcatcag 3540
ccgattatca tcaccgaata cggcgtggat acgttagccg ggctgcactc aatgtacacc 3600
gacatgtgga gtgaagagta tcagtgtgca tggctggata tgtatcaccg cgtctttgat 3660
cgcgtcagcg ccgtcgtcgg tgaacaggta tggaatttcg ccgattttgc gacctcgcaa 3720
ggcatattgc gcgttggcgg taacaagaaa gggatcttca ctcgcgaccg caaaccgaag 3780
tcggcggctt ttctgctgca aaaacgctgg actggcatga acttcggtga aaaaccgcag 3840
cagggaggca aacaatgaat caaacccagc tttcttgtac aaagtgggag ctcgatcgtt 3900
caaacatttg gcaataaagt ttcttaagat tgaatcctgt tgccggtctt gcgatgatta 3960
tcatataatt tctgttgaat tacgttaagc atgtaataat taacatgtaa tgcatgacgt 4020
tatttatgag atgggttttt atgattagag tcccgcaatt atacatttaa tacgcgatag 4080
aaaacaaaat atagcgcgca aactaggata aattatcgcg cgcggtgtca tctatgttac 4140
tagatc 4146

<210> 63

<211> 4510

<212> DNA

<213> Artificial

<220>
<223> Vector
<400> 63
ctacatttat gttatagagg cgcaagtaga agaatgtgtt ataagttgta catcggaaaa 60
atagcatgta aatctataga atcaattttc atctttcacc ccatgaattt gaggtatgct 120
tatatgataa ctttagaaag tggtggaatg tcatattcta aaaaaaatag cctatttcat 180
tagtaagatt ccaattcctc gaaatgaaag aaaacaaacg gggccttatt agaatggaat 240
tcaattccaa tgatccaaat ggggcgtaag ggatttccac tttcctaaga aaaattagct 300
cattttccct tggtgtttaa taattatgtt ttgttaaaag gtcagcttcc cgagaatgcg 360
ttgtgtaaaa gcccagtata agggtctgtt tggttgggct gtggctgtga aaaaagttgc 420
tgtgggctgt gagctgtgga aaaagctgct gtagactgta agctgttaaa aagctaaaaa 480
ccgtttggtg gaaaccacta aaagtcgtta aaaaatcttc gatatatgtt ttcacagttc 540
catccgaaaa gccactaaaa gcaggtccag aggtgctttc agatttgcac tacgagaaag 600
tcggttttta gaaaaagctg cttcctggat ccagcccttt ggttggcttt tggcttttag 660
gggcacaaaa gccaaagcca aaagtcaaac caaacacacc ctaagtcgcg tgagtgggcc 720
atttatctat tccctatgca aatctcctga aaaattacaa cattagctcg ttaaaaagta 780
aaataatttt aaaaaattag agactaatta tttgataact ttatagttca tcacattttc 840
attgaaaatg ttttattttt tttgccgaac tgatctacac aatatttgaa tctacacttt 900
tggcttgggc tcggagaaaa atagtgcgtg cccagccctt tgctctttcc ccgataacaa 960
cgcggtatgt ggcctaacgc ccaaatccgg cccatttatt ccggtgcgta gatgcagacg 1020
cgggagcagg tgccaagtca ctggctcact gctgcaggtg gagtggtgga cactagtgcc 1080
gcggttcatc tgacacgtgt cgccacgtgc cgccatggca gcacctcagc ccggccggcg 1140
ggccgactga cgtcttgggc aaagcggcga gcgacgcagg cggcgaaagc catccgattt 1200
gacccctcgc tagacctttc aagaacgaac gctgtgctgc tcagatcaga ccgtgtctgc 1260
ctcaaagcga tgccaggacg ccacgtccaa gcaaagcacc cgatgccatt gccacctccc 1320
agcactcacg cgtgagcgtg actataaaaa acgcaccctc tgcatccgcc cccgtctgcc 1380
tgccctaccg aatctttcgc cgtcccatca gcccagcaat tcttcgctgt tcgaggaccc 1440
ctcggtttcg accgaagccc agcaagccga ccacacaccg ctgccgttgg ttccgtccca 1500
agagatgcgg ccgcactaag cgctatttaa atgccagctg tacactagtt atcgtacggc 1560
ctaggccttc acctgcggag ggtaagatcc gatcaccatc ttctgaattt ctgttcttga 1620
tctgtcatgt ataataactg tctagtcttg gtgttggtga gatggaaatt cggtggatct 1680
cggaagggat attgttcgtt tgctggggtt ttttttgtgt gttgtgatcc gtagagaatt 1740
tgtgtttatc catgttgttg atcttggtat gtattcatga catattgaca tgcatgtgtt 1800
gtatgtgtca tatgtgtgcc tctccttggg atttgttttg gataatagaa catgttatgg 1860
actcaatagt ctgtgaacaa atcttttttt agatggtggc caaatctgat gatgatcttt 1920
cttgagagga aaaagttcat gatagaaaaa tctttttttga gatggtggct taatgtgatg 1980
atgatctttc ttgagaggaa aaaaaagatt cattatagga gattttgatt tagctccttt 2040
ccaccgatat taaatgagga gcatgcatgc tgattgctga taaggatctg atttttttat 2100
cccctcttct ttgaacagac aagaaatagg ctctgaattt ctgattgatt atttgtacat 2160
gcagaagggc gaattcgacc taggccaagt ttgtacaaaa aagcaggctt gataaccaac 2220
catggtccgt cctgtagaaa ccccaacccg tgaaatcaaa aaactcgacg gcctgtgggc 2280

attcagtctg gatcgcgaaa actgtggaat tgatcagcgt tggtgggaaa gcgcgttaca 2340
agaaagccgg gcaattgctg tgccaggcag ttttaacgat cagttcgccg atgcagatat 2400
tcgtaattat gcgggcaacg tctggtatca gcgcgaagtc tttataccga aaggttgggc 2460
aggccagcgt atcgtgctgc gtttcgatgc ggtcactcat tacggcaaag tgtgggtcaa 2520
taatcaggaa gtgatggagc atcagggcgg ctatacgcca tttgaagccg atgtcacgcc 2580
gtatgttatt gccgggaaaa gtgtacgtaa gtttctgctt ctacctttga tatatatata 2640
ataattatca ttaattagta gtaatataat atttcaaata ttttttcaa aataaaagaa 2700
tgtagtatat agcaattgct tttctgtagt ttataagtgt gtatatttta atttataact 2760
tttctaatat atgaccaaaa tttgttgatg tgcaggtatc accgtttgtg tgaacaacga 2820
actgaactgg cagactatcc cgccgggaat ggtgattacc gacgaaaacg gcaagaaaaa 2880
gcagtcttac ttccatgatt tctttaacta tgccggaatc catcgcagcg taatgctcta 2940
caccacgccg aacacctggg tggacgatat caccgtggtg acgcatgtcg cgcaagactg 3000
taaccacgcg tctgttgact ggcaggtggt ggccaatggt gatgtcagcg ttgaactgcg 3060
tgatgcggat caacaggtgg ttgcaactgg acaaggcact agcgggactt tgcaagtggt 3120
gaatccgcac ctctggcaac cgggtgaagg ttatctctat gaactgtgcg tcacagccaa 3180
aagccagaca gagtgtgata tctacccgct tcgcgtcggc atccggtcag tggcagtgaa 3240
gggcgaacag ttcctgatta accacaaacc gttctacttt actggctttg gtcgtcatga 3300
agatgcggac ttgcgtggca aaggattcga taacgtgctg atggtgcacg accacgcatt 3360
aatggactgg attggggcca actcctaccg tacctcgcat tacccttacg ctgaagagat 3420
gctcgactgg gcagatgaac atggcatcgt ggtgattgat gaaactgctg ctgtcggctt 3480
taacctctct ttaggcattg gtttcgaagc gggcaacaag ccgaaagaac tgtacagcga 3540
agaggcagtc aacggggaaa ctcagcaagc gcacttacag gcgattaaag agctgatagc 3600
gcgtgacaaa aaccacccaa gcgtggtgat gtggagtatt gccaacgaac cggatacccg 3660
tccgcaaggt gcacgggaat atttcgcgcc actggcggaa gcaacgcgta aactcgaccc 3720
gacgcgtccg atcacctgcg tcaatgtaat gttctgcgac gctcacaccg ataccatcag 3780
cgatctcttt gatgtgctgt gcctgaaccg ttattacgga tggtatgtcc aaagcggcga 3840
tttggaaacg gcagagaagg tactggaaaa agaacttctg gcctggcagg agaaactgca 3900
tcagccgatt atcatcaccg aatacggcgt ggatacgtta gccgggctgc actcaatgta 3960
caccgacatg tggagtgaag agtatcagtg tgcatggctg gatatgtatc accgcgtctt 4020
tgatcgcgtc agcgccgtcg tcggtgaaca ggtatggaat ttcgccgatt ttgcgacctc 4080
gcaaggcata ttgcgcgttg gcggtaacaa gaaagggatc ttcactcgcg accgcaaacc 4140
gaagtcggcg gcttttctgc tgcaaaaacg ctggactggc atgaacttcg gtgaaaaacc 4200
gcagcaggga ggcaaacaat gaatcaaacc cagctttctt gtacaaagtg ggagctcgat 4260
cgttcaaaca tttggcaata aagtttctta agattgaatc ctgttgccgg tcttgcgatg 4320
attatcatat aatttctgtt gaattacgtt aagcatgtaa taattaacat gtaatgcatg 4380
acgttattta tgagatgggt ttttatgatt agagtcccgc aattatacat ttaatacgcg 4440
atagaaaaca aaatatagcg cgcaaactag gataaattat cgcgcgcggt gtcatctatg 4500
ttactagatc 4510

<210> 64

<211> 3880

<212> DNA

<213> Artificial

<220>

<223> Vector

<400> 64

catacgattt cctaagcgga atctcgagac gaaccacatt tttcatccgg tctatgagcg 60
cgctcaatcg tcagtgacag agaacactgt aggtccttaa ctggaactaa caattcagaa 120
acaatcctaa atgtttatta cactggatgg attttatttg gatagatgat actaccgttc 180
gtgggctaaa aagcaaggta aaaactggcc taacgcacgt gatcctccca tggtctcaga 240
agaaaaccac tcgcaaaaaa aaaaacgcag caatgaggct gctttcagtt gacggtgtgc 300
acgactcgct gaagccagcg tgattttgtc ccggttcttg gagcccagca aaaagggcgc 360
ctgaaattcc acaggttgaa ggggaaaacg gcaacaaaag ggctccgac atgcggtgca 420
gaaagtgaaa acgcgagaaa agcgcccgtc ctttctcgtt cccttccgtg cagttgcaag 480
tgttgccacg ccgcgccgcg acacaccacg caggaggcag cgagcgcaat aatgcgcacg 540
cagaggagca cagcggcgtc actgctgcgg cagtaatggc cgcctggttc cacttccacc 600
acggtgtgca ctagcgctac accgaggcca ccttttcttg gttccttcaa cacgcctact 660
ccggcctccg gatcgggcac cacgtctgcc ccctgctccc ggaatctttg ccaagcggcg 720
ccgctactgt ccggcaaggc gaccccgtct ctcggccggc gttgatctgg tgatgcgctt 780
ttcacggtgt tttttttttt ttttgtttca ctcccccctc cgtcctgtgt gattgtgctg 840
cagttttgtg ccggtgctaa cggaaaccgg aaagtttgcg tctttatgtt actgtggttg 900
aggcgggcgg agtactagtt atcgtacggc ctaggccttc acctgcggag ggtaagatcc 960
gatcaccatc ttctgaattt ctgttcttga tctgtcatgt ataataactg tctagtcttg 1020
gtgttggtga gatggaaatt cggtggatct cggaagggat attgttcgtt tgctggggtt 1080
tttttgtgt gttgtgatcc gtagagaatt tgtgtttatc catgttgttg atcttggtat 1140
gtattcatga catattgaca tgcatgtgtt gtatgtgtca tatgtgtgcc tctccttggg 1200
atttgttttg gataatagaa catgttatgg actcaatagt ctgtgaacaa atcttttttt 1260
agatggtggc caaatctgat gatgatcttt cttgagagga aaaagttcat gatagaaaaa 1320
tctttttga gatggtggct taatgtgatg atgatctttc ttgagaggaa aaaaaagatt 1380
cattatagga gattttgatt tagctccttt ccaccgatat taaatgagga gcatgcatgc 1440
tgattgctga taaggatctg atttttttat cccctcttct ttgaacagac aagaaatagg 1500
ctctgaattt ctgattgatt atttgtacat gcagaagggc gaattcgacc taggccaagt 1560
ttgtacaaaa aagcaggctt gataaccaac catggtccgt cctgtagaaa ccccaacccg 1620
tgaaatcaaa aaactcgacg gcctgtgggc attcagtctg gatcgcgaaa actgtggaat 1680
tgatcagcgt tggtgggaaa gcgcgttaca agaaagccgg gcaattgctg tgccaggcag 1740
ttttaacgat cagttcgccg atgcagatat tcgtaattat gcgggcaacg tctggtatca 1800
gcgcgaagtc tttataccga aaggttgggc aggccagcgt atcgtgctgc gtttcgatgc 1860
ggtcactcat tacggcaaag tgtgggtcaa taatcaggaa gtgatggagc atcagggcgg 1920
ctatacgcca tttgaagccg atgtcacgcc gtatgttatt gccgggaaaa gtgtacgtaa 1980
gtttctgctt ctacctttga tatatatata ataattatca ttaattagta gtaatataat 2040
atttcaaata tttttttcaa aataaaagaa tgtagtatat agcaattgct tttctgtagt 2100
ttataagtgt gtatatttta atttataact tttctaatat atgaccaaaa tttgttgatg 2160
tgcaggtatc accgtttgtg tgaacaacga actgaactgg cagactatcc cgcccgggaat 2220

ggtgattacc gacgaaaacg gcaagaaaaa gcagtcttac ttccatgatt tctttaacta 2280
tgccggaatc catcgcagcg taatgctcta caccacgccg aacacctggg tggacgatat 2340
caccgtggtg acgcatgtcg cgcaagactg taaccacgcg tctgttgact ggcaggtggt 2400
ggccaatggt gatgtcagcg ttgaactgcg tgatgcggat caacaggtgg ttgcaactgg 2460
acaaggcact agcgggactt tgcaagtggt gaatccgcac ctctggcaac cgggtgaagg 2520
ttatctctat gaactgtgcg tcacagccaa aagccagaca gagtgtgata tctacccgct 2580
tcgcgtcggc atccggtcag tggcagtgaa gggcgaacag ttcctgatta accacaaacc 2640
gttctacttt actggctttg gtcgtcatga agatgcggac ttgcgtggca aaggattcga 2700
taacgtgctg atggtgcacg accacgcatt aatggactgg attggggcca actcctaccg 2760
tacctcgcat tacccttacg ctgaagagat gctcgactgg gcagatgaac atggcatcgt 2820
ggtgattgat gaaactgctg ctgtcggctt taacctctct ttaggcattg gtttcgaagc 2880
gggcaacaag ccgaaagaac tgtacagcga agaggcagtc aacggggaaa ctcagcaagc 2940
gcacttacag gcgattaaag agctgatagc gcgtgacaaa aaccacccaa gcgtggtgat 3000
gtggagtatt gccaacgaac cggatacccg tccgcaaggt gcacgggaat atttcgcgcc 3060
actggcggaa gcaacgcgta aactcgaccc gacgcgtccg atcacctgcg tcaatgtaat 3120
gttctgcgac gctcacaccg ataccatcag cgatctcttt gatgtgctgt gcctgaaccg 3180
ttattacgga tggtatgtcc aaagcggcga tttggaaacg gcagagaagg tactggaaaa 3240
agaacttctg gcctggcagg agaaactgca tcagccgatt atcatcaccg aatacggcgt 3300
ggatacgtta gccgggctgc actcaatgta caccgacatg tggagtgaag agtatcagtg 3360
tgcatggctg gatatgtatc accgcgtctt tgatcgcgtc agcgccgtcg tcggtgaaca 3420
ggtatggaat ttcgccgatt ttgcgacctc gcaaggcata ttgcgcgttg gcggtaacaa 3480
gaaagggatc ttcactcgcg accgcaaacc gaagtcggcg gcttttctgc tgcaaaaacg 3540
ctggactggc atgaacttcg gtgaaaaacc gcagcaggga ggcaaacaat gaatcaaacc 3600
cagctttctt gtacaaagtg ggagctcgat cgttcaaaca tttggcaata aagtttctta 3660
agattgaatc ctgttgccgg tcttgcgatg attatcatat aatttctgtt gaattacgtt 3720
aagcatgtaa taattaacat gtaatgcatg acgttattta tgagatgggt ttttatgatt 3780
agagtcccgc aattatacat ttaatacgcg atagaaaaca aaatatagcg cgcaaactag 3840
gataaattat cgcgcgcggt gtcatctatg ttactagatc 3880

<210> 65
<211> 4135
<212> DNA
<213> Artificial
<220>
<223> Vector
<400> 65

ccagccatcg tgcttgagtg aatgtttttt tgcgtgtgtg taggtgtgcg tgtgtgttta 60
attgatataa aataacccaa ccaaacatct cctaagattt ccataagaca gagaggtgag 120
gaatagtatt agttctcaaa tgaattcaat caactaaata tcaccatcat atcaacatta 180
ataatagaac aataaaattt tcatgataga gaggctggta aactttattt tttcatttga 240
ttccagtgtg gaaaatagta atataagcag taaatttttt cttaaaaaag agtggaatat 300
tcaccttttt atataaatta gttattcttc gaagcgtcac acacggatgt cctaaagata 360

ttcttcaact gcaacacaag cattctgaaa aggttagtaa caatcagatt aatgttgtaa 420
aaatatgtga cacgaaaatt taaagaacgc gaagtacaat acattggtaa gaaatcaaag 480
acacatatta accaactatt agagaacata tacatgtttc ttctaatcct ggttaggaca 540
taggacgagg gtttcacttc tatcacaatg tgctgcaact ttcagagtta atcaaatctg 600
ttattcagtg ttatgcagcg acaaattcca aatgtataat cccacgatga ttcatgtgac 660
tccatttcgt ctgtaattga tgcgaaactg ccgaattgtt actgctccca taagttaatt 720
gtaccacgct tgatgaagac gttcacgtat cttgtgttcg atttatgtag ccgttattca 780
gaccattgca gcgctgagct tcaaggcaat tagttttgcc ccgcgatgtg aggcaggtga 840
tgacacgcgc ccttcgggcc gatgccgacg cgccaccgtg cagactctac cgaggatgag 900
agcagcgatc gagagacgtg gagcgaggag gagactttag gagaggagag accagagcct 960
taagcggtga cacacccaat gatcaggacc acgcttgcac gtgtcaggtg cctctcctcc 1020
acgcgtcgcg cgcatcacac tgatactgtc atccagctcg acgctatata aagtggtaag 1080
catcttcgtc ctcggggccg ccccaacggc ccttgcgatc gccaccacgt cgcggccgca 1140
ctaagcgcta tttaaatgcc agctgtacac tagttatcgt acggcctagg ccttcacctg 1200
cggagggtaa gatccgatca ccatcttctg aatttctgtt cttgatctgt catgtataat 1260
aactgtctag tcttggtgtt ggtgagatgg aaattcggtg gatctcggaa gggatattgt 1320
tcgtttgctg ggtttttttt tgtgtgttgt gatccgtaga gaatttgtgt ttatccatgt 1380
tgttgatctt ggtatgtatt catgacatat tgacatgcat gtgttgtatg tgtcatatgt 1440
gtgcctctcc ttgggatttg ttttggataa tagaacatgt tatggactca atagtctgtg 1500
aacaaatctt tttttagatg gtggccaaat ctgatgatga tctttcttga gaggaaaaag 1560
ttcatgatag aaaaatcttt tttgagatgg tggcttaatg tgatgatgat ctttcttgag 1620
aggaaaaaaa agattcatta taggagattt tgatttagct cctttccacc gatattaaat 1680
gaggagcatg catgctgatt gctgataagg atctgatttt tttatcccct cttctttgaa 1740
cagacaagaa ataggctctg aatttctgat tgattatttg tacatgcaga agggcgaatt 1800
cgacctaggc caagtttgta caaaaaagca ggcttgataa ccaaccatgg tccgtcctgt 1860
agaaacccca acccgtgaaa tcaaaaaact cgacggcctg tgggcattca gtctggatcg 1920
cgaaaactgt ggaattgatc agcgttggtg ggaaagcgcg ttacaagaaa gccgggcaat 1980
tgctgtgcca ggcagtttta acgatcagtt cgccgatgca gatattcgta attatgcggg 2040
caacgtctgg tatcagcgcg aagtctttat accgaaaggt tgggcaggcc agcgtatcgt 2100
gctgcgtttc gatgcggtca ctcattacgg caaagtgtgg gtcaataatc aggaagtgat 2160
ggagcatcag ggcggctata cgccatttga agccgatgtc acgccgtatg ttattgccgg 2220
gaaaagtgta cgtaagtttc tgcttctacc tttgatatat atataataat tatcattaat 2280
tagtagtaat ataatatttc aaatattttt ttcaaaataa aagaatgtag tatatagcaa 2340
ttgcttttct gtagtttata agtgtgtata ttttaattta taacttttct aatatatgac 2400
caaaatttgt tgatgtgcag gtatcaccgt ttgtgtgaac aacgaactga actggcagac 2460
tatcccgccg ggaatggtga ttaccgacga aaacggcaag aaaaagcagt cttacttcca 2520
tgatttcttt aactatgccg gaatccatcg cagcgtaatg ctctacacca cgccgaacac 2580
ctgggtggac gatatcaccg tggtgacgca tgtcgcgcaa gactgtaacc acgcgtctgt 2640
tgactggcag gtggtggcca atggtgatgt cagcgttgaa ctgcgtgatg cggatcaaca 2700
ggtggttgca actggacaag gcactagcgg gactttgcaa gtggtgaatc cgcacctctg 2760
gcaaccgggt gaaggttatc tctatgaact gtgcgtcaca gccaaaagcc agacagagtg 2820

tgatatctac ccgcttcgcg tcggcatccg gtcagtggca gtgaagggcg aacagttcct 2880
gattaaccac aaaccgttct actttactgg ctttggtcgt catgaagatg cggacttgcg 2940
tggcaaagga ttcgataacg tgctgatggt gcacgaccac gcattaatgg actggattgg 3000
ggccaactcc taccgtacct cgcattaccc ttacgctgaa gagatgctcg actgggcaga 3060
tgaacatggc atcgtggtga ttgatgaaac tgctgctgtc ggctttaacc tctctttagg 3120
cattggtttc gaagcgggca acaagccgaa agaactgtac agcgaagagg cagtcaacgg 3180
ggaaactcag caagcgcact tacaggcgat taaagagctg atagcgcgtg acaaaaacca 3240
cccaagcgtg gtgatgtgga gtattgccaa cgaaccggat acccgtccgc aaggtgcacg 3300
ggaatatttc gcgccactgg cggaagcaac gcgtaaactc gacccgacgc gtccgatcac 3360
ctgcgtcaat gtaatgttct gcgacgctca caccgatacc atcagcgatc tctttgatgt 3420
gctgtgcctg aaccgttatt acggatggta tgtccaaagc ggcgatttgg aaacggcaga 3480
gaaggtactg gaaaaagaac ttctggcctg gcaggagaaa ctgcatcagc cgattatcat 3540
caccgaatac ggcgtggata cgttagccgg gctgcactca atgtacaccg acatgtggag 3600
tgaagagtat cagtgtgcat ggctggatat gtatcaccgc gtctttgatc gcgtcagcgc 3660
cgtcgtcggt gaacaggtat ggaatttcgc cgattttgcg acctcgcaag gcatattgcg 3720
cgttggcggt aacaagaaag ggatcttcac tcgcgaccgc aaaccgaagt cggcggcttt 3780
tctgctgcaa aaacgctgga ctggcatgaa cttcggtgaa aaaccgcagc agggaggcaa 3840
acaatgaatc aaacccagct ttcttgtaca aagtgggagc tcgatcgttc aaacatttgg 3900
caataaagtt tcttaagatt gaatcctgtt gccggtcttg cgatgattat catataattt 3960
ctgttgaatt acgttaagca tgtaataatt aacatgtaat gcatgacgtt atttatgaga 4020
tgggttttta tgattagagt cccgcaatta tacatttaat acgcgataga aaacaaaata 4080
tagcgcgcaa actaggataa attatcgcgc gcggtgtcat ctatgttact agatc 4135

<210> 66

<211> 3530

<212> DNA

<213> Artificial

<220>

<223> Vector

<400> 66

cattgttata catcggtgat gcagatcttc gcttcgcatg ggtggcaatt tagtaacaga 60
attgctctat gagttattgc ttttagtttc tgtctttccc caactgcgtt tgagtttgca 120
ttgtcacatg tggccttttt tggttgtaag acttgtttga tcggttgtgg tgggctgtca 180
tcctttggat gtaggccgga acctcttttc ccattatcta aaaaaataca tcgtcatttc 240
cagtgagacc cagcggtcgt gtcatcagaa aagcggacac gcgtcgcgtc gcgaggccag 300
gtgggttatg cacggacctg ctatgctagc catgctccag gctccagctc cacccgtcca 360
ccgacttttc ttctcaacca ggctgcctcc tcaccatgca cgacgacacg tgcgatgcca 420
tcacgcatcc acactcacct gttgcctcta tatccaccgc cccaccgccg tagcaccaga 480
aagaaatgta cagtctacac agctgaccag agagctagtg caagcgatct agcaagtttt 540
aatcttggaa gaagccagct aggactagtt atcgtacggc ctaggccttc acctgcggag 600
ggtaagatcc gatcaccatc ttctgaattt ctgttcttga tctgtcatgt ataataactg 660
tctagtcttg gtgttggtga gatggaaatt cggtggatct cggaagggat attgttcgtt 720

tgctggggtt ttttttgtgt gttgtgatcc gtagagaatt tgtgtttatc catgttgttg 780
atcttggtat gtattcatga catattgaca tgcatgtgtt gtatgtgtca tatgtgtgcc 840
tctccttggg atttgttttg gataatagaa catgttatgg actcaatagt ctgtgaacaa 900
atcttttttt agatggtggc caaatctgat gatgatcttt cttgagagga aaaagttcat 960
gatagaaaaa tctttttga gatggtggct taatgtgatg atgatctttc ttgagaggaa 1020
aaaaaagatt cattatagga gattttgatt tagctccttt ccaccgatat taaatgagga 1080
gcatgcatgc tgattgctga taaggatctg attttttat cccctcttct ttgaacagac 1140
aagaaatagg ctctgaattt ctgattgatt atttgtacat gcagaagggc gaattcgacc 1200
taggccaagt ttgtacaaaa aagcaggctt gataaccaac catggtccgt cctgtagaaa 1260
ccccaacccg tgaaatcaaa aaactcgacg gcctgtgggc attcagtctg gatcgcgaaa 1320
actgtggaat tgatcagcgt tggtgggaaa gcgcgttaca agaaagccgg gcaattgctg 1380
tgccaggcag ttttaacgat cagttcgccg atgcagatat tcgtaattat gcgggcaacg 1440
tctggtatca gcgcgaagtc tttataccga aaggttgggc aggccagcgt atcgtgctgc 1500
gtttcgatgc ggtcactcat tacggcaaag tgtgggtcaa taatcaggaa gtgatggagc 1560
atcagggcgg ctatacgcca tttgaagccg atgtcacgcc gtatgttatt gccgggaaaa 1620
gtgtacgtaa gtttctgctt ctacctttga tatatatata ataattatca ttaattagta 1680
gtaatataat atttcaaata tttttttcaa aataaaagaa tgtagtatat agcaattgct 1740
tttctgtagt ttataagtgt gtatatttta atttataact tttctaatat atgaccaaaa 1800
tttgttgatg tgcaggtatc accgtttgtg tgaacaacga actgaactgg cagactatcc 1860
cgccgggaat ggtgattacc gacgaaaacg gcaagaaaaa gcagtcttac ttccatgatt 1920
tctttaacta tgccggaatc catcgcagcg taatgctcta caccacgccg aacacctggg 1980
tggacgatat caccgtggtg acgcatgtcg cgcaagactg taaccacgcg tctgttgact 2040
ggcaggtggt ggccaatggt gatgtcagcg ttgaactgcg tgatgcggat caacaggtgg 2100
ttgcaactgg acaaggcact agcgggactt tgcaagtggt gaatccgcac ctctggcaac 2160
cgggtgaagg ttatctctat gaactgtgcg tcacagccaa aagccagaca gagtgtgata 2220
tctacccgct tcgcgtcggc atccggtcag tggcagtgaa gggcgaacag ttcctgatta 2280
accacaaacc gttctacttt actggctttg gtcgtcatga agatgcggac ttgcgtggca 2340
aaggattcga taacgtgctg atggtgcacg accacgcatt aatggactgg attggggcca 2400
actcctaccg tacctcgcat tacccttacg ctgaagagat gctcgactgg gcagatgaac 2460
atggcatcgt ggtgattgat gaaactgctg ctgtcggctt taacctctct ttaggcattg 2520
gtttcgaagc gggcaacaag ccgaaagaac tgtacagcga agaggcagtc aacggggaaa 2580
ctcagcaagc gcacttacag gcgattaaag agctgatagc gcgtgacaaa aaccacccaa 2640
gcgtggtgat gtggagtatt gccaacgaac cggatacccg tccgcaaggt gcacgggaat 2700
atttcgcgcc actggcggaa gcaacgcgta aactcgaccc gacgcgtccg atcacctgcg 2760
tcaatgtaat gttctgcgac gctcacaccg ataccatcag cgatctcttt gatgtgctgt 2820
gcctgaaccg ttattacgga tggtatgtcc aaagcggcga tttggaaacg gcagagaagg 2880
tactggaaaa agaacttctg gcctggcagg agaaactgca tcagccgatt atcatcaccg 2940
aatacggcgt ggatacgtta gccgggctgc actcaatgta caccgacatg tggagtgaag 3000
agtatcagtg tgcatggctg gatatgtatc accgcgtctt tgatcgcgtc agcgccgtcg 3060
tcggtgaaca ggtatggaat ttcgccgatt ttgcgacctc gcaaggcata ttgcgcgttg 3120
gcggtaacaa gaaagggatc ttcactcgcg accgcaaacc gaagtcggcg gcttttctgc 3180

tgcaaaaacg ctggactggc atgaacttcg gtgaaaaacc gcagcaggga ggcaaacaat 3240
gaatcaaacc cagctttctt gtacaaagtg ggagctcgat cgttcaaaca tttggcaata 3300
aagtttctta agattgaatc ctgttgccgg tcttgcgatg attatcatat aatttctgtt 3360
gaattacgtt aagcatgtaa taattaacat gtaatgcatg acgttattta tgagatgggt 3420
ttttatgatt agagtcccgc aattatacat ttaatacgcg atagaaaaca aaatatagcg 3480
cgcaaactag gataaattat cgcgcgcggt gtcatctatg ttactagatc 3530

<210> 67
<211> 3995
<212> DNA
<213> Artificial
<220>
<223> Vector
<400> 67

atcatcaccc taccccgagc tgactcgggc cgcagggaac cagaccggtg tcccatctgg 60
ctagctccgc cagataggca atgaaggcgc cccgcatgct ctgtgacgac ggcggctctc 120
agccccctta cggaagcaag aggacgtcag caaggaccca accgctccga cagctgtccc 180
tccgccaggc tccatcgctc ctccgacggc cacgacatcg caccagctgg gtgccaaaat 240
ctctccggct gccacgacgg catgtactta gggcgctagc tctcccccgc tagacacgta 300
gcactctgct acacccccca ttgtacacct ggatcctctc cttacaccta taaaaggaag 360
gaccagggcc ctctcagaga aggttggccg cgcggggacg aggacgagac aggcgctcgc 420
ttggggccgc tcgctccctc tcccacgtgg acgcttgtaa ccccctactg caagcgcacc 480
cgacctgggc gcgggacgaa cacgaaggcc gcgggattcc cacctctctc acgccggtct 540
ccggccgcct cgctctcccc ccttcgcgct cgccctcgcg ctcgatccat ctgggctggg 600
gcacgcggcg acactcactc gtcggcccaa gggacccccg gtctcgaaac gccgacaata 660
tgatatatag aaactataat gatacacatg caattaacta gtgctgataa tatatttaca 720
ctgtagattt ttccgataag tagaaaccaa atatttcacc gccacaaaag gcttcatccc 780
atcagttacc catgtagcaa aaccaaaata cttgtgtgcg agaggccaag agcccaaacc 840
accgccaagg aaacccccag cccaccaggc gccttcctct ataaataccc taactccggt 900
ggccgaagtt cctcacccat tcactcactc atctcaaggc ctaggtagca ccgtagcagc 960
tactagaagc agctagccag aacaactcgt cgcggccgca ctaagcgcta tttaaatgcc 1020
agctgtacac tagttatcgt acggcctagg ccttcacctg cggagggtaa gatccgatca 1080
ccatcttctg aatttctgtt cttgatctgt catgtataat aactgtctag tcttggtgtt 1140
ggtgagatgg aaattcggtg gatctcggaa gggatattgt tcgtttgctg ggtttttttt 1200
tgtgtgttgt gatccgtaga gaatttgtgt ttatccatgt tgttgatctt ggtatgtatt 1260
catgacatat tgacatgcat gtgttgtatg tgtcatatgt gtgcctctcc ttgggatttg 1320
ttttggataa tagaacatgt tatggactca atagtctgtg aacaaatctt tttttagatg 1380
gtggccaaat ctgatgatga tctttcttga gaggaaaaag ttcatgatag aaaaatcttt 1440
tttgagatgg tggcttaatg tgatgatgat ctttcttgag aggaaaaaaa agattcatta 1500
taggagattt tgatttagct cctttccacc gatattaaat gaggagcatg catgctgatt 1560
gctgataagg atctgatttt tttatcccct cttctttgaa cagacaagaa ataggctctg 1620
aatttctgat tgattatttg tacatgcaga agggcgaatt cgacctaggc caagtttgta 1680

caaaaaagca ggcttgataa ccaaccatgg tccgtcctgt agaaacccca acccgtgaaa 1740
tcaaaaaact cgacggcctg tgggcattca gtctggatcg cgaaaactgt ggaattgatc 1800
agcgttggtg ggaaagcgcg ttacaagaaa gccgggcaat tgctgtgcca ggcagtttta 1860
acgatcagtt cgccgatgca gatattcgta attatgcggg caacgtctgg tatcagcgcg 1920
aagtctttat accgaaaggt tgggcaggcc agcgtatcgt gctgcgtttc gatgcggtca 1980
ctcattacgg caaagtgtgg gtcaataatc aggaagtgat ggagcatcag ggcggctata 2040
cgccatttga agccgatgtc acgccgtatg ttattgacgg gaaaagtgta cgtaagtttc 2100
tgcttctacc tttgatatat atataataat tatcattaat tagtagtaat ataatatttc 2160
aaatattttt ttcaaaataa aagaatgtag tatatagcaa ttgcttttct gtagtttata 2220
agtgtgtata ttttaattta taacttttct aatatatgac caaaatttgt tgatgtgcag 2280
gtatcaccgt ttgtgtgaac aacgaactga actggcagac tatcccgccg ggaatggtga 2340
ttaccgacga aaacggcaag aaaaagcagt cttacttcca tgatttcttt aactatgccg 2400
gaatccatcg cagcgtaatg ctctacacca cgccgaacac ctgggtggac gatatcaccg 2460
tggtgacgca tgtcgcgcaa gactgtaacc acgcgtctgt tgactggcag gtggtggcca 2520
atggtgatgt cagcgttgaa ctgcgtgatg cggatcaaca ggtggttgca actggacaag 2580
gcactagcgg gactttgcaa gtggtgaatc cgcacctctg gcaaccgggt gaaggttatc 2640
tctatgaact gtgcgtcaca gccaaaagcc agacagagtg tgatatctac ccgcttcgcg 2700
tcggcatccg gtcagtggca gtgaagggcg aacagttcct gattaaccac aaaccgttct 2760
actttactgg ctttggtcgt catgaagatg cggacttgcg tggcaaagga ttcgataacg 2820
tgctgatggt gcacgaccac gcattaatgg actggattgg ggccaactcc taccgtacct 2880
cgcattaccc ttacgctgaa gagatgctcg actgggcaga tgaacatggc atcgtggtga 2940
ttgatgaaac tgctgctgtc ggctttaacc tctctttagg cattggtttc gaagcgggca 3000
acaagccgaa agaactgtac agcgaagagg cagtcaacgg ggaaactcag caagcgcact 3060
tacaggcgat taaagagctg atagcgcgtg acaaaaacca cccaagcgtg gtgatgtgga 3120
gtattgccaa cgaaccggat acccgtccgc aaggtgcacg ggaatatttc gcgccactgg 3180
cggaagcaac gcgtaaactc gacccgacgc gtccgatcac ctgcgtcaat gtaatgttct 3240
gcgacgctca caccgatacc atcagcgatc tctttgatgt gctgtgcctg aaccgttatt 3300
acggatggta tgtccaaagc ggcgatttgg aaacggcaga gaaggtactg gaaaaagaac 3360
ttctggcctg gcaggagaaa ctgcatcagc cgattatcat caccgaatac ggcgtggata 3420
cgttagccgg gctgcactca atgtacaccg acatgtggag tgaagagtat cagtgtgcat 3480
ggctggatat gtatcaccgc gtctttgatc gcgtcagcgc cgtcgtcggt gaacaggtat 3540
ggaatttcgc cgattttgcg acctcgcaag gcatattgcg cgttggcggt aacaagaaag 3600
ggatcttcac tcgcgaccgc aaaccgaagt cggcggcttt tctgctgcaa aaacgctgga 3660
ctggcatgaa cttcggtgaa aaaccgcagc agggaggcaa acaatgaatc aaacccagct 3720
ttcttgtaca aagtgggagc tcgatcgttc aaacatttgg caataaagtt tcttaagatt 3780
gaatcctgtt gccggtcttg cgatgattat catataattt ctgttgaatt acgttaagca 3840
tgtaataatt aacatgtaat gcatgacgtt atttatgaga tgggttttta tgattagagt 3900
cccgcaatta tacatttaat acgcgataga aaacaaaata tagcgcgcaa actaggataa 3960
attatcgcgc gcggtgtcat ctatgttact agatc 3995

<210> 68

<211> 5523

<212> DNA
<213> Artificial
<220>
<223> Vector
<400> 68

cttccgataa aaatatttgg aaccgcatcc tgcatattta tttagaaaca ttgcatcgat 60
aaccgttgca gagcaatata cagggtactc accaactaaa tccataccat acagtgacag 120
gtgaaattgg aatacttgat cagcttgaat aacagagagc aaagataaat ctagccctgg 180
taataaccat ggctcacatc ttatgtacaa gagccaattc taaagacaca gctgcgggat 240
gtagcagtta ggcaaggaaa gaaacaaaat taccagctaa ctgctaagtg tcaaaacatt 300
gctttacact aaagggttaa atatttgcca agaaggtagt gggcacctct cctctgcaag 360
cagcagcata atctagggcg aggcttggtg cagcttcctt gtaggggcat atgaacttat 420
ccatgaaggc actctcactc atctggacag cagtatagta gctccggtcc tgctccaaca 480
atccattttc cttcacgcct agtttggatc actgtaattg aattccattc taataatagt 540
aatttagaca tatatcaatt aagataattc acttttgtac aaaatatatt tgtatactat 600
tattagcaat atatcctaaa tatttatgtg ctacattttt actatagagg agtagaacga 660
agagtgtcat ataagttata gattagaaac aaattctaat catgcataaa atcatttccc 720
atcctccacc ctatgaattt gagataggct tatatctgaa ctttagaaag tggtggaatg 780
tcaaattcca aactaaataa gttactttat tgagtgaatt ctaattcttt tgatttgaag 840
gaatccaaac gccccgtcag gtaattcaca acatagtgtc ggggcatgag gcggcgttcc 900
aagctatatg taagcagagc aggcctgtga gcaatgtact ctggctccaa cccaaccttg 960
gtgatcagga acgcggatcc gggcgtcgcc gggggcgcga ccctgtgctt gccaaattga 1020
aaatttagca aagtattttt tttaccttgt tgattatgtt ggataaccgc atgtccctaa 1080
tttgaatgat attgctcctt atctctaaac gaccaactgt agcttgttct attgaaaact 1140
catcagaata atccatagag ataacatcac caacaaggac tgaatcatcc agagatgcat 1200
caatggccat tggagctagg gaagtcggcc taactacctt caacccgact tggccaactt 1260
gtacagtaga agacaccatg gaaatcgatt taacggactc taagggagtg tttgaataca 1320
ctaaaactaa tagttttagt ggctaaaatt agttggagac atccaaacac cctagctaat 1380
attttagcta ttaactattt ttagtaaatt agttacaagt tagctagcta tttattagct 1440
agctaattct actaacaaat ttttagctaa ctaacgcatt caaacactgt ctaaacaggt 1500
aatctaggca cgcgaagtct gactggccag cccaaactca gcctttgtag tagagagagc 1560
cgaccttggc ggcctgcacg acatttttga actttttta tcggtttggt cttcattact 1620
ttatattctt tgtcaaccaa atactccctt cctcttaaat tataattcgt ttgatttttt 1680
ttatgtaaag ttttatgtat agtccaccta gttaacttta aaagttggtt agtctagatt 1740
ttatggaggc cgactacaat agcaaatcta acaataaaaa agttagctag atcagatttc 1800
aagaacagtc tgaccgattt tgaagatttg attcaggtta attctacgat tttgaagatc 1860
cgacctaacc taattttacg ttcagactaa agacatgttt gtttaagatt atgatatgtc 1920
tagttatata atttaactta ttttaaacta acacttaatt taaaataagt tagattatat 1980
gatcagaata ttatatttat ataattccaa acaaataact ctaatatcga ttctggttta 2040
tattgtataa tttttatgtt gaatattacg gcgcgccaac aaattgcgat cgttatgtaa 2100
tcatgtatac gaccgactgt actattcata aaatataatt caaaatagga gatgctcgag 2160

atagcctggc aagggaggag gtatcggagg cggcgtcgcc tgccgcgagc gcaccatgcg 2220
tcaacgctca ggacctctcg caggcgcgct gttggatcgt gagcaagcac gcgtggcaaa 2280
tggcatgcat tcctctcgtg caagtgccga gctgagggtc ccgacgcggg gggcaggacg 2340
tggcagagcg ccgcccaggc ggacgccaag tgccgagcta ccagggcacc ttctttattc 2400
cgtccttgct cagtcacacc tcgctctcgc tcactctcgc cgtccgcaca gccgctcatc 2460
gtctcccact gcctgccctc tccctgcgcc ggccggtgcc ccgacgcgcc atgtcgtacg 2520
cggccgcact aagcgctatt taaatgccag ctgtacacta gttatcgtac ggcctaggcc 2580
ttcacctgcg gagggtaaga tccgatcacc atcttctgaa tttctgttct tgatctgtca 2640
tgtataataa ctgtctagtc ttggtgttgg tgagatggaa attcggtgga tctcggaagg 2700
gatattgttc gtttgctggg gtttttttg tgtgttgtga tccgtagaga atttgtgttt 2760
atccatgttg ttgatcttgg tatgtattca tgacatattg acatgcatgt gttgtatgtg 2820
tcatatgtgt gcctctcctt gggatttgtt ttggataata gaacatgtta tggactcaat 2880
agtctgtgaa caaatctttt tttagatggt ggccaaatct gatgatgatc tttcttgaga 2940
ggaaaaagtt catgatagaa aaatcttttt tgagatggtg gcttaatgtg atgatgatct 3000
ttcttgagag gaaaaaaaag attcattata ggagattttg atttagctcc tttccaccga 3060
tattaaatga ggagcatgca tgctgattgc tgataaggat ctgatttttt tatcccctct 3120
tctttgaaca gacaagaaat aggctctgaa tttctgattg attatttgta catgcagaag 3180
ggcgaattcg acctaggcca agtttgtaca aaaaagcagg cttgataacc aaccatggtc 3240
cgtcctgtag aaaccccaac ccgtgaaatc aaaaaactcg acggcctgtg ggcattcagt 3300
ctggatcgcg aaaactgtgg aattgatcag cgttggtggg aaagcgcgtt acaagaaagc 3360
cgggcaattg ctgtgccagg cagttttaac gatcagttcg ccgatgcaga tattcgtaat 3420
tatgcgggca acgtctggta tcagcgcgaa gtctttatac cgaaaggttg ggcaggccag 3480
cgtatcgtgc tgcgtttcga tgcggtcact cattacggca aagtgtgggt caataatcag 3540
gaagtgatgg agcatcaggg cggctatacg ccatttgaag ccgatgtcac gccgtatgtt 3600
attgccggga aaagtgtacg taagtttctg cttctacctt tgatatatat ataataatta 3660
tcattaatta gtagtaatat aatatttcaa atattttttt caaaataaaa gaatgtagta 3720
tatagcaatt gcttttctgt agtttataag tgtgtatatt ttaatttata acttttctaa 3780
tatatgacca aaatttgttg atgtgcaggt atcaccgttt gtgtgaacaa cgaactgaac 3840
tggcagacta tcccgccggg aatggtgatt accgacgaaa acggcaagaa aaagcagtct 3900
tacttccatg atttctttaa ctatgccgga atccatcgca gcgtaatgct ctacaccacg 3960
ccgaacacct gggtggacga tatcaccgtg gtgacgcatg tcgcgcaaga ctgtaaccac 4020
gcgtctgttg actggcaggt ggtggccaat ggtgatgtca gcgttgaact gcgtgatgcg 4080
gatcaacagg tggttgcaac tggacaaggc actagcggga ctttgcaagt ggtgaatccg 4140
cacctctggc aaccgggtga aggttatctc tatgaactgt gcgtcacagc caaaagccag 4200
acagagtgtg atatctaccc gcttcgcgtc ggcatccggt cagtggcagt gaagggcgaa 4260
cagttcctga ttaaccacaa accgttctac tttactggct ttggtcgtca tgaagatgcg 4320
gacttgcgtg gcaaaggatt cgataacgtg ctgatggtgc acgaccacgc attaatggac 4380
tggattgggg ccaactccta ccgtacctcg cattaccctt acgctgaaga gatgctcgac 4440
tgggcagatg aacatggcat cgtggtgatt gatgaaactg ctgctgtcgg ctttaacctc 4500
tctttaggca ttggtttcga agcgggcaac aagccgaaag aactgtacag cgaagaggca 4560
gtcaacgggg aaactcagca agcgcactta caggcgatta aagagctgat agcgcgtgac 4620

aaaaaccacc caagcgtggt gatgtggagt attgccaacg aaccggatac ccgtccgcaa 4680
ggtgcacggg aatatttcgc gccactggcg gaagcaacgc gtaaactcga cccgacgcgt 4740
ccgatcacct gcgtcaatgt aatgttctgc gacgctcaca ccgataccat cagcgatctc 4800
tttgatgtgc tgtgcctgaa ccgttattac ggatggtatg tccaaagcgg cgatttggaa 4860
acggcagaga aggtactgga aaaagaactt ctggcctggc aggagaaact gcatcagccg 4920
attatcatca ccgaatacgg cgtggatacg ttagccgggc tgcactcaat gtacaccgac 4980
atgtggagtg aagagtatca gtgtgcatgg ctggatatgt atcaccgcgt ctttgatcgc 5040
gtcagcgccg tcgtcggtga acaggtatgg aatttcgccg attttgcgac ctcgcaaggc 5100
atattgcgcg ttggcggtaa caagaaaggg atcttcactc gcgaccgcaa accgaagtcg 5160
gcggcttttc tgctgcaaaa acgctggact ggcatgaact tcggtgaaaa accgcagcag 5220
ggaggcaaac aatgaatcaa acccagcttt cttgtacaaa gtgggagctc gatcgttcaa 5280
acatttggca ataaagtttc ttaagattga atcctgttgc cggtcttgcg atgattatca 5340
tataatttct gttgaattac gttaagcatg taataattaa catgtaatgc atgacgttat 5400
ttatgagatg ggtttttatg attagagtcc cgcaattata catttaatac gcgatagaaa 5460
acaaaatata gcgcgcaaac taggataaat tatcgcgcgc ggtgtcatct atgttactag 5520
atc 5523

<210> 69
<211> 4305
<212> DNA
<213> Artificial
<220>
<223> Vector
<400> 69

tcatcggtac tcgcgatgtc gtgcgcgtcc gacgactact atatcaagga ctatgacgtc 60
atcttggcac cagaggtgac acagaggagg accaacaaca ataaccactc gccaccgtcg 120
gtcggtctgc ctttaattct cttcgcaaac acatacactt gcttttttgt ttaagcaagc 180
gtgtatgatg gtgcgtacct gatgactgac aatgtacgag ggaacttcta ccggcaggtg 240
taacacgtgc tcttcaatga taagatcatg caaatcgtgt catatatcga agcctgcatg 300
atactgatgg ttgaaatata gtagtgaaac agctaaatta aaataacaaa atttatgtat 360
ggctaggatt acaaataaat tataaaatat ttttttataa caatataaga cacattttgt 420
atataagtta ttatattatt atatgtttct gttgcaacgc acgagtactc acctagtata 480
tccataggct tttgatcctc tacgtcgagt ttttgcttgc atgagagtcg acctcttctc 540
ttttatccct tcttctctat cttagcattt gctcgatcaa ctgtaaacta attattatac 600
gtacttcaag tcatattgaa accaatggca tttaaaaaag taaccatctg aatggaaata 660
tcaagctaaa taaagaagtt acgggagtat gtgctgagaa aacctagaca cattcagaga 720
agttaccaag caagccttaa ataacaattt ttggtgcttt attgcagata gatactccta 780
taggttggag agaagggcgg cgatgctaat gcaccatgca tgtgtgtctg cccatatact 840
ctatcttatc tgaatcctac caaactcttt aatttgactc tttatgaatg gaattcccat 900
atgaattcta taacacaaca gtacgacgtc tactggagca tactgccaag cacgctagcc 960
gacggctcgg cgaccgtctg aggctgtcca tgcatgcaaa tgtgcacaca tgtacgcccc 1020
ttttagcgct ggcatgcacc cacgaccacg acacggctcc cgtggccgtg aatctgacat 1080

cccgggactc ccgcctccgg ccgcgcgtgc cgtgtcagcc tccatcgagc ggtgtactcc 1140
atgcaagcct cgacccgggc cacgtacccc cctcccctcc gctacgtgtc accgctctcg 1200
tcacctatat atggcggtct tgtccagtct ttccacttaa ctactccgtc actttgtttg 1260
caaagctcct cctcgatcca tcgatcactg caccggccgg cggcaccgcg cgcgctcgca 1320
ggggctagcc aacgagacgg cagcaacgta cggcctaggc cttcacctgc ggagggtaag 1380
atccgatcac catcttctga atttctgttc ttgatctgtc atgtataata actgtctagt 1440
cttggtgttg gtgagatgga aattcggtgg atctcggaag ggatattgtt cgtttgctgg 1500
ggtttttttt gtgtgttgtg atccgtagag aatttgtgtt tatccatgtt gttgatcttg 1560
gtatgtattc atgacatatt gacatgcatg tgttgtatgt gtcatatgtg tgcctctcct 1620
tgggatttgt tttggataat agaacatgtt atggactcaa tagtctgtga acaaatcttt 1680
ttttagatgg tggccaaatc tgatgatgat ctttcttgag aggaaaaagt tcatgataga 1740
aaaatctttt ttgagatggt ggcttaatgt gatgatgatc tttcttgaga ggaaaaaaaa 1800
gattcattat aggagatttt gatttagctc ctttccaccg atattaaatg aggagcatgc 1860
atgctgattg ctgataagga tctgattttt ttatcccctc ttctttgaac agacaagaaa 1920
taggctctga atttctgatt gattatttgt acatgcagaa gggcgaattc gacctaggcc 1980
aagtttgtac aaaaaagcag gcttgataac caaccatggt ccgtcctgta gaaaccccaa 2040
cccgtgaaat caaaaaactc gacggcctgt gggcattcag tctggatcgc gaaaactgtg 2100
gaattgatca gcgttggtgg gaaagcgcgt tacaagaaag ccgggcaatt gctgtgccag 2160
gcagttttaa cgatcagttc gccgatgcag atattcgtaa ttatgcgggc aacgtctggt 2220
atcagcgcga agtctttata ccgaaaggtt gggcaggcca gcgtatcgtg ctgcgtttcg 2280
atgcggtcac tcattacggc aaagtgtggg tcaataatca ggaagtgatg gagcatcagg 2340
gcggctatac gccatttgaa gccgatgtca cgccgtatgt tattgccggg aaaagtgtac 2400
gtaagtttct gcttctacct ttgatatata tataataatt atcattaatt agtagtaata 2460
taatatttca aatatttttt tcaaaataaa agaatgtagt atatagcaat tgcttttctg 2520
tagtttataa gtgtgtatat tttaatttat aacttttcta atatatgacc aaaatttgtt 2580
gatgtgcagg tatcaccgtt tgtgtgaaca acgaactgaa ctggcagact atcccgccgg 2640
gaatggtgat taccgacgaa aacggcaaga aaaagcagtc ttacttccat gatttcttta 2700
actatgccgg aatccatcgc agcgtaatgc tctacaccac gccgaacacc tgggtggacg 2760
atatcaccgt ggtgacgcat gtcgcgcaag actgtaacca cgcgtctgtt gactggcagg 2820
tggtggccaa tggtgatgtc agcgttgaac tgcgtgatgc ggatcaacag gtggttgcaa 2880
ctggacaagg cactagcggg actttgcaag tggtgaatcc gcacctctgg caaccgggtg 2940
aaggttatct ctatgaactg tgcgtcacag ccaaaagcca gacagagtgt gatatctacc 3000
cgcttcgcgt cggcatccgg tcagtggcag tgaagggcga acagttcctg attaaccaca 3060
aaccgttcta ctttactggc tttggtcgtc atgaagatgc ggacttgcgt ggcaaaggat 3120
tcgataacgt gctgatggtg cacgaccacg cattaatgga ctggattggg gccaactcct 3180
accgtacctc gcattaccct tacgctgaag agatgctcga ctgggcagat gaacatggca 3240
tcgtggtgat tgatgaaact gctgctgtcg gctttaacct ctctttaggc attggtttcg 3300
aagcgggcaa caagccgaaa gaactgtaca gcgaagaggc agtcaacggg gaaactcagc 3360
aagcgcactt acaggcgatt aaagagctga tagcgcgtga caaaaaccac ccaagcgtgg 3420
tgatgtggag tattgccaac gaaccggata cccgtccgca aggtgcacgg gaatatttcg 3480
cgccactggc ggaagcaacg cgtaaactcg acccgacgcg tccgatcacc tgcgtcaatg 3540

taatgttctg cgacgctcac accgatacca tcagcgatct ctttgatgtg ctgtgcctga 3600
accgttatta cggatggtat gtccaaagcg gcgatttgga aacggcagag aaggtactgg 3660
aaaaagaact tctggcctgg caggagaaac tgcatcagcc gattatcatc accgaatacg 3720
gcgtggatac gttagccggg ctgcactcaa tgtacaccga catgtggagt gaagagtatc 3780
agtgtgcatg gctggatatg tatcaccgcg tctttgatcg cgtcagcgcc gtcgtcggtg 3840
aacaggtatg gaatttcgcc gattttgcga cctcgcaagg catattgcgc gttggcggta 3900
acaagaaagg gatcttcact cgcgaccgca aaccgaagtc ggcggctttt ctgctgcaaa 3960
aacgctggac tggcatgaac ttcggtgaaa aaccgcagca gggaggcaaa caatgaatca 4020
aacccagctt tcttgtacaa agtgggacct aggatcgttc aaacatttgg caataaagtt 4080
tcttaagatt gaatcctgtt gccggtcttg cgatgattat catataattt ctgttgaatt 4140
acgttaagca tgtaataatt aacatgtaat gcatgacgtt atttatgaga tgggttttta 4200
tgattagagt cccgcaatta tacatttaat acgcgataga aaacaaaata tagcgcgcaa 4260
actaggataa attatcgcgc gcggtgtcat ctatgttact agatc 4305

<210> 70

<211> 3726

<212> DNA

<213> Artificial

<220>

<223> Vector

<400> 70

ctcacacaaa tctaaatagt aaagtagatc tggttacaga gcagaaattt tgaagtgtca 60
tagataggct gagaatatgt agtcacgagc aacacattac tacttgaggc catcaatact 120
gttttttctt ctccaaccta tgtaactatg ttattctttt cctccttaaa tgtaaggcaa 180
acctcctatc ctttttcttt caaattcatt gcattaactg agtggttctt acatgtagaa 240
atgaagttgg agcaaagagg cttggtgcgg aaccgaaaat ttgttagtct ataaatcatt 300
agagggcgcc tgaagttcgc caaatgttct agtagatttt agtaaccacc cacgctagct 360
gactcgacca ccgtctgctg gctggcgctg tccatgcaca tgtacagccc ctggcgctgg 420
cacgcaccca cggcatacct cccatgactc tgacagcccg ggactccctc cgctcgtgcc 480
gtgtcagcct cactcgagcg gtgtactcca tgcaagcctc cacctcggcc acgtaccccc 540
tccccttcct tacgtgtcac cgctctcgct ccctatatac ggctgcctgg ccaggccttt 600
ccacttcact actctctctc tctctgcctg caaagctgct tgatccatca tcgatcacat 660
cactccacca gccgcagcgc acgaacgtac gactcatggc agatcgcgac cgcagcggca 720
tctacggcgg cggcgcctac gggcagcagc aggggggcgg ccgccccgcgt acggcctagg 780
ccttcacctg cggagggtaa gatccgatca ccatcttctg aatttctgtt cttgatctgt 840
catgtataat aactgtctag tcttggtgtt ggtgagatgg aaattcggtg gatctcggaa 900
gggatattgt tcgtttgctg gggttttttt tgtgtgttgt gatccgtaga gaatttgtgt 960
ttatccatgt tgttgatctt ggtatgtatt catgacatat tgacatgcat gtgttgtatg 1020
tgtcatatgt gtgcctctcc ttgggatttg ttttggataa tagaacatgt tatggactca 1080
atagtctgtg aacaaatctt tttttagatg gtggccaaat ctgatgatga tctttcttga 1140
gaggaaaaag ttcatgatag aaaaatcttt tttgagatgg tggcttaatg tgatgatgat 1200
ctttcttgag aggaaaaaaa agattcatta taggagattt tgatttagct cctttccacc 1260

gatattaaat gaggagcatg catgctgatt gctgataagg atctgatttt tttatcccct 1320
cttctttgaa cagacaagaa ataggctctg aatttctgat tgattatttg tacatgcaga 1380
agggcgaatt cgacctaggc caagtttgta caaaaaagca ggcttgataa ccaaccatgg 1440
tccgtcctgt agaaacccca acccgtgaaa tcaaaaaact cgacggcctg tgggcattca 1500
gtctggatcg cgaaaactgt ggaattgatc agcgttggtg ggaaagcgcg ttacaagaaa 1560
gccgggcaat tgctgtgcca ggcagtttta acgatcagtt cgccgatgca gatattcgta 1620
attatgcggg caacgtctgg tatcagcgcg aagtctttat accgaaaggt tgggcaggcc 1680
agcgtatcgt gctgcgtttc gatgcggtca ctcattacgg caaagtgtgg gtcaataatc 1740
aggaagtgat ggagcatcag ggcggctata cgccatttga agccgatgtc acgccgtatg 1800
ttattgccgg gaaaagtgta cgtaagtttc tgcttctacc tttgatatat atataataat 1860
tatcattaat tagtagtaat ataatatttc aaatattttt ttcaaaataa aagaatgtag 1920
tatatagcaa ttgcttttct gtagtttata agtgtgtata ttttaattta taacttttct 1980
aatatatgac caaaatttgt tgatgtgcag gtatcaccgt ttgtgtgaac aacgaactga 2040
actggcagac tatcccgccg ggaatggtga ttaccgacga aaacggcaag aaaaagcagt 2100
cttacttcca tgatttcttt aactatgccg gaatccatcg cagcgtaatg ctctacacca 2160
cgccgaacac ctgggtggac gatatcaccg tggtgacgca tgtcgcgcaa gactgtaacc 2220
acgcgtctgt tgactggcag gtggtggcca atggtgatgt cagcgttgaa ctgcgtgatg 2280
cggatcaaca ggtggttgca actggacaag gcactagcgg gactttgcaa gtggtgaatc 2340
cgcacctctg gcaaccgggt gaaggttatc tctatgaact gtgcgtcaca gccaaaagcc 2400
agacagagtg tgatatctac ccgcttcgcg tcggcatccg gtcagtggca gtgaagggcg 2460
aacagttcct gattaaccac aaaccgttct actttactgg ctttggtcgt catgaagatg 2520
cggacttgcg tggcaaagga ttcgataacg tgctgatggt gcacgaccac gcattaatgg 2580
actggattgg ggccaactcc taccgtacct cgcattaccc ttacgctgaa gagatgctcg 2640
actgggcaga tgaacatggc atcgtggtga ttgatgaaac tgctgctgtc ggctttaacc 2700
tctctttagg cattggtttc gaagcgggca acaagccgaa agaactgtac agcgaagagg 2760
cagtcaacgg ggaaactcag caagcgcact tacaggcgat taaagagctg atagcgcgtg 2820
acaaaaacca cccaagcgtg gtgatgtgga gtattgccaa cgaaccggat acccgtccgc 2880
aaggtgcacg ggaatatttc gcgccactgg cggaagcaac gcgtaaactc gacccgacgc 2940
gtccgatcac ctgcgtcaat gtaatgttct gcgacgctca caccgatacc atcagcgatc 3000
tctttgatgt gctgtgcctg aaccgttatt acggatggta tgtccaaagc ggcgatttgg 3060
aaacggcaga gaaggtactg gaaaaagaac ttctggcctg gcaggagaaa ctgcatcagc 3120
cgattatcat caccgaatac ggcgtggata cgttagccgg gctgcactca atgtacaccg 3180
acatgtggag tgaagagtat cagtgtgcat ggctggatat gtatcaccgc gtctttgatc 3240
gcgtcagcgc cgtcgtcggt gaacaggtat ggaatttcgc cgattttgcg acctcgcaag 3300
gcatattgcg cgttggcggt aacaagaaag ggatcttcac tcgcgaccgc aaaccgaagt 3360
cggcggcttt tctgctgcaa aaacgctgga ctggcatgaa cttcggtgaa aaaccgcagc 3420
agggaggcaa acaatgaatc aaacccagct ttcttgtaca aagtgggacc taggatcgtt 3480
caaacatttg gcaataaagt ttcttaagat tgaatcctgt tgccggtctt gcgatgatta 3540
tcatataatt tctgttgaat tacgttaagc atgtaataat taacatgtaa tgcatgacgt 3600
tatttatgag atgggttttt atgattagag tcccgcaatt atacatttaa tacgcgatag 3660
aaaacaaaat atagcgcgca aactaggata aattatcgcg cgcggtgtca tctatgttac 3720

tagatc 3726

<210> 71

<211> 4959

<212> DNA

<213> Artificial

<220>

<223> Vector

<400> 71

ggttcaagat atgtatgtga tgaacataat tttactcaaa agtgatggtt tcattgtact 60
aactagcatc aatacttatg ccagagtgga gcagtgtgtc tatgcactac tccgtacaag 120
agacatgtcc ctcacgctac aaggagtatg ggatcccagt tgactggttg tctgattccg 180
gagtagttgg caaggtgtgc atctctagca tacactccaa tttcgccaaa catgtgttaa 240
aaacatgcat ttttaattcc ttatgaaggt gggtatgatt ttactcagag tatgcaccta 300
aatgaactca ataatttatt tgcatgttgg atatatcaaa ttggcatctg ttcagcttgc 360
gaacaagtat atgaagaggg ttgcatcaga acttgatgcg ttggaaggca ctgagaaaga 420
gcccaataga gagtttttgc ttctccaggg tggcagggtg tgaggttctc tttccgtgtt 480
catcaggtaa aatcctcacg ccaatatttt cagaacccag gtacttcaca ggtgatatga 540
atatttcgaa tcagtaactt attcatctac cttctgcagt ttgctggggg atttgatgca 600
gaaagcatga aagcttttga agagctaaga agcaagatga gtacacataa atctgctcca 660
cagatatctg aaggctgaag catgagttga acacaggatt caacacacat ctggtcttac 720
ttgctggata ctgattttta gttgtgtaca aaagagaagt ttaaattcca gtgtggtgtc 780
aagcaagttg tatatgctaa gagaatacaa gtcatcaggt tatgttcatt tattttttat 840
gaaagtaagg gcaggacagg ttatgttcat gcatataaca gttttagctc aagtgtatac 900
cttttcattt taaaggctgt aactgtaaac aggcagctcc agaaaatgaa tcacttgggc 960
atgtttttgt tgctacgcat atactactaa taaaatgtca aaagcgtcca ttcggttagt 1020
ccggtaccca tggagataat tcgtctgaat gaaccttgcc gtctttgtta aaagaaaaaa 1080
aaattacttt gtgcctcacc gcattcgata ctttgaagaa attgacccgt taccagttac 1140
cacccacgca catacagtgc tagaacagca gatctgactt gaagaagcat atatcaagac 1200
aaaagtccac ctgaacggag acagtcgagg ttgtggcgga gagctccacc tgaacgccga 1260
agtggaaccg aacagttcca gtggttggcc gtggcgagct ggccacctac tagtctccgg 1320
ggaggggggct ggctccagcg cctccgagcg cggtgccagt gtcgccgtcg cgtgggttcc 1380
ggttccggcc gccgccgctt gccggggaat ccaaacggca gacggggtcg ccgtgaccgt 1440
gaatcgtgtt tacttgggcc ttatctaggc cacaacgttt gtgactctgt gagcttattg 1500
tttgggccca tctctttggc gtgtctgaga acgaatttta tcatcaagcc cacgaagcca 1560
aaccaggatc ctacccacct agcacatact agtgaagaag gtaaacaaag tgtattttca 1620
tgttaattca catgcgcgtg gtcgacacca caccacacga cttaaaactc ggtgttatgt 1680
tctacgacca caagagccat gttaatttta agccgtgtct gcgccatgat ccgaagagcg 1740
cgtacagtga gagaagaaac gcacggccgc cagcagctgc cgcgggagcg aggcagcacg 1800
tccgttggtc ggcccgggcg ggagcgaggc acacgtcgcg catgcacgcg cggcgcccgg 1860
acacgtgccg gcgcgggcac gtccctctcc gcgccttata ctcctccccg ccatttccca 1920
gcagccacaa caagacagag agatacccga tcgccaccgc cgaccggact cccctcaaag 1980

gcggccacag cacagcgagc cgtacggcct aggccttcac ctgcggaggg taagatccga 2040
tcaccatctt ctgaatttct gttcttgatc tgtcatgtat aataactgtc tagtcttggt 2100
gttggtgaga tggaaattcg gtggatctcg gaagggatat tgttcgtttg ctggggtttt 2160
ttttgtgtgt tgtgatccgt agagaatttg tgtttatcca tgttgttgat cttggtatgt 2220
attcatgaca tattgacatg catgtgttgt atgtgtcata tgtgtgcctc tccttgggat 2280
ttgttttgga taatagaaca tgttatggac tcaatagtct gtgaacaaat cttttttag 2340
atggtggcca aatctgatga tgatctttct tgagaggaaa aagttcatga tagaaaaatc 2400
ttttttgaga tggtggctta atgtgatgat gatctttctt gagaggaaaa aaaagattca 2460
ttataggaga ttttgattta gctcctttcc accgatatta aatgaggagc atgcatgctg 2520
attgctgata aggatctgat ttttttatcc cctcttcttt gaacagacaa gaaataggct 2580
ctgaatttct gattgattat ttgtacatgc agaagggcga attcgaccta ggccaagttt 2640
gtacaaaaaa gcaggcttga taaccaacca tggtccgtcc tgtagaaacc ccaacccgtg 2700
aaatcaaaaa actcgacggc ctgtgggcat tcagtctgga tcgcgaaaac tgtggaattg 2760
atcagcgttg gtgggaaagc gcgttacaag aaagccgggc aattgctgtg ccaggcagtt 2820
ttaacgatca gttcgccgat gcagatattc gtaattatgc gggcaacgtc tggtatcagc 2880
gcgaagtctt tataccgaaa ggttgggcag gccagcgtat cgtgctgcgt ttcgatgcgg 2940
tcactcatta cggcaaagtg tgggtcaata atcaggaagt gatggagcat cagggcggct 3000
atacgccatt tgaagccgat gtcacgccgt atgttattgc cgggaaaagt gtacgtaagt 3060
ttctgcttct acctttgata tatatataat aattatcatt aattagtagt aatataatat 3120
ttcaaatatt tttttcaaaa taaaagaatg tagtatatag caattgcttt tctgtagttt 3180
ataagtgtgt atattttaat ttataacttt tctaatatat gaccaaaatt tgttgatgtg 3240
caggtatcac cgtttgtgtg aacaacgaac tgaactggca gactatcccg ccgggaatgg 3300
tgattaccga cgaaaacggc aagaaaaagc agtcttactt ccatgatttc tttaactatg 3360
ccggaatcca tcgcagcgta atgctctaca ccacgccgaa cacctgggtg gacgatatca 3420
ccgtggtgac gcatgtcgcg caagactgta accacgcgtc tgttgactgg caggtggtgg 3480
ccaatggtga tgtcagcgtt gaactgcgtg atgcggatca acaggtggtt gcaactggac 3540
aaggcactag cgggactttg caagtggtga atccgcacct ctggcaaccg ggtgaaggtt 3600
atctctatga actgtgcgtc acagccaaaa gccagacaga gtgtgatatc tacccgcttc 3660
gcgtcggcat ccggtcagtg gcagtgaagg gcgaacagtt cctgattaac cacaaaccgt 3720
tctactttac tggctttggt cgtcatgaag atgcggactt gcgtggcaaa ggattcgata 3780
acgtgctgat ggtgcacgac cacgcattaa tggactggat tggggccaac tcctaccgta 3840
cctcgcatta cccttacgct gaagagatgc tcgactgggc agatgaacat ggcatcgtgg 3900
tgattgatga aactgctgct gtcggcttta acctctcttt aggcattggt ttcgaagcgg 3960
gcaacaagcc gaaagaactg tacagcgaag aggcagtcaa cggggaaact cagcaagcgc 4020
acttacaggc gattaaagag ctgatagcgc gtgacaaaaa ccacccaagc gtggtgatgt 4080
ggagtattgc caacgaaccg gatacccgtc cgcaaggtgc acgggaatat ttcgcgccac 4140
tggcggaagc aacgcgtaaa ctcgacccga cgcgtccgat cacctgcgtc aatgtaatgt 4200
tctgcgacgc tcacaccgat accatcagcg atctctttga tgtgctgtgc ctgaaccgtt 4260
attacggatg gtatgtccaa agcggcgatt tggaaacggc agagaaggta ctggaaaaag 4320
aacttctggc ctggcaggag aaactgcatc agccgattat catcaccgaa tacggcgtgg 4380
atacgttagc cgggctgcac tcaatgtaca ccgacatgtg gagtgaagag tatcagtgtg 4440

catggctgga tatgtatcac cgcgtctttg atcgcgtcag cgccgtcgtc ggtgaacagg 4500
tatggaattt cgccgatttt gcgacctcgc aaggcatatt gcgcgttggc ggtaacaaga 4560
aagggatctt cactcgcgac cgcaaaccga agtcggcggc ttttctgctg caaaaacgct 4620
ggactggcat gaacttcggt gaaaaaccgc agcagggagg caaacaatga atcaaaccca 4680
gctttcttgt acaaagtggg acctaggatc gttcaaacat ttggcaataa agtttcttaa 4740
gattgaatcc tgttgccggt cttgcgatga ttatcatata atttctgttg aattacgtta 4800
agcatgtaat aattaacatg taatgcatga cgttatttat gagatgggtt tttatgatta 4860
gagtcccgca attatacatt taatacgcga tagaaaacaa aatatagcgc gcaaactagg 4920
ataaattatc gcgcgcggtg tcatctatgt tactagatc 4959

<210> 72

<211> 5084

<212> DNA

<213> Artificial

<220>

<223> Vector

<400> 72

actaggttgg tgagatcctt aggttttgga aggctttatg tggcagctct gaagactcca 60
acatctatgg cctctagcta tgcctccata tgtattttgt aataagaaaa atcatctccc 120
ttaaacatgg gagagggttc atctccatcg aacatctttc tttaggtggt gaagcctaaa 180
ataatgagca cgacactttg atactaattg aaaggattac tatgcccaag aggtagggtg 240
gattagacca atctaaaatt tcttgtagca ataaaatctt atcgaatatc ccacttcacc 300
acttgtgctt aagagtgata tatattctat cacaaaaggt tttacaccct agtttgaacc 360
ctattctagt atgttagttc tagtaatgta aagacaagaa ttaaattgct aaaatgcaaa 420
tgcttaagat aaagaagggt tagaaacatg acagcatttt tccaaagtat taaagagccg 480
ggactctcca atagtcctcg ttggagcacc cacacaagga tattgctccc cattgaactt 540
cacaaggatc aagggctctc tatgggtttg ttcctcgcca ctctagcatg gtgagtcacc 600
cacagttgtt tacaccttga gtggtgatat acactagatt tcttaagtgt gtatcatcag 660
acttatttag gtcaaactat cattcatggc ccctttatag tatagtcaaa ggaaaaaaca 720
aaggtgctat cactagtata tatgtctatc aactctatta actattagaa ctagccagtc 780
cttaaccttt ttgctcatcc ttctaaaatc aataattgaa taccatcaat aggggcacaa 840
aaactatata gccaattatt attactcata gccaataatt tagtgttgtc attagtcact 900
aaaactagat gctttcatat ctatcatgct cgtgtttgcc tgatccaatt cctaggtcat 960
gtttgggctc cacttagagc ttgtaatggc atcacaacat gatctgatta agaaaatgtt 1020
gactaaagac cttctcaccc acatataaaa tgccatgact tcgctcaaac ttcatcccct 1080
agtctcctac tacacccatg tctatgcttc gcaacttcac aagtctacca catgtacttg 1140
caaagggctg attctcctag ctcccatgtc gtcgccatct ttccccttta ccaccacaaa 1200
atcacaaatg tgctcactta ttatccccgc gtcttccctt ccctacccta aacactatgg 1260
gcaacaacgt cgcatcactt ggtggtcctt gtagatctag acgaggtctc ctccccgttt 1320
tgtgcttgtt gatatagtga tcttgtcctt gtcttgtcgc tgtcctttgg agtcgtcgca 1380
gagaggcggt gaaaattcta gccgcttcta gaaggtgcgg tcgccggtcg gatgagagtc 1440
gagtgagtgc aggcctataa aaagtagcaa ttggataaca ttttgaaaaa aatgaaagaa 1500

ataaaaaaac tagggttaaa taagattcta aattaaatct aagaaatcta tagacattcg 1560
gaaatctatt tatcttttgt actcctttga ttggatttct agattttgac gtttgtgtaa 1620
ttgggcttga cagccccatc aatacaggca ggccagctga tttcgggtgc cgctgatttg 1680
gcccatggag gatagagcag gcctttcaac aggcaggcag tccatgacgt agtgcctcgg 1740
cctcaaagcc catgcgatta atatccactg gtacgtacgc gacagccgag ggagtcactg 1800
agtcagtgcc gaacggcctg gaaacccccc gccgtgtgcc ggtgtccacg cgcgccccgg 1860
tgcggccgcc acgaccgcgg acacgtgccg ggcctcgcac acgcgtcccc cacggccccc 1920
tcgcatcatt aaaaccgcgg tgcccgcgcc acggatcccc ggcacttcat tccggtttcc 1980
ggatagctta cctcccatcg acggtcggtg agccgttggt gagtggcgag acagcgggtg 2040
cgctgggcaa gaacaggcga cgaagaagat ggcgccctag gcggccgcac taagcgctat 2100
ttaaatgcca gctgtacact agttatcgta cggcctaggc cttcacctgc ggagggtaag 2160
atccgatcac catcttctga atttctgttc ttgatctgtc atgtataata actgtctagt 2220
cttggtgttg gtgagatgga aattcggtgg atctcggaag ggatattgtt cgtttgctgg 2280
ggtttttttt gtgtgttgtg atccgtagag aatttgtgtt tatccatgtt gttgatcttg 2340
gtatgtattc atgacatatt gacatgcatg tgttgtatgt gtcatatgtg tgcctctcct 2400
tgggatttgt tttggataat agaacatgtt atggactcaa tagtctgtga acaaatcttt 2460
ttttagatgg tggccaaatc tgatgatgat ctttcttgag aggaaaaagt tcatgataga 2520
aaaatctttt ttgagatggt ggcttaatgt gatgatgatc tttcttgaga ggaaaaaaaa 2580
gattcattat aggagatttt gatttagctc ctttccaccg atattaaatg aggagcatgc 2640
atgctgattg ctgataagga tctgattttt ttatcccctc ttctttgaac agacaagaaa 2700
taggctctga atttctgatt gattatttgt acatgcagaa gggcgaattc gacctaggcc 2760
aagtttgtac aaaaaagcag gcttgataac caaccatggt ccgtcctgta gaaaccccaa 2820
cccgtgaaat caaaaaactc gacggcctgt gggcattcag tctggatcgc gaaaactgtg 2880
gaattgatca gcgttggtgg gaaagcgcgt tacaagaaag ccgggcaatt gctgtgccag 2940
gcagttttaa cgatcagttc gccgatgcag atattcgtaa ttatgcgggc aacgtctggt 3000
atcagcgcga agtctttata ccgaaaggtt gggcaggcca gcgtatcgtg ctgcgtttcg 3060
atgcggtcac tcattacggc aaagtgtggg tcaataatca ggaagtgatg gagcatcagg 3120
gcggctatac gccatttgaa gccgatgtca cgccgtatgt tattgccggg aaaagtgtac 3180
gtaagtttct gcttctacct ttgatatata tataataatt atcattaatt agtagtaata 3240
taatatttca aatatttttt tcaaaataaa agaatgtagt atatagcaat tgcttttctg 3300
tagtttataa gtgtgtatat tttaatttat aacttttcta atatatgacc aaaatttgtt 3360
gatgtgcagg tatcaccgtt tgtgtgaaca acgaactgaa ctggcagact atcccgccgg 3420
gaatggtgat taccgacgaa aacggcaaga aaaagcagtc ttacttccat gatttcttta 3480
actatgccgg aatccatcgc agcgtaatgc tctacaccac gccgaacacc tgggtggacg 3540
atatcaccgt ggtgacgcat gtcgcgcaag actgtaacca cgcgtctgtt gactggcagg 3600
tggtggccaa tggtgatgtc agcgttgaac tgcgtgatgc ggatcaacag gtggttgcaa 3660
ctggacaagg cactagcggg actttgcaag tggtgaatcc gcacctctgg caaccgggtg 3720
aaggttatct ctatgaactg tgcgtcacag ccaaaagcca gacagagtgt gatatctacc 3780
cgcttcgcgt cggcatccgg tcagtggcag tgaagggcga acagttcctg attaaccaca 3840
aaccgttcta ctttactggc tttggtcgtc atgaagatgc ggacttgcgt ggcaaaggat 3900
tcgataacgt gctgatggtg cacgaccacg cattaatgga ctggattggg gccaactcct 3960

accgtacctc gcattaccct tacgctgaag agatgctcga ctgggcagat gaacatggca 4020
tcgtggtgat tgatgaaact gctgctgtcg gctttaacct ctctttaggc attggtttcg 4080
aagcgggcaa caagccgaaa gaactgtaca gcgaagaggc agtcaacggg gaaactcagc 4140
aagcgcactt acaggcgatt aaagagctga tagcgcgtga caaaaaccac ccaagcgtgg 4200
tgatgtggag tattgccaac gaaccggata cccgtccgca aggtgcacgg gaatatttcg 4260
cgccactggc ggaagcaacg cgtaaactcg acccgacgcg tccgatcacc tgcgtcaatg 4320
taatgttctg cgacgctcac accgatacca tcagcgatct ctttgatgtg ctgtgcctga 4380
accgttatta cggatggtat gtccaaagcg gcgatttgga aacggcagag aaggtactgg 4440
aaaaagaact tctggcctgg caggagaaac tgcatcagcc gattatcatc accgaatacg 4500
gcgtggatac gttagccggg ctgcactcaa tgtacaccga catgtggagt gaagagtatc 4560
agtgtgcatg gctggatatg tatcaccgcg tctttgatcg cgtcagcgcc gtcgtcggtg 4620
aacaggtatg gaatttcgcc gattttgcga cctcgcaagg catattgcgc gttggcggta 4680
acaagaaagg gatcttcact cgcgaccgca aaccgaagtc ggcggctttt ctgctgcaaa 4740
aacgctggac tggcatgaac ttcggtgaaa aaccgcagca gggaggcaaa caatgaatca 4800
aacccagctt tcttgtacaa agtgggagct cgatcgttca aacatttggc aataaagttt 4860
cttaagattg aatcctgttg ccggtcttgc gatgattatc atataatttc tgttgaatta 4920
cgttaagcat gtaataatta acatgtaatg catgacgtta tttatgagat gggtttttat 4980
gattagagtc ccgcaattat acatttaata cgcgatagaa aacaaaatat agcgcgcaaa 5040
ctaggataaa ttatcgcgcg cggtgtcatc tatgttacta gatc 5084

<210> 73
<211> 3185
<212> DNA
<213> Zea mays
<400> 73

ttggtttttt gataatttgt ttatctcaca tgatggttct aacaatatgc gcagatctta 60
tacatctctc tcgtagtttt tcataaacta cgagaaagat ataggtttta tgaacaaatt 120
tacttttatt ttgtcatata atgaaatggt caaaatataa attgtacatc atgatgagtt 180
atacaaattt gtagtccaaa atctttttcat ttaaattaat ttactgcttt aaaatgtgat 240
ttttaattgt ctttgcctag tgttgaaaaa aagcactcgg caaagaaact ctttgccgag 300
tgttaaaaaa acactcggca aagaatctct ttgccgagtg tcaaaaataa aatatttggc 360
aaatagcttc tttgctgagt gtttttttgc ttagcacttg gtaaagaact tctttgccga 420
gtgtccgaaa aagcactcga taaaatattt ggcactcagc aaagaaccga attccagtag 480
cgtgagtcag aagttggcat ttgaaccctt tgaaataatc aaacaaatca acgcagttgc 540
atatgaattg gctttgccac atggtagtgc agtccatcga gtgttccatg tttcacagct 600
taaactaacc catggttttg gtaacagaac acacaccagt gactccatcg ttaccggatt 660
ttgatactac actgaaggtt ccggaacaaa tactggattc tagattgatt ctagattgct 720
tcgatcaaat ggggatcatc aacctaacac agcttaaatt atacatgcat gcgtacattt 780
ggtccttcaa gaggaggcag gtgatgatca aggtctctct agcggcctgg tgtgcagcca 840
cgtacccatc cacgcgtcgc cgcgggcgac gacaggtgtt ggccaacctg gacatcagac 900
aagcacgaca atgacaggcg ttcgccaagc gtacgtacag gagacgaaca cgacaagtag 960
caccgtcctg gccgagagcc cgtcatgctc atgcatgcat gcatgctgat catgcatact 1020

cgatwaatat acagcagaga gctgagctac gtacacaaac aagaagcttg cattgttcgt 1080
tgctcgatcg ttgcaggtaa tggagaaccc tgctccgagt atcgtagtgc cccccatcgc 1140
cgcacctgta tcagcgaact tcagcagact cgccttccgc aacctgtaca tccgacggac 1200
gggtccagac agcagggaga tggtgactgt ggagggaaga agaggctcta gtgatcagac 1260
aggcgacatg aggtacgtga gtgacttccc cgtctacgat ggccgtggct ccgacgccgt 1320
cctggtggct cgcgtgcagg gcgtcacaac cacgttcggg aactccaacc agttcttcac 1380
cgtcgccttc gaggctggca ggttcttctt cgtttagaca gatagctaga tagatgctag 1440
cttgtggttc atatatagca gatagatata tgcatgcatc tgccattgat cgtccatcgt 1500
catcgatcag caggctaaag ggctccacgc tccttaccga aggagtggtg acggatggat 1560
ccgatgaatg ggcaatctac ggcgggaccg gagagtttgc tatggcgaga ggtgtcgtca 1620
aaagaaggta ccttgccgat agggacggcg ccgggaacac tgacgagctc agcatgcagg 1680
ttttctgccc ggtgtttggc tcatcacagc aaccaaacaa gcaggcacgt gctgtactgt 1740
atatatgtcc gagagagaga aacagacagt agaatcctca tcagtagtat aatcagctgc 1800
atgtatgtag gactccacca tctctgtcac caagattggg ttatggggtg gcgaaggagg 1860
gtcggcgcag gacatcacga cgacggagcc accgcagcgc ctgcacagcc tcaccgttcg 1920
tgccagtgcc gccgtcgact ccatcgagtt cacgtatact gacagaggtg gccagaggcg 1980
cgccgctggg cgatggggtg gacttggcgg caaccttcga acggtgagca gcaagctcta 2040
gcccctacct acctacattg attcatgaag catgtatata gcttcctcat gcatgcatgc 2100
acatgcatat gcgcgcagat cgatcttggc gacgccgagg atgtcaggga ggtctcagga 2160
acgtacggca catttgaagg agccaccacg ctgacctcgt tcagaattct caccagcagc 2220
aggacatggg ggccatgggg cgtcgagaac gggacgcgtt tctgcatcac cgcgccggtc 2280
ggcagcagca tcgtggggtt ctatggacgc gcgaccagca ggctcgtcgc tgcgcttggt 2340
gtttacctgc gccgactttg atttgatctg aacagagcag gctacctact agcaattgcc 2400
tgctcgatca attatctttc gcacccttta ttattaatat aatctcgatc ggtcatcgta 2460
ctcctatgtc aatgtcaaat aaaggtcata tgtagtcatc gatattgttg gatttattgg 2520
catggtctaa ttaagaaatt catttaattc ctgaaaaatc ataaaagctg aatccgacga 2580
tcagatcgca acctaatcgg atcgtgagct tatcgcttct tgatcgtgag cttatcgctt 2640
cgtgaacgtg gatctatata aagtgtctac cagcctttgt cacataccat cttgtcacat 2700
accatctcat ttcaaccgag ttaggttttg tctctgctta ctgttgcctc gttgccagag 2760
gcggacatag gtagagcggg tatagtttcc tcgctcattt atatattgta aatagtagag 2820
cttaaatttt catcataaga tatcctgctc agtttaactt agatgtctat gctatgctat 2880
ggtactttgg tgttcgccct acaaatatgc cttcaatccc attttgtctt aggtccaccc 2940
ttagccgtcg ttgtggcctt cttcatcccg agctccgacg tacatctacg acagggagag 3000
catgtctcta gaacctgaca ccttggatat cctgcaccgg gagagggcaa ataaggtttc 3060
tgggaagtgt cttcacacga ctgctcgcgt tcttctcatc accttgtcgc cgacgtgagg 3120
gcaaataagg tttctgagaa gtgtcttcac acgactgctc gcgttcttct catcacctgt 3180
cgccg 3185

<210> 74
<211> 3546
<212> DNA
<213> Zea mays

<220>
<221> misc_feature
<222> (3048)..(3048)
<223> n is a, c, g, or t
<400> 74
ttagtagaga ataacacaca tctccctaag tatcacaaaa aaattaattt taagaggcaa 60
ttttagtttc caaagcaatt tattaagatt taagggaggg tatttattag aaggaagtac 120
attgatcaaa cagagaaaat ttcatcatta tggtccccca tagaaatttg tcactcgatt 180
gtcgcaaaaa aagttaccag taaatttgat aaattttgga taccggtctg ttttaactca 240
tcaagaatag cacgacgaaa taatagattg cgtacgtttt cattcataac atctctcact 300
aaagcatttt ttacacacgc tattaaaatg gttcggaaac tgatccttaa gcggtctatt 360
catatccatc tatcgttcta agattcttcc tgtacgtaga ttctttccga ttaaattctc 420
atgaaaaatt aaaaacaaac aggtccttaa actagtcgcc ggaagttctc gcttttgctg 480
tagttgtagt gtcgatgcat gggtggaaac gtggacagat agattcatga aatgtggaca 540
acccacctgg tccagagcat catgatacat tgatacacgt ggcacgctgc ccactgatgc 600
cctcagcttt gaagctaacg tgtcaagtgt ggccggcccc tgcattgcgt cgtgccaatc 660
gtccaagtcc catggcaaaa acccagcgct ttgtgccgcc gccgtccgcc ggccccctctg 720
cccttgtacg tgcacctaga cacatcgtca tcgatcatca cacgcaatcg acacaagaag 780
ttaataaaca gcccaaggac gcagagatca gctgatcgag aaggacttgt actactactc 840
agtattgtcg tcacatgcac atatatgtac ataaagagct agctacctga gctctaccca 900
aggtcgcgtt gatcgatcga tcatggcgcg gttcgtggac ccgctggtgg tggggcgggt 960
gatcggcgag gtggtggacc tgttcgtgcc ctccgtctcc atgaccgtcg cctatggccc 1020
caaagacatc agcaacggct gcctcctcaa gccgtccgcc accgccgcgc cgccgctcgt 1080
ccgcatctcc ggccgccgcg acgacctcta cacgctggta tatatatgct ggcacttgtc 1140
tgctcgatct gtgcctccag ttgacatgca tggatggatt gcacaacttg ctgcgtgtcg 1200
cagatcatga cggacccaga tgcgcctagc cccagcgacc cgaccatgag ggagtacctc 1260
cactggtatg aactgctaca gcatgctacg tacttgtgtc atgatctggc agcgtgtgtc 1320
gccaaagagt agatgcactg tatatttgta cgtactactc attaaattat actgtatgta 1380
tgtatgtgtc caggccagtt gcctaatttc tattctcttg cattgttgcc acacatgcat 1440
ggatgtttaa tttctcggtc ccagcgtgaa tttgtggcgt gcaggatagt gactaacata 1500
ccaggaggaa cggatgcaaa caaaggtgag tttaatttaa agataattac ttaattagtt 1560
atctacttat gtactctctc tctctctatt ttaaaattta acatattttg atttttttag 1620
atacattgtt ttcctaaata catagcttgt acacataagt gcatgtgtgt ctcgaaagaa 1680
acaaaatatg ttataatttt taataaagga aatatcaaat aatcaattta acaaaatatg 1740
ttataccaca actgcatggc acaaacttcc atgtgagccg tgatcgcgcc gttcccgagt 1800
tggcgccgcc agcacgtact acgcgctgcc actgcacggc acatgtcgcc gcacgccaag 1860
cgtcgtcggc acagaagcgt tgacacctgt tgggggctac tactggtgtg tgctgcaggt 1920
gaggaggtgg tggagtacat gggcccgcgg ccgccggtcg gaatccaccg ctacgtgctg 1980
gtgctgttcg agcagaagac gcgtgtgcac gcggagggtc ccggtgagcg cgccaacttc 2040
aacacacgcg cgttcgcggc ggcgcacgag ctcggcctcc ccaccgccgt cgtgtacttc 2100
aacgcgcaga aagagccggc caaccaccgc cgccgctagc tagtagctcc aacaagggcg 2160

cgccagctga gctgcgtgcg tgcaacccac cacacagccg ccggcgaagg ctgcctatat 2220
gaccggcgaa taaaaagtct tactgcaccg tccgtaagcg tactctctgt tggtatatgc 2280
ttgtcttcag gctcttgagt ctatctactt aaatgtggtt accactgagt aatagaagca 2340
gttggcgctt cgatcgatca ttctaatatc cgtacgtgtc aatcattcct gtttccatca 2400
tcttgcattt gaagacgcat tggttctaca ccaaggtgtg gtaggcccac tttggaattt 2460
tcgggaaaaa aagaagaagt ttctgttcgt acttttttcc ctgtgagaaa atgaagcgat 2520
tcactgaaat tcctgtataa ttttctataa aatttgcgtt ccaaagggcc cttacagttg 2580
gatcagtggt gggctttctg acttttgaag tttagtttgg ttgtttactg agcagtttat 2640
ccataccatt cccatgttcg aactccattt tacgaacaat acagtttata gtgcaaaaca 2700
gtggcacaaa aatagagtaa actgctttag tctctattcc ctcgagaggt gactaaaagg 2760
aactaaatca tattaattct ctcttttgcc tcttatttat ttcagttgca ttaatgatag 2820
gagaatgata attaatacat tctgaatact tttagtcact cataccaaac gagtagggac 2880
taaactttag tctctaaact aaactttagt tctggactaa gaaaccaaac agtccctaac 2940
agcagttggc gcttcgacca tttttcagag gtgtcaaatc atgcctatct tgataatttc 3000
acaaataagg atatgttcat tgcatggtgt tgaaggtcta aagaaatncg taacgtatag 3060
aaggagacct aattttatgt agaaacacat aaaaatgcca caatataaag tgaagtgatc 3120
aatttcaaca ttagggcatc attacttcat acaaccctag aataaaatag tatagactca 3180
tatgatccaa ttctctcata tgcacatcga tgtgtatgta aaattaacct tgattgacga 3240
catatgtatg taaaattaat cacacgactg ttctactccc ttccatatgg tctaagcaat 3300
gctttatgag accatctgtg tgcatgaaaa tgatggattt attatctgca tgatttttag 3360
atgctatgtg tcggccatca aatagaggtc catgtgagtg aattgaagcc tagatcaatc 3420
catttttaca tttttattcc cttcgcccca ggctcttgtt tcccatatgc gcacagcatc 3480
aacctcattc acctctatct cctatcaaat cctttctttc tctaaagatc ctcctaaagt 3540
cgctac 3546

<210> 75
<211> 4160
<212> DNA
<213> Zea mays
<400> 75

cattttttg ttatatacag cttatccaac attctactat attaagacat ttttttggtg 60
aacttcttgc gaaatcaaaa ggttctgctc aaatatcatt tctctccatt agctgctctg 120
ctgccttgtt ttatcttatg acttatgttc cctattctaa aaaatatgct ctctgcactc 180
acctttatat tactgcaagt ttatttatgc ttgactgaat attcatgttt ccatggtatg 240
cgttgaatta cggttttgta acaggcgtac tgtgaagaac atcccaaagt atgcttcagc 300
acagttttat attgataatg tacttccaag gatcaaagag aaaaggataa tgtctatcaa 360
gccatttgtg gacagattgg ggtaagtaaa cttttgacga caacatttcg gtttttcctg 420
ttcattttaa ttttgattca gaacatctgg tcagcttaaa tgatgtggta aagggagcat 480
tattctttta ttttgttctt tgttctgtgc aatggccata tattttcgta ttgatatgct 540
ctcagtggaa cagtctgaag ctaatgtgaa acttcgatgt tcaggtatga taatgtacca 600
acagagatta accggctgag atgcagagtt aactatcatg cattaaagtt cctacctgat 660
attgaggaaa tggctgttaa gcttgctgca agaatgagga acaaaactgg cagcataaat 720

ccatacatgt acgttataat tatccttttc gctgggtgat attgtgtaca gtagagcttg 780
tatctaccca agacactatc cagtgcatag ctgaagttga cgctctcaaa gtttatttca 840
caatcaaata ttgacacatt ttttttgttc ttaaagggct cttcacctga gatttgaaaa 900
agggatggtg gggctttctt tctgtgattt tgccggcaca cgggaggaga aggcaatgat 960
ggcggcttat aggcagaaag aatggccaag gcgcttcaag gtgacgcgga ctggactaga 1020
tacatctgtt acattggact gagaaagaat ccctaggaga aatgaagcac cgacactaac 1080
tatacgcgct agttcgggaa ccccgttttc tcacgagatt tttatttttc caagaaaaat 1140
tagttcattt tttttaggaa aatataaatc tcttaagaaa atgtagttcc gaaactagcc 1200
ctacaagtct aacgattttc tcttttttgt atctgccacg cagaatggat cccatctatg 1260
gccactggcg ctgcagaaga gaaaagaagg gcggtgccct cttgagcccg gtgagatcgc 1320
tgtgatcctg cgggcactgg ggtacacgag cggcacacag atatacgtcg cgtccgggca 1380
agtgtacggc ggcaagaacc ggatggctcc cctcaggaac atgttcccca acctggtagg 1440
caggctcaaa tccacttcag attgtgtcaa gctgaagcca gtgataactg ataagccact 1500
gccgtgcatt gtgcatcgct gcaggtgacg aaggaggagc tagcgagcgc ggaggagctg 1560
gcgccgttcc ggcggcacgt gacgagcctg gcggcgctgg acttcctggt gtgcctgcgg 1620
tcggacgcgt tcgtgatgac gcacggcggc aacttcgcca aactcatcat cggggcgcgc 1680
cgctacgcgg ggcaccgcct caagtcggtg aagcccgaca agggcctcat gtccaagtcc 1740
ctgggcgacc ccgacatggg ctgggcctcc ttcacggagg acgtcgtcgt cacgcaccgc 1800
acgaggacgg gcctccccga gcccaccttc ccggctacg atctctggga gaacccgctg 1860
acaccctgca tgtgcagggc atgagacggg ccatccctca gatcagagtg gccgtctggc 1920
cgagtcctcg tcttcgctag gctacgacta cgagtagtct cgtgctcact tccatcagcg 1980
cgcatttcag cgagcatatg cttctactag ttaaacacct agatttgtat gtatttggct 2040
ggcctaatct gtagtttcac atagatcact tcatgctgaa aatgtcttgg acaaggcagt 2100
gaatgaagat aacatcagta acgacctgct tttccaaagc tcctcttgga tttaccaagc 2160
tgagccaaga aatggctaac ctgagttttg acttgcagcc ttgcaggcat gacgtgggcg 2220
acctgaactc gctagtcgta gagtttcttt ccacgcggca gctcggcaga gctacgcagc 2280
ctctctgccc gccacgttct cctgcaaggc tgcaactcga agcactgaga agctattaat 2340
taatggattc tcgagcatgc ggccttgttc gctaaacctc gcgcacagaa acacaagcga 2400
gtaccagagc agctaggagt ggtcagcaat ggcgcagctt aaagcagccg ccgacgagca 2460
gcaggaggcg gcgctgcacg gcgtcgcctc cggcggcggg aacgagagca acaagaaggc 2520
gcgcgcgggg ctctgcggcc tgctccgcga gcgcaaggtg gtggacctgg cgcgggccaa 2580
gcggcggctg gtggaggttc cctacaccgc gacgctggcg cacacggcca acgcgctcct 2640
ggcagcgcgc gtctctgccg tggccgtggc tgcgccaccg ggccactgga tcggcgccgg 2700
cggctccatg atcctcgagt ccgacccggc caccggcgcc gtccgcaagc actacatcgg 2760
catggtgaac atgctcgaca tccttgccca catcgccgag gccagcgacg acgacgaggc 2820
ggccgacctc gaccgccgga tggccgtgcc ggtgtcctcc gtcatcggac actccctcga 2880
gggcctcact ctgtggacgc tccacccgag caccaggtac gccggccgat ctccagctca 2940
cactcgtatt tttcttcttc ttcctcgcat tgtacgcaat ctgcgcgtgt gcgacatgtt 3000
cgttgcgttg cgccagcgtg ctggactgca tggagacgtt cagcaagggt gtgcaccgcg 3060
cgctggtgcc gctggagagc tcggcggaca acgtggtggc gctggagctg gtggagtcgg 3120
cgccggggta ccggatgctc acccagatgg acgtggtgag gttcctcagg gcgcacggcg 3180

cggagctcag gggcgtgctg ttgcgcaccg tgcgcgagct cggcgccgtg aacgacaccg 3240
tgttcgccgt ctctggcgtt accaaggtga tcgacgtcat caaggcgatg cgggcggcgt 3300
cgctgaccgc cgtgcccgtc gtggacgccg tcggtagcgg tacagagacc cttcaatacg 3360
tgagcactgc acgcgcctcc gatccgaaca cgccatctcc tgccggattt cctcctgtct 3420
gaaattcctg tcgtgctgct gcaggggagc gggcaaaggg cggtcgagac gttctcggcg 3480
acggacctgc gggactgccc ggtggcgcgg ctgcaggctt ggctggggat cagcgtgatg 3540
gagttcaaga ggaaggtggc tgagtaccgc gccagcacca ggcttgtggt ccccggcgcc 3600
gacgccacgg acactggcac ccctgtcgcc gggtacgccg acaccccgc ggctgccgcc 3660
gccactgacg aagagcagag cgagcagcag gagtcggcgc tggtgacgtg ctcccaggag 3720
agcaccctcg gcgaggcgat cgaggcggcg acatcgaggc acgtgcaccg gctgtgggtg 3780
gtcgacgagg aagggctctt gcgtggggtc gtgtcgctca ccgacatcct ccgggcagtg 3840
cgggaggccg cgctcggcga ggacctggag ctgcacagca tcgtgccgtg agcccgtggg 3900
ccgtgagtcg tgacgtccaa gaacaagctc gtagctgttt ctgcccatag tgtccgtcca 3960
cctgtcgtgc tcctgcagag gagggggaaa agggcgatcg agacgtgttg gcgacggccg 4020
accggcgacc gccgccgagc tcgttttgcc ttgttgcttt gtttgttata tggccctggc 4080
tccgatccgg gcttcgtatt tcgttgacag gccttcatcg tgacgcctta ttttgcatcg 4140
ttttattgat caaccaaaaa 4160

<210> 76

<211> 2131

<212> DNA

<213> Zea mays

<400> 76

cccgtcatga taataatatt ttcacatgcg gtttcttaag caaaccgcca gtgctaatga 60
tatttacact agcgggctgc taaagaaaac cgccagtgct aaagatattt acactagcgg 120
ttggtgaaca actgcctgtg aaaaaagccg atttctacta gcccctagct agcactggcg 180
acataaaaaa cgtcagtgaa aatagctcta ggatcgtcac tatagagctt ctatgtactt 240
agtggttaga actgatattg tagtgcacca agtgccgatt ttaattaaac caatactaaa 300
tactagtaaa taatactagt ggtctgaatt cgatttctat agtaatgttt gcttgcaagc 360
cgcaaataga gtaaacattc gtcgtcacag aaatccacat tacatcaagg tccatggcgg 420
ccggccacgt acccatccca cgcgtcgctg cggaggacac gtgttggctg accggacagt 480
tggccgatca gacagtggac agaccggaca atagaagaag aagacgacga cggcggcggc 540
accgccgagt aggtgcatgg tcacgctagc tgtagctttt tgcagagcgt cgtctgtaaa 600
tacgtagccc ttccacaagc gaggcaaggg gggagagagt atcgtcagct agcagagaga 660
gtgcgtagca actagcaatg gcggagaacc ctgctccgag ttccccgcta gtgacttctc 720
ccgacgccgc tgcgccgcct ctgccagcgg agttcaccaa gctcaccttc cgtagcctgt 780
acatccgtcg gacaggtcca ggcagccggg agatgactgt ggacgggagg ccgtctgacc 840
agctgggacg acggttcgtg agcgactttc ccatatacga tggccgtggc tccggcgccc 900
acctcgtcgc tcgcttgcag ggagtcactg tccaaatcgg cagctcgcac cagctagtca 960
gcatcgtctt cgaggccgag aggtagttca atgtatgcat gcatgcatgt agtgtgtata 1020
tatataatgc atgtgcgatt cattcattgg ttcatcgtcg tcaggcttaa gggctccacg 1080
ctgctcacca acggagtgat aacggatggg tcagacgagt gggccatcta cggtggcacc 1140

ggggtgttcg ccatggccac cggtgtcata cgaagaaggt ttctcgccgg tagcaacgac 1200
gggaactctg atgagcttac cagcatcgaa gttttctgcc cggcgtttgg ctcggcacaa 1260
caacagccaa acaagcaggt acgtagtgtg tatatgtcag agaaaaaatc aataactctt 1320
ttaagaatga cagcagaatt ctcagtaaca caatcagctg gatgcacgca cgtaggactc 1380
cgtctctgtc accaagattg gggtatgggg tggaggtgga gggtcggcgc aggacatcac 1440
gacgacggag ccaccacagc gtctgcacag cctctccgtt cgaactggtt ttgccgtcga 1500
ctccatcgag ttcacgtata ctgatagagg tggccagagg cgcactgctg ggcgatgggg 1560
tggacttggc ggcaaccttc gaacggtgag cagagcagca agtccctacg accctaccta 1620
cctttgattc atgcagcatg tatatcttcc tcatgcacat gcgtgcagat cgatcttggc 1680
gacgccgagg ttgtcaggga ggtctcagga acatacggca tgtttgaagg cgccaccacg 1740
ctgacctcga tcagaattct caccagcagc agaacctggg ggccatgggg gatcgaggac 1800
gggacacgtt tctgcatcac cgcgccgatc ggcagcagca tcgtggggtt ctatggacgc 1860
tcgaccagca ggctcgtcgc tgcgatcggt gtttacctgc gtcaacaact ctgatctggt 1920
catctagcta tcatcgtcgt ccggtgctgt cgtatgtgtg ttcatgtcaa ataaataaaa 1980
gtggtcttgt gtctacggct gccatcgcca tgtgccaaat aaatgccatg catgcatgtg 2040
tctaaattta tatttccgtc gtcaccctgt agtatacgta tttatatcaa atataaaagc 2100
ctgctgcaat tcactaagga atcgcgtttt t 2131

<210> 77

<211> 1996

<212> DNA

<213> Zea mays

<400> 77

gtgattaagt tgactggcaa attgcataga cgataataca ctctatgctt ttgagataac 60
cactgtacac caaccgactt gttgtttgaa ggctgatctt taaccattac tactgtaact 120
aactgttgtt gaataaacta ttcatacgac ttggagactt tgatgtatat tatgttttgt 180
acctatacta ttaactaccg cagatacgta tttacatctc ggtaacatct gccagtcgca 240
caaatgcact aaggtgactg acagactgta gcgaccgata gagcgctgcc ttgttctagt 300
caaaattcaa gcctggtggt cttctcgatc cctctgcgcg cgccctgctc ttttatcctg 360
tgtgtctcct ccacgaggga aggcgtcatg agaggacgac acgacgacca tgcagtgctg 420
cgcaaccgcg cgcgtctgcc tgttcactga gccccaggcg gccggtccaa gctattacca 480
cgtcgccccc ctccagggct gcgggcagca acgtgtcgac cggtccgcct ctgacgtgtc 540
cctcccgtcc tctgcataaa agggtgcggc tggcggggag cctcagcttc atatcgtcgc 600
aagctctccg tgtttctctc gtttcttcac tgctgccaac tgtgctgtgc agtagttgca 660
agagctgatt ggtagctagg tacgctaggc aagtaggcat ggctcccaag cggcgcggaa 720
acaaggtggt gggctccgtg gtgaagacca aactagtgca ggagaccgtg gaggtcatcg 780
tcgccgacga cgatgggttg cacgcggaga agcagcaggt cccggaggct ctggccctgg 840
cgcaccccac cgtggacgtc tctggctcca cggtggtgca tgtcgtcgag gtcactgcca 900
aaagaggccg tggtggtggt ggtggtggtg gtggtggtgg ccggcgcaac gaggggaagc 960
cgccgccgga ggaggattct gcagcagtac ccgtgccgca gagccaggag acgcaggacc 1020
ccaacgagga gctggagttt gagctggagg acgaggagga gaagcagccg gagactccgc 1080
gcgtggcgtc ggagaagagg aagaaagcag caacaccaac aaagaagacg aagacgcagc 1140

agccgcggcg gcggcggcag cggctggggc aggccagctc cggaggcgac gccgggatgg 1200
gcggcgtggg agggtacagg cggtacgtgt ggcgcgtgct gaagcaggtg cacccggacc 1260
tgggcgtgtc ggggcacgcc atgcaggtgc tggacatgat gatggccgac atgttcgagc 1320
gcctcgcgga ggaggcggcg cgcctgtcca aggccacggg cagggcgacg ctcacctccc 1380
gcgaggtgca gagcgccgtc aggctcgtgc tccccgggga gctgggcagg cacgccatct 1440
ctgagggcac caaagccatc tccaagtaca tgtcctatgc cgcctaggta gctaccgtac 1500
atgttttctg taccttggaa gtagtgcgta ctgcagctga tgtattttca tgcatgccaa 1560
ctagactagc tagtagttac tgtacgtatg tagttgatag tgtaatcagg ccgggtagca 1620
gctagctaga ccaacttaat tagctaatgt agtttgatgt acctgtctgt cagtctgtgt 1680
atgtttcgtt gttttaatcg accaacttgt tccgtgtcct ttttgttgtc tagttctgtc 1740
tactgttctg tttgtctgat tggatccatc agcccatcca tgtaccgcct ggctagctta 1800
tcaggtattg caatccaatt caggaactca cctctatctc cttttctcag ctttttctgt 1860
aaacaggatc cgcacttttg ctacaaaaca agttagattc actacagtaa actaacaaac 1920
atccgactgc cgccttatct ccgacgggtg tgtacgaggc tgtcggaggc acttttgcta 1980
caaaacaagt tagatt 1996

<210> 78

<211> 2247

<212> DNA

<213> Zea mays

<400> 78

tataaattag aacggagggg tatgtttttt tacgtttcca ttatttgagt ctgctcatca 60
cgtttaattt gtttatacag ttcaatgagt tcagttacag cggatgataa ttcgacggcg 120
tttatttgca tgcacttact ttttacatta cgcctagaat gtaatcggat ggacgaatat 180
tacttgtttt gtgtttattt tcatattttt ttctcttcgg atacggatat tatcgagttg 240
tgccgaacaa gattttgaat gtatatcgac atcttaaata tataactttg aatatacgga 300
tacgtatata gatcggatat tgaataccca gctagactca gattaaattg gatcttagtt 360
attttagggg gtgtttgaat gcactaaaac taatagttag tggctaaaat tagttgaaac 420
atccaaacac cctatctaat agttcagcta ttagttattt ttggaaaatt agttaatagt 480
taggtagtta tctgttagct agctaattca actaattata agttctagtg catttaaata 540
ccccttagac ggatcgaatt agaacaccga tggataatat ccataacatt ttacacctat 600
aaatatgtcc tatatgtata tagatgtcaa aaacaggttg ggcatgttgg atgacccgtg 660
gcacgataca gttttaagca gtgccaaaca tggcatggcg gcgagctaga catgatacgg 720
tccggtttta ttttgtatt tttttttcgt aataatatct gtattatgct tgttcgaacc 780
tcacttgctt atgtggatgt gatagtatca actcgctggc gttccgtgca tcgtaacatc 840
tatcacatcc cacagatcat gtcgcttatc ttttctgata ctatagcagt agcacacaga 900
tgccggtcga acccagaagc atcacgccat caccccctcc ccttttccca atcaaaacca 960
cgtccgtccc aatggtaacc cagcaccaat ctcgtgttcg tgtgagacag agcatgcaaa 1020
caaacagcat gcatgcgcga aggaaagcgt gcagcgcgcg ggacgtgaac cgcgcgcgaa 1080
ccctgccgcc cgtccggtct cagctcacgc ccagcgtcct tcccaggccg cgatccggcc 1140
atcctgacgc gcgtttcgtc tgggccccaa aaccacgtcc gccgcggcgg cggcgcgccc 1200
acgggctccc gtgcccgccg atagtttgcc ggaccctgcc gcctcctata aatgcagagc 1260

cctccgccag cgtctcccgc atctcatctt cttcctccat ccgcactact gagagcgaca 1320
aagctagctc tctccaggga tcggattcgg atcaccatgc cgtccaccgt gaactccgcg 1380
tccctcgacc agaaccttga gctcgccaag caatgctcgc gaggtataca tacagatcaa 1440
tcgatccgtt tactgatgct tgcttgagca cataattaat catgtcttcc tgctgcagag 1500
gccgccctcg cagggttcaa ggcagcagct gtcgcgaccg tcgcctctgc agttcccacc 1560
gtcagtgttc ttcatctgtc tcagcatgta aacctcgctc taataatcct cgtcgcctgt 1620
atgtgattcg atcgatctcc cctgttgttg tttcagctgg ttagcgtcag gatgctgcca 1680
tgggccaagg ccaacatcaa ccccgctggc caggccctca tcgtctccac aggtcgatct 1740
ctgaatctct ccctgatccc tctacgacga cctagctagc tattactacc aagatgcctt 1800
cacatgtctt tctgtgtgta actaatggcg gaatgcctgc tttaatttgc tccacgaaaa 1860
ctgggtcgtc gtgcagttgc tggcatggcg tacttcatcg cggcggacaa gaagatcctc 1920
tcgctggcga ggcagcactc gtacgagaac gcgccggagc acctcaagga cacctccttc 1980
cagggcaccg gccgtccgca cccagggttc ttcaggcctt gagcgagaga ccatcaaact 2040
accgccctag ctagctggac gcactagttc gtcagcggcc gccgattcat gtcgtccaag 2100
atcatatcaa ctactgctag catatatata gtgtactagc tagtagtagg caccggcgat 2160
ccgtgcatgc atgtgctcgg agaaactgta atggcttagt gctctgctcc agtttactgt 2220
atctaccttc cattatcata taataat 2247

<210> 79

<211> 3000

<212> DNA

<213> Zea mays

<400> 79

ctccccgtcg caatgaggag actgtkgatc tatttggttc tcatctaact ctatcacaac 60
ttgtccaaga ttatgcgact aaggcctcct ctggtagggc tcattcaaca actccttgaa 120
aaaactacca aaagggaggt tttttatggg ctctttctcg aaaagggagg aactcctcaa 180
aaattcaaca ggaaccgccg gaactaaatt taagtaaaaa aaactaaaga aacaatgata 240
agttacaccg aactataaag ttcgttcgtg cgccacatga ccccacgtgt cccacgacct 300
gaagggactc gtggcctaca cacgcgcaca ataatcagtt accatgaatt attttatatg 360
cgaatatcga cattacacat gaataatgtg aatttgaatt tccgcagcat gcaaaattcc 420
cgtgagtttg aagggcgata aaaacgggtg gccaaaattg gggcggcaat gcaagaagat 480
aagcctatcc atcccttgca ttggtattgt catctggggc agggcagggg agggcaggag 540
aagctatcta ttggccatct atccagcagc cgctctcccc agccccactc tctccatcta 600
gagctcccta actgcaccgg ccgccacacc accgtacccc gacccctcga caacaatggc 660
caccgtccta ggcagccccc gcgcgccggc cttcttcttc tcgccgtcct ccctccgtgc 720
cgcgccggcg cccaccgccg tggcgctgcc tgcggccaag gtgggcatca tgggccgtag 780
cgccagcagc aggggcaggc tgcgcgcgca ggccacctac aacgtgaagc tgatcacgcc 840
ggagggggag gtggagctgc aggtgcccga cgacgtgtac atcctggacc aggccgagga 900
ggacggcatc gacctgccct actcctgccg cgcggggtcc tgctcctcct gcgccggcaa 960
ggtcgtctcc ggctccgtgg accagtccga ccagagctac ctcgacgacg gccagatcgc 1020
cgccggctgg gtgctcacct gccacgccta ccccacctct gacgtcgtca tcgagacgca 1080
caaggaggag gagctcaccg gcgcataatc attcatgccc atccatctct atctctgtgg 1140

gtcgtcgttg tgtgtaattt gagtacgcgc gtacacgtac tgcttttgtt tcatttgagc 1200
actgttcgtg taagcgtcga gttgccctgc catcgtctct ctctctctcg atctctacta 1260
tttctgtgtg tgcgcgcttc tatttttcca atgttgagtt acgcatattt gtccagtaac 1320
cttgttttgc ttaatcacac acagctccca aattccagtc gttctgtggt gacggttggt 1380
tgtggggtgt ggcggcgacg tcagtcatgg gagaagagat ccgaatgcat gggggtgttg 1440
gcgctttctc tgtccctctc cctctccctg tcttctcgcg cgctcgccgt cgtcatggct 1500
cgacgaacag acattcactg ccttccccgc gcgcttcaga cagaccacgc ttgtacgtag 1560
cgccgcttct ctagtcgccg gagcaggcac gagtgggtgg cgaagaggcc acggcccgac 1620
ctgctagcgc cagcgaccgc atctcgccac cagccgctgc ccgctggccg ctgccaccgt 1680
atgttgggtt gggctgaagc ctgaagcccg tgaccttttt ctttttcttt attctttcct 1740
tttcttttgc cggacaagac aagtgatgtt gcaaaatcca tataaaatcc tagagaaact 1800
ataaagtttt tttttgttga actccttata acaacgtcta ccctttaata tatggtatat 1860
aatttcacat gttttagtat aaattgttta ctgtatttct atgtctatat gctcttggta 1920
agattgttta taaattttaa atttaaaatc ttaaaagtca taaacttata gaaaaaaatt 1980
aggatcctaa acaattttaa acaaaaaaaa aaacttttca attacaaaat cgtatgtgtt 2040
ctttatgcat gcatgcaact gagaagctgc ccaacggcca gcgcagcaga agggcagagc 2100
ggtgggcggc cacgtcgccg ccgcaaagga actgacgtgt cccggcccga cgacgacgac 2160
gcgaaccttc accgcgccgc gctccacctc tcttaagcta aatacgctag tgcaggcgca 2220
ggcatccagc tgcgacaacg tagcatacac tagtgctttt gctagctact tgtgcgagaa 2280
gagagagcgc acggcgatag atatggcgca tagcaagcgc gaaatcaagc ccgggagggc 2340
gcataccaag gtggccggcg acgacgagat gctgcggacc gggttcttcg acggcacgcc 2400
gctggagggc ggcaagatcg ccgactccca gcccgtcgac ctcttcgccg ctgcccgccg 2460
cgtcgccgac gccccgtact catcgcagca ggaaggacca cacgaggagg ggaccaacaa 2520
gaacgccgtc atcgcggagt cggagcccgt cgacctgccc gcgagcgcgc ggggcgtggc 2580
ggaggtgaac cgtgctgggc cggaggagca gacaggccgc ggtcggcagg gcatggccga 2640
gcccaccgcc ggtggacgtc gcttgggatt aggtcgcccg gccccggctt aacgatccag 2700
atcaagcagt actcgactac cgccactaca ctacctgctc tgctcgtgtc ttgtttccag 2760
ttttctttct ctgtaagcgg tgtagggttt gccaaacttt tatgtacgaa ttatgaatat 2820
caataaagtc gatctgctgc tgcgtttctt ggcaccaaca atctttccgc ctcatcagag 2880
caactccccc ataaatttaa atttcctata gtgaatttgc tagtctgcta gcgacttact 2940
aagtagttca tactgaacgg gagaacaagt gccatcgagg cttcatgcac atttcactga 3000

<210> 80
<211> 1719
<212> DNA
<213> Zea mays
<400> 80

gccatcctta cgtcccgcgg cttcccgact cttctagcag gaaccgagcg acctcggact 60
cagcggctcc gcctgtcggc gagcgcacgc cgcgtgcctg cccgcggtct ctcgtcgtct 120
ccagaagatg cgccccgata ctcgtctttc caccettctt ctacctcctt ccaccccctt 180
ctccccacgc cccatcggat cgcacgcgca ccgcaattgt tgttcatcac ctctccgaag 240
aagcgtcaca gagcaccgcc cggaggttcc ccttcccgtc gccgtcgctt ggccaggtaa 300

gtaatgacta atcgcctgag tctcctgcgc ctcattactt cgaactgtgg tttcgttagt 360
cggacgtcgg agcaatgcta tgaaccttgc cattccatgt tacttggtgc tgcgtgtcca 420
cattcgacgc cacatttttt tcttgctagc caaccattgc gcgtctgtgt tacagttcca 480
ccggcgatat acattatgcg atcatttgat gtggatattg gtgttttttt ggttagcgga 540
tcaccttagt tttgcactga acatggaaca aatgtggatg ttggtgtttt tttgttagtg 600
gatcacctta gttttgcact gaagatggaa cgaggaaatg ggaaggggga actgaacgaa 660
gcacatagtt tcagagacta tgtgcactgc agagactatg cgaatggtca agggaatttc 720
tcgccttggt ttcctaatcc tcgcttcctt cttgtttcga attcggtgta tatatgtggt 780
ttattcggca cgtttaggaa tttagttggg gaatggggcg cagtatgtca gggttgtgtc 840
atctgaatat ataatgcaag atctattact agtttaaatt gacttattat gcaagtcgat 900
taacctggct gggctcagtg gtgtaccatg gaagctttgt gattgtagcc gggcagtaga 960
acgctaaacc tgaggtttgg tactgtagca ctcggctagt cagctgtatg ccaagatgtt 1020
acttactcga tactttctgc catgtctcat ctgtgttaac cagggtgtgt gccacatagg 1080
gcttctttcc atgctgtttg ttaacctgtt cacacttctt ccatatatat attcataata 1140
gctcgatcta caattttcct gtcttgagct tctttgttcc taaaaacgaa ttgtatacca 1200
gcaatgtata tctgatgcaa tattgtttgt aggatggctg aacaattcta taccgtggca 1260
tcggatagtg aaaccaccgg ggaagacaaa tcacaggctt cattccctga tgttgctgtt 1320
ggcattgaca ttgggacttc caaatgcagt gttgctattt ggaatggcca ccaagttgag 1380
ctcttaaaga atacccgtaa tcagaaaggg atgaggtcat atgtcatgtt caaagatgat 1440
actctttcag caggagttac tggaggtgca gccaaggaaa atgcacacga ggaaagagac 1500
atcttgtcag gaagtgccat attcaacatg aaacgcctga taggaagaat ggacactgat 1560
gaagtggttc aagctagtaa gactctccca tttcttgtgc agacattggg tatcggtgtg 1620
agaccattca ttgcagcgct cgtgaacaac atgtggcgat ctacaactcc ggaggaggtc 1680
cttgccatct ttctacttga actgaaggcc ttggtggag 1719

<210> 81

<211> 3602

<212> DNA

<213> Zea mays

<400> 81

ccattttaat aattataatt tagacaaaac taattaagtt tatatattta tatatgtaat 60
atatttgtat attattctaa atcatatgag agagatagtt atatactaca tttatgttat 120
agaggcgcaa gtagaagaat gtgttataag ttgtacatcg gaaaaatagc atgtaaatct 180
atagaatcaa ttttcatctt tcaccccatg aatttgaggt atgcttatat gataacttta 240
gaaagtggtg gaatgtcata ttctaaaaaa aatagcctat ttcattagta agattccaat 300
tcctcgaaat gaaagaaaac aaacggggcc ttattagaat ggaattcaat tccaatgatc 360
caaatggggc gtaagggatt tccactttcc taagaaaaat tagctcattt tcccttggtg 420
tttaataatt atgttttgtt aaaaggtcag cttcccgaga atgcgttgtg taaaagccca 480
gtataagggt ctgtttggtt gggctgtggc tgtgaaaaaa gttgctgtgg gctgtgagct 540
gtggaaaaag ctgctgtaga ctgtaagctg ttaaaaagct aaaaaccgtt tggtggaaac 600
cactaaaagt cgttaaaaaa tcttcgatat atgttttcac agttccatcc gaaaagccac 660
taaaagcagg tccagaggtg ctttcagatt tgcactacga gaaagtcggt ttttagaaaa 720

agctgcttcc tggatccagc cctttggktg gcttttggct ttwaggggca caaaagccaa 780
agccaaaagt caaaccaaac acaccctaag tcgcgtgagt gggccattta tctattccct 840
atgcaaatct cctgaaaaat tacaacatta gctcgttaaa aagtaaaata attttaaaaa 900
attagagact aattatttga taactttata gttcatcaca ttttcattga aaatgtttta 960
ttttttttgc cgaactgatc tacacaatat ttgaatctac acttttggct tgggctcgga 1020
gaaaaatagt gcgtgcccag ccctttgctc tttccccgat aacaacgcgg tatgtggcct 1080
aacgcccaaa tccggcccat ttattccggt gcgtagatgc agacgcggga gcaggtgcca 1140
agtcactggc tcactgctgc aggtggagtg gtggacacta gtgccgcggt tcatctgaca 1200
cgtgtcgcca cgtgccgcca tggcagcacc tcagcccggc cggcgggccg actgacgtct 1260
tgggcaaagc ggcgagcgac gcaggcggcg aaagccatcc gatttgaccc ctcgctagac 1320
ctttcaagaa cgaacgctgt gctgctcaga tcagaccgtg tctgcctcaa agcgatgcca 1380
ggacgccacg tccaagcaaa gcacccgatg ccattgccac ctcccagcac tcacgcgtga 1440
gcgtgactat aaaaaacgca ccctctgcat ccgccccccgt ctgcctgccc taccgaatct 1500
ttcgccgtcc catcagccca gcaattcttc gctgttcgag gacccctcgg tttcgaccga 1560
agcccagcaa gccgaccaca caccgctgcc gttggttccg tcccaagaga tgggcaagtc 1620
ctggtcgctc atcagccacc tccacaccgt cgccgggtag tttcgctgct ccctcgcagt 1680
tcgttttccg attcctcctc gtccatctat tgggctcgct cgacgctggt tgcctgacgt 1740
gtgcgtttgc tgcttctttg tcttgtctgt ccctccctcc cgtttcgcag gccaagcatc 1800
accctgctgt accctctgta agttccttca tcaccctaat aatagcaggg accagtttta 1860
ccagtgcagc agtgaccgac cacggtccac ggcatacgtg agctgagagc atcgtgctgg 1920
gacatgcgtg ccgttggtgg tgctgcaggt acgcgtcggt gtgcgcgatg gagagcccgt 1980
ccaaggcgga cgacgagcag tggctgtcat actggatcat acactccttc gtcaccctcc 2040
tggagatgct cgccgagccg ctcctccact gggtacccgt gtggtacccg gccaaggtgc 2100
tgttcgcggc gtggctggcg ctgccgcagt tcaagggcgc ctccttcgtc tacgagaagc 2160
tcgtcaggga acagctccgg aactaccgcg ccagataccc gcgcaagggc gccgccgccg 2220
ccgccgtcgg cggcgacgac gacgaccata aggtgcacat agccaaggta ggtaaggatc 2280
ggactagatg aagccgtcgc gttttgtcgt tctagcgtat atgtgctgta gctgttaacc 2340
cacctgtttc ttcctgtcgg gctgggaaac aaaacaaatg ctaggctgag gctgagcacg 2400
accatgtgca gtgaaagcgt gtggagacga cgcaggagct gaccggagat catcacagga 2460
acttctgtag atctggtgtt ccaaggtgtt cattcgatgt atcatcctac gtatctctgt 2520
aactcttgtg gactgccggt gtcgcgtaat gtatctgaac tcttcgtgag tgcgatggaa 2580
ccccccctccc cttgcagcac gagatgcttc tgtaagaacg ataacatgta gacaagccaa 2640
gcgcctcagc tccttaagtg aaattctcta ctactactac tactataatg caccagcttt 2700
gtgcacggat gaattcatgc atcgtgccca tccccaccca aggactagat tcaagtcaac 2760
tacatgctat gcattacaac aagcatctgt tgcttgcaca aatggataaa tggcataaag 2820
ggaataaaaa ttgggctaga taaatggcat aaagggaata aaaattgggc tattgctatg 2880
gtgcagaaac aagacagtac tgcaatttgt ctagatttag tgcctaaaaa tatcgaagct 2940
gtggtcatag actaattctc ttccccccttc gagatactat gatcacacca tgtccctatt 3000
gcctgatata gtctagtgaa gtccaaacca tatatactca ccttctcaaa aggagccata 3060
tatatatact caccttctca aaaggatacg agatatgtag gacacgatga gatcgaaaaa 3120
caagttcagt ccatgtttga taatggcatt atttcagtaa gttgtcaaaa agtgttagtt 3180

catttgctac cctagtcgta ttaggtgaaa gaaaaaggat ggcacatgaa gattttgcat 3240
agactacatg aaattgaatg atgtaacagt atggaacaaa tttcatatgc tagttattga 3300
tgaatttaat tcttggatga actggtcggt gctaagtttt ttttagtatg gaataaattt 3360
catatgctag ttattgatga attcttggat gaactggccg gtgctaagtt cttttctaaa 3420
ctggatatgg cattaggatt accctgattt caccaaatca ggatgtcaat catcagaatt 3480
ttaccaaaat cagtatgtca gtggatgagt ataagatagc ctttaaaacc catcgtggct 3540
agacccgtca tggccacttt tcaatttcga gtaatgcctt ttgagttgac caatgcgcca 3600
aa 3602

<210> 82
<211> 2441
<212> DNA
<213> Zea mays
<400> 82

tattacggtt agtcaagact ttacagggga acctttggag acagacaaag aagtaagaac 60
ccaatgcacc tgtgcaaaga ctgtactttc cagtctttcc ctgaagaatt atgacacatg 120
attagaagac ggttcaacct ttcacacata aacacggaag atggctgtac ctataaatac 180
tagagaactt ggcataaggg aattcgcatg acctcacaaa acctctcctg atggaatttt 240
agtttatatc tggtggaata tttggaaggg gaatcagata acctttcaac aacaggagct 300
gcaaccgtcg aaagtagccc aactgatcag agataacaac ccacagttta gcctggccat 360
gcagcaaacc accgactaac cttgagcttt ctgggctgtt tttctacttc tttggtaggt 420
tctgcactag agtttttgtt tttattccgc ttgtaagttt ctctttctag tttctatcgt 480
gtaatatata ttatggcgaa gcactcgccg ttaattcaaa agaaagacaa gcatctgaat 540
ttgaatccgg caacctatcc aaattgtgcc gtccaacgag actttgatat atatagacca 600
ctgaatgata acgggcgtac cagtcgtgga ctaacaaaga caggtaacat ggcagcatgg 660
cgctcgccgc ctgtttccga gaagaagagt atatgtcgtt ttctgatcag tagccgactg 720
accggttact gttcattgtt cgctgttcat tggcagcagc agagcagtgg tagtagtact 780
ggtagtggta ctctggtagc tgatagcacg ttactaataa ttatcatacg atttcctaag 840
cggaatctcg agacgaacca catttttcat ccggtctatg agcgcgctca atcatcagtg 900
acagagaaca ctgtaggtcc ttaactggaa ctaacaattc agaaacaatc ctaaatgttt 960
attacactgg atgaatttta tttggataga tgatactacc gttcctgggc taaaaagcaa 1020
ggtaaaaact ggcctaacgc acgtgatcct cccatggtct cagaagaaaa ccactcgcaa 1080
aaaataaaaa aatcgcagca atgaggctgc tttcagttga cggtgtgcac gactcgctga 1140
acccagcgtg attttgtccc ggttcttgga gcccagcaaa aagggcgcct gaaattccac 1200
aggatgaagg ggaaaacggc aacaaaaggg actccgacat gcggtgcaga aagtgaaaac 1260
gcgagaaaag cgcccgtcct ttctcgttcc cttccgtgca gttgcaagtg ttgccacgcc 1320
gcgccgcgac acaccacgca ggaggcagcg agcgcaataa tgcgcacgca gaggagcaca 1380
gcggcgtcac tgctgcggca gtaatggccg cctggttcca cttccaccac ggtgtgcact 1440
agcgctacac cgaggccacc ttttcttggt tccttcaaca cgcctactcc ggcctccgga 1500
tcgggcacca cgtctgcccc ctgctcccgg aatctttgcc aagcggcgcc gctactgtcc 1560
ggcaaggcga ccccgtctct cggccggcgt tgatctggtg atgcgctttt cacggtgttt 1620
tttttttttg tttcactccc ccctccgtcc tgtgtgattg tgctgcagtt ttgtgccggt 1680

gctaacggaa accggaaagt ttgcgtcttt atgttactgt ggttgaggcg ggcggagtgg 1740
cggcgcgatg ggcatctcgg cgaggtggct caagtcgtta gttgggttga ggaaggtggg 1800
gcggcagcag tagcagcggc gaaaggatga tgcagatgtt ggtcgaatgg tacgcgccgc 1860
gccgagtttc ttccaatcat tatccagttc tggcgtatct tgttcttaaa ttggtggttg 1920
gtgcattcat ttcgcttatt gcctggacct atgtggatta tgcaagaatc atttttcata 1980
cccgagtcaa tccacaccac caaaagaatg aactatttaa tcaggcttct tgatcatatc 2040
ctgggaaggt ctgtactgat caatgttagt tatccatcct tgactgaatc gggattgcag 2100
tcggtaattc cttgtgcact ggttgaatta attttaattt ttttttactg aggagagcat 2160
attgttgagt aatttgttac ctgctttgtt caaagcttac ttaagtactt gacttcattg 2220
gcacttcttg aactatgaga acaaaaaatc ttggtggtta tgcaatcaca agagctttaa 2280
attaactgat ggatatgtat agcttagtat ggccaaacct tgccttctga tatacacatg 2340
gagaacatat ttttgtgcca cttccctcag actcagaata tacattgttt tgcttacgag 2400
ttgccaactg gagaaactgc agaaaaccga tgtcgccgac c 2441

<210> 83

<211> 2548

<212> DNA

<213> Zea mays

<400> 83

tcaacagaga gtgacaacaa tcaacaggtt aatggacatt tttccagcca tcgtgcttga 60
gtgaatgttt ttttgcgtgt gtgtaggtgt gcgtgtgtgt ttaattgata taaaataacc 120
caaccaaaca tctcctaaga tttccataag acagagaggt gaggaatagt attagttctc 180
aaatgaattc aatcaactaa atatcaccat catatcaaca ttaataatag aacaataaaa 240
ttttcatgat agagaggctg gtaaacttta ttttttcatt tgattccagt gtagaaaata 300
gtaatataag cagtaaattt tttcttaaaa aagagtggaa tattcacctt tttatataaa 360
ttagttattc ttcgaagcgt cacacacgga tgtcctaaag atattcttca actgcaacac 420
aagcattctg aaaaggttag taacaatcag attaatgttg taaaaatatg tgacacgaaa 480
atttaaagaa cacgaagtac aatacattgg taagaaatca aagacacata ttaaccaact 540
attagagaac atatacatgt ttcttctaat cctggttagg acataggacg agggtttcac 600
ttctatcaca atgtgctgca actttcagag ttaatcaaat ctgttattca gtgttatgca 660
gcgacaaatt ccaaatgtat aatcccacga tgattcatgt gactccattt cgtctgtaat 720
tgatgcgaaa ctgccgaatt gttactgctc ccataagtta attgtaccac gcttgatgaa 780
gacgttcacg tatcttgtgt tcgatttatg tagccgttat tcagaccatt gcagcgctga 840
gcttcaaggc aattagtttt gcccccgcgat gtgaggcagg tgatgacacg cgcccttcgg 900
gccgatgccg acgcgccacc gtgcagactc taccgaggat gagagcagcg atcgagagac 960
gtggagcgag gaggagactt taggagagga gagaccagag ccttaagcgg tgacacaccc 1020
aatgatcagg accacgcttg cacgtgtcag gtgcctctcc tccacgcgtc gcgcgcatca 1080
cactgatact gtcatccagc tcgacgctat ataaagtggt aagcatcttc gtcctcgggg 1140
ccgccccaac ggcccttgcg atcgccacca cgtcagcgcg gaggatgcac ggccaccagc 1200
agcagccgta cccatacggg ctgcggcaag acccgctgcg gcggatgcga ggtctggacg 1260
tgaaatcggc gctccaggcc agcccgcgca cgtccacggt ggccacggcc gcactcgtcg 1320
tcccgctggg tgccgcgctg ctgtgcgcgt cggggatcgc gctggctgcg accgtgaccg 1380

gtctcgcgct cgccacgcct cccctcgtga ttttcagccc ggtgctcata cccttcgcgc 1440
tggccgcggg gctcatcgcg tcggggctcc tcgcgtcggg cgcgctcggc gtcgcgggcg 1500
tctcggcgct cacgtgggcc gtcgggtacg tctggcaacg gcagggcgaa ggcggcggcg 1560
gggtcactgg gatggtggtg cagccgctgg accaggggtc gaagcgccat ggtgtccaag 1620
gcgccgccgc gttcgtgggg caccgccgcc tgccacggga catcgacgtt gctggtgaat 1680
gatggtttgg cggcgtgtgt gcaaactttt ctatgctagt ttagtttgca ggtgtgtatg 1740
gattttgatt tatggcctgg atggatcaca ctatgtagcg aacgatgtgt tcaatttact 1800
gttggaataa aactgcagat tggtgttact taataataac gtcctaggga aaattgaaat 1860
actctcagcg tcctaaaaaa ctttatattc tagaatttaa taaatactgt atgatgtttt 1920
tggagaatat tgttttttac acaaaagact ggaccttcca gatttattag cgcgtaaaaa 1980
taaaatttgc tacctggcac tctttgaaat tttgtctca actgacgcac tcagctcgcg 2040
gtgttcagtt acattcttgt gaagaaacat tttctagcta gaaaagtcaa atccccttcc 2100
aatccctatt ccaatctatt actcaaatcc ccttcaaatc ggatccacgt tgcggaatgg 2160
gacgccttcc tcccggccag ccgcggggct ctccacaggt cctgaaagtt cgtcaggaca 2220
gttgcatcca gggggctagc ccgactgtct ctagcaatgt tatatatata tatatatata 2280
tatatatata tatatatata tatatatata tatatatata tatatatata tatatatcct 2340
atattaggag cacttgactt ccaataacta aaaggaagcc caggccagac agtgcaatcc 2400
gacaggtcta gacgtctata aaagaaaacc cgcaatgcta gggatcagtt tttcacgccc 2460
catatcgatt cattagcgat ggcggttgac aggtataacg gaagcctgag cttccaatag 2520
ttaaagtaag ctcaggccga ccacccct 2548

<210> 84

<211> 2907

<212> DNA

<213> Zea mays

<400> 84

cttctatttt attctatatt atatatatat aattatatat atatttgcaa acattgttat 60
acatcggtga tgcagatctt cgcttcgcat gggtggcaat ttagtaacag aattgctcta 120
tgagttattg cttttagttt ctgtctttcc ccaactgcgt ttgagtttgc attgtcacat 180
gtggcctttt ttggttgtaa gacttgtttg atcggttgtg gtgggctgtc atcctttgga 240
tgtaggccgg aacctctttt cccattatct aaaaaaatac atcgtcattt ccagtgagac 300
ccagcggtcg tgtcatcaga aaagcggaca cgcgtcgcgt cgcgaggcca ggtgggttat 360
gcacggacct gctatgctag ccatgctcca ggctccagct ccacccgtcc accgactttt 420
cttctcaacc aggctgcctc ctcaccatgc acgacgacac gtgcgatgcc atcacacatc 480
cacactcacc tgttgcctct atatccaccg ccccaccgcc gtagcaccag aaagaaatgt 540
acagtctaca cagctgacca gagagctagt gcaagcgatc tagcaagttt taatcttgga 600
agaagccagc taggcgcgtg aatatggatc gcctcactgt gatggcgccg ccgctgctcg 660
tcctcctcct gctgctcctc tcgcggtgct cggcggcggc gtcacggcgc ggcgggggct 720
ggtgggagga gggcgagggg gagtggaggc cgagcgagga ggaagagaag ggcaaaggca 780
aagggagggg gctgttcctg ctgcaccggg tggagaaggt ggtggagtcg gagggcgggc 840
aggtgcgcgt ggtgcgcggc cagccgtggc cgccggcgtc cttcgcctgc cgcgaggggc 900
tcatgcacat cggcttcatc accatggagc ccaagacgct gttcgtgccg cagtacctcg 960

actccagcat cactctcttc gtgcagcggg gtacatgctg ttattatttc tatctatgcg 1020
cgtggcggag gctcgtgtcg ttcccggtcg atatgagatt gcaatcgcgt ggtattgtga 1080
tgatgagcag gggaagcgaa ggttgggtat attcacaagg acgagctcgt ggagaggaag 1140
ctcaagatgg gcgacgtcct ccacatcgac gccggctcca ccttctacat ggtcaacccc 1200
ggcaaaggcc agaggctgca gatcatatgc agcgtggacg cctccgatag ccttggcttt 1260
ggacttcctt atcaggtctc gccccctcct cttccttttt gcactctcgt cgtcttcctg 1320
ttcttgttgc ttgcaggcaa gagaacatat agtatagtac atgcaatccg cgattcccag 1380
cactgatgaa ccatgatcca acaaatccaa tacaggcctt cttcctcggc ggcgcagggg 1440
acccggcgtc ggtcattgcc ggttttgggc cgaagacgct tacccgtgcc ttcaacgtga 1500
gatacatata ctaatctaat atgaacattt cgcatggatc gtcgtgctct aattgacaca 1560
ggtaggcggt agcagctagc agtacccata acttgcatgt cttctgctag gccacctacg 1620
acgagttggc gcgtatcctg ttgccacgaa ccggcggccc catcgtgtac tacaccgcgg 1680
acgcggagcc agagagcggt gccgccgaag aggagcgcgg gcaggtcgac gggcacgacg 1740
gtgtgctgga caggggagcg cgacgcgagg gcgccggcgc gtgggtgccg ggtggcaggg 1800
gagacggagg cgacgagtgc ggcggcagcg atgacgcgcg tgaggcgacg tggtggtgga 1860
cgaagctggt taacagggtc gtcggagggg cggctggcgg cggcggcgct gcggaggcga 1920
acaggaaggg caagaagaag aagggcggcg cgccggagcc gtacaacctc tacgacagcg 1980
agcccgggtt ccggaacgct acggctggac cgtctccgtc gacaagcacc agtacgagcc 2040
cctcaagcac ccggacatcg gcgtctacct cgtgaacctc accgcggtgc gcgccctgcc 2100
tgaatcatat cgacctgccc gctttcttgt tggattactt tcgccgtttc gcgtcatggc 2160
agaggagggg ttgctgctgg taagctcgtc gcaagcgcag ccaagctgat catcgttctg 2220
ttcctccatc tgcatggcag gggtcgatgc tggcgccgca cgtgaaccct cgggcgacgg 2280
agtacggcgt ggtgctgggc ggggaaggca cggtccaggt ggtgttcccg aacgggtccc 2340
trgcgatgag cgaggtggtg cgcccccggcg acgtgttctg gatcccgcgc tacttcccct 2400
tctgccaggt ggcggcgcgg gccgggccct tcgagttctt cggcttcacc acctcggcgc 2460
gccgcaaccg gccgcagttc ctggtcggcg cctcctcggt gctccgtacc atgctagggc 2520
cggagatcgc cgccgcgttc ggcgctcgcg agaaggagtt cagtaagctg gtgcgtgcgc 2580
aacgggaggc cctgattatg ccgtcctctc ctgggaagga ggaggaggag catgggaaga 2640
aagggaggga gaaagaggag tcactgccga tggttgtcga gcaggcggcg gcggagtgag 2700
ctgcttgatc tttcagtctt ggactcttgg cttcctcaga agcgcgcgcg cgccaccgga 2760
aagtgtttct actcttgtta ctgcgccttg tcagtgatcg tgttgttggt tttttttttc 2820
gtggcgccgt ctataaataa aagtgctttg tgcgtttaca gctatgaaat caggattatc 2880
atccgtaact cgtaagctgg acgccaa 2907

<210> 85

<211> 5105

<212> DNA

<213> Zea mays

<400> 85

gccatcatca ccctaccccg agctgactcg ggccgcaggg acccagaccg gtgtcccatc 60
tggctagctc cgccagatag gcaatgaagg cgcccccgcat gctctgtgac gacggcggct 120
ctcagcctcc ttacggaagc aagaggacgt cagcaaggac ccaaccgctc cgacagctgt 180

ccctccgcca ggctccatcg ctcctccgac ggccacgaca tcgcaccagc tgggtgccaa 240
aatctctccg gctgccacga cggcatgtac ttagggcgct agctctcccc cgctagacac 300
gtagcactct gctacacccc ccattgtaca cctggatcct ctccttacac ctataaaagg 360
aaggaccagg gccctctcag agaaggttgg ccgcgcgggg acgaggacga gacaggcgct 420
cgcttggggc cgctcgctcc ctctcccacg tggacgcttg taacccccta ctgcaagcgc 480
acccgacctg ggcgcgggac gaacacgaag gccgcgggat tcccacctct ctcacgccgg 540
tctccggccg cctcgctctc ccccttcgc gctcgccctc gcgctcgatc catctaggct 600
ggggcacgcg gcgacactca ctcgtcggcc caagagaccc ccggtctcga aacgccgaca 660
atatgatata tagaaactat aatgatacac atgcaattaa ctagtgctga taatatattt 720
acactgtaga tttttccgat aagtagaaac caaatatttc accgccacaa aaggcttcat 780
cccatcagtt acccatgtag caaaaccaaa atacttgtgt gcgagaggcc aagagcccaa 840
accaccgcca aggaaacccc cagcccacca ggcgccttcc tctataaata ccctaactcc 900
ggtggccgaa gttcctcacc cattcactca ctcatctcaa ggcctaggta gcaccgtagc 960
agctactaga agcagctagc cagaacaact cgtccatggc gatggcctcc gcggcttgct 1020
catgcacgga cggcacgtgg tgggtgtacg cgctcccggc gctgctcggc tccgacaccc 1080
tgtgcgccca cccggccctc ctggctggcc tgatctttct ggccaccgtc tcggtggctc 1140
tgctggcgtg ggccacgtcg ccgggcggtc cggcgtggac gaacggccgc ggccgcctcg 1200
gcgtcactcc tatcgtggga ccccgtggtc tgcccgtgtt cggcagcatc ttcgcgctgt 1260
cccgcgggct gccgcaccgc gccctcgccg agatggcccg cgccgcaggg ccccgggcca 1320
aggagctcat ggcgttctcc gtcggtgaca cgcccgcggt cgtgtcgtcc tgcccggcca 1380
cggcacgtga ggtgctcgcg cacccgtcat tcgccgaccg ccctgtgaag cggtcggccc 1440
gggagctcat gttcgcgcgt gccatcgggt tcgcgcccaa cggcgagtac tggcgccgcc 1500
tccgccgcgt cgcgtccacg cacctattct ccccgcgccg ggtcgcctcg cacgagccgg 1560
gacgccaagg tgacgcggag gccatgctcc gctccatcgc cgccgaacag tcggcctctg 1620
gcgccgtcgc cctccgcccg cacctccagg ccgccgctct caacaacatc atgggcagcg 1680
tcttcggcac gcggtacgac gtcacatcag gcgccggcgc cgcggaggcc gagcatctca 1740
agagcatggt gcgcgagggg ttcgagctcc tcggcgcctt caactggtcc gaccacctcc 1800
cctggctcgc ccacctgtac gacccaagca acgtcacccg ccggtgcgcc gcgctcgtgc 1860
cgcgcgtcca gaccttcgtc cgtggcgtca tcgacgagca ccggcgccgc cgccaaaact 1920
ccgccgccct caacgacaat gctgacttcg tcgacgtgct cctctccctc gagggtgacg 1980
agaagctcgg cgacgacgac atggtcgcca tcctctgggt aaagttcaaa tcgatcgctt 2040
tcctagcttg tttaactgcg catacttctc agttctcaac tgcgcatacc tgtcggttct 2100
acagttttgt gtcgggctgt cggttgttcc cggaagggaa aaaaaagaac aaagctctgt 2160
cgctgaaaaa aacatactgt acatgcatat aatttgtttt tgcaggagat ggtcttccgc 2220
ggtacggaca cgacggcgct tctgaccgag tggtgcatgg cggagctggt gcgccacccg 2280
gcggtgcagg cgagggtgcg cgccgaggtc gacgcggctg tcggtgccgg aggttgcccc 2340
accgacgccg acgtggcgcg catgccgtac ctgcaggcgg ttgtgaagga gacgctgcgc 2400
gcccacccgc ctggcccgct gctgagctgg gctcgcctcg ccaccgccga cgtgccactc 2460
tgcaacggca tggtggtccc ggctggcacc acggcgatgg tgaatatgtg ggccataacc 2520
cacgatgccg ccgtgtgggc cgacccggac gcgttcgcgc cggagcggtt cctgccctcc 2580
gagggcggcg ccgacgtgga cgtccgcggc gtcgacctcc gcctggcccc gttcggcgcc 2640

gggcgtcgcg tctgccccgg caagaacctg ggcctcacca ccgtgggcct ctgggttgcc 2700
cgcctcgtgc acgccttcca gtgggccctg cctgacggcg cggcggccgt ttgcctcgac 2760
gaggtcctca agctctccct ggagatgaag acgccgctcg tcgccgcagc catccccgc 2820
accgcctgat ccgtcctgcc gccgacgcgt cacgtcacgc gttgtttgca tggatgatgg 2880
tatctttgtc tgtctgtgtg gtcttcgcta aagtttgctt cttctcgatc gtcggttcgt 2940
tcgtgcctcc accttagcct agggtttggt ttcttgcaag gtagtgagtg tgtcttagtc 3000
tcaccatcac cggggctcca attttggaaa gctgcgtgtt aggagttaac ccctagacat 3060
gtttgcgtct tgatcgccac cacccatcag tatcagcgca gaaactacat atagatcagt 3120
gtttgtcgac cagtcatgga agtcgtgtgc tctcaagtct gatgtattat atacatatat 3180
atgtattgta atgtgattat caagaaccgt gctatttaca tattgctgtt taattgaact 3240
aattaactaa atgtccctta tatatatgtc cagatcggcc actaattaac tagttatttt 3300
acaaatattt acaagtcggt cgtcttaaga aatcaacgca cttcaacagt attgagccgt 3360
ctggtgtcaa atgagacttg cgttcactaa agaacttaat caggttcttt ttgaagcacg 3420
tatttcgatt gcctgctgct ggctgtaata tatcaattca agcacttaaa caggttcttt 3480
ttcagtgcaa caagggatgt agaggtgcca tggtttgggt aagcactcat ttctgtggtt 3540
tatagttcgt gagtagttcc ttatgtgttt gcctgcccct ggttgtaata tatgataatg 3600
cagaacaatc tcttgcaata ctatagggcc ctccattcta atttatagat tgttttggcc 3660
tagtcgatta actgttcatg aacatcttta ttggatggac taaccaacgt cggcctaagt 3720
ctaggggaaa aggcccaacc agcgccatga gtagatcagc ctgcaccagc gtgaacagtg 3780
aaggcaggcc caaccagcgc cagcatcaga tcagcccgca ccagcgcgaa cagtagacga 3840
gtgaatagta accgagtgaa cgaaggcaag agctaaataa atatgacagt ttctatattt 3900
gtatatttat cttaggagtc aagttgatta gtttctatat ccattggatt agagtttagg 3960
taagttaaat atgtaacttg aggaccaaat tgatgcctgc caaggctata taaggaccta 4020
gattatactc tcttaaattg agtttcaatc caatctgatg acaaaatcac ggcacagacc 4080
accgaggtgt cctccctgaa agtcgaccag gttgtttgta ttcacatcgc cgtcaacaat 4140
atgcagtccc aaacaagtgg atggtaagca caggctcact acggaccaca tcgcctctgc 4200
cagcaacacc tacgcaaggt acaaggttgt cgtcaccttc atcaacagta ctaaccacaa 4260
gctatgcttt tcccaagtag gacggcatga gaataatggc gatgcgagga gagggctcta 4320
cgggctcact aaggtggaaa tgagaagaag aaaacgagaa gggcatgaaa aggtcaaagt 4380
tatatggcga cactttaaat gagaactgaa aaacaccttt aatacaagtt cttagacgtg 4440
tgagcgtgaa ctccaaaggg aaattgaaag tgaaattggt actaaaaacc tttagggtcg 4500
atttggtcac aaggggctca cgggggattg gaggggattg agtgaaaaat aaactaattt 4560
tcctcttaat atccttcaat cgccccgtga ttcatggaca ccaaatcagc tcttaaagag 4620
aaatttaaag tgaacattta gtatcggttc atgaaaaaac tgataccata gggggtctcc 4680
agagagatgt cagactgaaa gaggggtgtt ctttagagag attagagagt aaacaaagca 4740
aataagtaat tcatcttgaa gattacggtg ccaggctcct ctatgttctt ggcaccggct 4800
taaggggagt gagtgtttac aaagtaggta catcagcatt tgttgaagac accaactgag 4860
atgctgatgt taagttatcg gtaccggtta gagacaccaa ccaacttcaa ccgatatcga 4920
caacttaata ctccctccgt ttcgttttag ttgtcgctgg atagtgtaaa attgaactat 4980
ccagcgacaa ctaaaaaaac ggagagagta tcagtattag ttgatatctt caagcgacac 5040
taatgctctc tacgggatcg cgatggttaa aactgatact gacacgtact tgagctagta 5100

tcggt 5105

<210> 86

<211> 4000

<212> DNA

<213> Zea mays

<400> 86

cttccgataa aaatatttgg aaccgcatcc tgcatattta tttagaaaca ttgcatcgat 60
aaccgttgca gagcaatata cagggtactc accaactaaa tccataccat acagtgacag 120
gtgaaattgg aatacttgat cagcttgaat aacagagagc aaagataaat ctagccctgg 180
taataaccat ggctcacatc ttatgtacaa gagccaattc taaagacaca gctgcgggat 240
gtagcagtta ggcaaggaaa gaaacaaaat taccagctaa ctgctaagtg tcaaaacatt 300
gctttacact aaagggttaa atatttgcca agaaggtagt gggcacctct cctctgcaag 360
cagcagcata atctagggcg aggcttggtg cagcttcctt gtaggggcat atgaacttat 420
ccatgaaggc actctcactc atctggacag cagtatagta gctccggtcc tgctccaaca 480
atccattttc cttcacgcct agtttggatc actgtaattg aattccattc taataatagt 540
aatttagaca tatatcaatt aagataattc acttttgtac aaaatatatt tgtatactat 600
tattagcaat atatcctaaa tatttatgtg ctacattttt actatagagg agtagaacga 660
agagtgtcat ataagttata gattagaaac aaattctaat catgcataaa atcatttccc 720
atcctccacc ctatgaattt gagataggct tatatctgaa ctttagaaag tggtggaatg 780
tcaaattcca aactaaataa gttactttat tgagtgaatt ctaattcttt tgatttgaag 840
gaatccaaac gccccgtcag gtaattcaca acatagtgtc ggggcatgag gcggcgttcc 900
aagctatatg taagcagagc aggcctgtga gcaatgtact ctggctccaa cccaaccttg 960
gtgatcagga acgcggatcc gggcgtcgcc gggggcgcga ccctgtgctt gccaaattga 1020
aaatttagca aagtattttt tttaccttgt tgattatgtt ggataaccgc atgtccctaa 1080
tttgaatgat attgctcctt atctctaaac gaccaactgt agcttgttct attgaaaact 1140
catcagaata atccatagag ataacatcac caacaaggac tgaatcatcc agagatgcat 1200
caatggccat tggagctagg gaagtcggcc taactacctt caacccgact tggccaactt 1260
gtacagtaga agacaccatg gaaatcgatt taacggactc taagggagtg tttgaataca 1320
ctaaaactaa tagttttagt ggctaaaatt agttggagac atccaaacac cctagctaat 1380
attttagcta ttaactattt ttagtaaatt agttacaagt tagctagcta tttattagct 1440
agctaattct actaacaaat ttttagctaa ctaacgcatt caaacactgt ctaaacaggt 1500
aatctaggca cgcgaagtct gactggccag cccaaactca gcctttgtag tagagagagc 1560
cgaccttggc ggcctgcacg acatttttga acttttttta tcggtttggt cttcattact 1620
ttatattctt tgtcaaccaa atactccctt cctcttaaat tataattcgt ttgatttttt 1680
ttatgtaaag ttttatgtat agtccaccta gttaacttta aaagttggtt agtctagatt 1740
ttatggaggc cgactacaat agcaaatcta acaataaaaa agttagctag atcagatttc 1800
aagaacagtc tgaccgattt tgaagatttg attcaggtta attctacgat tttgaagatc 1860
cgacctaacc taattttacg ttcagactaa agacatgttt gtttaagatt atgatatgtc 1920
tagttatata atttaactta ttttaaacta acacttaatt taaaataagt tagattatat 1980
gatcagaata ttatatttat ataattccaa acaaataact ctaatatcga ttctggttta 2040
tattgtataa tttttatgtt gaatattacg gcgcgccaac aaattgcgat cgttatgtaa 2100

tcatgtatac gaccgactgt actattcata aaatataatt caaaatagga gatgctcgag 2160
atagcctggc aagggaggag gtatcggagg cggcgtcgcc tgccgcgagc gcaccatgcg 2220
tcaacgctca ggacctctcg caggcgcgct gttggatcgt gagcaagcac gcgtggcaaa 2280
tggcatgcat tcctctcgtg caagtgccga gctgagggtc ccgacgcggg gggcaggacg 2340
tggcagagcg ccgcccaggc ggacgccaag tgccgagcta ccagggcacc ttctttattc 2400
cgtccttgct cagtcacacc tcgctctcgc tcactctcgc cgtccgcaca gccgctcatc 2460
gtctcccact gcctgccctc tccctgcgcc ggccggtgcc ccgacgcgcc atgtcgtaca 2520
acaaggcccc gtccatcacc gccgagacca taaatcccag ggtttcttct tcagaaatca 2580
tatatatgtt catccattgc attcttccga tttaaactga aaagcttagg tgacgtacgt 2640
tgtcccttcg tttgaaggtc aagatcatca gatacgcgcc ttgcggggag atcgtcaagc 2700
acgcagcggt aaattgcacc actgctgtta attcatgctg ctttacgacg acccctcgca 2760
cctttttttt ttgcaaaatt acgatgacta ataggaggac aaaaaatatt gcctttcgct 2820
atgtgtctcg ttcagcggct ggagcaggag atagaggaga accctgcttc tatccctttc 2880
caagaggtga aaactcctcc cctgtgcagc aagcaagtgc gtgcgtgcgt gtatcacaat 2940
ctatatatca cctaacttga ccagccttcc atgctgtcca acagataata tactgcaacc 3000
tcgggaaccc ccaggtcctt ggtcagccgc cactaacttt ctttcgtgag gttaccaccc 3060
ttaattaccc tcagaaacac aaaagaacaa gacaacgcaa gtgtgtttga tggtcgaaat 3120
tgctggctcc aggttctctc cctgtgcgac aacccagccc tcatggacag agatgaagct 3180
cgtgccttat tcaggttgga ctatgcagct gagctcgctt tactgtttac tgtactatta 3240
cctccgtcct ttttttttat ttgtcgtgtt ttagttaaaa taaactaacg gacgacaaat 3300
attcgagaaa gtagcttcat aagctgagta tgatacatag tttggcagaa acttgactct 3360
gacaatgttt ccatctactg tgtcgcagcc cttgtagcat aagaagggcc aggaggatcc 3420
taaactcgat ccccagcaaa gacactggtg gatatactga ttgccgggta cccaactgaa 3480
actgcttagc tttcttataa ataaacaaac actgggatgg tgtatatttt ccccttcaca 3540
cttactggtt acatcgttca atcacacttg cttggtgtga tcagggaatc aaatgcctgc 3600
gtcaagtagt agcagatggg attaccgcaa gagacggttt tccatcgaca gcggacgata 3660
tctttctgac agatggagcc acctcagcgg taagactctg catttcagaa cctacatcta 3720
catgacgcag cacagatgga actggatcct ttacatacag atacgaacat cagtgtcaat 3780
accttcagct cgaccgcggc tcctttctgc cccttttttg cagatcaact tgatgatgca 3840
gatactcatc aggtctcacg aagacggcat cttgagccct ctacccgaat acccccttgta 3900
ctcggcctcc atcatactgc acggtggaac gatggtacag cactagtgta gttcaaaaag 3960
atagccaagc aatatctgcg aaacgcagta gaagtggctt 4000

<210> 87

<211> 2865

<212> DNA

<213> Zea mays

<400> 87

tcatcggtac tcgcgatgtc gtgcgcgtcg acgactacta tatcaaggac tatgacgtca 60
tcttgacacc agaggtgaca cagaggagga ccaacaacaa taaccactcg ccaccgtcgg 120
tcggtctgcc tttaattctc ttcgcaaaca catacacttg cttttttgtt taagcaagcg 180
tgtatgatgg tgcgtacctg atgactgaca atgtacgagg gaacttctac cggcaggtgt 240

aacacgtgct cttcaatgat aagatcatgc aaatcgtgtc atatatcgaa gcctgcatga 300
tactgatggt tgaaatatag tagtgaaaca gctaaattaa aataacaaaa tttatgtatg 360
gctaggatta caaataaatt ataaaatatt tttttataac aatataagac acattttgta 420
tataagttat tatattatta tatgtttctg ttgcaacgca cgagtactca cctagtatat 480
ccataggctt ttgatcctct acgtcgagtt tttgcttgca tgagagtcga cctcttctct 540
tttatccctt cttctctatc ttagcatttg ctcgatcaac tgtaaactaa ttattatacg 600
tacttcaagt catattgaaa ccaatggcat ttaaaaaagt aaccatctga atggaaatat 660
caagctaaat aaagaagtta cgggagtatg tgctgagaaa acctagacac attcagagaa 720
gttaccaagc aagccttaaa taacaatttt tggtgcttta ttgcagatag atactcctat 780
aggttggaga gaagggcggc gatgctaatg caccatgcat gtgtgtctgc ccatatactc 840
tatcttatct gaatcctacc aaactcttta atttgactct ttatgaatgg aattcccata 900
tgaattctat aacacaacag tacgacgtct actggagcat actgccaagc acgctagccg 960
acggctcggc gaccgtctga ggctgtccat gcatgcaaat gtgcacacat gtacgcccct 1020
tttagcgctg gcatgcaccc acgaccacga cacggctccc gtggccgtga atctgacatc 1080
ccgggactcc cgcctccggc cgcgcgtgcc gtgtcagcct ccatcgagcg gtgtactcca 1140
tgcaagcctc gacccgggcc acgtaccccc ctcccctccg ctacgtgtca ccgctctcgt 1200
cacctatata tggcggtctt gtccagtctt tccacttaac tactccgtca ctttgtttgc 1260
aaagctcctc ctcgatccat cgatcactgc accggccggc ggcaccgcgc tcgcaggggc 1320
tagccaacga gacggcagca atggcggacc gtgaccgcag cggcatctac ggcggcgccc 1380
acgccaccta cgggcagcag cagcagcagg gaggaggcgg gcgcccgatg ggtgagcagg 1440
tgaagggcat gctccacgac aaggggccga cggcgtcgca ggcgctgacg gtggcgacgc 1500
tgttcccgct gggcgggctg ctgctggtgc tgtcggggct ggcgctgacg gcctccgtgg 1560
tggggctggc cgtggccacg ccggtgttcc tgatcttcag ccccgtgctg gtccccgccg 1620
cgctgctcat cgggacggcc gtcatggggt tcctcacgtc gggcgcgctg gggctcgggg 1680
gcctgtcctc gctcacgtgc ctcgccaaca cggcgcggca ggcgttccag cgcacccccgg 1740
actacgtgga ggaggcgcac cgcaggatgg cggaggccgc ggcgcacgcg ggccacaaga 1800
ccgcgcaggc aggccaggcc atccagggca gggcgcagga ggccggcgcc gggggaggtg 1860
caggtgccgg cgctggcggc ggcggcaggg cttcctcgta agcaagtcat ccatgcatgg 1920
attatggata gatgcgcgcg tgcgtgtcta tcagtatcag cagccagcag ggtcgtcgcg 1980
gaatgctgtg ttcctgtacg tgtgggtgac cgtccttccg tccttcgtct ttctcccccc 2040
gagtgtgtgt tacgtatgtc ctggtgttcg tcgtgtgtgt tcatcgccgc tccagttgaa 2100
ttccggtgtc tgttcatcgc cgctccaggt cgtagatgtg aatatacttt gctaggggaa 2160
taagtgataa gtctgtctgg aaggtaatgt ttgagctttg ctagtgtggc tgggcactct 2220
ggtcactggt tgtgttgtgc atgcatcagc tgtatgatcg tcgtctgttg tggaaaattg 2280
gtcaatgtat tctcttgctg aataatttgt gacatctaat tgttatgtat cgtctctttg 2340
ctgaataatc agtttctgat ttatcttgca ttattttgag aatgaaatga tgatggtagc 2400
tcagtctgca gctattcggg cgcacactgg gtggctcact gtttggccac ccagttactt 2460
acgtttagtg ttgtggccca ctgttttgcc acccagttac ttgaacgttg ttttatcgta 2520
aatttgagta ttattaagag aaacattcaa aagaaaatca tctccaacga actctatgaa 2580
tgcctcttta aattttattc atcaaccttt gctctatact tttaggctat ttattcctca 2640
ctctctagtt ttatatagcc tacttcattt ttatatagct tacttcactc gccttggatt 2700

agtctacttt tctctcgtgg tagagagtaa caaaattaat aaatttggtt aatcttatga 2760
gctgagttgc tctatttcat ctcctattta ttttctattt catcttctat ttattttctt 2820
tttaagctta ctacaaaaat agtgtcatct ctcttattta ttttt 2865

<210> 88
<211> 3425
<212> DNA
<213> Zea mays
<400> 88

ctcacacaaa tctaaatagt aaagtagatc tggttacaga gcagaaattt gaaagtgtca 60
tagataggct gaaaatatgt agtcacgagc aacacattac tacttaaagg ccatcaatac 120
tgttttttct tctccaacct atgtaactat gttattcttt tcctccttaa atgtaaggca 180
aacctcctat cctttttctt tcaaattcat tgcattaact gagtggttct tacatgtaga 240
aatgaagttg gagcaaagag gcttggtgcg gaaccgaaaa tttgttagtc tataaatcat 300
tagagggcgc ctgaagttcg ccaaatgttc tagtagattt tagtaaccac ccacgctagc 360
tgactcgacc accgtctgct ggctggcgct gtccatgcac atgtacagcc cctggcgctg 420
gcacgcaccc acggcatacc tcccatgact ctgacagccc gggactccct ccgctcgtgc 480
cgtgtcagcc tcactcgagc ggtgtactcc atgcaarcct ccacctcggc cacgtacccc 540
ctccccttcc ttacgtgtca ccgctctcgc tccctatata cggctgcctg gccaggcctt 600
tccacttcac tactctctct ctctgcctgc aaagctgctt gatccatcat cgatcacatc 660
actccaccag ccgcagcgca cgaacgtacg actcatggca gatcgcgacc gcagcggcat 720
ctacggcggc ggcgcctacg ggcagcagca ggggggcggc cgcccgatgg gtgagcaggt 780
gaagggcatg atccacgaca aggggccgac ggcgtcccag gcgctgacgg tggcgacgct 840
gttccctctg ggcgggctgc tcctggtgct gtcgggcctg gcgctggcgg cctccacggt 900
ggggctggcc ctggccacgc ccgtgttcct gctcttcagc cccgtgctcg tccccgccgc 960
gctgctcatc ggcacggccg tcgcggggtt cctcacgtcg ggcgcgctgg ggctcggcgg 1020
cctgtcctcg ctcacgtgcc tcgccaacac ggcgcggcag gcgttccagc gcaccccgga 1080
ctacgtcgag gaggcgcgcc gcaggatggc ggaggccgcg gcgcacgcgg gccacaagac 1140
cgcgcaggcc gggcacggca tccagagcaa ggcgcaggag gccggtgctg gcactggcgc 1200
cggcggcggc aggacttcct cgtaaataag caaatcatcc atggatgcgt gcgtgtcaat 1260
gtcagcaggg tcgtcgtgga atgctcttct ctgtacgtgg gtaaccgtct tttggtcgtc 1320
ttccccgagt gcgtgataaa tatcctggtg ttcgtcgtgt gtggtgctgc cctaggctgg 1380
ggcctggggt cggctgtgta ccggtatctg ttcatcgccg ctccgtcgcg ttcggagatg 1440
tgaataagtg atgtgaaggt taagtttcag ctttgctatt gctattgtgg cttgtgcatc 1500
agcggtttgc tcgttgactg ttatggaaat aagcattggt gaaggtattg tctattattt 1560
gctgaataat tttgttatat atgataaaat tgagtttact ttttatggaa ctttgcaaac 1620
ttaatgctaa ctaaaaacaa gtatttacac agaagattca atatccttca atatcctatt 1680
tattcacgat aaaaccaaaa aactaatatt caaataagta tccgtttttt atctctgatg 1740
ccaaagctgc gaggaaaacc catatttctt tgctgcagac acgactgctg agcttttttt 1800
aaaatttcca atgctctaat tgcagtatac agagcatgcg tgcgaactgc aatttgtgca 1860
agagcttgga acctgtgtaa atttttgact gtagcagaaa aatcacacaa attatacgga 1920
ttgccatttc gttgtaaata gtcctgcaat tgaataaaaa atgttgctga ttgttgtaaa 1980

ttcagagatg tacaaacgtt gccgttccac tgcatccagt aggtgatcgc tggtcatacg 2040
aattttacac ttaaaccaca gatccaaaac ctgttctcca aaayaatcta tgtttcagac 2100
ggcagaagac attcaaacaa atttgcaaaa agttattcag atggcaccaa atgtgaattc 2160
agcaataaca tcatctgtgt tttcccctgc aaaattaaaa gttcagaaaa aaacagtgta 2220
tggttcagac ttcagaggaa cagtctatta ctttgaatat taccatgaat agctgagaga 2280
aggtgcataa ttcagacatt tcttccttgt aattgaagaa atagtttgta ttgtcgcacg 2340
cttccacatg aagttaaact cactgctcta tctacctaac caactcagct aataatccct 2400
tatctttaag cattcccctt ttcttatctt ccttcttttc aaccttatac ttttttgtta 2460
tgtcaaattc tattgcgctt aaaacttttt tgtttgcacc tcttttttc ttcgggctta 2520
tttcgttaga gcatctccaa tcgtgtttta aatcaatttc ttatcttgaa atatatgaca 2580
tattttataa tttttatcaa aaaaaaatga tacaccatta agtaactcaa atatactata 2640
ccgcggacta gtatccttgt tttctacacc attattatca ttcctaataa tataatttac 2700
tttttaaaat atatgattta tggtttaatt gttggagcac aatttgtttt tagtgtacta 2760
aacaatttaa ataatgtaat attttgattt ttggacgcta ttttgagtca ccttgttggc 2820
tctttattta aaattaaata gggagagatc taattcttct cattcctcty taatactctc 2880
caaccgaata aagccttagg attgttgtat tcgaagtcaa tagggggagc aacacctgtg 2940
ggaacaatga ycggatccat ggcactgaga aaactactca tttcttcttc ggcttgctta 3000
ctaagcatgt aagtgtgccg tttattaata ataagttaca cactagatga aaacagggat 3060
ggtaacggga aattctttgt cagggaatag ttcaccattc ccgtccccgc gataaatttg 3120
tttccacgcg catctacacg aacgtttgcg gggacgtttc tttatcatcc ccgtttcccg 3180
cggggataaa tcaaattata attaagaagt atgtactccg ttattaatac aaaacattgt 3240
cacttatgct catcatcata tgtaagaaat tattatgagt acatcaaatt aagcatattt 3300
atacaataaa tgatctattg ataagataat tatttatttt tatcattaac tattacacga 3360
aacatcatca cgtgtaaaat ttaacggggt ctgcgaggaa caggaatggg taatgcttcc 3420
tcaac 3425

<210> 89

<211> 5254

<212> DNA

<213> Zea mays

<400> 89

tctttaggat caatgtcatc caatgtttat gatgccagaa gtaacatgat tggagagatc 60
gagaacagat caacattcct cttagctgta agtatttttt gccggcacca cctggcacaa 120
gaatcctgcc aaatttatga gttccactat taatacataa ttataactag taaccaaatt 180
ttaataatgc aaagtgacaa atgttttata aaaacatgga atgctccagg taaaagctga 240
tgtggagaca caaggagaat ttgttgagtc tctagcaaat gaggtccgag cagcaagctt 300
tgtaaatatt gatgatgttg ttgcatttgt aaattggttg gatgaggagt tatctttctt 360
ggtaagcagc ccgaacaggc cctccatact acacctttgt tacaaagtac cagttattgt 420
acttatgaca cattttcaca ttttaggttg atgagcgagc agtgttaaag cattttgatt 480
ggccagagag caaaacagat gcaataagag aagcagcttt tgaataccag gatctgataa 540
agttacagaa caaggtttca tcctttaccg atgatccaca acttgcatgt gaataagctc 600
tcaagaagat gtattctttg cttgaaaagt aagtgtctca tcagacctag tattctctct 660

agcatagtcc ttcagagtga aacagcaaca tttttgcctg gttcaagata tgtatgtgat 720
gaacataatt ttactcaaaa gtgatggttt cattgtacta actagcatca atacttatgc 780
cagagtggag cagtgtgtct atgcactact ccgtacaaga gacatgtccc tcacgctaca 840
aggagtatgg gatcccagtt gactggttgt ctgattccgg agtagttggc aaggtgtgca 900
tctctagcat acactccaat ttcgccaaac atgtgttaaa aacatgcatt tttaattcct 960
tatgaaggtg ggtatgattt tactcagagt atgcacctaa atgaactcaa taatttattt 1020
gcatgttgga tatatcaaat tggcatctgt tcagcttgcg aacaagtata tgaagagggt 1080
tgcatcagaa cttgatgcgt tggaaggcac tgagaaagag cccaatagag agtttttgct 1140
tctccagggt ggcagggtgt gaggttctct ttccgtgttc atcaggtaaa atcctcacgc 1200
caatattttc agaacccagg tacttcacag gtgatatgaa tatttcgaat cagtaactta 1260
ttcatctacc ttctgcagtt tgctggggga tttgatgcag aaagcatgaa agcttttgaa 1320
gagctaagaa gcaagatgag tacacataaa tctgctccac agatatctga aggctgaagc 1380
atgagttgaa cacaggattc aacacacatc tggtcttact tgctggatac tgatttttag 1440
ttgtgtacaa aagagaagtt taaattccag tgtggtgtca agcaagttgt atatgctaag 1500
agaatacaag tcatcaggtt atgttcattt attttttatg aaagtaaggg caggacaggt 1560
tatgttcatg catataacag ttttagctca agtgtatacc ttttcatttt aaaggctgta 1620
actgtaaaca ggcagctcca gaaaatgaat cacttgggca tgtttttgtt gctacgcata 1680
tactactaat aaaatgtcaa aagcgtccat tcggttagtc cggtacccat ggagataatt 1740
cgtctgaatg aaccttgccg tctttgttaa aagaaaaaaa aattactttg tgcctcaccg 1800
cattcgatac tttgaagaaa ttgacccgtt accagttacc acccacgcac atacagtgct 1860
agaacagcag atctgacttg aagaagcata tatcaagaca aaagtccacc tgaacggaga 1920
cagtcgaggt tgtggcggag agctccacct gaacgccgaa gtggaaccga acagttccag 1980
tggttggccg tggcgagctg gccacctact agtctccggg gagggggctg gctccagcgc 2040
ctccgagcgc ggtgccagtg tcgccgtcgc gtgggttccg gttccggccg ccgccgcttg 2100
ccggggaatc caaacggcag acggggtcgc cgtgaccgtg aatcgtgttt acttgggcct 2160
tatctaggcc acaacgtttg tgactctgtg agcttattgt ttgggcccat ctctttggcg 2220
tgtctgagaa cgaattttat catcaagccc acgaagccaa accaggatcc tacccaccta 2280
gcacatacta gtgaagaagg taaacaaagt gtattttcat gttaattcac atgcgcgtgg 2340
tcgacaccac accacacgac ttaaaactcg gtgttatgtt ctacgaccac aagagccatg 2400
ttaattttaa gccgtgtctg cgccatgatc cgaagagcgc gtacagtgag agaagaaacg 2460
cacggccgcc agcagctgcc gcgggagcga ggcagcacgt ccgttggtcg gcccgggcgg 2520
gagcgaggca cacgtcgcgc atgcacgcgc ggcgcccgga cacgtgccgg cgcgggcacg 2580
tccctctccg cgccttatac tcctccccgc catttcccag cagccacaac aagacagaga 2640
gatacccgat cgccaccgcc gaccggactc ccctcaaagg cggccacagc acagcgagca 2700
tgggagaccg gcatcagcat gggcctggca gcggcgtgga ggacccggcg acgctgctgc 2760
ggcgggtgca gacccgcgcg cccaactcga cgcaggtggt ggggttcctg acgctgctcg 2820
tctctggcgc cgtgctgctg ctgctgacgg gggtgacgct gacgggcgcc gtggtggcgc 2880
tcgtcttcct gggccccatc gcgctgctca ccagccccat ctgggtgccc gtcgccgtcg 2940
tgatctccgc catcgtcgcc gccgcgctct ccgcctgcgc cttcgccgcc gccgcgctcc 3000
ccgtggccac ctggatgtac cgctacttca cgggccgcca ccccgtgggc gccgaccggg 3060
tggactacgc gcgcagccgg atcgccgaca ccgccagcca cgtcaaggac tacgcgcgcg 3120

agtacggcgg atacctgcgc acccgcgcca aggacgccgc gcccggcgcg taggcgcgcg 3180
ccgcccggcg tgcatgtgct gcaacccccg tccgtggtgc cgctagctat aggtcatcat 3240
tgctgtcgtc gtcgtcgtct tagttcctct tgcattgcgt gtgcgtactc tgctccaatc 3300
atccccgcc cgtgttgtca agcaatttct ggtcctcttg cttgctttct ctaggagatg 3360
ctgttcattt cgatctcttg gcgcaatttg atctttcgct tggtgtaatt aggtcattag 3420
ttaaatcata agagactcgt gttcatggtc tttctcacaa ccttaaaaca actaattgtt 3480
gttgaaaaga atatatatag gtaaccttgc tcagttttcg tcgaacagaa tgatggcaac 3540
ctttcttctg caaacagttg cagataatgc ctaggatttt tgttctctgt gctgaatcat 3600
ctatacaatc cgacgtatgt actcacctcc cgaggttgct ccatcccaaa actggagttg 3660
gcatagcgtg gtccccgtac gatgcataac aagtgtcata cagaaggttg tgtcatgtgt 3720
gtatctagtg tgccacatgc tataatctct gtagtctttt aattaccctt tggggtgtta 3780
aagggcgatc agagtgacat cagtgatcta aggtacgtag accgcataaa tggtcgtgtc 3840
tcgtgaaccc aagcacgact tattatggac ctcagactga cttagtatgg ttattgtggt 3900
gacatgcagt acatgtgctg agctaaaatg ctcgaccaat atagtccgac ctatttaatt 3960
agttgtaccc tgtcactaca tgtaaatttt ctcatccagt cttccccgtc ccgtcgttgc 4020
tcctgaatag agttagtaat agaataatag atcacaaccc gaatatttcc tcacaaattg 4080
attgagtcct taattaattc tagcttaaaa cttaattaat tctagcttac aagtccgatc 4140
cttaaatttt attactagtc ctacttcgag taatcaccgt atacatgtac tcgcgtattt 4200
aacagtcatt ccaggacatc acacatgttc tgagtgcgaa catatgcaac agatcatgtt 4260
tagtcgtttc aaacacgtga acgcgcccgc aaaaacatac acactcgcga ggcaattaag 4320
tcgttgacca ttaccggaca gacatgttcc atttcgctac ggcattttac tttgaccatc 4380
catccgatga tccaatccaa gatgctgtct tggacccgac cacgtacaca acaaccctga 4440
ttccctgaag ccttccgccc ctctttctga cttgggttta gtttcgcagc agcagtgaaa 4500
gtgccccgcc caaagaaacc agccgcgcac gtacttgcat tgcataagca taaatgaagg 4560
gtccggccgg cgccatagcc atatataccg agcctgaatt tgaaaccccc ctcccatgga 4620
ggctgaggcg ttcccaatac ggtccacgcg ggctgtccgt cgccgcaagt accaccgcct 4680
gggccagctg acggacgtcg acgacggcac cgagctccgg gcgcggcagc tggtggggcg 4740
cctgcgctcc ggcggcggcg cacgcttgcg gcggcgggtg cgcgtgctcc tggcgggccc 4800
gcggcgtgcg ctggcacggg cgcgggacgc gtgcgtacgc ggcatgctgg ctctggccag 4860
gcgggcgtcg gcactcgcgc tgccctgcgg cgccgtcgcg aagcagcagc gggtcgcggc 4920
gcccggcaag cccaccgagt tcgagcggcg cctcgtcttg gagatgtaca agtccatcgt 4980
cgcgtccaag gagctcaccg ccatgctcca ctcctccgcc gcgcacctgc cgcccaagag 5040
tacgcccgct gcggggatcc cgtcgtccac gcatctgctc gacatgtgat ccaccatttc 5100
accgccgatg gaggaggaaa acgtacgagc tgattcgctt aatcactgct agagaaatta 5160
attcagagga tcgtcccaag ctgttcagca gcgtcgtcgt tatatacgac agtagctaat 5220
tttatgatgc atgtgcacat gtcatgcatc cacg 5254

<210> 90

<211> 3177

<212> DNA

<213> Zea mays

<400> 90

atctcacggt gtatattttg gagattgcat ttctctatat atatatgact atacttcgtt 60
ttgtgacatg tttttactgt ttccgtagta tatactcatg agacgttctc tcacgtatat 120
attatacata tggcacgttg cttgattatt ttggactgtc gttccattat tgagatgttc 180
ggtagcttag tcttccttcc caaccctatt ggcctatata tttggatgga attgaaattg 240
tgaatctttg tattaaatat ttttttcat gatatgtgga tttatttgga tggagtggaa 300
attgtgaatc tttgctagat acgtgttttg tctatacgcg ttactctaaa gggttatcag 360
tgacgtgttt tgtctatacg tgtcactact aaagggacat caattagaca attacacatt 420
tttacatgcc aataatgtgt ccatattaga ggcacaatat gagtgattat tttccacatc 480
ggtaatgata tgatagtcta gatgtgttat aaggaaaata gaccgaggtc cattaataat 540
taacgttttg gtatttgatg atcaacataa ccatttgcac tagtgttatt tgttggtgtt 600
tttgttatag ttcaaatgat ggaatgtgaa cttggacaaa gacaaagtgg tgatcgagat 660
gatcaatacc tcaagaaaga tattagaagg ctcgttgaag atatacaata acatgcaaga 720
gttcaagata aaactaagcc agatgtgcag atcatgaaga aacaaagcaa gaaaaaggtt 780
gaggagcgtc agaccaaggg gttggaccat cccaagggag acatcagatt atacggtaga 840
ataaaatcct aactagcaac aaacataagc attagaccct agaagttgga ctatatgatc 900
gcactattca tctcatagag acataagact gtaactacct tgaaccatct caaacagagt 960
atgatggaga cagaaaggtt ccaaagagga gaaaggggtg tcacattagt gtgatgatag 1020
gtgttggact atataggaat acccaatagt caataatgta aagactaggt tggtgagatc 1080
cttaggtttt ggaaggcttt atgtggcagc tctgaagact ccaacatcta tggcctctag 1140
ctatgcctcc atatgtattt tgtaataaga aaaatcatct cccttaaaca tgggagaggg 1200
ttcatctcca tcgaacatct ttctttaggt ggtgaagcct aaaataatga gcacgacact 1260
ttgataccaa ttgaaaggat tactatgccc aagaggtagg gtggattaga ccaatctaaa 1320
atttcttgta gcaataaaat cttatcgaat atcccacttc accacttgtg cttaagagtg 1380
atatatattc tatcacaaaa ggttttacac cctagtttga accctattct agtatgttag 1440
ttctagtaat gtaaagacaa gaattaaatt gctaaaatgc aaatgcttaa gataaagaag 1500
ggttagaaac atgacaacat ttttccaaag tattaaagag ccgggactct ccaatagtcc 1560
tcgttggagc acccacacaa ggatattgct ccccattgaa cttcacaagg atcaagggct 1620
ctctatgggt ttgttcctcg ccactctagc atggtgaatc acccacagtt gtttacacct 1680
tgagtggtga tatacactag atttcttaag tgtgtatcat cagacttatt taggtcaaac 1740
tatcattcat ggccccttta tagtatagtc aaaggaaaaa acaaaggtgc tatcactagt 1800
atatatgtct atcaactcta ttaactatta gaactagcca gtccttaacc tttttgctca 1860
tccttctaaa atcaataatt gaataccatc aataggggca caaaaactat atagccaatt 1920
attattactc atagccaata atttagtgtt gtcattagtc actaaaacta gatgctttca 1980
tatctatcat gctcgtgttt gcctgatcca attcctaggt catgtttggg ctccacttag 2040
agcttgtaat ggcatcacaa catgatctga ttaagaaaat gttgactaaa gaccttctca 2100
cccacatata aaatgccatg acttcgctca aacttcatcc cctagtctcc tactacaccc 2160
atgtctatgc ttcgcaactt cacaagtcta ccacatgtac ttgcaaaggg ctgattctcc 2220
tagctcccat gtcgtcgcca tctttcccct ttaccaccac aaaatcacaa atgtgctcac 2280
ttattatccc cgcgtcttcc cttccctacc ctaaacacta tgggcaacaa cgtcgcatca 2340
cttggtggtc cttgtagatc tagacgaggt ctcctccccg ttttgtgctt gttgatatag 2400
tgatcttgtc cttgtcttgt cgctgtcctt tggagtcgtc gcagagaggc ggtgaaaatt 2460

ctagccgctt ctagaaggtg cggtcgccgg tcggatgaga gtcgagtgag tgcaggccta 2520
taaaaagtag caattgggat aacattttga aaaaaatgaa agaaataaaa aaactagggt 2580
taaataagat tctaaattaa atctaagaaa tctatagaca ttcggaaatc tatttatctt 2640
ttgtactcct ttgattggat ttctagattt tgacgtttgt gtaattgggc ttgacagccc 2700
catcaataca ggcaggccag ctgatttcgg gtgccgctga ttcggcccat ggaggataga 2760
gcaggccttt caacaggcag gcagtccatg acgtagtgcc tcggcctcaa agcccatgcg 2820
attaatatcc actggtacgt acgcgacagc cgagggagtc actgagtcag tgccgaacgg 2880
cctggaaacc ccccgccgtg tgccggtgtc cacgcgcgcc ccggtgcggc cgccacgacc 2940
gcggacacgt gccgggcctc gcacacgcgt cccccacggc cccctcgcat cattaaaacc 3000
gcggtgcccg cgccacggat ccccggcact tcattccggt ttccggatag cttacctccc 3060
atcgacggtc ggtgagccgt tggtgagtgg cgagacagcg ggtgcgctgg gcaagaacag 3120
gcgacgaaga agatgagcac gccggacacg cggacgctca aggacgagct gacgagc 3177

<210> 91

<211> 636

<212> DNA

<213> Zea mays

<400> 91

accctgctcc gagtatcgta gtgcccccca ttgccgcacc tgtatcagcg aacttcagca 60
gactcgcctt ccgcaacctg tacatccgac ggacgggtcc agacagcagg gagatggtga 120
ctgtggaggg aagaagaggc tctagtgatc agacaggcga catgaggtac gtgagtgact 180
tccccgtcta cgatggccgt ggctccgacg ccgtcctggt ggctcgcgtg cagggcgtca 240
caaccacgtt cgggaactcc aaccagttct tcaccgtcgc cttcgaggcc ggcagcaggc 300
tcaagggctc cacgctcctt accgaaggag tggtgacgga tggatcagat gaatgggcaa 360
tctacggcgg gaccggagag tttgctatgg cgagaggtgt cgtcaaaaga aggtaccttg 420
ccgataggga tggcgccggg aacactgacg agctcagcat gcaggttttc tgcccggtgt 480
ttggctcatc acagcaacca aacaagcagg actccagcat ctctgtcacc aagattgggt 540
tatggggcgc cgactttgat ttgatctgaa cagcaggcta cctactagca attgcctgct 600
cgatcaatta tctttcgtac cctttattat taatat 636

<210> 92

<211> 1017

<212> DNA

<213> Zea mays

<220>

<221> misc_feature

<222> (994)..(994)

<223> n is a, c, g, or t

<220>

<221> misc_feature

<222> (1006)..(1006)

<223> n is a, c, g, or t

<220>

<221> misc_feature
<222> (1017)..(1017)
<223> n is a, c, g, or t
<400> 92
gacacatcgt catcgatcat cacacgcaat cgacacaaga agttaataaa cagcccaagg 60
acgcagagat catgctgatc gagaaggact tgtactacta ctcagtattg tcgtcacatg 120
cacatatatg tacataaaga gctagctacc tgagctctac ccaaggtcgc gttgatcgat 180
cgatcatggc gcggttcgtg gacccgctgg tggtggggcg ggtgatcggc gaggtggtgg 240
acctgttcgt gcccggctcc aaggacatca gcaacggctg cctcctcaag ccgtccgcca 300
ccgccgcgcc gccgctcgtc cgcatctccg gccgccgcaa cgacctctac acgctgatca 360
tgacggaccc cgatgcgcct agccccagca acccgaccat gagagagtac ctccactgga 420
tagtgattaa cataccagga ggaacagatg ctactaaagg tgaggaggtg gtggagtaca 480
tgggcccgcg gccgccggtg ggcatccacc gctacgtgct ggtgctgttc gagcagaaga 540
cgcgcgtgca cgcggaggcc cccggcgacc gcgccaactt caagacgcgc gcgttcgcgg 600
cggcgcacga gctcggcctc cccactgccg tcgtctactt caacgcgcag aaggagcccg 660
ccagccgccg ccgctagcta gcagctcctc tctgaggcat gccagatgca tgcgtgtgcg 720
tgcaggtgca accaccgcac tgccggcggc tacgtatgac cggtgaataa aaagttttac 780
tgcaccgtaa gcatgctcgc cctgttgcta ttggtatatg ttagcagtgt ggcagtctgt 840
atgtagtagc tattcgcttg catctatgca ctctatgtta gtatgcgtac gtgtggttcc 900
ggaacttttg gagtcttatc taaatactag aagtgcccgt gcgtttgtaa cgggaaccgg 960
acgcgtgggc ggacgcgtgg gaacgtatgc gacnatagca tacacntcga atcaacn 1017
<210> 93
<211> 10
<212> DNA
<213> Zea mays
<220>
<221> misc_feature
<222> (1)..(10)
<223> n is a, c, g, or t
<400> 93
nnnnnnnnnn 10
<210> 94
<211> 837
<212> DNA
<213> Zea mays
<400> 94
ggtcgacttc gctgtcgacg atttcgtacg aaatcgtcga cagcgaaggc agctcgcacc 60
agctagtcag catcgtcttc gaggccgaga ggcttaaggg ctccacgctg ctcaccaacg 120
gagtgataac ggatgggtca gacgagtggg ccatctacgg tggcaccggg gtgttcgcca 180
tggccaccgg tgtcatacga agaaggtttc tcgccggtag caacgacggg aactctgatg 240
agcttaccag catcgaagtt ttctgcccgg cgtttggctc ggcacaacaa cagccaaaca 300

agcaggactc cgtctctgtc accaagattg gggtatgggg tggaggtgga gggtcggcgc 360
aggacatcac gacgacggag ccaccacagc gtctgcacag cctctccgtt cgaactggtt 420
ttgccgtcga ctccatcgag ttcacgtata ctgatagagg tggccagagg cgcactgctg 480
ggcgatgggg tggacttggc ggcaaccttc gaacgatcga tcttggcgac gccgaggttg 540
tcagggaggt ctcaggaaca tacggcatgt ttgaaggcgc caccacgctg acctcgatca 600
gaattctcac cagcagcaga acctgggggc catgggggat cgaggacggg acacgtttct 660
gcatcaccgc gccgatcggc agcagcatcg tggggttcta tggacgctcg accagcaggc 720
tcgtcgctgc gatcggtgtt tacctgcgtc aacaactctg atctggtcat ctagctatca 780
tcgtcgtccg gtgctgtcgt atgtgtgttc atgtcaaata aataaaagtg gtcttgt 837

<210> 95
<211> 1125
<212> DNA
<213> Zea mays
<400> 95

gctgccactg tgctgtgcag tagttgcaag agctgattgg tagctaggta cgctaggcaa 60
gtaggcatgg ctcccaagcg gcgcggaaac aaggtggtgg gctccgtggt gaagaccaaa 120
ctagtgcagg agaccgtgga ggtcatcgtc gccgacgacg atgggttgca tgcggagaag 180
cagcaggtcc cggacccgga ggctttggcc ctggcgcacc ccaccgtgga cgtctctggc 240
tccacggtgg tgcatgtcgt cgaggtcact gccaaaagag gccgtggtgg tggtggtggc 300
cggcgcgagg aggggaagcc gccgccggag gaggattctg cagcagtacc cgtgccgcag 360
agccaggaga cgcaggaccc caacgaggag ctggaggacg aggaggagaa gcagccggag 420
actccgcgcg tggcgtcgga gaagaggaac aaagcagcaa caccaacaaa gaagccgaag 480
gcgaagtcgc cgcggcgggg tggtggcgac gacggcggcg gcggcaagaa gcgtacggcg 540
cggcggcggc ggctggggca ggccagctcc ggaggcgacg ccgggatggg cggcgtggga 600
gggtacaggc ggtacgtgtg gcgcgtgctg aagcaggtgc acccggacct gggcgtgtcg 660
gggcacgcca tgcaggtgct ggacatgatg atggccgaca tgttcgagcg cctcgcggag 720
gaggcggcgc gcctgtccaa ggccacgggc agggcgacgc tcacctcccg cgaggtgcag 780
agcgccgtca ggctcgtgct ccccggggag ctgggcaggc acgccatctc tgagggcacc 840
aaagccatct ccaagtacat gtcctatgcc gcctagctag gtagctacat gttttctgta 900
ccttggaagt agtgcgtact gcagctgatg tattttcatg catgccaact agactagcta 960
gctactatac gtatgtatgt agttgatagt gtttatcagg ccgggtagca gctagctaga 1020
ccaacttaat tagctaatgt agtttgatgt acctgtctgt cagtctgtgt atgtgtatgt 1080
ttcgttgttt taatcgacca acttgttccg tgtcaaaaaa aaaaa 1125

<210> 96
<211> 702
<212> DNA
<213> Zea mays
<400> 96

gtacgaggca tccgcactac tgagagcgac aaagctagct ctctccaggg atcggattcg 60
gatcaccatg ccgtccaccg tgaactccgc gtccctcgac cagaaccttg agctcgccaa 120
gcaatgctcg cgagaggccg ccctcgcagg gttcaaggca gcagctgtcg cgaccgtcgc 180

ctctgcagtt cccaccctgg ttagcgtcag gatgctgcca tgggccaagg ccaacatcaa 240
ccccgctggc caggccctca tcgtctccac agttgctggc atggcgtact tcatcgcggc 300
ggacaagaag atcctctcgc tggcgaggca gcactcgtac gagaacgcgc cggagcacct 360
caaggacacc tccttccagg gcaccggccg tccgcaccca gggttcttca ggccttgagc 420
gagagaccat caaactaccg ccctagctag ctggacgcac tagttcgtca gcggccgccg 480
attcatgtcg tccaagatca tatcaactac tgctagcata tatatagtgt actagctagt 540
agtaggcacc ggcgatccgt gcatgcatgt gctcggagaa actgtaatgg cttagtgctc 600
tgctccagtt tactgtatct accttccatt atcatataat aatatcatcc tcctgtttaa 660
aaaaaaaaaa aaaaaaaaaa caagaggggg ggcccggtac cc 702

<210> 97
<211> 629
<212> DNA
<213> Zea mays
<220>
<221> misc_feature
<222> (615)..(615)
<223> n is a, c, g, or t
<400> 97

gcatacacta gtgcttttgc tagctacttg tgcgagaaga gagagcgcac ggcgatagat 60
atggcgcata gcaagcgcga aatcaagccc gggagggcgc ataccaaggt ggccggcgac 120
gacgagatgc tgcggaccgg gttcttcgac ggcacgccgc tggagggcgg caagatcgcc 180
gactcccagc ccgtcgacct cttcgccgct gcccgccgcg tcgccgacgc cccgtactca 240
tcgcagcagg aaggaccaca cgaggagggg accaacaaga acgccgtcat cgcggagtcg 300
gagcccgtcg acctgcccgc gagcgcgcgg ggcgtggcgg aggtgaaccg tgctgggccg 360
gaggagcaga caggccgcgg tcggcagggc atgaccgagc ccaccgccgg tggacgtcgc 420
ttgggattag gtcgcccggc cccggcttaa cgatccagat caagcagtac tcgactaccg 480
ccactacact acctgctctg ctcgtgtctt gtttccagtt ttctttctct gtaagcggtg 540
tagggtttgc caaactttta tgtacgaatt atgaatatca ataaagtcga tctgctgctg 600
cgtttcttgg caccnaaaaa aaaaaaaaa 629

<210> 98
<211> 2040
<212> DNA
<213> Zea mays
<400> 98

agcgtcacag agcaccgccc ggaggttccc cttcccgtcg ccgtcgccgt cgcttggcca 60
agatggctga acaattctat accgtggcat cggatagtga aaccactggt gaagacaaat 120
cacagccttc attccctgat gttgctgttg gcattgacat tgggacttcc aaatgcagtg 180
ttgctatttg gaatggccac caagttgagc tcttaaagaa tacccgtaat cagaaaggga 240
tgaggtcata tgtcatgttc aaagatgata ctctttcagc aggagttact ggaggtgcag 300
ccaaggaaaa tgcacacgag gaaagagaca tcttgtcagg aagtgccata ttcaacatga 360
aacgcctgat aggaagaatg gacactgatg aagtggttca agctagtaag accctcccat 420

ttcttgtgca gacattgggt atcggtgtga gaccattcat tgcggccctg gtgaacaaca 480
tgtggcgatc tacaactcca gaggaggtcc ttgccatctt tctacttgaa ttgaaggcct 540
tggtggagat gcaccttaag catcccgtta ggaatgctgt gcttacaata ccagttgcgt 600
tcagtcggtt tcaacagacc aggattgaga gagcctgtgc aatggctggg ctgcatgtgt 660
tgaggttgat gccggaacca actgctgtgg cgcttctgta cgctcagcag cagcagcagc 720
ttgtgcatga caatatggga agcggcatcg agaaaattgc tctgatattt aacatgggtg 780
ctgggtactg tgatgtggcc gtggctgcca cagctggagg tgtttctcag ataagagcat 840
tatcaggatg cacagctggt ggggaggata tacttcagaa cataatgcgc catgttcttc 900
ccaattttga cggtatatat gctggccaga caatggatag aatcaagccg atgagcttac 960
tgcggattgc tactcaagat gcaattcaca ggctcgttag tcaagaatcc gtggagatca 1020
atatagattt ggggaatggc cacatggtgt ccaaagttct agaccattca gagtttgaac 1080
aagtcaaccg ggcaattttc gacaagtgcg aaaagatcat caaccagtgc ttggctgatg 1140
cgaagttggt accagaggac atcaatgatg tcatactggt cggaggttgt tcgaagatcc 1200
ccagaatcag aagccttgtc ctgggtttgt gcaagaagga ggtctcctac aagaacatag 1260
atgctctaga agctgctgtt tcaggtgctg cgctggaagg agccatcgct tcgggagtaa 1320
atgatccttc ggggagcttg gatcttctca cgatccaggc aactcccatg aacctgggaa 1380
tccgtgccga cggtgatagc ttcgctgcga tcatccctag gaacacgacg gttccagcaa 1440
ggagggacat gctgttcaca acgacacacg acaagcagac cgaagcccta attgctgtgt 1500
atgaaggaga aggggaacgt gctgaagata accatctgtt agggtacttc aagatcactg 1560
gaattccggc agcccctaag ggagcagttg atatcagtgt atgcatggac atcgatgctg 1620
ccaatgtcct cagggtgttc gcaggggtcg tgaagcctca aggcccagcc attccaccgt 1680
tcatcgaggt gaggatgcca acgctggacg atggccatgg ctggtgtggg caagctctag 1740
caaagatgta tgggaagact ctcgaccttg ctgttcttcc gaagaagctg cagccatgat 1800
atgtgccttc cgtggcccgt aaagtagctg gtgcgttgtg cgaatgtagg ctgctcagtg 1860
ttcagtattc actgtgtcac tgtcccctgt ggtgtggcca ctgtttatgt gctgttggtg 1920
ttgggcagtc tacagaactg aactaccagt ttcatccttg taggcgtgta caataatgtc 1980
ccgtgcatgt gaaatgaatg ttgcggatgt ttacttcata gttttcagga aaaaaaaaaa 2040

<210> 99

<211> 811

<212> DNA

<213> Zea mays

<400> 99

atctttcgcc gtcccatcag cccagcaatt cttcgctgtt cgaggacccc tcggtttcga 60
ccgaagccca gcaagccgac cacacaccgc tgccgttggt tccgtcccaa gagatgggca 120
agtcctggtc gctcatcagc cacctccaca ccgtcgccgg gccaagcatc accctgctgt 180
accctctgta cgcgtcggtg tgcgcgatgg agagcccgtc caaggcggac gacgagcagt 240
ggctgtcata ctggatcata cactccttcg tcaccctcct ggagatgctc gccgagccgc 300
tcctccactg ggtacccgtg tggtacccgg ccaaggtgct gttcgcggcg tggctggcgc 360
tgccgcagtc aagggcgcct ccttcgtcta cgagaagctc gtcagggaac agctccggaa 420
ctaccgcgcc agatacccgc gcaagggcgc cgccgccgcc gccgtcggcg gcgacgacga 480
cgaccataag gtgcacatag ccaaggctga ggctgagcac gaccatgtgc agtgaaagcg 540

tgtggagacg acgcaggagc tgaccggaga tcatcacagg aacttctgta gatctggtgt 600
tccaaggtgt tcattcgatg tatcatccta cgtatctctg taactcttgt ggactgccgg 660
tgtcgcgtaa tgtatctgaa ctcttcgtga gtgcgatgga acccccctcc ccttgcagca 720
cgagatgctt ctgtaagaac gataacatgt agacaagcca agcgcctcag ctccttaagt 780
gaaattctct actactactc aaaaaaaaaa a 811

<210> 100
<211> 1965
<212> DNA
<213> Zea mays
<400> 100

cgcctggttc cacttccacc acggtgtgca ctagcgctac accgaggcca ccttttcttg 60
gttccttcaa cacgcctact ccggcctccg gatcgggcac cacgtctgcc ccctgctccc 120
ggaatctttg ccaagcggcg ccgctactgt ccggcaaggc gaccccgtct ctcggccggc 180
gttgatctgg cgggcggagt ggcggcgcga tgggcatctc ggcgaggtgg ctcaagtcgt 240
tagttgggtt gaggaaggtg gggcggcagc agcagcagcg gcgaaaggat gatgcagatg 300
ttggtcgaat gaaaaccgat gtcgccgacc agtttcattt tcagattcag cactctcaag 360
atgacaatag cattgctgca caagaaattc cagaggtttc ttatggaaat gatccacctg 420
aagatgattc taatgtacct tcatgcttcg agcctgctcg tagttcagct catatgccat 480
tttgtcaaac tgaagaagcc cagaaagaga tctgggctgc tacaattatt cagacagcat 540
ttagagcttt cctggcaagg agagcccgcc gagctttaaa agggttggtt aggcttcaag 600
cccttgtaag aggtcatata gtaagaaagc gagcagctac gacactccgt tgtatgcaag 660
ctttggtcag ggttcaggcc cgtgttagag caaggcgagt tcgcatggct ttggaaaacc 720
agactgatcg gcaaaatact tcaccagagc acacgatcga ggcacgtgtc agagaaatcg 780
aggatggctg gtgtgatagt atcggttctg tgggagatat ccaagcaaaa cttctaaaga 840
ggcaggaagc ggcagctaaa cgtgagcgag ccatggccta tgctctagct catcagtggc 900
aggcaagttc aagacaacct acagcatttg aacctgacaa gaacagctgg ggctggaatt 960
ggctagagag atggatggct gttcggcctt gggagagtcg gttccttggc acttacggga 1020
cagatgggat tttgtagtt aatgaaacca gacaacctga cagaagtgca accaagaccc 1080
catacagaaa acctgttaaa aagcatgatt caacccttca atcaaacatg ttgaaccaga 1140
aggtcttccc atcaaactca gagggtggtg gctcctcgac aaatcgatca agtggtttgg 1200
tatcaactaa atctagactg aaggttttac ccagagaagg ttctgaggaa gcctcatctc 1260
atccttcagt aattgtgcgg tccactagta atccgaagga gaggatttcc agtattaatt 1320
caaaggagag gactggggac ttggattttc aagttcataa aagatcctcc ttgcctagca 1380
ttggtccaga agctggcaaa cacctgacaa agaaaggcat ggttaaccga tccctgaagg 1440
ccacaaaaga ttcccaaata ctggggtcaa ggcatcatct tgccagttcc attgatccag 1500
ttcccaccag agttgaactg gagacttgag aaggcatgtt atgctcgaac atcgccatgg 1560
attgcaccta gcagcttgga tgccgttcat gcctcaatgg ctgcatgaat gtgtcgcaac 1620
atctatgtta tactggtggt ggcggttttc gagtttcgac accatatatg taggctcatt 1680
tcatgagata aaatcagatg catgtgagca tgtccatata tgttaagagg attaccattg 1740
gttttattgt tgtatttgta agttgccgtt aggaggtgta aatttgtgtc gtcgagtggt 1800
acttattatg tgttgtcgta tagttgtgac tcgaaggtta aaggtggttt tggttctact 1860

gcgaagtgtg aactgtagtg agctgtgttc tgtaaaaaaa taaagttgtt gtgatttgtc 1920
tactgtaaaa aaaatggaag taacttggtt caaaaaaaaa aaaaa 1965

<210> 101
<211> 699
<212> DNA
<213> Zea mays

<400> 101
cggcccttgc gatcgccacc acgtcagcgc ggaggatgca cggccaccag cagcagccgt 60
acccatacgg gctgcggcaa gacccgctgc ggcggatgcg aggtctggac gtgaaatcgg 120
cgctccaggc cagcccgcgc acgtccacgg tggccacggc cgcactcgtc gtcccgctgg 180
gtgccgcgct gctgtgcgcg tcggggatcg cgctggctgc gaccgtgacc ggtctcgcgc 240
tcgccacgcc tcccctcgtg attttcagcc cggtgctcat acccttcgcg ctggccgcgg 300
ggctcatcgc gtcggggctc ctcgcgtcgg gcgcgctcgg cgtcgcgggc gtctcggcgc 360
tcacgtgggc cgtcgggtac gtctggcaac ggcagggcga aggcggcggc ggggtcactg 420
ggatggtggt gcagccgctg gaccaggggt cgaagcgcca tggtgtccaa ggcgccgccg 480
cgttcgtggg gcaccgccgc ctgccacggg acatcgacgt tgctggtgaa tgatggtttg 540
gcggcgtgtg tgcaaacttt tctatgctag tttagtttgc aggtgtgtat ggattttgat 600
ttatggcctg gatggatcac actatgtagc gaacgatgtg ttcaatttac tgttggaata 660
aaactgcaga ttggtgttac ttaataaaaa aaaaaaaaa 699

<210> 102
<211> 1250
<212> DNA
<213> Zea mays

<400> 102
agatcatatg cagcgtggac gcctccgata gccttggctt tggacctcct tatcaggcct 60
tcttcctcgg cggcgcaggg gacccggcgt cggtcattgc cggttttggg ccgaagacgc 120
ttacccgtgc cttcaacgcc acctacgacg agttggcgcg tatcctgttg ccacgaaccg 180
gcggccccat cgtgtactac accgcggacg cggagccaga gagcggtgcc gccgaagagg 240
agcgcgggca ggtcgacggg cacgacggtg tgctggacag gggagcgcga cgcgagggcg 300
ccggcgcgtg ggtgccgggt ggcaggggag acggaggcga cgagtgcggc ggcagcgatg 360
acgcgcgtga ggcgacgtgg tggtggacga agctggttaa cagggtcgtc ggaggggcgg 420
ctggcggcgg cggcgctgcg gaggcgaaca ggaagggcaa gaagaagaag ggcggcgcgc 480
cggagccgta caacctctac gacagcgagc ccgggttccg gaacgcgtac ggctggaccg 540
tctccgtcga caagcaccag tacgagcccc tcaagcaccc ggacatcggc gtctacctcg 600
tgaacctcac cgcggggtcg atgctggcgc cgcacgtgaa ccctcgggcg acggagtacg 660
gcgtggtgct gggcggggaa ggcacggtcc aggtggtgtt cccgaacggg tccctggcga 720
tgagcgaggt ggtgcgcccc ggcgacgtgt tctggatccc gcgctacttc cccttctgcc 780
aggtggcggc gcgggccggg cccttcgagt tcttcggctt caccacctcg gcgcgccgca 840
accggccgca gttcctggtc ggcgcctcct cggtgctccg taccatgcta gggccggaga 900
tcgccgccgc gttcggcgct cgcgagaagg agttcagtaa gctggtgcgt gcgcaacggg 960
aggccctgat tatgccgtcc tctcctggga aggaggagga ggagcatggg aagaaaggga 1020

gggagaaaga ggagtcactg ccgatggttg tcgagcaggc ggcggcggag tgagctgctt 1080
gatctttcag tcttggactc ttggcttcct cagaagcgcg cgcgcgccac cggaaagtgt 1140
ttctactctt gttactgcgc cttgtcagtg atcgtgttgt tggttttttt ttttcgtggc 1200
gccgtctata aataaaagtg ctttgtgcgt ttacagctta aaaaaaaaa 1250

<210> 103

<211> 1856

<212> DNA

<213> Zea mays

<400> 103

tcggcgtcac tcctatcgtg ggaccccgtg gtctgcccgt gttcggcagc atcttcgcgc 60
tgtcccgcgg gctgccgcac cgcgccctcg ccgagatggc ccgcgccgca gggccccggg 120
ccaaggagct catggcgttc tccgtcggtg acacgcccgc ggtcgtgtcg tcctgcccgg 180
ccacggcacg tgaggtgctc gcgcacccgt cattcgccga ccgccctgtg aagcggtcgg 240
cccgggagct catgttcgcg cgtgccatcg ggttcgcgcc caacggcgag tactggcgcc 300
gcctccgccg cgtcgcgtcc acgcacctat tctccccgcg ccgggtcgcc tcgcacgagc 360
cgggacgcca aggtgacgcg gaggccatgc tccgctccat cgccgccgaa cagtcggcct 420
ctggcgccgt cgccctccgc ccgcacctcc aggccgccgc tctcaacaac atcatgggca 480
gcgtcttcgg cacgcggtac gacgtcacat caggcgccgg cgccgcggag gccgagcatc 540
tcaagagcat ggtgcgcgag ggggttcgagc tcctcggcgc cttcaactgg tccgaccacc 600
tcccctggct cgcccacctg tacgacccaa gcaacgtcac ccgccggtgc gccgcgctcg 660
tgccgcgcgt ccagaccttc gtccgtggcg tcatcgacga gcaccggcgc cgccgccaaa 720
actccgccgc cctcaacgac aatgctgact tcgtcgacgt gctcctctcc ctcgagggtg 780
acgagaagct cggcgacgac gacatggtcg ccatcctctg ggagatggtc ttccgcggta 840
cggacacgac ggcgcttctg accgagtggt gcatggcgga gctggtgcgc cacccggcgg 900
tgcaggcgag ggtgcgcgcc gaggtcgacg cggctgtcgg tgccggaggt tgccccaccg 960
acgccgacgt ggcgcgcatg ccgtacctgc aggcggttgt gaaggagacg ctgcgcgccc 1020
acccgcctgg cccgctgctg agctgggctc gcctcgccac cgccgacgtg ccactctgca 1080
acggcatggt ggtcccggct ggcaccacgg cgatggtgaa tatgtgggcc ataacccacg 1140
atgccgccgt gtgggccgac ccggacgcgt tcgcgccgga gcggttcctg ccctccgagg 1200
gcggcgccga cgtggacgtc cgcggcgtcg acctccgcct ggccccgttc ggcgccgggc 1260
gtcgcgtctg ccccggcaag aacctgggcc tcaccaccgt gggcctctgg gttgcccgcc 1320
tcgtgcacgc cttccagtgg gccctgcctg acggcgcggc ggccgtttgc ctcgacgagg 1380
tcctcaagct ctccctggag atgaagacgc cgctcgtcgc cgcagccatc ccccgcaccg 1440
cctgatccgt cctgccgccg acgcgtcacg tcacgcgttg tttgcatgga tgatggtatc 1500
tttgtctgtc tgtgtggtct tcgctaaagt ttgcttcttc tcgatcgtcg gttcgttcgt 1560
gcctccacct tagcctaggg tttggtttct tgcaaggtag tgagtgtgtc ttagtctcac 1620
catcaccggg gctccaattt tggaaagctg cgtgttagga gttaacccct agacatgttt 1680
gcgtcttgat cgccaccacc catcagtatc agcgcagaaa ctacatatag atcagtgttt 1740
gtcgaccagt catggaagtc gtgtgctctc aagtctgatg tattatatac atatatatgt 1800
attgtaatgt gattatcaag aaccgtgcta tttacatatt gctgtttaat tgaact 1856

<210> 104

<211> 1765
<212> DNA
<213> Zea mays
<400> 104
ttttttttt ttttttatgc acaagctgca gactttttat tcctgaaagc acagcttatc 60
tttaagcagt acagactaca gacagaccac tgactgcata agtgaggaca aacgggcaca 120
gctcaacgag gaagcaagaa cacagaacaa caaacaacaa ctccagagct cagaagttgt 180
cttgattgcc catgggcaca cgcacaggcc taaccgtgcc atgcatcgac accatcagcg 240
acggagatcg ttcatgacct gctgagagcc ccggaactcg ttcatgaacg ccttatggaa 300
cgcccctcaag cgtgcgacca tcgcctcgat ttttctttcg tcaggaagga tcgtgcatcg 360
gatatggatc gtcccagagg cctggtggaa cccagagccc ggcacaacgg caatcccggt 420
ggcatcaagc aggcggtgag cgtagaatat gtcaggtgaa actccctcga aatcggcggc 480
cctgatcgca gcggagggga ggtggagccg tgggaacaag tacagcgcac cctctaactt 540
gttgcaagtc acgccctcca ggccgctgaa agctttctcc agggtcttgg ctcgcttgca 600
gaaggatgaa cggatctgtt ccttctcctt atcaaatgac tcgaaagatt catctcctag 660
cttcggtgga tccattgcaa ggctgacgag aatctggcca gcaatgttgg ggcagagtgt 720
taaagaagcc accttgtaaa tctgatcctt cacattagct gcaacaccag acacctccat 780
gtagcctcct cttctgccac tctccccata gctcattgaa acggaatgaa gtgatactag 840
ggtgaggtca ttttcgtcgt aagcaagtga tctggccact ttcttgaagg aatggaactt 900
cttgttctct gcgtagatat tttcctggta tacctgtcca gtagggttgc cgggatttat 960
taccaccata gctctaacag tgaggccagc cgaacgagcc tcttccaagc acctcttgac 1020
ttcaaagatc tctaggcccc aaccgttatc ttcagtgaga ttgtacggca ccatcgttcc 1080
accgtgcagt atgatggagg ccgagtacaa ggggtattcg ggtagagggc tcaagatgcc 1140
gtcttcgtga gacctgatga gtatctgcat catcaagttg atcgctgagg tggctccatc 1200
tgtcagaaag atatcgtccg ctgtcgatgg aaaaccgtct cttgcggtaa tcccatctgc 1260
tactacttga cgcaggcatt tgattccccg gcaatcagta tatccaccag tgtctttgct 1320
ggggatcgag tttaggatcc tcctggccct tcttatgcta caagggctga ataaggcacg 1380
agcttcatct ctgtccatga gggctgggtt gtcgcacagg gagagaacct cacgaaagaa 1440
agttagtggc ggctgaccaa ggacctgggg gttcccgagg ttgcagtata ttatctcttg 1500
gaaagggata gaagcagggt tctcctctat ctcctgctcc agccgcgctg cgtgcttgac 1560
gatctccccg caaggcgcgt atctgatgat cttgaccttc aaacgaaggg acaacgtacg 1620
tcacctaagc ttttcagttt aaatcggaag aatgcaatgg atgaacatat atatgatttc 1680
tgaagaagaa accctgggat ttatggtctc ggcggtgatg gacggggcct tgttgtacga 1740
catggcgcgt cggggcaccg gccgg 1765
<210> 105
<211> 1047
<212> DNA
<213> Zea mays
<400> 105
ctcctcctcg atccatcgat cactgcaccg gccggcggca ccgcgctcgc aggggctagc 60
caacgagacg gcagcaatgg cggaccgtga ccgcagcggc atctacggcg gcgcccacgc 120

cacctacggg cagcagcagc agcagggagg aggcgggcgc ccgatgggtg agcaggtgaa 180
gggcatgctc cacgacaagg ggccgacggc gtcgcaggcg ctgacggtgg cgacgctgtt 240
cccgctgggc gggctgctgc tggtgctgtc ggggctggcg ctgacggcct ccgtggtggg 300
gctggccgtg gccacgccgg tgttcctgat cttcagcccc gtgctggtcc ccgccgcgct 360
gctcatcggg acggccgtca tggggttcct cacgtcgggc gcgctggggc tcgggggcct 420
gtcctcgctc acgtgcctcg ccaacacggc gcggcaggcg ttccagcgca ccccggacta 480
cgtggaggag gcgcaccgca ggatggcgga ggccgcggcg cacgcgggcc acaagaccgc 540
gcaggcaggc caggccatcc agggcagggc gcaggaggcc ggcgccgggg gaggtgcagg 600
tgccggcgct ggcggcggcg gcagggcttc ctcgtaagca agtcatccat gcatggatta 660
tggatagatg cgcgcgtgcg tgtctatcag tatcagcagc cagcagggtc gtcgcggaat 720
gctgtgttcc tgtacgtgtg ggtgaccgtc cttccgtcct tcgtctttct ccccccgagt 780
gtgtgttacg tatgtcctgg tgttcgtcgt gtgtgttcat cgccgctcca gttgaattcc 840
ggtgtctgtt catcgccgct ccaggtcgta gatgtgaata tactttgcta ggggaataag 900
tgataagtct gtctggaagg taatgtttga gctttgctag tgtggctggg cactctggtc 960
actggttgtg ttgtgcatgc atcagctgta tgatcgtcgt ctgttgtgga aaattggtca 1020
atgtattctc ttgctgaaaa aaaaaaa 1047

<210> 106
<211> 540
<212> DNA
<213> Zea mays
<400> 106

cgatgggtga gcaggtgaag ggcatgatcc acgacaaggg gccgacggcg tcccaggcgc 60
tgacggtggc gacgctgttc cctctgggcg ggctgctcct ggtgctgtcg ggcctggcgc 120
tggcggcctc cacggtgggg ctggccctgg ccacgcccgt gttcctgctc ttcagccccg 180
tgctcgtccc cgccgcgctg ctcatcggca cggccgtcgc ggggttcctc acgtcgggcg 240
cgctggggct cggcggcctg tcctcgctca cgtgcctcgc caacacggcg cggcagggtt 300
ccagcgcacc ccggactacg tcgaggaggc gcgccgcagg atggcggagg ccgcggcgca 360
cgcgggccac aagaccgcgc aggccgggca cggcatccag agcaaggcgc aggaggccgg 420
tgctggcact ggcgccggcg gcggcaggac ttcctcgtaa ataagcaaat catccatgga 480
tgcgtgcgtg tcaatgtcag cagggtcgtc gtggaatgct cttctctgta cgtgggtaac 540

<210> 107
<211> 877
<212> DNA
<213> Zea mays
<400> 107

tcgccaccgc cgaccggact cccctcaaag gcggccacag cacagcgagc atgggagacc 60
ggcatcagca tgggcctggc agcggcgtgg aggacccggc gacgctgctg cggcgggtgc 120
agacccgcgc gcccaactcg acgcaggtgg tggggttcct gacgctgctc gtctctggcg 180
ccgtgctgct gctgctgacg gggtgacgc tgacgggcgc cgtggtggcg ctcgtcttcc 240
tgggccccat cgcgctgctc accagcccca tctgggtgcc cgtcgccgtc gtgatctccg 300
ccatcgtcgc cgccgcgctc tccgcctgcg ccttcgccgc cgccgcgctc cccgtggcca 360

cctggatgta ccgctacttc acgggccgcc accccgtggg cgccgaccgg gtggactacg 420
cgcgcagccg gatcgccgac accgccagcc acgtcaagga ctacgcgcgc gagtacggcg 480
gatacctgcg cacccgcgcc aaggacgccg cgcccggcgc gtaggcgcgc gccgcccggc 540
gtgcatgtgc tgcaaccccc gtccgtggtg ccgctagcta taggtcatca ttgctgtcgt 600
cgtcgtcgtc ttagttcctc ttgcattgcg tgtgcgtact ctgctccaat catcccccgc 660
ccgtgttgtc aagcaatttc tggtcctctt gcttgctttc tctaggagat gctgttcatt 720
tcgatctctt ggcgcaattt gatctttcgc ttggtgtaat taggtcatta gttaaatcat 780
aagagactcg tgttcatggt ctttctcaca accttaaaac aactaattgt tgttgaaaag 840
aatatatata ggtaaccttg ctcaaaaaaa caaaaaa 877

<210> 108

<211> 846

<212> DNA

<213> Zea mays

<400> 108

atagcttacc tcccatcgac ggtcggtgag ccgttggtga gtggcgagac agcgggtgcg 60
ctgggcaaga acaggcgacg aagaagatga gcacgctgga cacgcggacg ctcaaggacg 120
agctgacgag catggacagg aagtgcctcg tggacctcgg ccacccgctg ctcaaccgcg 180
tcgccgacag cttcatccgc gccgctgggg tcggggcggc cagggccgtc tccagggagg 240
cctacgtcgt caccgtcgaa gggctttcag gagactcgtc cgggctagac gccgacggcg 300
gcaagcggag ccatttctcc agcatcagag gtgacgacgg ccagaggtcg ctcgacgcag 360
tggtaaaaac ggccggcaag gaggccttcc agtgggggtt ggcagccgga gtctactcag 420
ggctcacgta cgcgctgcgg gaggccaggg gatgccacga ctggaagaac agcgccatcg 480
caggcgccat cgctggggcg gcggtggcgc tcacgggtga cgccggcggc cactccgaca 540
agctcgtcaa cttcgccatc accggcgccg cgctctccag cgccgggagc ttgctctccg 600
gcatattctg attggccccc agggctgcac ccgcctgaca cgggttcgct gcccggttga 660
gcccttcatc cgaggggttt tagcatgaaa ggttgatttc aggccagcaa tgaaatatgt 720
attcctaggg ccctgccggt ctagcactgt gcgcttccag tgtaatcgtt gcttcgtgta 780
cgagtttcag acatttcgaa atatcttctc cgagttccgg tggtttgaca aaaaaaaaaa 840
aaaaaa 846

<210> 109

<211> 1106

<212> DNA

<213> Zea

<400> 109

ttggtttttt gataatttgt ttatctcacb vhbvhgttct aacaatbvhc gcagatctta 60
tacatctctc tcgtagtttt tcataaacta cgagaaagat ataggttttb vhaacaaatt 120
tacttttatt ttgtcatata bvhaabvhgt caaaatataa attgtacatc bvhbvhagtt 180
atacaaattt gtagtccaaa atcttttcat ttaaattaat ttactgcttt aaabvhtgat 240
ttttaattgt ctttgcctag tgttgaaaaa aagcactcgg caaagaaact ctttgccgag 300
tgttaaaaaa acactcggca aagaatctct ttgccgagtg tcaaaaataa aatatttggc 360
aaatagcttc tttgctgagt gtttttttgc ttagcacttg gtaaagaact tctttgccga 420

gtgtccgaaa aagcactcga taaaatattt ggcactcagc aaagaaccga attccagtag 480
cgtgagtcag aagttggcat ttgaaccctt tgaaataatc aaacaaatca acgcagttgc 540
atbvhaattg gctttgccac bvhgtagtgc agtccatcga gtgttccbvh tttcacagct 600
taaactaacc cbvhgttttg gtaacagaac acacaccagt gactccatcg ttaccggatt 660
ttgatactac actgaaggtt ccggaacaaa tactggattc tagattgatt ctagattgct 720
tcgatcaabv hgggatcatc aacctaacac agcttaaatt atacbvhcbv hcgtacattt 780
ggtccttcaa gaggaggcag gtgbvhatca aggtctctct agcggcctgg tgtgcagcca 840
cgtacccatc cacgcgtcgc cgcgggcgac gacaggtgtt ggccaacctg gacatcagac 900
aagcacgaca bvhacaggcg ttcgccaagc gtacgtacag gagacgaaca cgacaagtag 960
caccgtcctg gccgagagcc cgtcbvhctc bvhcbvhcbv hcbvhctgat cbvhcatact 1020
cgataaatat acagcagaga gctgagctac gtacacaaac aagaagcttg cattgttcgt 1080
tgctcgatcg ttgcaggtab vhgaga 1106

<210> 110
<211> 922
<212> DNA
<213> Zea
<400> 110

ttagtagaga ataacacaca tctccctaag tatcacaaaa aaattaattt taagaggcaa 60
ttttagtttc caaagcgatt tattaagatt taagggaggg tatttattag aaggaagtac 120
attgatcaaa cagagaaaat tccatcattb vhgtcccccca tagaaatttg tcactcgatt 180
gtcgcaaaaa aagttaccag taaatttgat aaattttgga taccggtctg ttttaactca 240
tcaagaatag cacgacgaaa taatagattg cgtacgtttt cattcataac atctctcact 300
aaagcatttt ttacacacgc tattaaabvh gttcggaaac tgatccttaa gcggtctatt 360
catatccatc tatcgttcta agattcttcc tgtacgtaga ttctttccga ttaaattctc 420
bvhaaaaatt aaaaacaaac aggtccttaa actagtcgcc ggaagttctc gcttttgctg 480
tagttgtagt gtcgbvhcbv hggtggaaac gtggacagat agattcbvha abvhtggaca 540
acccacctgg tccagagcat cbvhatacat tgatacacgt ggcacgctgc ccactgbvhc 600
cctcagcttt gaagctaacg tgtcaagtgt ggccggcccc tgcattgcgt cgtgccaatc 660
gtccaagtcc cbvhgcaaaa acccagcgct ttgtgccgcc gccgtccgcc ggcccctctg 720
cccttgtacg tgcacctaga cacatcgtca tcgatcatca cacgcaatcg acacaagaag 780
ttaataaaca gcccaaggac gcagagatca gctgatcgag aaggacttgt actactactc 840
agtattgtcg tcacbvhcac atatbvhtac ataaagagct agctacctga gctctaccca 900
aggtcgcgtt gatcgatcga tc 922

<210> 111
<211> 1945
<212> DNA
<213> Zea
<400> 111

gattcagaac atctggtcag cttaabvhbv htggtaaagg gagcattatt cttttatttt 60
gttctttgtt ctgtgcabvh gccatatatt ttcgtattga tbvhctctca gtggaacagt 120
ctgaagctab vhtgaaactt cgbvhttcag gtbvhatabv htaccaacag agattaaccg 180

gctgagbvhc agagttaact atcbvhcatt aaagttccta cctgatattg aggaabvhgc 240
tgttaagctt gctgcaagab vhaggaacaa aactggcagc ataaatccat acbvhtacgt 300
tataattatc cttttcgctg ggtgatattg tgtacagtag agcttgtatc tacccaagac 360
actatccagt gcatagctga agttgacgct ctcaaagttt atttcacaat caaatattga 420
cacatttttt ttgttcttaa agggctcttc acctgagatt tgaaaaaggg bvhgtggggc 480
tttctttctg tgattttgcc ggcacacggg aggagaaggc abvhbvhgcg gcttataggc 540
agaaagabvh gccaaggcgc ttcaaggtga cgcggactgg actagataca tctgttacat 600
tggactgaga aagaatccct aggagaabvh aagcaccgac actaactata cgcgctagtt 660
cgggaacccc gttttctcac gagactttta tttttccaag aaaaattagt tcattttttt 720
taggaaaata taaatctctt aagaaabvht agttccgaaa ctagccctac aagtctaacg 780
attttctctt ttttgtatct gccacgcaga bvhgatccca tctbvhgcca ctggcgctgc 840
agaagagaaa agaagggcgg tgccctcttg agcccggtga gatcgctgtg atcctgcggg 900
cactggggta cacgagcggc acacagatat acgtcgcgtc cgggcaagtg tacggcggca 960
agaaccggbv hgctcccctc aggaacbvht tccccaacct ggtaggcagg ctcaaatcca 1020
cttcagattg tgtcaagctg aagccagtga taactgataa gccactgccg tgcattgtgc 1080
atcgctgcag gtgacgaagg aggagctagc gagcgcggag gagctggcgc cgttccggcg 1140
gcacgtgacg agcctggcgg cgctggactt cctggtgtgc ctgcggtcgg acgcgttcgt 1200
gbvhacgcac ggcggcaact tcgccaaact catcatcggg gcgcgccgct acgcggggca 1260
ccgcctcaag tcggtgaagc ccgacaaggg cctcbvhtcc aagtccctgg gcgaccccga 1320
cbvhggctgg gcctccttca cggaggacgt cgtcgtcacg caccgcacga ggacgggcct 1380
ccccgagccc accttccccg gctacgatct ctgggagaac ccgctgacac cctgcbvhtg 1440
cagggcbvha gacgggccat ccctcagatc agagtggccg tctggccgag tcctcgtctt 1500
cgctaggcta cgactacgag tagtctcgtg cccacttcca tcagcgcgca tttcagcgag 1560
catbvhcttc tactagttaa acacctagat ttgtbvhtat ttggctggcc taatctgtag 1620
tttcacatag atcacttcbv hctgaaabvh tcttggacaa ggcagtgabv haagataaca 1680
tcagtaacga cctgcttttc caaagctcct cttggattta ccaagctgag ccaagaabvh 1740
gctaacctga gttttgactt gcagccttgc aggcbvhacg tgggcgacct gaactcgcta 1800
gtcgtagagt ttctttccac gcggcagctc ggcagagcta cgcagcctct ctgcccgcca 1860
cgttctcctg caaggctgca actcgaagca ctgagaagct attaattabv hgattctcga 1920
gcbvhcggcc ttgttcgcta aacct 1945

<210> 112

<211> 512

<212> DNA

<213> Zea

<400> 112

gccagtgcta bvhatattta cactagcggg ctgctaaaga aaaccgccag tgctaaagat 60
atttacacta gcggttggtg aacaactgcc tgtgaaaaaa gccgatttct actagcccct 120
agctagcact ggcgacataa aaaacgtcag tgaaaatagc tctaggatcg tcactataga 180
gcttctbvht acttagtggt tagaactgat attgtagtgc accaagtgcc gattttaatt 240
aaaccaatac taaatactag taaataatac tagtggtctg aattcgattt ctatagtabv 300
htttgcttgc aagccgcaaa tagagtaaac attcgtcgtc acagaaatcc acattacatc 360

aaggtccbvh gcggccggcc acgtacccat cccacgcgtc gctgcggagg acacgtgttg 420
gctgaccgga cagttggccg atcagacagt ggacagaccg gacaatagaa gaagaagacg 480
acgacggcgg cggcaccgcc gagtaggtgc at 512

<210> 113
<211> 698
<212> DNA
<213> Zea
<400> 113

gtgattaagt tgactggcaa attgcataga cgataataca ctctbvhctt ttgagataac 60
cactgtacac caaccgactt gttgtttgaa ggctgatctt taaccattac tactgtaact 120
aactgttgtt gaataaacta ttcatacgac ttggagactt tgbvhtatat tbvhttttgt 180
acctatacta ttaactaccg cagatacgta tttacatctc ggtaacatct gccagtcgca 240
caabvhcact aaggtgactg acagactgta gcgaccgata gagcgctgcc ttgttctagt 300
caaaattcaa gcctggtggt cttctcgatc cctctgcgcg cgccctgctc ttttatcctg 360
tgtgtctcct ccacgaggga aggcgtcbvh agaggacgac acgacgaccb vhcagtgctg 420
cgcaaccgcg cgcgtctgcc tgttcactga gccccaggcg gccggtccaa gctattacca 480
cgtcgccccc ctccagggct gcgggcagca acgtgtcgac cggtccgcct ctgacgtgtc 540
cctcccgtcc tctgcataaa agggtgcggc tggcggggag cctcagcttc atatcgtcgc 600
aagctctccg tgtttctctc gtttcttcac tgctgccaac tgtgctgtgc agtagttgca 660
agagctgatt ggtagctagg tacgctaggc aagtaggc 698

<210> 114
<211> 1355
<212> DNA
<213> Zea
<400> 114

tataaattag aacggagggt bvhttttttt acgtttccat tatttgagtc tgctcatcac 60
gtttaatttg tttatacagt tcabvhagtt cagttacagc ggbvhataat tcgacggcgt 120
ttatttgcbv hcacttactt tttacattac gcctagabvh taatcggbvh gacgaatatt 180
acttgttttg tgtttatttt catatttttt tctcttcgga tacggatatt atcgagttgt 240
gccgaacaag attttgabvh tatatcgaca tcttaaatat ataactttga atatacggat 300
acgtatatag atcggatatt gaatacccag ctagactcag attaaattgg atcttagtta 360
ttttaggggg tgtttgabvh cactaaaact aatagttagt ggctaaaatt agttgaaaca 420
tccaaacacc ctatctaata gttcagctat tagttatttt tggaaaatta gttaatagtt 480
aggtagttat ctgttagcta gctaattcaa ctaattataa gttctagtgc atttaaatac 540
cccttagacg gatcgaatta gaacaccgbv hgataatatc cataacattt tacacctata 600
aatbvhtcct atbvhtatat agbvhtcaaa aacaggttgg gcbvhttggb vhacccgtgg 660
cacgatacag ttttaagcag tgccaaacbv hgcbvhgcgg cgagctagac bvhatacggt 720
ccggttttat ttttgtattt ttttttcgta ataatatctg tattbvhctt gttcgaacct 780
cacttgcttb vhtggbvhtg atagtatcaa ctcgctggcg ttccgtgcat cgtaacatct 840
atcacatccc acagatcbvh tcgcttatct tttctgatac tatagcagta gcacacagbv 900
hccggtcgaa cccagaagca tcacgccatc accccctccc cttttcccaa tcaaaaccac 960

gtccgtccca bvhgtaaccc agcaccaatc tcgtgttcgt gtgagacaga gcbvhcaaac 1020
aaacagcbvh cbvhcgcgaa ggaaagcgtg cagcgcgcgg gacgtgaacc gcgcgcgaac 1080
cctgccgccc gtccggtctc agctcacgcc cagcgtcctt cccaggccgc gatccggcca 1140
tcctgacgcg cgtttcgtct gggccccaaa accacgtccg ccgcggcggc ggcgcgccca 1200
cgggctcccg tgcccgccga tagtttgccg gaccctgccg cctcctataa bvhcagagcc 1260
ctccgccagc gtctcccgca tctcatcttc ttcctccatc cgcactactg agagcgacaa 1320
agctagctct ctccagggat cggattcgga tcacc 1355

<210> 115

<211> 1941

<212> DNA

<213> Zea

<400> 115

aagggactcg tggcctacac acgcgcacaa taatcagtta ccbvhaatta ttttatbvhc 60
gaatatcgac attacacbvh aatabvhtga atttgaattt ccgcagcbvh caaaattccc 120
gtgagtttga agggcgataa aaacgggtgg ccaaaattgg ggcggcabvh caagaagata 180
agcctatcca tcccttgcat tggtattgtc atctggggca gggcagggga gggcaggaga 240
agctatctat tggccatcta tccagcagcc gctctcccca gccccactct ctccatctag 300
agctccctaa ctgcaccggc cgccacacca ccgtaccccg acccctcgac aacabvhgcc 360
accgtcctag gcagcccccg cgcgccggcc ttcttcttct cgccgtcctc cctccgtgcc 420
gcgccggcgc ccaccgccgt ggcgctgcct gcggccaagg tgggcatcbv hggccgtagc 480
gccagcagca ggggcaggct gcgcgcgcag gccacctaca acgtgaagct gatcacgccg 540
gaggggggagg tggagctgca ggtgcccgac gacgtgtaca tcctggacca ggccgaggag 600
gacggcatcg acctgcccta ctcctgccgc gcggggtcct gctcctcctg cgccggcaag 660
gtcgtctccg gctccgtgga ccagtccgac cagagctacc tcgacgacgg ccagatcgcc 720
gccggctggg tgctcacctg ccacgcctac ccccacctctg acgtcgtcat cgagacgcac 780
aaggaggagg agctcaccgg cgcataatca ttcbvhccca tccatctcta tctctgtggg 840
tcgtcgttgt gtgtaatttg agtacgcgcg tacacgtact gcttttgttt catttgagca 900
ctgttcgtgt aagcgtcgag ttgccctgcc atcgtctctc tctctctcga tctctactat 960
ttctgtgtgt gcgcgcttct atttttccab vhttgagtta cgcatatttg tccagtaacc 1020
ttgttttgct taatcacaca cagctcccaa attccagtcg ttctgtggtg acggttggtt 1080
gtggggtgtg gcggcgacgt cagtcbvhgg agaagagatc cgabvhcbvh ggggtgttgg 1140
cgctttctct gtccctctcc ctctccctgt cttctcgcgc gctcgccgtc gtcbvhgctc 1200
gacgaacaga cattcactgc cttccccgcg cgcttcagac agaccacgct tgtacgtagc 1260
gccgcttctc tagtcgccgg agcaggcacg agtgggtggc gaagaggcca cggcccgacc 1320
tgctagcgcc agcgaccgca tctcgccacc agccgctgcc cgctggccgc tgccaccgtb 1380
vhttgggttg ggctgaagcc tgaagcccgt gaccttttc ttttcttta ttctttcctt 1440
ttcttttgcc ggacaagaca agtgbvhttg caaaatccat ataaaatcct agagaaacta 1500
taaagttttt ttttgttgaa ctccttataa caacgtctac cctttaatat bvhgtatata 1560
atttcacbvh ttttagtata aattgtttac tgtatttctb vhtctatbvh ctcttggtaa 1620
gattgtttat aaattttaaa tttaaaatct taaaagtcat aaacttatag aaaaaaatta 1680
ggatcctaaa caattttaaa caaaaaaaaa aactttcaa ttacaaaatc gtbvhtgttc 1740

tttbvhcbvh cbvhcaactg agaagctgcc caacggccag cgcagcagaa gggcagagcg 1800
gtgggcggcc acgtcgccgc cgcaaaggaa ctgacgtgtc ccggcccgac gacgacgacg 1860
cgaaccttca ccgcgccgcg ctccacctct cttaagctaa atacgctagt gcaggcgcag 1920
gcatccagct gcgacaacgt a 1941

<210> 116
<211> 1188
<212> DNA
<213> Zea
<400> 116

gagcgacctc ggactcagcg gctccgcctg tcggcgagcg cacgccgcgt gcctgcccgc 60
ggtctctcgt cgtctccaga agbvhcgccc cgatactcgt ctttccaccc ttcttctacc 120
tccttccacc cccttctccc cacgccccat cggatcgcac gcgcaccgca attgttgttc 180
atcacctctc cgaagaagcg tcacagagca ccgcccggag gttccccttc ccgtcgccgt 240
cgcttggcca ggtaagtabv hactaatcgc ctgagtctcc tgcgcctcat tacttcgaac 300
tgtggtttcg ttagtcggac gtcggagcab vhctbvhaac cttgccattc cbvhttactt 360
ggtgctgcgt gtccacattc gacgccacat ttttttcttg ctagccaacc attgcgcgtc 420
tgtgttacag ttccaccggc gatatacatt bvhcgatcat ttgbvhtgga tattggtgtt 480
tttttggtta gcggatcacc ttagttttgc actgaacbvh gaacaabvht ggbvhttggt 540
gtttttttgt tagtggatca ccttagtttt gcactgaagb vhgaacgagg aabvhgggaa 600
ggggaactga acgaagcaca tagtttcaga gactbvhtgc actgcagaga ctbvhcgabv 660
hgtcaaggga atttctcgcc ttggtttcct aatcctcgct tccttcttgt ttcgaattcg 720
gtgtatatbv htggtttatt cggcacgttt aggaatttag ttggggabvh gggcgcagtb 780
vhtcagggtt gtgtcatctg aatatatabv hcaagatcta ttactagttt aaattgactt 840
attbvhcaag tcgattaacc tggctgggct cagtggtgta ccbvhgaagc tttgtgattg 900
tagccgggca gtagaacgct aaacctgagg tttggtactg tagcactcgg ctagtcagct 960
gtbvhccaag bvhttactta ctcgatactt tctgccbvht ctcatctgtg ttaaccaggg 1020
tgtgtgccac atagggcttc tttccbvhct gtttgttaac ctgttcacac ttcttccata 1080
tatatattca taatagctcg atctacaatt ttcctgtctt gagcttcttt gttcctaaaa 1140
acgaattgta taccagcabv htatatctgb vhcaatattg tttgtagg 1188

<210> 117
<211> 1507
<212> DNA
<213> Zea
<400> 117

ctacatttbv httatagagg cgcaagtaga agabvhtgtt ataagttgta catcggaaaa 60
atagcbvhta aatctataga atcaattttc atctttcacc ccbvhaattt gaggtbvhct 120
tatbvhataa ctttagaaag tggtggabvh tcatattcta aaaaaaatag cctatttcat 180
tagtaagatt ccaattcctc gaabvhaaag aaaacaaacg gggccttatt agabvhgaat 240
tcaattccab vhatccaabv hgggcgtaag ggatttccac tttcctaaga aaaattagct 300
cattttccct tggtgtttaa taattbvhtt ttgttaaaag gtcagcttcc cgagabvhcg 360
ttgtgtaaaa gcccagtata agggtctgtt tggttgggct gtggctgtga aaaaagttgc 420

tgtgggctgt gagctgtgga aaaagctgct gtagactgta agctgttaaa aagctaaaaa 480
ccgtttggtg gaaaccacta aaagtcgtta aaaaatcttc gatatbvhtt ttcacagttc 540
catccgaaaa gccactaaaa gcaggtccag aggtgctttc agatttgcac tacgagaaag 600
tcggttttta gaaaaagctg cttcctggat ccagcccttt ggktggcttt tggctttwag 660
gggcacaaaa gccaaagcca aaagtcaaac caaacacacc ctaagtcgcg tgagtgggcc 720
atttatctat tccctbvhca aatctcctga aaaattacaa cattagctcg ttaaaaagta 780
aaataatttt aaaaaattag agactaatta tttgataact ttatagttca tcacattttc 840
attgaaabvh ttttattttt tttgccgaac tgatctacac aatatttgaa tctacacttt 900
tggcttgggc tcggagaaaa atagtgcgtg cccagccctt tgctctttcc ccgataacaa 960
cgcggtbvht ggcctaacgc ccaaatccgg cccatttatt ccggtgcgta gbvhcagacg 1020
cgggagcagg tgccaagtca ctggctcact gctgcaggtg gagtggtgga cactagtgcc 1080
gcggttcatc tgacacgtgt cgccacgtgc cgccbvhgca gcacctcagc ccggccggcg 1140
ggccgactga cgtcttgggc aaagcggcga gcgacgcagg cggcgaaagc catccgattt 1200
gacccctcgc tagacctttc aagaacgaac gctgtgctgc tcagatcaga ccgtgtctgc 1260
ctcaaagcgb vhccaggacg ccacgtccaa gcaaagcacc cgbvhccatt gccacctccc 1320
agcactcacg cgtgagcgtg actataaaaa acgcaccctc tgcatccgcc cccgtctgcc 1380
tgccctaccg aatctttcgc cgtcccatca gcccagcaat tcttcgctgt tcgaggaccc 1440
ctcggtttcg accgaagccc agcaagccga ccacacaccg ctgccgttgg ttccgtccca 1500
agagbvh 1507

<210> 118
<211> 910
<212> DNA
<213> Zea
<400> 118

catacgattt cctaagcgga atctcgagac gaaccacatt tttcatccgg tctbvhagcg 60
cgctcaatcg tcagtgacag agaacactgt aggtccttaa ctggaactaa caattcagaa 120
acaatcctaa bvhtttatta cactggbvhg attttatttg gatagbvhat actaccgttc 180
gtgggctaaa aagcaaggta aaaactggcc taacgcacgt gatcctcccb vhgtctcaga 240
agaaaaccac tcgcaaaaaa aaaaacgcag cabvhaggct gctttcagtt gacggtgtgc 300
acgactcgct gaagccagcg tgattttgtc ccggttcttg gagcccagca aaaagggcgc 360
ctgaaattcc acaggttgaa ggggaaaacg gcaacaaaag gggctccgac bvhcggtgca 420
gaaagtgaaa acgcgagaaa agcgcccgtc ctttctcgtt cccttccgtg cagttgcaag 480
tgttgccacg ccgcgccgcg acacaccacg caggaggcag cgagcgcaat abvhcgcacg 540
cagaggagca cagcggcgtc actgctgcgg cagtabvhgc cgcctggttc cacttccacc 600
acggtgtgca ctagcgctac accgaggcca ccttttcttg gttccttcaa cacgcctact 660
ccggcctccg gatcgggcac cacgtctgcc ccctgctccc ggaatctttg ccaagcggcg 720
ccgctactgt ccggcaaggc gaccccgtct ctcggccggc gttgatctgg tgbvhcgctt 780
ttcacggtgt tttttttttt ttttgtttca ctcccccctc cgtcctgtgt gattgtgctg 840
cagttttgtg ccggtgctaa cggaaaccgg aaagtttgcg tctttbvhtt actgtggttg 900
aggcgggcgg 910

<210> 119

<211> 1131
<212> DNA
<213> Zea
<400> 119
ccagccatcg tgcttgagtg abvhtttttt tgcgtgtgtg taggtgtgcg tgtgtgttta 60
attgatataa aataacccaa ccaaacatct cctaagattt ccataagaca gagaggtgag 120
gaatagtatt agttctcaab vhaattcaat caactaaata tcaccatcat atcaacatta 180
ataatagaac aataaaattt tcbvhataga gaggctggta aactttattt tttcatttga 240
ttccagtgtg gaaaatagta atataagcag taaatttttt cttaaaaaag agtggaatat 300
tcaccttttt atataaatta gttattcttc gaagcgtcac acacggbvht cctaaagata 360
ttcttcaact gcaacacaag cattctgaaa aggttagtaa caatcagatt abvhttgtaa 420
aaatbvhtga cacgaaaatt taaagaacgc gaagtacaat acattggtaa gaaatcaaag 480
acacatatta accaactatt agagaacata tacbvhtttc ttctaatcct ggttaggaca 540
taggacgagg gtttcacttc tatcacabvh tgctgcaact ttcagagtta atcaaatctg 600
ttattcagtg ttbvhcagcg acaaattcca abvhtataat cccacgbvha ttcbvhtgac 660
tccatttcgt ctgtaattgb vhcgaaactg ccgaattgtt actgctccca taagttaatt 720
gtaccacgct tgbvhaagac gttcacgtat cttgtgttcg atttbvhtag ccgttattca 780
gaccattgca gcgctgagct tcaaggcaat tagttttgcc ccgcgbvhtg aggcaggtgb 840
vhacacgcgc ccttcgggcc gbvhccgacg cgccaccgtg cagactctac cgaggbvhag 900
agcagcgatc gagagacgtg gagcgaggag gagactttag gagaggagag accagagcct 960
taagcggtga cacacccabv hatcaggacc acgcttgcac gtgtcaggtg cctctcctcc 1020
acgcgtcgcg cgcatcacac tgatactgtc atccagctcg acgctatata aagtggtaag 1080
catcttcgtc ctcggggccg ccccaacggc ccttgcgatc gccaccacgt c 1131
<210> 120
<211> 563
<212> DNA
<213> Zea
<400> 120
cattgttata catcggtgbv hcagatcttc gcttcgcbvh ggtggcaatt tagtaacaga 60
attgctctbv hagttattgc ttttagtttc tgtctttccc caactgcgtt tgagtttgca 120
ttgtcacbvh tggccttttt tggttgtaag acttgtttga tcggttgtgg tgggctgtca 180
tcctttggbv htaggccgga acctcttttc ccattatcta aaaaaataca tcgtcatttc 240
cagtgagacc cagcggtcgt gtcatcagaa aagcggacac gcgtcgcgtc gcgaggccag 300
gtgggttbvh cacggacctg ctbvhctagc cbvhctccag gctccagctc cacccgtcca 360
ccgacttttc ttctcaacca ggctgcctcc tcaccbvhca cgacgacacg tgcgbvhcca 420
tcacgcatcc acactcacct gttgcctcta tatccaccgc cccaccgccg tagcaccaga 480
aagaabvhta cagtctacac agctgaccag agagctagtg caagcgatct agcaagtttt 540
aatcttggaa gaagccagct agg 563
<210> 121
<211> 991
<212> DNA

<213> Zea

<400> 121

atcatcaccc taccccgagc tgactcgggc cgcagggaac cagaccggtg tcccatctgg 60
ctagctccgc cagataggca bvhaaggcgc cccgcbvhct ctgtgacgac ggcggctctc 120
agccccctta cggaagcaag aggacgtcag caaggaccca accgctccga cagctgtccc 180
tccgccaggc tccatcgctc ctccgacggc cacgacatcg caccagctgg gtgccaaaat 240
ctctccggct gccacgacgg cbvhtactta gggcgctagc tctcccccgc tagacacgta 300
gcactctgct acacccccca ttgtacacct ggatcctctc cttacaccta taaaggaag 360
gaccagggcc ctctcagaga aggttggccg cgcggggacg aggacgagac aggcgctcgc 420
ttggggccgc tcgctccctc tcccacgtgg acgcttgtaa ccccctactg caagcgcacc 480
cgacctgggc gcgggacgaa cacgaaggcc gcgggattcc cacctctctc acgccggtct 540
ccggccgcct cgctctcccc ccttcgcgct cgccctcgcg ctcgatccat ctgggctggg 600
gcacgcggcg acactcactc gtcggcccaa gggacccccg gtctcgaaac gccgacaatb 660
vhatatatag aaactatabv hatacacbvh caattaacta gtgctgataa tatatttaca 720
ctgtagattt ttccgataag tagaaaccaa atatttcacc gccacaaaag gcttcatccc 780
atcagttacc cbvhtagcaa aaccaaaata cttgtgtgcg agaggccaag agcccaaacc 840
accgccaagg aaaccccccag cccaccaggc gccttcctct ataaataccc taactccggt 900
ggccgaagtt cctcacccat tcactcactc atctcaaggc ctaggtagca ccgtagcagc 960
tactagaagc agctagccag aacaactcgt c 991

<210> 122

<211> 2519

<212> DNA

<213> Zea

<400> 122

cttccgataa aaatatttgg aaccgcatcc tgcatattta tttagaaaca ttgcatcgat 60
aaccgttgca gagcaatata cagggtactc accaactaaa tccataccat acagtgacag 120
gtgaaattgg aatacttgat cagcttgaat aacagagagc aaagataaat ctagccctgg 180
taataaccbv hgctcacatc ttbvhtacaa gagccaattc taaagacaca gctgcgggbv 240
htagcagtta ggcaaggaaa gaaacaaaat taccagctaa ctgctaagtg tcaaaacatt 300
gctttacact aaagggttaa atatttgcca agaaggtagt gggcacctct cctctgcaag 360
cagcagcata atctagggcg aggcttggtg cagcttcctt gtaggggcat bvhaacttat 420
ccbvhaaggc actctcactc atctggacag cagtatagta gctccggtcc tgctccaaca 480
atccattttc cttcacgcct agtttggatc actgtaattg aattccattc taataatagt 540
aatttagaca tatatcaatt aagataattc acttttgtac aaaatatatt tgtatactat 600
tattagcaat atatcctaaa tatttbvhtg ctacattttt actatagagg agtagaacga 660
agagtgtcat ataagttata gattagaaac aaattctaat cbvhcataaa atcatttccc 720
atcctccacc ctbvhaattt gagataggct tatatctgaa ctttagaaag tggtggabvh 780
tcaaattcca aactaaataa gttactttat tgagtgaatt ctaattcttt tgatttgaag 840
gaatccaaac gccccgtcag gtaattcaca acatagtgtc ggggcbvhag gcggcgttcc 900
aagctatbvh taagcagagc aggcctgtga gcabvhtact ctggctccaa cccaaccttg 960
gtgatcagga acgcggatcc gggcgtcgcc gggggcgcga ccctgtgctt gccaaattga 1020

aaatttagca aagtattttt tttaccttgt tgattbvhtt ggataaccgc bvhtccctaa 1080
tttgabvhat attgctcctt atctctaaac gaccaactgt agcttgttct attgaaaact 1140
catcagaata atccatagag ataacatcac caacaaggac tgaatcatcc agagbvhcat 1200
cabvhgccat tggagctagg gaagtcggcc taactacctt caacccgact tggccaactt 1260
gtacagtaga agacaccbvh gaaatcgatt taacggactc taagggagtg tttgaataca 1320
ctaaaactaa tagttttagt ggctaaaatt agttggagac atccaaacac cctagctaat 1380
attttagcta ttaactattt ttagtaaatt agttacaagt tagctagcta tttattagct 1440
agctaattct actaacaaat ttttagctaa ctaacgcatt caaacactgt ctaaacaggt 1500
aatctaggca cgcgaagtct gactggccag cccaaactca gcctttgtag tagagagagc 1560
cgaccttggc ggcctgcacg acatttttga actttttta tcggtttggt cttcattact 1620
ttatattctt tgtcaaccaa atactccctt cctcttaaat tataattcgt ttgatttttt 1680
ttbvhtaaag ttttbvhtat agtccaccta gttaacttta aaagttggtt agtctagatt 1740
ttbvhgaggc cgactacaat agcaaatcta acaataaaaa agttagctag atcagatttc 1800
aagaacagtc tgaccgattt tgaagatttg attcaggtta attctacgat tttgaagatc 1860
cgacctaacc taattttacg ttcagactaa agacbvhttt gtttaagatt bvhatbvhtc 1920
tagttatata atttaactta ttttaaacta acacttaatt taaaataagt tagattatbv 1980
hatcagaata ttatatttat ataattccaa acaaataact ctaatatcga ttctggttta 2040
tattgtataa tttttbvhtt gaatattacg gcgcgccaac aaattgcgat cgttbvhtaa 2100
tcbvhtatac gaccgactgt actattcata aaatataatt caaaatagga gbvhctcgag 2160
atagcctggc aagggaggag gtatcggagg cggcgtcgcc tgccgcgagc gcaccbvhcg 2220
tcaacgctca ggacctctcg caggcgcgct gttggatcgt gagcaagcac gcgtggcaab 2280
vhgcbvhcat tcctctcgtg caagtgccga gctgagggtc ccgacgcggg gggcaggacg 2340
tggcagagcg ccgcccaggc ggacgccaag tgccgagcta ccagggcacc ttctttattc 2400
cgtccttgct cagtcacacc tcgctctcgc tcactctcgc cgtccgcaca gccgctcatc 2460
gtctcccact gcctgccctc tccctgcgcc ggccggtgcc ccgacgcgcc bvhtcgtac 2519

<210> 123

<211> 1264

<212> DNA

<213> Zea

<400> 123

tcatcggtac tcgcgbvhtc gtgcgcgtcg acgactacta tatcaaggac tbvhacgtca 60
tcttgacacc agaggtgaca cagaggagga ccaacaacaa taaccactcg ccaccgtcgg 120
tcggtctgcc tttaattctc ttcgcaaaca catacacttg cttttttgtt taagcaagcg 180
tgtbvhbvhg tgcgtacctg bvhactgaca bvhtacgagg gaacttctac cggcaggtgt 240
aacacgtgct cttcabvhat aagatcbvhc aaatcgtgtc atatatcgaa gcctgcbvha 300
tactgbvhgt tgaaatatag tagtgaaaca gctaaattaa aataacaaaa tttbvhtbvh 360
gctaggatta caaataaatt ataaaatatt tttttataac aatataagac acattttgta 420
tataagttat tatattatta tbvhtttctg ttgcaacgca cgagtactca cctagtatat 480
ccataggctt ttgatcctct acgtcgagtt tttgcttgcb vhagagtcga cctcttctct 540
tttatccctt cttctctatc ttagcatttg ctcgatcaac tgtaaactaa ttattatacg 600
tacttcaagt catattgaaa ccabvhgcat ttaaaaaagt aaccatctga bvhgaaatat 660

caagctaaat aaagaagtta cgggagtbvh tgctgagaaa acctagacac attcagagaa 720
gttaccaagc aagccttaaa taacaatttt tggtgcttta ttgcagatag atactcctat 780
aggttggaga gaagggcggc gbvhctabvh caccbvhcbv htgtgtctgc ccatatactc 840
tatcttatct gaatcctacc aaactcttta atttgactct ttbvhabvhg aattcccatb 900
vhaattctat aacacaacag tacgacgtct actggagcat actgccaagc acgctagccg 960
acggctcggc gaccgtctga ggctgtccbv hcbvhcaabv htgcacacbv htacgcccct 1020
tttagcgctg gcbvhcaccc acgaccacga cacggctccc gtggccgtga atctgacatc 1080
ccgggactcc cgcctccggc cgcgcgtgcc gtgtcagcct ccatcgagcg gtgtactccb 1140
vhcaagcctc gacccgggcc acgtaccccc ctcccctccg ctacgtgtca ccgctctcgt 1200
cacctatatb vhgcggtctt gtccagtctt tccacttaac tactccgtca ctttgtttgc 1260
aaag 1264

<210> 124
<211> 623
<212> DNA
<213> Zea
<400> 124

ctcacacaaa tctaaatagt aaagtagatc tggttacaga gcagaaattt tgaagtgtca 60
tagataggct gagaatbvht agtcacgagc aacacattac tacttgaggc catcaatact 120
gttttttctt ctccaacctb vhtaactbvh ttattctttt cctccttaab vhtaaggcaa 180
acctcctatc ctttttcttt caaattcatt gcattaactg agtggttctt acbvhtagaa 240
bvhaagttgg agcaaagagg cttggtgcgg aaccgaaaat ttgttagtct ataaatcatt 300
agagggcgcc tgaagttcgc caabvhttct agtagatttt agtaaccacc cacgctagct 360
gactcgacca ccgtctgctg gctggcgctg tccbvhcacb vhtacagccc ctggcgctgg 420
cacgcaccca cggcatacct cccbvhactc tgacagcccg ggactccctc cgctcgtgcc 480
gtgtcagcct cactcgagcg gtgtactccb vhcaagcctc cacctcggcc acgtaccccc 540
tccccttcct tacgtgtcac cgctctcgct ccctatatac ggctgcctgg ccaggccttt 600
ccacttcact actctctctc tct 623

<210> 125
<211> 1950
<212> DNA
<213> Zea
<400> 125

ggttcaagat bvhtbvhtgb vhaacataat tttactcaaa agtgbvhgtt tcattgtact 60
aactagcatc aatacttbvh ccagagtgga gcagtgtgtc tbvhcactac tccgtacaag 120
agacbvhtcc ctcacgctac aaggagtbvh ggatcccagt tgactggttg tctgattccg 180
gagtagttgg caaggtgtgc atctctagca tacactccaa tttcgccaaa cbvhtgttaa 240
aaacbvhcat ttttaattcc ttbvhaaggt gggtbvhatt ttactcagag tbvhcaccta 300
abvhaactca ataatttatt tgcbvhttgg atatatcaaa ttggcatctg ttcagcttgc 360
gaacaagtat bvhaagaggg ttgcatcaga acttgbvhcg ttggaaggca ctgagaaaga 420
gcccaataga gagtttttgc ttctccaggg tggcagggtg tgaggttctc tttccgtgtt 480
catcaggtaa aatcctcacg ccaatatttt cagaacccag gtacttcaca ggtgatbvha 540

atatttcgaa tcagtaactt attcatctac cttctgcagt ttgctggggg atttgbvhca 600
gaaagcbvha aagcttttga agagctaaga agcaagbvha gtacacataa atctgctcca 660
cagatatctg aaggctgaag cbvhagttga acacaggatt caacacacat ctggtcttac 720
ttgctggata ctgattttta gttgtgtaca aaagagaagt ttaaattcca gtgtggtgtc 780
aagcaagttg tatbvhctaa gagaatacaa gtcatcaggt tbvhttcatt tattttttbv 840
haaagtaagg gcaggacagg ttbvhttcbv hcatataaca gttttagctc aagtgtatac 900
cttttcattt taaaggctgt aactgtaaac aggcagctcc agaaabvhaa tcacttgggc 960
bvhtttttgt tgctacgcat atactactaa taaabvhtca aaagcgtcca ttcggttagt 1020
ccggtacccb vhgagataat tcgtctgabv haaccttgcc gtctttgtta aaagaaaaaa 1080
aaattacttt gtgcctcacc gcattcgata ctttgaagaa attgacccgt taccagttac 1140
cacccacgca catacagtgc tagaacagca gatctgactt gaagaagcat atatcaagac 1200
aaaagtccac ctgaacggag acagtcgagg ttgtggcgga gagctccacc tgaacgccga 1260
agtggaaccg aacagttcca gtggttggcc gtggcgagct ggccacctac tagtctccgg 1320
ggagggggct ggctccagcg cctccgagcg cggtgccagt gtcgccgtcg cgtgggttcc 1380
ggttccggcc gccgccgctt gccggggaat ccaaacggca gacggggtcg ccgtgaccgt 1440
gaatcgtgtt tacttgggcc ttatctaggc cacaacgttt gtgactctgt gagcttattg 1500
tttgggccca tctctttggc gtgtctgaga acgaatttta tcatcaagcc cacgaagcca 1560
aaccaggatc ctacccacct agcacatact agtgaagaag gtaaacaaag tgtattttcb 1620
vhttaattca cbvhcgcgtg gtcgacacca caccacacga cttaaaactc ggtgttbvht 1680
tctacgacca caagagccbv httaatttta agccgtgtct gcgccbvhat ccgaagagcg 1740
cgtacagtga gagaagaaac gcacggccgc cagcagctgc cgcgggagcg aggcagcacg 1800
tccgttggtc ggcccgggcg ggagcgaggc acacgtcgcg cbvhcacgcg cggcgcccgg 1860
acacgtgccg gcgcgggcac gtccctctcc gcgccttata ctcctccccg ccatttccca 1920
gcagccacaa caagacagag agatacccga 1950

<210> 126

<211> 2112

<212> DNA

<213> Zea

<400> 126

ctaggttggt gagatcctta ggttttggaa ggctttbvht ggcagctctg aagactccaa 60
catctbvhgc ctctagctbv hcctccatbv htattttgta ataagaaaaa tcatctccct 120
taaacbvhgg agagggttca tctccatcga acatctttct ttaggtggtg aagcctaaaa 180
tabvhagcac gacactttga tactaattga aaggattact bvhcccaaga ggtagggtgg 240
attagaccaa tctaaaattt cttgtagcaa taaaatctta tcgaatatcc cacttcacca 300
cttgtgctta agagtgatat atattctatc acaaaaggtt ttacacccta gtttgaaccc 360
tattctagtb vhttagttct agtabvhtaa agacaagaat taaattgcta aabvhcaabv 420
hcttaagata aagaagggtt agaaacbvha cagcattttt ccaaagtatt aaagagccgg 480
gactctccaa tagtcctcgt tggagcaccc acacaaggat attgctcccc attgaacttc 540
acaaggatca agggctctct bvhggtttgt tcctcgccac tctagcbvhg tgagtcaccc 600
acagttgttt acaccttgag tggtgatata cactagattt cttaagtgtg tatcatcaga 660
cttatttagg tcaaactatc attcbvhgcc cctttatagt atagtcaaag gaaaaaacaa 720

aggtgctatc actagtatat bvhtctatca actctattaa ctattagaac tagccagtcc 780
ttaacctttt tgctcatcct tctaaaatca ataattgaat accatcaata ggggcacaaa 840
aactatatag ccaattatta ttactcatag ccaataattt agtgttgtca ttagtcacta 900
aaactagbvh ctttcatatc tatcbvhctc gtgtttgcct gatccaattc ctaggtcbvh 960
tttgggctcc acttagagct tgtabvhgca tcacaacbvh atctgattaa gaaabvhttg 1020
actaaagacc ttctcaccca catataaabv hccbvhactt cgctcaaact tcatccccta 1080
gtctcctact acacccbvht ctbvhcttcg caacttcaca agtctaccac bvhtacttgc 1140
aaagggctga ttctcctagc tcccbvhtcg tcgccatctt tccccttac caccacaaaa 1200
tcacaabvht gctcacttat tatccccgcg tcttcccttc cctaccctaa acactbvhgg 1260
caacaacgtc gcatcacttg gtggtccttg tagatctaga cgaggtctcc tccccgtttt 1320
gtgcttgttg atatagtgat cttgtccttg tcttgtcgct gtcctttgga gtcgtcgcag 1380
agaggcggtg aaaattctag ccgcttctag aaggtgcggt cgccggtcgg bvhagagtcg 1440
agtgagtgca ggcctataaa aagtagcaat tggataacat tttgaaaaaa bvhaaagaaa 1500
taaaaaaact agggttaaat aagattctaa attaaatcta agaaatctat agacattcgg 1560
aaatctattt atcttttgta ctcctttgat tggatttcta gattttgacg tttgtgtaat 1620
tgggcttgac agccccatca atacaggcag gccagctgat ttcgggtgcc gctgatttgg 1680
cccbvhgagg atagagcagg cctttcaaca ggcaggcagt ccbvhacgta gtgcctcggc 1740
ctcaaagccc bvhcgattaa tatccactgg tacgtacgcg acagccgagg gagtcactga 1800
gtcagtgccg aacggcctgg aaaccccccg ccgtgtgccg gtgtccacgc gcgccccggt 1860
gcggccgcca cgaccgcgga cacgtgccgg gcctcgcaca cgcgtccccc acggcccccct 1920
cgcatcatta aaaccgcggt gcccgcgcca cggatccccg gcacttcatt ccggtttccg 1980
gatagcttac ctcccatcga cggtcggtga gccgttggtg agtggcgaga cagcgggtgc 2040
gctgggcaag aacaggcgac gaagaagbvh agcacgccgg acacgcggac gctcaaggac 2100
gagctgacga gc 2112

<210> 127
<211> 1106
<212> DNA
<213> Zea
<400> 127

ttggtttttt gataatttgt ttatctracb vhbvhgttct aacaatbvhc gcagatctta 60
taratctctc tcgtagtttt trataaacta rgagaaagat ataggttttb vhaacaaatt 120
tarttttatt ttgtratata bvhaabvhgt raaaatataa attgtacatc bvhbvhagtt 180
ataraaattt gtagtccaaa atcttttrat ttaaattaat ttartgcttt aaabvhtgat 240
ttttaattgt ctttgcctag tgttgaaaaa aagcactcgg caaagaaact ctttgccgag 300
tgttaaaaaa acactcggca aagaatctct ttgccgagtg traaaaataa aatatttggc 360
aaatagcttc tttgctgagt gtttttttgc ttagcacttg gtaaagaact tctttgccga 420
gtgtccgaaa aagcactcga taaaatartt ggcactragc aaagaaccga attccagtag 480
cgtgagtrag aagttggcat ttgaacccttt tgaaataatr aaacaaatra acgcagttgc 540
atbvhaattg gctttgccac bvhgtagtgr agtccatcga gtgttccbvh tttracagct 600
taaactaacc cbvhgttttg gtaacagaac acacaccagt gactccatcg ttarcggatt 660
ttgatartar actgaaggtt ccggaacaaa tartggattc tagattgatt ctagattgct 720

tcgatraabv hgggatratc aacctaacac agcttaaatt atacbvhcbv hcgtarattt 780
ggtccttraa gaggaggcag gtgbvhatca aggtctctct agcggcctgg tgtgcagcca 840
cgtarccatc cacgcgtcgc cgcgggcgac gacaggtgtt ggccaacctg gacatragac 900
aagcacgaca bvhacaggcg ttcgccaagc gtacgtarag gagacgaaca cgacaagtag 960
caccgtcctg gccgagagcc cgtcbvhctc bvhcbvhcbv hcbvhctgat cbvhcatart 1020
cgataaatat aragcagaga gctgagctar gtacacaaac aagaagcttg cattgttcgt 1080
tgctcgatcg ttgcaggtab vhgaga 1106

<210> 128

<211> 922

<212> DNA

<213> Zea

<400> 128

ttagtagaga ataacacaca tctccctaag tatracaaaa aaattaattt taagaggcaa 60
ttttagtttc caaagcgatt tartaagatt taagggaggg tatttartag aaggaagtar 120
attgatraaa cagagaaaat tccatrattb vhgtcccccca tagaaatttg tcactcgatt 180
gtcgcaaaaa aagttaccag taaatttgat aaattttgga taccggtctg ttttaactca 240
tcaagaatag cacgacgaaa taatagattg cgtacgtttt rattcataac atctctract 300
aaagcatttt ttaracacgc tattaaabvh gttcggaaac tgatccttaa gcggtctatt 360
catatccatc tatcgttcta agattcttcc tgtargtaga ttctttccga ttaaattctc 420
bvhaaaaatt aaaaacaaac aggtccttaa actagtcgcc ggaagttctc gcttttgctg 480
tagttgtagt gtcgbvhcbv hggtggaaac gtggacagat agattcbvha abvhtggaca 540
acccacctgg trragagcat cbvhatacat tgataracgt ggcacgctgc ccactgbvhc 600
cctragcttt gaagctaacg tgtcaagtgt ggccggcccc tgcattgcgt cgtgccaatc 660
gtccaagtcc cbvhgcaaaa acccagcgct ttgtgccgcc gccgtccgcc ggccccctctg 720
cccttgtarg tgcacctaga cacatcgtra tcgatcatra cacgcaatcg acacaagaag 780
ttaataaaca gcccaaggac gcagagatra gctgatcgag aaggacttgt artactactc 840
agtattgtcg tracbvhcac atatbvhtac ataaagagct agctarctga gctctaccca 900
aggtcgcgtt gatcgatcga tc 922

<210> 129

<211> 1945

<212> DNA

<213> Zea

<400> 129

gattcagaac atctggtcag cttaabvhbv htggtaaagg gagcattatt cttttatttt 60
gttctttgat ctgtgcabvh gccatatart ttcgtattga tbvhctctra gtggaacagt 120
ctgaagctab vhtgaaactt cgbvhttcag gtbvhatabv htaccaacag agatraaccg 180
gctgagbvhc agagttaact arcbvhcatt aaagttccta rctgatattg aggaabvhgc 240
tgttaagctt gctgcaagab vhaggaacaa aactggcagc ataaatccat arbvhtacgt 300
tataattatc cttttcgctg ggtgatartg tgtaragtag agcttgtatc tacccaagac 360
actarccagt gcatagctga agttgacgct ctcaaagttt atttcacaat caaatartga 420
cacatttttt ttgttcttaa agggctcttc acctgagatt tgaaaaaggg bvhgtggggc 480

tttctttctg tgattttgrc ggcacacggg aggagaaggc abvhbvhgcg gcttataggc 540
agaaagabvh gccaaggcgc ttcaaggtga cgcggactgg actagatara tctgttarat 600
tggactgaga aagaatccct aggagaabvh aagcaccgac actaactata rgcgctagtt 660
cgggaacccc gttttctcac gagactttta rttttccaag aaaaattagt tcattttttt 720
taggaaaata raaatctctt aagaaabvht agttccgaaa ctagccctac aagtctaacg 780
attttctctt ttttgtarct gccacgcaga bvhgatccca tctbvhgcca ctggcgctgc 840
agaagagaaa agaagggcgg tgccctcttg agcccggtga gatcgctgtg atcctgcggg 900
cactggggta racgagcggc acacagatat argtcgcgtc cgggcaagtg targgcggca 960
agaaccggbv hgctcccctc aggaacbvht tccccaacct ggtaggcagg ctcaaatcca 1020
cttcagattg tgtraagctg aagccagtga taactgataa gccactgccg tgcattgtgc 1080
atcgctgcag gtgacgaagg aggagctagc gagcgcggag gagctrrcgc cgttccggcg 1140
gcacgtgacg agcctggcgg cgctggactt cctggtgtgc ctgcggtrrg acgcgttcgt 1200
gbvhacgcac ggcggcaact tcgccaaact ratcatcggg gcgcgccgct argcggggca 1260
ccgcctraag tcggtgaagc ccgacaaggg cctcbvhtcc aagtccctrr gcgaccccga 1320
cbvhggctgg gcctccttra cggaggacgt cgtcgtracg caccgcacga ggacgggcct 1380
ccccgagccc accttccccg gctargatct ctgggagaac ccgctgacac cctgcbvhtg 1440
cagggcbvha gacgggccat ccctragatr agagtggccg tctggccgag tcctcgtctt 1500
cgctaggcta rgactacgag tagtctcgtg cccacttcca tcagcgcgca tttragcgag 1560
catbvhcttc tartagttaa acacctagat ttgtbvhtat ttggctggcc taatctgtag 1620
tttcacatag atracttcbv hctgaaabvh tcttggacaa ggcagtgabv haagataaca 1680
tragtaacga cctgcttttc caaagctcct cttggattta rcaagctgag ccaagaabvh 1740
gctaacctga gttttgactr rcagccttgc aggcbvhacg tgggcgacct gaactcgcta 1800
gtcgtagagt ttctttccac gcggcagctc ggcagagcta rgcagcctct ctgrccgcca 1860
cgttctcctg caaggctgca actcgaagca ctgagaagct artaattabv hgattctcga 1920
gcbvhcggcc ttgttcgcta aacct 1945

<210> 130

<211> 512

<212> DNA

<213> Zea

<400> 130

gccagtgcta bvhatattta ractagcggg ctgctaaaga aaaccgccag tgctaaagat 60
arttacacta gcggttggtg aacaactgcc tgtgaaaaaa gccgatttct artagcccct 120
agctagcact ggcgacataa aaaacgtrag tgaaaatagc tctaggatcg tractataga 180
gcttctbvht acttagtggt tagaactgat attgtagtgc accaagtgcc gattttaatt 240
aaaccaatar taaatartag taaataatar tagtggtctg aattcgattt ctaragtabv 300
htttgcttgc aagccgcaaa tagagtaaac attcgtcgtr acagaaatcc acattaratr 360
aaggtccbvh gcggccggcc acgtacccat rccacgcgtc gctgcggagg acacgtgttg 420
gctgaccgga cagttggccg atragacagt ggacagaccg gacaatagaa gaagaagacg 480
acgacggcgg cggcaccgcc gagtaggtgc at 512

<210> 131

<211> 698

<212> DNA
<213> Zea
<400> 131
gtgattaagt tgactggcaa attgcataga cgataatara ctctbvhctt ttgagataac 60
cactgtarac caaccgactt gttgtttgaa ggctgatctt taaccattac tartgtaact 120
aactgttgtt gaataaacta ttratacgac ttggagactt tgbvhtatat tbvhttttgt 180
arctatacta ttaactaccg cagatacgta tttaratctc ggtaacatct gccagtcgca 240
caabvhcact aaggtgactg acagactgta gcgaccgata gagcgctgcc ttgttctagt 300
raaaattcaa gcctggtggt cttctcgatc cctctgcgcg cgccctgctc ttttatcctg 360
tgtgtctcct ccacgaggga aggcgtcbvh agaggacgac acgacgaccb vhcagtgctg 420
cgcaaccgcg cgcgtctgcc tgttcactga gccccaggcg gccggtccaa gctattarca 480
cgtcgccccc ctccagggct gcgggcagca acgtgtcgac cggtccgcct ctgacgtgtc 540
cctcccgtcc tctgcataaa agggtgcggc tggcggggag cctragcttr atatcgtcgc 600
aagctctccg tgtttctctc gtttcttrac tgctgccaac tgtgctgtgc agtagttgca 660
agagctgatt ggtagctagg targctaggc aagtaggc 698
<210> 132
<211> 1355
<212> DNA
<213> Zea
<400> 132
tataaattag aacggagggt bvhttttttt argtttccat tatttgagtc tgctcatrac 60
gtttaatttg tttataragt tcabvhagtt ragttaragc ggbvhataat tcgacggcgt 120
ttarttgcbv hcacttartt tttacattar gcctagabvh taatcggbvh gacgaatart 180
arttgttttg tgtttatttt ratatttttt tctcttcgga tacggatatt arcgagttgt 240
gccgaacaag atttgabvh tatatcgaca tcttaaatat ataactttga atatarggat 300
argtatatag atcggatatt gaatarccag ctagactrag attaaattgg atcttagtta 360
ttttaggggg tgtttgabvh cactaaaact aatagttagt ggctaaaatt agttgaaaca 420
tccaaacacc ctarctaata gttragctat tagttatttt tggaaaatta gttaatagtt 480
aggtagttar ctgttagcta gctaattraa ctaattataa gttctagtgc atttaaatar 540
cccttagacg gatcgaatta gaacaccgbv hgataatatc cataacattt taracctata 600
aatbvhtcct arbvhtatar agbvhtraaa aacaggttgg gcbvhttggb vhacccgtgg 660
cacgatarag ttttaagcag tgccaaacbv hgcbvhgcgg cgagctagac bvhatarggt 720
ccggttttat ttttgtattt ttttttcgta ataatarctg tattbvhctt gttcgaacct 780
cacttgcttb vhtggbvhtg atagtatraa ctcgctggcg ttccgtgcat cgtaacatct 840
arcacatccc acagatcbvh tcgcttarct tttctgatar tatagcagta gcacacagbv 900
hccggtcgaa cccagaagca tcacgccatr accccctccc cttttcccaa traaaaccac 960
gtccgtccca bvhgtaaccc agcaccaatr rcgtgttcgt gtgagacaga gcbvhcaaac 1020
aaacagcbvh cbvhcgcgaa ggaaagcgtg cagcgcgcgg gacgtgaacc gcgcgcgaac 1080
cctgccgccc gtccggtctr agctcacgcc cagcgtcctt cccaggccgc gatccggcca 1140
tcctgacgcg cgttrrgtct gggccccaaa accacgtccg ccgcggcggc ggcgcgccca 1200
cgggctcccg tgcccgccga tagtttgccg gaccctgccg cctcctataa bvhcagagcc 1260

ctccgccagc gtctcccgca tctratcttc ttcctccatc cgcactartg agagcgacaa 1320
agctagctct ctccagggat cggattcgga tracc 1355

<210> 133
<211> 1941
<212> DNA
<213> Zea
<400> 133

aagggactcg tggcctarac acgcgcacaa taatragtta ccbvhaatta ttttarbvhc 60
gaatatcgac attaracbvh aatabvhtga atttgaattt ccgcagcbvh caaaattccc 120
gtgagtttga agggcgataa aaacgggtgg ccaaaatrgg ggcggcabvh caagaagata 180
agcctatcca tcccttgcat tggtartgtr atctggggca gggcagggga gggcaggaga 240
agctarctar tggccatcta tccagcagcc gctctcccca gccccactct ctccatctag 300
agctccctaa ctgcaccggc cgccacacca ccgtarcccg acccctcgac aacabvhgcc 360
accgtcctag gcagcccccg cgcgccggcc ttctrrttct cgccgtcctc cctccgtgcc 420
gcgccggcgc ccaccgccgt ggcgctgcct gcggccaagg tgggcatcbv hggccgtagc 480
gccagcagca ggggcaggct grgcgcgcag gccacctara acgtgaagct gatracgccg 540
gagggggagg tggagctgca ggtgcccgac gacgtgtara tcctggacca ggccgaggag 600
gacggcatcg acctgcccta rtcctgccgc gcggggtcct gctcctcctg cgccggcaag 660
gtcgtctccg gctccgtgga ccagtccgac cagagctarc tcgacgacgg ccagatcgcc 720
gccggctggg tgctracctg ccacgcctar cccacctctg acgtcgtrat cgagacgcac 780
aaggaggagg agctraccgg cgcataatra ttcbvhccca tccatctcta tctctgtggg 840
tcgtcgttgt gtgtaatttg agtargcgcg taracgtact gcttttgttt catttgagca 900
ctgttcgtgt aagcgtcgag ttgccctgcc atcgtctctc tctctctcga tctctartat 960
ttctgtgtgt gcgcgcttct atttttccab vhttgagtta rgcatatttg tccagtaacc 1020
ttgttttgct taatracaca cagctcccaa attccagtcg ttctgtggtg acggttggtt 1080
gtggggtgtg gcggcgacgt ragtcbvhgg agaagagatc cgabvhcbvh ggggtgttgg 1140
cgctttctct gtccctctcc ctctccctgt cttctcgcgc gctcgccgtc gtcbvhgctc 1200
gacgaacaga cattractgc cttccccgcg cgcttcagac agaccacgct tgtargtagc 1260
gccgcttctc tagtcgccgg agcaggcacg agtgggtggc gaagaggcca cggcccgacc 1320
tgctagcgcc agcgaccgca trrcgccacc agccgctgcc cgctggccgc tgccaccgtb 1380
vhttgggttg ggctgaagcc tgaagcccgt gaccttttc ttttcttta rtctttcctt 1440
ttcttttgcc ggacaagaca agtgbvhttg caaaatccat ataaaatcct agagaaacta 1500
taaagttttt ttttgttgaa ctccttataa caacgtctar cctttaatat bvhgtatata 1560
atttracbvh ttttagtata aattgtttar tgtatttctb vhtctatbvh ctcttggtaa 1620
gattgtttat aaattttaaa tttaaaatct taaaagtrat aaacttatag aaaaaaatta 1680
ggatcctaaa caattttaaa caaaaaaaaa aacttttcaa ttacaaaatc gtbvhtgttc 1740
tttbvhcbvh cbvhcaactg agaagctgcc caacggccag cgcagcagaa gggcagagcg 1800
gtgggcggcc acgtcgccgc cgcaaaggaa ctgacgtgtc ccggcccgac gacgacgacg 1860
cgaaccttra ccgcgccgcg ctccacctct cttaagctaa atargctagt gcaggcgcag 1920
gcatccagct gcgacaacgt a 1941

<210> 134

<211> 1188
<212> DNA
<213> Zea
<400> 134
gagcgacctc ggactragcg gctccgcctg tcggcgagcg cacgccgcgt gcctgrccgc 60
ggtctctcgt cgtctccaga agbvhcgccc cgatartcgt ctttccaccc ttcttctarc 120
tccttccacc cccttctccc cacgccccat cggatcgcac gcgcaccgca attgttgttr 180
atcacctctc cgaagaagcg tracagagca ccgcccggag gttccccttc ccgtcgccgt 240
cgcttggcca ggtaagtabv hactaatcgc ctgagtctcc tgcgcctrat tacttcgaac 300
tgtggtttcg ttagtcggac gtcggagcab vhctbvhaac cttgrcattc cbvhttartt 360
ggtgctgcgt gtccacattc gacgccacat ttttttcttg ctagccaacc attgcgcgtc 420
tgtgttarag ttccaccggc gatataratt bvhcgatcat ttgbvhtgga tartggtgtt 480
tttttggtta gcggatracc ttagttttgc actgaacbvh gaacaabvht ggbvhttggt 540
gtttttttgt tagtggatra ccttagtttt gcactgaagb vhgaacgagg aabvhgggaa 600
ggggaactga acgaagcaca tagtttraga gactbvhtgc actgragaga ctbvhcgabv 660
hgtcaaggga atttctcgcc ttggtttcct aatcctcgct tccttcttgt ttcgaattcg 720
gtgtaratbv htggtttart cggcacgttt aggaatttag ttggggabvh gggcgcagtb 780
vhtcagggtt gtgtratctg aataratabv hcaagatcta rtactagttt aaattgactt 840
artbvhcaag tcgattaacc tggctgggct ragtggtgta rcbvhgaagc tttgtgattg 900
tagccgggca gtagaacgct aaacctgagg tttggtartg tagcactcgg ctagtragct 960
gtbvhccaag bvhttactta rtcgatactt tctgccbvht ctratctgtg ttaaccaggg 1020
tgtgtgccac atagggcttc tttccbvhct gtttgttaac ctgttracac ttcttccata 1080
ratatattca taatagctcg atctacaatt ttcctgtctt gagcttcttt gttcctaaaa 1140
acgaattgta raccagcabv htatatctgb vhcaatattg tttgtagg 1188
<210> 135
<211> 1507
<212> DNA
<213> Zea
<400> 135
ctacatttbv httatagagg cgcaagtaga agabvhtgtr ataagttgta ratcggaaaa 60
atagcbvhta aatctaraga atraattttc atctttracc ccbvhaattt gaggtbvhct 120
tarbvhataa ctttagaaag trgtggabvh tcatartcta aaaaaaatag cctarttcat 180
tagtaagatt ccaattcctr gaabvhaaag aaaacaaacg gggccttart agabvhgaat 240
traattccab vhatccaabv hgggcgtaag ggatttccac tttcctaaga aaaatragct 300
cattttccct rggtgtttaa taattbvhtt ttgrtaaaag gtragcttcc cgagabvhcg 360
ttgtgtaaaa gcccagtara agggtctgtt tggttgggct gtggctgrga aaaaagttgc 420
tgtgggctgt gagctgtgga aaaagctgct gtagactgta agctgttaaa aagctaaaaa 480
ccgtttggtg raaaccacta aaagtrgtta aaaaatcttc gatatbvhtt ttracagttc 540
catccgaaaa gccactaaaa gcaggtccag aggtgrtttc agatttgcac targagaaag 600
trggttttta gaaaaagctg cttcctggat ccagcccttt ggktggcttt tggctttrag 660
gggcacaaaa gccaaagcca aaagtraaac caaacacacc ctaagtcgcg tgagtgggcc 720

atttarctat tccctbvhca aatctcctga aaaatracaa catragctcg traaaaagta 780
aaataatttr aaaaaatrag agactaatta rttgataact ttatagttca tracattttc 840
attgaaabvh ttttartttt tttgccgaac tgatctarac aatatttgaa tctaracttt 900
tggcttgggc trggagaaaa atagtgcgtg cccagccctt tgctctttcc ccgataacaa 960
cgcggtbvht ggcctaacgc ccaaatccgg cccattratt ccggtgcgta gbvhcagacg 1020
cgggagcagg tgccaagtra ctggctcact grtgcaggtg gagtggtgga cactagtgcc 1080
gcggttratc tgacacgtgt cgccacgtgc cgccbvhgca gcacctragc ccggccggcg 1140
ggccgactga cgtcttgggc aaagcggcga gcgacgcagg cggcgaaagc catccgattt 1200
gacccctcgc tagacctttc aagaacgaac gctgtgctgc tragatcaga ccgtgtctgc 1260
ctraaagcgb vhccaggacg ccacgtccaa gcaaagcacc cgbvhccatt gccacctccc 1320
agcactracg cgtgagcgtg actataaaaa acgcaccctc tgcatccgcc cccgtctgcc 1380
tgccctarcg aatctttcgc cgtcccatca gcccagcaat tcttcgctgr tcgaggaccc 1440
ctcggtttcg accgaagccc agcaagccga ccacacaccg ctgrcgttgg ttccgtccca 1500
agagbvh 1507

<210> 136
<211> 910
<212> DNA
<213> Zea
<400> 136

catacgattt cctaagcgga atctcgagac gaaccacatt tttratccgg tctbvhagcg 60
cgctraatcg tcagtgacag agaacactgt aggtccttaa ctggaactaa caattragaa 120
acaatcctaa bvhtttarta ractggbvhg attttarttg gatagbvhat actaccgttc 180
gtgggctaaa aagcaaggta aaaactggcc taacgcacgt gatrrtcccb vhgtctraga 240
agaaaaccac tcgcaaaaaa aaaaacgcag cabvhaggct gctttragtt gacggtgtgc 300
acgactcgct gaagccagcg tgattttgtc ccggttctrg gagcccagca aaaagggcgc 360
ctgaaattcc acaggttgaa ggggaaaacg gcaacaaaag gggctrrgac bvhcggtgca 420
gaaagtgaaa acgcgagaaa agcgcccgtc ctttctcgtt cccttccgtg cagtrgcaag 480
tgttgrcacg ccgcgccgcg acacaccacg caggaggcag cgagcgcaat abvhcgcacg 540
cagaggagca cagcggcgtr actgctgrgg cagtabvhgc cgcctggttc cacttccacc 600
acggtgtgca ctarcgctac accgaggcca ccttttrrtg gttccttraa cacgcctart 660
ccggcctccg gatcgggcac cacgtctgcc ccctgctccc ggaatctttg ccaagcggcg 720
ccgctartgt ccggcaaggc gaccccgtct ctcggccggc gttgatctgg tgbvhcgctt 780
ttracggtgt ttttttttt ttttgtttca ctcccccctc cgtcctgtgt rattgtgctg 840
cagttttgtg ccggtgctaa cggaaaccgg aaagtttgcg tctttbvhtt actgtggttr 900
aggcgggcgg 910

<210> 137
<211> 1131
<212> DNA
<213> Zea
<400> 137

ccagccatcg tgcttgagtg abvhtttttt tgcgtgtgtg taggtgtgcg tgtgtgttta 60

attgatataa aataacccaa ccaaacatct cctaagattt ccataagaca gagaggtgag 120
gaatagtart agttctraab vhaattraat caactaaata traccatcat atcaacatta 180
ataatagaac aataaaattt tcbvhataga gaggctggta aactttartt tttcatttga 240
ttccagtgtg gaaaatagta atataagcag taaatttttt cttaaaaaag agtggaatar 300
tcaccttttt arataaatra gttartcttc gaagcgtrac acacggbvht cctaaagata 360
ttcttraact gcaacacaag cattctraaa aggttagtaa caatragatt abvhttgtaa 420
aaatbvhtga cacgaaaatt taaagaacgc gaagtacaat arattggtaa gaaatraaag 480
acacatatta accaactart agagaacata racbvhtttc ttctaatcct ggttaggaca 540
taggacgagg gtttracttc tatracabvh tgctgcaact ttragagtta atcaaatctg 600
ttattragtg ttbvhcagcg acaaattcca abvhtataat cccacgbvha ttcbvhtgac 660
tccatttcgt rrgtaattgb vhcgaaactg ccgaattgtt artgctccca taagttaatt 720
gtarcacgct tgbvhaagac gttracgtar cttgtgttcg atttbvhtag ccgttartca 780
gaccattgca gcgctgagct traaggcaat tagttttgcc ccgcgbvhtg aggcaggtgb 840
vhacacgcgc cctrrgggcc gbvhccgacg cgccaccgtg cagactctar cgaggbvhag 900
agcagcgatc gagagacgtr ragcgaggag gagactttag gagaggagag accagagcct 960
taagcggtga cacacccabv hatcaggacc acgcttgcac gtgtraggtg cctctcctcc 1020
acgcgtcgcg cgcatracac tgatactgtc atccagctcg acgctatata aagtggtaag 1080
catcttcgtc ctcggggccg ccccaacggc ccttgcgatr rccaccacgt c 1131

<210> 138

<211> 563

<212> DNA

<213> Zea

<400> 138

cattgttata ratcggtgbv hcagatcttr rcttcgcbvh ggtggcaatt tagtaacaga 60
attgrtctbv hagttartgc ttttagtttc tgtctttccc caactgcgtt tgagtttgca 120
ttgtcacbvh tggccttttt tggttgtaag acttgtttga tcggttgtgg tgggctgtra 180
tcctttggbv htaggccgga acctcttttc ccattarcta aaaaaataca tcgtratttc 240
cagtgagacc cagcggtcgt gtcatragaa aagcggacac gcgtcgcgtc gcgaggccag 300
gtgggttbvh cacggacctg rtbvhctagc cbvhctccag gctccagctc cacccgtcca 360
ccgacttttc ttctraacca ggctgcctcc traccbvhca cgacgacacg tgrgbvhcca 420
tcacgcatcc acactracct gttgcctcta tarccaccgc ccccaccgccg tagcaccaga 480
aagaabvhta cagtctarac agctgaccag agagctagtg caagcgatct agcaagtrrt 540
aatcttggaa gaagccagct agg 563

<210> 139

<211> 991

<212> DNA

<213> Zea

<400> 139

atcatcaccc tarcccgagc tgactcgggc cgcagggaac cagaccggtg rcccatctgg 60
ctagctrrgc cagataggca bvhaaggcgc cccgcbvhct ctgtgacgac ggcggctctc 120
agccccctra cggaagcaag aggacgtrag caaggaccca accgctccga cagctgtccc 180

tccgccaggc tccatcgctc ctccgacggc cacgacatcg caccagctgg gtgrcaaaat 240
ctctccggct gccacgacgg cbvhtactra gggcgctagc tctcccccgc tagacacgta 300
gcactctgct araccccca ttgtacacct ggatcctctc cttaraccta taaaaggaag 360
gaccagggcc ctctragaga aggttggccg cgcggggacg aggacgagac aggcgctcgc 420
ttggggccgc tcgctccctc tcccacgtgg acgcttgtaa ccccctartg caagcgcacc 480
cgacctgggc gcgggacgaa cacgaaggcc gcgggattcc cacctctctr acgccggtct 540
ccggccgcct cgctctcccc ccttcgcgct cgccctcgcg ctcgatccat ctgggctggg 600
gcacgcggcg acactractc gtcggcccaa gggaccccg gtctcgaaac gccgacaatb 660
vhatatarag aaactatabv hataracbvh caattaacta gtgctgataa tatatttara 720
ctgtagattt ttccgataag tagaaaccaa atarttcacc gccacaaaag gcttcatccc 780
atragttacc cbvhtagcaa aaccaaaata rttgtgtgcg agaggccaag agcccaaacc 840
accgccaagg aaaccccag cccaccaggc gccttcctct araaataccc taactccggt 900
ggccgaagtt cctracccat tcactcactc atctcaaggc ctaggtagca ccgtagcagc 960
tartagaagc agctagccag aacaactcgt c 991

<210> 140

<211> 2519

<212> DNA

<213> Zea

<400> 140

cttccgataa aaatatttgg aaccgcatcc tgcatartta tttagaaaca ttgcatcgat 60
aaccgttgca gagcaatara cagggtartc accaactaaa tccatarcat acagtgacag 120
gtgaaattgg aatarttgat cagcttgaat aacagagagc aaagataaat ctagccctgg 180
taataaccbv hgctcacatc ttbvhtaraa gagccaattc taaagacaca gctgcgggbv 240
htagcagtra ggcaaggaaa gaaacaaaat tarcagctaa ctgctaagtg traaaacatt 300
gctttaract aaagggttaa atarttgcca agaaggtagt gggcacctct cctctgcaag 360
cagcagcata atctagggcg aggcttggtg cagcttcctt gtaggggcat bvhaactrat 420
ccbvhaaggc actctractc atctggacag cagtatagta gctccggtcc tgctccaaca 480
atccattttc cttracgcct agtttggatr actgtaattg aattccattc taataatagt 540
aatttagaca tatatraatt aagataattr acttttgtac aaaatatatt tgtatartat 600
tattagcaat atatcctaaa tarttbvhtg ctarattttt actatagagg agtagaacga 660
agagtgtrat ataagttata gatragaaac aaattctaat cbvhcataaa atratttccc 720
atcctccacc ctbvhaattt gagataggct tatarctgaa ctttagaaag tggtggabvh 780
tcaaattcca aactaaataa gttactttat tgagtgaatt ctaattcttt tgatttgaag 840
gaatccaaac gccccgtrag gtaattraca acatagtgtc ggggcbvhag gcggcgtrrc 900
aagctatbvh taagcagagc aggcctgtga gcabvhtact ctggctccaa cccaaccttg 960
gtgatragga acgcggatcc gggcgtcgcc gggggcgcga ccctgtgctt gccaaattga 1020
aaatttagca aagtartttt tttaccttgt tgattbvhtt ggataaccgc bvhtccctaa 1080
tttgabvhat attgctcctt atctctaaac gaccaactgt agcttgttct attgaaaact 1140
ratcagaata atccatagag ataacatrac caacaaggac tgaatratcc agagbvhcat 1200
cabvhgccat tggagctagg gaagtcggcc taactarctt raacccgact tggccaactt 1260
gtaragtaga agacaccbvh gaaatcgatt taacggactc taagggagtg tttgaatara 1320

ctaaaactaa tagttttagt ggctaaaatt agttggagac atccaaacac cctagctaat 1380
atttragcta ttaactattt ttagtaaatr agttacaagt tagctagcta tttattagct 1440
agctaattct artaacaaat ttttagctaa ctaacgcatt caaacactgt ctaaacaggt 1500
aatctaggca cgcgaagtct gactggccag cccaaactra gcctttgtag tagagagagc 1560
cgaccttggc ggcctgracg acatttttga actttttta rcggtttggt cttrattact 1620
ttatattctt tgtraaccaa atartccctt cctcttaaat tataattcgt ttgatttttt 1680
ttbvhtaaag ttttbvhtat agtccaccta gttaacttta aaagttggtt agtctagatt 1740
ttbvhgaggc cgactaraat agcaaatcta acaataaaaa agttagctag atragatttc 1800
aagaacagtc tgaccgattt tgaagatttg attraggtta attctacgat tttgaagatc 1860
cgacctaacc taattttacg ttragactaa agacbvhttt gtttaagatt bvhatbvhtc 1920
tagttarata atttaactra ttttaaacta acacttaatt taaaataagt tagattatbv 1980
hatragaata ttatatttat ataattccaa acaaataact ctaatatcga ttctggttta 2040
rattgtataa tttttbvhtt gaatattarg gcgcgccaac aaattgcgat cgttbvhtaa 2100
tcbvhtatar gaccgactgt artattcata aaatataatt caaaatagga gbvhctcgag 2160
atagcctggc aagggaggag gtarcggagg cggcgtcgcc tgccgcgagc gcaccbvhcg 2220
tcaacgctra ggacctctcg caggcgcgct gttgratcgt gagcaagcac gcgtggcaab 2280
vhgcbvhcat tcctctcgtg caagtgccga gctgagggtc ccgacgcggg gggcaggacg 2340
tggcagagcg ccgcccaggc ggacgccaag tgccgagcta rcagggcacc ttctttartc 2400
cgtccttgct ragtcacacc tcgctctcgc tcactctcgc cgtccgcaca gccgctratc 2460
gtctcccact gcctgccctc tccctgcgcc ggccggtgrc ccgacgcgcc bvhtcgtac 2519

<210> 141
<211> 1264
<212> DNA
<213> Zea
<400> 141

tcatcggtac tcgcgbvhtc gtgcgcgtcg acgactarta tatraaggac tbvhacgtra 60
tcttgacacc agaggtgaca cagaggagga ccaacaacaa taaccactcg ccaccgtcgg 120
tcggtctgcc tttaattctc ttcgcaaaca cataracttg cttttttgtt taagcaagcg 180
tgtbvhbvhg tgcgtacctg bvhactgaca bvhtargagg gaacttctar cggcaggtgt 240
aacacgtgct cttrabvhat aagatcbvhc aaatcgtgtr atatatcgaa gcctgcbvha 300
tartgbvhgt tgaaatarag tagtgaaaca gctaaattaa aataacaaaa tttbvhtbvh 360
gctaggatta raaataaatt araaaatatt tttttataac aatataagac acattttgta 420
rataagttat tatartatta tbvhtttctg ttgcaacgca cgagtartca cctagtatar 480
ccataggctt ttgatcctct argtcgagtt tttgcttgcb vhagagtcga cctcttctct 540
tttarccctt cttctctarc ttagcatttg ctcgatraac tgtaaactaa ttattaracg 600
tarttcaagt catattgaaa ccabvhgcat ttaaaaaagt aaccatctga bvhgaaatat 660
raagctaaat aaagaagtta rgggagtbvh tgctgagaaa acctagacac attragagaa 720
gttarcaagc aagccttaaa taacaatttt tggtgcttta rtgcagatag atartcctat 780
aggttggaga gaagggcggc gbvhctabvh caccbvhcbv htgtgtctgc ccatatartc 840
tatcttarct gaatcctarc aaactcttta atttgactct ttbvhabvhg aattcccatb 900
vhaattctat aacacaacag targacgtct artggagcat actgccaagc acgctagccg 960

acggctcggc gaccgtctga ggctgtccbv hcbvhcaabv htgcacacbv htargcccct 1020
tttagcgctg gcbvhcaccc acgaccacga cacggctccc gtggccgtga atctgacatc 1080
ccgggactcc cgcctccggc cgcgcgtgcc gtgtragcct ccatcgagcg gtgtartccb 1140
vhcaagcctc gacccgggcc acgtarcccc ctcccctccg ctargtgtra ccgctctcgt 1200
racctatatb vhgcggtctt gtccagtctt tccacttaac tartccgtca ctttgtttgc 1260
aaag 1264

<210> 142
<211> 623
<212> DNA
<213> Zea
<400> 142

ctcacacaaa tctaaatagt aaagtagatc tggtracaga gcagaaattt tgaagtgtra 60
tagataggct gagaatbvht agtracgagc aacacattar tacttgaggc catraatact 120
gttttttctt ctccaacctb vhtaactbvh ttartctttt cctccttaab vhtaaggcaa 180
acctcctarc ctttttcttt caaattratt gcattaactg agtggttctt acbvhtagaa 240
bvhaagttgg agcaaagagg cttggtgcgg aaccgaaaat ttgttagtct ataaatratt 300
agagggcgcc tgaagttcgc caabvhttct agtagatttt agtaaccacc cacgctagct 360
gactcgacca ccgtrrgctg gctggcgctg tccbvhcacb vhtaragccc ctggcgctgg 420
cacgcaccca cggcatarct cccbvhactc tgacagcccg ggactccctc cgctcgtgcc 480
gtgtragcct cactcgagcg gtgtartccb vhcaagcctc cacctcggcc acgtarcccc 540
tccccttcct targtgtcac cgctctcgct ccctatatar ggctgcctgg ccaggccttt 600
ccacttract actctctctc tct 623

<210> 143
<211> 1950
<212> DNA
<213> Zea
<400> 143

ggttcaagat bvhtbvhtgb vhaacataat tttartcaaa agtgbvhgtt trattgtart 60
aactagcatr aatarttbvh ccagagtgga gcagtgrgtc tbvhcactar tccgtaraag 120
agacbvhtcc ctracgctac aaggagtbvh ggatcccagt tgactggttg tctgattccg 180
gagtagttgg caaggtgtgc atctctagca taractccaa tttcgccaaa cbvhtgttaa 240
aaacbvhcat ttttaattcc ttbvhaaggt gggtbvhatt ttartragag tbvhcaccta 300
abvhaactra ataatttart tgcbvhttgg atatatraaa ttggcatctg ttragcttgc 360
gaacaagtat bvhaagaggg ttgcatraga acttgbvhcg ttggaaggca ctgagaaaga 420
gcccaataga gagtttttgc ttctccaggg tggcagggtg tgaggttctc tttccgtgtt 480
ratraggtaa aatcctracg ccaatatttt cagaacccag gtarttraca ggtgatbvha 540
atatttcgaa tragtaactt attratctar cttctgcagt ttgctggggg atttgbvhca 600
gaaagcbvha aagcttttga agagctaaga agcaagbvha gtaracataa atctgctcca 660
cagatarctg aaggctgaag cbvhagttga acacaggatt raacacacat ctggtcttar 720
ttgctggata ctgattttta gttgtgtara aaagagaagt ttaaattcca gtgtggtgtc 780
aagcaagttg tatbvhctaa gagaataraa gtcatraggt tbvhttratt tattttttbv 840

haaagtaagg gcaggacagg ttbvhttcbv hcatataaca gttttagctr aagtgtatar 900
cttttrattt taaaggctgt aactgtaaac aggcagctcc agaaabvhaa tracttgggc 960
bvhtttttgt tgctacgcat atartactaa taaabvhtca aaagcgtcca ttcggttagt 1020
ccggtarccb vhgagataat tcgtctgabv haaccttgcc gtctttgtta aaagaaaaaa 1080
aaattarttt gtgcctracc gcattcgata rtttgaagaa attgacccgt tarcagttar 1140
cacccacgca cataragtgc tagaacagca gatctgactt gaagaagcat atatraagac 1200
aaaagtccac ctgaacggag acagtcgagg ttgtggcgga gagctccacc tgaacgccga 1260
agtggaaccg aacagttcca gtggttggcc gtggcgagct ggccacctar tagtctccgg 1320
ggagggggct ggctccagcg cctccgagcg cggtgccagt gtcgccgtcg cgtgggttcc 1380
ggttccggcc gccgccgctt gccggggaat ccaaacggca gacggggtcg ccgtgaccgt 1440
gaatcgtgtt tarttgggcc ttarctaggc cacaacgttt gtgactctgt gagcttartg 1500
tttgggccca tctctttggc gtgtctgaga acgaatttta tratcaagcc cacgaagcca 1560
aaccaggatc ctarccacct agcacatart agtgaagaag gtaaacaaag tgtattttcb 1620
vhttaattca cbvhcgcgtg gtcgacacca caccacacga cttaaaactc ggtgttbvht 1680
tctargacca caagagccbv httaatttta agccgtgtct gcgccbvhat ccgaagagcg 1740
cgtaragtga gagaagaaac gcacggccgc cagcagctgc cgcgggagcg aggcagcacg 1800
tccgttggtc ggcccgggcg ggagcgaggc acacgtcgcg cbvhcacgcg cggcgcccgg 1860
acacgtgccg gcgcgggcac gtccctctcc gcgccttata rtcctccccg ccatttccca 1920
gcagccacaa caagacagag agatarccga 1950

<210> 144

<211> 2112

<212> DNA

<213> Zea

<400> 144

ctaggttggt gagatcctta ggttttggaa ggctttbvht ggcagctctg aagactccaa 60
catctbvhgc ctctagctbv hcctccatbv htattttgta ataagaaaaa tratctccct 120
taaacbvhgg agagggttra tctccatcga acatctttct traggtggtg aagcctaaaa 180
tabvhagcac gacactttga tartaattga aaggattart bvhcccaaga ggtagggtgg 240
attagaccaa tctaaaattt cttgtagcaa taaaatctta rcgaatarcc cacttcacca 300
cttgtgctta agagtgatar atartctarc acaaaaggtt ttacacccta gtttgaaccc 360
tartctagtb vhttarttct artabvhtaa agacaagaat taaattgcta aabvhcaabv 420
hctraagata aagaagggtt agaaacbvha cagcattttt ccaaagtart aaagagccgg 480
gactctccaa tagtcctcgt tggagcaccc acacaaggat artgctcccc attgaacttr 540
acaaggatra agggctctct bvhggtttgt tcctcgccac tctagcbvhg tgagtraccc 600
acagtrgttt araccttgag tggtgatata ractagattt cttaagtgtg tatratraga 660
cttarttagg tcaaactatr attcbvhgcc ccttratagt atagtraaag gaaaaaacaa 720
aggtgctatr actagtarat bvhtctatra actctartaa ctartagaac tagccagtcc 780
ttaacctttt tgctratcct tctaaaatra ataattgaat arcatraata ggggcacaaa 840
aactaratag ccaattarta rtactratag ccaataattt agtgttgtra ttagtracta 900
aaactagbvh ctttratatc tarcbvhctc gtgtttgcct gatccaattc ctaggtcbvh 960
tttgggctcc acttagagct tgtabvhgca tracaacbvh atctgattaa gaaabvhttg 1020

actaaagacc ttctraccca catataaabv hccbvhactt cgctraaact tratccccta 1080
gtctcctart araccсbvht ctbvhcttcg caacttraca agtctarcac bvhtarttgc 1140
aaagggctga ttctcctagc tcccbvhtcg tcgccatctt tcccctttar caccacaaaa 1200
tracaabvht gctracttar tatccccgcg tcttcccttc cctarcctaa acactbvhgg 1260
caacaacgtc gcatracttg gtggtccttg tagatctaga cgaggtctcc tccccgtttt 1320
gtgcttgttg atatagtgat cttgtccttg tcttgtcgct gtcctttgga gtcgtcgcag 1380
agaggcggtg aaaattctag ccgcttctag aaggtgcggt cgccggtcgg bvhagagtcg 1440
agtgagtgca ggcctataaa aagtagcaat rggataacat tttgaaaaaa bvhaaagaaa 1500
taaaaaaact agggtraaat aagattctaa attaaatcta agaaatctat agacattcgg 1560
aaatctattt arcttttgta rtcctttgat tggatttcta gattttgacg tttgtgtaat 1620
tgggcttgac agccccatra ataraggcag gccagctgat ttcgggtgcc gctgatttgg 1680
cccbvhgagg atagagcagg cctttraaca ggcaggcagt ccbvhacgta gtgcctcggc 1740
ctraaagccc bvhcgattaa tarccactgg targtargcg acagccgagg gagtractga 1800
gtragtgccg aacggcctgg aaaccccccg ccgtgtgrcg gtgtrracgc gcgccccggt 1860
grggccgcca cgaccgcgga cacgtgrcgg gcctcgcaca cgcgtccccc acggccccct 1920
cgcatcatta aaaccgcggt gcccgcgcca cggatccccg gcacttratt ccggtttccg 1980
gatagcttar ctcccatcga cggtcggtga gccgttggtg agtggcgaga cagcgggtgc 2040
gctgrgcaag aacaggcgac gaagaagbvh agcacgccgg acacgcggac gctraaggac 2100
gagctgacga gc 2112

<210> 145
<211> 111
<212> DNA
<213> Zea mays
<400> 145

tgcgtacgag acgtgttggc gacggccgac cggcgaccgc cgccgagctc gttttgcctt 60
gttgctttgt ttgttatatg gccctggctc cgatccgggc ttcgtattta a 111

<210> 146
<211> 165
<212> DNA
<213> Zea mays
<400> 146

gtacgatgct ggtaagctca tcagagttcc cgtcgttgct accggcgaga aaccttcttc 60
gtatgacacc ggtggccatg gcgaacaccc cggtgccacc gtagatggcc cactcgtctg 120
acccatccgt tatcactccg ttggtgagca gcgtggagcc cttaa 165

<210> 147
<211> 117
<212> DNA
<213> Zea mays
<400> 147

tcgattgatc tatccatagt ctggccagca tatatccggg caaaattggg aagaacgtgg 60
cgcattatgt ctgaagtata tcctccccac cagctgtgca tcctgatgat gctctta 117

<210> 148
<211> 164
<212> DNA
<213> Zea mays
<400> 148
gacaccttgg aacaccagat ctacagaagt cctgtgatat ctccggtcag ctcctgcgtc 60
gtctccacac gctttcactg cacatggtcg tgctcagcct cagcctagca tttgttttgt 120
ttcccagccc gacaggaaga aacaggtggg ttactcagga ctca 164
<210> 149
<211> 70
<212> DNA
<213> Zea mays
<400> 149
tcgaaccata tatgtaggct catttcatga gataaaatca gatgcatgtg agcatgtcca 60
tatatgttaa 70
<210> 150
<211> 172
<212> DNA
<213> Zea mays
<400> 150
tgcgtacgac gttgctggtg aatgatggtt tggcggcgtg tgtgcaaact tttctatgct 60
agtttagttt gcaggtgtgt atggattttg atttatggcc tggatggatc acactatgta 120
gcgaacgatg tgttcaattt actgttggaa taaaactgca gattggtgtt aa 172
<210> 151
<211> 156
<212> DNA
<213> Zea mays
<400> 151
ggtacgacgg agacggtcag ccgtacgcgt tccggaaccc gggctcgctg tcgtagaggt 60
tgtacggctc cggcgcgccg cccttcttct tcttgccctt cctgttcgcc tccgcagcgc 120
cgccgccgcc agccgcccct ccgacgaccc tgttaa 156
<210> 152
<211> 123
<212> DNA
<213> Zea mays
<400> 152
tcgacgtcgg ttcgttcgtg cctccacctt agcctagggt tggtttcttg caaggtagtg 60
agtgtgtctt agtctcacca tcaccggggc tccaattttg gaaagctgcg tgttaggagt 120
taa 123
<210> 153
<211> 171

<212> DNA
<213> Zea mays
<400> 153
gcgtacgatg catggcacgg ttaggcctgt gcgtgtgccc atgggcaatc aagacaactt 60
ctgagctctg gagttgttgt tgttgttctg tgttcttgct tcctcgttga gctgtgcccg 120
tttgtcctca cttatgcagt cagtggtctg tctgtagtct gtactgctta a 171
<210> 154
<211> 25
<212> DNA
<213> Artificial
<220>
<223> Primer
<400> 154
ctacatttat gttatagagg cgcaa 25
<210> 155
<211> 21
<212> DNA
<213> Artificial
<220>
<223> Primer
<400> 155
catctcttgg gacggaacca a 21
<210> 156
<211> 23
<212> DNA
<213> Artificial
<220>
<223> Primer
<400> 156
catacgattt cctaagcgga atc 23
<210> 157
<211> 20
<212> DNA
<213> Artificial
<220>
<223> Primer
<400> 157
ccgcccgcct caaccacagt 20
<210> 158
<211> 20
<212> DNA

<213> Artificial

<220>

<223> Primer

<400> 158

ccagccatcg tgcttgagtg 20

<210> 159

<211> 20

<212> DNA

<213> Artificial

<220>

<223> Primer

<400> 159

gacgtggtgg cgatcgcaag 20

<210> 160

<211> 21

<212> DNA

<213> Artificial

<220>

<223> Primer

<400> 160

cattgttata catcggtgat g 21

<210> 161

<211> 21

<212> DNA

<213> Artificial

<220>

<223> Primer

<400> 161

cctagctggc ttcttccaag c 21

<210> 162

<211> 23

<212> DNA

<213> Artificial

<220>

<223> Primer

<400> 162

gagcgacctc ggactcagcg gct 23

<210> 163

<211> 22

<212> DNA

<213> Artificial

<220>
<223> Primer
<400> 163
cctacaaaca atattgcatc ag 22
<210> 164
<211> 20
<212> DNA
<213> Artificial
<220>
<223> Primer
<400> 164
gattcagaac atctggtcag 20
<210> 165
<211> 18
<212> DNA
<213> Artificial
<220>
<223> Primer
<400> 165
aggtttagcg aacaaggc 18
<210> 166
<211> 21
<212> DNA
<213> Artificial
<220>
<223> Primer
<400> 166
atcatcaccc taccccgagc t 21
<210> 167
<211> 19
<212> DNA
<213> Artificial
<220>
<223> Primer
<400> 167
gacgagttgt tctggctag 19
<210> 168
<211> 23
<212> DNA
<213> Artificial
<220>

<223> Primer

<400> 168

cttccgataa aaatatttgg aac 23

<210> 169

<211> 19

<212> DNA

<213> Artificial

<220>

<223> Primer

<400> 169

gtacgacatg gcgcgtcgg 19

<210> 170

<211> 20

<212> DNA

<213> Artificial

<220>

<223> Primer

<400> 170

gccagtgcta atgatattta 20

<210> 171

<211> 19

<212> DNA

<213> Artificial

<220>

<223> Primer

<400> 171

atgcacctac tcggcggtg 19

<210> 172

<211> 20

<212> DNA

<213> Artificial

<220>

<223> Primer

<400> 172

tcatcggtac tcgcgatgtc 20

<210> 173

<211> 22

<212> DNA

<213> Artificial

<220>

<223> Primer

<400> 173
ctttgcaaac aaagtgacgg ag 22

<210> 174
<211> 24
<212> DNA
<213> Artificial
<220>
<223> Primer
<400> 174
tataaattag aacggagggg tatg 24

<210> 175
<211> 20
<212> DNA
<213> Artificial
<220>
<223> Primer
<400> 175
ggtgatccga atccgatccc 20

<210> 176
<211> 24
<212> DNA
<213> Artificial
<220>
<223> Primer
<400> 176
ctcacacaaa tctaaatagt aaag 24

<210> 177
<211> 21
<212> DNA
<213> Artificial
<220>
<223> Primer
<400> 177
gagagagaga gtagtgaagt g 21

<210> 178
<211> 22
<212> DNA
<213> Artificial
<220>
<223> Primer
<400> 178

ggttcaagat atgtatgtga tg 22

<210> 179

<211> 23

<212> DNA

<213> Artificial

<220>

<223> Primer

<400> 179

tcgggtatct ctctgtcttg ttg 23

<210> 180

<211> 25

<212> DNA

<213> Artificial

<220>

<223> Primer

<400> 180

ttggtttttt gataatttgt ttatc 25

<210> 181

<211> 21

<212> DNA

<213> Artificial

<220>

<223> Primer

<400> 181

tctccattac ctgcaacgat c 21

<210> 182

<211> 20

<212> DNA

<213> Artificial

<220>

<223> Primer

<400> 182

aagggactcg tggcctacac 20

<210> 183

<211> 20

<212> DNA

<213> Artificial

<220>

<223> Primer

<400> 183

tacgttgtcg cagctggatg 20

<210> 184
<211> 22
<212> DNA
<213> Artificial
<220>
<223> Primer
<400> 184
ttagtagaga ataacacaca tc 22
<210> 185
<211> 18
<212> DNA
<213> Artificial
<220>
<223> Primer
<400> 185
gatcgatcga tcaacgcg 18
<210> 186
<211> 24
<212> DNA
<213> Artificial
<220>
<223> Primer
<400> 186
gtgattaagt tgactggcaa attg 24
<210> 187
<211> 20
<212> DNA
<213> Artificial
<220>
<223> Primer
<400> 187
gcctacttgc ctagcgtacc 20
<210> 188
<211> 21
<212> DNA
<213> Artificial
<220>
<223> Primer
<400> 188
aaaagtagca attgggataa c 21
<210> 189

<211> 21
<212> DNA
<213> Artificial
<220>
<223> Primer
<400> 189
gctcgtcagc tcgtccttga g 21
<210> 190
<211> 21
<212> DNA
<213> Artificial
<220>
<223> Primer
<400> 190
ctaggttggt gagatcctta g 21
<210> 191
<211> 21
<212> DNA
<213> Artificial
<220>
<223> Primer
<400> 191
catcttcttc gacgcctgtt c 21
<210> 192
<211> 23
<212> DNA
<213> Artificial
<220>
<223> Primer
<400> 192
gtggcagctc tgaagactcc aac 23
<210> 193
<211> 23
<212> DNA
<213> Artificial
<220>
<223> Primer
<400> 193
tgaggccgag gcactacgtc atg 23
<210> 194
<211> 23

<212> DNA
<213> Artificial
<220>
<223> Primer
<400> 194
tgacgtttgt gtaattgggc ttg 23
<210> 195
<211> 21
<212> DNA
<213> Artificial
<220>
<223> Primer
<400> 195
gctcgtcagc tcgtccttga g 21
<210> 196
<211> 1696
<212> DNA
<213> Zea mays
<400> 196
aaaagtagca attgggataa cattttgaaa aaaatgaaag aaataaaaaa actagggtta 60
aataagattc taaattaaat ctaagaaatc tatagacatt cggaaatcta tttatctttt 120
gtactccttt gattggattt ctagattttg acgtttgtgt aattgggctt gacagcccca 180
tcaatacagg caggccagct gatttcgggt gccgctgatt cggcccatgg aggatagagc 240
aggcctttca acaggcaggc agtccatgac gtagtgcctc ggcctcaaag cccatgcgat 300
taatatccac tggtacgtac gcgacagccg agggagtcac tgagtcagtg ccgaacggcc 360
tggaaacccc ccgccgtgtg ccggtgtcca cgcgcgcccc ggtgcggccg ccacgaccgc 420
ggacacgtgc cgggcctcgc acacgcgtcc cccacggccc cctcgcatca ttaaaaccgc 480
ggtgcccgcg ccacggatcc ccggcacttc attccggttt ccggatagct tacctcccat 540
cgacggtcgg tgagccgttg gtgagtggcg agacagcggg tgcgctgggc aagaacaggc 600
gacgaagaag atgagcacgc cggacacgcg gacgctcaag gacgagctga cgagcatgga 660
caggaagtgc ctcgtggacc tcggccaccc gctgctcaac cgcgtcgccg acagcttcat 720
ccgcgccgct ggggtcgggg cggccagggc cgtctccagg gaggcctacg tcgtcaccgt 780
cgaaggtacc gcgtcccggc cgagtctacc taactagcga gcggctggtg ctcacccgcc 840
cgttgcaaat ctatctgcag ggctttcagg agactcgtcc gggctagacg ccgacggcgg 900
caagcggagc catttctcca gcatcagagg tgacgacggc cagaggtcgc tcgacgcagt 960
ggtatgtcac aaacatagct cagagctgcg attcgttgtg cagattcatt aatggtttct 1020
ctctcacccc ctcttgacaa tttgcaggta aaaacggccg gcaaggaggc cttccagtgg 1080
ggttagtggt gtgattagtc tgttcagatc taacagtacg ttggaggtcc acaagtagta 1140
taaccctgtt tctctcgacc tcgacatgca gggttggcag ccggagtcta ctcagggctc 1200
acgtacgcgc tgcgggaggc caggggatgc cacgactggg tcagtgccat ttcctagctt 1260
ttaaccagac ggccgttttt ggcttggtct cgcgtgtgaa ctttgctgtc ctcgtgcaga 1320

agaacagcgc catcgcaggc gccatcgctg gggcggcggt ggcgctcacg ggtgacgccg 1380
gcggccactc cgacaagctc gtcaacttcg ccatcaccgg cgccgcgctc tccagcgccg 1440
ggagcttgct ctccggcata ttctgattgg cccccagggc tgcacccgcc tgacacgggt 1500
tcgctgcccg gttgagccct tcatccgagg ggttttagca tgaaaggttg atttcaggcc 1560
agcaatgaaa tatgtattcc tagggccctg ccggtctagc actgtgcgct tccagtgtaa 1620
tcgttgcttc gtgtacgagt ttcagacatt tcgaaatatc ttctccgagt tccggtggtt 1680
tgacatgtgc aaacat 1696

## Description

FIELD OF THE INVENTION

The present invention relates to expression cassettes comprising transcription regulating nucleotide sequences with whole seed and/or embryo-specific expression profiles in plants obtainable from the Zea mays. The transcription regulating nucleotide sequences preferably exhibit strong expression activity especially in whole seeds and, particularly, in the endosperm.

BACKGROUND OF THE INVENTION

Manipulation of plants to alter and/or improve phenotypic characteristics (such as productivity or quality) requires the expression of heterologous genes in plant tissues. Such genetic manipulation relies on the availability of a means to drive and to control gene expression as required. For example, genetic manipulation relies on the availability and use of suitable promoters which are effective in plants and which regulate gene expression so as to give the desired effect(s) in the transgenic plant.

A fertile corn plant contains both male and female reproductive tissues, commonly known as the tassel and the ear, respectively. The tassel tissues form the haploid pollen grains with two nuclei in each grain, which, when shed at anthesis, contact the silks of a female ear. The ear may be on the same plant as that which shed the pollen, or on a different plant. The pollen cell develops a structure known as a pollen tube, which extends down through an individual female silk to the ovule. The two male nuclei travel through this tube to reach the haploid female egg at the base of the silk. One of the male nuclei fuses with and fertilizes the female haploid egg nuclei to form the zygote, which is diploid in chromosome numberand will become the embryo within the kernel. The remaining male nucleus fuses with and fertilizes a second female nucleus to form the primary endosperm nucleus, which is triploid in number and will become the endosperm of the kernel, or seed, of the corn plant. Non-fertilized ovules do not produce kernels and the unfertilized tissues eventually degenerate.

The kernel consists of a number of parts, some derived from maternal tissue and others from the fertilization process. Maternally, the kernel inherits a number of tissues, including a protective, surrounding pericarp and a pedicel. The pedicel is a short stalk-like tissue which attaches the kernel to the cob and provides nutrient transfer from maternal tissue into the kernel. The kernel contains tissues resulting from the fertilization activities, including the new embryo as well as the endosperm. The embryo is comprised of the cells that will develop into the roots and shoots of the next generation corn plant. It is also the tissue in which oils and quality proteins are stored in the kernel. The endosperm functions as a nutritive tissue and provides the energy in the form of stored starch and proteins needed for germination and the initial growth of the embryo.

Considering the complex regulation that occurs during embryo and kernel development in higher plants, and considering that grain is commonly used as a primary source of nutrition for animals and humans, it is important to develop key tools that can be used to improve these tissues from a nutritional standpoint. One class of such tools would be transcriptional promoters that can drive the expression of nutrition enhancing genes specifically in these tissues. Unfortunately, relatively few promoters specifically directing this expression pattern have been identified. Accordingly, there is a need in the art for novel promoter sequences which drive expression during kernel development, and more particularly, embryo development.

The embryo-specific promoters are useful for expressing genes as well as for producing large quantities of protein, for expressing genes involved in the synthesis of oils or proteins of interest, e.g., antibodies, genes for increasing the nutritional value of the whole seed, and, particularly, the embryo and the like. It is advantageous to have the choice of a variety of different promoters so that the most suitable promoter may be selected for a particular gene, construct, cell, tissue, plant or environment. Moreover, the increasing interest in cotransforming plants with multiple plant transcription units (PTU) and the potential problems associated with using common regulatory sequences for these purposes merit having a variety of promoter sequences available.

Only a few embryo or whole seed-specific promoters have been cloned and studied in detail; these include promoters for seed storage protein genes, such as a globulin promoter (Wu et al. (1998) Plant Cell Physiol 39(8) 885-889), phaseolin promoter (US Patent No: 5,504,200) and a napin promoter (US Patent No : 5,608,152). Storage proteins are usually present in large amounts, making it relatively easy to isolate storage protein genes and the gene promoters. Even so, the number of available seed specific promoters is still limited. Furthermore, most of these promoters suffer from several drawbacks; they may drive expression only in a limited period during seed development, and they may be expressed in other tissues as well. For example, storage protein gene promoters are expressed mainly in the mid to late embryo development stage (Chen et al., Dev. Genet., 10 (2): 112-122 (1989); Keddie et al., Plant Mol. Biol., 19 (3): 443-53 (1992); Sjodahl et al., Planta. , 197 (2): 264-71 (1995); Reidt et al., Plant J. , 21 (5): 401-8 (2000)), and also may have activity in other tissues, such as pollen, stamen and/or anthers (as, for example, the phaseolin promoter, as reported by Ahm, V, et al. Plant Phys 109: 1151- 1158 (1995); or the zmHyPRP promoter as described in Gene 356 (2005), 146-152; or promoters described in US patent 5,912,414).

There is, therefore, a great need in the art for the identification of novel sequences that can be used for expression of selected transgenes in economically important plants. Thus, the problem underlying the present invention is to provide new and alternative expression cassettes for embryo-expression of transgenes in plants. The problem is solved by the present invention.

SUMMARY OF THE INVENTION

Accordingly, a first embodiment of the invention relates to an expression cassette for regulating seed-specific expression of a polynucleotide of interest, said expression cassette comprising a transcription regulating nucleotide sequence selected from the group of sequences consisting of:

(a) a nucleic acid sequence of SEQ ID NO: 1, 2, 3, 4, 5, 6, 7, 8, 9, 10, 11, 12, 13, 14, 15, 16, 17, or 18, or a variant thereof;
(b) a nucleic acid sequence which is at least 80% identical to a nucleic acid sequence shown in any one of SEQ ID NO: 1, 2, 3, 4, 5, 6, 7, 8, 9, 10, 11, 12, 13, 14, 15, 16, 17, or 18;
(c) a nucleic acid sequence which hybridizes under stringent conditions to a nucleic acid sequence of SEQ ID NO: 1, 2, 3, 4, 5, 6, 7, 8, 9, 10, 11, 12, 13, 14, 15, 16, 17, or 18, or a variant thereof;
(d) a nucleic acid sequence which hybridizes to a nucleic acid sequence located upstream of an open reading frame sequence of SEQ ID NOs: 19, 20, 21, 22, 23, 24, 25, 26, 27, 28, 29, 30, 31, 32, 33, 34, 35 or 36, or a variant thereof;
(e) a nucleic acid sequence which hybridizes to a nucleic acid sequences located upstream of an open reading frame sequence encoding an amino acid sequence of SEQ ID NOs: 37, 38, 39, 40, 41, 42, 43, 44, 45, 46, 47, 48, 49, 50, 51, 52, 53 or 54, or a variant thereof;
(f) a nucleic acid sequence which hybridizes to a nucleic acid sequence located upstream of an open reading frame sequence being at least 80% identical to an open reading frame sequence of SEQ ID NOs: 19, 20, 21, 22, 23, 24, 25, 26, 27, 28, 29, 30, 31, 32, 33, 34, 35 or 36, wherein the open reading frame encodes a seed protein;
(g) a nucleic acid sequence which hybridizes to a nucleic acid sequences located upstream of an open reading frame encoding an amino acid sequence being at least 80% identical to an amino acid sequence as shown in SEQ ID NOs: 37, 38, 39, 40, 41, 42, 43, 44, 45, 46, 47, 48, 49, 50, 51, 52, 53 or 54, wherein the open reading frame encodes a seed protein;
(h) a nucleic acid sequence obtainable by 5' genome walking or by thermal asymmetric interlaced polymerase chain reaction (TAIL-PCR) on genomic DNA from the first exon of an open reading frame sequence as shown in SEQ ID NOs: 19, 20, 21, 22, 23, 24, 25, 26, 27, 28, 29, 30, 31, 32, 33, 34, 35 or 36; and
(i) a nucleic acid sequence obtainable by 5' genome walking or TAIL PCR on genomic DNA from the first exon of an open reading frame sequence being at least 80% identical to an open reading frame as shown in SEQ ID NOs: 19, 20, 21, 22, 23, 24, 25, 26, 27, 28, 29, 30, 31, 32, 33, 34, 35 or 36, wherein the open reading frame encodes a seed protein; and
(j) a nucleic acid sequence obtainable by 5' genome walking or TAIL PCR on genomic DNA from the first exon of an open reading frame sequence encoding an amino acid sequence being at least 80% identical to an amino acid sequence encoded by an open reading frame as shown in any one of SEQ ID NOs: 37, 38, 39, 40, 41, 42, 43, 44, 45, 46, 47, 48, 49, 50, 51, 52, 53 or 54, wherein the open reading frame encodes a seed protein.

In a preferred embodiment, the expression cassette further comprises at least one polynucleotide of interest being operatively linked to the transcription regulating nucleotide sequence, preferably being heterologous with respect to the transcription regulating nucleotide sequence.

In another aspect, the present invention refers to a transgenic plant tissue, plant organ, plant or seed comprising the expression cassette or the vector of the present inventtion. Preferably, the transgenic plant is a monocotyledone.

In another aspect, the present invention refers method for producing a transgenic plant tissue, plant organ, plant or seed comprising

(a) introducing the expression cassette or the vector of the present invention into a plant cell; and
(b) regenerating said plant cell to form a plant tissue, plant organ, plant or seed.

In another aspect, the present invention refers to a method for producing a transgenic plant tissue, plant organ, plant or seed comprising

(a) integrating the expression cassette or the vector of the present invention into the genome of a plant cell;
(b) regenerating said plant cell to form a plant tissue, plant organ, plant or seed, and

(c) selecting said plant cell to form a plant tissue, plant organ, plant or seed for the presence of the expression cassette or the vector of the present invention.

Other embodiments of the invention relate to vectors comprising an expression cassette of the invention, and transgenic host cells or transgenic plant comprising an expression cassette or a vector of the invention, and methods of producing the same.

DESCRPTION OF THE DRAWINGS

Fig. 1: q-RT-PCR results of the KG candidates showing whole seed or embryo specific or preferable expression pattern [Root_dv: a mixture of roots at 5, 15, 30 days after pollination(DAP); Leaf_dv: a mixture of leaves at 5, 15, 30 DAP; Ear: a mixture of ear at 5 and 10 DAP; whole seeds: a mixture of whole seeds at 15, 20, 30 DAP; Endosperm: a mixture of endosperm at 15, 20, 30 DAP; Embryo: a mixture of embryo at 15, 20, 30 DAP; Root_V2+V4: a mixture of root at V2 and V4 stages; Shoot/leaf_V2 +V4: a mixture of V2 shoot and V4 leaves; Flower_GS: a mixture of flower and geminating seeds.]

Fig. 2 (A) and (B) Diagrams of binary KG vectors

Fig. 3: GUS expression in different tissues at different developmental stages driven by p-KG24 in transgenic maize with RHF155

Fig. 4: GUS expression in different tissues at different developmental stages driven by p-KG37 in transgenic maize with RKF109

Fig. 5: GUS expression in different tissues at different developmental stages driven by p-KG45 in transgenic maize with RKF106

Fig. 6: GUS expression in different tissues at different developmental stages driven by p-KG46 in transgenic maize with RKF107

Fig. 7: GUS expression in different tissues at different developmental stages driven by p-KG49 in transgenic maize with RKF108

Fig. 8: GUS expression in different tissues at different developmental stages driven by p-KG56 in transgenic maize with RKF125

Fig. 9: GUS expression in different tissues at different developmental stages driven by p-KG103 in transgenic maize with RHF128

Fig. 10: GUS expression in different tissues at different developmental stages driven by p-KG119 in transgenic maize with RHF138

Fig. 11: GUS expression in different tissues at different developmental stages driven by p-KG129 in transgenic maize with RTP1047

Fig. 12: q-RT-PCR results of the MA candidates [Root_dv: a mixture of roots at 5, 15, 30 days after pollination(DAP); Leaf_dv: a mixture of leaves at 5, 15, 30 DAP; Ear: a mixture of ear at 5 and 10 DAP; whole seeds: a mixture of whole seeds at 15, 20, 30 DAP; Endosperm: a mixture of endosperm at 15, 20, 30 DAP; Embryo: a mixture of embryo at 15, 20, 30 DAP; Root_V2+V4: a mixture of root at V2 and V4 stages; Shoot/leaf_V2 +V4: a mixture of V2 shoot and V4 leaves; Flower_GS: a mixture of flower and geminating seeds.]

Fig. 13: Vector RCB 1006 for MAWS promoters

Fig. 14: GUS expression in different tissues at different developmental stages driven by p-MAWS23 in transgenic maize with RTP1060

Fig. 15: GUS expression in different tissues at different developmental stages driven by p-MAWS27 in transgenic maize with RTP1059

Fig. 16: GUS expression in different tissues at different developmental stages driven by p-MAWS30 in transgenic maize with RTP1053

Fig. 17: GUS expression in different tissues at different developmental stages driven by p-MAWS57 in transgenic maize with RTP1049

Fig. 18: GUS expression in different tissues at different developmental stages driven by p-MAWS60 in transgenic maize with RTP1056

Fig. 19: GUS expression in different tissues at different developmental stages driven by p-MAWS63 in transgenic maize with RTP1048

Fig. 20: GUS expression in different tissues at different developmental stages driven by p-MAEM1 in transgenic maize with RTP1061

Fig. 21: GUS expression in different tissues at different developmental stages driven by p-MAEM20 in transgenic maize with RTP1064

Fig. 22: qRT-PCR results of the Zm.8705.1.S1_at

Fig. 23: Digital image of the GenomeWalk (GW) run on a 1 % w/v agarose gel and stained with ethidium bromide. The lanes (L) represent as follows: (L1)1kb plus ladders (Promega, Madison, WI, USA), (L2) no DNA(replaced GW library with sterile $ddH_2O$) as negative control; (L3) Human *Pvu*II GW library and primers from Human tissue-type plasminogen activator provided by the kit as a positive control, (L4)B73 *Pvu*II GW library, (L5)B73 EcoRV GW library, (L6)B73 *Dra*I GW library, (7)B73 *Stu*I GW library. L3 using primers from Human tissue-type plasminogen activator (tPA) provided by the kit. L2, and L4 through L7) using ZmNP28-specific primers.

Fig. 24: Final binary vectors RLN 90 (A) and RLN 93 (B); Figure 24 (C) is a diagram of RHF160 and Figure 24 (D) is a diagram of RHF158.

Fig 25: (A) GUS expression in different tissues at different developmental stages driven by pZmNP28_655 in transgenic maize with RLN90; (B) GUS expression in different tissues at different developmental stages driven by pZmNP28_507 in transgenic maize with RLN93; (C) GUS expression in different tissues at different developmental stages driven by pZmNP28_1706 in transgenic maize with RHF158; (D) GUS expression in different tissues at different developmental stages driven by pZmNP28_2070 in transgenic maize with RHF160.

DESCRIPTION OF THE SEQUENCE IDENTIFICATION NUMBERS REFERRING TO THE PROMOTERS

| Name | Promoter | CDS | amino acid | vector | Gene | ESTs | Variant 1 | Variant 2 | Fragments |
|---|---|---|---|---|---|---|---|---|---|
| MAWS60 | 1 | 19 | 37 | 55 | 73 | 91 | 109 | 127 | |
| MAEM1 | 2 | 20 | 38 | 56 | 74 | 92 | 110 | 128 | |
| KG_56 | 3 | 21 | 39 | 57 | 75 | 93 | 111 | 129 | 145 |
| KG_129 | 4 | 22 | 40 | 58 | 76 | 94 | 112 | 130 | 146 |
| MAEM20 | 5 | 23 | 41 | 59 | 77 | 95 | 113 | 131 | |
| MAWS27 | 6 | 24 | 42 | 60 | 78 | 96 | 114 | 132 | |
| MAWS63 | 7 | 25 | 43 | 61 | 79 | 97 | 115 | 133 | |
| KG_49 | 8 | 26 | 44 | 62 | 80 | 98 | 116 | 134 | 147 |

(continued)

| Name | Promoter | CDS | amino acid | vector | Gene | ESTs | Variant 1 | Variant 2 | Fragments |
|---|---|---|---|---|---|---|---|---|---|
| KG_24 | 9 | 27 | 45 | 63 | 81 | 99 | 117 | 135 | 148 |
| KG_37 | 10 | 28 | 46 | 64 | 82 | 100 | 118 | 136 | 149 |
| KG_45 | 11 | 29 | 47 | 65 | 83 | 101 | 119 | 137 | 150 |
| KG_46 | 12 | 30 | 48 | 66 | 84 | 102 | 120 | 138 | 151 |
| KG_103 | 13 | 31 | 49 | 67 | 85 | 103 | 121 | 139 | 152 |
| KG_119 | 14 | 32 | 50 | 68 | 86 | 104 | 122 | 140 | 153 |
| MAWS23 | 15 | 33 | 51 | 69 | 87 | 105 | 123 | 141 | |
| MAWS30 | 16 | 34 | 52 | 70 | 88 | 106 | 124 | 142 | |
| MAWS57 | 17 | 35 | 53 | 71 | 89 | 107 | 125 | 143 | |
| ZmNP28 | 18 | 36 | 54 | 72 | 90 | 108 | 126 | 144 | |

GENERAL DEFINITIONS

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention, which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower).

As used herein, the word "or" means any one member of a particular list and also includes any combination of members of that list.

"Expression cassette" as used herein means a linear or circular nucleic acid molecule. It encompasses DNA as well as RNA sequences which are capable of directing expression of a particular nucleotide sequence in an appropriate host cell. In general, it comprises a promoter operably linked to a polynucleotide of interest, which is - optionally - operably linked to termination signals and/or other regulatory elements. The expression cassette of the present invention is characterized in that it shall comprise a transcription regulating nucleotide sequence as defined hereinafter. An expression cassette may also comprise sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the polynucleotide sequence of interest may be chimeric, meaning that at least one of its components is heterologous with respect to at least one of its other components. The expression cassette may also be one, which is naturally occurring but has been obtained in a recombinant form useful for heterologous expression. An expression cassette may be assembled entirely extracellularly (e.g., by recombinant cloning techniques). However, an expression cassette may also be assembled using in part endogenous components. For example, an expression cassette may be obtained by placing (or inserting) a promoter sequence upstream of an endogenous sequence, which thereby becomes functionally linked and controlled by said promoter sequences. Likewise, a nucleic acid sequence to be expressed may be placed (or inserted) downstream of an endogenous promoter sequence thereby forming an expression cassette. The expression of the nucleotide sequence in the expression cassette may be under the control of a constitutive promoter or of an inducible promoter, which initiates transcription only when the host cell is exposed to some particular external stimulus. In the case of a multicellular organism, the promoter can also be specific to a particular tissue or organ or stage of development (e.g., the embryo preferential or embryo specific promoters of the invention). In a preferred embodiment, such expression cassettes will comprise the transcriptional initiation region of the invention linked to a nucleotide sequence of interest. Such an expression cassette is preferably provided with a plurality of restriction sites for insertion of the gene of interest to be under the transcriptional regulation of the regulatory regions. The expression cassette may additionally contain selectable marker genes. The cassette will include in the 5'-3' direction of transcription, a transcriptional and translational initiation region, a DNA sequence of interest, and a transcriptional and translational termination region functional in plants. The termination region may be native with the transcriptional initiation region, may be native with the DNA sequence of interest, or may be derived from another source. Convenient termination regions are available from the Ti-plasmid of *A. tumefaciens*, such as the octopine synthase and nopaline synthase termination regions and others described below (see also, Guerineau 1991; Proudfoot 1991; Sanfacon 1991; Mogen 1990; Munroe 1990; Ballas 1989; Joshi 1987). The expression cassette can also comprise a multiple cloning site. In such a case, the multiple cloning site is, preferably, arranged in a manner as to allow for operative linkage of a polynucleotide to be introduced in the multiple cloning site with the transcription regulating sequence. In addition to the aforementioned components, the expression cassette of the present invention, preferably, could comprise components required for homologous recombination, i.e. flanking genomic sequences from a target locus. However, also contemplated is an expression cassette which essentially consists of the transcription regulating nucleotide sequence, as defined hereinafter.

"Promoter" refers to a nucleotide sequence, usually upstream (5') to its coding sequence, which controls the expression of the coding sequence by providing the recognition for RNA polymerase and other factors required for proper transcription. "Promoter" includes a minimal promoter that is a short DNA sequence comprised, in some cases, of a TATA box and other sequences that serve to specify the site of transcription initiation, to which regulatory elements are added for enhancement of expression. "Promoter" also refers to a nucleotide sequence that includes a minimal promoter plus regulatory elements and that is capable of controlling the expression of a coding sequence or functional RNA. This type of promoter sequence consists of proximal and more distal upstream elements, the latter elements often referred to as enhancers. Accordingly, an "enhancer" is a DNA sequence, which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements, derived from different promoters found in nature, or even be comprised of synthetic DNA segments. A promoter may also contain DNA sequences that are involved in the binding of protein factors, which control the effectiveness of transcription initiation in response to physiological or developmental conditions. The "initiation site" is the position surrounding the first nucleotide that is part of the transcribed sequence, which is also defined as position +1. With respect to this site all other sequences of the gene and its controlling regions are numbered. Downstream sequences (i.e., further protein encoding sequences in the 3' direction) are denominated positive, while upstream sequences (mostly of the controlling regions in the 5' direction) are denominated negative. Promoter elements, such as a TATA element, that are inactive or have greatly reduced promoter activity in the absence of upstream activation are referred as "minimal" or "core" promoters. In the presence of a suitable transcription factor, the minimal promoter functions to permit transcription. A "minimal" or "core' promoter thus consists only of all basal elements needed for transcription initiation, e.g., a TATA box and/or an initiator.

"Constitutive promoter" refers to a promoter that is able to express the open reading frame (ORF) in all or nearly all of the plant tissues during all or nearly all developmental stages of the plant. Each of the transcription-activating elements do not exhibit an absolute tissue-specificity, but mediate transcriptional activation in most plant tissues at a level of at least 1 % reached in the plant tissue in which transcription is most active. "Constitutive expression" refers to expression using a constitutive promoter.

"Regulated promoter" refers to promoters that direct gene expression not constitutively, but in a temporally- and/or spatially-regulated manner, and includes both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered, numerous examples may be found in the compilation by Okamuro et al. (1989). Typical regulated promoters useful in plants include but are not limited to safener-inducible promoters, promoters derived from the tetracycline-inducible system, promoters derived from salicylate-inducible systems, promoters derived from alcohol-inducible systems, promoters derived from glucocorticoid-inducible system, promoters derived from pathogen-inducible systems, and promoters derived from ecdysone-inducible systems. "Conditional" and "regulated expression" refer to expression controlled by a regulated promoter.

"Inducible promoter" refers to those regulated promoters that can be turned on in one or more cell types by an external stimulus, such as a chemical, light, hormone, stress, or a pathogen.

As used herein, "transcription regulating nucleotide sequence", refers to nucleotide sequences influencing the transcription, RNA processing or stability, or translation of the associated (or functionally linked) nucleotide sequence to be transcribed. The transcription regulating nucleotide sequence may have various localizations with the respect to the nucleotide sequences to be transcribed. The transcription regulating nucleotide sequence may be located upstream (5' non-coding sequences), within, or downstream (3' non-coding sequences) of the sequence to be transcribed (e.g., a coding sequence). The transcription regulating nucleotide sequences may be selected from the group comprising enhancers, promoters, translation leader sequences, introns, 5'-untranslated sequences, 3'-untranslated sequences, and polyadenylation signal sequences. They include natural and synthetic sequences as well as sequences, which may be a combination of synthetic and natural sequences. As is noted above, the term "transcription regulating nucleotide sequence" is not limited to promoters. However, preferably a transcription regulating nucleotide sequence of the invention comprises at least one promoter sequence (*e.g.*, a sequence localized upstream of the transcription start of a gene capable to induce transcription of the downstream sequences). In one preferred embodiment the transcription regulating nucleotide sequence of the invention comprises the promoter sequence of the corresponding gene and - optionally and preferably - the native 5'-untranslated region of said gene. Furthermore, the 3'-untranslated region and/or the polyadenylation region of said gene may also be employed.

As used herein, the term "cis-regulatory element" or "promoter motif" refers to a cis-acting transcriptional regulatory element that confers an aspect of the overall control of gene expression. A cis-element may function to bind transcription factors, trans-acting protein factors that regulate transcription. Some cis-elements bind more than one transcription factor, and transcription factors may interact in different affinities with more than one cis-element. The promoters of the present invention desirably contain cis-elements that can confer or modulate gene expression. Cis-elements can be identified by a number of techniques, including deletion analysis, i.e., deleting one or more nucleotides from the 5' end or internal of a promoter; DNA binding protein analysis using DNase I footprinting, methylation interference, electrophoresis mobility-shift assays, in vivo genomic footprinting by ligation-mediated PCR, and other conventional assays; or by DNA sequence similarity analysis with known cis-element motifs by conventional DNA sequence comparison methods. The fine structure of a cis-element can be further studied by mutagenesis (or substitution) of one or more nucleotides or by other conventional methods. Cis-elements can be obtained by chemical synthesis or by isolation from promoters that include such elements, and they can be synthesized with additional flanking nucleotides that contain useful restriction enzyme sites to facilitate subsequence manipulation.

The "expression pattern" of a promoter (with or without enhancer) is the pattern of expression levels, which shows where in the plant and in what developmental stage transcription is initiated by said promoter. Expression patterns of a set of promoters are said to be complementary when the expression pattern of one promoter shows little overlap with the expression pattern of the other promoter. The level of expression of a promoter can be determined by measuring the 'steady state' concentration of a standard transcribed reporter mRNA. This measurement is indirect since the concentration of the reporter mRNA is dependent not only on its synthesis rate, but also on the rate with which the mRNA is degraded. Therefore, the steady state level is the product of synthesis rates and degradation rates. The rate of degradation can however be considered to proceed at a fixed rate when the transcribed sequences are identical, and thus this value can serve as a measure of synthesis rates. When promoters are compared in this way, techniques available to those skilled in the art are hybridization S1-RNAse analysis, northern blots and competitive RT-PCR. This list of techniques in no way represents all available techniques, but rather describes commonly used procedures used to analyze transcription activity and expression levels of mRNA. The analysis of transcription start points in practically all promoters has revealed that there is usually no single base at which transcription starts, but rather a more or less clustered set of initiation sites, each of which accounts for some start points of the mRNA. Since this distribution varies from promoter to promoter the sequences of the reporter mRNA in each of the populations would differ from each other. Since each mRNA species is more or less prone to degradation, no single degradation rate can be expected for different reporter mRNAs. It has been shown for various eukaryotic promoter sequences that the sequence surrounding the initiation site ('initiator') plays an important role in determining the level of RNA expression directed by that specific promoter. This includes also part of the transcribed sequences. The direct fusion of promoter to reporter sequences would therefore lead to suboptimal levels of transcription. A commonly used procedure to analyze expression patterns and levels is through determination of the 'steady state' level of protein accumulation in a cell. Commonly used candidates for the reporter gene, known to those skilled in the art are beta-glucuronidase (GUS), chloramphenicol acetyl transferase (CAT) and proteins with fluorescent properties, such as green fluorescent protein (GFP) from Aequora victoria. In principle, however, many more proteins are suitable for this purpose, provided the protein does not interfere with essential plant functions. For quantification and determination of localization a number of tools are suited. Detection systems can readily be created or are available which are based on, e.g., immunochemical, enzymatic, fluorescent detection and quantification. Protein levels can be determined in plant tissue extracts or in intact tissue using in situ analysis of protein expression. Generally, individual transformed lines with one chimeric promoter reporter construct may vary in their levels of expression of the reporter gene. Also frequently observed is the phenomenon that such transformants do not express any detectable product (RNA or protein). The variability in expression is commonly ascribed to 'position effects', although the molecular mechanisms underlying this inactivity are usually not clear.

"Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of senescence. For the purposes of the present invention, "tissue-specific" preferably refers to "seed-specific" or "seed-preferential" or embryo-specific or embryo-preferential.

"Seed" as used herein refers, preferably, to whole seed, endosperm and embryonic tissues, more preferably to embryonic tissue. "Specific" in the sense of the invention means that the polynucleotide of interest being operatively linked to the transcription regulating nucleotide sequence referred to herein will be predominantly expressed in the indicated tissues or cells when present in a plant. A predominant expression as meant herein is characterized by a statistically significantly higher amount of detectable transcription in the said tissue or cells with respect to other plant tissues. A statistically significant higher amount of transcription is, preferably, an amount being at least two-fold, three-fold, four-fold, five-fold, ten-fold, hundred-fold, five hundred-fold or thousand-fold the amount found in at least one of the other tissues with detectable transcription. Alternatively, it is an expression in the indicated tissue or cell whereby the amount of transcription in other tissues or cells is less than 1%, 2%, 3%, 4% or, most preferably, 5% of the overall (whole plant) amount of expression. The amount of transcription directly correlates to the amount of transcripts (i.e. RNA) or polypeptides encoded by the transcripts present in a cell or tissue. Suitable techniques for measuring transcription either based on RNA or polypeptides are well known in the art. Tissue or cell specificity alternatively and, preferably in addition to the above, means that the expression is restricted or almost restricted to the indicated tissue or cells, i.e. there is essentially no detectable transcription in other tissues. Almost restricted as meant herein means that unspecific expression is detectable in less than ten, less than five, less than four, less than three, less than two or one other tissue(s). "Seed-preferential" or "embryo-preferential" in the context of this invention means the transcription of a nucleic acid sequence by a transcription regulating element in a way that transcription of said nucleic acid sequence in seeds contribute to more than 50%, preferably more than 70%, more preferably more than 80% of the entire quantity of the RNA transcribed from said nucleic acid sequence in the entire plant during any of its developmental stage.

"Expression" refers to the transcription and/or translation of an endogenous gene, ORF or portion thereof, or a transgene in plants. For example, in the case of antisense constructs, expression may refer to the transcription of the antisense DNA only. In addition, expression refers to the transcription and stable accumulation of sense (mRNA) or functional RNA. Expression may also refer to the production of protein.

Seed specific expression can be determined by comparing the expression of a nucleic acid of interest, e.g., a reporter gene such as GUS, operatively linked to the expression control sequence in the following tissues and stages: 1) roots and leafs at 5-leaf stage, 2) stem at V-7 stage, 3) Leaves, husk, and silk at flowering stage at the first emergence of silk, 4) Spikelets/Tassel at pollination, 5) Ear or Kernels at 5, 10, 15, 20, and 25 days after pollination. Preferably, expression of the nucleic acid of interest can be determined only in Ear or Kernels at 5, 10, 15, 20, and 25 days after pollination in said assay as shown in the accompanying Figures. The expression of the polynucleotide of interest can be determined by various well known techniques, e.g., by Northern Blot or in situ hybridization techniques as described in WO 02/102970, and, preferably, by GUS histochemical analysis as described in the accompanying Examples. Transgenic plants for analyzing seed specific expression can be also generated by techniques well known to the person skilled in the art and as discussed elsewhere in this specification.

The term "nucleic acid" refers to deoxyribonucleotides or ribonucleotides and their polymers thereof in either single- or double-stranded form, composed of monomers (nucleotides) containing a sugar, phosphate and a base, which is either a purine or pyrimidine. Unless specifically limited, the term encompasses nucleic acids containing known analogs of natural nucleotides, which have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer 1991; Ohtsuka 1985; Rossolini 1994). A "nucleic acid fragment" is a fraction of a given nucleic acid molecule. In higher plants, deoxyribonucleic acid (DNA) is the genetic material while ribonucleic acid (RNA) is involved in the transfer of information contained within DNA into proteins. The term "nucleotide sequence" refers to a polymer of DNA or RNA which can be single- or double-stranded, optionally containing synthetic, non-natural or altered nucleotide bases capable of incorporation into DNA or RNA polymers. The terms "nucleic acid" or "nucleic acid sequence" may also be used interchangeably with gene, cDNA, DNA and RNA encoded by a gene.

The invention encompasses isolated or substantially purified nucleic acid or protein compositions. In the context of the present invention, an "isolated" or "purified" DNA molecule or an "isolated" or "purified" polypeptide is a DNA molecule or polypeptide that, by the hand of man, exists apart from its native environment and is therefore not a product of nature. An isolated DNA molecule or polypeptide may exist in a purified form or may exist in a non-native environment such as, for example, a transgenic host cell. For example, an "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein or polypeptide having less than about 30%, 20%, 10%, 5%, (by dry weight) of contaminating protein. When the protein of the invention, or biologically active portion thereof, is recombinantly produced, preferably culture medium represents less than about 30%, 20%, 10%, or 5% (by dry weight) of chemical precursors or non-protein of interest chemicals. The nucleotide sequences of the invention include both the naturally occurring sequences as well as mutant (variant) forms. Such variants will continue to possess the desired activity, i.e., either promoter activity or the activity of the product encoded by the open reading frame of the non-variant nucleotide sequence.

The term "variant" with respect to a sequence (e.g., a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence, i.e. for example to SEQ ID NO's:1 to 18 or 19 to 36.

The nucleic acid molecules of the invention can be "optimized" for enhanced expression in plants of interest (see, for example, WO 91/16432; Perlak 1991; Murray 1989). In this manner, the open reading frames in genes or gene fragments can be synthesized utilizing plant-preferred codons (see, for example, Campbell & Gowri, 1990 for a discussion of host-preferred codon usage). Thus, the nucleotide sequences can be optimized for expression in any plant. It is recognized that all or any part of the gene sequence may be optimized or synthetic. That is, synthetic or partially optimized sequences may also be used. Variant nucleotide sequences and proteins also encompass sequences and protein derived from a mutagenic and recombinogenic procedure such as DNA shuffling. With such a procedure, one or more different coding sequences can be manipulated to create a new polypeptide possessing the desired properties. In this manner, libraries of recombinant polynucleotides are generated from a population of related sequence polynucleotides comprising sequence regions that have substantial sequence identity and can be homologously recombined in vitro or in vivo. Strategies for such DNA shuffling are known in the art (see, for example, Stemmer 1994; Stemmer 1994; Crameri 1997; Moore 1997; Zhang 1997; Crameri 1998; and US 5,605,794, 6, 8, 10, and 12,837,458).

The following terms are used to describe the sequence relationships between two or more nucleic acids or polynucleotides: (a) "reference sequence", (b) "comparison window", (c) "sequence identity", (d) "percentage of sequence identity", and (e) "substantial identity".

(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.

(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.
Methods of alignment of sequences for comparison are well known in the art. Thus, the determination of percent identity between any two sequences can be accomplished using a mathematical algorithm. Preferred, non-limiting examples of such mathematical algorithms are the algorithm of Myers and Miller, 1988; the local homology algorithm of Smith et al. 1981; the homology alignment algorithm of Needleman and Wunsch 1970; the search-for-similarity-method of Pearson and Lipman 1988; the algorithm of Karlin and Altschul, 1990, modified as in Karlin and Altschul, 1993.
Computer implementations of these mathematical algorithms can be utilized for comparison of sequences to determine sequence identity. Such implementations include, but are not limited to: CLUSTAL in the PC/Gene program (available from Intelligenetics, Mountain View, Calif.); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described (Higgins 1988, 1989; Corpet 1988; Huang 1992; Pearson 1994). The ALIGN program is based on the algorithm of Myers and Miller, supra. The BLAST programs of Altschul et al., 1990, are based on the algorithm of Karlin and Altschul, supra. Multiple aligments (i.e. of more than 2 sequences) are preferably performed using the Clustal W algorithm (Thompson 1994; e.g., in the software VectorNTI™ , version 9; Invitrogen Inc.) with the scoring matrix BLOSUM62MT2 with the default settings (gap opening penalty 15/19, gap extension penalty 6.66/0.05; gap separation penalty range 8; % identity for alignment delay 40; using residue specific gaps and hydrophilic residue gaps).

Software for performing BLAST analyses is publicly available through the National Center for Biotechnology Information (http://www.ncbi.nim.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences (see, e.g., Karlin & Altschul (1993). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

To obtain gapped alignments for comparison purposes, Gapped BLAST (in BLAST 2.0) can be utilized as described in Altschul et al. 1997. Alternatively, PSI-BLAST (in BLAST 2.0) can be used to perform an iterated search that detects distant relationships between molecules. See Altschul et al., supra. When utilizing BLAST, Gapped BLAST, PSI-BLAST, the default parameters of the respective programs (e.g. BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, 1989). See http://www.ncbi.nlm.nih.gov. Alignment may also be performed manually by inspection.

For purposes of the present invention, comparison of nucleotide sequences for determination of percent sequence identity to specific nucleotide sequences (e.g., the promoter sequences disclosed herein) is preferably made using the BlastN program (version 1.4.7 or later) with its default parameters (wordlength (W) of 11, an expectation (E) of 10, a cutoff of 100, M=5, N=-4, and a comparison of both strands) or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.

For purposes of the present invention, comparison of polypeptide or amino acid sequences for determination of percent sequence identity / homology to specific polypeptide or amino acid sequences is preferably made using the BlastP program (version 1.4.7 or later) with its default parameters (wordlength (W) of 3, an expectation (E) of 10, and the BLOSUM62 scoring matrix (Henikoff & Henikoff, 1989); see http://www.ncbi.nlm.nih.gov) or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.

(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, Calif.).

(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

(e)

(i) The term "substantial identity" of polynucleotide sequences means that a polynucleotide comprises a sequence that has at least 38%, e.g., 39%, 40%, 42%, 44%, 46%, 48%, 50%, 52%, 54%, 56%, 58%, 60%, 62%, 64%, 65%, 66%, 67%, 68%, 69%, 70%, 71%, 72%, 73%, 74%, 75%, 76%, 77%, 78%, or 79%, preferably at least 80%, 81%, 82%, 83%, 84%, 85%, 86%, 87%, 88%, or 89%, more preferably at least 90%, 91%, 92%, 93%, or 94%, and most preferably at least 95%, 96%, 97%, 98%, or 99% sequence identity, compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 38%, 50% or 60%, preferably at least 70% or 80%, more preferably at least 90%, 95%, and most preferably at least 98%.
Another indication that nucleotide sequences are substantially identical is if two molecules hybridize to each other under stringent conditions (see below). Generally, stringent conditions are selected to be about 5°C lower than the thermal melting point ($T_m$) for the specific sequence at a defined ionic strength and pH. However, stringent conditions encompass temperatures in the range of about 1°C to about 20°C, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.

(ii) The term "substantial identity" in the context of a peptide indicates that a peptide comprises a sequence with at least 38%, e.g. 39%, 40%, 42%, 44%, 46%, 48%, 50%, 52%, 54%, 56%, 58%, 60%, 62%, 64%, 65%, 66%, 67%, 68%, 69%, 70%, 71%, 72%, 73%, 74%, 75%, 76%, 77%, 78%, or 79%, preferably 80%, 81%, 82%, 83%, 84%, 85%, 86%, 87%, 88%, or 89%, more preferably at least 90%, 91%, 92%, 93%, or 94%, or even more preferably, 95%, 96%, 97%, 98% or 99%, sequence identity to the reference sequence over a specified comparison window. Preferably, optimal alignment is conducted using the homology alignment algorithm of Needleman and Wunsch (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridization are sequence dependent, and are different under different environmental parameters. The $T_m$ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the $T_m$ can be approximated from the equation of Meinkoth and Wahl, 1984:

$$T_m = 81.5°C + 16.6 (\log_{10} M) + 0.41 (\%GC) - 0.61 (\% \text{form}) - 500 / L$$

where M is the molarity of monovalent cations, %GC is the percentage of guanosine and cytosine nucleotides in the DNA, % form is the percentage of formamide in the hybridization solution, and L is the length of the hybrid in base pairs. $T_m$ is reduced by about 1°C for each 1 % of mismatching; thus, $T_m$, hybridization, and/or wash conditions can be adjusted to hybridize to sequences of the desired identity. For example, if sequences with >90% identity are sought, the $T_m$ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. An extensive guide to the hybridization of nucleic acids is found in Tijssen, 1993. Generally, highly stringent hybridization and wash conditions are selected to be about 5°C lower than the thermal melting point $T_m$ for the specific sequence at a defined ionic strength and pH.

An example of highly stringent wash conditions is 0.15 M NaCl at 72°C for about 15 minutes. An example of stringent wash conditions is a 0.2 X SSC wash at 65°C for 15 minutes (see, Sambrook, infra, for a description of SSC buffer). Often, a high stringency wash is preceded by a low stringency wash to remove background probe signal. An example medium stringency wash for a duplex of, e.g., more than 100 nucleotides, is 1 X SSC at 45°C for 15 minutes. An example low stringency wash for a duplex of, e.g., more than 100 nucleotides, is 4 to 6 X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (e.g., >50 nucleotides). Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

Very stringent conditions are selected to be equal to the $T_m$ for a particular probe. An example of highly stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0.1 x SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1X to 2X SSC (20 X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5 X to 1 X SSC at 55 to 60°C.

The following are examples of sets of hybridization/wash conditions that may be used to clone nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M $NaPO_4$, 1 mM EDTA at 50°C with washing in 2 X SSC, 0. 1 % SDS at 50°C (very low stringency conditions), more desirably in 7% sodium dodecyl sulfate (SDS), 0.5 M $NaPO_4$, 1 mM EDTA at 50°C with washing in 1 X SSC, 0.1% SDS at 50°C (low stringency conditions), more desirably still in 7% sodium dodecyl sulfate (SDS), 0.5 M $NaPO_4$, 1 mM EDTA at 50°C with washing in 0.5 X SSC, 0.1% SDS at 50°C (moderate stringency conditions), preferably in 7% sodium dodecyl sulfate (SDS), 0.5 M $NaPO_4$, 1 mM EDTA at 50°C with washing in 0.1 X SSC, 0.1% SDS at 50°C (high stringency conditions), more preferably in 7% sodium dodecyl sulfate (SDS), 0.5 M $NaPO_4$, 1 mM EDTA at 50°C with washing in 0.1 X SSC, 0.1 % SDS at 65°C (very high stringency conditions).

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

"Encoding" or "Coding sequence" refers to a DNA or RNA sequence that codes for a specific amino acid sequence and excludes the non-coding sequences. It may constitute an "uninterrupted coding sequence", i.e., lacking an intron, such as in a cDNA or it may include one or more introns bounded by appropriate splice junctions. An "intron" is a sequence of RNA which is contained in the primary transcript but which is removed through cleavage and re-ligation of the RNA within the cell to create the mature mRNA that can be translated into a protein.

"Operably-linked" or "functionally linked" refers preferably to the association of nucleic acid sequences on single nucleic acid fragment so that the function of one is affected by the other. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (i.e., that the coding sequence or functional RNA is under the transcriptional control of the promoter). Coding sequences can be operably-linked to regulatory sequences in sense or antisense orientation.

The terms "heterologous DNA sequence", "exogenous DNA segment" or "heterologous nucleic acid," as used herein, each refer to a sequence that originates from a source foreign to the particular host cell or, if from the same source, is modified from its original form. Thus, a heterologous gene in a host cell includes a gene that is endogenous to the particular host cell but has been modified through, for example, the use of DNA shuffling. The terms also include non-naturally occurring multiple copies of a naturally occurring DNA sequence. Thus, the terms refer to a DNA segment that is foreign or heterologous to the cell, or homologous to the cell but in a position within the host cell nucleic acid in which the element is not ordinarily found. Exogenous DNA segments are expressed to yield exogenous polypeptides. A "homologous" DNA sequence is a DNA sequence that is naturally associated with a host cell into which it is introduced.

"Homologous to" in the context of nucleotide sequence identity refers to the similarity between the nucleotide sequences of two nucleic acid molecules or between the amino acid sequences of two protein molecules. Estimates of such homology are provided by either DNA-DNA or DNA-RNA hybridization under conditions of stringency as is well understood by those skilled in the art (as described in Haines and Higgins (eds.), Nucleic Acid Hybridization, IRL Press, Oxford, U.K.), or by the comparison of sequence similarity between two nucleic acids or proteins.

"Vector" is defined to include, inter alia, any plasmid, cosmid, phage or Agrobacterium binary nucleic acid molecule in double or single stranded linear or circular form which may or may not be self transmissible or mobilizable, and which can transform prokaryotic or eukaryotic host either by integration into the cellular genome or exist extrachromosomally (e.g. autonomous replicating plasmid with an origin of replication).

Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells).

Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance, kanamycin resistance, streptomycin resistance or ampicillin resistance.

A "transgene" or "trangenic" refers to a gene that has been introduced into the genome by transformation and is stably or transiently maintained. Transgenes may include, for example, genes that are either heterologous or homologous to the genes of a particular plant to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

The term "transformation" refers to the transfer of a nucleic acid fragment into the genome of a host cell. Host cells containing the transformed nucleic acid fragments are referred to as "transgenic" cells, and organisms comprising transgenic cells are referred to as "transgenic organisms". Examples of methods of transformation of plants and plant cells include Agrobacterium-mediated transformation (De Blaere 1987) and particle bombardment technology (US 4,945,050). Whole plants may be regenerated from transgenic cells by methods well known to the skilled artisan (see, for example, Fromm 1990).

"Transformed," "transgenic and "recombinant" refer to a host organism such as a bacterium or a plant into which a heterologous nucleic acid molecule has been introduced. The nucleic acid molecule can be stably integrated into the genome generally known in the art and are disclosed (Sambrook 1989; Innis 1995; Gelfand 1995; Innis & Gelfand 1999). For example, "transformed," "transformant," and "transgenic" plants or calli have been through the transformation process and contain a foreign gene integrated into their chromosome. The term "untransformed" refers to normal plants that have not been through the transformation process.

"Transiently transformed" refers to cells in which transgenes and foreign DNA have been introduced (for example, by such methods as Agrobacterium-mediated transformation or biolistic bombardment), but not selected for stable maintenance.

"Stably transformed" refers to cells that have been selected and regenerated on a selection media following transformation.

"Chromosomally-integrated" refers to the integration of a foreign gene or DNA construct into the host genome by covalent bonds. Where genes are not "chromosomally integrated", they may be "transiently expressed". Transient expression of a gene refers to the expression of a gene that is not integrated into the host chromosome but functions independently, either as part of an autonomously replicating plasmid or expression cassette, for example, or as part of another biological system such as a virus. "Genetically stable" and "heritable" refer to chromosomally-integrated genetic elements that are stably maintained in the plant and stably inherited by progeny through successive generations.

A "transgenic plant" is a plant having one or more plant cells that contain an expression vector as defined hereinafter in the detailed description.

"Primary transformant" and "T0 generation" refer to transgenic plants that are of the same genetic generation as the tissue which was initially transformed (i.e., not having gone through meiosis and fertilization since transformation).

"Secondary transformants" and the "T1, T2, T3, etc. generations" refer to transgenic plants derived from primary transformants through one or more meiotic and fertilization cycles. They may be derived by self-fertilization of primary or secondary transformants or crosses of primary or secondary transformants with other transformed or untransformed plants.

"Plant tissue" includes differentiated and undifferentiated tissues or plants, including but not limited to roots, stems, shoots, leaves, pollen, seeds, tumor tissue and various forms of cells and culture such as single cells, protoplast, embryos, and callus tissue. The plant tissue may be in plants or in organ, tissue or cell culture.

The term "altered plant trait" means any phenotypic or genotypic change in a transgenic plant relative to the wild-type or non-transgenic plant host.

The word "plant" refers to any plant, particularly to agronomically useful plants (e.g., seed plants), and "plant cell" is a structural and physiological unit of the plant, which comprises a cell wall but may also refer to a protoplast. The plant cell may be in form of an isolated single cell or a cultured cell, or as a part of higher organized unit such as, for example, a plant tissue, or a plant organ differentiated into a structure that is present at any stage of a plant's development. Such structures include one or more plant organs including, but are not limited to, fruit, shoot, stem, leaf, flower petal, etc. Preferably, the term "plant" includes whole plants, shoot vegetative organs/structures (*e.g.* leaves, stems and tubers), roots, flowers and floral organs/structures (*e.g.* bracts, sepals, petals, stamens, carpels, anthers and ovules), seeds (including embryo, endosperm, and seed coat) and fruits (the mature ovary), plant tissues (*e.g.* vascular tissue, ground tissue, and the like) and cells (*e.g.* guard cells, egg cells, trichomes and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous. Included within the scope of the invention are all genera and species of higher and lower plants of the plant kingdom. Included are furthermore the mature plants, seed, shoots and seedlings, and parts, propagation material (for example seeds and fruit) and cultures, for example cell cultures, derived therefrom.

## DETAILED DESCRIPTION OF THE INVENTION

The present invention thus provides isolated nucleic acid molecules comprising a plant nucleotide sequence that directs seed-preferential or seed-specific transcription of an operably linked nucleic acid fragment in a plant cell.

Specifically, the present invention provides an expression cassette for regulating seed-specific expression of a polynucleotide of interest, said expression cassette comprising a transcription regulating nucleotide sequence selected from the group of sequences consisting of:

(a) a nucleic acid sequence of SEQ ID NO: 1, 2, 3, 4, 5, 6, 7, 8, 9, 10, 11, 12, 13, 14, 15, 16, 17, or 18, or a variant thereof.

(b) a nucleic acid sequence which is at least 80% identical to a nucleic acid sequence shown in any one of SEQ ID NO: 1, 2, 3, 4, 5, 6, 7, 8, 9, 10, 11, 12, 13, 14, 15, 16, 17, or 18;
(c) a nucleic acid sequence which hybridizes under stringent conditions to a nucleic acid sequence of SEQ ID NO: 1, 2, 3, 4, 5, 6, 7, 8, 9, 10, 11, 12, 13, 14, 15, 16, 17, or 18;
(d) a nucleic acid sequence which hybridizes to a nucleic acid sequence located upstream of an open reading frame sequence of SEQ ID NOs: 19, 20, 21, 22, 23, 24, 25, 26, 27, 28, 29, 30, 31, 32, 33, 34, 35 or 36;
(e) a nucleic acid sequence which hybridizes to a nucleic acid sequence located upstream of an open reading frame sequence encoding an amino acid sequence of SEQ ID NOs: 37, 38, 39, 40, 41, 42, 43, 44, 45, 46, 47, 48, 49, 50, 51, 52, 53 or 54;
(f) a nucleic acid sequence which hybridizes to a nucleic acid sequence located upstream of an open reading frame sequence being at least 80% identical to an open reading frame sequence of SEQ ID NOs: 19, 20, 21, 22, 23, 24, 25, 26, 27, 28, 29, 30, 31, 32, 33, 34, 35 or 36, wherein the open reading frame encodes a seed protein;
(g) a nucleic acid sequence which hybridizes to a nucleic acid sequences located upstream of an open reading frame encoding an amino acid sequence being at least 80% identical to an amino acid sequence as shown in SEQ ID NOs: 37, 38, 39, 40, 41, 42, 43, 44, 45, 46, 47, 48, 49, 50, 51, 52, 53 or 54, wherein the open reading frame encodes a seed protein;
(h) a nucleic acid sequence obtainable by 5' genome walking or by thermal asymmetric interlaced polymerase chain reaction (TAIL-PCR) on genomic DNA from the first exon of an open reading frame sequence as shown in SEQ ID NOs: 19, 20, 21, 22, 23, 24, 25, 26, 27, 28, 29, 30, 31, 32, 33, 34, 35 or 36; and
(i) a nucleic acid sequence obtainable by 5' genome walking or TAIL PCR on genomic DNA from the first exon of an open reading frame sequence being at least 80% identical to an open reading frame as shown in SEQ ID NOs: 19, 20, 21, 22, 23, 24, 25, 26, 27, 28, 29, 30, 31, 32, 33, 34, 35 or 36, wherein the open reading frame encodes a seed protein; and
(j) a nucleic acid sequence obtainable by 5' genome walking or TAIL PCR on genomic DNA from the first exon of an open reading frame sequence encoding an amino acid sequence being at least 80% identical to an amino acid sequence encoded by an open reading frame as shown in SEQ ID NOs: 37, 38, 39, 40, 41, 42, 43, 44, 45, 46, 47, 48, 49, 50, 51, 52, 53 or 54, wherein the open reading frame encodes a seed protein.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1507, 125 to about 1507, 250 to about 1507, 400 to about 1507, 600 to about 1507, upstream of the ATG (1610-1612) located at position 106 to 1612 of SEQ ID NO: 81, which include the minimal promoter region.
In a particular embodiment of the invention said consecutive stretch of about 25 to 3000, including 50 to 2000 or 100 to 500, and up to 1000 or 1500, contiguous nucleotides, e.g., 40 to about 2500, 60 to about 1507, 125 to about 1507, 250 to about 1507, 400 to about 1507, 600 to about 1507, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1507, 125 to about 1507, 250 to about 1507, 400 to about 1507, 600 to about 1507, upstream of the ATG located at position 1610 to 1612 of SEQ ID NO: 81, which include the minimal promoter region. The above-defined stretch of contiguous nucleotides preferably comprises one or more promoter motifs, as shown in Table 22, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 9, or a variant thereof.
Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 910, 125 to about 910, 250 to about 910, 400 to about 910, 600 to about 910, upstream of the ATG (1748-1750) located at position 825 to 1735 of SEQ ID NO: 82, which include the minimal promoter region.
In a particular embodiment of the invention said consecutive stretch of about 25 to 3000, including 50 to 2000 or 100 to 500, and up to 1000 or 1500, contiguous nucleotides, e.g., 40 to about 2500, 60 to about 910, 125 to about 910, 250 to about 910, 400 to about 910, 600 to about 910, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 910, 125 to about 910, 250 to about 910, 400 to about 910, 600 to about 910, upstream of the ATG (1748-1750) located at position 825 to 1735 of SEQ ID NO: 82, which include the minimal promoter region. The above-defined stretch of contiguous nucleotides preferably comprises one or more promoter motifs, as shown in Table 23, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 10, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1131, 125 to about 1131, 250 to about 1131, 400 to about 1131, 600 to about 1131, upstream of the ATG (1185-1160) located at position 44 to 1174 of SEQ ID NO: 83, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1131, 125 to about 1131, 250 to about 1131, 400 to about 1131, 600 to about 1131, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1131, 125 to about 1131, 250 to about 1131, 400 to about 1131, 600 to about 1131, upstream of the ATG (1185-1160) located at position 44 to 1174 of SEQ ID NO: 83, which include the minimal promoter region. The above-defined stretch of contiguous nucleotides preferably comprises one or more promoter motifs, as shown in Table 24, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 11, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 563, 125 to about 563, 250 to about 563, 400 to about 563, upstream of the ATG (624-626) located at position 52 to 614 of SEQ ID NO: 84, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 563, 125 to about 563, 250 to about 563, 400 to about 563, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 563, 125 to about 563, 250 to about 563, 400 to about 563, upstream of the ATG (624-626) located at position 52 to 614 of SEQ ID NO: 84, which include the minimal promoter region. The above-defined stretch of contiguous nucleotides preferably comprises one or more promoter motifs, as shown in Table 25, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 12, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1188, 125 to about 1188, 250 to about 1188, 400 to about 1188, 600 to about 1188, upstream of the ATG (1234-1236) located at position 46 to 1233 of SEQ ID NO: 80, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1188, 125 to about 1188, 250 to about 1188, 400 to about 1188, 600 to about 1188, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1188, 125 to about 1188, 250 to about 1188, 400 to about 1188, 600 to about 1188, upstream of the ATG (1234-1236) located at position 46 to 1233 of SEQ ID NO: 80, which include the minimal promoter region. The above-defined stretch of contiguous nucleotides preferably comprises one or more promoter motifs, as shown in Table 26, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 8, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1945, 125 to about 1945, 250 to about 1945, 400 to about 1945, 600 to about 1945, upstream of the ATG (2428 to 2430) located at position 435 to 2379 of SEQ ID NO: 75, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1945, 125 to about 1945, 250 to about 1945, 400 to about 1945, 600 to about 1945, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1945, 125 to about 1945, 250 to about 1945, 400 to about 1945, 600 to about 1945, upstream of the ATG (2428 to 2430) located at position 435 to 2379 of SEQ ID NO: 75, which include the minimal promoter region. The above-defined stretch of contiguous nucleotides preferably comprises one or more promoter motifs, as shown in Table 27, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 3, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 991, 125 to about 991, 250 to about 991, 400 to about 991, 600 to about 991, upstream of the ATG (996 to 998) located at position 4 to 994 of SEQ ID NO: 85, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 991, 125 to about 991, 250 to about 991, 400 to about 991, 600 to about 991, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 991, 125 to about 991, 250 to about 991, 400 to about 991, 600 to about 991, upstream of the ATG (996 to 998) located at position 4 to 994 of SEQ ID NO: 85, which include the minimal promoter region. The above-defined stretch of contiguous nucleotides preferably comprises one or more promoter motifs, as shown in Table 28, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 13, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 2519, 125 to about 2519, 250 to about 2519, 400 to about 2519, 600 to about 2519, 5 base pairs downstream of the ATG (2511 to 2513) located at position 1 to 2519 of SEQ ID NO: 86, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 2519, 125 to about 2519, 250 to about 2519, 400 to about 2519, 600 to about 2519, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 2519, 125 to about 2519, 250 to about 2519, 400 to about 2519, 600 to about 2519, upstream of the ATG (2511 to 2513) located at position 1 to 2519 of SEQ ID NO: 86, which include the minimal promoter region. The above-defined stretch of contiguous nucleotides preferably comprises one or more promoter motifs, as shown in Table 29, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 14, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 512, 125 to about 512, 250 to about 512, 400 to about 512, upstream of the ATG (678 to 680) located at position 47 to 558 of SEQ ID NO: 76, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 512, 125 to about 512, 250 to about 512, 400 to about 512, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 512, 125 to about 512, 250 to about 512, 400 to about 512, 600 to about 512, upstream of the ATG (678 to 680) located at position 47 to 558 of SEQ ID NO: 76, which include the minimal promoter region. The above-defined stretch of contiguous nucleotides preferably comprises one or more promoter motifs, as shown in Table 30, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 4, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1264, 125 to about 1264, 250 to about 1264, 400 to about 1264, 600 to about 1264, upstream of the ATG (1341 to 1343) located at position 1 to 1264 of SEQ ID NO: 87, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1264, 125 to about 1264, 250 to about 1264, 400 to about 1264, 600 to about 1264, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1264, 125 to about 1264, 250 to about 1264, 400 to about 1264, 600 to about 1264, upstream of the ATG (1341 to 1343) located at position 1 to 1264 of SEQ ID NO: 87, which include the minimal promoter region. The above-defined stretch of contiguous nucleotides preferably comprises one or more promoter motifs, as shown in Table 49, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 15, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1355, 125 to about 1355, 250 to about 1355, 400 to about 1355, 600 to about 1355, upstream of the ATG (1357 to 1359) located at position 1 to 1355 of SEQ ID NO: 78, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1355, 125 to about 1355, 250 to about 1355, 400 to about 1355, 600 to about 1355, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1355, 125 to about 1355, 250 to about 1355, 400 to about 1355, 600 to about 1355, upstream of the ATG (1357 to 1359) located at position 1 to 1355 of SEQ ID NO: 78, which include the minimal promoter region. The above-defined stretch of contiguous nucleotides preferably comprises one or more promoter motifs, as shown in Table 50, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 6, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 623, 125 to about 623, 250 to about 623, 400 to about 623, 500 to about 623, upstream of the ATG (695 to 697) located at position 1 to 623 of SEQ ID NO: 88, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 623, 125 to about 623, 250 to about 623, 400 to about 623, 500 to about 623, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 623, 125 to about 623, 250 to about 623, 400 to about 623, 500 to about 1355, upstream of the ATG (695 to 697) located at position 1 to 623 of SEQ ID NO: 88, which include the minimal promoter region. The above-defined stretch of contiguous nucleotides preferably comprises one or more promoter motifs, as shown in Table 51, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 16, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1950, 125 to about 1950, 250 to about 1950, 400 to about 1950, 600 to about 1950, upstream of the ATG (2700 to 2702) located at position 700 to 2649 of SEQ ID NO: 89, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1950, 125 to about 1950, 250 to about 1950, 400 to about 1950, 600 to about 1950, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1950, 125 to about 1950, 250 to about 1950, 400 to about 1950, 600 to about 1355, upstream of the ATG (2700 to 2702) located at position 700 to 2649 of SEQ ID NO: 89, which include the minimal promoter region. The above-defined stretch of contiguous nucleotides preferably comprises one or more promoter motifs, as shown in Table 52, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 17, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1106, 125 to about 1106, 250 to about 1106, 400 to about 1106, 600 to about 1106, upstream of the ATG (1220 to 1222) located at position 1 to 1106 of SEQ ID NO: 73, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1106, 125 to about 1106, 250 to about 1106, 400 to about 1106, 600 to about 1106, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1106, 125 to about 1106, 250 to about 1106, 400 to about 1106, 600 to about 1355, upstream of the ATG (1220 to 1222) located at position 1 to 1106 of SEQ ID NO: 73, which include the minimal promoter region. The above-defined stretch of contiguous nucleotides preferably comprises one or more promoter motifs, as shown in Table 53, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 1, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 1941, 125 to about 1941, 250 to about 1941, 400 to about 1941, 600 to about 1941, upstream of the ATG (2303 to 2305) located at position 302 to 2242 of SEQ ID NO: 79, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 1941, 125 to about 1941, 250 to about 1941, 400 to about 1941, 600 to about 1941, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 1941, 125 to about 1941, 250 to about 1941, 400 to about 1941, 600 to about 1355, upstream of the ATG (2303 to 2305) located at position 302 to 2242 of SEQ ID NO: 79, which include the minimal promoter region. The above-defined stretch of contiguous nucleotides preferably comprises one or more promoter motifs, as shown in Table 54, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 7, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 922, 125 to about 922, 250 to about 922, 400 to about 922, 600 to about 922, upstream of the ATG (923 to 925) located at position 1 to 922 of SEQ ID NO: 74, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 922, 125 to about 922, 250 to about 922, 400 to about 922, 600 to about 922, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 922, 125 to about 922, 250 to about 922, 400 to about 922, 600 to about 1355, upstream of the ATG (923 to 925) located at position 1 to 922 of SEQ ID NO: 74, which include the minimal promoter region. The above-defined stretch of contiguous nucleotides preferably comprises one or more promoter motifs, as shown in Table 55, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 2, or a variant thereof.

Preferably, the transcription regulating nucleotide sequence and promoters of the invention include a consecutive stretch of about 25 to 3000, including 50 to 2000 or 100 to 500, and up to 1000 or 1500, contiguous nucleotides, e.g., 40 to about 2500, 60 to about 698, 125 to about 698, 250 to about 698, 400 to about 698, 500 to about 698, upstream of the ATG (699 to 671) located at position 1 to 698 of SEQ ID NO: 77, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 698, 125 to about 698, 250 to about 698, 400 to about 698, 500 to about 698, has at least 50% or 60%, preferably at least 70% or 80%, more preferably at least 90% and most preferably at least 95%, nucleic acid sequence identity with a corresponding consecutive stretch of about 25 to 3000, including 50 to 2000 or 100 to 500, and up to 1000 or 1500, contiguous nucleotides, e.g., 40 to about 2500, 60 to about 698, 125 to about 698, 250 to about 698, 400 to about 698, 500 to about 1355, upstream of the ATG (699 to 671) located at position 1 to 698 of SEQ ID NO: 77, which include the minimal promoter region. The above-defined stretch of contiguous nucleotides preferably comprises one or more promoter motifs, as shown in Table 56, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 5, or a variant thereof.

Preferably, the transcription regulating nucleotide sequence and promoters of the invention include a consecutive stretch of about 25 to 3500, including 50 to 3000 or 100 to 500, and up to 1000 or 1500, contiguous nucleotides, e.g., 40 to about 3500, 60 to about 3000, 125 to about 2500, 250 to about 2300, 400 to about 2000, 600 to about 1700, upstream of the ATG located at position 656 to 658 of SEQ ID NO: 196, which include the minimal promoter region.

In a particular embodiment of the invention said consecutive stretch of about 25 to 3000, including 50 to 2000 or 100 to 500, and up to 1000 or 1500, contiguous nucleotides, e.g., 40 to about 2500, 60 to about 922, 125 to about 922, 250 to about 922, 400 to about 922, 600 to about 922, has at least 50% or 60%, preferably at least 70% or 80%, more preferably at least 90% and most preferably at least 95%, nucleic acid sequence identity with a corresponding consecutive stretch of about 25 to 3500, including 50 to 3000 or 100 to 500, and up to 1000 or 1500, contiguous nucleotides, e.g., 40 to about 3500, 60 to about 3000, 125 to about 2500, 250 to about 2300, 400 to about 2000, 600 to about 1700, upstream of the ATG located at position 656 to 658 of SEQ ID NO: 196, which include the minimal promoter region. The above-defined stretch of contiguous nucleotides preferably comprises one or more promoter motifs, as shown in Table 61, preferably selected from the group consisting of TATA box, GC-box, CAAT-box and a transcription start site. A preferred transcription regulating nucleotide sequence to be included into an expression cassette of the present invention has a nucleic acid sequence as shown in SEQ ID NO: 18, or a variant thereof.

In a particularly preferred embodiment said consecutive stretch of nucleotides comprises nucleotide 1440 to 2112 of SEQ ID NO: 18, nucleotide 1600 to 2112 of SEQ ID NO: 18, even more preferred nucleotide 1740 to 2112 of SEQ ID NO: 18, and most preferred nucleotide 1740 to 1999 of SEQ ID NO: 18.

The present invention also contemplates a transcription regulating nucleotide sequences which can be derived from a transcription regulating nucleotide sequence shown in SEQ ID NO: 1, 2, 3, 4, 5, 6, 7, 8, 9, 10, 11, 12, 13, 14, 15, 16, 17, or 18. Said transcription regulating nucleotide sequences are capable of hybridizing, preferably under stringent conditions, to the upstream sequences of the open reading frame shown in SEQ ID NO: 19, 20, 21, 22, 23, 24, 25, 26, 27, 28, 29, 30, 31, 32, 33, 34, 35 or 36, or a variant thereof, i.e. to the transcription regulating nucleotide sequences shown in SEQ ID NOs: 1, 2, 3, 4, 5, 6, 7, 8, 9, 10, 11, 12, 13, 14, 15, 16, 17, or 18, or a variant thereof.

Stringent hybridization conditions as meant herein are, preferably, hybridization conditions in 6 × sodium chloride/sodium citrate (= SSC) at approximately 45°C, followed by one or more wash steps in 0.2 × SSC, 0.1 % SDS at 53 to 65°C, preferably at 55°C, 56°C, 57°C, 58°C, 59°C, 60°C, 61°C, 62°C, 63°C, 64°C or 65°C. The skilled worker knows that these hybridization conditions differ depending on the type of nucleic acid and, for example when organic solvents are present, with regard to the temperature and concentration of the buffer. Examples for stringent hybridization conditions are given in the "General Definitions" section.

Moreover, transcription regulating nucleotide sequences of the present invention can not only be found upstream of the aforementioned open reading frames having a nucleic acid sequence as shown in SEQ ID NO: 19, 20, 21, 22, 23, 24, 25, 26, 27, 28, 29, 30, 31, 32, 33, 34, 35 or 36. Rather, transcription regulating nucleotide sequences can also be found upstream of orthologous, paralogous or homologous genes (i.e. open reading frames). Thus, also preferably, a variant transcription regulating nucleotide sequence comprised by an expression cassette of the present invention has a nucleic acid sequence which hybridizes to a nucleic acid sequences located upstream of an open reading frame sequence being at least 70%, more preferably, at least 80%, at least 90%, at least 91%, at least 92%, at least 93%, at least 94% at least 95%, at least 96%, at least 97%, at least 98%, or at least 99% identical to a sequence as shown in SEQ ID NOs: 19, 20, 21, 22, 23, 24, 25, 26, 27, 28, 29, 30, 31, 32, 33, 34, 35 or 36. The said variant open reading shall encode a polypeptide having the biological activity of the corresponding polypeptide being encoded by the open reading frame shown in SEQ ID NOs: 19, 20, 21, 22, 23, 24, 25, 26, 27, 28, 29, 30, 31, 32, 33, 34, 35 or 36. In this context it should be mentioned that the open reading frame shown in SEQ ID NOs: 19, 20, 21, 22, 23, 24, 25, 26, 27, 28, 29, 30, 31, 32, 33, 34, 35 or 36 encodes a polypeptide having the amino acid sequence shown in SEQ ID NOs: 37, 38, 39, 40, 41, 42, 43, 44, 45, 46, 47, 48, 49, 50, 51, 52, 53 or 54 and, preferably, encodes a seed protein.

Also preferably, a variant transcription regulating nucleotide sequence of the present invention is (i) obtainable by 5' genome walking or TAIL PCR from an open reading frame sequence as shown in SEQ ID NOs: 19, 20, 2.1, 22, 23, 24, 25, 26, 27, 28, 29, 30, 31, 32, 33, 34, 35 or 36 or (ii) obtainable by 5' genome walking or TAIL PCR from a open reading frame sequence being at least 80% identical to an open reading frame as shown in SEQ ID NOs: 19, 20, 21, 22, 23, 24, 25, 26, 27, 28, 29, 30, 31, 32, 33, 34, 35 or 36. Variant expression control sequences are obtainable without further ado by the genome walking technology or by thermal asymmetric interlaced polymerase chain reaction (TAIL-PCR) which can be carried out as described by Liu and Huang, Plant Molecular Biology Reporter, 1998, Vol. 16, pages 175 to 181, as well as references therein, or Liu et al., The Plant Journal, 1995, Vol. 8, pages 457 - 463, and references therein, by using, e.g., commercially available kits.

Suitable oligonucleotides corresponding to a nucleotide sequence of the invention, e.g., for use as primers in probing or amplification reactions as the PCR reaction described abobe, may be about 30 or fewer nucleotides in length (e.g., 9, 12, 15, 18, 20, 21, 22, 23, or 24, or any number between 9 and 30). Generally specific primers are upwards of 14 nucleotides in length. For optimum specificity and cost effectiveness, primers of 16 to 24 nucleotides in length may be preferred. Those skilled in the art are well versed in the design of primers for use processes such as PCR. If required, probing can be done with entire restriction fragments of the gene disclosed herein which may be 100's or even 1000's of nucleotides in length.

Variant transcription regulating nucleotide sequences referred to in this specification for the transcription regulating nucleotide sequence shown in SEQ ID NO: 9, preferably, comprise at least 10, at least 20, at least 30, or all of the sequence motifs recited in Table 22.

Variant transcription regulating nucleotide sequences referred to in this specification for the transcription regulating nucleotide sequence shown in SEQ ID NO: 10, preferably, comprise at least 10, at least 20, at least 30, or all of the sequence motifs recited in Table 23.

Variant transcription regulating nucleotide sequences referred to in this specification for the transcription regulating nucleotide sequence shown in SEQ ID NO: 11, preferably, comprise at least 10, at least 20, at least 30, or all of the sequence motifs recited in Table 24.

Variant transcription regulating nucleotide sequences referred to in this specification for the transcription regulating nucleotide sequence shown in SEQ ID NO: 12, preferably, comprise at least 10, at least 20, at least 30, or all of the sequence motifs recited in Table 25.

Variant transcription regulating nucleotide sequences referred to in this specification for the transcription regulating nucleotide sequence shown in SEQ ID NO: 8, preferably, comprise at least 10, at least 20, at least 30, or all of the sequence motifs recited in Table 26.

Variant transcription regulating nucleotide sequences referred to in this specification for the transcription regulating nucleotide sequence shown in SEQ ID NO: 3, preferably, comprise at least 10, at least 20, at least 30, or all of the sequence motifs recited in Table 27.

Variant transcription regulating nucleotide sequences referred to in this specification for the transcription regulating nucleotide sequence shown in SEQ ID NO: 13, preferably, comprise at least 10, at least 20, at least 30, or all of the sequence motifs recited in Table 28

Variant transcription regulating nucleotide sequences referred to in this specification for the transcription regulating nucleotide sequence shown in SEQ ID NO: 14, preferably, comprise at least 10, at least 20, at least 30, or all of the sequence motifs recited in Table 29.

Variant transcription regulating nucleotide sequences referred to in this specification for the transcription regulating nucleotide sequence shown in SEQ ID NO: 4, preferably, comprise at least 10, at least 20, at least 30, or all of the sequence motifs recited in Table 30.

Variant transcription regulating nucleotide sequences referred to in this specification for the transcription regulating nucleotide sequence shown in SEQ ID NO: 15, preferably, comprise at least 10, at least 20, at least 30, or all of the sequence motifs recited in Table 49.

Variant transcription regulating nucleotide sequences referred to in this specification for the transcription regulating nucleotide sequence shown in SEQ ID NO: 6, preferably, comprise at least 10, at least 20, at least 30, or all of the sequence motifs recited in Table 50.

Variant transcription regulating nucleotide sequences referred to in this specification for the transcription regulating nucleotide sequence shown in SEQ ID NO: 16, preferably, comprise at least 10, at least 20, at least 30, or all of the sequence motifs recited in Table 51.

Variant transcription regulating nucleotide sequences referred to in this specification for the transcription regulating nucleotide sequence shown in SEQ ID NO: 17 preferably, comprise at least 10, at least 20, at least 30, or all of the sequence motifs recited in Table 52.

Variant transcription regulating nucleotide sequences referred to in this specification for the transcription regulating nucleotide sequence shown in SEQ ID NO: 1, preferably, comprise at least 10, at least 20, at least 30, or all of the sequence motifs recited in Table 53.

Variant transcription regulating nucleotide sequences referred to in this specification for the transcription regulating nucleotide sequence shown in SEQ ID NO: 7, preferably, comprise at least 10, at least 20, at least 30, or all of the sequence motifs recited in Table 54.

Variant transcription regulating nucleotide sequences referred to in this specification for the transcription regulating nucleotide sequence shown in SEQ ID NO: 2, preferably, comprise at least 10, at least 20, at least 30, or all of the sequence motifs recited in Table 55.

Variant transcription regulating nucleotide sequences referred to in this specification for the transcription regulating nucleotide sequence shown in SEQ ID NO: 5, preferably, comprise at least 10, at least 20, at least 30, or all of the sequence motifs recited in Table 56.

Variant transcription regulating nucleotide sequences referred to in this specification for the transcription regulating nucleotide sequence shown in SEQ ID NO: 18, preferably, comprise at least 10, at least 20, at least 30, or all of the sequence motifs recited in Table 61.

Examples for preferred variant transcription regulating sequences are shown in SEQ ID NOs 109 to 126 as well as 127 to 144.

Compared to the corresponding transcription regulating nucleotide sequences, the aforementioned variants (as shown in SEQ ID NOs: 109 to 144) do not comprise start codons (ATG). The start codons are either replaced by BVH (SEQ ID NOs: 109, 110, 111, 112, 113, 114, 115, 116, 117, 118, 119, 120, 121, 122, 123, 124, 125, 126) or by BVH plus stop codons ( SEQ ID NOs: 127, 128, 129, 130, 131, 132, 133, 134, 135, 136, 137, 138, 139, 140, 141, 142, 143, 144) between any two start codons (according to the IUPAC nomenclature: B represents C or G or T, V represents A or C or G, and H represents A or C or T). Thus, variant transcription regulating sequences may be obtained by mutating putative start codons as described above.

Without significantly impairing the properties mentioned, non-essential sequences of the transcription regulating nucleotide sequence of the invention can be deleted. Delimitation of the expression control sequence to particular essential regulatory regions can also be undertaken with the aid of a computer program such as the PLACE program ("Plant Cis-acting Regulatory DNA Elements") (Higo K et al. (1999) Nucleic Acids Res 27:1, 297-300), see Table 5, or the BIOBASE database "Transfac" (Biologische Datenbanken GmbH, Braunschweig). By such measures, variant transcription regulating nucleotide sequences as specified above can be artificially generated. Moreover, processes for mutagenizing nucleic acid sequences are known to the skilled worker and include, e.g., the use of oligonucleotides having one or more mutations compared with the region to be mutated (e.g. within the framework of a site-specific mutagenesis). Primers having approximately 15 to approximately 75 nucleotides or more are typically employed, with preferably about 10 to about 25 or more nucleotide residues being located on both sides of a sequence to be modified. Details and procedure for said mutagenesis processes are familiar to the skilled worker (Kunkel et al. (1987) Methods Enzymol 154:367-382; Tomic et al. (1990) Nucl Acids Res 12:1656; Upender et al. (1995) Biotechniques 18(1):29-30; U.S. Pat. No. 4,237,224). A mutagenesis can also be achieved by treatment of, for example, vectors comprising the transcription regulating nucleotide sequence of the invention with mutagenizing agents such as hydroxylamine. Mutagenesis also yields variant expression cassettes of the invention as specified above.

Generally, the transcription regulating nucleotide sequences and promoters of the invention may be employed to express a nucleic acid segment that is operably linked to said promoter such as, for example, an open reading frame, or a portion thereof, an anti-sense sequence, a sequence encoding for a double-stranded RNA sequence, or a transgene in plants.

Accordingly, a further embodiment of the present invention, the expression cassette of the present invention comprises at least one polynucleotide of interest being operatively linked to the transcription regulating nucleotide sequence and/or at least one a termination sequence or transcription. Thus, the expression cassette of the present invention, preferably, comprises a transcription regulating nucleotide sequence for the expression of at least one polynucleotide of interest. However, expression cassettes comprising transcription regulating nucleotide sequences with at least two, three, four or five or even more transcription regulating nucleotide sequences for polynucleotides of interest are also contemplated by the present invention.

The term "polynucleotide of interest" refers to a nucleic acid which shall be expressed under the control of the transcription regulating nucleotide sequence referred to herein. Preferably, a polynucleotide of interest encodes a polypeptide the presence of which is desired in a cell or plant seed as referred to herein. Such a polypeptide may be an enzyme which is required for the synthesis of seed storage compounds or may be a seed storage protein. It is to be understood that if the polynucleotide of interest encodes a polypeptide, transcription of the nucleic acid in RNA and translation of the transcribed RNA into the polypeptide may be required. A polynucleotide of interest, also preferably, includes biologically active RNA molecules and, more preferably, antisense RNAs, ribozymes, micro RNAs or siRNAs. For example, an undesired enzymatic activity in a seed can be reduced due to the seed specific expression of an antisense RNAs, ribozymes, micro RNAs or siRNAs. The underlying biological principles of action of the aforementioned biologically active RNA molecules are well known in the art. Moreover, the person skilled in the art is well aware of how to obtain nucleic acids which encode such biologically active RNA molecules. It is to be understood that the biologically active RNA molecules may be directly obtained by transcription of the nucleic acid of interest, i.e. without translation into a polypeptide. Preferably, at least one polynucleotide of interest to be expressed under the control of the transcription regulating nucleotide sequence of the present invention is heterologous in relation to said expression control sequence, i.e. it is not naturally under the control thereof, but said control has been produced in a non-natural manner (for example by genetic engineering processes)

An operable linkage may - for example - comprise an sequential arrangement of the transcription regulating nucleotide sequence of the invention (for example a sequence as described by SEQ ID NO: 1, 2, 3, 4, 5, 6, 7, 8, 9, 10, 11, 12, 13, 14, 15, 16, 17, or 18) with a nucleic acid sequence to be expressed, and - optionally - additional regulatory elements such as for example polyadenylation or transcription termination elements, enhancers, introns etc, in a way that the transcription regulating nucleotide sequence can fulfill its function in the process of expression the nucleic acid sequence of interest under the appropriate conditions. The term "appropriate conditions" mean preferably the presence of the expression cassette in a plant cell. Preferred are arrangements, in which the nucleic acid sequence of interest to be expressed is placed down-stream (i.e., in 3'-direction) of the transcription regulating nucleotide sequence of the invention in a way, that both sequences are covalently linked. Optionally additional sequences may be inserted in-between the two sequences. Such sequences may be for example linker or multiple cloning sites. Furthermore, sequences can be inserted coding for parts of fusion proteins (in case a fusion protein of the protein encoded by the nucleic acid of interest is intended to be expressed). Preferably, the distance between the polynucleotide of interest to be expressed and the transcription regulating nucleotide sequence of the invention is not more than 200 base pairs, preferably not more than 100 base pairs, more preferably no more than 50 base pairs.

An operable linkage in relation to any expression cassette or of the invention may be realized by various methods known in the art, comprising both *in vitro* and *in vivo* procedure. Thus, an expression cassette of the invention or an vector comprising such expression cassette may by realized using standard recombination and cloning techniques well known in the art (see e.g., Maniatis 1989; Silhavy 1984; Ausubel 1987).

An expression cassette may also be assembled by inserting a transcription regulating nucleotide sequence of the invention (for example a sequence as described by SEQ ID NO: 1, 2, 3, 4, 5, 6, 7, 8, 9, 10, 11, 12, 13, 14, 15, 16, 17, or 18) into the plant genome. Such insertion will result in an operable linkage to a nucleic acid sequence of interest, which as such already existed in the genome. By the insertion the nucleic acid of interest is expressed in a seed-preferential or seed-specific way due to the transcription regulating properties of the transcription regulating nucleotide sequence. The insertion may be directed or by chance. Preferably the insertion is directed and realized by for example homologous recombination. By this procedure a natural promoter may be exchanged against the transcription regulating nucleotide sequence of the invention, thereby modifying the expression profile of an endogenous gene. The transcription regulating nucleotide sequence may also be inserted in a way, that antisense mRNA of an endogenous gene is expressed, thereby inducing gene silencing.

Similar, a polynucleotide of interest to be expressed may by inserted into a plant genome comprising the transcription regulating nucleotide sequence in its natural genomic environment (i.e. linked to its natural gene) in a way that the inserted sequence becomes operably linked to the transcription regulating nucleotide sequence, thereby forming an expression cassette of the invention.

The expression cassette may be employed for numerous expression purposes such as for example expression of a protein, or expression of a antisense RNA, sense or double-stranded RNA. Preferably, expression of the nucleic acid sequence confers to the plant an agronomically valuable trait.

The polynucleotide of interest to be linked to the transcription regulating nucleotide sequence of the invention may be obtained from an insect resistance gene, a disease resistance gene such as, for example, a bacterial disease resistance gene, a fungal disease resistance gene, a viral disease resistance gene, a nematode disease resistance gene, a herbicide resistance gene, a gene affecting grain composition or quality, a nutrient utilization gene, a mycotoxin reduction gene, a male sterility gene, a selectable marker gene, a screenable marker gene, a negative selectable marker, a positive selectable marker, a gene affecting plant agronomic characteristics, i.e., yield, standability, and the like, or an environment or stress resistance gene, i.e., one or more genes that confer herbicide resistance or tolerance, insect resistance or tolerance, disease resistance or tolerance (viral, bacterial, fungal, oomycete, or nematode), stress tolerance or resistance (as exemplified by resistance or tolerance to drought, heat, chilling, freezing, excessive moisture, salt stress, or oxidative stress), increased yields, food content and makeup, physical appearance, male sterility, drydown, standability, prolificacy, starch properties or quantity, oil quantity and quality, amino acid or protein composition, and the like. By "resistant" is meant a plant, which exhibits substantially no phenotypic changes as a consequence of agent administration, infection with a pathogen, or exposure to stress. By "tolerant" is meant a plant, which, although it may exhibit some phenotypic changes as a consequence of infection, does not have a substantially decreased reproductive capacity or substantially altered metabolism.

Seed-specific transcription regulating nucleotide sequences (e.g., promoters) are useful for expressing a wide variety of genes including those which alter metabolic pathways, confer disease resistance, for protein production, e.g., antibody production, or to improve nutrient uptake and the like. Seed-specific transcription regulating nucleotide sequences (e.g., promoters) may be modified so as to be regulatable, e.g., inducible. The genes and transcription regulating nucleotide sequences (e.g., promoters) described hereinabove can be used to identify orthologous genes and their transcription regulating nucleotide sequences (e.g., promoters) which are also likely expressed in a particular tissue and/or development manner. Moreover, the orthologous transcription regulating nucleotide sequences (e.g., promoters) are useful to express linked open reading frames. In addition, by aligning the transcription regulating nucleotide sequences (e.g., promoters) of these orthologs, novel cis elements can be identified that are useful to generate synthetic transcription regulating nucleotide sequences (e.g., promoters).

Another object of the present invention refers to a vector comprising the expression cassette of the present invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, natural competence, carbon-based clusters, chemically mediated transfer, electroporation or particle bombardment (e.g., "gene-gun"). Suitable methods for the transformation or transfection of host cells, including plant cells, can be found in Sambrook et al. (Molecular Cloning: A Laboratory Manual, 2nd ed., Cold Spring Harbor Laboratory, Cold Spring Harbor Laboratory Press, Cold Spring Harbor, NY, 1989) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

Preferably, the vector referred to herein is suitable as a cloning vector, i.e. replicable in microbial systems. Such vectors ensure efficient cloning in bacteria and, preferably, yeasts or fungi and make possible the stable transformation of plants. Those which must be mentioned are, in particular, various binary and co-integrated vector systems which are suitable for the T-DNA-mediated transformation. Such vector systems are, as a rule, characterized in that they contain at least the vir genes, which are required for the Agrobacterium-mediated transformation, and the sequences which delimit the T-DNA (T-DNA border). These vector systems, preferably, also comprise further cis-regulatory regions such as promoters and terminators and/or selection markers with which suitable transformed host cells or organisms can be identified. While co-integrated vector systems have vir genes and T-DNA sequences arranged on the same vector, binary systems are based on at least two vectors, one of which bears vir genes, but no T-DNA, while a second one bears T-DNA, but no vir gene. As a consequence, the last-mentioned vectors are relatively small, easy to manipulate and can be replicated both in E. coli and in Agrobacterium. An overview of binary vectors and their use can be found in Hellens et al, Trends in Plant Science (2000) 5, 446-451. Furthermore, by using appropriate cloning vectors, the expression cassette of the invention can be introduced into host cells or organisms such as plants or animals and, thus, be used in the transformation of plants, such as those which are published, and cited, in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225.

More preferably, the vector of the present invention is an expression vector. In such an expression vector, the expression cassette comprises a transcription regulating nucleotide sequence as specified above allowing for expression in eukaryotic cells or isolated fractions thereof. An expression vector may, in addition to the expression cassette of the invention, also comprise further regulatory elements including transcriptional as well as translational enhancers. Preferably, the expression vector is also a gene transfer or targeting vector. Expression vectors derived from viruses such as retroviruses, vaccinia virus, adeno-associated virus, herpes viruses, or bovine papilloma virus, may be used for delivery of the expression cassettes or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Suitable expression vector backbones are, preferably, derived from expression vectors known in the art such as Okayama-Berg cDNA expression vector pcDV1 (Pharmacia), pCDM8, pRc/CMV, pcDNA1, pcDNA3 (Invitrogene) or pSPORT1 (GIBCO BRL). Further examples of typical fusion expression vectors are pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), where glutathione S-transferase (GST), maltose E-binding protein and protein A, respectively, are fused with the nucleic acid of interest encoding a protein to be expressed. The target gene expression of the pTrc vector is based on the transcription from a hybrid trp-lac fusion promoter by host RNA polymerase. The target gene expression from the pET 11d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) from a resident λ-prophage which harbors a T7 gn1 gene under the transcriptional control of the lacUV 5 promoter. Examples of vectors for expression in the yeast S. cerevisiae comprise pYepSecl (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and processes for the construction of vectors which are suitable for use in other fungi, such as the filamentous fungi, comprise those which are described in detail in: van den Hondel, C.A.M.J.J., & Punt, P.J. (1991) "Gene transfer systems and vector development for filamentous fungi, in: Applied Molecular Genetics of fungi, J.F. Peberdy et al., Ed., pp. 1-28, Cambridge University Press: Cambridge, or in: More Gene Manipulations in Fungi (J.W. Bennett & L.L. Lasure, Ed., pp. 396-428: Academic Press: San Diego). Further suitable yeast vectors are, for example, pAG-1, YEp6, YEp13 or pEMBLYe23.

The vector of the present invention comprising the expresseion cassette will have to be propagated and amplified in a suitable organism, i.e. expression host.

Accordingly, another embodiment of the invention relates to transgenic host cells or non-human, transgenic organisms comprising an expression cassette of the invention. Preferred are prokaryotic and eukaryotic organisms. Both microorganism and higher organisms are comprised. Preferred microorganisms are bacteria, yeast, algae, and fungi. Preferred bacteria are those of the genus *Escherichia, Erwinia*, *Agrobacterium, Flavobacterium, Alcaligenes, Pseudomonas, Bacillus or Cyanobacterim* such as - for example - *Synechocystis* and other bacteria described in Brock Biology of Microorganisms Eighth Edition (pages A-8, A-9, A10 and A11). Most preferably the transgenic cells or non-human, transgenic organisms comprising an expression cassette of the invention is a plant cell or plant (as defined above), more preferably a plant used for oil production such as - for example - *Brassica napus, Brassica juncea, Linum usitatissimum,* soybean, Camelina or sunflower.

Especially preferred are microorganisms capable to infect plants and to transfer DNA into their genome, especially bacteria of the genus Agrobacterium, preferably Agrobacterium tumefaciens and rhizogenes. Preferred yeasts are Candida, Saccharomyces, Hansenula and Pichia. Preferred fungi are Aspergillus, Trichoderma, Ashbya, Neurospora, Fusarium, and Beauveria.

In a preferred embodiment of the present invention, the host cell relates to a plant cell, plant, a plant seed, a non-human animal or a multicellular micro-organism.

Accordingly, the present invention further refers to a transgenic plant cell, plant tissue, plant organ, or plant seed, comprising the expression cassette or the vector of the present invention.

The expression cassette or vector may be present in the cytoplasm of the organism or may be incorporated into the genome either heterologous or by homologous recombination. Host cells, in particular those obtained from plants or animals, may be introduced into a developing embryo in order to obtain mosaic or chimeric organisms, i.e. transgenic organisms, i.e. plants, comprising the host cells of the present invention. Suitable transgenic organisms are, preferably, all organisms which are suitable for the expression of recombinant genes.

The nature of the transgenic plant cells is not limited, for example, the plant cell can be a monocotyledonous plant cell, or a dicotyledonous plant cell. Preferably, the transgenic plant transgenic plant tissue, plant organ, plant or seed is a monocotyledonous plant or a plant cell, plant tissue, plant organ, plant seed from a monocotyledonous plant.

Examples of transgenic plant cells finding use with the invention include cells (or entire plants or plant parts) derived from the genera: Ananas, Musa, Vitis, Fragaria, Lotus, Medicago, Onobrychis, Trifolium, Trigonella, Vigna, Citrus, Carica, Persea, Prunus, Syragrus, Theobroma, Coffea, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Mangifera, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucurbita, Cucumis, Browaalia, Lolium, Malus, Apium, Gossypium, Vicia, Lathyrus, Lupinus, Pachyrhizus, Wisteria, Stizolobium, Agrostis, Phleum, Dactylis, Sorghum, Setaria, Zea, Oryza, Triticum, Secale, Avena, Hordeum, Saccharum, Poa, Festuca, Stenotaphrum, Cynodon, Coix, Olyreae, Phareae, Glycine, Pisum, Psidium, Passiflora, Cicer, Phaseolus, Lens, and Arachis

Preferably, the transgenic plant cells finding use with the invention include cells (or entire plants or plant parts) from the family of poaceae, such as the genera Hordeum, Secale, Avena, Sorghum, Andropogon, Holcus, Panicum, Oryza, Zea, Triticum, for example the genera and species *Hordeum vulgare, Hordeum jubatum, Hordeum murinum, Hordeum secalinum, Hordeum distichon, Hordeum aegiceras, Hordeum hexastichon, Hordeum hexastichum, Hordeum irregulare, Hordeum sativum, Hordeum secalinum, Secale cereale,* Avena sativa, *Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum, Oryza sativa, Oryza latifolia, Zea mays, Triticum aestivum, Triticum durum, Triticum turgidum, Triticum hybernum, Triticum macha, Triticum sativum* or *Triticum vulgare*.

In particular, preferred plants to be used as transgenic plants in accordance with the present invention are oil fruit crops which comprise large amounts of lipid compounds, such as peanut, oilseed rape, canola, sunflower, safflower, poppy, mustard, hemp, castor-oil plant, olive, sesame, Calendula, Punica, evening primrose, mullein, thistle, wild roses, hazelnut, almond, macadamia, avocado, bay, pumpkin/squash, linseed, soybean, pistachios, borage, trees (oil palm, coconut, walnut) or crops such as maize, wheat, rye, oats, triticale, rice, barley, cotton, cassava, pepper, Tagetes, Solanaceae plants such as potato, tobacco, eggplant and tomato, Vicia species, pea, alfalfa or bushy plants (coffee, cacao, tea), Salix species, and perennial grasses and fodder crops. Preferred plants according to the invention are oil crop plants such as peanut, oilseed rape, canola, sunflower, safflower, poppy, mustard, hemp, castor-oil plant, olive, Calendula, Punica, evening primrose, pumpkin/squash, linseed, soybean, borage, trees (oil palm, coconut).

In another aspect, the present invention relates to a method for producing a transgenic plant tissue, plant organ, plant or seed comprising

(a) introducing the expression cassette or the vector of the invention into a plant cell; and
(b) regenerating said plant cell to form a plant tissue, plant organ, plant or seed.

Expression cassettes can be introduced into plant cells in a number of art-recognized ways. Plant species may be transformed with the DNA construct of the present invention by the DNA-mediated transformation of plant cell protoplasts and subsequent regeneration of the plant from the transformed protoplasts in accordance with procedures well known in the art.

Any plant tissue capable of subsequent clonal propagation, whether by organogenesis or embryogenesis, may be transformed with a vector of the present invention. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristems, axillary buds, and root meristems), and induced meristem tissue (e.g., cotyledon meristem and ultilane meristem).

Plants of the present invention may take a variety of forms. The plants may be chimeras of transformed cells and non-transformed cells; the plants may be clonal transformants (e.g., all cells transformed to contain the expression cassette); the plants may comprise grafts of transformed and untransformed tissues (e.g., a transformed root stock grafted to an untransformed scion in citrus species). The transformed plants may be propagated by a variety of means, such as by clonal propagation or classical breeding techniques. For example, first generation (or T1) transformed plants may be selfed to give homozygous second generation (or T2) transformed plants, and the T2 plants further propagated through classical breeding techniques. A dominant selectable marker (such as npt II) can be associated with the expression cassette to assist in breeding.

Transformation of plants can be undertaken with a single DNA molecule or multiple DNA molecules (i.e., co-transformation), and both these techniques are suitable for use with the expression cassettes of the present invention. Numerous transformation vectors are available for plant transformation, and the expression cassettes of this invention can be used in conjunction with any such vectors. The selection of vector will depend upon the preferred transformation technique and the target species for transformation.

A variety of techniques are available and known to those skilled in the art for introduction of constructs into a plant cell host. These techniques generally include transformation with DNA employing *A. tumefaciens* or A. rhizogenes as the transforming agent, liposomes, PEG precipitation, electroporation, DNA injection, direct DNA uptake, microprojectile bombardment, particle acceleration, and the like (See, for example, EP 295959 and EP 138341) (see below). However, cells other than plant cells may be transformed with the expression cassettes of the invention. The general descriptions of plant expression vectors and reporter genes, and Agrobacterium and Agrobacterium-mediated gene transfer, can be found in Gruber et al. (1993).

Expression vectors containing genomic or synthetic fragments can be introduced into protoplasts or into intact tissues or isolated cells. Preferably expression vectors are introduced into intact tissue. General methods of culturing plant tissues are provided for example by Maki et al., (1993); and by Phillips et al. (1988). Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device. See, for example, Tomes et al. (1995). The vectors of the invention can not only be used for expression of structural genes but may also be used in exon-trap cloning, or promoter trap procedures to detect differential gene expression in varieties of tissues (Lindsey 1993; Auch & Reth 1990).

It is particularly preferred to use the binary type vectors of Ti and Ri plasmids of Agrobacterium spp. Ti-derived vectors transform a wide variety of higher plants, including monocotyledonous and dicotyledonous plants, such as soybean, cotton, rape, tobacco, and rice (Pacciotti 1985: Byrne 1987; Sukhapinda 1987; Lorz 1985; Potrykus, 1985; Park 1985: Hiei 1994). The use of T-DNA to transform plant cells has received extensive study and is amply described (EP 120516; Hoekema, 1985; Knauf, 1983; and An 1985). For introduction into plants, the chimeric genes of the invention can be inserted into binary vectors as described in the examples.

Other transformation methods are available to those skilled in the art, such as direct uptake of foreign DNA constructs (see EP 295959), techniques of electroporation (Fromm 1986) or high velocity ballistic bombardment with metal particles coated with the nucleic acid constructs (Kline 1987, and US 4,945,050). Once transformed, the cells can be regenerated by those skilled in the art. Of particular relevance are the recently described methods to transform foreign genes into commercially important crops, such as rapeseed (De Block 1989), sunflower (Everett 1987), soybean (McCabe 1988; Hinchee 1988; Chee 1989; Christou 1989; EP 301749), rice (Hiei 1994), and corn (Gordon-Kamm 1990; Fromm 1990).

Those skilled in the art will appreciate that the choice of method might depend on the type of plant, i.e., monocotyledonous or dicotyledonous, targeted for transformation. Suitable methods of transforming plant cells include, but are not limited to, microinjection (Crossway 1986), electroporation (Riggs 1986), Agrobacterium-mediated transformation (Hinchee 1988), direct gene transfer (Paszkowski 1984), and ballistic particle acceleration using devices available from Agracetus, Inc., Madison, Wis. And BioRad, Hercules, Calif. (see, for example, US 4,945,050; and McCabe 1988). Also see, Weissinger 1988; Sanford 1987 (onion); Christou 1988 (soybean); McCabe 1988 (soybean); Datta 1990 (rice); Klein 1988 (maize); Klein 1988 (maize); Klein 1988 (maize); Fromm 1990 (maize); and Gordon-Kamm 1990 (maize); Svab 1990 (tobacco chloroplast); Koziel 1993 (maize); Shimamoto 1989 (rice); Christou 1991 (rice); European Patent Application EP 0 332 581 (orchardgrass and other Pooideae); Vasil 1993 (wheat); Weeks 1993 (wheat).

In another embodiment, a nucleotide sequence of the present invention is directly transformed into the plastid genome. Plastid transformation technology is extensively described in US 5,451,513, 5,545,817, and 5,545,818, in PCT application no. WO 95/16783, and in McBride et al., 1994. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate orthologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab 1990; Staub 1992). This resulted in stable homoplasmic transformants at a frequency of approximately one per 100 bombardments of target leaves. The presence of cloning sites between these markers allowed creation of a plastid-targeting vector for introduction of foreign genes (Staub 1993). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3N-adenyltransferase (Svab 1993). Other selectable markers useful for plastid transformation are known in the art and encompassed within the scope of the invention. Typically, approximately 15-20 cell division cycles following transformation are required to reach a homoplastidic state. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein. In a preferred embodiment, a nucleotide sequence of the present invention is inserted into a plastid-targeting vector and transformed into the plastid genome of a desired plant host. Plants homoplastic for plastid genomes containing a nucleotide sequence of the present invention are obtained, and are preferentially capable of high expression of the nucleotide sequence.

*Agrobacterium tumefaciens* cells containing a vector comprising an expression cassette of the present invention, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous Agrobacterium vector systems useful in carrying out the present invention are known.

Various *Agrobacterium* strains can be employed, preferably disarmed *Agrobacterium tumefaciens* or *rhizogenes* strains. In a preferred embodiment, *Agrobacterium* strains for use in the practice of the invention include octopine strains, e.g., LBA4404 or agropine strains, e.g., EHA101 or EHA105. Suitable strains of *A. tumefaciens* for DNA transfer are for example EHA101[pEHA101] (Hood 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are *Agrobacterium* tumefaciens C58, a nopaline strain. Other suitable strains are *A. tumefaciens* C58C1 (Van Larebeke 1974), A136 (Watson 1975) or LBA4011 (Klapwijk 1980). In another preferred embodiment the soil-borne bacterium is a disarmed variant of *Agrobacterium rhizogenes* strain K599 (NCPPB 2659). Preferably, these strains are comprising disarmed plasmid variants of a Ti- or Ri-plasmid providing the functions required for T-DNA transfer into plant cells (e.g., the vir genes). In a preferred embodiment, the *Agrobacterium* strain used to transform the plant tissue pre-cultured with the plant phenolic compound contains a L,L-succinamopine type Ti-plasmid, preferably disarmed, such as pEHA101. In another preferred embodiment, the *Agrobacterium* strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the virF gene be deleted or inactivated (Jarschow 1991).

The method of the invention can also be used in combination with particular *Agrobacterium* strains, to further increase the transformation efficiency, such as *Agrobacterium* strains wherein the vir gene expression and/or induction thereof is altered due to the presence of mutant or chimeric virA or virG genes (e.g. Hansen 1994; Chen and Winans 1991; Scheeren-Groot, 1994). Preferred are further combinations of *Agrobacterium* tumefaciens strain LBA4404 (Hiei 1994) with super-virulent plasmids. These are preferably pTOK246-based vectors (Ishida 1996).

A binary vector or any other vector can be modified by common DNA recombination techniques, multiplied in E. coli, and introduced into *Agrobacterium* by e.g., electroporation or other transformation techniques (Mozo & Hooykaas 1991).

*Agrobacterium* is grown and used in a manner similar to that described in Ishida (1996). The vector comprising *Agrobacterium* strain may, for example, be grown for 3 days on YP medium (5 g/l yeast extract, 10 g/l peptone, 5 g/l NaCl, 15 g/l agar, pH 6.8) supplemented with the appropriate antibiotic (e.g., 50 mg/l spectinomycin). Bacteria are collected with a loop from the solid medium and resuspended. In a preferred embodiment of the invention, *Agrobacterium* cultures are started by use of aliquots frozen at -80°C.

The transformation of the target tissue (e.g., an immature embryo) by the *Agrobacterium* may be carried out by merely contacting the target tissue with the *Agrobacterium.* The concentration of *Agrobacterium* used for infection and co-cultivation may need to be varied. For example, a cell suspension of the *Agrobacterium* having a population density of approximately from $10^5$ - $10^{11}$, preferably $10^6$ to $10^{10}$, more preferably about $10^8$ cells or cfu / ml is prepared and the target tissue is immersed in this suspension for about 3 to 10 minutes. The resulting target tissue is then cultured on a solid medium for several days together with the *Agrobacterium.*

Preferably, the bacterium is employed in concentration of $10^6$ to $10^{10}$ cfu/ml. In a preferred embodiment for the co-cultivation step about 1 to 10 $\mu$l of a suspension of the soil-borne bacterium (e.g., *Agrobacteria*) in the co-cultivation medium are directly applied to each target tissue explant and air-dried. This is saving labor and time and is reducing unintended Agrobacterium-mediated damage by excess Agrobacterium usage.

For *Agrobacterium* treatment, the bacteria are resuspended in a plant compatible co-cultivation medium. Supplementation of the co-culture medium with antioxidants (e.g., silver nitrate), phenol-absorbing compounds (like polyvinylpyrrolidone, Perl 1996) or thiol compounds (e.g., dithiothreitol, L-cysteine, Olhoft 2001) which can decrease tissue necrosis due to plant defence responses (like phenolic oxidation) may further improve the efficiency of *Agrobacterium*-mediated transformation. In another preferred embodiment, the co-cultivation medium of comprises least one thiol compound, preferably selected from the group consisting of sodium thiolsulfate, dithiotrietol (DTT) and cysteine. Preferably the concentration is between about 1 mM and 10mM of L-Cysteine, 0.1 mM to 5 mM DTT, and/or 0.1 mM to 5 mM sodium thiolsulfate. Preferably, the medium employed during co-cultivation comprises from about 1 $\mu$M to about 10 $\mu$M of silver nitrate and from about 50 mg/L to about 1,000 mg/L of L-Cystein. This results in a highly reduced vulnerability of the target tissue against Agrobacterium-mediated damage (such as induced necrosis) and highly improves overall transformation efficiency.

Various vector systems can be used in combination with Agrobacteria. Preferred are binary vector systems. Common binary vectors are based on "broad host range"-plasmids like pRK252 (Bevan 1984) or pTJS75 (Watson 1985) derived from the P-type plasmid RK2. Most of these vectors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (e.g. pPZP; Hajdukiewicz 1994). Improved vector systems are described also in WO 02/00900.

Methods using either a form of direct gene transfer or Agrobacterium-mediated transfer usually, but not necessarily, are undertaken with a selectable marker, which may provide resistance to an antibiotic (e.g., kanamycin, hygromycin or methotrexate) or a herbicide (e.g., phosphinothricin). The choice of selectable marker for plant transformation is not, however, critical to the invention.

For certain plant species, different antibiotic or herbicide selection markers may be preferred. Selection markers used routinely in transformation include the nptII gene which confers resistance to kanamycin and related antibiotics (Messing & Vierra, 1982; Bevan 1983), the bar gene which confers resistance to the herbicide phosphinothricin (White 1990, Spencer 1990), the hph gene which confers resistance to the antibiotic hygromycin (Blochlinger & Diggelmann), and the dhfr gene, which confers resistance to methotrexate (Bourouis 1983).

Methods for the production and further characterization of stably transformed plants are well-known to the person skilled in the art. As an example, transgenic plant cells are placed in an appropriate selective medium for selection of transgenic cells, which are then grown to callus. Shoots are grown from callus. Plantlets are generated from the shoot by growing in rooting medium. The various constructs normally will be joined to a marker for selection in plant cells. Conveniently, the marker may be resistance to a biocide (particularly an antibiotic, such as kanamycin, G418, bleomycin, hygromycin, chloramphenicol, herbicide, or the like). The particular marker used will allow for selection of transformed cells as compared to cells lacking the DNA, which has been introduced. Components of DNA constructs including transcription cassettes of this invention may be prepared from sequences, which are native (endogenous) or foreign (exogenous) to the host. By "foreign" it is meant that the sequence is not found in the wild-type host into which the construct is introduced. Heterologous constructs will contain at least one region, which is not native to the gene from which the transcription-initiation-region is derived.

To confirm the presence of the transgenes in transgenic cells and plants, a variety of assays may be performed. Such assays include, for example, "molecular biological" assays well known to those of skill in the art, such as Southern and Northern blotting, in situ hybridization and nucleic acid-based amplification methods such as PCR or RT-PCR or TaqMan; "biochemical" assays, such as detecting the presence of a protein product, e.g., by immunological means (ELISAs and Western blots) or by enzymatic function; plant part assays, such as seed assays; and also, by analyzing the phenotype of the whole regenerated plant, e.g., for disease or pest resistance.

DNA may be isolated from cell lines or any plant parts to determine the presence of the preselected nucleic acid segment through the use of techniques well known to those skilled in the art. Note that intact sequences will not always be present, presumably due to rearrangement or deletion of sequences in the cell.

The presence of nucleic acid elements introduced through the methods of this invention may be determined by polymerase chain reaction (PCR). Using these technique discreet fragments of nucleic acid are amplified and detected by gel electrophoresis. This type of analysis permits one to determine whether a preselected nucleic acid segment is present in a stable transformant, but does not prove integration of the introduced preselected nucleic acid segment into the host cell genome. In addition, it is not possible using PCR techniques to determine whether transformants have exogenous genes introduced into different sites in the, genome, i.e., whether transformants are of independent origin. It is contemplated that using PCR techniques it would be possible to clone fragments of the host genomic DNA adjacent to an introduced preselected DNA segment. Known methods of PCR include, but are not limited to, methods using paired primers, nested primers, single specific primers, degenerate primers, gene-specific primers, vector-specific primers, partially mismatched primers, and the like.

Positive proof of DNA integration into the host genome and the independent identities of transformants may be determined using the technique of Southern hybridization. Using this technique specific DNA sequences that were introduced into the host genome and flanking host DNA sequences can be identified. Hence the Southern hybridization pattern of a given transformant serves as an identifying characteristic of that transformant. In addition it is possible through Southern hybridization to demonstrate the presence of introduced preselected DNA segments in high molecular weight DNA, i.e., confirm that the introduced preselected, DNA segment has been integrated into the host cell genome. The technique of Southern hybridization provides information that is obtained using PCR, e.g., the presence of a preselected DNA segment, but also demonstrates integration into the genome and characterizes each individual transformant.

It is contemplated that using the techniques of dot or slot blot hybridization which are modifications of Southern hybridization techniques one could obtain the same information that is derived from PCR, e.g., the presence of a preselected DNA segment.

Both PCR and Southern hybridization techniques can be used to demonstrate transmission of a preselected DNA segment to progeny. In most instances the characteristic Southern hybridization pattern for a given transformant will segregate in progeny as one or more Mendelian genes (Spencer 1992); Laursen 1994) indicating stable inheritance of the gene. The non-chimeric nature of the callus and the parental transformants (Ro) was suggested by germline transmission and the identical Southern blot hybridization patterns and intensities of the transforming DNA in callus, $R_0$ plants and $R_1$ progeny that segregated for the transformed gene.

Whereas DNA analysis techniques may be conducted using DNA isolated from any part of a plant, RNA may only be expressed in particular cells or tissue types and hence it will be necessary to prepare RNA for analysis from these tissues. PCR techniques may also be used for detection and quantitation of RNA produced from introduced preselected DNA segments. In this application of PCR it is first necessary to reverse transcribe RNA into DNA, using enzymes such as reverse transcriptase, and then through the use of conventional PCR techniques amplify the DNA. In most instances PCR techniques, while useful, will not demonstrate integrity of the RNA product. Further information about the nature of the RNA product may be obtained by Northern blotting. This technique will demonstrate the presence of an RNA species and give information about the integrity of that RNA. The presence or absence of an RNA species can also be determined using dot or slot blot Northern hybridizations. These techniques are modifications of Northern blotting and will only demonstrate the presence or absence of an RNA species.

While Southern blotting and PCR may be used to detect the preselected DNA segment in question, they do not provide information as to whether the preselected DNA segment is being expressed. Expression may be evaluated by specifically identifying the protein products of the introduced preselected DNA segments or evaluating the phenotypic changes brought about by their expression.

Assays for the production and identification of specific proteins may make use of physical-chemical, structural, functional, or other properties of the proteins. Unique physical-chemical or structural properties allow the proteins to be separated and identified by electrophoretic procedures, such as native or denaturing gel electrophoresis or isoelectric focusing, or by chromatographic techniques such as ion exchange or gel exclusion chromatography. The unique structures of individual proteins offer opportunities for use of specific antibodies to detect their presence in formats such as an ELISA assay. Combinations of approaches may be employed with even greater specificity such as Western blotting in which antibodies are used to locate individual gene products that have been separated by electrophoretic techniques. Additional techniques may be employed to absolutely confirm the identity of the product of interest such as evaluation by amino acid sequencing following purification. Although these are among the most commonly employed, other procedures may be additionally used.

Assay procedures may also be used to identify the expression of proteins by their functionality, especially the ability of enzymes to catalyze specific chemical reactions involving specific substrates and products. These reactions may be followed by providing and quantifying the loss of substrates or the generation of products of the reactions by physical or chemical procedures. Examples are as varied as the enzyme to be analyzed.

Very frequently the expression of a gene product is determined by evaluating the phenotypic results of its expression. These assays also may take many forms including but not limited to analyzing changes in the chemical composition, morphology, or physiological properties of the plant. Morphological changes may include greater stature or thicker stalks. Most often changes in response of plants or plant parts to imposed treatments are evaluated under carefully controlled conditions termed bioassays.

The following section provides examples of particular polynucleotides of interest, which can be operably linked to the expression cassette of the present invention.

1. Exemplary Transgenes

1.1. Herbicide Resistance

The genes encoding phosphinothricin acetyltransferase (bar and pat), glyphosate tolerant EPSP synthase genes, the glyphosate degradative enzyme gene gox encoding glyphosate oxidoreductase, deh (encoding a dehalogenase enzyme that inactivates dalapon), herbicide resistant (e.g., sulfonylurea and imidazolinone) acetolactate synthase, and bxn genes (encoding a nitrilase enzyme that degrades bromoxynil) are good examples of herbicide resistant genes for use in transformation. The bar and pat genes code for an enzyme, phosphinothricin acetyltransferase (PAT), which inactivates the herbicide phosphinothricin and prevents this compound from inhibiting glutamine synthetase enzymes. The enzyme 5-enolpyruvylshikimate 3-phosphate synthase (EPSP Synthase), is normally inhibited by the herbicide N-(phosphonomethyl) glycine (glyphosate). However, genes are known that encode glyphosate-resistant EPSP Synthase enzymes. The deh gene encodes the enzyme dalapon dehalogenase and confers resistance to the herbicide dalapon. The bxn gene codes for a specific nitrilase enzyme that converts bromoxynil to a non-herbicidal degradation product.

1.2 Insect Resistance

An important aspect of the present invention concerns the introduction of insect resistance-conferring genes into plants. Potential insect resistance genes, which can be introduced, include Bacillus thuringiensis crystal toxin genes or Bt genes (Watrud 1985). Bt genes may provide resistance to lepidopteran or coleopteran pests such as European Corn Borer (ECB) and corn rootworm (CRW). Preferred Bt toxin genes for use in such embodiments include the CryIA(b) and CryIA(c) genes. Endotoxin genes from other species of B. thuringiensis, which affect insect growth or development, may also be employed in this regard. Protease inhibitors may also provide insect resistance (Johnson 1989), and will thus have utility in plant transformation. The use of a protease inhibitor II gene, pinII, from tomato or potato is envisioned to be particularly useful. Even more advantageous is the use of a pinII gene in combination with a Bt toxin gene, the combined effect of which has been discovered by the present inventors to produce synergistic insecticidal activity. Other genes, which encode inhibitors of the insects' digestive system, or those that encode enzymes or co-factors that facilitate the production of inhibitors, may also be useful. Cystatin and amylase inhibitors, such as those from wheat and barley, may exemplify this group.

Also, genes encoding lectins may confer additional or alternative insecticide properties. Lectins (originally termed phytohemagglutinins) are multivalent carbohydrate-binding proteins, which have the ability to agglutinate red blood cells from a range of species. Lectins have been identified recently as insecticidal agents with activity against weevils, ECB and rootworm (Murdock 1990; Czapla & Lang, 1990). Lectin genes contemplated to be useful include, for example, barley and wheat germ agglutinin (WGA) and rice lectins (Gatehouse 1984), with WGA being preferred.

Genes controlling the production of large or small polypeptides active against insects when introduced into the insect pests, such as, e.g., lytic peptides, peptide hormones and toxins and venoms, form another aspect of the invention. For example, it is contemplated, that the expression of juvenile hormone esterase, directed towards specific insect pests, may also result in insecticidal activity, or perhaps cause cessation of metamorphosis (Hammock 1990).

Transgenic plants expressing genes, which encode enzymes that affect the integrity of the insect cuticle form yet another aspect of the invention. Such genes include those encoding, e.g., chitinase, proteases, lipases and also genes for the production of nikkomycin, a compound that inhibits chitin synthesis, the introduction of any of which is contemplated to produce insect resistant maize plants. Genes that code for activities that affect insect molting, such those affecting the production of ecdysteroid UDP-glucosyl transferase, also fall within the scope of the useful transgenes of the present invention.

Genes that code for enzymes that facilitate the production of compounds that reduce the nutritional quality of the host plant to insect pests are also encompassed by the present invention. It may be possible, for instance, to confer insecticidal activity on a plant by altering its sterol composition. Sterols are obtained by insects from their diet and are used for hormone synthesis and membrane stability. Therefore alterations in plant sterol composition by expression of novel genes, e.g., those that directly promote the production of undesirable sterols or those that convert desirable sterols into undesirable forms, could have a negative effect on insect growth and/or development and hence endow the plant with insecticidal activity. Lipoxygenases are naturally occurring plant enzymes that have been shown to exhibit anti-nutritional effects on insects and to reduce the nutritional quality of their diet. Therefore, further embodiments of the invention concern transgenic plants with enhanced lipoxygenase activity which may be resistant to insect feeding.

The present invention also provides methods and compositions by which to achieve qualitative or quantitative changes in plant secondary metabolites. One example concerns transforming plants to produce DIMBOA which, it is contemplated, will confer resistance to European corn borer, rootworm and several other maize insect pests. Candidate genes that are particularly considered for use in this regard include those genes at the bx locus known to be involved in the synthetic DIMBOA pathway (Dunn 1981). The introduction of genes that can regulate the production of maysin, and genes involved in the production of dhurrin in sorghum, is also contemplated to be of use in facilitating resistance to earworm and rootworm, respectively.

Tripsacum dactyloides is a species of grass that is resistant to certain insects, including corn rootworm. It is anticipated that genes encoding proteins that are toxic to insects or are involved in the biosynthesis of compounds toxic to insects will be isolated from Tripsacum and that these novel genes will be useful in conferring resistance to insects. It is known that the basis of insect resistance in Tripsacum is genetic, because said resistance has been transferred to Zea mays via sexual crosses (Branson & Guss, 1972).

Further genes encoding proteins characterized as having potential insecticidal activity may also be used as transgenes in accordance herewith. Such genes include, for example, the cowpea trypsin inhibitor (CpTI; Hilder 1987), which may be used as a rootworm deterrent; genes encoding avermectin (Campbell 1989; Ikeda 1987) which may prove particularly useful as a corn rootworm deterrent; ribosome inactivating protein genes; and even genes that regulate plant structures. Transgenic maize including anti-insect antibody genes and genes that code for enzymes that can covert a non-toxic insecticide (pro-insecticide) applied to the outside of the plant into an insecticide inside the plant are also contemplated.

1.3 Environment or Stress Resistance

Improvement of a plant's ability to tolerate various environmental stresses such as, but not limited to, drought, excess moisture, chilling, freezing, high temperature, salt, and oxidative stress, can also be effected through expression of heterologous, or overexpression of homologous genes. Benefits may be realized in terms of increased resistance to freezing temperatures through the introduction of an "antifreeze" protein such as that of the Winter Flounder (Cutler 1989) or synthetic gene derivatives thereof. Improved chilling tolerance may also be conferred through increased expression of glycerol-3-phosphate acetyltransferase in chloroplasts (Murata 1992; Wolter 1992). Resistance to oxidative stress (often exacerbated by conditions such as chilling temperatures in combination with high light intensities) can be conferred by expression of superoxide dismutase (Gupta 1993), and may be improved by glutathione reductase (Bowler 1992). Such strategies may allow for tolerance to freezing in newly emerged fields as well as extending later maturity higher yielding varieties to earlier relative maturity zones.

Expression of novel genes that favorably effect plant water content, total water potential, osmotic potential, and turgor can enhance the ability of the plant to tolerate drought. As used herein, the terms "drought resistance" and "drought tolerance" are used to refer to a plants increased resistance or tolerance to stress induced by a reduction in water availability, as compared to normal circumstances, and the ability of the plant to function and survive in lower-water environments, and perform in a relatively superior manner. In this aspect of the invention it is proposed, for example, that the expression of a gene encoding the biosynthesis of osmotically active solutes can impart protection against drought. Within this class of genes are DNAs encoding mannitol dehydrogenase (Lee and Saier, 1982) and trehalose-6-phosphate synthase (Kaasen 1992). Through the subsequent action of native phosphatases in the cell or by the introduction and coexpression of a specific phosphatase, these introduced genes will result in the accumulation of either mannitol or trehalose, respectively, both of which have been well documented as protective compounds able to mitigate the effects of stress. Mannitol accumulation in transgenic tobacco has been verified and preliminary results indicate that plants expressing high levels of this metabolite are able to tolerate an applied osmotic stress (Tarczynski 1992).

Similarly, the efficacy of other metabolites in protecting either enzyme function (e.g. alanopine or propionic acid) or membrane integrity (e.g., alanopine) has been documented (Loomis 1989), and therefore expression of gene encoding the biosynthesis of these compounds can confer drought resistance in a manner similar to or complimentary to mannitol. Other examples of naturally occurring metabolites that are osmotically active and/or provide some direct protective effect during drought and/or desiccation include sugars and sugar derivatives such as fructose, erythritol (Coxson 1992), sorbitol, dulcitol (Karsten 1992), glucosylglycerol (Reed 1984; Erdmann 1992), sucrose, stachyose (Koster & Leopold 1988; Blackman 1992), ononitol and pinitol (Vernon & Bohnert 1992), and raffinose (Bernal-Lugo & Leopold 1992). Other osmotically active solutes, which are not sugars, include, but are not limited to, proline and glycine-betaine (Wyn-Jones and Storey, 1981). Continued canopy growth and increased reproductive fitness during times of stress can be augmented by introduction and expression of genes such as those controlling the osmotically active compounds discussed above and other such compounds, as represented in one exemplary embodiment by the enzyme myoinositol 0-methyltransferase.

It is contemplated that the expression of specific proteins may also increase drought tolerance. Three classes of Late Embryogenic Proteins have been assigned based on structural similarities (see Dure 1989). All three classes of these proteins have been demonstrated in maturing (i.e., desiccating) seeds. Within these 3 types of proteins, the Type-II (dehydrin-type) have generally been implicated in drought and/or desiccation tolerance in vegetative plant parts (e.g.. Mundy and Chua, 1988; Piatkowski 1990; Yamaguchi-Shinozaki 1992). Recently, expression of a Type-III LEA (HVA-1) in tobacco was found to influence plant height, maturity and drought tolerance (Fitzpatrick, 1993). Expression of structural genes from all three groups may therefore confer drought tolerance. Other types of proteins induced during water stress include thiol proteases, aldolases and transmembrane transporters (Guerrero 1990), which may confer various protective and/or repair-type functions during drought stress. The expression of a gene that effects lipid biosynthesis and hence membrane composition can also be useful in conferring drought resistance on the plant.

Many genes that improve drought resistance have complementary modes of action. Thus, combinations of these genes might have additive and/or synergistic effects in improving drought resistance in maize. Many of these genes also improve freezing tolerance (or resistance); the physical stresses incurred during freezing and drought are similar in nature and may be mitigated in similar fashion. Benefit may be conferred via constitutive expression or tissue-specific of these genes, but the preferred means of expressing these novel genes may be through the use of a turgor-induced promoter (such as the promoters for the turgor-induced genes described in Guerrero et al. 1990 and Shagan 1993). Spatial and temporal expression patterns of these genes may enable maize to better withstand stress.

Expression of genes that are involved with specific morphological traits that allow for increased water extractions from drying soil would be of benefit. For example, introduction and expression of genes that alter root characteristics may enhance water uptake. Expression of genes that enhance reproductive fitness during times of stress would be of significant value. For example, expression of DNAs that improve the synchrony of pollen shed and receptiveness of the female flower parts, i.e., silks, would be of benefit. In addition, expression of genes that minimize kernel abortion during times of stress would increase the amount of grain to be harvested and hence be of value. Regulation of cytokinin levels in monocots, such as maize, by introduction and expression of an isopentenyl transferase gene with appropriate regulatory sequences can improve monocot stress resistance and yield (Gan 1995).

Given the overall role of water in determining yield, it is contemplated that enabling plants to utilize water more efficiently, through the introduction and expression of novel genes, will improve overall performance even when soil water availability is not limiting. By introducing genes that improve the ability of plants to maximize water usage across a full range of stresses relating to water availability, yield stability or consistency of yield performance may be realized.

Improved protection of the plant to abiotic stress factors such as drought, heat or chill, can also be achieved - for example - by overexpressing antifreeze polypeptides from Myoxocephalus Scorpius (WO 00/00512), Myoxocephalus octodecemspinosus, the Arabidopsis thaliana transcription activator CBF1, glutamate dehydrogenases (WO 97/12983, WO 98/11240), calcium-dependent protein kinase genes (WO 98/26045), calcineurins (WO 99/05902), casein kinase from yeast (WO 02/052012), farnesyltransferases (WO 99/06580; Pei ZM et al. (1998) Science 282:287-290), ferritin (Deak M et al. (1999) Nature Biotechnology 17:192-196), oxalate oxidase (WO 99/04013; Dunwell JM (1998) Biotechn Genet Eng Rev 15:1-32), DREB1A factor ("dehydration response element B 1A"; Kasuga M et al. (1999) Nature Biotech 17:276-286), genes of mannitol or trehalose synthesis such as trehalose-phosphate synthase or trehalose-phosphate phosphatase (WO 97/42326) or by inhibiting genes such as trehalase (WO 97/50561).

1.4 Disease Resistance

It is proposed that increased resistance to diseases may be realized through introduction of genes into plants period. It is possible to produce resistance to diseases caused, by viruses, bacteria, fungi, root pathogens, insects and nematodes. It is also contemplated that control of mycotoxin producing organisms may be realized through expression of introduced genes.

Resistance to viruses may be produced through expression of novel genes. For example, it has been demonstrated that expression of a viral coat protein in a transgenic plant can impart resistance to infection of the plant by that virus and perhaps other closely related viruses (Cuozzo 1988, Hemenway 1988, Abel 1986). It is contemplated that expression of antisense genes targeted at essential viral functions may impart resistance to said virus. For example, an antisense gene targeted at the gene responsible for replication of viral nucleic acid may inhibit said replication and lead to resistance to the virus. It is believed that interference with other viral functions through the use of antisense genes may also increase resistance to viruses. Further it is proposed that it may be possible to achieve resistance to viruses through other approaches, including, but not limited to the use of satellite viruses.

It is proposed that increased resistance to diseases caused by bacteria and fungi may be realized through introduction of novel genes. It is contemplated that genes encoding so-called "peptide antibiotics," pathogenesis related (PR) proteins, toxin resistance, and proteins affecting host-pathogen interactions such as morphological characteristics will be useful. Peptide antibiotics are polypeptide sequences, which are inhibitory to growth of bacteria and other microorganisms. For example, the classes of peptides referred to as cecropins and magainins inhibit growth of many species of bacteria and fungi. It is proposed that expression of PR proteins in plants may be useful in conferring resistance to bacterial disease. These genes are induced following pathogen attack on a host plant and have been divided into at least five classes of proteins (Bol 1990). Included amongst the PR proteins are beta-1,3-glucanases, chitinases, and osmotin and other proteins that are believed to function in plant resistance to disease organisms. Other genes have been identified that have antifungal properties, e.g., UDA (stinging nettle lectin) and hevein (Broakgert 1989; Barkai-Golan 1978). It is known that certain plant diseases are caused by the production of phytotoxins. Resistance to these diseases could be achieved through expression of a novel gene that encodes an enzyme capable of degrading or otherwise inactivating the phytotoxin. Expression novel genes that alter the interactions between the host plant and pathogen may be useful in reducing the ability the disease organism to invade the tissues of the host plant, e.g., an increase in the waxiness of the leaf cuticle or other morphological characteristics.

Plant parasitic nematodes are a cause of disease in many plants. It is proposed that it would be possible to make the plant resistant to these organisms through the expression of novel genes. It is anticipated that control of nematode infestations would be accomplished by altering the ability of the nematode to recognize or attach to a host plant and/or enabling the plant to produce nematicidal compounds, including but not limited to proteins.

Furthermore, a resistance to fungi, insects, nematodes and diseases, can be achieved by by targeted accumulation of certain metabolites or proteins. Such proteins include but are not limited to glucosinolates (defense against herbivores), chitinases or glucanases and other enzymes which destroy the cell wall of parasites, ribosome-inactivating proteins (RIPs) and other proteins of the plant resistance and stress reaction as are induced when plants are wounded or attacked by microbes, or chemically, by, for example, salicylic acid, jasmonic acid or ethylene, or lysozymes from nonplant sources such as, for example, T4-lysozyme or lysozyme from a variety of mammals, insecticidal proteins such as *Bacillus thuringiensis* endotoxin, a-amylase inhibitor or protease inhibitors (cowpea trypsin inhibitor), lectins such as wheatgerm agglutinin, RNAses or ribozymes. Further examples are nucleic acids which encode the Trichoderma harzianum chit42 endochitinase (GenBank Acc. No.: S78423) or the N-hydroxylating, multi-functional cytochrome P-450 (CYP79) protein from Sorghum bicolor (GenBank Acc. No.: U32624), or functional equivalents of these. The accumulation of glucosinolates as protection from pests (Rask L et al. (2000) Plant Mol Biol 42:93-113; Menard R et al. (1999) Phytochemistry 52:29-35), the expression of *Bacillus thuringiensis* endotoxins (Vaeck et al. (1987) Nature 328:33-37) or the protection against attack by fungi, by expression of chitinases, for example from beans (Broglie et al. (1991) Science 254:1194-1197), is advantageous. Resistance to pests such as, for example, the rice pest *Nilaparvata lugens* in rice plants can be achieved by expressing the snowdrop (*Galanthus nivalis*) lectin agglutinin (Rao et al. (1998) Plant J 15(4):469-77).The expression of synthetic cryIA(b) and cryIA(c) genes, which encode lepidoptera-specific *Bacillus thuringiensis* D-endotoxins can bring about a resistance to insect pests in various plants (Goyal RK et al. (2000) Crop Protection 19(5):307-312). Further target genes which are suitable for pathogen defense comprise "polygalacturonase-inhibiting protein" (PGIP), thaumatine, invertase and antimicrobial peptides such as lactoferrin (Lee TJ et al. (2002) J Amer Soc Horticult Sci 127(2):158-164). Other nucleic acid sequences which may be advantageously used herein include traits for insect control (U.S. Pat. Nos. 6,063,597; 6,063,756; 6,093,695; 5,942,664; and 6,110,464), fungal disease resistance (U.S. Pat. Nos. 5,516,671; 5,773,696; 6,121,436; 6,316,407; and 6,506,962), virus resistance (U.S. Pat. Nos. 5,304,730 and 6,013,864), nematode resistance (U.S. Pat. No. 6,228,992), and bacterial disease resistance (U.S. Pat. No. 5,516,671).

1.5 Mycotoxin Reduction/Elimination

Production of mycotoxins, including aflatoxin and fumonisin, by fungi associated with plants is a significant factor in rendering the grain not useful. These fungal organisms do not cause disease symptoms and/or interfere with the growth of the plant, but they produce chemicals (mycotoxins) that are toxic to animals. Inhibition of the growth of these fungi would reduce the synthesis of these toxic substances and, therefore, reduce grain losses due to mycotoxin contamination. Novel genes may be introduced into plants that would inhibit synthesis of the mycotoxin without interfering with fungal growth. Expression of a novel gene, which encodes an enzyme capable of rendering the mycotoxin nontoxic, would be useful in order to achieve reduced mycotoxin contamination of grain. The result of any of the above mechanisms would be a reduced presence of mycotoxins on grain.

1.6 Grain Composition or Quality

Genes may be introduced into plants, particularly commercially important cereals such as maize, wheat or rice, to improve the grain for which the cereal is primarily grown. A wide range of novel transgenic plants produced in this manner may be envisioned depending on the particular end use of the grain.

For example, the largest use of maize grain is for feed or food. Introduction of genes that alter the composition of the grain may greatly enhance the feed or food value. The primary components of maize grain are starch, protein, and oil. Each of these primary components of maize grain may be improved by altering its level or composition. Several examples may be mentioned for illustrative purposes but in no way provide an exhaustive list of possibilities.

The protein of many cereal grains is suboptimal for feed and food purposes especially when fed to pigs, poultry, and humans. The protein is deficient in several amino acids that are essential in the diet of these species, requiring the addition of supplements to the grain. Limiting essential amino acids may include lysine, methionine, tryptophan, threonine, valine, arginine, and histidine. Some amino acids become limiting only after the grain is supplemented with other inputs for feed formulations. For example, when the grain is supplemented with soybean meal to meet lysine requirements, methionine becomes limiting. The levels of these essential amino acids in seeds and grain may be elevated by mechanisms which include, but are not limited to, the introduction of genes to increase the biosynthesis of the amino acids, decrease the degradation of the amino acids, increase the storage of the amino acids in proteins, or increase transport of the amino acids to the seeds or grain.

One mechanism for increasing the biosynthesis of the amino acids is to introduce genes that deregulate the amino acid biosynthetic pathways such that the plant can no longer adequately control the levels that are produced. This may be done by deregulating or bypassing steps in the amino acid biosynthetic pathway that are normally regulated by levels of the amino acid end product of the pathway. Examples include the introduction of genes that encode deregulated versions of the enzymes aspartokinase or dihydrodipicolinic acid (DHDP)-synthase for increasing lysine and threonine production, and anthranilate synthase for increasing tryptophan production. Reduction of the catabolism of the amino acids may be accomplished by introduction of DNA sequences that reduce or eliminate the expression of genes encoding enzymes that catalyse steps in the catabolic pathways such as the enzyme lysine-ketoglutarate reductase.

The protein composition of the grain may be altered to improve the balance of amino acids in a variety of ways including elevating expression of native proteins, decreasing expression of those with poor composition, changing the composition of native proteins, or introducing genes encoding entirely new proteins possessing superior composition. DNA may be introduced that decreases the expression of members of the zein family of storage proteins. This DNA may encode ribozymes or antisense sequences directed to impairing expression of zein proteins or expression of regulators of zein expression such as the opaque-2 gene product. The protein composition of the grain may be modified through the phenomenon of cosuppression, i.e., inhibition of expression of an endogenous gene through the expression of an identical structural gene or gene fragment introduced through transformation (Goring 1991). Additionally, the introduced DNA may encode enzymes, which degrade zeines. The decreases in zein expression that are achieved may be accompanied by increases in proteins with more desirable amino acid composition or increases in other major seed constituents such as starch. Alternatively, a chimeric gene may be introduced that comprises a coding sequence for a native protein of adequate amino acid composition such as for one of the globulin proteins or 10 kD zein of maize and a promoter or other regulatory sequence designed to elevate expression of said protein. The coding sequence of said gene may include additional or replacement codons for essential amino acids. Further, a coding sequence obtained from another species, or, a partially or completely synthetic sequence encoding a completely unique peptide sequence designed to enhance the amino acid composition of the seed may be employed.

The introduction of genes that alter the oil content of the grain may be of value. Increases in oil content may result in increases in metabolizable energy content and density of the seeds for uses in feed and food. The introduced genes may encode enzymes that remove or reduce rate-limitations or regulated steps in fatty acid or lipid biosynthesis. Such genes may include, but are not limited to, those that encode acetyl-CoA carboxylase, ACP-acyltransferase, beta-ketoacyl-ACP synthase, plus other well-known fatty acid biosynthetic activities. Other possibilities are genes that encode proteins that do not possess enzymatic activity such as acyl carrier protein. Additional examples include 2-acetyltransferase, oleosin pyruvate dehydrogenase complex, acetyl CoA synthetase, ATP citrate lyase, ADP-glucose pyrophosphorylase and genes of the carnitine-CoA-acetyl-CoA shuttles. It is anticipated that expression of genes related to oil biosynthesis will be targeted to the plastid, using a plastid transit peptide sequence and preferably expressed in the seed embryo. Genes may be introduced that alter the balance of fatty acids present in the oil providing a more healthful or nutritive feedstuff. The introduced DNA may also encode sequences that block expression of enzymes involved in fatty acid biosynthesis, altering the proportions of fatty acids present in the grain such as described below. Genes may be introduced that enhance the nutritive value of the starch component of the grain, for example by increasing the degree of branching, resulting in improved utilization of the starch in cows by delaying its metabolism.

Besides affecting the major constituents of the grain, genes may be introduced that affect a variety of other nutritive, processing, or other quality aspects of the grain as used for feed or food. For example, pigmentation of the grain may be increased or decreased. Enhancement and stability of yellow pigmentation is desirable in some animal feeds and may be achieved by introduction of genes that result in enhanced production of xanthophylls and carotenes by eliminating rate-limiting steps in their production. Such genes may encode altered forms of the enzymes phytoene synthase, phytoene desaturase, or lycopene synthase. Alternatively, unpigmented white corn is desirable for production of many food products and may be produced by the introduction of DNA, which blocks or eliminates steps in pigment production pathways.

Feed or food comprising some cereal grains possesses insufficient quantities of vitamins and must be supplemented to provide adequate nutritive value. Introduction of genes that enhance vitamin biosynthesis in seeds may be envisioned including, for example, vitamins A, E, $B_{12}$, choline, and the like. For example, maize grain also does not possess sufficient mineral content for optimal nutritive value. Genes that affect the accumulation or availability of compounds containing phosphorus, sulfur, calcium, manganese, zinc, and iron among others would be valuable. An example may be the introduction of a gene that reduced phytic acid production or encoded the enzyme phytase, which enhances phytic acid breakdown. These genes would increase levels of available phosphate in the diet, reducing the need for supplementation with mineral phosphate.

Numerous other examples of improvement of cereals for feed and food purposes might be described. The improvements may not even necessarily involve the grain, but may, for example, improve the value of the grain for silage. Introduction of DNA to accomplish this might include sequences that alter lignin production such as those that result in the "brown midrib" phenotype associated with superior feed value for cattle.

In addition to direct improvements in feed or food value, genes may also be introduced which improve the processing of grain and improve the value of the products resulting from the processing. The primary method of processing certain grains such as maize is via wetmilling. Maize may be improved though the expression of novel genes that increase the efficiency and reduce the cost of processing such as by decreasing steeping time.

Improving the value of wetmilling products may include altering the quantity or quality of starch, oil, corn gluten meal, or the components of corn gluten feed. Elevation of starch may be achieved through the identification and elimination of rate limiting steps in starch biosynthesis or by decreasing levels of the other components of the grain resulting in proportional increases in starch. An example of the former may be the introduction of genes encoding ADP-glucose pyrophosphorylase enzymes with altered regulatory activity or which are expressed at higher level. Examples of the latter may include selective inhibitors of, for example, protein or oil biosynthesis expressed during later stages of kernel development.

The properties of starch may be beneficially altered by changing the ratio of amylose to amylopectin, the size of the starch molecules, or their branching pattern. Through these changes a broad range of properties may be modified which include, but are not limited to, changes in gelatinization temperature, heat of gelatinization, clarity of films and pastes, Theological properties, and the like. To accomplish these changes in properties, genes that encode granule-bound or soluble starch synthase activity or branching enzyme activity may be introduced alone or combination. DNA such as antisense constructs may also be used to decrease levels of endogenous activity of these enzymes. The introduced genes or constructs may possess regulatory sequences that time their expression to specific intervals in starch biosynthesis and starch granule development. Furthermore, it may be advisable to introduce and express genes that result in the in vivo derivatization, or other modification, of the glucose moieties of the starch molecule. The covalent attachment of any molecule may be envisioned, limited only by the existence of enzymes that catalyze the derivatizations and the accessibility of appropriate substrates in the starch granule. Examples of important derivations may include the addition of functional groups such as amines, carboxyls, or phosphate groups, which provide sites for subsequent in vitro derivatizations or affect starch properties through the introduction of ionic charges. Examples of other modifications may include direct changes of the glucose units such as loss of hydroxyl groups or their oxidation to aldehyde or carboxyl groups.

Oil is another product of wetmilling of corn and other grains, the value of which may be improved by introduction and expression of genes. The quantity of oil that can be extracted by wetmilling may be elevated by approaches as described for feed and food above. Oil properties may also be altered to improve its performance in the production and use of cooking oil, shortenings, lubricants or other oil-derived products or improvement of its health attributes when used in the food-related applications. Novel fatty acids may also be synthesized which upon extraction can serve as starting materials for chemical syntheses. The changes in oil properties may be achieved by altering the type, level, or lipid arrangement of the fatty acids present in the oil. This in turn may be accomplished by the addition of genes that encode enzymes that catalyze the synthesis of novel fatty acids and the lipids possessing them or by increasing levels of native fatty acids while possibly reducing levels of precursors. Alternatively DNA sequences may be introduced which slow or block steps in fatty acid biosynthesis resulting in the increase in precursor fatty acid intermediates. Genes that might be added include desaturases, epoxidases, hydratases, dehydratases, and other enzymes that catalyze reactions involving fatty acid intermediates. Representative examples of catalytic steps that might be blocked include the desaturations from stearic to oleic acid and oleic to linolenic acid resulting in the respective accumulations of stearic and oleic acids.

Improvements in the other major cereal wetmilling products, gluten meal and gluten feed, may also be achieved by the introduction of genes to obtain novel plants. Representative possibilities include but are not limited to those described above for improvement of food and feed value.

In addition it may further be considered that the plant be used for the production or manufacturing of useful biological compounds that were either not produced at all, or not produced at the same level, in the plant previously. The novel plants producing these compounds are made possible by the introduction and expression of genes by transformation methods. The possibilities include, but are not limited to, any biological compound which is presently produced by any organism such as proteins, nucleic acids, primary and intermediary metabolites, carbohydrate polymers, etc. The compounds may be produced by the plant, extracted upon harvest and/or processing, and used for any presently recognized useful purpose such as pharmaceuticals, fragrances, industrial enzymes to name a few.

Further possibilities to exemplify the range of grain traits or properties potentially encoded by introduced genes in transgenic plants include grain with less breakage susceptibility for export purposes or larger grit size when processed by dry milling through introduction of genes that enhance gamma-zein synthesis, popcorn with improved popping, quality and expansion volume through genes that increase pericarp thickness, corn with whiter grain for food uses though introduction of genes that effectively block expression of enzymes involved in pigment production pathways, and improved quality of alcoholic beverages or sweet corn through introduction of genes which affect flavor such as the shrunken gene (encoding sucrose synthase) for sweet corn.

1.7 Tuber or Seed Composition or Quality

Various traits can be advantageously expressed especially in seeds or tubers to improve composition or quality. Useful nucleic acid sequences that can be combined with the promoter nucleic acid sequence of the present invention and provide improved end-product traits include, without limitation, those encoding seed storage proteins, fatty acid pathway enzymes, tocopherol biosynthetic enzymes, amino acid biosynthetic enzymes, and starch branching enzymes. A discussion of exemplary heterologous DNAs useful for the modification of plant phenotypes may be found in, for example, U.S. Pat. Nos. 6,194,636; 6,207,879; 6,232,526; 6,426,446; 6,429,357; 6,433,252; 6,437,217; 6,515,201; and 6,583,338 and PCT Publication WO 02/057471, each of which is specifically incorporated herein by reference in its entirety. Such traits include but are not limited to:

- Expression of metabolic enzymes for use in the food-and-feed sector, for example of phytases and cellulases. Especially preferred are nucleic acids such as the artificial cDNA, which encodes a microbial phytase (GenBank Acc. No.: A19451) or functional equivalents thereof.
- Expression of genes, which bring about an accumulation of fine chemicals such as of tocopherols, tocotrienols or carotenoids. An example, which may be mentioned is phytoene desaturase. Preferred are nucleic acids, which encode the Narcissus pseudonarcissus photoene desaturase (GenBank Acc. No.: X78815) or functional equivalents thereof. Preferred tocopherol biosynthetic enzymes include tyrA, slr1736, ATPT2, dxs, dxr, GGPPS, HPPD, GMT, MT1, tMT2, AANT1, slr 1737, and an antisense construct for homogentisic acid dioxygenase (Kridl et al., Seed Sci. Res., 1:209:219 (1991); Keegstra, Cell, 56(2):247-53 (1989); Nawrath et al., Proc. Natl. Acad. Sci. USA, 91:12760-12764 (1994); Xia et al., J. Gen. Microbiol., 138:1309-1316 (1992); Lois et al., Proc. Natl. Acad. Sci. USA, 95 (5):2105-2110 (1998); Takahashi et al., Proc. Natl. Acad. Sci. USA, 95(17):9879-9884 (1998); Norris et al., Plant Physiol., 117:1317-1323 (1998); Bartley and Scolnik, Plant Physiol., 104:1469-1470 (1994); Smith et al., Plant J., 11:83-92 (1997); WO 00/32757; WO 00/10380; Saint Guily et al., Plant Physiol., 100(2):1069-1071 (1992); Sato et al., J. DNA Res., 7(1):31-63 (2000)) all of which are incorporated herein by reference.
- starch production (U.S. Pat. Nos. 5,750,876 and 6,476,295), high protein production (U.S. Pat. No. 6,380,466), fruit ripening (U.S. Pat. No. 5,512,466), enhanced animal and human nutrition (U.S. Pat. Nos. 5,985,605 and 6,171,640), biopolymers (U.S. Pat. No. 5,958,745 and U.S. Patent Publication No. 2003/0028917), environmental stress resistance (U.S. Pat. No. 6,072,103), pharmaceutical peptides (U.S. Pat. No. 6,080,560), improved processing traits (U.S. Pat. No. 6,476,295), improved digestibility (U.S. Pat. No. 6,531,648), low raffinose (U.S. Pat. No. 6,166,292), industrial enzyme production (U.S. Pat. No. 5,543,576), improved flavor (U.S. Pat. No. 6,011,199), nitrogen fixation (U.S. Pat. No. 5,229,114), hybrid seed production (U.S. Pat. No. 5,689,041), and biofuel production (U.S. Pat. No. 5,998,700), the genetic elements and transgenes described in the patents listed above are herein incorporated by reference. Preferred starch branching enzymes (for modification of starch properties) include those set forth in U.S. Pat. Nos. 6,232,122 and 6,147,279; and PCT Publication WO 97/22703, all of which are incorporated herein by reference.
- Modified oils production (U.S. Pat. No. 6,444,876), high oil production (U.S. Pat. Nos. 5,608,149 and 6,476,295), or modified fatty acid content (U.S. Pat. No. 6,537,750). Preferred fatty acid pathway enzymes include thioesterases (U.S. Pat. Nos. 5,512,482; 5,530,186; 5,945,585; 5,639,790; 5,807,893; 5,955,650; 5,955,329; 5,759,829; 5,147,792; 5,304,481; 5,298,421; 5,344,771; and 5,760,206), diacylglycerol acyltransferases (U.S. Patent Publications 20030115632A1, 2, 3, 4, 5, 6, 7, 8, and 90030028923A1), and desaturases (U.S. Pat. Nos. 5,689,050; 5,663,068; 5,614,393; 5,856,157; 6,117,677; 6,043,411; 6,194,167; 5,705,391; 5,663,068; 5,552,306; 6,075,183; 6,051,754; 5,689,050; 5,789,220; 5,057,419; 5,654,402; 5,659,645; 6,100,091; 5,760,206; 6,172,106; 5,952,544; 5,866,789; 5,443,974; and 5,093,249) all of which are incorporated herein by reference.

- Preferred amino acid biosynthetic enzymes include anthranilate synthase (U.S. Pat. No. 5,965,727 and PCT Publications WO 97/26366, WO 99/11800, WO 99/49058), tryptophan decarboxylase (PCT Publication WO 99/06581), threonine decarboxylase (U.S. Pat. Nos. 5,534,421 and 5,942,660; PCT Publication WO 95/19442), threonine deaminase (PCT Publications WO 99/02656 and WO 98/55601), dihydrodipicolinic acid synthase (U.S. Pat. No. 5,258,300), and aspartate kinase (U.S. Pat. Nos. 5,367,110; 5,858,749; and 6,040,160) all of which are incorporated herein by reference.
- Production of nutraceuticals such as, for example, polyunsaturated fatty acids (for example arachidonic acid, eicosapentaenoic acid or docosahexaenoic acid) by expression of fatty acid elongases and/or desaturases, or production of proteins with improved nutritional value such as, for example, with a high content of essential amino acids (for example the high-methionine 2S albumin gene of the brazil nut). Preferred are nucleic acids which encode the Bertholletia excelsa high-methionine 2S albumin (GenBank Acc. No.: AB044391), the Physcomitrella patens Delta-6-acyl-lipid desaturase (GenBank Acc. No.: AJ222980; Girke et al. (1998) Plant J 15:39-48), the Mortierella alpina Delta-6-desaturase (Sakuradani et al. 1999 Gene 238:445-453), the Caenorhabditis elegans Delta-5-desaturase (Michaelson et al. 1998, FEBS Letters 439:215-218), the Caenorhabditis elegans Delta-5-fatty acid desaturase (des-5) (GenBank Acc. No.: AF078796), the Mortierella alpina Delta-5-desaturase (Michaelson et al. JBC 273:19055-19059), the Caenorhabditis elegans Delta-6-elongase (Beaudoin et al. 2000, PNAS 97:6421-6426), the Physcomitrella patens Delta-6-elongase (Zank et al. 2000, Biochemical Society Transactions 28:654-657), or functional equivalents of these.
- Production of high-quality proteins and enzymes for industrial purposes (for example enzymes, such as lipases) or as pharmaceuticals (such as, for example, antibodies, blood clotting factors, interferons, lymphokins, colony stimulation factor, plasminogen activators, hormones or vaccines, as described by Hood EE, Jilka JM (1999) Curr Opin Biotechnol 10(4): 382-6; Ma JK, Vine ND (1999) Curr Top Microbiol Immunol 236:275-92). For example, it has been possible to produce recombinant avidin from chicken albumen and bacterial beta-glucuronidase (GUS) on a large scale in transgenic maize plants (Hood et al. (1999) Adv Exp Med Biol 464:127-47. Review).
- Obtaining an increased storability in cells which normally comprise fewer storage proteins or storage lipids, with the purpose of increasing the yield of these substances, for example by expression of acetyl-CoA carboxylase. Preferred nucleic acids are those, which encode the Medicago sativa acetyl-CoA carboxylase (ACCase) (GenBank Acc. No.: L25042), or functional equivalents thereof. Alterenatively, in some scenarios an increased storage protein content might be advantageous for high-protewin product production. Preferred seed storage proteins include zeins (U.S. Pat. Nos. 4,886,878; 4,885,357; 5,215,912; 5,589,616; 5,508,468; 5,939,599; 5,633,436; and 5,990,384; PCT Publications WO 90/01869, WO 91/13993, WO 92/14822, WO 93/08682, WO 94/20628, WO 97/28247, WO 98/26064, and WO 99/40209), 7S proteins (U.S. Pat. Nos. 5,003,045 and 5,576,203), brazil nut protein (U.S. Pat. No. 5,850,024), phenylalanine free proteins (PCT Publication WO 96/17064), albumin (PCT Publication WO 97/35023), b-conglycinin (PCT Publication WO 00/19839), 11S (U.S. Pat. No. 6,107,051), alpha-hordothionin (U.S. Pat. Nos. 5,885,802 and 5,88,5801), arcelin seed storage proteins (U.S. Pat. No. 5,270,200), lectins (U.S. Pat. No. 6,110,891), and glutenin (U.S. Pat. Nos. 5,990,389 and 5,914,450) all of which are incorporated herein by reference.
- Reducing levels of alpha-glucan L-type tuber phosphorylase (GLTP) or alpha-glucan H-type tuber phosphorylase (GHTP) enzyme activity preferably within the potato tuber (see US 5,998,701). The conversion of starches to sugars in potato tubers, particularly when stored at temperatures below 7°C., is reduced in tubers exhibiting reduced GLTP or GHTP enzyme activity. Reducing cold-sweetening in potatoes allows for potato storage at cooler temperatures, resulting in prolonged dormancy, reduced incidence of disease, and increased storage life. Reduction of GLTP or GHTP activity within the potato tuber may be accomplished by such techniques as suppression of gene expression using homologous antisense or double-stranded RNA, the use of co-suppression, regulatory silencing sequences. A potato plant having improved cold-storage characteristics, comprising a potato plant transformed with an expression cassette having a TPT promoter sequence operably linked to a DNA sequence comprising at least 20 nucleotides of a gene encoding an alpha-glucan phosphorylase selected from the group consisting of alpha-glucan L-type tuber phosphorylase (GLTP) and alpha-glucan H-type phosphorylase (GHTP).

Further examples of advantageous genes are mentioned for example in Dunwell JM, Transgenic approaches to crop improvement, J Exp Bot. 2000; 51 Spec No; pages 487-96.

1.8 Plant Agronomic Characteristics

Two of the factors determining where plants can be grown are the average daily temperature during the growing season and the length of time between frosts. Within the areas where it is possible to grow a particular plant, there are varying limitations on the maximal time it is allowed to grow to maturity and be harvested. The plant to be grown in a particular area is selected for its ability to mature and dry down to harvestable moisture content within the required period of time with maximum possible yield. Therefore, plants of varying maturities are developed for different growing locations. Apart from the need to dry down sufficiently to permit harvest is the desirability of having maximal drying take place in the field to minimize the amount of energy required for additional drying post-harvest. Also the more readily the grain can dry down, the more time there is available for growth and kernel fill. Genes that influence maturity and/or dry down can be identified and introduced into plant lines using transformation techniques to create new varieties adapted to different growing locations or the same growing location but having improved yield to moisture ratio at harvest. Expression of genes that are involved in regulation of plant development may be especially useful, e.g., the liguleless and rough sheath genes that have been identified in plants.

Genes may be introduced into plants that would improve standability and other plant growth characteristics. For example, expression of novel genes, which confer stronger stalks, improved root systems, or prevent or reduce ear droppage would be of great value to the corn farmer. Introduction and expression of genes that increase the total amount of photoassimilate available by, for example, increasing light distribution and/or interception would be advantageous. In addition the expression of genes that increase the efficiency of photosynthesis and/or the leaf canopy would further increase gains in productivity. Such approaches would allow for increased plant populations in the field.

Delay of late season vegetative senescence would increase the flow of assimilates into the grain and thus increase yield. Overexpression of genes within plants that are associated with "stay green" or the expression of any gene that delays senescence would be advantageous. For example, a non-yellowing mutant has been identified in *Festuca pratensis* (Davies 1990). Expression of this gene as well as others may prevent premature breakdown of chlorophyll and thus maintain canopy function.

1.9 Nutrient Utilization

The ability to utilize available nutrients and minerals may be a limiting factor in growth of many plants. It is proposed that it would be possible to alter nutrient uptake, tolerate pH extremes, mobilization through the plant, storage pools, and availability for metabolic activities by the introduction of novel genes. These modifications would allow a plant to more efficiently utilize available nutrients. It is contemplated that an increase in the activity of, for example, an enzyme that is normally present in the plant and involved in nutrient utilization would increase the availability of a nutrient. An example of such an enzyme would be phytase. It is also contemplated that expression of a novel gene may make a nutrient source available that was previously not accessible, e.g., an enzyme that releases a component of nutrient value from a more complex molecule, perhaps a macromolecule.

1.10 Male Sterility

Male sterility is useful in the production of hybrid seed. It is proposed that male sterility may be produced through expression of novel genes. For example, it has been shown that expression of genes that encode proteins that interfere with development of the male inflorescence and/or gametophyte result in male sterility. Chimeric ribonuclease genes that express in the anthers of transgenic tobacco and oilseed rape have been demonstrated to lead to male sterility (Mariani 1990). For example, a number of mutations were discovered in maize that confer cytoplasmic male sterility. One mutation in particular, referred to as T cytoplasm, also correlates with sensitivity to Southern corn leaf blight. A DNA sequence, designated TURF-13 (Levings 1990), was identified that correlates with T cytoplasm. It would be possible through the introduction of TURF-13 via transformation to separate male sterility from disease sensitivity. As it is necessary to be able to restore male fertility for breeding purposes and for grain production, it is proposed that genes encoding restoration of male fertility may also be introduced.

1.11. Non-Protein-Expressing Sequences

1.11.1 RNA-Expressing

DNA may be introduced into plants for the purpose of expressing RNA transcripts that function to affect plant phenotype yet are not translated into protein. Two examples are antisense RNA and RNA with ribozyme activity. Both may serve possible functions in reducing or eliminating expression of native or introduced plant genes.

Genes may be constructed or isolated, which when transcribed, produce antisense RNA or double-stranded RNA that is complementary to all or part(s) of a targeted messenger RNA(s). The antisense RNA reduces production of the polypeptide product of the messenger RNA. The polypeptide product may be any protein encoded by the plant genome. The aforementioned genes will be referred to as antisense genes. An antisense gene may thus be introduced into a plant by transformation methods to produce a novel transgenic plant with reduced expression of a selected protein of interest. For example, the protein may be an enzyme that catalyzes a reaction in the plant. Reduction of the enzyme activity may reduce or eliminate products of the reaction which include any enzymatically synthesized compound in the plant such as fatty acids, amino acids, carbohydrates, nucleic acids and the like. Alternatively, the protein may be a storage protein, such as a zein, or a structural protein, the decreased expression of which may lead to changes in seed amino acid composition or plant morphological changes respectively. The possibilities cited above are provided only by way of example and do not represent the full range of applications.

Expression of antisense-RNA or double-stranded RNA by one of the expression cassettes of the invention is especially preferred. Also expression of sense RNA can be employed for gene silencing (co-suppression). This RNA is preferably a non-translatable RNA. Gene regulation by double-stranded RNA ("double-stranded RNA interference"; dsRNAi) is well known in the arte and described for various organism including plants (e.g., Matzke 2000; Fire A et al 1998; WO 99/32619; WO 99/53050; WO 00/68374; WO 00/44914; WO 00/44895; WO 00/49035; WO 00/63364).

Genes may also be constructed or isolated, which when transcribed produce RNA enzymes, or ribozymes, which can act as endoribonucleases and catalyze the cleavage of RNA molecules with selected sequences. The cleavage of selected messenger RNA's can result in the reduced production of their encoded polypeptide products. These genes may be used to prepare novel transgenic plants, which possess them. The transgenic plants may possess reduced levels of polypeptides including but not limited to the polypeptides cited above that may be affected by antisense RNA.

It is also possible that genes may be introduced to produce novel transgenic plants, which have reduced expression of a native gene product, by a mechanism of cosuppression. It has been demonstrated in tobacco, tomato, and petunia (Goring 1991; Smith 1990; Napoli 1990; van der Krol 1990) that expression of the sense transcript of a native gene will reduce or eliminate expression of the native gene in a manner similar to that observed for antisense genes. The introduced gene may encode all or part of the targeted native protein but its translation may not be required for reduction of levels of that native protein.

1.11.2 Non-RNA-Expressing

For example, DNA elements including those of transposable elements such as Ds, Ac, or Mu, may be, inserted into a gene and cause mutations. These DNA elements may be inserted in order to inactivate (or activate) a gene and thereby "tag" a particular trait. In this instance the transposable element does not cause instability of the tagged mutation, because the utility of the element does not depend on its ability to move in the genome. Once a desired trait is tagged, the introduced DNA sequence may be used to clone the corresponding gene, e.g., using the introduced DNA sequence as a PCR primer together with PCR gene cloning techniques (Shapiro, 1983; Dellaporta 1988). Once identified, the entire gene(s) for the particular trait, including control or regulatory regions where desired may be isolated, cloned and manipulated as desired. The utility of DNA elements introduced into an organism for purposed of gene tagging is independent of the DNA sequence and does not depend on any biological activity of the DNA sequence, i.e., transcription into RNA or translation into protein. The sole function of the DNA element is to disrupt the DNA sequence of a gene.

It is contemplated that unexpressed DNA sequences, including novel synthetic sequences could be introduced into cells as proprietary "labels" of those cells and plants and seeds thereof. It would not be necessary for a label DNA element to disrupt the function of a gene endogenous to the host organism, as the sole function of this DNA would be to identify the origin of the organism. For example, one could introduce a unique DNA sequence into a plant and this DNA element would identify all cells, plants, and progeny of these cells as having arisen from that labeled source. It is proposed that inclusion of label DNAs would enable one to distinguish proprietary germplasm or germplasm derived from such, from unlabelled germplasm.

Another possible element, which may be introduced, is a matrix attachment region element (MAR), such as the chicken lysozyme A element (Stief 1989), which can be positioned around an expressible gene of interest to effect an increase in overall expression of the gene and diminish position dependant effects upon incorporation into the plant genome (Stief 1989; Phi-Van 1990).

Further nucleotide sequences of interest that may be contemplated for use within the scope of the present invention in operable linkage with the promoter sequences according to the invention are isolated nucleic acid molecules, e.g., DNA or RNA, comprising a plant nucleotide sequence according to the invention comprising an open reading frame that is preferentially expressed in a specific tissue, i.e., seed-, root, green tissue (leaf and stem), panicle-, or pollen, or is expressed constitutively.

2. Marker Genes

In order to improve the ability to identify transformants, one may desire to employ a selectable or screenable marker gene as, or in addition to, the expressible gene of interest. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can 'select' for by chemical means, i.e., through the use of a selective agent (e.g., a herbicide, antibiotic, or the like), or whether it is simply a trait that one can identify through observation or testing, i.e., by 'screening' (e.g., the R-locus trait, the green fluorescent protein (GFP)). Of course, many examples of suitable marker genes are known to the art and can be employed in the practice of the invention.

Included within the terms selectable or screenable marker genes are also genes which encode a "secretable marker" whose secretion can be detected as a means of identifying or selecting for transformed cells. Examples include markers, which encode a secretable antigen that can be identified by antibody interaction, or even secretable enzymes, which can be detected by their catalytic activity. Secretable proteins fall into a number of classes, including small, diffusible proteins detectable, e.g., by ELISA; small active enzymes detectable in extracellular solution (e.g., alpha-amylase, beta-lactamase, phosphinothricin acetyltransferase); and proteins that are inserted or trapped in the cell wall (e.g., proteins that include a leader sequence such as that found in the expression unit of extensin or tobacco PR-S).

With regard to selectable secretable markers, the use of a gene that encodes a protein that becomes sequestered in the cell wall, and which protein includes a unique epitope is considered to be particularly advantageous. Such a secreted antigen marker would ideally employ an epitope sequence that would provide low background in plant tissue, a promoter-leader sequence that would impart efficient expression and targeting across the plasma membrane, and would produce protein that is bound in the cell wall and yet accessible to antibodies. A normally secreted wall protein modified to include a unique epitope would satisfy all such requirements.

One example of a protein suitable for modification in this manner is extensin, or hydroxyproline rich glycoprotein (HPRG). For example, the maize HPRG (Steifel 1990) molecule is well characterized in terms of molecular biology, expression and protein structure. However, any one of a variety of ultilane and/or glycine-rich wall proteins (Keller 1989) could be modified by the addition of an antigenic site to create a screenable marker.

One exemplary embodiment of a secretable screenable marker concerns the use of a maize sequence encoding the wall protein HPRG, modified to include a 15 residue epitope from the pro-region of murine interleukin, however, virtually any detectable epitope may be employed in such embodiments, as selected from the extremely wide variety of antigen-antibody combinations known to those of skill in the art. The unique extracellular epitope can then be straightforwardly detected using antibody labeling in conjunction with chromogenic or fluorescent adjuncts. Elements of the present disclosure may be exemplified in detail through the use of the bar and/or GUS genes, and also through the use of various other markers. Of course, in light of this disclosure, numerous other possible selectable and/or screenable marker genes will be apparent to those of skill in the art in addition to the one set forth herein below. Therefore, it will be understood that the following discussion is exemplary rather than exhaustive. In light of the techniques disclosed herein and the general recombinant techniques which are known in the art, the present invention renders possible the introduction of any gene, including marker genes, into a recipient cell to generate a transformed plant.

2.1 Selectable Markers

Various selectable markers are known in the art suitable for plant transformation. Such markers may include but are not limited to:

2.1.1 Negative selection markers

Negative selection markers confer a resistance to a biocidal compound such as a metabolic inhibitor (e.g., 2-deoxyglucose-6-phosphate, WO 98/45456), antibiotics (e.g., kanamycin, G 418, bleomycin or hygromycin) or herbicides (e.g., phosphinothricin or glyphosate). Transformed plant material (e.g., cells, tissues or plantlets), which express marker genes, are capable of developing in the presence of concentrations of a corresponding selection compound (e.g., antibiotic or herbicide), which suppresses growth of an untransformed wild type tissue. Especially preferred negative selection markers are those, which confer resistance to herbicides. Examples, which may be mentioned, are:

- Phosphinothricin acetyltransferases (PAT; also named Bialophos ®resistance; bar; de Block 1987; Vasil 1992, 1993; Weeks 1993; Becker 1994; Nehra 1994; Wan & Lemaux 1994; EP 0 333 033; US 4,975,374). Preferred are the bar gene from Streptomyces hygroscopicus or the pat gene from Streptomyces viridochromogenes. PAT inactivates the active ingredient in the herbicide bialaphos, phosphinothricin (PPT). PPT inhibits glutamine synthetase, (Murakami 1986; Twell 1989) causing rapid accumulation of ammonia and cell death.
- altered 5-enolpyruvylshikimate-3-phosphate synthase (EPSPS) conferring resistance to Glyphosate® (N-(phosphonomethyl)glycine) (Hinchee 1988; Shah 1986; Della-Cioppa 1987). Where a mutant EPSP synthase gene is employed, additional benefit may be realized through the incorporation of a suitable chloroplast transit peptide, CTP (EP-A1 0 218 571).
- Glyphosate® degrading enzymes (Glyphosate® oxidoreductase; gox),

- Dalapon® inactivating dehalogenases (deh)
- sulfonylurea- and/or imidazolinone-inactivating acetolactate synthases (*ahas* or ALS; for example mutated ahas/ALS variants with, for example, the S4, XI12, XA17, and/or Hra mutation (EP-A1 154 204)
- Bromoxynil® degrading nitrilases (bxn; Stalker 1988)
- Kanamycin- or geneticin (G418) resistance genes (NPTII; NPT or neo; Potrykus 1985) coding e.g., for neomycin phosphotransferases (Fraley 1983; Nehra 1994)
- 2-Desoxyglucose-6-phosphate phosphatase (DOG$^R$1-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- altered dihydrofolate reductase (Eichholtz 1987) conferring resistance against methotrexat (Thillet 1988);
- mutated anthranilate synthase genes that confers resistance to 5-methyl tryptophan.

Additional negative selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Especially preferred are negative selection markers that confer resistance against the toxic effects imposed by D-amino acids like e.g., D-alanine and D-serine (WO 03/060133; Erikson 2004). Especially preferred as negative selection marker in this contest are the *dao*I gene (EC: 1.4. 3.3: GenBank Acc.-No.: U60066) from the yeast *Rhodotorula gracilis* (*Rhodosporidium toruloides*) and the *E. coli* gene *dsd*A (D-serine dehydratase (D-serine deaminase) [EC: 4.3. 1.18; GenBank Acc.-No.: J01603).

Transformed plant material (e.g., cells, embryos, tissues or plantlets) which express such marker genes are capable of developing in the presence of concentrations of a corresponding selection compound (e.g., antibiotic or herbicide) which suppresses growth of an untransformed wild type tissue. The resulting plants can be bred and hybridized in the customary fashion. Two or more generations should be grown in order to ensure that the genomic integration is stable and hereditary. Corresponding methods are described (Jenes 1993; Potrykus 1991).

Furthermore, reporter genes can be employed to allow visual screening, which may or may not (depending on the type of reporter gene) require supplementation with a substrate as a selection compound.

Various time schemes can be employed for the various negative selection marker genes. In case of resistance genes (e.g., against herbicides or D-amino acids) selection is preferably applied throughout callus induction phase for about 4 weeks and beyond at least 4 weeks into regeneration. Such a selection scheme can be applied for all selection regimes. It is furthermore possible (although not explicitly preferred) to remain the selection also throughout the entire regeneration scheme including rooting.

For example, with the phosphinotricin resistance gene (bar) as the selective marker, phosphinotricin at a concentration of from about 1 to 50 mg/l may be included in the medium. For example, with the *dao1* gene as the selective marker, D-serine or D-alanine at a concentration of from about 3 to 100 mg/l may be included in the medium. Typical concentrations for selection are 20 to 40 mg/l. For example, with the mutated ahas genes as the selective marker, PURSUIT™ at a concentration of from about 3 to 100 mg/l may be included in the medium. Typical concentrations for selection are 20 to 40 mg/l.

2.1.2 Positive selection marker

Furthermore, positive selection marker can be employed. Genes like isopentenyltransferase from *Agrobacterium* tumefaciens (strain:PO22; Genbank Acc.-No.: AB025109) may - as a key enzyme of the cytokinin biosynthesis - facilitate regeneration of transformed plants (e.g., by selection on cytokinin-free medium). Corresponding selection methods are described (Ebinuma 2000a,b). Additional positive selection markers, which confer a growth advantage to a transformed plant in comparison with a non-transformed one, are described e.g., in EP-A 0 601 092. Growth stimulation selection markers may include (but shall not be limited to) beta-Glucuronidase (in combination with e.g., a cytokinin glucuronide), mannose-6-phosphate isomerase (in combination with mannose), UDP-galactose-4-epimerase (in combination with e.g., galactose), wherein mannose-6-phosphate isomerase in combination with mannose is especially preferred.

2.1.3 Counter-selection marker

Counter-selection markers are especially suitable to select organisms with defined deleted sequences comprising said marker (Koprek 1999). Examples for counter- selection marker comprise thymidin kinases (TK), cytosine deaminases (Gleave 1999; Perera 1993; Stougaard 1993), cytochrom P450 proteins (Koprek 1999), haloalkan dehalogenases (Naested 1999), iaaH gene products (Sundaresan 1995), cytosine deaminase codA (Schlaman & Hooykaas 1997), tms2 gene products (Fedoroff & Smith 1993), or alpha-naphthalene acetamide (NAM; Depicker 1988). Counter selection markers may be useful in the construction of transposon tagging lines. For example, by marking an autonomous transposable element such as Ac, Master Mu, or En/Spn with a counter selection marker, one could select for transformants in which the autonomous element is not stably integrated into the genome. This would be desirable, for example, when transient expression of the autonomous element is desired to activate in trans the transposition of a defective transposable element, such as Ds, but stable integration of the autonomous element is not desired. The presence of the autonomous element may not be desired in order to stabilize the defective element, i.e., prevent it from further transposing. However, it is proposed that if stable integration of an autonomous transposable element is desired in a plant the presence of a negative selectable marker may make it possible to eliminate the autonomous element during the breeding process.

2.2. Screenable Markers

Screenable markers that may be employed include, but are not limited to, a beta-glucuronidase (GUS) or uidA gene which encodes an enzyme for which various chromogenic substrates are known; an R-locus gene, which encodes a product that regulates the production of anthocyanin pigments (red color) in plant tissues (Dellaporta 1988); a beta-lactamase gene (Sutcliffe 1978), which encodes an enzyme for which various chromogenic substrates are known (e.g., PADAC, a chromogenic cephalosporin); a xylE gene (Zukowsky 1983) which encodes a catechol dioxygenase that can convert chromogenic catechols; an alpha-amylase gene (Ikuta 1990); a tyrosinase gene (Katz 1983) which encodes an enzyme capable of oxidizing tyrosine to DOPA and dopaquinone which in turn condenses to form the easily detectable compound melanin; beta-galactosidase gene, which encodes an enzyme for which there are chromogenic substrates; a luciferase (lux) gene (Ow 1986), which allows for bioluminescence detection; or even an aequorin gene (Prasher 1985), which may be employed in calcium-sensitive bioluminescence detection, or a green fluorescent protein gene (Niedz 1995).

Genes from the maize R gene complex are contemplated to be particularly useful as screenable markers. The R gene complex in maize encodes a protein that acts to regulate the production of anthocyanin pigments in most seed and plant tissue. A gene from the R gene complex was applied to maize transformation, because the expression of this gene in transformed cells does not harm the cells. Thus, an R gene introduced into such cells will cause the expression of a red pigment and, if stably incorporated, can be visually scored as a red sector. If a maize line is dominant for genes encoding the enzymatic intermediates in the anthocyanin biosynthetic pathway (C2, A1, A2, Bz1 and Bz2), but carries a recessive allele at the R locus, transformation of any cell from that line with R will result in red pigment formation. Exemplary lines include Wisconsin 22 which contains the rg-Stadler allele and TR112, a K55 derivative which is r-g, b, P1. Alternatively any genotype of maize can be utilized if the C1 and R alleles are introduced together.

It is further proposed that R gene regulatory regions may be employed in chimeric constructs in order to provide mechanisms for controlling the expression of chimeric genes. More diversity of phenotypic expression is known at the R locus than at any other locus (Coe 1988). It is contemplated that regulatory regions obtained from regions 5' to the structural R gene would be valuable in directing the expression of genes, e.g., insect resistance, drought resistance, herbicide tolerance or other protein coding regions. For the purposes of the present invention, it is believed that any of the various R gene family members may be successfully employed (e.g., P, S, Lc, etc.). However, the most preferred will generally be Sn (particularly Sn:bol3). Sn is a dominant member of the R gene complex and is functionally similar to the R and B loci in that Sn controls the tissue specific deposition of anthocyanin pigments in certain seedling and plant cells, therefore, its phenotype is similar to R.

A further screenable marker contemplated for use in the present invention is firefly luciferase, encoded by the lux gene. The presence of the lux gene in transformed cells may be detected using, for example, X-ray film, scintillation counting, fluorescent spectrophotometry, low-light video cameras, photon counting cameras or multiwell luminometry. It is also envisioned that this system may be developed for populational screening for bioluminescence, such as on tissue culture plates, or even for whole plant screening. Where use of a screenable marker gene such as lux or GFP is desired, benefit may be realized by creating a gene fusion between the screenable marker gene and a selectable marker gene, for example, a GFP-NPTII gene fusion. This could allow, for example, selection of transformed cells followed by screening of transgenic plants or seeds.

3. Uses of Transgenic Plants

Once an expression cassette of the invention has been transformed into a particular plant species, it may be propagated in that species or moved into other varieties of the same species, particularly including commercial varieties, using traditional breeding techniques. Particularly preferred plants of the invention include the agronomically important crops listed above. The genetic properties engineered into the transgenic seeds and plants described above are passed on by sexual reproduction and can thus be maintained and propagated in progeny plants. The present invention also relates to a transgenic plant cell, tissue, organ, seed or plant part obtained from the transgenic plant. Also included within the invention are transgenic descendants of the plant as well as transgenic plant cells, tissues, organs, seeds and plant parts obtained from the descendants.

Preferably, the expression cassette in the transgenic plant is sexually transmitted. In one preferred embodiment, the coding sequence is sexually transmitted through a complete normal sexual cycle of the R0 plant to the R1 generation. Additionally preferred, the expression cassette is expressed in the cells, tissues, seeds or plant of a transgenic plant in an amount that is different than the amount in the cells, tissues, seeds or plant of a plant, which only differs in that the expression cassette is absent.

The transgenic plants produced herein are thus expected to be useful for a variety of commercial and research purposes. Transgenic plants can be created for use in traditional agriculture to possess traits beneficial to the grower (e.g., agronomic traits such as resistance to water deficit, pest resistance, herbicide resistance or increased yield), beneficial to the consumer of the grain harvested from the plant (e.g., improved nutritive content in human food or animal feed; increased vitamin, amino acid, and antioxidant content; the production of antibodies (passive immunization) and nutriceuticals), or beneficial to the food processor (e.g., improved processing traits). In such uses, the plants are generally grown for the use of their grain in human or animal foods. Additionally, the use of root-specific promoters in transgenic plants can provide beneficial traits that are localized in the consumable (by animals and humans) roots of plants such as carrots, parsnips, and beets. However, other parts of the plants, including stalks, husks, vegetative parts, and the like, may also have utility, including use as part of animal silage or for ornamental purposes. Often, chemical constituents (e.g., oils or starches) of maize and other crops are extracted for foods or industrial use and transgenic plants may be created which have enhanced or modified levels of such components.

Transgenic plants may also find use in the commercial manufacture of proteins or other molecules, where the molecule of interest is extracted or purified from plant parts, seeds, and the like. Cells or tissue from the plants may also be cultured, grown in vitro, or fermented to manufacture such molecules. The transgenic plants may also be used in commercial breeding programs, or may be crossed or bred to plants of related crop species. Improvements encoded by the expression cassette may be transferred, e.g., from maize cells to cells of other species, e.g., by protoplast fusion.

The transgenic plants may have many uses in research or breeding, including creation of new mutant plants through insertional mutagenesis, in order to identify beneficial mutants that might later be created by traditional mutation and selection. An example would be the introduction of a recombinant DNA sequence encoding a transposable element that may be used for generating genetic variation. The methods of the invention may also be used to create plants having unique "signature sequences" or other marker sequences which can be used to identify proprietary lines or varieties.

Thus, the transgenic plants and seeds according to the invention can be used in plant breeding, which aims at the development of plants with improved properties conferred by the expression cassette, such as tolerance of drought, disease, or other stresses. The various breeding steps are characterized by well-defined human intervention such as selecting the lines to be crossed, directing pollination of the parental lines, or selecting appropriate descendant plants. Depending on the desired properties different breeding measures are taken. The relevant techniques are well known in the art and include but are not limited to hybridization, inbreeding, backcross breeding, multilane breeding, variety blend, interspecific hybridization, aneuploid techniques, etc. Hybridization techniques also include the sterilization of plants to yield male or female sterile plants by mechanical, chemical or biochemical means. Cross-pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines, which for example increase the effectiveness of conventional methods such as herbicide or pesticide treatment or allow dispensing with said methods due to their modified genetic properties. Alternatively new crops with improved stress tolerance can be obtained which, due to their optimized genetic "equipment", yield harvested product of better quality than products, which were not able to tolerate comparable adverse developmental conditions.

The invention will be further illustrated by the following examples.

## EXAMPLES

### EXAMPLE 1:

### IDENTIFICATION OF KG (KEYGENE) TRANSCRIPT CANDIDATES

A maize gene expression profiling analysis was carried out using a commercial supplier of AFLP comparative expression technology (Keygene N.V., P.O.Box 216, 6700 AE Wageningen, The Netherlands) using a battery of RNA samples from 23 maize tissues generated by the inventors of the present invention (Table 1). Nine fragments were identified as having embryo or whole seed specific expression. These fragments were designated as KG_Fragment 56, 129, 49, 24, 37, 45, 46, 103, 119, respectively. Sequences of each fragment are shown in SEQ ID NOs: 145 to 153.

| Table 1. Corn Tissues used for mRNA expression profiling experiment | | | |
|---|---|---|---|
| **Sample No.** | **Tissue** | **Timing and number of plants** | **Days after Pollination** |
| 1 | Root | 9am (4 plants) | 5 |
| 2 | | 9am (4 plants) | 15 |
| 3 | | 9am (4 plants) | 30 |
| 4 | leaf above the ear | 9 am (6 plants) | 5 |
| 5 | | 9 am (6 plants) | 15 |
| 6 | | 9 am (6 plants) | 30 |
| 7 | ear complete | 9 am (6 plants) | 5 |
| 8 | | 9 am (6 plants) | 10 |
| 9 | Whole seed | 9am (6 plants) | 15 |
| 10 | | 9am (6 plants) | 20 |
| 11 | | 9am (6 plants) | 30 |
| 12 | Endosperm | 9am(6 plants) | 15 |
| 13 | | 9am(6 plants) | 20 |
| 14 | | 9am(6 plants) | 30 |
| 15 | Embryo | 9 am (6 plants) | 15 |
| 16 | | 9 am (6 plants) | 20 |
| 17 | | 9 am (6 plants) | 30 |
| 18 | Female pistilate flower | 6 plants | before pollination |
| 19 | germinating seed | 20 seeds | imbibition for 3 days |
| 20 | root, veg. state | | V2 |
| 21 | root, veg. state | | V4 |
| 22 | leaf, veg. State | | V2 |
| 23 | leaf, veg. State | | V4 |

EXAMPLE 2:

IDENTIFICATION OF THE EST CORRESPONDING TO KG_FRAGMENT CANDIDATES

Sequences of the KG_Fragment candidates were used as query for BLASTN searching against inventor's in-house database, HySeq All EST. EST accessions showing highest identities to above KG_Fragments are listed in Table 2 and sequences of these ESTs are shown in SEQ ID NOs: 93, 94, and 98-104.

Table 2. Maize EST accession number showing highest identities to the KG fragment candidates

| KG Fragment ID | Hyseq Maize EST ID | % identities | SEQ ID NO: |
|---|---|---|---|
| 24 | 62001211.f01 | 100 | 99 |
| 37 | 62029487.f01 | 100 | 100 |
| 45 | 57894155.f01 | 100 | 101 |
| 46 | 62096689.f01 | 100 | 102 |
| 49 | 62158447.f01 | 91 | 98 |
| 56 | no | N/A | 93 |

(continued)

| KG Fragment ID | Hyseq Maize EST ID | % identities | SEQ ID NO: |
|---|---|---|---|
| 103 | ZM07MC01323_57619299 | 100 | 103 |
| 119 | ZM07MC15086_59463108 | 100 | 104 |
| 129 | 62092959.f01 | 100 | 94 |

EXAMPLE 3:

CONFIRMATION OF EXPRESSION PATTERN OF THE KG CANDIDATES USING QUANTITATIVE REVERSE TRANSCRIPTASE-POLYMERASE CHAIN REACTION (Q-RT-PCR)

In order to confirm the native expression pattern of the KG candidates, quantitative reverse transcription PCR (q-RT-PCR) was performed using total RNA isolated from the same materials as were used for the AFLP expression profiling (Table 1).

Primers for qRT-PCR were designed based on the sequences of either the KG_Fragments or the identified maize Hyseq EST using the Vector NTI software package (Invitrogen, Carlsbad, CA, USA). Two sets of primers were used for PCR amplification for each candidate. The glyceraldehyde-3-phosphate dehydrogenase (GAPDH) gene served as a control for normalization purposes. Sequences of primers for q-RT-PCR are listed in Table 3.

Table 3. Primer sequences for q-RT-PCR

| Primer | Sequences |
|---|---|
| KG24_forward_1 | GTGGCTGTCATACTGGAT |
| KG24_reverse_1 | GAGCTTCTCGTAGACGAA |
| KG24_forward_2 | TCACAGGAACTTCTGTAGAT |
| KG24_reverse_2 | TCGTTCTTACAGAAGCAT |
| KG37_forward_1 | AAGGCATGTTATGCTCGA |
| KG37_reverse_1 | AAACTCGAAAACCGCCAC |
| KG37_forward_2 | AGGCAAGTTCAAGACAAC |
| KG37_reverse_2 | AAAAATCCCATCTGTCCC |
| KG45_forward_1 | TGCTGGTGAATGATGGTT |
| KG45_reverse_1 | CACATCGTTCGCTACATA |
| KG45_forward_2 | ACGCCTCCCCTCGTGATT |
| KG45_reverse_2 | TGCCAGACGTACCCGACGG |
| KG46_forward_1 | CTGCGGAGGCGAACAGGA |
| KG46_reverse_1 | GCTTGTCGACGGAGACGG |
| KG46_forward_2 | CCGGACATCGGCGTCTACCTC |
| KG46_reverse_2 | CCGTTCGGGAACACCACC |
| KG49_forward_1 | CAGCTGGTGGGGAGGATAT |
| KG49_reverse_1 | CGAGCCTGTGAATTGCAT |
| KG49_forward_2 | ATCTTCTCACGATCCAGG |
| KG49_reverse_2 | TTGTGAACAGCATGTCCC |
| KG56_forward_1 | AAATACGAAGCCCGGATC |
| KG56_reverse_1 | TAGTGTCCGTCCACCTGT |
| KG56_forward_2 | AGCCAGGGCCATATAACA |

(continued)

| Primer | Sequences |
|---|---|
| KG56_reverse_2 | TAGCTGTTTCTGCCCATA |
| KG103_forward_1 | TCCACCTTAGCCTAGGGTT |
| KG103_reverse_1 | AACACGCAGCTTTCCAAA |
| KG103_forward_2 | CAAGCTCTCCCTGGAGAT |
| KG103_reverse_2 | GCGAAGACCACACAGACA |
| KG119_forward_1 | CAGACAGACCACTGACTGCAT |
| KG119_reverse_1 | GTTAGGCCTGTGCGTGTG |
| KG119_forward_2 | CTGAGAGCCCCGGAACTCGTT |
| KG119_reverse_2 | TGTGCCGGGCTCTGGGTT |
| KG129_forward_1 | GCTCACCAACGGAGTGAT |
| KG129_reverse_1 | CATCAGAGTTCCCGTCGT |
| KG129_forward_2 | GTCTCTCCCCGCTAGTGACTT |
| KG129_reverse_2 | GGGAAAGTCGCTCACGAA |
| GAPDH_Forward | GTAAAGTTCTTCCTGATCTGAAT |
| GAPDH_Reverse | TCGGAAGCAGCCTTAATA |

q-RT-PCR was performed using SuperScript III Reverse Transcriptase (Invitrogen, Carlsbad, CA, USA) and SYBR Green QPCR Master Mix (Eurogentec, San Diego, CA, USA) in an ABI Prism 7000 sequence detection system. In brief, cDNA was synthesized using 2-3 microgram of total RNA and 1 μL reverse transcriptase in a 20 pl volume. The cDNA was diluted to a range of concentrations (15-20 ng/μl). Thirty to forty ng of cDNA was used for quantitative PCR (qPCR) in a 30 μL volume with SYBR Green QPCR Master Mix following the manufacturer's instruction. The thermocycling conditions were as follows: incubate at 50°C for 2 minutes, denature at 95°C for 10 minutes, and run 40 cycles at 95°C for 15 seconds and 60°C for 1 minute for amplification. After the final cycle of the amplification, the dissociation curve analysis was carried out to verify that the amplification occurred specifically and no primer dimer product was generated during the amplification process. The housekeeping gene glyceraldehyde-3-phosphate-dehydrogenase (GAPDH, primer sequences in Table 3) was used as an endogenous reference gene to normalize the calculation using the Comparative Ct (Cycle of threshold) value method. The $\Delta C_T$ value was obtained by subtracting the Ct value of GAPDH gene from the Ct value of the candidate gene, and the relative transcription quantity (expression level) of the candidate gene was expressed as $2^{-\Delta CT}$. The q-RT-PCR results are summarized in Figure 1. All KG candidates showed similar expression patterns that are equivalent to the expression patterns obtained from the AFLP data: specifically or preferably expressed in embryo or whole seeds (Figure 1).

EXAMPLE 4:

ANNOTATION AND PROMOTER IDENTIFICATION OF THE KG CANDIDATES

The coding sequences corresponding to KG candidates were annotated based on the *in silico* results obtained from both BLASTX of each EST sequence against GenBank protein database (nr) and the result of *in silico* translation of the sequence using Vector NTI software package.

1). KG_fragment 24

Maize EST 62001211.f01 encodes a protein that has homology to a hypothetical protein of wheat (GenBank Accession: BAC80265). The top 10 homologous sequences identified in the BlastX query are presented in Table 4.

Table 4. BLASTX search results of KG_fragment 24/Hyseq EST 62001211.f01

| Accession | Description | Score | E-value | % Identities |
|---|---|---|---|---|
| BAC80265 | hypothetical protein [Triticum aestivum]. | 191 | 9.00E-56 | 81 |
| Q07764 | HVA22_HORVU Protein HVA22 | 191 | 2.00E-54 | 81 |
| EAY81013.1 | hypothetical protein OsJ_OsI_034972 [Oryza sativa (indica cultivar-group)] | 162 | 2.00E-39 | 67 |
| NP_001062004.1 | Os08g0467500 [Oryza sativa (japonica cultivar-group)] | 133 | 5.00E-39 | 76 |
| EAZ18437.1 | hypothetical protein OsJ_032646 [Oryza sativa (japonica cultivar-group)] | 161 | 6.00E-39 | 66 |
| NP_001067939.1 | Os11g0498600 [Oryza sativa (japonica cultivar-group)] | 161 | 6.00E-39 | 66 |
| NP_568744.1 | ATHVA22E (Arabidopsis thaliana HVA22 homologue E) | 148 | 1.00E-35 | 62 |
| BAD09552.1 | putative abscisic acid-induced protein [Oryza sativa Japonica Group] | 119 | 5.00E-35 | 75 |
| NP_567713.1 | ATHVA22D (Arabidopsis thaliana HVA22 homologue D) | 139 | 1.00E-31 | 57 |
| EAZ07285.1 | hypothetical protein OsI_028517[0ryza sativa (indica cultivar-group)] | 119 | 1.00E-31 | 75 |

The CDS sequence of the gene corresponding to KG_Fragment 24 is shown in SEQ ID NO: 27 and the translated amino acid sequence is shown in SEQ ID NO: 45

Identification of the promoter region of KG24

For our promoter identification purposes, the sequence upstream of the start codon of the predicted KG_Fragment 24 gene was defined as the promoter p-KG24. To identify this predicted promoter region, the EST sequence of 62001211.f_o1 was mapped to the BASF Plant Science proprietary maize genomic DNA sequence database, PUB_tigr_maize_genomic_partial_5.0.nt. One maize genomic DNA sequence, AZM5_23949 (3602 bp) was identified (SEQ ID NO: 81). This 3602bp sequence harbored the predicted CDS of the corresponding gene to KG_Fragment 24 and more than 1.6 kb upstream sequence of the ATG start codon of this gene

Isolation of the promoter region of KG24 by PCR amplification

The putative promoter region was isolated via genomic PCR using the following sequence specific primers:

Forward primer: CTACATTTATGTTATAGAGGCGCAA (SEQ ID NO: 154)
Reverse primer: CATCTCTTGGGACGGAACCAA (SEQ ID NO: 155). The expected 1507bp fragment was amplified from maize genomic DNA, and named as promoter KG24 (p-KG24). Sequence of p-KG24 is shown in SEQ ID NO: 9.

BLASTN results of p_KG24

The top 13 homologous sequences identified in the BlastN query are presented in Table 5.

Table 5. BlastN results of p_KG24

| Accession | Description | Max score | Total score | Query coverage | E value | Max ident |
|---|---|---|---|---|---|---|
| AF205807.1 | Zea mays subsp. huehuetenangensis isolate Beadle s.n. b1 gene, B-M033 allele, partial sequence | 522 | 660 | 22% | 2.00E-144 | 94% |
| EU961185.1 | Zea mays clone 233237 unknown mRNA | 491 | 491 | 21% | 3.00E-135 | 93% |
| EU945925.1 | Zea mays clone 290258 mRNA sequence | 491 | 491 | 21% | 3.00E-135 | 93% |

(continued)

| Accession | Description | Max score | Total score | Query coverage | E value | Max ident |
|---|---|---|---|---|---|---|
| AF448416.1 | Zea mays B73 chromosome 9S bz genomic region | 491 | 660 | 33% | 3.00E-135 | 94% |
| AC157319.2 | Zea mays clone ZMMBBb-136E2, complete sequence | 489 | 489 | 21% | 1.00E-134 | 93% |
| AY883458.1 | Zea mays subsp. parviglumis cultivar CIMMYT-11355 teosinte glume architecture 1 (tga1) gene, promoter region | 484 | 484 | 21% | 5.00E-133 | 92% |
| AY508163.1 | Zea mays cultivar F324 disrupted peroxidase (pox3) gene, exons 1 through 3; and transposon MITE, complete sequence | 479 | 479 | 21% | 2.00E-131 | 92% |
| AY508162.1 | Zea mays cultivar F227 disrupted peroxidase (pox3) gene, exons 1 through 3; and transposon MITE, complete sequence | 479 | 479 | 21% | 2.00E-131 | 92% |
| AY508161.1 | Zea mays cultivar F226 disrupted peroxidase (pox3) gene, exons 1 through 3; and transposon MITE, complete sequence | 479 | 479 | 21% | 2.00E-131 | 92% |
| AY508160.1 | Zea mays cultivar F7012 disrupted peroxidase (pox3) gene, exons 1 through 3; and transposon MITE, complete sequence | 479 | 479 | 21% | 2.00E-131 | 92% |
| AY508159.1 | Zea mays cultivar Quebec28 disrupted peroxidase (pox3) gene, pox3-2 allele, exons 1 through 3; and transposon MITE, complete sequence | 479 | 479 | 21% | 2.00E-131 | 92% |
| AY883461.1 | Zea mays subsp. parviglumis cultivar HGW-Wilkes Site 6 teosinte glume architecture 1 (tga1) gene, promoter region | 479 | 479 | 21% | 2.00E-131 | 93% |
| AY508516.1 | Zea mays disrupted peroxidase (pox3) gene, partial sequence; and transposon MITE, complete sequence | 475 | 475 | 21% | 3.00E-130 | 92% |

**2). KG_fragment 37**

KG_fragment 37/Maize EST 62029487.f01 encodes a protein that is homologous to a hypothetical protein of rice (GenBank Accession: NP_001051496). The top 15 homologous sequences identified in the BlastX query are presented in Table 6.

Table 6. BLASTX search results of KG_fragment 37/Hyseq EST 62029487.f01

| Accession | Description | Score | E-value | % Identities |
|---|---|---|---|---|
| NP_001051496 | Os03g0787200 [Oryza sativa (japonica cultivar-group)]. | 518 | e-145 | 65 |
| EAY92106 | hypothetical protein OsI_013339 [Oryza sativa (indica cultivar-group) | 518 | e-145 | 65 |
| ABF99245 | IQ calmodulin-binding motif family protein, expressed [Oryza sativa(japonica cultivar-group)] | 479 | e-133 | 67 |
| CAO70668 | unnamed protein product [Vitis vinifera] | 367 | 2.00E-99 | 51 |
| CAN68445 | hypothetical protein [Vitis vinifera]. | 318 | 8.00E-85 | 46 |

(continued)

| Accession | Description | Score | E-value | % Identities |
|---|---|---|---|---|
| NP_001067295 | Os12g0619000 [Oryza sativa (japonica cultivar-group)] | 301 | 1.00E-79 | 45 |
| NP_188858 | IQD5 (IQ-domain 5); calmodulin binding [Arabidopsis thaliana] | 301 | 2.00E-79 | 49 |
| BAB03067 | unnamed protein product [Arabidopsis thaliana] | 298 | 1.00E-78 | 44 |
| ACF85687 | unknown [Zea mays] | 294 | 2.00E-77 | 45 |
| EAY94673 | hypothetical protein OsI_015906 [Oryza sativa (indica cultivar-group)] | 280 | 4.00E-73 | 43 |
| EAY83925 | hypothetical protein OsI_037884 [Oryza sativa (indica cultivar-group)] | 279 | 5.00E-73 | 43 |
| NP_001050778 | Os03g0648300 [Oryza sativa (japonica cultivar-group)] | 276 | 6.00E-72 | 43 |
| AAU89191 | expressed protein [Oryza sativa (japonica cultivar-group)] | 276 | 6.00E-72 | 43 |
| EAZ27950 | hypothetical protein OsJ_011433 [Oryza sativa (japonica cultivar-group)] | 269 | 3.00E-27 | 44 |
| EAY92104 | hypothetical protein OsI_013337 [Oryza sativa (indica cultivar-group)] | 266 | 4.00E-69 | 73 |

The CDS sequence of the gene corresponding to KG_Fragment 37 is shown in SEQ ID NO: 28 and the translated amino acid sequence is shown in SEQ ID NO: 46.

**Identification of the promoter region of KG37**

For our promoter identification purposes, the sequence upstream of the start codon of the predicted KG_Fragment 37 gene was defined as the promoter p-KG37. To identify this predicted promoter region, the EST sequence of 62029487.f_o1 was mapped to the BASF Plant Science proprietary maize genomic DNA sequence database, PUB_tigr_maize_genomic_partial_5.0.nt. The reverse complement sequence of AZM5_22959 (2441 bp) was identified (SEQ ID NO: 82). This 2441 bp sequence harbored partial predicted CDS of the corresponding gene to KG_Fragment 37 and about 1.4 kb upstream sequence of the ATG start codon of this gene.

**Isolation of the promoter region of KG37 by PCR amplification**

The putative promoter region was isolated via genomic PCR using the following sequence specific primers:

Forward primer: CATACGATTTCCTAAGCGGAATC (SEQ ID NO: 156)
Reverse primer:CCGCCCGCCTCAACCACAGT (SEQ ID NO: 157). The expected 910bp fragment was amplified from maize genomic DNA, and named as promoter KG37 (p-KG37). Sequence of p-KG37 is shown in SEQ ID NO: 10.

**BLASTN results of p_KG37**

The top 11 homologous sequences identified in the BlastN query are presented in Table 7.

Table 7. BlastN results of p_KG37

| Accession | Description | Max score | Total score | Query coverage | E value | Max ident |
|---|---|---|---|---|---|---|
| EU966853.1 | Zea mays clone 297738 unknown mRNA | 619 | 619 | 38% | 6.00E-174 | 99% |
| AC084296.12 | Oryza sativa chromosome 3 BAC OSJNBb0024J04 genomic sequence, complete sequence | 51.8 | 51.8 | 7% | 0.006 | 78% |

(continued)

| Accession | Description | Max score | Total score | Query coverage | E value | Max ident |
|---|---|---|---|---|---|---|
| AP008209.1 | Oryza sativa (japonica cultivar-group) genomic DNA, chromosome 3 | 51.8 | 51.8 | 7% | 0.006 | 78% |
| BX284754.1 | Neurospora crassa DNA linkage group II BAC contig B23G1 | 50 | 50 | 3% | 0.02 | 93% |
| AC143357.1 | Pan troglodytes BAC clone RP43-171 L24 from chromosome 7, complete sequence | 48.2 | 48.2 | 3% | 0.069 | 96% |
| AC003013.1 | Human PAC clone RP1-205E24 from Xq23, complete sequence | 48.2 | 48.2 | 3% | 0.069 | 96% |
| AL136101.7 | Human DNA sequence from clone RP5-954O23 on chromosome Xq22.2-23, complete sequence | 48.2 | 48.2 | 3% | 0.069 | 96% |
| AM910995.1 | Plasmodium knowlesi strain H chromosome 13, complete genome | 46.4 | 46.4 | 4% | 0.24 | 82% |
| AY573057.1 | Plasmodium knowlesi merozoite surface protein 4 (MSP4) gene, complete cds | 46.4 | 46.4 | 3% | 0.24 | 90% |
| AC120393.16 | Mus musculus chromosome 7, clone RP24-312B12, complete sequence | 46.4 | 46.4 | 3% | 0.24 | 89% |
| AL357510.17 | Human DNA sequence from clone RP11-195F21 on chromosome 10 Contains the 5' end of a novel gene, complete sequence | 46.4 | 46.4 | 4% | 0.24 | 85% |

**3). KG_fragment 45**

KG_fragment 45/Maize EST 57894155.f01 encodes a protein that is homologous to a hypothetical protein Os06g0473800 of rice (GenBank Accession: NP_001057629). The top 10 homologous sequences identified in the BlastX query are presented in Table 8.

Table 8. BLASTX search results of KG_fragment 45/Hyseq EST 57894155.f01

| Accession | Description | Score | E-value | % Identities |
|---|---|---|---|---|
| NP_001057629 | Os06g0473800 [Oryza sativa (japonica cultivar-group)] | 176 | 1 e-42 | 60 |
| EAZ00929 | hypothetical protein Osl_022161 [Oryza sativa (indica cultivar-group)] | 172 | 2e-41 | 66 |
| AAG01171 | seed oleosin isoform 1 [Fagopyrum esculentum] | 97 | 1e-18 | 40 |
| AAG09751 | oleosin [Perilla frutescens] | 91 | 6e-17 | 36 |
| AAG24455 | 19 kDa oleosin [Perilla frutescens] | 91 | 8e-17 | 36 |
| AAB58402 | 15.5 kDa oleosin [Sesamum indicum] | 90 | 1e-16 | 45 |
| AAB24078 | lipid body membrane protein [Daucus carota] | 89 | 2e-16 | 42 |
| CAA57994 | high molecular weight oleosin [Hordeum vulgare subsp. Vulgare] | 89 | 2e-16 | 48 |
| AAG23840 | oleosin [Sesamum indicum] | 89 | 3e-16 | 37 |
| ABW90149 | oleosin 2 [Jatropha curcas] | 88 | 5e-16 | 35 |

The CDS sequence of the gene corresponding to KG_Fragment 45 is shown in SEQ ID NO: 29 and the translated amino acid sequence is shown in SEQ ID NO: 47.

Identification of the promoter region of KG45

For our promoter identification purposes, the sequence upstream of the start codon of the predicted KG_Fragment 45 gene was defined as the promoter p-KG45. To identify this predicted promoter region, the sequence of 57894155.f_o1 was mapped to the BASF Plant **Science proprietary** genomic DNA sequence database, PUB_tigr_maize_genomic_partia)_5.0.nt. The reverse complement sequence of a maize genomic DNA sequence, AZM5_29112 (2548bp) was identified (SEQ ID NO: 83). This 2548bp sequence harbored the predicted CDS of the corresponding gene to KG_Fragment 45 and about 1.2 kb upstream sequence of the ATG start codon of this gene.

Isolation of the promoter region of KG45 by PCR amplification

The putative promoter region was isolated via genomic PCR using the following sequence specific primers:

Forward primer: CCAGCCATCGTGCTTGAGTG (SEQ ID NO: 158)
Reverse primer: GACGTGGTGGCGATCGCAAG (SEQ ID NO: 159) The expected 1131bp fragment was amplified from maize genomic DNA, and named as promoter KG45 (p-KG45). Sequence of p-KG45 is shown in SEQ ID NO:11.

BLASTN results of p_KG45

The top 15 homologous sequences identified in the BlastN query are presented in Table 9.

Table 9. BlastN results of p_KG45

| Accession | Description | Max score | Total score | Query coverage | E value | Max ident |
|---|---|---|---|---|---|---|
| EU976834.1 | Zea mays clone 991429 unknown mRNA | 59 | 59 | 2% | 5.00E-05 | 100% |
| CU634021.8 | Zebrafish DNA sequence from clone CH73-96B22 in linkage group 20, complete sequence | 53.6 | 53.6 | 4% | 0.002 | 85% |
| AC158582.2 | Mus musculus chromosome 7, clone RP24-173K12, complete sequence | 51.8 | 51.8 | 4% | 0.007 | 83% |
| AC102506.9 | Mus musculus chromosome 1, clone RP24-139E15, complete sequence | 51.8 | 51.8 | 4% | 0.007 | 80% |
| AC114988.21 | Mus musculus chromosome 7, clone RP23-207N5, complete sequence | 51.8 | 51.8 | 4% | 0.007 | 83% |
| AY105760.2 | Zea mays PCO070107 mRNA sequence | 50 | 50 | 2% | 0.025 | 100% |
| DQ485452.1 | Homo sapiens protein kinase D1 (PRKD1) gene, complete cds | 50 | 50 | 3% | 0.025 | 89% |
| AC102004.7 | Mus musculus chromosome 15, clone RP24-489M6, complete sequence | 50 | 50 | 4% | 0.025 | 82% |
| AC158556.9 | Mus musculus chromosome 15, clone RP23-140F20, complete sequence | 50 | 50 | 4% | 0.025 | 82% |
| AC 111275.4 | Rattus norvegicus 4 BAC CH230-49L22 (Children's Hospital Oakland Research Institute) complete sequence | 50 | 50 | 5% | 0.025 | 78% |
| AC097745.8 | Rattus norvegicus 3 BAC CH230-11 N5 (Children's Hospital Oakland Research Institute) complete sequence | 50 | 50 | 4% | 0.025 | 80% |

(continued)

| Accession | Description | Max score | Total score | Query coverage | E value | Max ident |
|---|---|---|---|---|---|---|
| AL356756.4 | Human chromosome 14 DNA sequence BAC C-2503I6 of library CalTech-D from chromosome 14 of Homo sapiens (Human), complete sequence | 50 | 50 | 3% | 0.025 | 89% |
| AL445884.4 | Human chromosome 14 DNA sequence BACR-419C10 of library RPCI-11 from chromosome 14 of Homo sapiens (Human), complete sequence | 50 | 50 | 3% | 0.025 | 89% |
| AC199142.9 | Canis familiaris, clone XX-240A15, complete sequence | 48.2 | 48.2 | 4% | 0.087 | 82% |
| AC182436.1 | Mus musculus chromosome 5, clone wi1-1982K15, complete sequence | 48.2 | 48.2 | 4% | 0.087 | 81% |

4). KG_fragment 46

KG_fragment 46/Maize EST 62096689.f01 encodes a protein that is homologous to a Cupin family protein of rice (GenBank Accession: ABF95817.1). The top 10 homologous sequences identified in the BlastX query are presented in Table 10.

Table 10. BLASTX search results of KG_fragment 26/Hyseq EST 62096689.f01

| Accession | Description | Score | E-value | % Identities |
|---|---|---|---|---|
| ABF95817.1 | Cupin family protein, expressed [Oryza sativa (japonica cultivar-group)] | 313 | 1.00E-98 | 76 |
| ABK80758.1 | 7S globulin precursor [Ficus pumila var. awkeotsang] | 294 | 4.00E-90 | 67 |
| NP_001050038.1 | Os03g0336100 [Oryza sativa (japonica cultivar-group)] | 313 | 1.00E-98 | 76 |
| CAO43605.1 | unnamed protein product [Vitis vinifera] | 280 | 5.00E-87 | 63 |
| BAA06186.1 | preproMP27-MP32 [Cucurbita cv. Kurokawa Amakuri] | 278 | 6.00E-87 | 61 |
| AAT40548.1 | Putative vicilin, identical [Solanum demissum] | 291 | 5.00E-84 | 61 |
| CAN60323.1 | hypothetical protein [Vitis vinifera] | 263 | 7.00E-82 | 63 |
| AAC15238.1 | globulin-like protein [Daucus carota] | 250 | 4.00E-76 | 57 |
| NP_180416.1 | cupin family protein [Arabidopsis thaliana] | 253 | 5.00E-76 | 61 |
| ABD33075.1 | Cupin region [Medicago truncatula] | 249 | 2.00E-72 | 55 |

The CDS sequence of the gene corresponding to KG_Fragment 46 is shown in SEQ ID NO 30 and the translated amino acid sequence is shown in SEQ ID NO 48.

Identification of the promoter region of KG46

For our promoter identification purposes, the sequence upstream of the start codon of the predicted KG_Fragment 46 gene was defined as the promoter p-KG46. To identify this predicted promoter region, the sequence of 62096689.f_o1 was mapped to the BASF Plant Science proprietary genomic DNA sequence database, PUB_tigr_maize_genomic_partial_5.0.nt. One maize genomic DNA sequence, AZM5_23539 (2908 bp) was identified (SEQ ID NO: 84). This 2908bp sequence harbored the predicted CDS of the corresponding gene to KG_Fragment 24 and about 600bp upstream sequence of the ATG start codon of this gene (SEQ ID NO: 84).

Isolation of the promoter region of KG46 by PCR amplification

The putative promoter region was isolated via genomic PCR using the following sequence specific primers:

Forward primer: CATTGTTATACATCGGTGATG (SEQ ID NO: 160)
Reverse primer: CCTAGCTGGCTTCTTCCAAGC (SEQ ID NO: 161) The expected 563bp fragment was amplified from maize genomic DNA, and named as promoter KG46 (p-KG46). Sequence of p-KG46 is shown in SEQ ID NO:12.

BLASTN results of p_KG46

The top 10 homologous sequences identified in the BlastN query are presented in Table 11.

Table 11. BlastN results of p_KG46

| Accession | Description | Max score | Total score | Query coverage | E value | Max ident |
|---|---|---|---|---|---|---|
| EU953111.1 | Zea mays clone 1383292 unknown mRNA | 156 | 156 | 15% | 1.00E-34 | 100% |
| AY105246.1 | Zea mays PCO130570 mRNA sequence | 138 | 138 | 13% | 3.00E-29 | 100% |
| EU971630.1 | Zea mays clone 368362 unknown mRNA | 122 | 122 | 37% | 2.00E-24 | 75% |
| AY455286.1 | Zea mays chloroplast phytoene synthase (Y1) gene, complete cds; nuclear gene for chloroplast product | 107 | 107 | 22% | 5.00E-20 | 81% |
| EU968175.1 | Zea mays clone 316213 unknown mRNA | 64.4 | 64.4 | 11% | 5.00E-07 | 85% |
| AY664417.1 | Zea mays cultivar Mo17 locus 9002, complete sequence | 46.4 | 46.4 | 24% | 0.15 | 71% |
| AP008213.1 | Oryza sativa (japonica cultivar-group) genomic DNA, chromosome 7 | 44.6 | 44.6 | 8% | 0.51 | 81% |
| EU970588.1 | Zea mays clone 347636 unknown mRNA | 42.8 | 42.8 | 6% | 1.8 | 89% |
| EU958640.1 | Zea mays clone 1706905 unknown mRNA | 42.8 | 42.8 | 5% | 1.8 | 90% |
| CP000964.1 | Klebsiella pneumoniae 342, complete genome | 42.8 | 42.8 | 6% | 1.8 | 88% |

5). KG_fragment 49

KG_fragment 49/Maize EST 62158447.f01 encodes a protein that is homologous to a hypothetical protein OsI_010295 of rice (GenBank Accession: EAY89062). The top 10 homologous sequences identified in the BlastX query are presented in Table 12.

Table 12. BLASTX search results of KG_fragment 49/Hyseq EST 62158447.f01

| Accession | Description | Score | E-value | % Identities |
|---|---|---|---|---|
| EAY89062 | hypothetical protein OsI_010295 [Oryza sativa (indica cultivar-group)] | 1021 | 0.0 | 87 |
| ABF94669 | dnaK protein, expressed [Oryza sativa (japonica cultivar-group)] | 1020 | 0.0 | 87 |
| CAN68225 | hypothetical protein [Vitis vinifera] | 776 | 0.0 | 65 |
| CAO71160 | unnamed protein product [Vitis vinifera] | 776 | 0.0 | 66 |
| NP_180771 | HSP70T-2; ATP binding [Arabidopsis thaliana] | 754 | 0.0 | 64 |
| AAM67201 | 70kD heat shock protein [Arabidopsis thaliana] | 749 | 0.0 | 64 |
| ACC93947 | heat-shock protein 70 [Hevea brasiliensis] | 297 | 3.00E-78 | 35 |

(continued)

| Accession | Description | Score | E-value | % Identities |
|---|---|---|---|---|
| XP_001785822 | predicted protein [Physcomitrella patens subsp. patens] | 289 | 7.00E-76 | 35 |
| XP_001783048 | predicted protein [Physcomitrella patens subsp. patens] | 288 | 1.00E-75 | 35 |
| XP_001781229 | predicted protein [Physcomitrella patens subsp. patens] | 288 | 2.00E-75 | 35 |

The CDS sequence of the gene corresponding to KG_Fragment 49 is shown in SEQ ID NO: 26 and the translated amino acid sequence is shown in SEQ ID NO: 44.

Identification of the promoter region of KG49

For our promoter identification purposes, the sequence upstream of the start codon of the predicted KG_Fragment 49 gene was defined as the promoter p-KG49. To identify this predicted promoter region, the sequence of 62001211.f_o1 was mapped to the BASF Plant Science proprietary genomic DNA sequence database, PUB_tigr_maize_genomic_partial_5.0.nt. The reverse complement sequence of a maize genomic DNA sequence, AZM5_34102 (1719 bp) was identified (SEQ ID NO: 80). This 1719 bp sequence harbored partial predicted CDS of the corresponding gene to KG_Fragment 49 and about 1.2 kb upstream sequence of the ATG start codon of this gene (SEQ ID NO: 80).

Isolation of the promoter region of KG49 by PCR amplification

The putative promoter region was isolated via genomic PCR using the following sequence specific primers:

Forward primer: GAGCGACCTCGGACTCAGCGGCT (SEQ ID NO: 162)
Reverse primer: CCTACAAACAATATTGCATCAG (SEQ ID NO: 163) The expected 1188bp fragment was amplified from maize genomic DNA, and named as promoter KG49 (p-KG49). Sequence of p-KG49 is shown in SEQ ID NO:8 .

BLASTN results of p_KG49

The 2 plant homologous sequences identified in the BlastN query are presented in Table 13.

Table 13. BlastN results of p_KG49

| Accession | Description | Max score | Total score | Query coverage | E value | Max ident |
|---|---|---|---|---|---|---|
| EU957595.1 | Zea mays clone 1598693 unknown mRNA | 237 | 237 | 12% | 9.00E-59 | 95% |
| EU966687.1 | Zea mays clone 296333 unknown mRNA | 93.3 | 93.3 | 14% | 2.00E-15 | 72% |

6). KG_fragment 56

KG_fragment 56 has no hits to the BPS in-house Hyseq EST database, but has 100% identities to a sequence disclosed in the patent application, pat_US20040034888A1_3514.

KG_Fragment56/pat_US20040034888A1_3514 encodes a protein that is homologous to a hypothetical protein Os02g0158900 of rice (GenBank Accession: NP_001045960.1). The top 10 homologous sequences identified in the BlastX query are presented in Table 14.

Table 14. BLASTX search results of KG_fragment 56

| Accession | Description | Score | E-value | % Identities |
|---|---|---|---|---|
| NP_001045960.1 | Os02g0158900 [Oryza sativa (japonica cultivar-group)] | 237 | e-128 | 76 |
| EAZ21814.1 | hypothetical protein OsJ_005297 [Oryza sativa (japonica cultivar-group)] | 233 | e-125 | 76 |
| CAO62717.1 | unnamed protein product [Vitis vinifera] | 205 | 2.00E-93 | 60 |
| CAN64662.1 | hypothetical protein [Vitis vinifera] | 202 | 3.00E-92 | 59 |
| AAF66638.1 | AF143742_1 SNF4 [Lycopersicon esculentum] | 167 | 6.00E-74 | 64 |
| AAA91175.1 | Pv42p | 103 | 2.00E-72 | 62 |
| AAO61675.1 | SNF4b [Medicago truncatula] | 111 | 3.00E-72 | 67 |
| NP_172985.1 | CBS domain-containing protein [Arabidopsis thaliana] | 108 | 3.00E-69 | 65 |
| XP_001761144.1 | predicted protein [Physcomitrella patens subsp. patens] | 163 | 1.00E-66 | 50 |
| BAC42835.1 | unknown protein [Arabidopsis thaliana] | 191 | 7.00E-64 | 46 |

The CDS sequence of the gene corresponding to KG_Fragment 56 is shown in SEQ ID NO:21 and the translated amino acid sequence is shown in SEQ ID NO:39.

Identification of the promoter region of KG56

For our promoter identification purposes, the sequence upstream of the start codon of the predicted KG_Fragment 56 gene was defined as the promoter p-KG56. To identify this predicted promoter region, the sequence of 62001211.f_o1 was mapped to the BASF Plant Science proprietary genomic DNA sequence database, PUB_zmdb_genomesurveyseqs.nt, One maize genomic DNA sequence, ZmGSStuc11-12-04.2541.1 (8495bp) was identified (SEQ ID NO: 75). The first 4.2 kb of ZmGSStuc11-12-04.254.1.1 sequence harbored the predicted CDS of the corresponding gene to KG_Fragment 56 and more than 2 kb upstream sequence of the ATG start codon of this gene (SEQ ID NO: 75).

Isolation of the promoter region of KG56 by PCR amplification

The putative promoter region was isolated via genomic PCR using the following sequence specific primers:

Forward primer: GATTCAGAACATCTGGTCAG (SEQ ID NO: 164)
Reverse primer: AGGTTTAGCGAACAAGGC (SEQ ID NO: 165) The expected 1945bp fragment was amplified from maize genomic DNA, and named as promoter KG56 (p-KG56). Sequence of p-KG56 is shown in SEQ ID NO:3.

BLASTN results of p_KG56

The top 15 homologous sequences identified in the BlastN query are presented in Table 15.

Table 15. BlastN results of p_KG56

| Accession | Description | Max score | Total score | Query coverage | E value | Max ident |
|---|---|---|---|---|---|---|
| EU971086.1 | Zea mays clone 357153 unknown mRNA | 1088 | 1895 | 54% | 0 | 99% |
| AP008208.1 | Oryza sativa (japonica cultivar-group) genomic DNA, chromosome 2 | 437 | 956 | 51% | 8.00E-119 | 87% |
| AP004843.2 | Oryza sativa Japonica Group genomic DNA, chromosome 2, BAC clone:B1103G11 | 437 | 956 | 51% | 8.00E-119 | 87% |
| NM_001052494.1 | Oryza sativa (japonica cultivar-group) Os02g0158800 (Os02g0158800) mRNA, complete cds | 430 | 975 | 41% | 1.00E-116 | 87% |
| AK119177.1 | Oryza sativa Japonica Group cDNA clone:001-037-G06, full insert sequence | 430 | 975 | 41% | 1.00E-116 | 87% |
| AK065389.1 | Oryza sativa Japonica Group cDNA clone:J013021 B10, full insert sequence | 430 | 975 | 41% | 1.00E-116 | 87% |
| BT041386.1 | Zea mays full-length cDNA clone ZM_BFc0117N09 mRNA, complete cds | 242 | 670 | 41% | 4.00E-60 | 83% |
| EU976055.1 | Zea mays clone 509800 unknown mRNA | 239 | 659 | 41% | 4.00E-59 | 82% |
| AP008212.1 | Oryza sativa (japonica cultivar-group) genomic DNA, chromosome 6 | 239 | 731 | 43% | 4.00E-59 | 84% |
| AP005395.3 | Oryza sativa Japonica Group genomic DNA, chromosome 6, PAC clone:P0623A10 | 239 | 683 | 43% | 4.00E-59 | 84% |
| NM_001064941.1 | Oryza sativa (japonica cultivar-group) Os06g0687400 (Os06g0687400) mRNA, partial cds | 237 | 663 | 41% | 2.00E-58 | 83% |
| AK072400.1 | Oryza sativa Japonica Group cDNA clone:J023078C17, full insert sequence | 237 | 663 | 41% | 2.00E-58 | 83% |
| AK060934.1 | Oryza sativa Japonica Group cDNA clone:006-201-B09, full insert sequence | 237 | 656 | 41% | 2.00E-58 | 83% |
| AP001298.1 | Arabidopsis thaliana genomic DNA, chromosome 3, BAC clone:F20C19 | 221 | 406 | 46% | 1.00E-53 | 77% |
| BT009221.1 | Triticum aestivum clone wle1n.pk0074.b4:fis, full insert mRNA sequence | 210 | 614 | 41% | 2.00E-50 | 82% |

7). KG_fragment 103

KG_fragment 103/Maize EST ZM07MC01323_57619299 encodes a Maize Cytochrome P450 78A1 protein (GenBank Accession: NP_001106069.1). The top 10 homologous sequences identified in the BlastX query are presented in Table 16.

Table16 .BLASTXsearch results of KG_fragment_103/EST ZM07MC01323_57619299

| Accession | Description | Score | E-value | % Identities |
|---|---|---|---|---|
| NP_001106069.1 | Cytochrome P450 78A1 [Zea mays] | 1057 | 0.0 | 100 |
| CAO70823.1 | unnamed protein product [Vitis vinifera] | 370 | 0.0 | 72 |
| CAN73323.1 | hypothetical protein [Vitis vinifera] | 367 | 0.0 | 71 |
| EAY78409.1 | hypothetical protein OsI_032368 [Oryza sativa (indica cultivar-group)] | 635 | 0.0 | 84 |
| NP_001064552.1 | Os10g0403000 [Oryza sativa (japonica cultivar-group)] | 634 | 0.0 | 84 |
| BAC76730.1 | cytochrome P450 78A11 [Oryza sativa Japonica Group] | 632 | 0.0 | 83 |
| EAY79271.1 | hypothetical protein OsI_033230 [Oryza sativa (indica cultivar-group)] | 634 | e-179 | 84 |
| 065012 | C78A4_PINRA Cytochrome P450 78A4 | 354 | e-171 | 65 |
| CAO71766.1 | unnamed protein product [Vitis vinifera] | 218 | e-163 | 70 |
| XP_001771134.1 | predicted protein [Physcomitrella patens subsp. patens] | 180 | e-158 | 54 |

The CDS sequence of the gene corresponding to KG_Fragment 103 is shown SEQ ID NO: 31 and the translated amino acid sequence is shown in SEQ ID NO: 49.

Identification of the promoter region of KG103

For our promoter identification purposes, the sequence upstream of the start codon of the predicted KG_Fragment 103 gene was defined as the promoter p-KG103. To identify this predicted promoter region, the sequence of EST ZM07MC01323_57619299 was mapped to the BASF Plant Science proprietary genomic DNA sequence database, PUB_zmdb_genomesurveyseqs.nt. The reverse complement sequence of a maize genomic DNA sequence, ZmGSStuc11-12-04.9475.1 (5105 bp) was identified (SEQ ID NO: 85). This 5105bp sequence harbored the predicted CDS of the corresponding gene to KG_Fragment 103 and about 1.2 kb upstream sequence of the ATG start codon of this gene.

Isolation of the promoter region of KG103 by PCR amplification

The putative promoter region was isolated via genomic PCR using the following sequence specific primers:

Forward primer: ATCATCACCCTACCCCGAGCT (SEQ ID NO: 166)
Reverse primer: GACGAGTTGTTCTGGCTAG (SEQ ID NO: 167) The expected 991bp fragment was amplified from maize genomic DNA, and named as promoter KG103 (p-KG103). Sequence of p-KG103 is shown in SEQ ID NO:13.

BLASTN results of p_KG103

The top 25 homologous sequences identified in the BlastN query are presented in Table 17.

Table 17. BlastN results of p_KG103

| Accession | Description | Max score | Total score | Query coverage | E value | Max ident |
|---|---|---|---|---|---|---|
| AC157319.2 | Zea mays clone ZMMBBb-136E2, complete sequence | 1079 | 3747 | 66% | 0 | 96% |

(continued)

| Accession | Description | Max score | Total score | Query coverage | E value | Max ident |
|---|---|---|---|---|---|---|
| AY530952.1 | Zea mays unknown (Z576C20.2), putative heme oxygenase 1 (Z576C20.3), anthocyanin biosynthesis regulatory protein PI1_B73 (Z576C20.4), putative growth-regulating factor 1 (Z576C20.6), and putative aminoalcoholphosphotr ansferase (Z576C20.14) genes, complete cds; and putative receptor protein kinase (Z576C20.21) gene, partial cds | 1068 | 2126 | 66% | 0 | 96% |
| EU952200.1 | Zea mays clone 1221105 unknown mRNA | 1058 | 1058 | 66% | 0 | 95% |
| EU338354.1 | Zea mays cultivar W22 bz gene locus, complete sequence | 1050 | 5123 | 68% | 0 | 95% |
| AF391808.3 | Zea mays cultivar McC bz locus region | 1050 | 5117 | 68% | 0 | 95% |
| AC165176.2 | Zea mays clone ZMMBBb-177G21, complete sequence | 1031 | 1.20E+04 | 66% | 0 | 94% |
| AY883559.2 | Zea mays cultivar inbred line B73 teosinte glume architecture 1 (tga1) gene, complete cds | 1018 | 3526 | 66% | 0 | 94% |
| AF466646.1 | Zea mays putative transposase (Z195D10.1) gene, partial cds; glycyl-tRNA synthetase (Z195D10.2), ornithine carbamoyltransferase (Z195D10.3), putative gag protein (Z195D10.5), putative SET-domain transcriptional regulator (Z195D10.7), putative oxysterol-binding protein (Z195D10.8), putative polyprotein (Z195D10.9), putative oxysterol-binding protein (Z195D10.10), putative gag-pol polyprotein (Z195D10.11), putative phosphatidylinositol-4-phosphate-5-kinase (Z195D10.12), hypothetical protein (Z195D10.15), putative gag-pol polyprotein (Z195D10.16), putative polyprotein (Z195D10.17), putative retrotransposon protein (Z195D10.18), and prpol (Z195D10.19) genes, complete cds; and putative teosinte branched2 (Z195D10.20) gene, partial cds | 1007 | 1007 | 66% | 0 | 94% |
| AC152495.1 | Zea mays BAC clone Z486N13, complete sequence | 1003 | 1984 | 66% | 0 | 94% |
| AF123535.1 | Zea mays alcohol dehydrogenase 1 (adh1) gene, adh1-F allele, complete cds | 991 | 1964 | 67% | 0 | 93% |

(continued)

| Accession | Description | Max score | Total score | Query coverage | E value | Max ident |
|---|---|---|---|---|---|---|
| AY691949.1 | Zea mays alcohol dehydrogenase 1 (adh1A) gene, complete cds; Fourf copia_LTR and Huck gypsy_LTR retrotransposons, complete sequence; Opie2 copia_LTR retrotransposon Zeon gypsy_LTR and Opie1 copia_LTR retrotransposons, complete sequence; Ji copia_LTR retrotransposon, complete sequence; and unknown protein (adh1B), cyclin H-1 (adh1C), unknown protein (adh1D), hypothetical protein (adh1E), and unknown protein (adh1 F) genes, complete cds | 991 | 1970 | 67% | 0 | 93% |
| DQ417752.1 | Zea mays B73 pathogenesis-related protein 2 and GASA-like protein genes, complete cds | 984 | 5530 | 66% | 0 | 93% |
| AF050440.1 | Zea mays retrotransposon Huck-2 3' LTR, partial sequence | 982 | 982 | 66% | 0 | 93% |
| DQ002408.1 | Zea mays gypsy retrotransposon huck, and copia retrotransposon ji, complete sequence; and helitron Mo17_14594, complete sequence | 976 | 3501 | 66% | 0 | 93% |
| U68404.1 | Zea mays retrotransposon Huck-2 5' LTR and primer binding site DNA sequence | 973 | 973 | 66% | 0 | 93% |
| AY530950.1 | Zea mays putative zinc finger protein (Z438D03.1), unknown (Z438D03.5), epsilon-COP (Z438D03.6), putative kinase (Z438D03.7), unknown (Z438D03.25), and C1-B73 (Z438D03.27) genes, complete cds | 971 | 4162 | 67% | 0 | 93% |
| AC160211.1 | Genomic seqeunce for Zea mays BAC clone ZMMBBb0448F23, complete sequence | 969 | 4518 | 66% | 0 | 93% |
| AC157487.1 | Genomic sequence for Zea mays clone ZMMBBb0614J24, from chromosome 8, complete sequence | 966 | 6419 | 66% | 0 | 93% |
| AY530951.1 | Zea mays putative growth-regulating factor 1 (Z214A02.12), putative 40S ribosomal protein S8 (Z214A02.25), and putative casein kinase I (Z214A02.27) genes, complete cds | 964 | 4254 | 66% | 0 | 93% |
| AY664416.1 | Zea mays cultivar Mo17 locus bz, complete sequence | 958 | 3019 | 66% | 0 | 92% |
| AY555142.1 | Zea mays BAC clone c573F08, complete sequence | 951 | 2719 | 66% | 0 | 92% |
| AY664419.1 | Zea mays cultivar Mo17 locus 9009, complete sequence | 951 | 4061 | 66% | 0 | 92% |
| AC165174.2 | Zea mays clone ZMMBBb-127F19, complete sequence | 921 | 1836 | 66% | 0 | 91% |
| AC165173.2 | Zea mays clone ZMMBBb-125O19, complete sequence | 921 | 2326 | 66% | 0 | 91% |
| DQ493649.1 | Zea mays cultivar Coroico bz locus region | 915 | 3472 | 66% | 0 | 91% |

8). KG_fragment 119

KG_fragment 119/Maize EST ZM07MC15086_59463108 encodes a protein that is homologous to a hypothetical protein Os09g0433900 of rice (GenBank Accession: NP_001063248). The top 10 homologous sequences identified in the BlastX query are presented in Table 18

Table 18. BLASTX search results of KG_fragment 119/Hyseq EST ZM07MC15086_59463108

| Accession | Description | Score | E-value | % Identities |
|---|---|---|---|---|
| NP_001063248 | Os09g0433900 [Oryza sativa (japonica cultivar-group)] | 696 | 0.0 | 67 |
| EAZ09214 | hypothetical protein OsI_030446 [Oryza sativa (indica cultivar-group)] | 657 | 0.0 | 67 |
| EAZ44840 | hypothetical protein OsJ_028323 [Oryza sativa (japonica cultivar-group)] | 611 | e-173 | 61 |
| AAV64199 | putative alanine aminotransferase [Zea mays] | 571 | e-161 | 56 |
| AAV64237 | putative alanine aminotransferase [Zea mays]. | 570 | e-160 | 56 |
| BAC79995 | putative alanine aminotransferase [Oryza sativa Japonica Group] | 559 | e-157 | 60 |
| EAZ40671 | hypothetical protein OsJ_024154 [Oryza sativa (japonica cultivar-group)] | 556 | e-156 | 58 |
| EAZ04721 | hypothetical protein OsI_025953 [Oryza sativa (indica cultivar-group)] | 555 | e-156 | 58 |
| CAO45546 | unnamed protein product [Vitis vinifera] | 555 | e-156 | 58 |
| CAA49199 | alanine aminotransferase [Panicum miliaceum] | 553 | e-155 | 57 |

The CDS sequence of the gene corresponding to KG_Fragment 119 is shown in SEQ ID NO: 32 and the translated amino acid sequence is shown in SEQ ID NO:50.

Identification of the promoter region of KG119

For our promoter identification purposes, the sequence upstream of the start codon of the predicted KG_Fragment 119 gene was defined as the promoter p-KG119. To identify this predicted promoter region, the sequence of ZM07MC15086_59463108 was mapped to the BASF Plant Science proprietary genomic DNA sequence database, PUB_tigr_maize_genomic_partial_5.0.nt. One maize genomic DNA sequence, AZM5_10092 (8208bp SEQ ID NO: 86) was identified. The reverse complement sequence of this sequence harbored the predicted CDS of the corresponding gene to KG_Fragment 119 and more than 2 kb upstream sequence of the ATG start codon of this gene (SEQ ID NO: 86).

Isolation of the promoter region of KG119 by PCR amplification

The putative promoter region was isolated via genomic PCR using the following sequence specific primers:

Forward primer: CTTCCGATAAAAATATTTGGAAC (SEQ ID NO: 168)
Reverse primer: GTACGACATGGCGCGTCGG (SEQ ID NO: 169) The expected 2519bp fragment was amplified from maize genomic DNA, and anotated as promoter KG119 (p-KG119). Sequence of p-KG119 is shown in SEQ ID NO: 14

BLASTN results of p_KG119

The top 15 homologous sequences identified in the BlastN query are presented in Table 19.

Table 19. BlastN results of p_KG119

| Accession | Description | Max score | Total score | Query coverage | E value | Max ident |
|---|---|---|---|---|---|---|
| EU966511.1 | Zea mays clone 294961 unknown mRNA | 821 | 1032 | 23% | 0 | 99% |
| AF215823.2 | Zea mays T cytoplasm male sterility restorer factor 2 (rf2a) gene, rf2a-B73 allele, complete cds | 545 | 545 | 15% | 3.00E-151 | 92% |
| AC157977.1 | Genomic sequence for Zea mays chromosome 8 BAC clone ZMMBBb0284N04, complete sequence | 535 | 535 | 15% | 5.00E-148 | 91% |
| EU957455.1 | Zea mays clone 1592915 unknown mRNA | 531 | 531 | 14% | 6.00E-147 | 92% |
| AY662985.1 | Zea luxurians YZ1 (yz1) gene, complete cds; transposons mPIF-like element and frequent flyer, complete sequence; and NADPH-dependent reductase (a1) gene, partial cds | 504 | 716 | 21% | 9.00E-139 | 89% |
| AC165178.2 | Zea mays clone ZMMBBb-272P17, complete sequence | 497 | 497 | 14% | 1.00E-136 | 90% |
| EF659468.1 | Zea mays clone BAC b0288K09 AP2 domain transcription factor (Rap2.7) gene, partial cds | 484 | 621 | 14% | 8.00E-133 | 90% |
| AJ005343.1 | Zea mays Ama gene encoding single-subunit RNA polymerase | 464 | 464 | 14% | 8.00E-127 | 89% |
| AC165171.2 | Zea mays clone CH201-145P10, complete sequence | 462 | 462 | 15% | 3.00E-126 | 87% |
| AF466646.1 | Zea mays putative transposase (Z195D10.1) gene, partial cds; glycyl-tRNA synthetase (Z195D10.2), ornithine carbamoyltransferase (Z195D10.3), putative gag protein (Z195D10.5), putative SET-domain transcriptional regulator (Z195D10.7), putative oxysterol-binding protein (Z195D10.8), putative polyprotein (Z195D10.9), putative oxysterol-binding protein (Z195D10.10), putative gag-pol polyprotein (Z195D10.11), putative phosphatidylinositol-4-phosphate-5-kinase (Z195D10.12), hypothetical protein (Z195D10.15), putative gag-pol polyprotein (Z195D10.16), putative polyprotein (Z195D10.17), putative retrotransposon protein (Z195D10.18), and prpol (Z195D10.19) genes, complete cds; and putative teosinte branched2 (Z195D10.20) gene, partial cds | 461 | 606 | 14% | 9.00E-126 | 87% |
| AY789036.1 | Zea mays subsp. parviglumis floricaula/leafy-like 2 (zfl2) gene, complete cds | 461 | 461 | 14% | 9.00E-126 | 88% |
| AC165174.2 | Zea mays clone ZMMBBb-127F19, complete sequence | 459 | 959 | 19% | 3.00E-125 | 88% |
| AF448416.1 | Zea mays B73 chromosome 9S bz genomic region | 459 | 459 | 14% | 3.00E-125 | 87% |

(continued)

| Accession | Description | Max score | Total score | Query coverage | E value | Max ident |
|---|---|---|---|---|---|---|
| AF416310.1 | Zea mays clone mPIF268 mPIF miniature inverted-repeat transposable element | 459 | 459 | 14% | 3.00E-125 | 88% |
| DQ493647.1 | Zea mays cultivar NalTel bz locus region | 453 | 453 | 14% | 1.00E-123 | 86% |

9). KG_fragment 129

KG_fragment 129/maize EST 62092959.f01 encodes a protein that is homologous to a maize unknown protein (GenBank Accession: ACF78165.1). The top 10 homologous sequences identified in the BlastX query are presented in Table 20.

Table 20. BLASTX search results of KG_fragment 129/Hyseq EST 62092959.f01

| Accession | Description | Score | E-value | % identities |
|---|---|---|---|---|
| ACF78165.1 | unknown[Zea Mays]. | 513 | e-143 | 91 |
| ACF83516.1 | unknown [Zea mays] | 401 | e-110 | 69 |
| ACF86030.1 | unknown [Zea mays] | 243 | 7e-96 | 69 |
| ACF78865.1 | unknown [Zea mays] | 243 | 1 e-84 | 69 |
| EAY82651.1 | hypothetical protein OsI_036610 | 129 | 3e-42 | 45 |
| NP_001066495.1 | Os12g0247700 [Oryza sativa (japonica cultivar-group)] | 121 | 1 e-39 | 44 |
| NP_001066367.1 | Os12g0198700 [Oryza sativa (japonica cultivar-group)] | 88 | 2e-35 | 47 |
| ABE11623.1 | unknown [Oryza sativa (japonica cultivar-group)] | 102 | 6e-34 | 41 |
| ABS82785.1 | jasmonate-induced protein [Triticum aestivum] | 92 | 1e-32 | 51 |
| AAR20919.1 | jasmonate-induced protein [Triticum aestivum] | 91 | 3e-32 | 50 |

The CDS sequence of the gene corresponding to KG_Fragment 129 is shown in SEQ ID NO: 22 and the translated amino acid sequence is shown in SEQ ID NO:40.

Identification of the promoter region of KG129

For our promoter identification purposes, the sequence upstream of the start codon of the predicted KG_Fragment 129 gene was defined as the promoter p-KG129. To identify this predicted promoter region, the sequence of 62092959.f_o1 was mapped to the BASF Plant Science proprietary genomic DNA sequence database, PUB_tigr_maize_genomic_partial_5.0.nt. One maize genomic DNA sequence, AZM5_91706 (2131 bp) was identified (SEQ ID NO: 76). This 2131bp sequence harbored the predicted CDS of the corresponding gene to KG_Fragment 129 and about 600bp upstream sequence of the ATG start codon of this gene.

Isolation of the promoter region of KG129by PCR amplification

The putative promoter region was isolated via genomic PCR using the following sequence specific primers:

Forward primer: GCCAGTGCTAATGATATTTA (SEQ ID NO: 170)
Reverse primer: ATGCACCTACTCGGCGGTG (SEQ ID NO: 171) The expected 512bp fragment was amplified from maize genomic DNA, and annotated as promoter KG129 (p-KG129). Sequence of p-KG129 is shown in SEQ ID NO:4.

BLASTN results of p_KG129

The top 20 homologous sequences identified in the BlastN query are presented in Table 21.

Table 21. BlastN results of p_KG129

| Accession | Description | Max score | Total score | Query coverage | E value | Max ident |
|---|---|---|---|---|---|---|
| EU241905.1 | Zea mays ZCN14 (ZCN14) gene, complete cds | 230 | 353 | 38% | 6.00E-57 | 86% |
| AY682272.1 | Zea mays subsp. mexicana barren stalk 1 gene, promoter region I | 228 | 465 | 37% | 2.00E-56 | 86% |
| AY682271.1 | Zea mays subsp. mexicana barren stalk 1 gene, promoter region I | 228 | 465 | 37% | 2.00E-56 | 86% |
| AY682262.1 | Zea mays barren stalk 1 gene, promoter region I | 228 | 459 | 37% | 2.00E-56 | 86% |
| AY682258.1 | Zea mays barren stalk 1 gene, promoter region I | 228 | 459 | 37% | 2.00E-56 | 86% |
| AY682256.1 | Zea mays barren stalk 1 gene, promoter region I | 228 | 459 | 37% | 2.00E-56 | 86% |
| AY743721.1 | Zea mays subsp. parviglumis cultivar INIFAP-JSG 374 barren stalk 1 gene, promoter I region | 228 | 459 | 37% | 2.00E-56 | 86% |
| AY682254.1 | Zea mays barren stalk 1 gene, promoter region I | 224 | 455 | 37% | 2.00E-55 | 86% |
| AY743723.1 | Zea mays subsp. parviglumis cultivar CIMMYT-11355 barren stalk 1 gene, promoter I region | 215 | 283 | 37% | 1.00E-52 | 85% |
| AY753906.1 | Zea mays subsp. parviglumis barren stalk 1 gene, promoter I region | 206 | 206 | 37% | 6.00E-50 | 84% |
| AY683001.1 | Zea mays cultivar B73 barren stalk1 (BA1) gene, complete cds | 201 | 527 | 38% | 3.00E-48 | 85% |
| AY682281.1 | Zea mays subsp. parviglumis barren stalk 1 gene, promoter region I | 199 | 541 | 37% | 9.00E-48 | 85% |
| AY682274.1 | Zea mays subsp. parviglumis barren stalk 1 gene, promoter region I | 199 | 525 | 37% | 9.00E-48 | 85% |
| AY682273.1 | Zea mays subsp. parviglumis barren stalk 1 gene, promoter region I | 199 | 525 | 37% | 9.00E-48 | 85% |
| AY682270.1 | Zea mays subsp. mexicana barren stalk 1 gene, promoter region I | 199 | 525 | 37% | 9.00E-48 | 85% |
| AY682269.1 | Zea mays subsp. mexicana barren stalk 1 gene, promoter region I | 199 | 525 | 37% | 9.00E-48 | 85% |
| AY682268.1 | Zea mays barren stalk 1 gene, promoter region I | 199 | 525 | 37% | 9.00E-48 | 85% |
| AY682267.1 | Zea mays barren stalk 1 gene, promoter region I | 199 | 525 | 37% | 9.00E-48 | 85% |
| AY682266.1 | Zea mays barren stalk 1 gene, promoter region I | 199 | 525 | 37% | 9.00E-48 | 85% |
| AY682265.1 | Zea mays barren stalk 1 gene, promoter region I | 199 | 525 | 37% | 9.00E-48 | 85% |

EXAMPLE 5:

PLACE ANALYSIS OF THE PROMOTERS

*Cis*-acting motifs in the promoter regions were identified using PLACE (a database of Plant Cis-acting Regulatory DNA elements) using the Genomatix database suite.

1.) p-KG24

PLACE analysis results of p-KG24 are listed in Table 22. No TATA box motif is found in this promoter, but there are 2 CAAT Box motifs at nucleotide position 191 - 195 and 247-251 of the forward strand, respectively. These CAAT Box motifs are distal from the 3' end of the promoter and therefore may not be functional motifs.

Table 22. PLACE analysis results of the 1507bp promoter of p-KG24

| IUPAC Family | IUPAC | Start pos. | End pos. | Strand | Mismatches | Score | Sequence |
|---|---|---|---|---|---|---|---|
| FAM156 | L1BOXATPDF1 | 2 | 9 | - | 0 | 1 | TAAATGTA |
| FAM290 | GT1GMSCAM4 | 56 | 61 | + | 0 | 1 | GAAAAA |
| FAM311 | EECCRCAH1 | 68 | 74 | - | 0 | 1 | GATTTAC |
| FAM087 | BOXIINTPATPB | 76 | 81 | + | 0 | 1 | ATAGAA |
| FAM303 | OSE1ROOTNODULE | 90 | 96 | - | 0 | 1 | AAAGATG |
| FAM012 | IBOXCORE | 126 | 132 | + | 0 | 1 | GATAACT |
| FAM267 | NTBBF1ARROLB | 130 | 136 | + | 0 | 1 | ACTTTAG |
| FAM267 | TAAAGSTKST1 | 131 | 137 | - | 0 | 1 | TCTAAAG |
| FAM272 | SV40COREEN HAN | 143 | 150 | + | 0 | 1 | GTGGAATG |
| FAM322 | BIHD1OS | 149 | 153 | + | 0 | 1 | TGTCA |
| FAM027 | -10PEHVPSBD | 154 | 159 | + | 0 | 1 | TATTCT |
| FAM100 | CCAATBOX1 | 191 | 195 | + | 0 | 1 | CCAAT |
| FAM305 | ANAERO1CONSENSUS | 212 | 218 | + | 0 | 1 | AAACAAA |
| FAM039 | AACACOREOSGLUB1 | 213 | 219 | + | 0 | 1 | AACAAAC |
| FAM325 | MYBCOREATCYCB1 | 217 | 221 | + | 0 | 1 | AACGG |
| FAM302 | SORLIP2AT | 220 | 230 | + | 0 | 1 | GGGGCCTTATT |
| FAM310 | CPBCSPOR | 227 | 232 | + | 0 | 1 | TATTAG |
| FAM311 | EECCRCAH1 | 236 | 242 | - | 0 | 1 | GAATTCC |
| FAM100 | CCAATBOX1 | 247 | 251 | + | 0 | 1 | CCAAT |
| FAM311 | EECCRCAH1 | 272 | 278 | + | 0 | 1 | GATTTCC |
| FAM290 | GT1GMSCAM4 | 289 | 294 | + | 0 | 1 | GAAAAA |
| FAM006 | HDZIP2ATATHB2 | 318 | 326 | + | 0 | 1 | TAATAATTA |
| FAM170 | MYBGAHV | 330 | 336 | - | 0 | 1 | TAACAAA |
| FAM010 | WBOXNTCHN48 | 332 | 346 | - | 0 | 1 | GCTGACCTTTTAACA |
| FAM205 | PYRIMIDINEBOXOSRAM | 336 | 341 | - | 0 | 1 | CCTTTT |

(continued)

| IUPAC Family | IUPAC | Start pos. | End pos. | Strand | Mismatches | Score | Sequence |
|---|---|---|---|---|---|---|---|
| FAM010 | QELEMENTZMZM13 | 337 | 351 | + | 0 | 1 | AAAGGTCAGCTTCCC |
| FAM202 | -300ELEMENT | 364 | 372 | + | 0 | 1 | TGTAAAAGC |
| FAM302 | SITEIIATCYTC | 365 | 375 | - | 0 | 1 | TGGGCTTTTAC |
| FAM003 | MYBPLANT | 385 | 395 | - | 0 | 1 | AACCAAACAGA |
| FAM 171 | MYBPZM | 392 | 398 | - | 0 | 1 | CCCAACC |
| FAM302 | SITEIIATCYTC | 395 | 405 | + | 0 | 1 | TGGGCTGTGGC |
| FAM002 | SORLIP1AT | 399 | 411 | - | 0 | 1 | TTCACAGCCACAG |
| FAM290 | GT1GMSCAM4 | 409 | 414 | + | 0 | 1 | GAAAAA |
| FAM302 | SITEIIATCYTC | 423 | 433 | + | 0 | 1 | TGGGCTGTGAG |
| FAM290 | GT1GMSCAM4 | 439 | 444 | + | 0 | 1 | GAAAAA |
| FAM306 | ANAERO2CONSENSUS | 445 | 450 | - | 0 | 1 | AGCAGC |
| FAM003 | MYBPLANT | 480 | 490 | - | 0 | 1 | CACCAAACGGT |
| FAM325 | MYBCOREATCYCB1 | 481 | 485 | - | 0 | 1 | AACGG |
| FAM266 | MYB1AT | 492 | 497 | + | 0 | 1 | AAACCA |
| FAM099 | CCA1ATLHCB1 | 511 | 518 | + | 0 | 1 | AAAAATCT |
| FAM162 | LTRE1HVBLT49 | 544 | 549 | + | 0 | 1 | CCGAAA |
| FAM002 | SORLIP1AT | 545 | 557 | + | 0 | 1 | CGAAAAGCCACTA |
| FAM311 | EECCRCAH1 | 582 | 588 | + | 0 | 1 | GATTTGC |
| FAM311 | EECCRCAH1 | 596 | 602 | - | 0 | 1 | GACTTTC |
| FAM013 | DRE2COREZMRAB17 | 599 | 605 | - | 0 | 1 | ACCGACT |
| FAM290 | GT1GMSCAM4 | 611 | 616 | + | 0 | 1 | GAAAAA |
| FAM306 | ANAERO2CONSENSUS | 617 | 622 | - | 0 | 1 | AGCAGC |
| FAM010 | WBBOXPCWRKY1 | 675 | 689 | - | 0 | 1 | TTTGACTTTTGGCTT |
| FAM266 | MYB1AT | 687 | 692 | + | 0 | 1 | AAACCA |
| FAM003 | MYBPLANT | 688 | 698 | + | 0 | 1 | AACCAAACACA |

(continued)

| IUPAC Family | IUPAC | Start pos. | End pos. | Strand | Mismatches | Score | Sequence |
|---|---|---|---|---|---|---|---|
| FAM024 | 2SSEEDPROTBANAPA | 691 | 699 | + | 0 | 1 | CAAACACAC |
| FAM302 | SITEIIATCYTC | 715 | 725 | + | 0 | 1 | TGGGCCATTTA |
| FAM012 | IBOXCORE | 721 | 727 | - | 0 | 1 | GATAAAT |
| FAM311 | EECCRCAH1 | 738 | 744 | - | 0 | 1 | GATTTGC |
| FAM202 | -300ELEMENT | 748 | 756 | + | 0 | 1 | TGAAAAATT |
| FAM290 | GT1GMSCAM4 | 749 | 754 | + | 0 | 1 | GAAAAA |
| FAM270 | RAV1AAT | 758 | 762 | + | 0 | 1 | CAACA |
| FAM069 | SURECOREATSULTR11 | 800 | 806 | + | 0 | 1 | GAGACTA |
| FAM012 | IBOXCORE | 814 | 820 | + | 0 | 1 | GATAACT |
| FAM267 | NTBBF1ARROLB | 818 | 824 | + | 0 | 1 | ACTTTAT |
| FAM267 | TAAAGSTKST1 | 819 | 825 | - | 0 | 1 | TATAAAG |
| FAM307 | ANAERO3CONSENSUS | 828 | 834 | + | 0 | 1 | TCATCAC |
| FAM182 | OBP1ATGST6 | 893 | 903 | + | 0 | 1 | TACACTTTTGG |
| FAM302 | SITEIIATCYTC | 906 | 916 | + | 0 | 1 | TGGGCTCGGAG |
| FAM290 | GT1GMSCAM4 | 916 | 921 | + | 0 | 1 | GAAAAA |
| FAM304 | OSE2ROOTNODULE | 943 | 947 | + | 0 | 1 | CTCTT |
| FAM012 | IBOXCORE | 953 | 959 | + | 0 | 1 | GATAACA |
| FAM324 | CGCGBOXAT | 960 | 965 | + | 0 | 1 | ACGCGG |
| FAM324 | CGCGBOXAT | 960 | 965 | - | 0 | 1 | CCGCGT |
| FAM002 | SORLIP1AT | 967 | 979 | - | 0 | 1 | CGTTAGGCCACAT |
| FAM302 | SITEIIATCYTC | 984 | 994 | - | 0 | 1 | TGGGCCGGATT |
| FAM302 | UP1ATMSD | 988 | 998 | + | 0 | 1 | CGGCCCATTTA |
| FAM324 | CGCGBOXAT | 1018 | 1023 | + | 0 | 1 | ACGCGG |
| FAM324 | CGCGBOXAT | 1018 | 1023 | - | 0 | 1 | CCGCGT |
| FAM002 | RAV1BAT | 1023 | 1035 | - | 0 | 1 | TGGCACCTGCTCC |

(continued)

| IUPAC Family | IUPAC | Start pos. | End pos. | Strand | Mismatches | Score | Sequence |
|---|---|---|---|---|---|---|---|
| FAM010 | WBOXHVISO1 | 1028 | 1042 | - | 0 | 1 | AGTGACTTGGCACCT |
| FAM002 | RAV1BAT | 1051 | 1063 | - | 0 | 1 | CTCCACCTGCAGC |
| FAM151 | INTRONLOWER | 1053 | 1058 | + | 0 | 1 | TGCAGG |
| FAM263 | DPBFCOREDCDC3 | 1070 | 1076 | + | 0 | 1 | ACACTAG |
| FAM324 | CGCGBOXAT | 1079 | 1084 | + | 0 | 1 | CCGCGG |
| FAM324 | CGCGBOXAT | 1079 | 1084 | - | 0 | 1 | CCGCGG |
| FAM002 | GADOWNAT | 1090 | 1102 | - | 0 | 1 | CGACACGTGTCAG |
| FAM002 | GADOWNAT | 1091 | 1103 | + | 0 | 1 | TGACACGTGTCGC |
| FAM322 | BIHD1OS | 1091 | 1095 | - | 0 | 1 | TGTCA |
| FAM263 | DPBFCOREDCDC3 | 1093 | 1099 | + | 0 | 1 | ACACGTG |
| FAM263 | DPBFCOREDCDC3 | 1094 | 1100 | - | 0 | 1 | ACACGTG |
| FAM002 | SORLIP1AT | 1096 | 1108 | + | 0 | 1 | CGTGTCGCCACGT |
| FAM002 | ABREATRD2 | 1100 | 1112 | - | 0 | 1 | CGGCACGTGGCGA |
| FAM002 | GBOX10NT | 1101 | 1113 | + | 0 | 1 | CGCCACGTGCCGC |
| FAM061 | GCCCORE | 1108 | 1114 | + | 0 | 1 | TGCCGCC |
| FAM302 | SORLIP2AT | 1139 | 1149 | + | 0 | 1 | CGGGCCGACTG |
| FAM013 | DRECRTCOREAT | 1142 | 1148 | + | 0 | 1 | GCCGACT |
| FAM002 | TGACGTVMAMY | 1143 | 1155 | + | 0 | 1 | CCGACTGACGTCT |
| FAM002 | HEXMOTIFTAH3H4 | 1145 | 1157 | - | 0 | 1 | CAAGACGTCAGTC |
| FAM057 | ACGTCBOX | 1149 | 1154 | + | 0 | 1 | GACGTC |
| FAM057 | ACGTCBOX | 1149 | 1154 | - | 0 | 1 | GACGTC |
| FAM107 | CGACGOSAMY3 | 1172 | 1176 | + | 0 | 1 | CGACG |
| FAM061 | GCCCORE | 1179 | 1185 | - | 0 | 1 | CGCCGCC |
| FAM010 | ELRECOREPCRP1 | 1198 | 1212 | + | 0 | 1 | TTTGACCCCTCGCTA |
| FAM306 | ANAERO2CONSENSUS | 1236 | 1241 | - | 0 | 1 | AGCAGC |

(continued)

| IUPAC Family | IUPAC | Start pos. | End pos. | Strand | Mismatches | Score | Sequence |
|---|---|---|---|---|---|---|---|
| FAM002 | SORLIP1AT | 1274 | 1286 | + | 0 | 1 | CAGGACGCCACGT |
| FAM002 | ACGTABREMOTIFA20SE | 1278 | 1290 | - | 0 | 1 | TTGGACGTGGCGT |
| FAM262 | CIACADIANLELHC | 1302 | 1311 | - | 0 | 1 | CAATGGCATC |
| FAM002 | SORLIP1AT | 1305 | 1317 | + | 0 | 1 | GCCATTGCCACCT |
| FAM324 | CGCGBOXAT | 1328 | 1333 | + | 0 | 1 | ACGCGT |
| FAM324 | CGCGBOXAT | 1328 | 1333 | - | 0 | 1 | ACGCGT |
| FAM010 | WBOXHVISO1 | 1337 | 1351 | + | 0 | 1 | CGTGACTATAAAAA |
| FAM171 | MYBPZM | 1383 | 1389 | + | 0 | 1 | CCCTACC |
| FAM303 | OSE1ROOTNODULE | 1391 | 1397 | - | 0 | 1 | AAAGATT |
| FAM194 | PALBOXAPC | 1400 | 1406 | + | 0 | 1 | CCGTCCC |
| FAM302 | SITEIIATCYTC | 1405 | 1415 | - | 0 | 1 | TGGGCTGATGG |
| FAM311 | EECCRCAH1 | 1416 | 1422 | - | 0 | 1 | GAATTGC |
| FAM302 | SITEIIATCYTC | 1451 | 1461 | - | 0 | 1 | TGGGCTTCGGT |
| FAM013 | DRECRTCOREAT | 1466 | 1472 | + | 0 | 1 | GCCGACC |
| FAM003 | REALPHALGLHCB21 | 1482 | 1492 | - | 0 | 1 | AACCAACGGCA |
| FAM325 | MYBCOREATCYCB1 | 1484 | 1488 | - | 0 | 1 | AACGG |
| FAM194 | PALBOXAPC | 1493 | 1499 | + | 0 | 1 | CCGTCCC |
| FAM304 | OSE2ROOTNODULE | 1500 | 1504 | - | 0 | 1 | CTCTT |

2.) p-KG37

PLACE analysis results of p-KG37 are listed in Table 23, neither TATA box nor CAAT motifs are found in this promoter.

Table 23. PLACE analysis results of the 910bp promoter of p-KG37

| IUPAC Family | IUPAC | Start pos. | End pos. | Strand | Mismatches | Score | Sequence |
|---|---|---|---|---|---|---|---|
| FAM311 | EECCRCAH1 | 6 | 12 | + | 0 | 1 | GATTTCC |
| FAM069 | SURECOREATSULTR11 | 26 | 32 | + | 0 | 1 | GAGACGA |
| FAM272 | SV40COREENHAN | 30 | 37 | - | 0 | 1 | GTGGTTCG |
| FAM202 | -300ELEMENT | 37 | 45 | - | 0 | 1 | TGAAAAATG |
| FAM290 | GT1GMSCAM4 | 39 | 44 | - | 0 | 1 | GAAAAA |
| FAM324 | CGCGBOXAT | 58 | 63 | + | 0 | 1 | GCGCGC |
| FAM324 | CGCGBOXAT | 58 | 63 | - | 0 | 1 | GCGCGC |
| FAM002 | ASF1MOTIFCAMV | 66 | 78 | - | 0 | 1 | GTCACTGACGATT |
| FAM271 | SEBFCONSSTPR10A | 74 | 80 | - | 0 | 1 | CTGTCAC |
| FAM322 | BIHD1OS | 75 | 79 | - | 0 | 1 | TGTCA |
| FAM008 | MYB2AT | 94 | 104 | + | 0 | 1 | TCCTTAACTGG |
| FAM281 | MYB1LEPR | 105 | 111 | - | 0 | 1 | GTTAGTT |
| FAM263 | DPBFCOREDCDC3 | 140 | 146 | + | 0 | 1 | ACACTGG |
| FAM273 | TATCCAOSAMY | 158 | 164 | - | 0 | 1 | TATCCAA |
| FAM014 | MYBST1 | 159 | 165 | + | 0 | 1 | TGGATAG |
| FAM325 | MYBCOREATCYCB1 | 175 | 179 | - | 0 | 1 | AACGG |
| FAM278 | UPRMOTIFIIAT | 180 | 198 | - | 0 | 1 | CCTTGCTTTTTAGCCCACG |
| FAM302 | SITEIIATCYTC | 182 | 192 | + | 0 | 1 | TGGGCTAAAAA |
| FAM002 | CACGTGMOTIF | 212 | 224 | - | 0 | 1 | GATCACGTGCGTT |
| FAM002 | CACGTGMOTIF | 213 | 225 | + | 0 | 1 | ACGCACGTGATCC |
| FAM069 | SURECOREATSULTR11 | 231 | 237 | - | 0 | 1 | GAGACCA |
| FAM266 | MYB1AT | 244 | 249 | + | 0 | 1 | AAACCA |
| FAM306 | ANAERO2CONSENSUS | 278 | 283 | - | 0 | 1 | AGCAGC |
| FAM002 | ASF1MOTIFCAMV | 285 | 297 | + | 0 | 1 | TCAGTTGACGGTG |
| FAM010 | WBOXATNPR1 | 288 | 302 | + | 0 | 1 | GTTGACGGTGTGCAC |

(continued)

| IUPAC Family | IUPAC | Start pos. | End pos. | Strand | Mismatches | Score | Sequence |
|---|---|---|---|---|---|---|---|
| FAM302 | SITEIIATCYTC | 337 | 347 | - | 0 | 1 | TGGGCTCCAAG |
| FAM205 | PYRIMIDINEBOXOSRAM | 351 | 356 | - | 0 | 1 | CCTTTT |
| FAM311 | EECCRCAH1 | 363 | 369 | - | 0 | 1 | GAATTTC |
| FAM325 | MYBCOREATCYCB1 | 387 | 391 | + | 0 | 1 | AACGG |
| FAM270 | RAV1AAT | 392 | 396 | + | 0 | 1 | CAACA |
| FAM205 | PYRIMIDINEBOXOSRAM | 396 | 401 | - | 0 | 1 | CCTTTT |
| FAM013 | LTRECOREATCOR15 | 405 | 411 | + | 0 | 1 | TCCGACA |
| FAM194 | PALBOXAPC | 446 | 452 | + | 0 | 1 | CCGTCCT |
| FAM263 | DPBFCOREDCDC3 | 477 | 483 | - | 0 | 1 | ACACTTG |
| FAM002 | SORLIP1AT | 479 | 491 | + | 0 | 1 | AGTGTTGCCACGC |
| FAM270 | RAV1AAT | 481 | 485 | - | 0 | 1 | CAACA |
| FAM324 | CGCGBOXAT | 491 | 496 | + | 0 | 1 | CCGCGC |
| FAM324 | CGCGBOXAT | 491 | 496 | - | 0 | 1 | GCGCGG |
| FAM002 | ASF1MOTIFCAMV | 554 | 566 | - | 0 | 1 | AGCAGTGACGCCG |
| FAM061 | GCCCORE | 578 | 584 | + | 0 | 1 | GGCCGCC |
| FAM002 | SORLIP1AT | 621 | 633 | + | 0 | 1 | ACCGAGGCCACCT |
| FAM205 | PYRIMIDINEBOXOSRAM | 631 | 636 | + | 0 | 1 | CCTTTT |
| FAM003 | REALPHALGLHCB21 | 633 | 643 | - | 0 | 1 | AACCAAGAAAA |
| FAM270 | RAV1AAT | 648 | 652 | + | 0 | 1 | CAACA |
| FAM002 | ABRELATERD | 677 | 689 | - | 0 | 1 | GCAGACGTGGTGC |
| FAM303 | OSE1ROOTNODULE | 703 | 709 | - | 0 | 1 | AAAGATT |
| FAM069 | SURECOREATSULTR11 | 745 | 751 | - | 0 | 1 | GAGACGG |
| FAM305 | ANAERO1CONSENSUS | 802 | 808 | - | 0 | 1 | AAACAAA |
| FAM194 | PALBOXAPC | 820 | 826 | + | 0 | 1 | CCGTCCT |
| FAM263 | DPBFCOREDCDC3 | 824 | 830 | - | 0 | 1 | ACACAGG |

(continued)

| IUPAC Family | IUPAC | Start pos. | End pos. | Strand | Mismatches | Score | Sequence |
|---|---|---|---|---|---|---|---|
| FAM325 | MYBCOREATCYCB1 | 859 | 863 | + | 0 | 1 | AACGG |
| FAM267 | TAAAGSTKST1 | 882 | 888 | - | 0 | 1 | CATAAAG |

3.) p-KG45

PLACE analysis results of p-KG45 are listed in Table 24, Three TATA Box motifs are found at nucleotide position 310-316, 312-318, and 1065-1071 of the forward strand, respectively. One CAAT Box motif is found at nucleotide position 976-980 of the forward strand that may be the functional motif working with the TATA box at position 1065-1071 to facilitate transcriptional initiation.

Table 24. PLACE analysis results of the 1131bp promoter of p-KG45

| IUPAC Family | IUPAC | Start pos. | End pos. | Strand | Mis-Matches | Score | Sequence |
|---|---|---|---|---|---|---|---|
| FAM276 | TRANSINITDICOTS | 4 | 11 | - | 0 | 1 | ACGATGGC |
| FAM263 | DPBFCOREDCDC3 | 33 | 39 | - | 0 | 1 | ACACACG |
| FAM263 | DPBFCOREDCDC3 | 49 | 55 | - | 0 | 1 | ACACACG |
| FAM228 | SEF3MOTIFGM | 74 | 79 | + | 0 | 1 | AACCCA |
| FAM171 | MYBPZM | 76 | 82 | + | 0 | 1 | CCCAACC |
| FAM003 | MYBPLANT | 79 | 89 | + | 0 | 1 | AACCAAACATC |
| FAM311 | EECCRCAH1 | 96 | 102 | + | 0 | 1 | GATTTCC |
| FAM234 | SP8BFIBSP8BIB | 122 | 128 | - | 0 | 1 | TACTATT |
| FAM310 | CPBCSPOR | 127 | 132 | + | 0 | 1 | TATTAG |
| FAM270 | RAV1AAT | 173 | 177 | + | 0 | 1 | CAACA |
| FAM242 | TATABOX3 | 177 | 183 | - | 0 | 1 | TATTAAT |
| FAM087 | BOXIINTPATPB | 184 | 189 | + | 0 | 1 | ATAGAA |
| FAM267 | NTBBF1ARROLB | 222 | 228 | + | 0 | 1 | ACTTTAT |
| FAM267 | TAAAGSTKST1 | 223 | 229 | - | 0 | 1 | AATAAAG |
| FAM290 | GT1GMSCAM4 | 229 | 234 | - | 0 | 1 | GAAAAA |
| FAM263 | DPBFCOREDCDC3 | 243 | 249 | - | 0 | 1 | ACACTGG |
| FAM234 | SP8BFIBSP8BIB | 254 | 260 | - | 0 | 1 | TACTATT |
| FAM290 | GT1GMSCAM4 | 276 | 281 | - | 0 | 1 | GAAAAA |
| FAM304 | OSE2ROOTNODULE | 288 | 292 | - | 0 | 1 | CTCTT |
| FAM295 | P1BS | 295 | 302 | + | 0 | 1 | GAATATTC |
| FAM295 | P1BS | 295 | 302 | - | 0 | 1 | GAATATTC |
| FAM205 | PYRIMIDINEBOXOSRAM | 304 | 309 | + | 0 | 1 | CCTTTT |
| FAM019 | TATAPVTRNALEU | 306 | 318 | - | 0 | 1 | ATTTATATAAAAA |
| FAM019 | TATAPVTRNALEU | 307 | 319 | + | 0 | 1 | TTTTATATAAATT |
| FAM243 | TATABOX4 | 309 | 315 | - | 0 | 1 | TATATAA |

(continued)

| IUPAC Family | IUPAC | Start pos. | End pos. | Strand | Mis-Matches | Score | Sequence |
|---|---|---|---|---|---|---|---|
| FAM243 | TATABOX4 | 310 | 316 | + | 0 | 1 | TATATAA |
| FAM241 | TATABOX2 | 312 | 318 | + | 0 | 1 | TATAAAT |
| FAM027 | -10PEHVPSBD | 323 | 328 | + | 0 | 1 | TATTCT |
| FAM002 | ASF1MOTIFCAMV | 332 | 344 | - | 0 | 1 | GTGTGTGACGCTT |
| FAM263 | DPBFCOREDCDC3 | 339 | 345 | + | 0 | 1 | ACACACG |
| FAM267 | TAAAGSTKST1 | 351 | 357 | + | 0 | 1 | CCTAAAG |
| FAM303 | OSE1ROOTNODULE | 354 | 360 | + | 0 | 1 | AAAGATA |
| FAM027 | -10PEHVPSBD | 359 | 364 | + | 0 | 1 | TATTCT |
| FAM270 | RAV1AAT | 372 | 376 | + | 0 | 1 | CAACA |
| FAM263 | DPBFCOREDCDC3 | 374 | 380 | + | 0 | 1 | ACACAAG |
| FAM202 | -300ELEMENT | 386 | 394 | + | 0 | 1 | TGAAAAGGT |
| FAM205 | PYRIMIDINEBOXOSRAM | 388 | 393 | - | 0 | 1 | CCTTTT |
| FAM270 | RAV1AAT | 413 | 417 | - | 0 | 1 | CAACA |
| FAM275 | TGTCACACMCUCUMISIN | 426 | 432 | - | 0 | 1 | TGTCACA |
| FAM322 | BIHD1OS | 428 | 432 | - | 0 | 1 | TGTCA |
| FAM103 | CELLCYCLESC | 431 | 438 | + | 0 | 1 | CACGAAAA |
| FAM267 | TAAAGSTKST1 | 439 | 445 | + | 0 | 1 | TTTAAAG |
| FAM289 | LEAFYATAG | 461 | 467 | - | 0 | 1 | CCAATGT |
| FAM100 | CCAATBOX1 | 463 | 467 | - | 0 | 1 | CCAAT |
| FAM021 | GT1CORE | 484 | 494 | - | 0 | 1 | TGGTTAATATG |
| FAM266 | MYB1AT | 489 | 494 | + | 0 | 1 | TAACCA |
| FAM003 | REALPHALGLHCB21 | 490 | 500 | + | 0 | 1 | AACCAACTATT |
| FAM310 | CPBCSPOR | 497 | 502 | + | 0 | 1 | TATTAG |
| FAM169 | MYBATRD2 | 530 | 536 | - | 0 | 1 | CTAACCA |
| FAM266 | MYB1AT | 530 | 535 | - | 0 | 1 | TAACCA |

(continued)

| IUPAC Family | IUPAC | Start pos. | End pos. | Strand | Mis-Matches | Score | Sequence |
|---|---|---|---|---|---|---|---|
| FAM087 | BOXIINTPATPB | 558 | 563 | - | 0 | 1 | ATAGAA |
| FAM170 | AMYBOX1 | 597 | 603 | - | 0 | 1 | TAACAGA |
| FAM278 | UPRMOTIFIIAT | 628 | 646 | + | 0 | 1 | CCAAATGTATAATCCCACG |
| FAM172 | MYCATRD2 | 652 | 658 | - | 0 | 1 | CACATGA |
| FAM172 | MYCATERD | 653 | 659 | + | 0 | 1 | CATGTGA |
| FAM010 | WBOXHVISO1 | 655 | 669 | + | 0 | 1 | TGTGACTCCATTTCG |
| FAM002 | ABRELATERD | 740 | 752 | - | 0 | 1 | AGATACGTGAACG |
| FAM263 | DPBFCOREDCDC3 | 751 | 757 | - | 0 | 1 | ACACAAG |
| FAM024 | CANBNNAPA | 752 | 760 | - | 0 | 1 | CGAACACAA |
| FAM325 | MYBCOREATCYCB1 | 771 | 775 | - | 0 | 1 | AACGG |
| FAM002 | RAV1BAT | 830 | 842 | - | 0 | 1 | CATCACCTGCCTC |
| FAM307 | ANAERO3CONSENSUS | 837 | 843 | - | 0 | 1 | TCATCAC |
| FAM322 | BIHD1OS | 841 | 845 | - | 0 | 1 | TGTCA |
| FAM263 | DPBFCOREDCDC3 | 843 | 849 | + | 0 | 1 | ACACGCG |
| FAM324 | CGCGBOXAT | 845 | 850 | + | 0 | 1 | ACGCGC |
| FAM324 | CGCGBOXAT | 845 | 850 | - | 0 | 1 | GCGCGT |
| FAM302 | SORLIP2AT | 855 | 865 | + | 0 | 1 | CGGGCCGATGC |
| FAM013 | DRECRTCOREAT | 864 | 870 | + | 0 | 1 | GCCGACG |
| FAM002 | SORLIP1AT | 866 | 878 | + | 0 | 1 | CGACGCGCCACCG |
| FAM107 | CGACGOSAMY3 | 866 | 870 | + | 0 | 1 | CGACG |
| FAM324 | CGCGBOXAT | 868 | 873 | + | 0 | 1 | ACGCGC |
| FAM324 | CGCGBOXAT | 868 | 873 | - | 0 | 1 | GCGCGT |
| FAM306 | ANAERO2CONSENSUS | 901 | 906 | + | 0 | 1 | AGCAGC |
| FAM002 | ABRELATERD | 912 | 924 | + | 0 | 1 | AGAGACGTGGAGC |
| FAM050 | ABREBZMRAB28 | 913 | 922 | - | 0 | 1 | TCCACGTCTC |

(continued)

| IUPAC Family | IUPAC | Start pos. | End pos. | Strand | Mis-Matches | Score | Sequence |
|---|---|---|---|---|---|---|---|
| FAM069 | SURECOREATSULTR11 | 913 | 919 | + | 0 | 1 | GAGACGT |
| FAM069 | SURECOREATSULTR11 | 931 | 937 | + | 0 | 1 | GAGACTT |
| FAM267 | NTBBF1ARROLB | 934 | 940 | + | 0 | 1 | ACTTTAG |
| FAM267 | TAAAGSTKST1 | 935 | 941 | - | 0 | 1 | CCTAAAG |
| FAM069 | SURECOREATSULTR11 | 948 | 954 | + | 0 | 1 | GAGACCA |
| FAM322 | BIHD1OS | 968 | 972 | - | 0 | 1 | TGTCA |
| FAM278 | UPRMOTIFIIAT | 975 | 993 | + | 0 | 1 | CCCAATGATCAGGACCACG |
| FAM100 | CCAATBOX1 | 976 | 980 | + | 0 | 1 | CCAAT |
| FAM002 | CACGTGMOTIF | 994 | 1006 | - | 0 | 1 | TGACACGTGCAAG |
| FAM002 | GADOWNAT | 995 | 1007 | + | 0 | 1 | TTGCACGTGTCAG |
| FAM263 | DPBFCOREDCDC3 | 998 | 1004 | - | 0 | 1 | ACACGTG |
| FAM002 | RAV1BAT | 1001 | 1013 | - | 0 | 1 | AGGCACCTGACAC |
| FAM322 | BIHD1OS | 1002 | 1006 | + | 0 | 1 | TGTCA |
| FAM324 | CGCGBOXAT | 1021 | 1026 | + | 0 | 1 | ACGCGT |
| FAM324 | CGCGBOXAT | 1021 | 1026 | - | 0 | 1 | ACGCGT |
| FAM107 | CGACGOSAMY3 | 1024 | 1028 | - | 0 | 1 | CGACG |
| FAM324 | CGCGBOXAT | 1028 | 1033 | + | 0 | 1 | GCGCGC |
| FAM324 | CGCGBOXAT | 1028 | 1033 | - | 0 | 1 | GCGCGC |
| FAM322 | BIHD1OS | 1047 | 1051 | + | 0 | 1 | TGTCA |
| FAM107 | CGACGOSAMY3 | 1059 | 1063 | + | 0 | 1 | CGACG |
| FAM019 | TATAPVTRNALEU | 1061 | 1073 | - | 0 | 1 | CTTTATATAGCGT |
| FAM243 | TATABOX4 | 1065 | 1071 | + | 0 | 1 | TATATAA |
| FAM267 | TAAAGSTKST1 | 1067 | 1073 | + | 0 | 1 | TATAAAG |
| FAM267 | NTBBF1ARROLB | 1068 | 1074 | - | 0 | 1 | ACTTTAT |
| FAM272 | SV40COREENHAN | 1073 | 1080 | + | 0 | 1 | GTGGTAAG |

(continued)

| IUPAC Family | IUPAC | Start pos. | End pos. | Strand | Mis-Matches | Score | Sequence |
|---|---|---|---|---|---|---|---|
| FAM302 | SORLIP2AT | 1094 | 1104 | + | 0 | 1 | GGGGCCGCCCC |
| FAM061 | GCCCORE | 1096 | 1102 | + | 0 | 1 | GGCCGCC |
| FAM302 | SORLIP2AT | 1103 | 1113 | - | 0 | 1 | AGGGCCGTTGG |
| FAM325 | MYBCOREATCYCB1 | 1105 | 1109 | + | 0 | 1 | AACGG |
| FAM278 | UPRMOTIFIIAT | 1111 | 1129 | + | 0 | 1 | CCTTGCGATCGCCACCACG |
| FAM002 | SORLIP1AT | 1115 | 1127 | + | 0 | 1 | GCGATCGCCACCA |

4.) p-KG46

PLACE analysis results of p-KG46 are listed in Table 25, neither TATA box nor CAAT motifs are found in this promoter.

Table 25. PLACE analysis results of the 563bp promoter of p-KG46

| IUPAC Family | IUPAC | Start pos. | End pos. | Strand | Mismatches | Score | Sequence |
|---|---|---|---|---|---|---|---|
| FAM002 | SORLIP1AT | 40 | 52 | - | 0 | 1 | TAAATTGCCACCC |
| FAM170 | AMYBOX1 | 54 | 60 | + | 0 | 1 | TAACAGA |
| FAM311 | EECCRCAH1 | 59 | 65 | + | 0 | 1 | GAATTGC |
| FAM292 | PREATPRODH | 69 | 74 | - | 0 | 1 | ACTCAT |
| FAM271 | SEBFCONSSTPR10A | 121 | 127 | + | 0 | 1 | TTGTCAC |
| FAM275 | TGTCACACMCUCUMISIN | 122 | 128 | + | 0 | 1 | TGTCACA |
| FAM322 | BIHD1OS | 122 | 126 | + | 0 | 1 | TGTCA |
| FAM172 | MYCATERD | 124 | 130 | - | 0 | 1 | CATGTGA |
| FAM172 | MYCATRD2 | 125 | 131 | + | 0 | 1 | CACATGT |
| FAM172 | MYCATRD2 | 126 | 132 | - | 0 | 1 | CACATGT |
| FAM172 | MYCATERD | 127 | 133 | + | 0 | 1 | CATGTGG |
| FAM002 | SORLIP1AT | 128 | 140 | - | 0 | 1 | AAAAAGGCCACAT |
| FAM205 | PYRIMIDINEBOXOSRAM | 134 | 139 | + | 0 | 1 | CCTTTT |
| FAM003 | REALPHALGLHCB21 | 135 | 145 | - | 0 | 1 | AACCAAAAAAG |
| FAM302 | SITEIIATCYTC | 171 | 181 | + | 0 | 1 | TGGGCTGTCAT |
| FAM322 | BIHD1OS | 176 | 180 | + | 0 | 1 | TGTCA |
| FAM304 | OSE2ROOTNODULE | 203 | 207 | + | 0 | 1 | CTCTT |
| FAM012 | IBOXCORE | 212 | 218 | - | 0 | 1 | GATAATG |
| FAM002 | ASF1MOTIFCAMV | 229 | 241 | - | 0 | 1 | GGAAATGACGATG |
| FAM069 | SURECOREATSULTR11 | 245 | 251 | + | 0 | 1 | GAGACCC |
| FAM322 | BIHD1OS | 260 | 264 | + | 0 | 1 | TGTCA |
| FAM263 | DPBFCOREDCDC3 | 277 | 283 | + | 0 | 1 | ACACGCG |
| FAM324 | CGCGBOXAT | 279 | 284 | + | 0 | 1 | ACGCGT |
| FAM324 | CGCGBOXAT | 279 | 284 | - | 0 | 1 | ACGCGT |
| FAM107 | CGACGOSAMY3 | 282 | 286 | - | 0 | 1 | CGACG |

(continued)

| IUPAC Family | IUPAC | Start pos. | End pos. | Strand | Mismatches | Score | Sequence |
|---|---|---|---|---|---|---|---|
| FAM107 | CGACGOSAMY3 | 287 | 291 | - | 0 | 1 | CGACG |
| FAM002 | RAV1BAT | 294 | 306 | - | 0 | 1 | ACCCACCTGGCCT |
| FAM002 | SITEIOSPCNA | 295 | 307 | + | 0 | 1 | GGCCAGGTGGGTT |
| FAM228 | SEF3MOTIFGM | 302 | 307 | - | 0 | 1 | AACCCA |
| FAM194 | PALBOXAPC | 354 | 360 | + | 0 | 1 | CCGTCCA |
| FAM194 | CMSRE1IBSPOA | 354 | 360 | - | 0 | 1 | TGGACGG |
| FAM013 | DRE2COREZMRAB17 | 360 | 366 | + | 0 | 1 | ACCGACT |
| FAM026 | RYREPEATLEGUMINBOX | 393 | 403 | + | 0 | 1 | ACCATGCACGA |
| FAM107 | CGACGOSAMY3 | 401 | 405 | + | 0 | 1 | CGACG |
| FAM002 | GADOWNAT | 403 | 415 | - | 0 | 1 | TCGCACGTGTCGT |
| FAM002 | CACGTGMOTIF | 404 | 416 | + | 0 | 1 | CGACACGTGCGAT |
| FAM047 | ABRE2HVA22 | 405 | 414 | - | 0 | 1 | CGCACGTGTC |
| FAM263 | DPBFCOREDCDC3 | 406 | 412 | + | 0 | 1 | ACACGTG |
| FAM002 | RAV1BAT | 433 | 445 | + | 0 | 1 | ACTCACCTGTTGC |
| FAM270 | RAV1AAT | 440 | 444 | - | 0 | 1 | CAACA |
| FAM014 | MYBST1 | 450 | 456 | - | 0 | 1 | TGGATAT |
| FAM025 | TATCCAYMOTIFOSRAMY | 451 | 457 | + | 0 | 1 | TATCCAC |
| FAM273 | TATCCACHVAL21 | 451 | 457 | + | 0 | 1 | TATCCAC |
| FAM010 | WBOXNTCHN48 | 502 | 516 | + | 0 | 1 | GCTGACCAGAGAGCT |

5.) p-KG49

PLACE analysis results of p-KG49 is listed in Table 26, One TATA Box motif is found at nucleotide position 803-809 of the forward strand and one CAAT Box motif is found at nucleotide position 472-476 at the reverse strand.

Table 26. PLACE analysis results of the 1188bp promoter of p-KG49

| IUPAC Family | IUPAC | Start pos. | End pos. | Str and | Mismatch es | Score | Sequence |
|---|---|---|---|---|---|---|---|
| FAM013 | DRECRTCOREAT | 29 | 35 | - | 0 | 1 | GCCGACA |
| FAM324 | CGCGBOXAT | 45 | 50 | + | 0 | 1 | CCGCGT |
| FAM324 | CGCGBOXAT | 45 | 50 | - | 0 | 1 | ACGCGG |
| FAM324 | CGCGBOXAT | 57 | 62 | + | 0 | 1 | CCGCGG |
| FAM324 | CGCGBOXAT | 57 | 62 | - | 0 | 1 | CCGCGG |
| FAM069 | SURECOREATSULTR11 | 60 | 66 | - | 0 | 1 | GAGACCG |
| FAM107 | CGACGOSAMY3 | 68 | 72 | - | 0 | 1 | CGACG |
| FAM069 | SURECOREATSULTR11 | 70 | 76 | - | 0 | 1 | GAGACGA |
| FAM272 | SV40COREENHAN | 101 | 108 | - | 0 | 1 | GTGGAAAG |
| FAM278 | UPRMOTIFIIAT | 126 | 144 | + | 0 | 1 | CCACCCCCTTCTCCCCACG |
| FAM324 | CGCGBOXAT | 159 | 164 | + | 0 | 1 | ACGCGC |
| FAM324 | CGCGBOXAT | 159 | 164 | - | 0 | 1 | GCGCGT |
| FAM270 | RAV1AAT | 173 | 177 | - | 0 | 1 | CAACA |
| FAM307 | ANAERO3CONSENSUS | 179 | 185 | + | 0 | 1 | TCATCAC |
| FAM002 | ASF1MOTIFCAMV | 196 | 208 | - | 0 | 1 | CTCTGTGACGCTT |
| FAM147 | HEXAMERATH4 | 231 | 236 | + | 0 | 1 | CCGTCG |
| FAM107 | CGACGOSAMY3 | 232 | 236 | - | 0 | 1 | CGACG |
| FAM147 | HEXAMERATH4 | 237 | 242 | + | 0 | 1 | CCGTCG |
| FAM107 | CGACGOSAMY3 | 238 | 242 | - | 0 | 1 | CGACG |
| FAM152 | INTRONUPPER | 249 | 257 | + | 0 | 1 | CAGGTAAGT |
| FAM010 | WBOXHVISO1 | 258 | 272 | + | 0 | 1 | AATGACTAATCGCCT |
| FAM069 | SURECOREATSULTR11 | 273 | 279 | - | 0 | 1 | GAGACTC |
| FAM266 | MYB1AT | 303 | 308 | - | 0 | 1 | AAACCA |
| FAM013 | LTRECOREATCOR15 | 313 | 319 | - | 0 | 1 | TCCGACT |
| FAM057 | ACGTCBOX | 318 | 323 | + | 0 | 1 | GACGTC |

(continued)

| IUPAC Family | IUPAC | Start pos. | End pos. | Str an d | Mismatch es | Score | Sequence |
|---|---|---|---|---|---|---|---|
| FAM057 | ACGTCBOX | 318 | 323 | - | 0 | 1 | GACGTC |
| FAM013 | LTRECOREATCOR15 | 320 | 326 | - | 0 | 1 | TCCGACG |
| FAM107 | CGACGOSAMY3 | 320 | 324 | - | 0 | 1 | CGACG |
| FAM002 | SORLIP1AT | 378 | 390 | + | 0 | 1 | TTCGACGCCACAT |
| FAM107 | CGACGOSAMY3 | 380 | 384 | + | 0 | 1 | CGACG |
| FAM290 | GT1GMSCAM4 | 392 | 397 | - | 0 | 1 | GAAAAA |
| FAM171 | MYBPZM | 404 | 410 | + | 0 | 1 | GCCAACC |
| FAM324 | CGCGBOXAT | 414 | 419 | + | 0 | 1 | GCGCGT |
| FAM324 | CGCGBOXAT | 414 | 419 | - | 0 | 1 | ACGCGC |
| FAM025 | TATCCAYMOTIFOSRAMY | 466 | 472 | - | 0 | 1 | TATCCAC |
| FAM273 | TATCCACHVAL21 | 466 | 472 | - | 0 | 1 | TATCCAC |
| FAM014 | MYBST1 | 467 | 473 | + | 0 | 1 | TGGATAT |
| FAM100 | CCAATBOX1 | 472 | 476 | - | 0 | 1 | CCAAT |
| FAM003 | REALPHALGLHCB21 | 479 | 489 | - | 0 | 1 | AACCAAAAAAA |
| FAM169 | MYBATRD2 | 485 | 491 | - | 0 | 1 | CTAACCA |
| FAM266 | MYB1AT | 485 | 490 | - | 0 | 1 | TAACCA |
| FAM270 | RAV1AAT | 534 | 538 | - | 0 | 1 | CAACA |
| FAM170 | MYBGAHV | 546 | 552 | - | 0 | 1 | TAACAAA |
| FAM177 | NRRBNEXTA | 551 | 558 | + | 0 | 1 | TAGTGGAT |
| FAM069 | SURECOREATSULTR11 | 629 | 635 | + | 0 | 1 | GAGACTA |
| FAM069 | SURECOREATSULTR11 | 647 | 653 | + | 0 | 1 | GAGACTA |
| FAM010 | ELRECOREPCRP1 | 653 | 667 | - | 0 | 1 | CTTGACCATTCGCAT |
| FAM311 | EECCRCAH1 | 669 | 675 | + | 0 | 1 | GAATTTC |
| FAM003 | REALPHALGLHCB21 | 676 | 686 | - | 0 | 1 | AACCAAGGCGA |
| FAM266 | MYB1AT | 682 | 687 | - | 0 | 1 | AAACCA |

(continued)

| IUPAC Family | IUPAC | Start pos. | End pos. | Str an d | Mismatch es | Score | Sequence |
|---|---|---|---|---|---|---|---|
| FAM317 | SORLREP3AT | 722 | 730 | + | 0 | 1 | TGTATATAT |
| FAM266 | MYB1AT | 732 | 737 | - | 0 | 1 | AAACCA |
| FAM002 | T/GBOXATPIN2 | 740 | 752 | - | 0 | 1 | CTAAACGTGCCGA |
| FAM322 | BIHD1OS | 781 | 785 | + | 0 | 1 | TGTCA |
| FAM322 | BIHD1OS | 792 | 796 | + | 0 | 1 | TGTCA |
| FAM243 | TATABOX4 | 803 | 809 | + | 0 | 1 | TATATAA |
| FAM010 | WBOXATNPR1 | 833 | 847 | + | 0 | 1 | ATTGACTTATTATGC |
| FAM311 | EECCRCAH1 | 846 | 852 | - | 0 | 1 | GACTTGC |
| FAM021 | GT1CORE | 851 | 861 | - | 0 | 1 | AGGTTAATCGA |
| FAM302 | SITEIIATCYTC | 865 | 875 | + | 0 | 1 | TGGGCTCAGTG |
| FAM221 | S1FBOXSORPS1L21 | 879 | 884 | - | 0 | 1 | ATGGTA |
| FAM010 | WBOXNTCHN48 | 945 | 959 | - | 0 | 1 | GCTGACTAGCCGAGT |
| FAM321 | WRECSAA01 | 982 | 991 | - | 0 | 1 | AAAGTATCGA |
| FAM069 | ARFAT | 997 | 1003 | + | 0 | 1 | ATGTCTC |
| FAM069 | SURECOREATSULTR11 | 997 | 1003 | - | 0 | 1 | GAGACAT |
| FAM021 | GT1CORE | 1007 | 1017 | - | 0 | 1 | TGGTTAACACA |
| FAM266 | MYB1AT | 1012 | 1017 | + | 0 | 1 | TAACCA |
| FAM002 | SORLIP1AT | 1020 | 1032 | + | 0 | 1 | GTGTGTGCCACAT |
| FAM039 | AACACOREOSGLUB1 | 1051 | 1057 | - | 0 | 1 | AACAAAC |
| FAM021 | GT1CORE | 1052 | 1062 | - | 0 | 1 | AGGTTAACAAA |
| FAM170 | MYBGAHV | 1052 | 1058 | - | 0 | 1 | TAACAAA |
| FAM329 | XYLAT | 1125 | 1132 | - | 0 | 1 | ACAAAGAA |

6.) p-KG56

PLACE analysis results of p-KG56 are listed in Table 27. Two TATA Box motifs are found at nucleotide position 729-735, and 1900-1906 of the forward strand respectively. One CAAT Box motif is found at nucleotide position 599-603 of the reverse strand.

Table 27. PLACE analysis results of the 1188bp promoter of p-KG56

| IUPAC Family | IUPAC | Start pos. | End pos. | Strand | Mismatches | Score | Sequence |
|---|---|---|---|---|---|---|---|
| FAM010 | WBOXNTCHN48 | 7 | 21 | - | 0 | 1 | GCTGACCAGATGTTC |
| FAM267 | TAAAGSTKST1 | 33 | 39 | + | 0 | 1 | GGTAAAG |
| F AM 027 | -10PEHVPSBD | 47 | 52 | + | 0 | 1 | TATTCT |
| FAM329 | XYLAT | 62 | 69 | - | 0 | 1 | ACAAAGAA |
| FAM012 | IBOXCORE | 155 | 161 | + | 0 | 1 | GATAATG |
| FAM270 | RAV1AAT | 165 | 169 | + | 0 | 1 | CAACA |
| FAM021 | GT1CORE | 170 | 180 | - | 0 | 1 | CGGTTAATCTC |
| FAM026 | RYREPEATGMGY2 | 201 | 211 | + | 0 | 1 | ATCATGCATTA |
| FAM267 | TAAAGSTKST1 | 208 | 214 | + | 0 | 1 | ATTAAAG |
| FAM267 | NTBBF1ARROLB | 209 | 215 | - | 0 | 1 | ACTTTAA |
| FAM171 | MYBPZM | 216 | 222 | + | 0 | 1 | TCCTACC |
| FAM170 | GARE2OSREP1 | 296 | 302 | - | 0 | 1 | TAACGTA |
| FAM012 | IBOXCORE | 304 | 310 | - | 0 | 1 | GATAATT |
| FAM014 | SREATMSD | 305 | 311 | + | 0 | 1 | ATTATCC |
| FAM014 | MYBST1 | 306 | 312 | - | 0 | 1 | AGGATAA |
| FAM205 | PYRIMIDINEBOXOSRAM | 310 | 315 | + | 0 | 1 | CCTTTT |
| FAM014 | MYBST1 | 362 | 368 | - | 0 | 1 | TGGATAG |
| FAM273 | TATCCAOSAMY | 363 | 369 | + | 0 | 1 | TATCCAG |
| FAM002 | ASF1MOTIFCAMV | 379 | 391 | + | 0 | 1 | GAAGTTGACGCTC |
| FAM010 | WBOXATNPR1 | 382 | 396 | + | 0 | 1 | GTTGACGCTCTCAAA |
| FAM245 | TBOXATGAPB | 393 | 398 | - | 0 | 1 | ACTTTG |
| FAM010 | WBOXATNPR1 | 416 | 430 | + | 0 | 1 | ATTGACACATTTTTT |
| FAM322 | BIHD1OS | 418 | 422 | - | 0 | 1 | TGTCA |
| FAM267 | TAAAGSTKST1 | 436 | 442 | + | 0 | 1 | CTTAAAG |
| FAM304 | OSE2ROOTNODULE | 445 | 449 | + | 0 | 1 | CTCTT |

(continued)

| IUPAC Family | IUPAC | Start pos. | End pos. | Strand | Mismatches | Score | Sequence |
|---|---|---|---|---|---|---|---|
| FAM002 | RAV1BAT | 447 | 459 | + | 0 | 1 | CTTCACCTGAGAT |
| FAM202 | -300ELEMENT | 461 | 469 | + | 0 | 1 | TGAAAAAGG |
| FAM290 | GT1GMSCAM4 | 462 | 467 | + | 0 | 1 | GAAAAA |
| FAM205 | PYRIMIDINEBOXOSRAM | 464 | 469 | - | 0 | 1 | CCTTTT |
| FAM171 | BOXLCOREDCPAL | 469 | 475 | - | 0 | 1 | ACCATCC |
| FAM263 | DPBFCOREDCDC3 | 504 | 510 | + | 0 | 1 | ACACGGG |
| FAM061 | AGCBOXNPGLB | 527 | 533 | - | 0 | 1 | AGCCGCC |
| FAM002 | ASF1MOTIFCAMV | 563 | 575 | + | 0 | 1 | CAAGGTGACGCGG |
| FAM324 | CGCGBOXAT | 570 | 575 | + | 0 | 1 | ACGCGG |
| FAM324 | CGCGBOXAT | 570 | 575 | - | 0 | 1 | CCGCGT |
| FAM170 | AMYBOX1 | 591 | 597 | - | 0 | 1 | TAACAGA |
| FAM289 | LEAFYATAG | 597 | 603 | - | 0 | 1 | CCAATGT |
| FAM100 | CCAATBOX1 | 599 | 603 | - | 0 | 1 | CCAAT |
| FAM013 | DRE2COREZMRAB17 | 635 | 641 | + | 0 | 1 | ACCGACA |
| FAM163 | LTREATLTI78 | 635 | 641 | + | 0 | 1 | ACCGACA |
| FAM324 | CGCGBOXAT | 650 | 655 | + | 0 | 1 | ACGCGC |
| FAM324 | CGCGBOXAT | 650 | 655 | - | 0 | 1 | GCGCGT |
| FAM325 | MYBCOREATCYCB1 | 669 | 673 | - | 0 | 1 | AACGG |
| FAM069 | SURECOREATSULTR11 | 681 | 687 | + | 0 | 1 | GAGACTT |
| FAM290 | GT1GMSCAM4 | 691 | 696 | - | 0 | 1 | GAAAAA |
| FAM290 | GT1 GMSCAM4 | 700 | 705 | + | 0 | 1 | GAAAAA |
| FAM241 | TATABOX2 | 729 | 735 | + | 0 | 1 | TATAAAT |
| FAM304 | OSE2ROOTNODULE | 736 | 740 | + | 0 | 1 | CTCTT |
| FAM162 | LTRE1HVBLT49 | 755 | 760 | + | 0 | 1 | CCGAAA |
| FAM311 | EECCRCAH1 | 780 | 786 | + | 0 | 1 | GATTTTC |

(continued)

| IUPAC Family | IUPAC | Start pos. | End pos. | Strand | Mismatches | Score | Sequence |
|---|---|---|---|---|---|---|---|
| FAM304 | OSE2ROOTNODULE | 786 | 790 | + | 0 | 1 | CTCTT |
| FAM002 | SORLIP1AT | 795 | 807 | + | 0 | 1 | GTATCTGCCACGC |
| FAM002 | SORLIP1AT | 821 | 833 | + | 0 | 1 | TCTATGGCCACTG |
| FAM304 | OSE2ROOTNODULE | 843 | 847 | - | 0 | 1 | CTCTT |
| FAM304 | OSE2ROOTNODULE | 865 | 869 | + | 0 | 1 | CTCTT |
| FAM263 | DPBFCOREDCDC3 | 910 | 916 | + | 0 | 1 | ACACGAG |
| FAM107 | CGACGOSAMY3 | 932 | 936 | - | 0 | 1 | CGACG |
| FAM263 | DPBFCOREDCDC3 | 945 | 951 | - | 0 | 1 | ACACTTG |
| FAM061 | GCCCORE | 954 | 960 | - | 0 | 1 | TGCCGCC |
| FAM171 | MYBPZM | 993 | 999 | + | 0 | 1 | CCCAACC |
| FAM171 | MYBPZM | 1001 | 1007 | - | 0 | 1 | GCCTACC |
| FAM010 | WBOXATNPR1 | 1023 | 1037 | - | 0 | 1 | CTTGACACAATCTGA |
| FAM322 | BIHD1OS | 1031 | 1035 | + | 0 | 1 | TGTCA |
| FAM008 | MYB2AT | 1047 | 1057 | + | 0 | 1 | GTGATAACTGA |
| FAM012 | IBOXCORE | 1049 | 1055 | + | 0 | 1 | GATAACT |
| FAM002 | SORLIP1AT | 1055 | 1067 | + | 0 | 1 | TGATAAGCCACTG |
| FAM012 | IBOX | 1056 | 1062 | + | 0 | 1 | GATAAGC |
| FAM002 | RAV1BAT | 1084 | 1096 | - | 0 | 1 | CGTCACCTGCAGC |
| FAM151 | INTRONLOWER | 1086 | 1091 | + | 0 | 1 | TGCAGG |
| FAM002 | ASF1MOTIFCAMV | 1087 | 1099 | + | 0 | 1 | GCAGGTGACGAAG |
| FAM324 | CGCGBOXAT | 1113 | 1118 | + | 0 | 1 | GCGCGG |
| FAM324 | CGCGBOXAT | 1113 | 1118 | - | 0 | 1 | CCGCGC |
| FAM325 | MYBCOREATCYCB1 | 1130 | 1134 | - | 0 | 1 | AACGG |
| FAM061 | GCCCORE | 1137 | 1143 | - | 0 | 1 | TGCCGCC |
| FAM002 | CACGTGMOTIF | 1138 | 1150 | - | 0 | 1 | CGTCACGTGCCGC |

(continued)

| IUPAC Family | IUPAC | Start pos. | End pos. | Strand | Mismatches | Score | Sequence |
|---|---|---|---|---|---|---|---|
| FAM002 | CACGTGMOTIF | 1139 | 1151 | + | 0 | 1 | CGGCACGTGACGA |
| FAM002 | ASF1MOTIFCAMV | 1141 | 1153 | + | 0 | 1 | GCACGTGACGAGC |
| FAM061 | GCCCORE | 1156 | 1162 | - | 0 | 1 | CGCCGCC |
| FAM311 | EECCRCAH1 | 1166 | 1172 | + | 0 | 1 | GACTTCC |
| FAM263 | DPBFCOREDCDC3 | 1172 | 1178 | - | 0 | 1 | ACACCAG |
| FAM013 | LTRECOREATCOR15 | 1185 | 1191 | - | 0 | 1 | TCCGACC |
| FAM324 | CGCGBOXAT | 1191 | 1196 | + | 0 | 1 | ACGCGT |
| FAM324 | CGCGBOXAT | 1191 | 1196 | - | 0 | 1 | ACGCGT |
| FAM002 | ASF1MOTIFCAMV | 1198 | 1210 | + | 0 | 1 | CGTGATGACGCAC |
| FAM307 | ANAERO3CONSENSUS | 1199 | 1205 | - | 0 | 1 | TCATCAC |
| FAM061 | GCCCORE | 1211 | 1217 | - | 0 | 1 | TGCCGCC |
| FAM262 | CIACADIANLELHC | 1225 | 1234 | + | 0 | 1 | CAAACTCATC |
| FAM292 | PREATPRODH | 1228 | 1233 | + | 0 | 1 | ACTCAT |
| FAM324 | CGCGBOXAT | 1241 | 1246 | + | 0 | 1 | GCGCGC |
| FAM324 | CGCGBOXAT | 1241 | 1246 | - | 0 | 1 | GCGCGC |
| FAM324 | CGCGBOXAT | 1251 | 1256 | + | 0 | 1 | ACGCGG |
| FAM324 | CGCGBOXAT | 1251 | 1256 | - | 0 | 1 | CCGCGT |
| FAM013 | DRE2COREZMRAB17 | 1269 | 1275 | - | 0 | 1 | ACCGACT |
| FAM013 | LTRECOREATCOR15 | 1280 | 1286 | + | 0 | 1 | CCCGACA |
| FAM302 | SORLIP2AT | 1287 | 1297 | + | 0 | 1 | AGGGCCTCATG |
| FAM013 | LTRECOREATCOR15 | 1316 | 1322 | + | 0 | 1 | CCCGACA |
| FAM302 | SITEIIATCYTC | 1323 | 1333 | + | 0 | 1 | TGGGCTGGGCC |
| FAM302 | SITEIIATCYTC | 1328 | 1338 | + | 0 | 1 | TGGGCCTCCTT |
| FAM057 | ACGTCBOX | 1346 | 1351 | + | 0 | 1 | GACGTC |
| FAM057 | ACGTCBOX | 1346 | 1351 | - | 0 | 1 | GACGTC |

(continued)

| IUPAC Family | IUPAC | Start pos. | End pos. | Strand | Mismatches | Score | Sequence |
|---|---|---|---|---|---|---|---|
| FAM107 | CGACGOSAMY3 | 1348 | 1352 | - | 0 | 1 | CGACG |
| FAM002 | ASF1MOTIFCAMV | 1351 | 1363 | - | 0 | 1 | GTGCGTGACGACG |
| FAM107 | CGACGOSAMY3 | 1351 | 1355 | - | 0 | 1 | CGACG |
| FAM194 | PALBOXAPC | 1370 | 1376 | - | 0 | 1 | CCGTCCT |
| FAM302 | SORLIP2AT | 1374 | 1384 | + | 0 | 1 | CGGGCCTCCCC |
| FAM302 | SITEIIATCYTC | 1381 | 1391 | - | 0 | 1 | TGGGCTCGGGG |
| FAM171 | BOXLCOREDCPAL | 1391 | 1397 | + | 0 | 1 | ACCTTCC |
| FAM089 | BS1EGCCR | 1420 | 1425 | - | 0 | 1 | AGCGGG |
| FAM322 | BIHD1OS | 1425 | 1429 | - | 0 | 1 | TGTCA |
| FAM026 | RYREPEATBNNAPA | 1430 | 1440 | - | 0 | 1 | CACATGCAGGG |
| FAM151 | INTRONLOWER | 1431 | 1436 | - | 0 | 1 | TGCAGG |
| FAM172 | MYCATRD2 | 1434 | 1440 | - | 0 | 1 | CACATGC |
| FAM172 | MYCATERD | 1435 | 1441 | + | 0 | 1 | CATGTGC |
| FAM151 | INTRONLOWER | 1439 | 1444 | + | 0 | 1 | TGCAGG |
| FAM069 | SURECOREATSULTR11 | 1449 | 1455 | + | 0 | 1 | GAGACGG |
| FAM302 | SORLIP2AT | 1453 | 1463 | + | 0 | 1 | CGGGCCATCCC |
| FAM002 | SORLIP1AT | 1472 | 1484 | - | 0 | 1 | CAGACGGCCACTC |
| FAM069 | SURECOREATSULTR11 | 1521 | 1527 | - | 0 | 1 | GAGACTA |
| FAM324 | CGCGBOXAT | 1544 | 1549 | + | 0 | 1 | GCGCGC |
| FAM324 | CGCGBOXAT | 1544 | 1549 | - | 0 | 1 | GCGCGC |
| FAM295 | P1BS | 1560 | 1567 | + | 0 | 1 | GCATATGC |
| FAM295 | P1BS | 1560 | 1567 | - | 0 | 1 | GCATATGC |
| FAM098 | CATATGGMSAUR | 1561 | 1566 | + | 0 | 1 | CATATG |
| FAM098 | CATATGGMSAUR | 1561 | 1566 | - | 0 | 1 | CATATG |
| FAM012 | IBOXCORE | 1674 | 1680 | + | 0 | 1 | GATAACA |

(continued)

| IUPAC Family | IUPAC | Start pos. | End pos. | Strand | Mismatches | Score | Sequence |
|---|---|---|---|---|---|---|---|
| FAM304 | OSE2ROOTNODULE | 1709 | 1713 | + | 0 | 1 | CTCTT |
| FAM209 | RBCSCONSENSUS | 1712 | 1718 | - | 0 | 1 | AATCCAA |
| FAM311 | EECCRCAH1 | 1715 | 1721 | + | 0 | 1 | GATTTAC |
| FAM010 | WBBOXPCWRKY1 | 1753 | 1767 | + | 0 | 1 | TTTGACTTGCAGCCT |
| FAM311 | EECCRCAH1 | 1756 | 1762 | + | 0 | 1 | GACTTGC |
| FAM151 | INTRONLOWER | 1768 | 1773 | + | 0 | 1 | TGCAGG |
| FAM002 | TGACGTVMAMY | 1771 | 1783 | + | 0 | 1 | AGGCATGACGTGG |
| FAM002 | HEXMOTIFTAH3H4 | 1773 | 1785 | - | 0 | 1 | GCCCACGTCATGC |
| FAM002 | ABREOSRAB21 | 1774 | 1786 | + | 0 | 1 | CATGACGTGGGCG |
| FAM002 | UPRMOTIFIAT | 1775 | 1787 | - | 0 | 1 | TCGCCCACGTCAT |
| FAM311 | EECCRCAH1 | 1807 | 1813 | + | 0 | 1 | GAGTTTC |
| FAM272 | SV40COREENHAN | 1813 | 1820 | - | 0 | 1 | GTGGAAAG |
| FAM324 | CGCGBOXAT | 1819 | 1824 | + | 0 | 1 | ACGCGG |
| FAM324 | CGCGBOXAT | 1819 | 1824 | - | 0 | 1 | CCGCGT |
| FAM002 | SORLIP1AT | 1851 | 1863 | + | 0 | 1 | CTGCCCGCCACGT |
| FAM002 | ACGTABREMOTIFA20SE | 1855 | 1867 | - | 0 | 1 | GAGAACGTGGCGG |
| FAM151 | INTRONLOWER | 1867 | 1872 | - | 0 | 1 | TGCAGG |
| FAM311 | EECCRCAH1 | 1878 | 1884 | - | 0 | 1 | GAGTTGC |
| FAM260 | CAREOSREP1 | 1879 | 1884 | + | 0 | 1 | CAACTC |
| FAM242 | TATABOX3 | 1900 | 1906 | + | 0 | 1 | TATTAAT |
| FAM288 | WUSATAg | 1906 | 1912 | + | 0 | 1 | TTAATGG |

7.) p-KG103

PLACE analysis results of p-KG103 are listed in Table 28. Two TATA Box motifs are found at nucleotide position 879-888 and 880-886 of the forward strand, respectively. No CAAT Box motif is found.

Table 28. PLACE analysis results of the 992bp promoter of p-KG103

| IUPAC Family | IUPAC | Start pos. | End pos. | Strand | Mismatches | Score | Sequence |
|---|---|---|---|---|---|---|---|
| FAM307 | ANAERO3CONSENSUS | 2 | 8 | + | 0 | 1 | TCATCAC |
| FAM065 | AMMORESIIUDCRNIA1 | 7 | 14 | - | 0 | 1 | GGTAGGGT |
| FAM171 | MYBPZM | 8 | 14 | + | 0 | 1 | CCCTACC |
| FAM010 | WBOXNTCHN48 | 19 | 33 | + | 0 | 1 | GCTGACTCGGGCCGC |
| FAM302 | SORLIP2AT | 26 | 36 | + | 0 | 1 | CGGGCCGCAGG |
| FAM263 | DPBFCOREDCDC3 | 45 | 51 | - | 0 | 1 | ACACCGG |
| FAM007 | AUXREPSIAA4 | 49 | 57 | + | 0 | 1 | TGTCCCATC |
| FAM002 | ASF1MOTIFCAMV | 99 | 111 | + | 0 | 1 | CTCTGTGACGACG |
| FAM107 | CGACGOSAMY3 | 107 | 111 | + | 0 | 1 | CGACG |
| FAM147 | HEXAMERATH4 | 107 | 112 | - | 0 | 1 | CCGTCG |
| FAM061 | AGCBOXNPGLB | 111 | 117 | - | 0 | 1 | AGCCGCC |
| FAM304 | OSE2ROOTNODULE | 138 | 142 | - | 0 | 1 | CTCTT |
| FAM002 | HEXMOTIFTAH3H4 | 140 | 152 | + | 0 | 1 | GAGGACGTCAGCA |
| FAM002 | TGACGTVMAMY | 142 | 154 | - | 0 | 1 | CTTGCTGACGTCC |
| FAM057 | ACGTCBOX | 143 | 148 | + | 0 | 1 | GACGTC |
| FAM057 | ACGTCBOX | 143 | 148 | - | 0 | 1 | GACGTC |
| FAM171 | MYBPZM | 157 | 163 | + | 0 | 1 | CCCAACC |
| FAM013 | LTRECOREATCOR15 | 166 | 172 | + | 0 | 1 | TCCGACA |
| FAM013 | LTRECOREATCOR15 | 202 | 208 | + | 0 | 1 | TCCGACG |
| FAM002 | SORLIP1AT | 203 | 215 | + | 0 | 1 | CCGACGGCCACGA |
| FAM107 | CGACGOSAMY3 | 204 | 208 | + | 0 | 1 | CGACG |
| FAM147 | HEXAMERATH4 | 204 | 209 | - | 0 | 1 | CCGTCG |
| FAM002 | SORLIP1AT | 245 | 257 | + | 0 | 1 | CCGGCTGCCACGA |
| FAM107 | CGACGOSAMY3 | 255 | 259 | + | 0 | 1 | CGACG |
| FAM147 | HEXAMERATH4 | 255 | 260 | - | 0 | 1 | CCGTCG |

(continued)

| IUPAC Family | IUPAC | Start pos. | End pos. | Strand | Mismatches | Score | Sequence |
|---|---|---|---|---|---|---|---|
| FAM089 | BS1EGCCR | 286 | 291 | - | 0 | 1 | AGCGGG |
| FAM002 | ABREMOTIFAOSOSEM | 291 | 303 | - | 0 | 1 | TGCTACGTGTCTA |
| FAM002 | RAV1BAT | 323 | 335 | + | 0 | 1 | GTACACCTGGATC |
| FAM263 | DPBFCOREDCDC3 | 325 | 331 | + | 0 | 1 | ACACCTG |
| FAM205 | PYRIMIDINEBOXOSRAM | 352 | 357 | - | 0 | 1 | CCTTTT |
| FAM302 | SORLIP2AT | 362 | 372 | - | 0 | 1 | AGGGCCCTGGT |
| FAM302 | SORLIP2AT | 365 | 375 | + | 0 | 1 | AGGGCCCTCTC |
| FAM171 | MYBPZM | 382 | 388 | - | 0 | 1 | GCCAACC |
| FAM324 | CGCGBOXAT | 388 | 393 | + | 0 | 1 | CCGCGC |
| FAM324 | CGCGBOXAT | 388 | 393 | - | 0 | 1 | GCGCGG |
| FAM324 | CGCGBOXAT | 390 | 395 | + | 0 | 1 | GCGCGG |
| FAM324 | CGCGBOXAT | 390 | 395 | - | 0 | 1 | CCGCGC |
| FAM069 | ARFAT | 406 | 412 | - | 0 | 1 | CTGTCTC |
| FAM069 | SURECOREATSULTR11 | 406 | 412 | + | 0 | 1 | GAGACAG |
| FAM271 | SEBFCONSSTPR10A | 406 | 412 | - | 0 | 1 | CTGTCTC |
| FAM302 | SORLIP2AT | 423 | 433 | + | 0 | 1 | GGGGCCGCTCG |
| FAM002 | ABREZMRAB28 | 440 | 452 | - | 0 | 1 | GTCCACGTGGGAG |
| FAM085 | BOXCPSAS1 | 440 | 446 | + | 0 | 1 | CTCCCAC |
| FAM002 | ABREZMRAB28 | 441 | 453 | + | 0 | 1 | TCCCACGTGGACG |
| FAM013 | LTRECOREATCOR15 | 479 | 485 | + | 0 | 1 | CCCGACC |
| FAM324 | CGCGBOXAT | 489 | 494 | + | 0 | 1 | GCGCGG |
| FAM324 | CGCGBOXAT | 489 | 494 | - | 0 | 1 | CCGCGC |
| FAM024 | CANBNNAPA | 497 | 505 | + | 0 | 1 | CGAACACGA |
| FAM324 | CGCGBOXAT | 509 | 514 | + | 0 | 1 | CCGCGG |
| FAM324 | CGCGBOXAT | 509 | 514 | - | 0 | 1 | CCGCGG |

(continued)

| IUPAC Family | IUPAC | Start pos. | End pos. | Strand | Mismatches | Score | Sequence |
|---|---|---|---|---|---|---|---|
| FAM069 | SURECOREATSULTR11 | 535 | 541 | - | 0 | 1 | GAGACCG |
| FAM061 | GCCCORE | 543 | 549 | + | 0 | 1 | GGCCGCC |
| FAM302 | SITEIIATCYTC | 592 | 602 | + | 0 | 1 | TGGGCTGGGGC |
| FAM324 | CGCGBOXAT | 603 | 608 | + | 0 | 1 | ACGCGG |
| FAM324 | CGCGBOXAT | 603 | 608 | - | 0 | 1 | CCGCGT |
| FAM315 | SORLIP5AT | 614 | 620 | - | 0 | 1 | GAGTGAG |
| FAM302 | SITEIIATCYTC | 619 | 629 | - | 0 | 1 | TGGGCCGACGA |
| FAM013 | DRECRTCOREAT | 620 | 626 | - | 0 | 1 | GCCGACG |
| FAM107 | CGACGOSAMY3 | 620 | 624 | - | 0 | 1 | CGACG |
| FAM069 | SURECOREATSULTR11 | 639 | 645 | - | 0 | 1 | GAGACCG |
| FAM013 | DRECRTCOREAT | 651 | 657 | + | 0 | 1 | GCCGACA |
| FAM087 | BOXIINTPATPB | 667 | 672 | + | 0 | 1 | ATAGAA |
| FAM173 | NAPINMOTIFBN | 683 | 689 | + | 0 | 1 | TACACAT |
| FAM172 | MYCATERD | 684 | 690 | - | 0 | 1 | CATGTGT |
| FAM263 | DPBFCOREDCDC3 | 684 | 690 | + | 0 | 1 | ACACATG |
| FAM026 | RYREPEATBNNAPA | 685 | 695 | + | 0 | 1 | CACATGCAATT |
| FAM172 | MYCATRD2 | 685 | 691 | + | 0 | 1 | CACATGC |
| FAM012 | IBOXCORE | 706 | 712 | + | 0 | 1 | GATAATA |
| FAM099 | CCA1ATLHCB1 | 725 | 732 | - | 0 | 1 | AAAAATCT |
| FAM290 | GT1GMSCAM4 | 728 | 733 | - | 0 | 1 | GAAAAA |
| FAM012 | IBOX | 735 | 741 | + | 0 | 1 | GATAAGT |
| FAM266 | MYB1AT | 744 | 749 | + | 0 | 1 | AAACCA |
| FAM003 | REALPHALGLHCB21 | 745 | 755 | + | 0 | 1 | AACCAAATATT |
| FAM002 | SORLIP1AT | 755 | 767 | + | 0 | 1 | TTCACCGCCACAA |
| FAM205 | PYRIMIDINEBOXOSRAM | 766 | 771 | - | 0 | 1 | CCTTTT |

(continued)

| IUPAC Family | IUPAC | Start pos. | End pos. | Strand | Mismatches | Score | Sequence |
|---|---|---|---|---|---|---|---|
| FAM008 | MYB2AT | 782 | 792 | - | 0 | 1 | TGGGTAACTGA |
| FAM266 | MYB1AT | 800 | 805 | + | 0 | 1 | AAACCA |
| FAM003 | REALPHALGLHCB21 | 801 | 811 | + | 0 | 1 | AACCAAAAATAC |
| FAM263 | DPBFCOREDCDC3 | 811 | 817 | - | 0 | 1 | ACACAAG |
| FAM302 | SITEIIATCYTC | 826 | 836 | - | 0 | 1 | TGGGCTCTTGG |
| FAM304 | OSE2ROOTNODULE | 828 | 832 | - | 0 | 1 | CTCTT |
| FAM272 | SV40COREENHAN | 835 | 842 | - | 0 | 1 | GTGGTTTG |
| FAM266 | MYB1AT | 836 | 841 | + | 0 | 1 | AAACCA |
| FAM302 | SITEIIATCYTC | 854 | 864 | - | 0 | 1 | TGGGCTGGGGG |
| FAM240 | TATABOX1 | 879 | 888 | + | 0 | 1 | CTATAAATAC |
| FAM241 | TATABOX2 | 880 | 886 | + | 0 | 1 | TATAAAT |
| FAM002 | SORLIP1AT | 897 | 909 | - | 0 | 1 | ACTTCGGCCACCG |
| FAM315 | SORLIP5AT | 924 | 930 | - | 0 | 1 | GAGTGAG |
| FAM292 | PREATPRODH | 927 | 932 | + | 0 | 1 | ACTCAT |
| FAM306 | ANAERO2CONSENSUS | 955 | 960 | + | 0 | 1 | AGCAGC |
| FAM306 | ANAERO2CONSENSUS | 968 | 973 | + | 0 | 1 | AGCAGC |
| FAM260 | CAREOSREP1 | 983 | 988 | + | 0 | 1 | CAACTC |

**8.) p-KG119**

PLACE analysis results of p-KG119 are listed in Table 29. Two TATA Box motifs are found at nucleotide position 1925-1931 and 1998-2004 of the forward strand respectively. One CAAT Box motif is found at nucleotide position 214-218 of the forward strand.

Table 29. PLACE analysis results of the 2519bp promoter of p-KG119

| IUPAC Family | IUPAC | Start pos. | End pos. | Strand | Mismatches | Score | Sequence |
|---|---|---|---|---|---|---|---|
| FAM012 | IBOXCORE | 6 | 12 | + | 0 | 1 | GATAAAA |
| FAM151 | INTRONLOWER | 29 | 34 | - | 0 | 1 | TGCAGG |
| FAM227 | SEF1MOTIF | 34 | 42 | + | 0 | 1 | ATATTTATT |
| FAM012 | IBOXCORE | 58 | 64 | + | 0 | 1 | GATAACC |
| FAM325 | MYBCOREATCYCB1 | 63 | 67 | - | 0 | 1 | AACGG |
| FAM262 | CIACADIANLELHC | 93 | 102 | + | 0 | 1 | CAACTAAATC |
| FAM221 | S1FBOXSORPS1L21 | 105 | 110 | - | 0 | 1 | ATGGTA |
| FAM002 | RAV1BAT | 114 | 126 | - | 0 | 1 | TTTCACCTGTCAC |
| FAM271 | SEBFCONSSTPR10A | 114 | 120 | - | 0 | 1 | CTGTCAC |
| FAM322 | BIHD1OS | 115 | 119 | - | 0 | 1 | TGTCA |
| FAM100 | CCAATBOX1 | 126 | 130 | - | 0 | 1 | CCAAT |
| FAM262 | CIACADIANLELHC | 138 | 147 | - | 0 | 1 | CAAGCTGATC |
| FAM170 | AMYBOX1 | 150 | 156 | + | 0 | 1 | TAACAGA |
| FAM303 | OSE1ROOTNODULE | 161 | 167 | + | 0 | 1 | AAAGATA |
| FAM012 | IBOXCORE | 164 | 170 | + | 0 | 1 | GATAAAT |
| FAM266 | MYB1AT | 184 | 189 | + | 0 | 1 | TAACCA |
| FAM276 | TRANSINITDICOTS | 186 | 193 | + | 0 | 1 | ACCATGGC |
| FAM304 | OSE2ROOTNODULE | 209 | 213 | - | 0 | 1 | CTCTT |
| FAM100 | CCAATBOX1 | 214 | 218 | + | 0 | 1 | CCAAT |
| FAM267 | TAAAGSTKST1 | 219 | 225 | + | 0 | 1 | TCTAAAG |
| FAM008 | MYB2AT | 244 | 254 | - | 0 | 1 | TGCCTAACTGC |
| FAM305 | ANAERO1CONSENSUS | 262 | 268 | + | 0 | 1 | AAACAAA |
| FAM008 | MYB2AT | 274 | 284 | + | 0 | 1 | CAGCTAACTGC |
| FAM010 | WBOXATNPR1 | 281 | 295 | - | 0 | 1 | TTTGACACTTAGCAG |
| FAM322 | BIHD1OS | 289 | 293 | + | 0 | 1 | TGTCA |

(continued)

| IUPAC Family | IUPAC | Start pos. | End pos. | Strand | Mismatches | Score | Sequence |
|---|---|---|---|---|---|---|---|
| FAM030 | -300CORE | 300 | 308 | - | 0 | 1 | TGTAAAGCA |
| FAM267 | TAAAGSTKST1 | 302 | 308 | - | 0 | 1 | TGTAAAG |
| FAM267 | TAAAGSTKST1 | 308 | 314 | + | 0 | 1 | ACTAAAG |
| FAM021 | GT1CORE | 314 | 324 | + | 0 | 1 | GGGTTAAATAT |
| FAM244 | TATABOXOSPAL | 317 | 323 | - | 0 | 1 | TATTTAA |
| FAM306 | ANAERO2CONSENSUS | 359 | 364 | + | 0 | 1 | AGCAGC |
| FAM306 | ANAERO2CONSENSUS | 362 | 367 | + | 0 | 1 | AGCAGC |
| FAM098 | CATATGGMSAUR | 408 | 413 | + | 0 | 1 | CATATG |
| FAM098 | CATATGGMSAUR | 408 | 413 | - | 0 | 1 | CATATG |
| FAM012 | IBOX | 415 | 421 | - | 0 | 1 | GATAAGT |
| FAM014 | SREATMSD | 416 | 422 | + | 0 | 1 | CTTATCC |
| FAM014 | MYBST1 | 417 | 423 | - | 0 | 1 | TGGATAA |
| FAM025 | AMYBOX2 | 418 | 424 | + | 0 | 1 | TATCCAT |
| FAM273 | TATCCAOSAMY | 418 | 424 | + | 0 | 1 | TATCCAT |
| FAM315 | SORLIP5AT | 434 | 440 | - | 0 | 1 | GAGTGAG |
| FAM292 | PREATPRODH | 437 | 442 | + | 0 | 1 | ACTCAT |
| FAM270 | RAV1AAT | 476 | 480 | + | 0 | 1 | CAACA |
| FAM311 | EECCRCAH1 | 520 | 526 | + | 0 | 1 | GAATTCC |
| FAM310 | CPBCSPOR | 530 | 535 | - | 0 | 1 | TATTAG |
| FAM234 | SP8BFIBSP8BIB | 535 | 541 | - | 0 | 1 | TACTATT |
| FAM012 | IBOXCORE | 563 | 569 | + | 0 | 1 | GATAATT |
| FAM234 | SP8BFIBSP8BIB | 595 | 601 | + | 0 | 1 | TACTATT |
| FAM310 | CPBCSPOR | 601 | 606 | + | 0 | 1 | TATTAG |
| FAM014 | MYBST1 | 611 | 617 | - | 0 | 1 | AGGATAT |
| FAM304 | OSE2ROOTNODULE | 660 | 664 | - | 0 | 1 | CTCTT |

(continued)

| IUPAC Family | IUPAC | Start pos. | End pos. | Strand | Mismatches | Score | Sequence |
|---|---|---|---|---|---|---|---|
| FAM322 | BIHD1OS | 665 | 669 | + | 0 | 1 | TGTCA |
| FAM305 | ANAERO1CONSENSUS | 687 | 693 | + | 0 | 1 | AAACAAA |
| FAM026 | RYREPEATGMGY2 | 699 | 709 | + | 0 | 1 | ATCATGCATAA |
| FAM267 | NTBBF1ARROLB | 760 | 766 | + | 0 | 1 | ACTTTAG |
| FAM267 | TAAAGSTKST1 | 761 | 767 | - | 0 | 1 | TCTAAAG |
| FAM010 | WBOXATNPR1 | 771 | 785 | - | 0 | 1 | TTTGACATTCCACCA |
| FAM272 | SV40COREENHAN | 773 | 780 | + | 0 | 1 | GTGGAATG |
| FAM322 | BIHD1OS | 779 | 783 | + | 0 | 1 | TGTCA |
| FAM267 | NTBBF1ARROLB | 804 | 810 | + | 0 | 1 | ACTTTAT |
| FAM267 | TAAAGSTKST1 | 805 | 811 | - | 0 | 1 | AATAAAG |
| FAM209 | RBCSCONSENSUS | 842 | 848 | + | 0 | 1 | AATCCAA |
| FAM002 | ASF1MOTIFCAMV | 852 | 864 | - | 0 | 1 | TTACCTGACGGGG |
| FAM311 | EECCRCAH1 | 861 | 867 | - | 0 | 1 | GAATTAC |
| FAM270 | RAV1AAT | 869 | 873 | + | 0 | 1 | CAACA |
| FAM013 | LTRECOREATCOR15 | 877 | 883 | - | 0 | 1 | CCCGACA |
| FAM061 | GCCCORE | 890 | 896 | - | 0 | 1 | CGCCGCC |
| FAM171 | MYBPZM | 946 | 952 | + | 0 | 1 | TCCAACC |
| FAM228 | SEF3MOTIFGM | 949 | 954 | + | 0 | 1 | AACCCA |
| FAM171 | MYBPZM | 951 | 957 | + | 0 | 1 | CCCAACC |
| FAM324 | CGCGBOXAT | 971 | 976 | + | 0 | 1 | ACGCGG |
| FAM324 | CGCGBOXAT | 971 | 976 | - | 0 | 1 | CCGCGT |
| FAM190 | OCTAMERMOTIFTAH3H4 | 972 | 979 | + | 0 | 1 | CGCGGATC |
| FAM107 | CGACGOSAMY3 | 984 | 988 | - | 0 | 1 | CGACG |
| FAM245 | TBOXATGAPB | 1029 | 1034 | - | 0 | 1 | ACTTTG |
| FAM270 | RAV1AAT | 1048 | 1052 | - | 0 | 1 | CAACA |

(continued)

| IUPAC Family | IUPAC | Start pos. | End pos. | Strand | Mismatches | Score | Sequence |
|---|---|---|---|---|---|---|---|
| FAM262 | CIACADIANLELHC | 1052 | 1061 | - | 0 | 1 | CAACATAATC |
| FAM270 | RAV1AAT | 1057 | 1061 | - | 0 | 1 | CAACA |
| FAM273 | TATCCAOSAMY | 1059 | 1065 | - | 0 | 1 | TATCCAA |
| FAM014 | MYBST1 | 1060 | 1066 | + | 0 | 1 | TGGATAA |
| FAM014 | SREATMSD | 1061 | 1067 | - | 0 | 1 | GTTATCC |
| FAM012 | IBOXCORE | 1062 | 1068 | + | 0 | 1 | GATAACC |
| FAM012 | IBOXCORENT | 1097 | 1103 | - | 0 | 1 | GATAAGG |
| FAM087 | BOXIINTPATPB | 1127 | 1132 | - | 0 | 1 | ATAGAA |
| FAM105 | CEREGLUBOX2PSLEGA | 1133 | 1140 | + | 0 | 1 | TGAAAACT |
| FAM292 | PREATPRODH | 1138 | 1143 | + | 0 | 1 | ACTCAT |
| FAM027 | -10PEHVPSBD | 1145 | 1150 | - | 0 | 1 | TATTCT |
| FAM012 | IBOXCORE | 1160 | 1166 | + | 0 | 1 | GATAACA |
| FAM270 | RAV1AAT | 1171 | 1175 | + | 0 | 1 | CAACA |
| FAM100 | CCAATBOX1 | 1209 | 1213 | - | 0 | 1 | CCAAT |
| FAM311 | EECCRCAH1 | 1220 | 1226 | - | 0 | 1 | GACTTCC |
| FAM013 | DRECRTCOREAT | 1223 | 1229 | - | 0 | 1 | GCCGACT |
| FAM013 | LTRECOREATCOR15 | 1244 | 1250 | + | 0 | 1 | CCCGACT |
| FAM311 | EECCRCAH1 | 1280 | 1286 | - | 0 | 1 | GATTTCC |
| FAM325 | MYBCOREATCYCB1 | 1292 | 1296 | + | 0 | 1 | AACGG |
| FAM024 | 2SSEEDPROTBANAPA | 1306 | 1314 | - | 0 | 1 | CAAACACTC |
| FAM310 | CPBCSPOR | 1327 | 1332 | - | 0 | 1 | TATTAG |
| FAM002 | SORLIP1AT | 1337 | 1349 | - | 0 | 1 | ATTTTAGCCACTA |
| FAM069 | ARFAT | 1356 | 1362 | - | 0 | 1 | ATGTCTC |
| FAM069 | SURECOREATSULTR11 | 1356 | 1362 | + | 0 | 1 | GAGACAT |
| FAM024 | PROXBBNNAPA | 1364 | 1372 | + | 0 | 1 | CAAACACCC |

(continued)

| IUPAC Family | IUPAC | Start pos. | End pos. | Strand | Mismatches | Score | Sequence |
|---|---|---|---|---|---|---|---|
| FAM310 | CPBCSPOR | 1376 | 1381 | - | 0 | 1 | TATTAG |
| FAM300 | LECPLEACS2 | 1379 | 1386 | - | 0 | 1 | TAAAATAT |
| FAM310 | CPBCSPOR | 1433 | 1438 | + | 0 | 1 | TATTAG |
| FAM170 | MYBGAHV | 1453 | 1459 | + | 0 | 1 | TAACAAA |
| FAM281 | MYB1LEPR | 1469 | 1475 | - | 0 | 1 | GTTAGTT |
| FAM024 | 2SSEEDPROTBANAPA | 1481 | 1489 | + | 0 | 1 | CAAACACTG |
| FAM010 | WBOXNTCHN48 | 1518 | 1532 | + | 0 | 1 | TCTGACTGGCCAGCC |
| FAM302 | SITEIIATCYTC | 1524 | 1534 | - | 0 | 1 | TGGGCTGGCCA |
| FAM013 | DRECRTCOREAT | 1559 | 1565 | + | 0 | 1 | GCCGACC |
| FAM061 | GCCCORE | 1568 | 1574 | - | 0 | 1 | GGCCGCC |
| FAM151 | INTRONLOWER | 1573 | 1578 | - | 0 | 1 | TGCAGG |
| FAM012 | IBOXCORE | 1596 | 1602 | - | 0 | 1 | GATAAAA |
| FAM267 | NTBBF1ARROLB | 1618 | 1624 | + | 0 | 1 | ACTTTAT |
| FAM267 | TAAAGSTKST1 | 1619 | 1625 | - | 0 | 1 | TATAAAG |
| FAM010 | WBOXATNPR1 | 1623 | 1637 | - | 0 | 1 | GTTGACAAAGAATAT |
| FAM027 | -10PEHVPSBD | 1624 | 1629 | + | 0 | 1 | TATTCT |
| FAM329 | XYLAT | 1626 | 1633 | - | 0 | 1 | ACAAAGAA |
| FAM322 | BIHD1OS | 1631 | 1635 | + | 0 | 1 | TGTCA |
| FAM003 | REALPHALGLHCB21 | 1635 | 1645 | + | 0 | 1 | AACCAAATACT |
| FAM304 | OSE2ROOTNODULE | 1652 | 1656 | + | 0 | 1 | CTCTT |
| FAM030 | EMHVCHORD | 1684 | 1692 | + | 0 | 1 | TGTAAAGTT |
| FAM202 | -300ELEMENT | 1684 | 1692 | + | 0 | 1 | TGTAAAGTT |
| FAM267 | TAAAGSTKST1 | 1684 | 1690 | + | 0 | 1 | TGTAAAG |
| FAM267 | NTBBF1ARROLB | 1685 | 1691 | - | 0 | 1 | ACTTTAC |
| FAM267 | NTBBF1ARROLB | 1715 | 1721 | + | 0 | 1 | ACTTTAA |

(continued)

| IUPAC Family | IUPAC | Start pos. | End pos. | Strand | Mismatches | Score | Sequence |
|---|---|---|---|---|---|---|---|
| FAM267 | TAAAGSTKST1 | 1716 | 1722 | - | 0 | 1 | TTTAAAG |
| FAM003 | REALPHALGLHCB21 | 1720 | 1730 | - | 0 | 1 | AACCAACTTTT |
| FAM169 | MYBATRD2 | 1726 | 1732 | - | 0 | 1 | CTAACCA |
| FAM266 | MYB1AT | 1726 | 1731 | - | 0 | 1 | TAACCA |
| FAM013 | DRECRTCOREAT | 1749 | 1755 | + | 0 | 1 | GCCGACT |
| FAM311 | EECCRCAH1 | 1762 | 1768 | - | 0 | 1 | GATTTGC |
| FAM010 | WBOXNTCHN48 | 1809 | 1823 | + | 0 | 1 | TCTGACCGATTTTGA |
| FAM021 | GT1CORE | 1835 | 1845 | + | 0 | 1 | AGGTTAATTCT |
| FAM013 | LTRECOREATCOR15 | 1859 | 1865 | + | 0 | 1 | TCCGACC |
| FAM267 | TAAAGSTKST1 | 1886 | 1892 | + | 0 | 1 | ACTAAAG |
| FAM039 | AACACOREOSGLUB1 | 1897 | 1903 | - | 0 | 1 | AACAAAC |
| FAM305 | ANAERO1CONSENSUS | 1898 | 1904 | - | 0 | 1 | AAACAAA |
| FAM243 | TATABOX4 | 1924 | 1930 | - | 0 | 1 | TATATAA |
| FAM243 | TATABOX4 | 1925 | 1931 | + | 0 | 1 | TATATAA |
| FAM281 | MYB1LEPR | 1946 | 1952 | - | 0 | 1 | GTTAGTT |
| FAM024 | CANBNNAPA | 1948 | 1956 | + | 0 | 1 | CTAACACTT |
| FAM027 | -10PEHVPSBD | 1985 | 1990 | - | 0 | 1 | TATTCT |
| FAM227 | SEF1MOTIF | 1993 | 2001 | + | 0 | 1 | ATATTTATA |
| FAM019 | TATAPVTRNALEU | 1995 | 2007 | + | 0 | 1 | ATTTATATAATTC |
| FAM241 | TATABOX2 | 1995 | 2001 | - | 0 | 1 | TATAAAT |
| FAM243 | TATABOX4 | 1997 | 2003 | - | 0 | 1 | TATATAA |
| FAM243 | TATABOX4 | 1998 | 2004 | + | 0 | 1 | TATATAA |
| FAM305 | ANAERO1CONSENSUS | 2009 | 2015 | + | 0 | 1 | AAACAAA |
| FAM310 | CPBCSPOR | 2021 | 2026 | - | 0 | 1 | TATTAG |
| FAM266 | MYB1AT | 2034 | 2039 | - | 0 | 1 | AAACCA |

(continued)

| IUPAC Family | IUPAC | Start pos. | End pos. | Strand | Mismatches | Score | Sequence |
|---|---|---|---|---|---|---|---|
| FAM270 | RAV1AAT | 2057 | 2061 | - | 0 | 1 | CAACA |
| FAM324 | CGCGBOXAT | 2071 | 2076 | + | 0 | 1 | GCGCGC |
| FAM324 | CGCGBOXAT | 2071 | 2076 | - | 0 | 1 | GCGCGC |
| FAM270 | RAV1AAT | 2077 | 2081 | + | 0 | 1 | CAACA |
| FAM013 | DRE2COREZMRAB17 | 2112 | 2118 | + | 0 | 1 | ACCGACT |
| FAM234 | SP8BFIBSP8BIB | 2120 | 2126 | + | 0 | 1 | TACTATT |
| FAM300 | LECPLEACS2 | 2129 | 2136 | + | 0 | 1 | TAAAATAT |
| FAM124 | ERELEE4 | 2137 | 2144 | + | 0 | 1 | AATTCAAA |
| FAM061 | GCCCORE | 2189 | 2195 | - | 0 | 1 | CGCCGCC |
| FAM107 | CGACGOSAMY3 | 2194 | 2198 | - | 0 | 1 | CGACG |
| FAM010 | WBOXATNPR1 | 2211 | 2225 | - | 0 | 1 | GTTGACGCATGGTGC |
| FAM002 | ASF1MOTIFCAMV | 2216 | 2228 | - | 0 | 1 | AGCGTTGACGCAT |
| FAM324 | CGCGBOXAT | 2244 | 2249 | + | 0 | 1 | GCGCGC |
| FAM324 | CGCGBOXAT | 2244 | 2249 | - | 0 | 1 | GCGCGC |
| FAM270 | RAV1AAT | 2250 | 2254 | - | 0 | 1 | CAACA |
| FAM324 | CGCGBOXAT | 2269 | 2274 | + | 0 | 1 | ACGCGT |
| FAM324 | CGCGBOXAT | 2269 | 2274 | - | 0 | 1 | ACGCGT |
| FAM002 | SORLIP1AT | 2271 | 2283 | - | 0 | 1 | CCATTTGCCACGC |
| FAM026 | RYREPEATGMGY2 | 2282 | 2292 | + | 0 | 1 | GGCATGCATTC |
| FAM013 | LTRECOREATCOR15 | 2320 | 2326 | + | 0 | 1 | CCCGACG |
| FAM107 | CGACGOSAMY3 | 2322 | 2326 | + | 0 | 1 | CGACG |
| FAM324 | CGCGBOXAT | 2324 | 2329 | + | 0 | 1 | ACGCGG |
| FAM324 | CGCGBOXAT | 2324 | 2329 | - | 0 | 1 | CCGCGT |
| FAM002 | LRENPCABE | 2334 | 2346 | + | 0 | 1 | CAGGACGTGGCAG |
| FAM002 | SORLIP1AT | 2338 | 2350 | - | 0 | 1 | CGCTCTGCCACGT |

(continued)

| IUPAC Family | IUPAC | Start pos. | End pos. | Strand | Mismatches | Score | Sequence |
|---|---|---|---|---|---|---|---|
| FAM061 | GCCCORE | 2349 | 2355 | + | 0 | 1 | CGCCGCC |
| FAM267 | TAAAGSTKST1 | 2393 | 2399 | - | 0 | 1 | AATAAAG |
| FAM194 | PALBOXAPC | 2400 | 2406 | + | 0 | 1 | CCGTCCT |
| FAM010 | WBOXHVISO1 | 2404 | 2418 | - | 0 | 1 | TGTGACTGAGCAAGG |
| FAM315 | SORLIP5AT | 2430 | 2436 | - | 0 | 1 | GAGTGAG |
| FAM194 | PALBOXAPC | 2440 | 2446 | + | 0 | 1 | CCGTCCG |
| FAM069 | SURECOREATSULTR11 | 2459 | 2465 | - | 0 | 1 | GAGACGA |
| FAM085 | BOXCPSAS1 | 2463 | 2469 | + | 0 | 1 | CTCCCAC |
| FAM013 | LTRECOREATCOR15 | 2500 | 2506 | + | 0 | 1 | CCCGACG |
| FAM107 | CGACGOSAMY3 | 2502 | 2506 | + | 0 | 1 | CGACG |
| FAM324 | CGCGBOXAT | 2504 | 2509 | + | 0 | 1 | ACGCGC |
| FAM324 | CGCGBOXAT | 2504 | 2509 | - | 0 | 1 | GCGCGT |

9.) p-KG129

PLACE analysis results of p-KG129 are listed in Table 30. No TATA Box motifs are found in this promoter. One CAAT Box motif is found at nucleotide position 244-248 of the forward strand.

Table 30. PLACE analysis results of the 512bp promoter of p-KG129

| IUPAC Family | IUPAC | Start pos. | End pos. | Strand | Mismatches | Score | Sequence |
|---|---|---|---|---|---|---|---|
| FAM263 | DPBFCOREDCDC3 | 20 | 26 | + | 0 | 1 | ACACTAG |
| FAM089 | BS1EGCCR | 25 | 30 | + | 0 | 1 | AGCGGG |
| FAM306 | ANAERO2CONSENSUS | 30 | 35 | - | 0 | 1 | AGCAGC |
| FAM267 | TAAAGSTKST1 | 33 | 39 | + | 0 | 1 | GCTAAAG |
| FAM267 | TAAAGSTKST1 | 52 | 58 | + | 0 | 1 | GCTAAAG |
| FAM303 | OSE1ROOTNODULE | 55 | 61 | + | 0 | 1 | AAAGATA |
| FAM263 | DPBFCOREDCDC3 | 65 | 71 | + | 0 | 1 | ACACTAG |
| FAM003 | MYBPLANT | 70 | 80 | - | 0 | 1 | CACCAACCGCT |
| FAM171 | BOXLCOREDCPAL | 73 | 79 | - | 0 | 1 | ACCAACC |
| FAM290 | GT1GMSCAM4 | 94 | 99 | + | 0 | 1 | GAAAAA |
| FAM002 | HEXMOTIFTAH3H4 | 140 | 152 | + | 0 | 1 | AAAAACGTCAGTG |
| FAM002 | TGACGTVMAMY | 142 | 154 | - | 0 | 1 | TTCACTGACGTTT |
| FAM002 | ASF1MOTIFCAMV | 166 | 178 | - | 0 | 1 | TATAGTGACGATC |
| FAM087 | BOXIINTPATPB | 183 | 188 | - | 0 | 1 | ATAGAA |
| FAM272 | SV40COREENHAN | 196 | 203 | + | 0 | 1 | GTGGTTAG |
| FAM169 | MYBATRD2 | 197 | 203 | - | 0 | 1 | CTAACCA |
| FAM266 | MYB1AT | 197 | 202 | - | 0 | 1 | TAACCA |
| FAM266 | MYB1AT | 241 | 246 | + | 0 | 1 | AAACCA |
| FAM003 | REALPHALGLHCB21 | 242 | 252 | + | 0 | 1 | AACCAATACTA |
| FAM100 | CCAATBOX1 | 244 | 248 | + | 0 | 1 | CCAAT |
| FAM087 | BOXIINTPATPB | 289 | 294 | - | 0 | 1 | ATAGAA |
| FAM002 | ASF1MOTIFCAMV | 334 | 346 | - | 0 | 1 | TTCTGTGACGACG |
| FAM107 | CGACGOSAMY3 | 334 | 338 | - | 0 | 1 | CGACG |
| FAM061 | GCCCORE | 370 | 376 | - | 0 | 1 | GGCCGCC |
| FAM002 | SORLIP1AT | 372 | 384 | + | 0 | 1 | CGGCCGGCCACGT |

(continued)

| IUPAC Family | IUPAC | Start pos. | End pos. | Strand | Mismatches | Score | Sequence |
|---|---|---|---|---|---|---|---|
| FAM002 | ABREATCONSENSUS | 376 | 388 | - | 0 | 1 | GGGTACGTGGCCG |
| FAM324 | CGCGBOXAT | 394 | 399 | + | 0 | 1 | ACGCGT |
| FAM324 | CGCGBOXAT | 394 | 399 | - | 0 | 1 | ACGCGT |
| FAM107 | CGACGOSAMY3 | 397 | 401 | - | 0 | 1 | CGACG |
| FAM002 | GADOWNAT | 408 | 420 | - | 0 | 1 | CAACACGTGTCCT |
| FAM002 | CACGTGMOTIF | 409 | 421 | + | 0 | 1 | GGACACGTGTTGG |
| FAM263 | DPBFCOREDCDC3 | 411 | 417 | + | 0 | 1 | ACACGTG |
| FAM263 | DPBFCOREDCDC3 | 412 | 418 | - | 0 | 1 | ACACGTG |
| FAM024 | CANBNNAPA | 413 | 421 | - | 0 | 1 | CCAACACGT |
| FAM270 | RAV1AAT | 416 | 420 | - | 0 | 1 | CAACA |
| FAM010 | WBOXNTCHN48 | 421 | 435 | + | 0 | 1 | GCTGACCGGACAGTT |
| FAM087 | BOXIINTPATPB | 465 | 470 | + | 0 | 1 | ATAGAA |
| FAM107 | CGACGOSAMY3 | 479 | 483 | + | 0 | 1 | CGACG |
| FAM107 | CGACGOSAMY3 | 482 | 486 | + | 0 | 1 | CGACG |
| FAM147 | HEXAMERATH4 | 482 | 487 | - | 0 | 1 | CCGTCG |
| FAM061 | GCCCORE | 486 | 492 | - | 0 | 1 | CGCCGCC |
| FAM061 | GCCCORE | 489 | 495 | - | 0 | 1 | TGCCGCC |

EXAMPLE 6:

BINARY VECTOR CONSTRUCTION FOR MAIZE TRANSFORMATION TO EVALUATE THE FUNCTION OF THE PROMOTERS

To facilitate subcloning, the promoter fragments of KG24, 37, 45, 46, 49, 103, 119, 129 were modified by the addition of a *Pac*I restriction enzyme site (for p_KG24, p_KG37, p_KG45, p_KG46, p_KG49, p_KG103, p_KG119, p_KG129) or a *NotI* (for p_KG56) at its 5' end and a *NotI* site (for p_KG24, p_KG103, p_KG129) or a *Bsi*WI site (for p_KG37, p_KG45, p_KG46, p_KG49, p_KG56,) at its 3'end. The *Pac*I-pKG37 (or 45, 46, 49)-*Bsi*WI, or *Pac*I-pK24 (or 103, *119)-NotI* or *NotI*-pKG56-*Bsi*WI promoter fragment was digested and ligated into a corresponding enzyme digested BPS basic binary vector HF84. HF84 comprises a plant selectable marker expression cassette (p-Ubi::c-EcEsdA::t-OCS3), as well as a promoter evaluation cassette that consists of a multiple cloning site (MCS) for insertion of promoter and the rice MET1-1 intron to supply intron-mediated enhancement in monocot cells, GUS reporter gene, and NOS terminator. Diagram of HF84 is shown in Figure 2 A. p-KG129 fragment was cloned into a binary vector backbone RCB1006 (Figure 2 B) via Gateway reaction.

Table 31 lists the resulting binary vector of the KG promoters, Sequences of the promoter cassettes in the binary vectors are shown in SEQ ID NO: 57, 58, and 62-68.

Table31. Binary vectors of the KG promoters for corn transformation

| Promoter ID | Vector ID | Description | SEQ ID OF VECTOR |
|---|---|---|---|
| p-KG24 | RHF155 | p-KG24::iMET1::GUS::t-NOS | 63 |
| p-KG37 | RKF109 | p-KG37::iMET1::GUS::t-NOS | 64 |
| p-KG45 | RKF106 | p-KG45::iMET1::GUS::t-NOS | 65 |
| p-KG46 | RKF107 | p-KG46::iMET1::GUS::t-NOS | 66 |
| p-KG49 | RKF108 | p-KG49::iMET1::GUS::t-NOS | 62 |
| p-KG56 | RKF125 | p-KG56::iMET1::GUS::t-NOS | 57 |
| p-KG103 | RHF128 | p-KG103::iMET1::GUS::t-NOS | 67 |
| p-KG119 | RHF138 | p-KG119::iMET1::GUS::t-NOS | 68 |
| p-KG129 | RTP1047 | p-KG129::iMET1::GUS::t-NOS | 58 |

EXAMPLE 7:

PROMOTER EVALUATION IN TRANSGENIC MAIZE WITH THE KG PROMOTERS

Expression patterns and levels driven by the KG promoters were measured using GUS histochemical analysis following the protocol in the art (Jefferson 1987). Maize transformation was conducted using an *Agrobacterium*-mediated transformation system. Ten and five single copy events for T0 and T1 plants were chosen for the promoter analysis. GUS expression was measured at various developmental stages:

1) Roots and leaves at 5-leaf stage
2) Stem at V-7 stage
2) Leaves, husk and silk at flowering stage (first emergence of silk)
3) Spikelets/Tassel (at pollination)
5) Ear or Kernels at 5, 10, 15, 20, and 25 days after pollination (DAP)

The results indicated that all these 9 promoters expressed specifically in pollen and in embryo (Figures 4 to 11).

EXAMPLE 8:

IDENTIFICATION OF MA-TRANSCRIPT CANDIDATES

A microarray study was conducted to identify transcripts with whole seed-specific and or embryo-specific expression in maize using a battery of RNA samples from 23 maize tissues generated by BASF (Table 32). The twenty-three labeled RNAs of these maize tissues were hybridized separately to 23 of our custom designed BPS maize Affymetrix chips, labeled with fluorescent streptavidin antibody, washed, stained and scanned as instructed in the Affymetrix Expression Analysis Technical Manual.

Table 32. Corn Tissues used for mRNA expression profiling experiment

| Sample No. | Tissue | Timing and number of plants | Days after Pollination |
|---|---|---|---|
| 1 | | 9am (4 plants) | 5 |
| 2 | | 9am (4 plants) | 15 |
| 3 | Root | 9am (4 plants) | 30 |
| 4 | | 9 am (6 plants) | 5 |
| 5 | | 9 am (6 plants) | 15 |
| 6 | leaf above the ear | 9 am (6 plants) | 30 |
| 7 | | 9 am (6 plants) | 5 |
| 8 | ear complete | 9 am (6 plants) | 10 |
| 9 | | 9am (6 plants) | 15 |
| 10 | | 9am (6 plants) | 20 |
| 11 | Whole seed | 9am (6 plants) | 30 |
| 12 | | 9am(6 plants) | 15 |
| 13 | | 9am(6 plants) | 20 |
| 14 | Endosperm | 9am(6 plants) | 30 |
| 15 | | 9 am (6 plants) | 15 |
| 16 | | 9 am (6 plants) | 20 |
| 17 | Embryo | 9 am (6 plants) | 30 |
| 18 | Female pistilate flower | 6 plants | before pollination |
| 19 | germinating seed | 20 seeds | imbibition for 3 days |
| 20 | root, veg. state | | V2 |
| 21 | root, veg. state | | V4 |
| 22 | leaf, veg. State | | V2 |
| 23 | leaf, veg. State | | V4 |

The chip hybridization data were analyzed using Genedata Specialist software and relative expression level was determined based on the hybridization signal intensity of each tissue.

Eight of the BPS maize chip probe sets were selected as candidate transcripts showing 3-8 fold higher expression in whole seeds and or in embryo as compared to other tissues. Corresponding transcripts of these probe sets were named as MAWS23, MAWS27, MAWS30, MAWS57, MAWS60, MAWS63, MAEM1 and MAEM20 (Table 32-1). Consensus sequences of the selected chip probe sets are shown in SEQ ID NOs 91, 92, 95-97, 105-107.

Table 32-1 Microarray candidates and probe sets

| MA Candidates | Proble set | SEQ ID |
|---|---|---|
| MAWS23 | ZM1s57912912 | 105 |
| MAWS27 | ZM3s00207 | 96 |
| MAWS30 | ZM1a61269071 | 106 |
| MAWS57 | ZM1s57500283 | 107 |

(continued)

| MA Candidates | Proble set | SEQ ID |
|---|---|---|
| MAWS60 | ZM4s20063 | 91 |
| MAWS63 | ZM1s62013293 | 97 |
| MAEM1 | ZM4s09689 | 92 |
| MAEM20 | ZM1s59153555 | 95 |

EXAMPLE 9:

CONFIRMATION OF EXPRESSION PATTERN OF THE MA CANDIDATES USING QUANTITATIVE REVERSE TRANSCRIPTASE-POLYMERASE CHAIN REACTION (Q-RT-PCR)

In order to confirm the native expression pattern of the MA candidates, quantitative reverse transcription PCR (q-RT-PCR) was performed using total RNA isolated from the same materials as were used for the chip hybridization (Table 32).

Primers for qRT-PCR were designed based on the consensus sequences of probe sets shown in Table 2 using the Vector NTI software package (Invitrogen, Carlsbad, CA, USA). Two sets of primers were used for PCR amplification for each candidate. The glyceraldehyde-3-phosphate dehydrogenase (GAPDH) gene served as a control for normalization purposes. Sequences of primers for q-RT-PCR are listed in Table 32-2.

Table 32-2. Primer sequences for q-RT-PCR

| Primer | Sequences |
|---|---|
| MAWS23_forward_1 | TCCTCCTCGATCCATCGATC |
| MAWS23_reverse_1 | TTCACCTGCTCACCCATCGG |
| MAWS23_forward_2 | GGCTTCCTCGTAAGCAAGTCATCCA |
| MAWS23_reverse_2 | AACACAGCATTCCGCGACGACC |
| MAWS27_forward_1 | CCGTCCACCGTGAACTCCGCGT |
| MAWS27_reverse_1 | TGGCAGCATCCTGACGCTAACCAG |
| MAWS27_forward_2 | CGTCAGGATGCTGCCATGGGC |
| MAWS27_reverse_2 | TCCGGCGCGTTCTCGTACGA |
| MAWS30_forward_1 | GATGGGTGAGCAGGTGAAGG |
| MAWS30_reverse_1 | AAGAGCAGGAACACGGGCGT |
| MAWS30_forward_2 | ATCCAGAGCAAGGCGCAGGA |
| MAWS30_reverse_2 | TTGACACGCACGCATCCATG |
| MAWS57_forward_1 | CGCCCAACTCGACGCAGGTG |
| MAWS57_reverse_1 | CTGGTGAGCAGCGCGATGGG |
| MAWS57_forward_2 | CTCCCCGTGGCCACCTGGATGT |
| MAWS57_reverse_2 | CGCAGGTATCCGCCGTACTCGC |
| MAWS60_forward_1 | CGACGGACGGGTCCAGACAGCA |
| MAWS60_reverse_1 | TGCACGCGAGCCACCAGGAC |
| MAWS60_forward_2 | AGGGCTCCACGCTCCTTACCGAA |
| MAWS60_reverse_2 | GTTCCCGGCGCCATCCCTATC |
| MAWS63_forward_1 | CAAGCGCGAAATCAAGCCCGG |
| MAWS63_reverse_1 | GGCAGCGGCGAAGAGGTCGA |

(continued)

| Primer | Sequences |
|---|---|
| MAWS63_forward_2 | GGGGACCAACAAGAACGCCGTC |
| MAWS63_reverse_2 | TCCCAAGCGACGTCCACCGG |
| MAEM1_forward_1 | CTGGTGGTGGGGCGGGTGAT |
| MAEM1_reverse_1 | GGGGTCCGTCATGATCAGCG |
| MAEM1_forward_2 | GACCATGAGAGAGTACCTCCAC |
| MAEM1_reverse_2 | GAACAGCACCAGCACGTAGC |
| MAEM20_forward_1 | TGCCACTGTGCTGTGCAGTA |
| MAEM20_reverse_1 | GAGCCCACCACCTTGTTTCC |
| MAEM20_forward_2 | TCCACGGTGGTGCATGTCGT |
| MAEM20_reverse_2 | TACTGCTGCAGAATCCTCCTCCGG |
| GAPDH_Forward | GTAAAGTTCTTCCTGATCTGAAT |
| GAPDH_Reverse | TCGGAAGCAGCCTTAATA |

q-RT-PCR was performed using SuperScript III Reverse Transcriptase (Invitrogen, Carlsbad, CA, USA) and SYBR Green QPCR Master Mix (Eurogentec, San Diego, CA, USA) in an ABI Prism 7000 sequence detection system. In brief, cDNA was synthesized using 2-3 μg of total RNA and 1 μL reverse transcriptase in a 20 μL volume. The cDNA was diluted to a range of concentrations (15-20 ng/ μL). Thirty to forty ng of cDNA was used for quantitative PCR (qPCR) in a 30 μL volume with SYBR Green QPCR Master Mix following the manufacturer's instruction. The thermocycling conditions were as follows: incubate at 50°C for 2 minutes, denature at 95°C for 10 minutes, and run 40 cycles at 95°C for 15 seconds and 60°C for 1 minute for amplification. After the final cycle of the amplification, the dissociation curve analysis was carried out to verify that the amplification occurred specifically and no primer dimer product was generated during the amplification process. The housekeeping gene glyceraldehyde-3-phosphate-dehydrogenase (GAPDH, primer sequences in Table 3) was used as an endogenous reference gene to normalize the calculation using the Comparative Ct (Cycle of threshold) value method. The ΔCT value was obtained by subtracting the Ct value of GAPDH gene from the Ct value of the candidate gene, and the relative transcription quantity (expression level) of the candidate gene expression was presented as 2-ΔCT. The q-RT-PCR results are summarized in Figure 12. All candidates showed similar expression patterns that are equivalent to the expression patterns obtained from the chip hybridization study.

EXAMPLE 10:

ANNOTATION AND PROMOTER IDENTIFICATION OF THE MA CANDIDATES

The coding sequences of the MA candidates were annotated based on *in silico* results obtained from both BLASTX of each EST sequence against GenBank protein database (nr) and the results of *in silico* translation of the sequence using Vector NTI software package.

1. Annotation of MAWS23

MAWS23 encodes Lipid body-associated protein L2 (Maize Oleosin 18 kDa) (GenBank Accession: P21641). The top 10 homologous sequences identified in the BlastX query are presented in Table 33.

Table 33. BLASTX search results of the maize ZM1s57912912 *(MAWS23)*

| Accession | Description | Score | E-value | % Identities |
|---|---|---|---|---|
| P21641 | OLEO3_MAIZE Oleosin Zm-II (Oleosin 18 kDa) (Lipid body-associated protein L2) | 69 | 4.00E-34 | 100 |
| AAA68066.1 | 17 kDa oleosin | 65 | 5.00E-29 | 93 |
| NP_001050984.1 | Os03g0699000 [Oryza sativa (japonica cultivar-group)] | 63 | 8.00E-20 | 93 |

(continued)

| Accession | Description | Score | E-value | % Identities |
|---|---|---|---|---|
| CAA57994.1 | high molecular weight oleosin [Hordeum vulgare subsp. vulgare] | 60 | 4.00E-19 | 86 |
| AAC02240.1 | 18 kDa oleosin [Oryza sativa] | 60 | 5.00E-19 | 90 |
| CAN80217.1 | hypothetical protein [Vitis vinifera] | 70 | 1.00E-09 | 54 |
| AAB24078.1 | lipid body membrane protein [Daucus carota] | 59 | 9.00E-07 | 83 |
| AAG43516.1 | AF210696_1 15kD oleosin-like protein 1 [Perilla frutescens] | 52 | 2.00E-06 | 66 |
| AAG43517.1 | AF210697_1 15kD oleosin-like protein 2 [Perilla frutescens] | 52 | 2.00E-06 | 66 |
| CAN80218.1 | hypothetical protein [Vitis vinifera] | 59 | 2.00E-06 | 80 |

The CDS sequence of the gene corresponding to MAWS23 is shown in SEQ ID NO: 33 and the translated amino acid sequence is shown in SEQ ID NO: 51.

Identification of the promoter region of MAWS23

For our promoter identification purposes, the sequence upstream of the start codon of the MAWS23 gene was defined as the promoter p-MAWS23. To identify this predicted promoter region, the sequence of ZM1s57912912 was mapped to the BASF Plant Science proprietary maize genomic DNA sequence database, PUB_tigr_maize_genomic_partial_5.0.nt. One maize genomic DNA sequences, AZM5_84556 (2036 bp, SEQ ID NO 87) was identified. This 2036bp sequence harbored the predicted CDS of the corresponding gene to MAWS23 and less than 0.5kb upstream sequence of the ATG start codon of this gene. In addition, a public available sequence CL990349 was overlapped with AZM5_84556. The contig of these 2 genomic sequences containing 1.3kb upstream region is shown in SEQ ID NO: 87.

Isolation of the promoter region by PCR amplification

The putative promoter region was isolated via genomic PCR using the following sequence specific primers:

Forward primer: TCATCGGTACTCGCGATGTC (SEQ ID NO: 172)
Reverse primer: CTTTGCAAACAAAGTGACGGAG (SEQ ID NO: 173). The expected 1264bp fragment was amplified from maize genomic DNA, and named as promoter MAWS23 (p-MAWS23). Sequence of p-MAWS23 is shown in SEQ ID NO: 15.

BLASTN results of p_MAWS23

The top 20 homologous sequences identified in the BlastN query of p_MAWS23 are presented in Table 34.

Table 34. BLASTN results of p_MAWS23

| Accession | Description | Max score | Total score | Query coverage | E value | Max ident |
|---|---|---|---|---|---|---|
| J05212.1 | Maize oleosin KD18 (KD18; L2) gene, complete cds | 2150 | 2150 | 100% | 0 | 97% |
| AY555143.1 | Zea may BAC clone c573L14, complete sequence | 682 | 682 | 37% | 0 | 91% |
| AF488416.1 | Zea mays chromosome 9 BAC 9C20 complete sequence | 641 | 1094 | 37% | 2.00E-180 | 88% |

(continued)

| Accession | Description | Max score | Total score | Query coverage | E value | Max ident |
|---|---|---|---|---|---|---|
| AF434192.1 | Zea mays line LH82 transposon Ins2, YZ1 (yz1) gene, YZ1-LH82 allele, complete cds; tRNA-Phe (trnF) gene, complete sequence; retrotransposon Machiavelli Gag and Pol (gag/pol) gene, complete cds; and retrotransposon-like Ozymandias and MITE Gnat1, complete sequence | 625 | 625 | 37% | 2.00E-175 | 88% |
| AY455286.1 | Zea mays chloroplast phytoene synthase (Y1) gene, complete cds; nuclear gene for chloroplast product | 619 | 696 | 37% | 8.00E-174 | 87% |
| AF090447.2 | Zea mays 22 kDa alpha zein gene cluster, complete sequence | 571 | 680 | 38% | 4.00E-159 | 86% |
| AC157977.1 | Genomic sequence for Zea mays chromosome 8 BAC clone ZMMBBb0284N04, complete sequence | 556 | 556 | 37% | 8.00E-155 | 84% |
| BT038288.1 | Zea mays full-length cDNA clone ZM_BFb0224G21 mRNA, complete cds | 522 | 522 | 36% | 2.00E-144 | 83% |
| L29505.1 | Zea mays high sulfur zein gene, complete cds | 520 | 520 | 29% | 6.00E-144 | 90% |
| EU943322.1 | Zea mays clone 1599166 mRNA sequence | 477 | 477 | 37% | 6.00E-131 | 80% |
| AC165176.2 | Zea mays clone ZMMBBb-177G21, complete sequence | 475 | 635 | 37% | 2.00E-130 | 90% |
| AC165171.2 | Zea mays clone CH201-145P10, complete sequence | 466 | 466 | 36% | 1.00E-127 | 81% |
| AC 152494.1 | Zea mays BAC clone Z418K17, complete sequence | 464 | 948 | 37% | 4.00E-127 | 80% |
| X73151.1 | Z.mays GapC2 gene | 461 | 461 | 38% | 5.00E-126 | 79% |
| AC165267.2 | Zea mays clone ZMMBBb-151F20, complete sequence | 446 | 446 | 37% | 1.00E-121 | 79% |
| DQ002407.1 | Zea mays copia retrotransposon opie1, gypsy retrotransposon grande1, xilon1 retrotransposon, helitron B73_14578, gypsy retrotransposon huck1 and ruda retrotransposon, complete sequence | 428 | 548 | 37% | 3.00E-116 | 82% |
| AF546189.1 | Contiguous genomic DNA sequence comprising the 19-kDa-zein gene family from Zea mays, complete sequence | 340 | 386 | 24% | 1.00E-89 | 84% |
| AY109359.1 | Zea mays CL2022_2 mRNA sequence | 306 | 306 | 27% | 2.00E-79 | 77% |
| BT038370.1 | Zea mays full-length cDNA clone ZM_BFb0229A02 mRNA, complete cds | 288 | 288 | 21% | 6.00E-74 | 83% |
| EU953088.1 | Zea mays clone 1381669 unknown mRNA | 201 | 201 | 20% | 7.00E-48 | 75% |

2. Annotation of MAWS27

MAWS27 encodes a maize unknown protein (GenBank Accession: ACF80385.1). The top 10 homologous sequences identified in the BlastX query are presented in Table 35.

Table 35. BLASTX search results of the maize ZM3s00207 *(MAWS27)*

| Accession | Description | Score | E-value | % Identities |
|---|---|---|---|---|
| ACF80385.1 | unknown [Zea mays] | 155 | 6.00E-41 | 89 |
| BAA83559.1 | putative early nodulin [Oryza sativa Japonica Group] | 155 | 1.00E-39 | 85 |
| BAA83567.1 | putative early nodulin [Oryza sativa Japonica Group] | 154 | 2.00E-39 | 84 |
| NP_001056762.1 | Os06g0141700 [Oryza sativa (japonica cultivar-group)] | 152 | 5.00E-39 | 85 |
| NP_001056767.1 | Os06g0142300 [Oryza sativa (japonica cultivar-group)] | 151 | 6.00E-39 | 85 |
| BAA33813.1 | early nodulin [Oryza sativa Japonica Group] | 154 | 6.00E-39 | 86 |
| BAA83566.1 | putative early nodulin [Oryza sativa Japonica Group] | 151 | 6.00E-39 | 85 |
| EAY99605.1 | hypothetical protein OsI_020838 [Oryza sativa (indica cultivar-group)] | 154 | 6.00E-39 | 86 |
| EAY99606.1 | hypothetical protein OsI_020839 [Oryza sativa (indica cultivar-group)] | 151 | 6.00E-39 | 85 |
| EAY99601.1 | hypothetical protein OsI_020834 [Oryza sativa (indica cultivar-group)] | 149 | 1.00E-38 | 84 |

The CDS sequence of the gene corresponding to MAWS27 is shown in SEQ ID NO: 24 and the translated amino acid sequence is shown in SEQ ID NO: 42.

Identification of the promoter region of MAWS27

For our promoter identification purposes, the sequence upstream of the start codon of the MAWS27 gene was defined as the promoter p-MAWS27. To identify this predicted promoter region, the sequence of ZM3s00207 was mapped to the BASF Plant Science proprietary maize genomic DNA sequence database, PUB_tigr_maize_genomic_partial_5.0.nt. One maize genomic DNA sequences, AZM5_32720 (2113 bp, SEQ ID NO: 78) was identified. This 2113bp sequence harbored the predicted CDS of the corresponding gene to MAWS27 and 1.2kb upstream sequence of the ATG start codon of this gene. In addition, a public available sequence DX863447 was overlapped with AZM5_32720. The contig of these 2 genomic sequences containing 1.35kb upstream region is shown in SEQ ID NO: 78.

Isolation of the promoter region by PCR amplification

The putative promoter region was isolated via genomic PCR using the following sequence specific primers:

Forward primer: TATAAATTAGAACGGAGGGGTATG (SEQ ID NO: 174)
Reverse primer: GGTGATCCGAATCCGATCCC (SEQ ID NO: 175). The expected 1355bp fragment was amplified from maize genomic DNA, and named as promoter MAWS27 (p-MAWS27). Sequence of p-MAWS27 is shown in SEQ ID NO: 6.

BLASTN results of p_MAWS27

The top 20 homologous sequences identified in the BlastN query of p-MAWS27 are presented in Table 36.

Table 36. BLASTN results of p_MAWS27

| Accession | Description | Max score | Total score | Query coverage | E value | Max ident |
|---|---|---|---|---|---|---|
| AY072300.1 | Zea mays cytochrome P450 monooxygenase CYP72A5 gene, complete cds | 228 | 443 | 13% | 5.00E-56 | 90% |
| BT042628.1 | Zea mays full-length cDNA clone ZM_BFb0383P13 mRNA, complete cds | 215 | 283 | 11% | 3.00E-52 | 93% |

(continued)

| Accession | Description | Max score | Total score | Query coverage | E value | Max ident |
|---|---|---|---|---|---|---|
| BT041400.1 | Zea mays full-length cDNA clone ZM_BFc0115C19 mRNA, complete cds | 210 | 273 | 13% | 1.00E-50 | 89% |
| AJ251453.1 | Zea mays see2a gene for putative legumain, exons 1-9 | 208 | 269 | 11% | 5.00E-50 | 90% |
| EU241894.1 | Zea mays ZCN3 (ZCN3) gene, complete cds | 199 | 271 | 13% | 3.00E-47 | 90% |
| EU943068.1 | Zea mays clone 1558247 mRNA sequence | 197 | 265 | 13% | 9.00E-47 | 93% |
| EU953408.1 | Zea mays clone 1408713 unknown mRNA | 190 | 255 | 11% | 1.00E-44 | 93% |
| AY662985.1 | Zea luxurians YZ1 (yz1) gene, complete cds; transposons mPIF-like element and frequent flyer, complete sequence; and NADPH-dependent reductase (a1) gene, partial cds | 187 | 238 | 13% | 2.00E-43 | 88% |
| AJ437282.1 | Zea mays ZmEBE-2 gene for ZmEBE-2 protein, exons 1-4 | 176 | 176 | 11% | 3.00E-40 | 85% |
| AJ437281.1 | Zea mays ZmEBE-1 gene for ZmEBE-1 protein, exons 1-5 | 169 | 215 | 11% | 4.00E-38 | 85% |
| AY530950.1 | Zea mays putative zinc finger protein (Z438D03.1), unknown (Z438D03.5), epsilon-COP (Z438D03.6), putative kinase (Z438D03.7), unknown (Z438D03.25), and C1-B73 (Z438D03.27) genes, complete cds | 167 | 231 | 13% | 2.00E-37 | 93% |
| DQ020097.1 | Zea mays cultivar B73 inbred aberrant pollen transmission 1 (apt1) gene, complete cds | 165 | 226 | 13% | 5.00E-37 | 89% |
| AY555143.1 | Zea may BAC clone c573L14, complete sequence | 163 | 309 | 13% | 2.00E-36 | 85% |
| AY111966.1 | Zea mays CL4954_1 mRNA sequence | 158 | 206 | 10% | 8.00E-35 | 96% |
| EU975033.1 | Zea mays clone 465494 unknown mRNA | 156 | 156 | 11% | 3.00E-34 | 83% |
| AF391808.3 | Zea mays cultivar McC bz locus region | 156 | 272 | 17% | 3.00E-34 | 82% |
| U09989.1 | Zea mays D3L H(+)-transporting ATPase (Mha1) gene, complete cds | 156 | 218 | 11% | 3.00E-34 | 89% |
| EU241912.1 | Zea mays ZCN21 (ZCN21) gene, complete cds | 154 | 204 | 11% | 1.00E-33 | 84% |
| BT039577.1 | Zea mays full-length cDNA clone ZM_BFc0031C07 mRNA, complete cds | 149 | 197 | 12% | 4.00E-32 | 83% |
| DQ219417.1 | Zea mays YZ1 (Yz1A) gene, Yz1A-1012M allele, partial cds; and a1 gene, A1-b alpha allele, transposon lns2, and cin4 retrotransposon, complete sequence | 149 | 362 | 13% | 4.00E-32 | 89% |

3. Annotation of MAWS30

MAWS30 encodes maize 17Kda oleosin (GenBank Accession: AAA68066.1). The top 10 homologous sequences identified in the BlastX query are presented in Table 37.

Table 37. BLASTX search results of the maize ZM1a61269071 *(MAWS30)*

| Accession | Description | Score | E-value | % Identities |
|---|---|---|---|---|
| AAA68066.1 | 17 kDa oleosin | 66 | 3.00E-21 | 100 |
| P21641 | OLEO3_MAIZE Oleosin Zm-II (Oleosin 18 kDa) (Lipid body-associated protein L2) | 54 | 1.00E-15 | 95 |
| CAA57994.1 | high molecular weight oleosin [Hordeum vulgare subsp. vulgare] | 54 | 2.00E-14 | 77 |
| CAN80217.1 | hypothetical protein [Vitis vinifera] | 81 | 2.00E-13 | 42 |
| NP_001050984.1 | Os03g0699000 [Oryza sativa (japonica cultivar-group)] | 48 | 3.00E-13 | 70 |
| AAC02240.1 | 18 kDa oleosin [Oryza sativa] | 48 | 3.00E-13 | 70 |
| AAG43516.1 | AF210696_1 15kD oleosin-like protein 1 [Perilla frutescens] | 39 | 8.00E-06 | 73 |
| AAG43517.1 | AF210697_1 15kD oleosin-like protein 2 [Perilla frutescens] | 39 | 8.00E-06 | 73 |
| AAB58402.1 | 15.5 kDa oleosin [Sesamum indicum] | 37 | 5.00E-05 | 66 |
| CAN80922.1 | hypothetical protein [Vitis vinifera] | 36 | 7.00E-05 | 57 |

The CDS sequence of the gene corresponding to MAWS30 is shown in SEQ ID NO: 34 and the translated amino acid sequence is shown in SEQ ID NO: 52.

Identification of the promoter region of MAWS30

For our promoter identification purposes, the sequence upstream of the start codon of the MAWS30 gene was defined as the promoter p-MAWS30. To identify this predicted promoter region, the sequence of ZM1a61269071 was mapped to the BASF Plant Science proprietary maize genomic DNA sequence database, PUB_tigr_maize_genomic_partia_5.0.nt. One maize genomic DNA sequences, AZM5_84557 (3426 bp) was identified. This 3426bp sequence harbored the predicted CDS of the corresponding gene to MAWS30 and about 0.6kb upstream sequence of the ATG start codon of this gene. Sequence of AZM5_84557 is shown in SEQ ID NO: 88.

Isolation of the promoter region by PCR amplification

The putative promoter region was isolated via genomic PCR using the following sequence specific primers:

Forward primer: CTCACACAAATCTAAATAGTAAAG (SEQ ID NO: 176)
Reverse primer: GAGAGAGAGAGTAGTGAAGTG (SEQ ID NO: 177). The expected 623bp fragment was amplified from maize genomic DNA, and named as promoter MAWS30 (p-MAWS30). Sequence of p-MAWS30 was shown in SEQ ID NO: 16.

BLASTN results of p_MAWS30

The top 20 homologous sequences identified in the BlastN query of p_MAWS30 are presented in Table 38.

Table 38. BLASTN results of p_MAWS30

| Accession | Description | Max score | Total score | Query coverage | E value | Max ident |
|---|---|---|---|---|---|---|
| U13702.1 | Zea mays oil body protein 17 kDa oleosin (ole17) gene, complete cds | 686 | 686 | 60% | 0 | 100% |
| J05212.1 | Maize oleosin KD18 (KD18; L2) gene, complete cds | 187 | 187 | 42% | 7.00E-44 | 75% |

(continued)

| Accession | Description | Max score | Total score | Query coverage | E value | Max ident |
|---|---|---|---|---|---|---|
| AY427563.1 | Oryza sativa (japonica cultivar-group) 18 kDa oleosin gene, promoter region | 98.7 | 98.7 | 18% | 3.00E-17 | 78% |
| AF019212.1 | Oryza sativa subsp. indica 18 kDa oleosin (ole18) gene, complete cds | 98.7 | 98.7 | 18% | 3.00E-17 | 78% |
| AP008209.1 | Oryza sativa (japonica cultivar-group) genomic DNA, chromosome 3 | 95.1 | 95.1 | 18% | 4.00E-16 | 77% |
| AC097368.3 | Oryza sativa chromosome 3 BAC OSJNBb0017F17 genomic sequence, complete sequence | 95.1 | 95.1 | 18% | 4.00E-16 | 77% |
| AF369906.1 | Sorghum bicolor clone BAC10J22 Sbb3766 sequence | 53.6 | 53.6 | 8% | 0.001 | 82% |
| FJ119498.1 | Pinus taeda isolate 8102 anonymous locus UMN_CL22Contig1_02 genomic sequence | 46.4 | 46.4 | 6% | 0.16 | 85% |
| FJ119497.1 | Pinus taeda isolate 8112 anonymous locus UMN_CL22Contig1_02 genomic sequence | 46.4 | 46.4 | 6% | 0.16 | 85% |
| FJ119496.1 | Pinus taeda isolate 8099 anonymous locus UMN_CL22Contig1_02 genomic sequence | 46.4 | 46.4 | 6% | 0.16 | 85% |
| FJ 119495.1 | Pinus taeda isolate 8105 anonymous locus UMN_CL22Contig1_02 genomic sequence | 46.4 | 46.4 | 6% | 0.16 | 85% |
| FJ119494.1 | Pinus taeda isolate 8108 anonymous locus UMN_CL22Contig1_02 genomic sequence | 46.4 | 46.4 | 6% | 0.16 | 85% |
| FJ119493.1 | Pinus taeda isolate 8103 anonymous locus UMN_CL22Contig1_02 genomic sequence | 46.4 | 46.4 | 6% | 0.16 | 85% |
| FJ119492.1 | Pinus taeda isolate 8100 anonymous locus UMN_CL22Contig1_02 genomic sequence | 46.4 | 46.4 | 6% | 0.16 | 85% |
| FJ119491.1 | Pinus taeda isolate 8107 anonymous locus UMN_CL22Contig1_02 genomic sequence | 46.4 | 46.4 | 6% | 0.16 | 85% |
| FJ119490.1 | Pinus taeda isolate 8113 anonymous locus UMN_CL22Contig1_02 genomic sequence | 46.4 | 46.4 | 6% | 0.16 | 85% |
| FJ119489.1 | Pinus taeda isolate 8101 anonymous locus UMN_CL22Contig1_02 genomic sequence | 46.4 | 46.4 | 6% | 0.16 | 85% |
| FJ119488.1 | Pinus taeda isolate 8111 anonymous locus UMN_CL22Contig1_02 genomic sequence | 46.4 | 46.4 | 6% | 0.16 | 85% |
| FJ119487.1 | Pinus taeda isolate 8114 anonymous locus UMN_CL22Contig1_02 genomic sequence | 46.4 | 46.4 | 6% | 0.16 | 85% |
| FJ119486.1 | Pinus taeda isolate 8110 anonymous locus UMN_CL22Contig1_02 genomic sequence | 46.4 | 46.4 | 6% | 0.16 | 85% |

4. Annotation of MAWS57

MAWS57 encodes a protein that has homolog to a rice unknown protein Os05g0576700 (GenBank Accession: NP_001056403.1). The top 10 homologous sequences identified in the BlastX query are presented in Table 39.

Table 39. BLASTX search results of the maize ZM1s57500283 *(MAWS57)*

| Accession | Description | Score | E-value | % Identities |
|---|---|---|---|---|
| NP_001056403.1 | Os05g0576700 [Oryza sativa (japonica cultivar-group)] | 112 | 8.00E-33 | 95 |
| ABK40507.1 | pollen oleosin [Lilium longiflorum] | 102 | 2.00E-26 | 82 |
| EAZ35378.1 | hypothetical protein OsJ_018861 [Oryza sativa (japonica cultivar-group)] | 112 | 5.00E-25 | 95 |
| AAX49393.1 | OLE-5 [Coffea canephora] | 92 | 4.00E-19 | 81 |
| CAO68008.1 | unnamed protein product [Vitis vinifera] | 89 | 2.00E-18 | 73 |
| NP_188487.1 | glycine-rich protein / oleosin [Arabidopsis thaliana] | 90 | 6.00E-16 | 78 |
| ACI87763.1 | putative oleosin [Cupressus sempervirens] | 84 | 5.00E-14 | 69 |
| ACA30297.1 | putative oleosin [Cupressus sempervirens] | 84 | 5.00E-14 | 69 |
| NP_175329.1 | glycine-rich protein / oleosin [Arabidopsis thaliana] | 74 | 5.00E-11 | 69 |
| CAN74835.1 | hypothetical protein [Vitis vinifera] | 66 | 1.00E-08 | 53 |

The CDS sequence of the gene corresponding to MAWS57 is shown in SEQ ID NO: 35 and the translated amino acid sequence is shown in SEQ ID NO: 55.

Identification of the promoter region of MAWS57

For our promoter identification purposes, the sequence upstream of the start codon of the MAWS57 gene was defined as the promoter p-MAWS57. To identify this predicted promoter region, the sequence of ZM1s57500283 was mapped to the BASF Plant Science proprietary maize genomic DNA sequence database, PUB_tigr_maize_genomic_partial_5.0.nt. One maize genomic DNA sequences, AZM5_16632 (5254 bp) was identified. This 5254bp sequence harbored the predicted CDS of the corresponding gene to MAWS57 and more than 2.5kb upstream sequence of the ATG start codon of this gene. Sequences of AZM5_16632 is shown in SEQ ID NO: 89.

Isolation of the promoter region by PCR amplification

The putative promoter region was isolated via genomic PCR using the following sequence specific primers:

Forward primer: GGTTCAAGATATGTATGTGATG (SEQ ID NO: 178)
Reverse primer: TCGGGTATCTCTCTGTCTTGTTG (SEQ ID NO: 179). The expected 1950bp fragment was amplified from maize genomic DNA, and named as promoter MAWS57 (p-MAWS57). Sequence of p-MAWS57 was shown in SEQ ID NO: 17.

BLASTN results of p_MAWS57

The top 20 homologous sequences identified in the BlastN query of p_MAWS57 are presented in Table 40.

Table 40. BLASTN results of p_MAWS57

| Accession | Description | Max score | Total score | Query coverage | E value | Max ident |
|---|---|---|---|---|---|---|
| AP008217.1 | Oryza sativa (japonica cultivar-group) genomic DNA, chromosome 11 | 259 | 415 | 26% | 5.00E-65 | 81% |
| BX000501.4 | Oryza sativa chromosome 11,. BAC OSJNBa0032J07 of library OSJNBa from chromosome 11 of cultivar Nipponbare of ssp. japonica of Oryza sativa (rice), complete sequence | 259 | 415 | 26% | 5.00E-65 | 81% |

(continued)

| Accession | Description | Max score | Total score | Query coverage | E value | Max ident |
|---|---|---|---|---|---|---|
| AP008218.1 | Oryza sativa (japonica cultivar-group) genomic DNA, chromosome 12 | 179 | 447 | 23% | 4.00E-41 | 83% |
| BX000494.2 | Oryza sativa chromosome 12, . BAC OSJNBa0052H10 of library OSJNBa from chromosome 12 of cultivar Nipponbare of ssp. japonica of Oryza sativa (rice), complete sequence | 179 | 447 | 23% | 4.00E-41 | 83% |
| BX000491.1 | Oryza sativa chromosome 12, . BAC OSJNBb0068K19 of library OSJNBb from chromosome 12 of cultivar Nipponbare of ssp. japonica of Oryza sativa (rice), complete sequence | 179 | 447 | 23% | 4.00E-41 | 83% |
| AK242870.1 | Oryza sativa Japonica Group cDNA, clone: J090076L04, full insert sequence | 176 | 389 | 18% | 4.00E-40 | 84% |
| NM_001072463.1 | Oryza sativa (japonica cultivar-group) Os12g0105300 (Os12g0105300) mRNA, complete cds | 167 | 389 | 18% | 2.00E-37 | 84% |
| AK099132.1 | Oryza sativa Japonica Group cDNA clone:J023051M04, full insert sequence | 167 | 389 | 18% | 2.00E-37 | 84% |
| AK072914.1 | Oryza sativa Japonica Group cDNA clone:J023150I16, full insert sequence | 167 | 389 | 18% | 2.00E-37 | 84% |
| AK062121.1 | Oryza sativa Japonica Group cDNA clone:001-045-D01, full insert sequence | 167 | 389 | 18% | 2.00E-37 | 84% |
| AK250796.1 | Hordeum vulgare subsp. vulgare cDNA clone: FLbaf94j01, mRNA sequence | 123 | 323 | 25% | 2.00E-24 | 84% |
| AP008213.1 | Oryza sativa (japonica cultivar-group) genomic DNA, chromosome 7 | 111 | 111 | 5% | 2.00E-20 | 81% |
| AP005768.3 | Oryza sativa Japonica Group genomic DNA, chromosome 7, BAC clone:OSJNBa0039C01 | 111 | 111 | 5% | 2.00E-20 | 81% |
| AP005255.4 | Oryza sativa Japonica Group genomic DNA, chromosome 7, BAC clone:OSJNBb0087F05 | 111 | 111 | 5% | 2.00E-20 | 81% |
| AC232448.1 | Brassica rapa subsp. pekinensis clone KBrB008D15, complete sequence | 100 | 191 | 9% | 3.00E-17 | 93% |
| CT828672.1 | Oryza sativa (indica cultivar-group) cDNA clone:OSIGCSA057D18, full insert sequence | 98.7 | 141 | 6% | 1.00E-16 | 87% |
| AM448932.2 | Vitis vinifera contig VV78X114050.3, whole genome shotgun sequence | 96.9 | 177 | 15% | 3.00E-16 | 79% |
| AP010508.1 | Lotus japonicus genomic DNA, chromosome 2, clone: LjT28N02, TM1615, complete sequence | 95.1 | 241 | 16% | 1.00E-15 | 90% |
| EF145201.1 | Populus trichocarpa clone WS01121_K10 unknown mRNA | 87.8 | 155 | 9% | 2.00E-13 | 85% |

(continued)

| Accession | Description | Max score | Total score | Query coverage | E value | Max ident |
|---|---|---|---|---|---|---|
| AC098571.2 | Oryza sativa Japonica Group chromosome 5 clone OJ1126_B10, complete sequence | 80.6 | 80.6 | 3% | 3.00E-11 | 84% |

5. Annotation of MAWS60

MAWS60 encodes a maize unknown protein (GenBank Accession: ACF78165.1). The top 10 homologous sequences identified in the BlastX query are presented in Table 41.

Table 41. BLASTX search results of the maize ZM4s20063 (*MAWS60*)

| Accession | Description | Score | E-value | % Identities |
|---|---|---|---|---|
| ACF78165.1 | unknown [Zea mays] | 138 | 3.00E-52 | 64 |
| ACF83516.1 | unknown [Zea mays] | 204 | 2.00E-50 | 82 |
| ACF86030.1 | unknown [Zea mays] | 124 | 2.00E-48 | 73 |
| ACF87441.1 | unknown [Zea mays] | 79 | 1.00E-46 | 73 |
| ACF78865.1 | unknown [Zea mays] | 102 | 1.00E-22 | 72 |
| ACF88449.1 | unknown [Zea mays] | 42 | 5.00E-11 | 48 |
| NP_001066367.1 | Os12g0198700 [Oryza sativa (japonica cultivar-group)] | 42 | 1.00E-10 | 46 |
| NP_001066495.1 | Os12g0247700 [Oryza sativa (japonica cultivar-group)] | 39 | 4.00E-10 | 61 |
| ABR25456.1 | beta-glucosidase aggregating factor precursor [Oryza sativa (indica cultivar-group)] | 46 | 7.00E-10 | 53 |
| NP_001066435.1 | Os12g0227500 [Oryza sativa (japonica cultivar-group)] | 46 | 7.00E-10 | 53 |

The CDS sequence of the gene corresponding to MAWS60 is shown in SEQ ID NO: 19 and the translated amino acid sequence is shown in SEQ ID NO: 37.

Identification of the promoter region of MAWS60

For our promoter identification purposes, the sequence upstream of the start codon of the MAWS60 gene was defined as the promoter p-MAWS60. To identify this predicted promoter region, the sequence of ZM4s20063 was mapped to the BASF Plant Science proprietary maize genomic DNA sequence database, PUB_tigr_maize_genomic_partial_5.0.nt. One maize genomic DNA sequences, AZM5_25938 (3185 bp) was identified. This 3185bp sequence harbored the predicted CDS of the corresponding gene to MAWS60 and 1.2kb upstream. sequence of the ATG start codon of this gene. Sequence of AZM5_25938 is shown in SEQ ID NO: 73.

Isolation of the promoter region by PCR amplification

The putative promoter region was isolated via genomic PCR using the following sequence specific primers:

Forward primer: TTGGTTTTTTGATAATTTGTTTATC (SEQ ID NO: 180)
Reverse primer: TCTCCATTACCTGCAACGATC (SEQ ID NO: 181). The expected 1106bp fragment was amplified from maize genomic DNA, and named as promoter MAWS60 (p-MAWS60). Sequence of p-MAWS60 was shown in SEQ ID NO: 1.

BLASTN results of p_MAWS60

The top 20 homologous sequences identified in the BlastN query of p_MAWS60 are presented in Table 42.

Table 42. BLASTN results of p_MAWS60

| Accession | Description | Max score | Total score | Query coverage | E value | Max ident |
|---|---|---|---|---|---|---|
| AC157320.2 | Zea mays clone ZMMBBb-7C14, complete sequence | 452 | 801 | 44% | 2.00E-123 | 79% |
| AF544161.1 | Zea mays subsp. mays cultivar A6 ADP-glucose pyrophosphorylase large subunit (shrunken2) gene, partial sequence | 320 | 320 | 22% | 9.00E-84 | 89% |
| AF544159.1 | Zea mays subsp. mays cultivar Tx601 ADP-glucose pyrophosphorylase large subunit (shrunken2) gene, partial sequence | 320 | 320 | 22% | 9.00E-84 | 89% |
| AF544158.1 | Zea mays subsp. mays cultivar Ki9 ADP-glucose pyrophosphorylase large subunit (shrunken2) gene, partial sequence | 320 | 320 | 22% | 9.00E-84 | 89% |
| AF544157.1 | Zea mays subsp. mays cultivar T232 ADP-glucose pyrophosphorylase large subunit (shrunken2) gene, partial sequence | 320 | 320 | 22% | 9.00E-84 | 89% |
| U07956.1 | Zea mays transposable element ILS-1 | 320 | 320 | 22% | 9.00E-84 | 89% |
| AC165178.2 | Zea mays clone ZMMBBb-272P17, complete sequence | 315 | 860 | 32% | 4.00E-82 | 84% |
| AF544160.1 | Zea mays subsp. mays cultivar A272 ADP-glucose pyrophosphorylase large subunit (shrunken2) gene, partial sequence | 315 | 315 | 22% | 4.00E-82 | 88% |
| AC160211.1 | Genomic seqeunce for Zea mays BAC clone ZMMBBb0448F23, complete sequence | 279 | 428 | 41% | 3.00E-71 | 86% |
| AY530950.1 | Zea mays putative zinc finger protein (Z438D03.1), unknown (Z438D03.5), epsilon-COP (Z438D03.6), putative kinase (Z438D03.7), unknown (Z438D03.25), and C1-B73 (Z438D03.27) genes, complete cds | 264 | 541 | 34% | 6.00E-67 | 82% |
| AF061282.1 | Sorghum bicolor 22 kDa kafirin cluster | 179 | 429 | 35% | 2.00E-41 | 96% |
| AY661657.1 | Sorghum bicolor clone BAC 60H10, complete sequence | 167 | 167 | 25% | 1.00E-37 | 74% |
| AY661656.1 | Sorghum bicolor clone BAC 88M4, complete sequence | 167 | 535 | 35% | 1.00E-37 | 88% |
| AC169377.4 | Sorghum bicolor clone SB_BBc0068O12, complete sequence | 154 | 154 | 17% | 8.00E-34 | 79% |
| AC169379.4 | Sorghum bicolor clone SB_BBc0088B22, complete sequence | 154 ’ | 154 | 17% | 8.00E-34 | 79% |
| AP008208.1 | Oryza sativa (japonica cultivar-group) genomic DNA, chromosome 2 | 131 | 4754 | 22% | 9.00E-27 | 78% |
| AP005066.2 | Oryza sativa Japonica Group genomic DNA, chromosome 2, PAC clone:P0047E05 | 131 | 218 | 22% | 9.00E-27 | 75% |
| AY144442.1 | Sorghum bicolor BAC 95A23/98N8.1 Rph region, partial sequence | 127 | 766 | 19% | 1.00E-25 | 88% |
| AP008218.1 | Oryza sativa (japonica cultivar-group) genomic DNA, chromosome 12 | 125 | 6385 | 22% | 4.00E-25 | 80% |

(continued)

| Accession | Description | Max score | Total score | Query coverage | E value | Max ident |
|---|---|---|---|---|---|---|
| AL831796.5 | Oryza sativa chromosome 12,. BAC OSJNBa0012G19 of library OSJNBa from chromosome 12 of cultivar Nipponbare of ssp. japonica of Oryza sativa (rice), complete sequence | 125 | 125 | 22% | 4.00E-25 | 71% |

6. Annotation of MAWS63

MAWS63 encodes a protein that is homologous to a rice hypothetical protein OsI-026531 (GenBank Accession: EAZ05299.1). The top 10 homologous sequences identified in the BlastX query are presented in Table 43.

Table 43. BLASTX search results of the maize ZM1s62013293 *(MAWS63)*

| Accession | Description | Score | E-value | % Identities |
|---|---|---|---|---|
| EAZ05299.1 | hypothetical protein OsI_026531 | 98 | 2.00E-20 | 59 |
| NP_001060778.1 | Os08g0104400 [Oryza sativa (japonica cultivar-group)] | 94 | 2.00E-19 | 72 |
| EAZ05298.1 | hypothetical protein OsI_026530 [Oryza sativa (indica cultivar-group)] | 99 | 9.00E-19 | 53 |
| CAO22190.1 | unnamed protein product [Vitis vinifera] | 58 | 2.00E-06 | 61 |
| CAO46216.1 | unnamed protein product [Vitis vinifera] | 58 | 2.00E-06 | 61 |
| CAN64204.1 | hypothetical protein [Vitis vinifera] | 58 | 2.00E-06 | 61 |
| ABB72396.1 | seed maturation protein [Glycine tomentella] | 58 | 2.00E-06 | 62 |
| ABB72388.1 | seed maturation protein [Glycine latifolia] | 58 | 2.00E-06 | 62 |
| ABB72387.1 | seed maturation protein [Glycine latifolia] | 58 | 2.00E-06 | 62 |
| ABB72392.1 | seed maturation protein [Glycine tomentella] | 58 | 2.00E-06 | 62 |

The CDS sequence of the gene corresponding to MAWS63 is shown in SEQ ID NO: 25 and the translated amino acid sequence is shown in SEQ ID NO: 43.

Identification of the promoter region of MAWS63

For our promoter identification purposes, the sequence upstream of the start codon of the MAWS63 gene was defined as the promoter p-MAWS63. To identify this predicted promoter region, the sequence of ZM1s62013293 was mapped to the BASF Plant Science proprietary maize genomic DNA sequence database, PUB_tigr_maize_genomic_partial_5.0.nt. One maize genomic DNA sequences, AZM5_12462 (5275 bp) was identified. This 5275bp sequence harbored the predicted CDS of the corresponding gene to MAWS63 and 2.3kb upstream sequence of the ATG start codon of this gene. The first 3kb sequence of AZM5_12462 is shown in SEQ ID NO: 79.

Isolation of the promoter region by PCR amplification

The putative promoter region was isolated via genomic PCR using the following sequence specific primers:

Forward primer: AAGGGACTCGTGGCCTACAC (SEQ ID NO: 182)
Reverse primer: TACGTTGTCGCAGCTGGATG (SEQ ID NO: 183). The expected 1941bp fragment was amplified from maize genomic DNA, and named as promoter MAWS63 (p-MAWS63). Sequence of p-MAWS63 is shown in SEQ ID NO: 7.

BLASTN results of p_MAWS63

The top 20 homologous sequences identified in the BlastN query of p_MAWS63 are presented in Table 44.

Table 44. BLASTN results of p_MAWS63

| Accession | Description | Max score | Total score | Query coverage | E value | Max ident |
|---|---|---|---|---|---|---|
| BT040012.1 | Zea mays full-length cDNA clone ZM_BFc0058K21 mRNA, complete cds | 1370 | 1370 | 39% | 0 | 100% |
| EU967309.1 | Zea mays clone 301323 unknown mRNA | 1308 | 1308 | 37% | 0 | 100% |
| BT043342.1 | Zea mays full-length cDNA clone ZM_BFc0164P09 mRNA, complete cds | 1301 | 1301 | 37% | 0 | 100% |
| DQ245437.1 | Zea mays clone 15518 mRNA sequence | 1148 | 1148 | 33% | 0 | 99% |
| AY103722.1 | Zea mays PCO142214 mRNA sequence | 1083 | 1083 | 38% | 0 | 91% |
| JM_001111875.1 | Zea mays ferredoxin1 (fdx1), nuclear gene encoding mitochondrial protein, mRNA >gb\|M73830.1\|MZEFD1P Maize ferredoxin I (Fd) isoprotein mRNA, pFD1' | 1074 | 1074 | 39% | 0 | 90% |
| M73829.1 | Maize ferredoxin I (Fd) isoprotein mRNA, pFD1 | 1027 | 1027 | 33% | 0 | 94% |
| EU328185.1 | Zea mays chloroplast ferredoxin 1 precursor (FDX1) mRNA, complete cds; nuclear gene for chloroplast product | 812 | 812 | 23% | 0 | 99% |
| EU328184.1 | Zea mays chloroplast ferredoxin 5 precursor (FDX5) mRNA, complete cds; nuclear gene for chloroplast product | 545 | 545 | 22% | 2.00E-151 | 87% |
| EU975349.1 | Zea mays clone 488257 unknown mRNA | 542 | 542 | 22% | 3.00E-150 | 86% |
| EU972749.1 | Zea mays clone 387187 unknown mRNA | 526 | 526 | 21% | 2.00E-145 | 87% |
| NM_001111874.1 | Zea mays ferredoxin5 (fdx5), mRNA >gb\|M73828.1\|MZEFD5 Maize ferredoxin (Fd) isoprotein mRNA, pFD5 | 495 | 495 | 22% | 4.00E-136 | 83% |
| NM_001111374.1 | Zea mays ferredoxin2 (fdx2), mRNA >dbj\|AB016810.1\| Zea mays mRNA for ferredoxin, complete cds | 443 | 443 | 15% | 2.00E-120 | 92% |
| BT039722.1 | Zea mays full-length cDNA clone ZM_BFc0041B09 mRNA, complete cds | 434 | 434 | 15% | 1.00E-117 | 92% |

(continued)

| Accession | Description | Max score | Total score | Query coverage | E value | Max ident |
|---|---|---|---|---|---|---|
| EU328186.1 | Zea mays chloroplast ferredoxin 2 precursor (FDX2) mRNA, complete cds; nuclear gene for chloroplast product | 434 | 434 | 15% | 1.00E-117 | 92% |
| EU974838.1 | Zea mays clone 459526 unknown mRNA | 430 | 430 | 15% | 1.00E-116 | 91% |
| CT841984.1 | Oryza rufipogon (W1943) cDNA clone: ORW1943C104F01, full insert sequence | 405 | 405 | 15% | 5.00E-109 | 89% |
| AK287537.1 | Oryza sativa Japonica Group cDNA, clone: J065007C21, full insert sequence | 405 | 405 | 15% | 5.00E-109 | 89% |
| CU406957.1 | Oryza rufipogon (W1943) cDNA clone: ORW1943C107I19, full insert sequence | 405 | 405 | 15% | 5.00E-109 | 89% |
| CU406556.1 | Oryza rufipogon (W1943) cDNA clone: ORW1943S102N16, full insert sequence | 405 | 405 | 15% | 5.00E-109 | 89% |

7. Annotation of MAEM1

MAEM1 encodes maize ZCN9 protein (GenBank Accession: ABX11011.1). The top 10 homologous sequences identified in the BlastX query are presented in Table 45.

Table 45. BLASTX search results of the maize ZM4s09689 *(MAEM1)*

| Accession | Description | Score | E-value | % Identities |
|---|---|---|---|---|
| ABX11011.1 | ZCN9 [Zea mays] | 326 | 3.00E-97 | 100 |
| NP_001106248.1 | ZCN9 protein [Zea mays] | 326 | 3.00E-97 | 100 |
| NP_001106249.1 | ZCN10 protein [Zea mays] | 309 | 5.00E-92 | 94 |
| EAY84662.1 | hypothetical protein OsI-005895 [Oryza sativa (indica cultivar-group)] | 210 | 6.00E-76 | 84 |
| NP_001041806.1 | Os01g0111600 [Oryza sativa (japonica cultivar-group)] | 208 | 7.00E-75 | 83 |
| NP_001057701.1 | Os06g0498800 [Oryza sativa (japonica cultivar-group)] | 193 | 1.00E-65 | 68 |
| ABB90591.1 | terminal flower 1 [Aquilegia formosa] | 212 | 8.00E-60 | 63 |
| CAO68168.1 | unnamed protein product [Vitis vinifera] | 170 | 2.00E-59 | 65 |
| BAD22677.1 | flowering locus T like protein [Populus nigra] | 173 | 5.00E-59 | 67 |
| CAN80336.1 | hypothetical protein [Vitis vinifera] | 170 | 9.00E-59 | 65 |

The CDS sequence of the gene corresponding to MAEM1 is shown in SEQ ID NO: 20 and the translated amino acid sequence is shown in SEQ ID NO: 38.

Identification of the promoter region of MAEM1

For our promoter identification purposes, the sequence upstream of the start codon of the MAEM1 gene was defined as the promoter p-MAEM1. To identify this predicted promoter region, the sequence of ZM4s09689 was mapped to the BASF Plant Science proprietary maize genomic DNA sequence database, PUB_tigr_maize_genomic_partial_5.0.nt. One maize genomic DNA sequences, AZM5_13765 (3272 bp) was identified. This 3272bp sequence harbored the predicted CDS of the corresponding gene to MAWS23 and less than 0.5kb upstream sequence of the ATG start codon of this gene. In addition, a public available sequence CL383739 was overlapped with AZM5_13765. The contig of these 2 genomic sequences containing 0.9kb upstream region is shown in SEQ ID NO: 74.

Isolation of the promoter region by PCR amplification

The putative promoter region was isolated via genomic PCR using the following sequence specific primers:

Forward primer: TTAGTAGAGAATAACACACATC (SEQ ID NO: 184)
Reverse primer: GATCGATCGATCAACGCG (SEQ ID NO: 185). The expected 922bp fragment was amplified from maize genomic DNA, and named as promoter MAEM1 (p-MAEM1). Sequence of p-MAEM1 was shown in SEQ ID NO: 2.

BLASTN results of p_MAEM1

The top 18 homologous sequences identified in the BlastN query of p_MAEM1 are presented in Table46.

Table 46. BLASTN results of p_MAEM1

| Accession | Description | Max score | Total score | Query coverage | E value | Max ident |
|---|---|---|---|---|---|---|
| EU241901.1 | Zea mays ZCN10 (ZCN10) gene, complete cds | 1651 | 1651 | 100% | 0 | 99% |
| NM_001112778.1 | Zea mays ZCN10 protein (ZCN10), mRNA >gb\|EU241926.1\| Zea mays ZCN10 (ZCN10) mRNA, complete cds | 446 | 446 | 27% | 7.00E-122 | 99% |
| AY530950.1 | Zea mays putative zinc finger protein (Z438D03.1), unknown (Z438D03.5), epsilon-COP (Z438D03.6), putative kinase (Z438D03.7), unknown (Z438D03.25), and C1-B73 (Z438D03.27) genes, complete cds | 208 | 208 | 39% | 3.00E-50 | 74% |
| EU952257.1 | Zea mays clone 1273313 unknown mRNA | 196 | 196 | 11% | 2.00E-46 | 100% |
| EU241900.1 | Zea mays ZCN9 (ZCN9) gene, complete cds | 176 | 176 | 19% | 2.00E-40 | 82% |
| AP008207.1 | Oryza sativa (japonica cultivar-group) genomic DNA, chromosome 1 | 57.2 | 57.2 | 11% | 1.00E-04 | 72% |
| AP004821.4 | Oryza sativa Japonica Group genomic DNA, chromosome 1, PAC clone: P0676G08 | 57.2 | 57.2 | 11% | 1.00E-04 | 72% |
| AP003854.2 | Oryza sativa Japonica Group genomic DNA, chromosome 1, BAC clone:OSJNBb0093M23 | 57.2 | 57.2 | 11% | 1.00E-04 | 72% |
| EU976588.1 | Zea mays clone 984310 unknown mRNA | 55.4 | 55.4 | 3% | 5.00E-04 | 100% |
| AC135864.5 | Oryza sativa Japonica Group chromosome 11 clone OSJNBb0071 K13, complete sequence | 53.6 | 53.6 | 13% | 0.002 | 70% |
| AP008217.1 | Oryza sativa (japonica cultivar-group) genomic DNA, chromosome 11 | 53.6 | 99 | 13% | 0.002 | 77% |

(continued)

| Accession | Description | Max score | Total score | Query coverage | E value | Max ident |
|---|---|---|---|---|---|---|
| AP008215.1 | Oryza sativa (japonica cultivar-group) genomic DNA, chromosome 9 | 51.8 | 187 | 5% | 0.006 | 85% |
| AP005767.3 | Oryza sativa Japonica Group genomic DNA, chromosome 9, BAC clone:OSJNBa0035G04 | 51.8 | 51.8 | 4% | 0.006 | 85% |
| AP005780.2 | Oryza sativa Japonica Group genomic DNA, chromosome 9, BAC clone:OSJNBb0051 H02 | 51.8 | 51.8 | 4% | 0.006 | 85% |
| AC139170.2 | Oryza sativa Japonica Group chromosome 11 clone OSJNBa0058P12, complete sequence | 46.4 | 46.4 | 6% | 0.25 | 77% |
| AP008218.1 | Oryza sativa (japonica cultivar-group) genomic DNA, chromosome 12 | 46.4 | 46.4 | 6% | 0.25 | 77% |
| AP008208.1 | Oryza sativa (japonica cultivar-group) genomic DNA, chromosome 2 | 46.4 | 92.7 | 5% | 0.25 | 81% |
| AP005559.3 | Oryza sativa Japonica Group genomic DNA, chromosome 9, BAC clone:OJ1163_C07 | 46.4 | 46.4 | 5% | 0.25 | 81% |

8. Annotation of MAEM20

MAEM20 encodes a protein that is homologous to rice hypothetical protein OsJ_029225 (GenBank Accession: EAZ45742.1). The top 10 homologous sequences identified in the BlastX query are presented in Table 47.

Table 47. BLASTX search results of the maize ZM1s59153555 *(MAEM20)*

| Accession | Description | Score | E-value | % Identities |
|---|---|---|---|---|
| EAZ45742.1 | hypothetical protein OsJ_029225 | 154 | 4.00E-35 | 80 |
| EAZ10155.1 | hypothetical protein OsI_031387 [Oryza sativa (indica cultivar-group)] | 154 | 4.00E-35 | 80 |
| BAD46602.1 | putative Histone H2B [Oryza sativa Japonica Group] | 154 | 4.00E-35 | 80 |
| CAN78957.1 | hypothetical protein [Vitis vinifera] | 139 | 1.00E-30 | 69 |
| NP_172295.1 | histone H2B family protein [Arabidopsis thaliana] | 133 | 7.00E-30 | 62 |
| XP_001104238.1 | PREDICTED: similar to Histone H2B [Macaca mulatta] | 132 | 2.00E-28 | 64 |
| XP_001914780.1 | PREDICTED: similar to histone H2B.3 [Equus caballus] | 131 | 4.00E-28 | 63 |
| XP_532763.2 | PREDICTED: similar to testis-specific histone 2b [Canis familiaris] | 129 | 1.00E-27 | 62 |
| XP_872016.1 | PREDICTED: similar to histone H2B.3 [Bos taurus] | 128 | 2.00E-27 | 62 |
| XP_002060453.1 | GJ19809 [Drosophila virilis] | 126 | 1.00E-26 | 59 |

The CDS sequence of the gene corresponding to MAEM20 is shown in SEQ ID NO: 23 and the translated amino acid sequence is shown in SEQ ID NO: 41.

Identification of the promoter region of MAEM20

For our promoter identification purposes, the sequence upstream of the start codon of the MAEM20 gene was defined as the promoter p-MAEM20. To identify this predicted promoter region, the sequence of ZM1s59153555 was mapped to the BASF Plant Science proprietary maize genomic DNA sequence database, PUB_tigr_maize_genomic_partial_5.0.nt. One maize genomic DNA sequences, AZM5_23292 (1996 bp) was identified. This 1996bp sequence harbored the predicted CDS of the corresponding gene to MAEM20 and 0.7kb upstream sequence of the ATG start codon of this gene. Sequence of AZM5_23292 is shown in SEQ ID NO: 77.

Isolation of the promoter region by PCR amplification

The putative promoter region was isolated via genomic PCR using the following sequence specific primers:

Forward primer: GTGATTAAGTTGACTGGCAAATTG (SEQ ID NO: 186)
Reverse primer: GCCTACTTGCCTAGCGTACC (SEQ ID NO: 187). The expected 698bp fragment was amplified from maize genomic DNA, and named as promoter MAEM20 (p-MAEM20). Sequence of p-MAEM20 was shown in SEQ ID NO: 5.

BLASTN results of p_MAEM20

The top 16 homologous sequences identified in the BlastN query of p_MAEM20 are presented in Table 48.

Table 48. BLASTN results of p_MAEM20

| Accession | Description | Max score | Total score | Query coverage | E value | Max ident |
|---|---|---|---|---|---|---|
| EU951788.1 | Zea mays clone 1000340 unknown mRNA | 196 | 196 | 15% | 2.00E-46 | 100% |
| CP000820.1 | Frankia sp. EAN 1 pec, complete genome | 44.6 | 44.6 | 5% | 0.64 | 86% |
| AC174361.12 | Medicago truncatula chromosome 8 clone mth2-39o9, complete sequence | 42.8 | 42.8 | 4% | 2.2 | 93% |
| AC146791.12 | Medicago truncatula chromosome 8 clone mth2-123m17, complete sequence | 42.8 | 42.8 | 4% | 2.2 | 93% |
| XM_001502388.2 | PREDICTED: Equus caballus similar to neurotrypsin (LOC100072455), mRNA | 41 | 41 | 3% | 7.8 | 92% |
| CP000548.1 | Burkholderia mallei NCTC 10247 chromosome I, complete sequence | 41 | 41 | 3% | 7.8 | 92% |
| CP000572.1 | Burkholderia pseudomallei 1106a chromosome I, complete sequence | 41 | 41 | 3% | 7.8 | 92% |
| CP000546.1 | Burkholderia mallei NCTC 10229 chromosome I, complete sequence | 41 | 41 | 3% | 7.8 | 92% |
| CP000526.1 | Burkholderia mallei SAVP1 chromosome I, complete sequence | 41 | 41 | 3% | 7.8 | 92% |
| CP000539.1 | Acidovorax sp. JS42, complete genome | 41 | 41 | 5% | 7.8 | 85% |
| CP000489.1 | Paracoccus denitrificans PD1222 chromosome 1, complete sequence | 41 | 41 | 4% | 7.8 | 90% |
| EF130439.1 | Sus scrofa clone KVL4379 microsatellite sequence | 41 | 41 | 5% | 7.8 | 86% |
| CP000383.1 | Cytophaga hutchinsonii ATCC 33406, complete genome | 41 | 41 | 3% | 7.8 | 96% |

(continued)

| Accession | Description | Max score | Total score | Query coverage | E value | Max ident |
|---|---|---|---|---|---|---|
| XM_001106371.1 | PREDICTED: Macaca mulatta similar to chromosome 9 open reading frame 58 isoform 1, transcript variant 4 (LOC715655), mRNA | 41 | 41 | 4% | 7.8 | 90% |
| CP000010.1 | Burkholderia mallei ATCC 23344 chromosome 1, complete sequence | 41 | 41 | 3% | 7.8 | 92% |

EXAMPLE 11

PLACE ANALYSIS OF THE PROMOTERS

*Cis*-acting motifs in the promoter regions were identified using PLACE (a database of Plant Cis-acting Regulatory DNA elements) using the Genomatix database suite.

1) p-MAWS23

PLACE analysis results of p-MAWS23 are listed in Table 49. Two TATA box motifs are found in this promoter, one located at nucleotide position 419 - 425 of the forward strand, the other located at nucleotide position 736 - 742 of the reverse strand. There is 1 CAAT Box motif at nucleotide position 621 - 625 of the forward strand.

Table 49. PLACE analysis results of the 1264bp promoter p-MAWS23

| IUPAC Family | IUPAC | Start pos. | End pos. | Strand | Misma tches | Score | Sequence |
|---|---|---|---|---|---|---|---|
| FAM324 | CGCGBOXAT | 23 | 28 | + | 0 | 1 | GCGCGT |
| FAM324 | CGCGBOXAT | 23 | 28 | - | 0 | 1 | ACGCGC |
| FAM107 | CGACGOSAMY3 | 26 | 30 | - | 0 | 1 | CGACG |
| FAM261 | CDTDREHVCBF2 | 27 | 32 | + | 0 | 1 | GTCGAC |
| FAM261 | CDTDREHVCBF2 | 27 | 32 | - | 0 | 1 | GTCGAC |
| FAM107 | CGACGOSAMY3 | 29 | 33 | + | 0 | 1 | CGACG |
| FAM002 | TGACGTVMAMY | 48 | 60 | + | 0 | 1 | GACTATGACGTCA |
| FAM002 | PALINDROMICCBOXGM | 50 | 62 | - | 0 | 1 | GATGACGTCATAG |
| FAM002 | PALINDROMICCBOXGM | 51 | 63 | + | 0 | 1 | TATGACGTCATCT |
| FAM002 | TGACGTVMAMY | 53 | 65 | - | 0 | 1 | CAAGATGACGTCA |
| FAM057 | ACGTCBOX | 54 | 59 | + | 0 | 1 | GACGTC |
| FAM057 | ACGTCBOX | 54 | 59 | - | 0 | 1 | GACGTC |
| FAM010 | WBOXATNPR1 | 62 | 76 | + | 0 | 1 | CTTGACACCAGAGGT |
| FAM322 | BIHD1OS | 64 | 68 | - | 0 | 1 | TGTCA |
| FAM263 | DPBFCOREDCDC3 | 66 | 72 | + | 0 | 1 | ACACCAG |
| FAM322 | BIHD1OS | 76 | 80 | - | 0 | 1 | TGTCA |
| FAM270 | RAV1AAT | 92 | 96 | + | 0 | 1 | CAACA |
| FAM270 | RAV1AAT | 95 | 99 | + | 0 | 1 | CAACA |
| FAM266 | MYB1AT | 101 | 106 | + | 0 | 1 | TAACCA |
| FAM002 | SORLIP1AT | 104 | 116 | + | 0 | 1 | CCACTCGCCACCG |
| FAM147 | HEXAMERATH4 | 114 | 119 | + | 0 | 1 | CCGTCG |
| FAM013 | DRE2COREZMRAB17 | 115 | 121 | - | 0 | 1 | ACCGACG |
| FAM107 | CGACGOSAMY3 | 115 | 119 | - | 0 | 1 | CGACG |
| FAM013 | DRE2COREZMRAB17 | 119 | 125 | - | 0 | 1 | ACCGACC |
| FAM267 | TAAAGSTKST1 | 130 | 136 | - | 0 | 1 | ATTAAAG |

(continued)

| IUPAC Family | IUPAC | Start pos. | End pos. | Strand | Misma tches | Score | Sequence |
|---|---|---|---|---|---|---|---|
| FAM304 | OSE2ROOTNODULE | 138 | 142 | + | 0 | 1 | CTCTT |
| FAM024 | 2SSEEDPROTBANAPA | 145 | 153 | + | 0 | 1 | CAAACACAT |
| FAM263 | DPBFCOREDCDC3 | 154 | 160 | + | 0 | 1 | ACACTTG |
| FAM305 | ANAERO1CONSENSUS | 165 | 171 | - | 0 | 1 | AAACAAA |
| FAM314 | SORLIP4AT | 182 | 190 | + | 0 | 1 | GTATGATGG |
| FAM010 | WBOXHVISO1 | 200 | 214 | + | 0 | 1 | GATGACTGACAATGT |
| FAM322 | BIHD1OS | 206 | 210 | - | 0 | 1 | TGTCA |
| FAM002 | RAV1BAT | 230 | 242 | - | 0 | 1 | TTACACCTGCCGG |
| FAM263 | DPBFCOREDCDC3 | 234 | 240 | - | 0 | 1 | ACACCTG |
| FAM002 | CACGTGMOTIF | 239 | 251 | - | 0 | 1 | GAGCACGTGTTAC |
| FAM002 | CACGTGMOTIF | 240 | 252 | + | 0 | 1 | TAACACGTGCTCT |
| FAM263 | DPBFCOREDCDC3 | 242 | 248 | + | 0 | 1 | ACACGTG |
| FAM304 | OSE2ROOTNODULE | 249 | 253 | + | 0 | 1 | CTCTT |
| FAM012 | IBOXCORENT | 258 | 264 | + | 0 | 1 | GATAAGA |
| FAM026 | RYREPEATBNNAPA | 264 | 274 | + | 0 | 1 | ATCATGCAAAT |
| FAM311 | EECCRCAH1 | 269 | 275 | - | 0 | 1 | GATTTGC |
| FAM322 | BIHD1OS | 277 | 281 | + | 0 | 1 | TGTCA |
| FAM026 | RYREPEATBNNAPA | 291 | 301 | - | 0 | 1 | ATCATGCAGGC |
| FAM151 | INTRONLOWER | 292 | 297 | - | 0 | 1 | TGCAGG |
| FAM170 | MYBGAHV | 343 | 349 | + | 0 | 1 | TAACAAA |
| FAM300 | LECPLEACS2 | 382 | 389 | + | 0 | 1 | TAAAATAT |
| FAM243 | TATABOX4 | 419 | 425 | + | 0 | 1 | TATATAA |
| FAM270 | RAV1AAT | 449 | 453 | - | 0 | 1 | CAACA |
| FAM295 | P1BS | 475 | 482 | + | 0 | 1 | GTATATCC |
| FAM295 | P1BS | 475 | 482 | - | 0 | 1 | GGATATAC |

(continued)

| IUPAC Family | IUPAC | Start pos. | End pos. | Strand | Misma tches | Score | Sequence |
|---|---|---|---|---|---|---|---|
| FAM014 | MYBST1 | 477 | 483 | - | 0 | 1 | TGGATAT |
| FAM025 | AMYBOX2 | 478 | 484 | + | 0 | 1 | TATCCAT |
| FAM273 | TATCCAOSAMY | 478 | 484 | + | 0 | 1 | TATCCAT |
| FAM107 | CGACGOSAMY3 | 502 | 506 | - | 0 | 1 | CGACG |
| FAM026 | RYREPEATBNNAPA | 514 | 524 | - | 0 | 1 | CTCATGCAAGC |
| FAM261 | CDTDREHVCBF2 | 526 | 531 | + | 0 | 1 | GTCGAC |
| FAM261 | CDTDREHVCBF2 | 526 | 531 | - | 0 | 1 | GTCGAC |
| FAM304 | OSE2ROOTNODULE | 532 | 536 | + | 0 | 1 | CTCTT |
| FAM304 | OSE2ROOTNODULE | 537 | 541 | + | 0 | 1 | CTCTT |
| FAM012 | IBOXCORE | 540 | 546 | - | 0 | 1 | GATAAAA |
| FAM014 | SREATMSD | 541 | 547 | + | 0 | 1 | TTTATCC |
| FAM014 | MYBST1 | 542 | 548 | - | 0 | 1 | GGGATAA |
| FAM006 | HDZIP2ATATHB2 | 588 | 596 | - | 0 | 1 | TAATAATTA |
| FAM015 | ACGTABOX | 597 | 602 | + | 0 | 1 | TACGTA |
| FAM015 | ACGTABOX | 597 | 602 | - | 0 | 1 | TACGTA |
| FAM010 | WBOXHVISO1 | 600 | 614 | - | 0 | 1 | TATGACTTGAAGTAC |
| FAM266 | MYB1AT | 618 | 623 | + | 0 | 1 | AAACCA |
| FAM003 | REALPHALGLHCB21 | 619 | 629 | + | 0 | 1 | AACCAATGGCA |
| FAM100 | CCAATBOX1 | 621 | 625 | + | 0 | 1 | CCAAT |
| FAM266 | MYB1AT | 640 | 645 | + | 0 | 1 | TAACCA |
| FAM267 | TAAAGSTKST1 | 668 | 674 | + | 0 | 1 | AATAAAG |
| FAM244 | TATABOXOSPAL | 736 | 742 | - | 0 | 1 | TATTTAA |
| FAM267 | TAAAGSTKST1 | 756 | 762 | - | 0 | 1 | AATAAAG |
| FAM171 | MYBPZM | 782 | 788 | - | 0 | 1 | TCCAACC |
| FAM061 | GCCCORE | 795 | 801 | - | 0 | 1 | CGCCGCC |

(continued)

| IUPAC Family | IUPAC | Start pos. | End pos. | Strand | Misma tches | Score | Sequence |
|---|---|---|---|---|---|---|---|
| FAM026 | RYREPEATVFLEB4 | 812 | 822 | + | 0 | 1 | ACCATGCATGT |
| FAM026 | RYREPEATVFLEB4 | 813 | 823 | - | 0 | 1 | CACATGCATGG |
| FAM 172 | MYCATRD2 | 817 | 823 | - | 0 | 1 | CACATGC |
| FAM 172 | MYCATERD | 818 | 824 | + | 0 | 1 | CATGTGT |
| FAM263 | DPBFCOREDCDC3 | 818 | 824 | - | 0 | 1 | ACACATG |
| FAM012 | IBOXCORENT | 843 | 849 | - | 0 | 1 | GATAAGA |
| FAM 171 | MYBPZM | 854 | 860 | + | 0 | 1 | TCCTACC |
| FAM304 | OSE2ROOTNODULE | 864 | 868 | + | 0 | 1 | CTCTT |
| FAM267 | TAAAGSTKST1 | 866 | 872 | - | 0 | 1 | ATTAAAG |
| FAM010 | WBBOXPCWRKY1 | 872 | 886 | + | 0 | 1 | TTTGACTCTTTATGA |
| FAM304 | OSE2ROOTNODULE | 877 | 881 | + | 0 | 1 | CTCTT |
| FAM267 | TAAAGSTKST1 | 879 | 885 | - | 0 | 1 | CATAAAG |
| FAM311 | EECCRCAH1 | 889 | 895 | - | 0 | 1 | GAATTCC |
| FAM311 | EECCRCAH1 | 890 | 896 | + | 0 | 1 | GAATTCC |
| FAM098 | CATATGGMSAUR | 897 | 902 | + | 0 | 1 | CATATG |
| FAM098 | CATATGGMSAUR | 897 | 902 | - | 0 | 1 | CATATG |
| FAM087 | BOXIINTPATPB | 905 | 910 | - | 0 | 1 | ATAGAA |
| FAM270 | RAV1AAT | 915 | 919 | + | 0 | 1 | CAACA |
| FAM107 | CGACGOSAMY3 | 923 | 927 | + | 0 | 1 | CGACG |
| FAM057 | ACGTCBOX | 924 | 929 | + | 0 | 1 | GACGTC |
| FAM057 | ACGTCBOX | 924 | 929 | - | 0 | 1 | GACGTC |
| FAM013 | DRECRTCOREAT | 957 | 963 | + | 0 | 1 | GCCGACG |
| FAM107 | CGACGOSAMY3 | 959 | 963 | + | 0 | 1 | CGACG |
| FAM 147 | HEXAMERATH4 | 959 | 964 | - | 0 | 1 | CCGTCG |
| FAM026 | SPHCOREZMC1 | 986 | 996 | + | 0 | 1 | TCCATGCATGC |

(continued)

| IUPAC Family | IUPAC | Start pos. | End pos. | Strand | Misma tches | Score | Sequence |
|---|---|---|---|---|---|---|---|
| FAM026 | RYREPEATVFLEB4 | 987 | 997 | - | 0 | 1 | TGCATGCATGG |
| FAM026 | RYREPEATBNNAPA | 990 | 1000 | + | 0 | 1 | TGCATGCAAAT |
| FAM 172 | MYCATERD | 1005 | 1011 | - | 0 | 1 | CATGTGT |
| FAM263 | DPBFCOREDCDC3 | 1005 | 1011 | + | 0 | 1 | ACACATG |
| FAM 172 | MYCATRD2 | 1006 | 1012 | + | 0 | 1 | CACATGT |
| FAM205 | PYRIMIDINEBOXOSRAM | 1018 | 1023 | + | 0 | 1 | CCTTTT |
| FAM026 | RYREPEATLEGUMINBO X | 1030 | 1040 | + | 0 | 1 | GGCATGCACCC |
| FAM002 | SORLIP1AT | 1059 | 1071 | - | 0 | 1 | TTCACGGCCACGG |
| FAM322 | BIHD1OS | 1074 | 1078 | - | 0 | 1 | TGTCA |
| FAM324 | CGCGBOXAT | 1100 | 1105 | + | 0 | 1 | CCGCGC |
| FAM324 | CGCGBOXAT | 1100 | 1105 | - | 0 | 1 | GCGCGG |
| FAM324 | CGCGBOXAT | 1102 | 1107 | + | 0 | 1 | GCGCGT |
| FAM324 | CGCGBOXAT | 1102 | 1107 | - | 0 | 1 | ACGCGC |
| FAM322 | BIHD1OS | 1112 | 1116 | + | 0 | 1 | TGTCA |
| FAM026 | RYREPEATBNNAPA | 1137 | 1147 | + | 0 | 1 | TCCATGCAAGC |
| FAM002 | SORLIP1AT | 1152 | 1164 | + | 0 | 1 | ACCCGGGCCACGT |
| FAM302 | SORLIP2AT | 1155 | 1165 | + | 0 | 1 | CGGGCCACGTA |
| FAM002 | ABREATCONSENSUS | 1156 | 1168 | - | 0 | 1 | GGGTACGTGGCCC |
| FAM002 | ABREMOTIFAOSOSEM | 1179 | 1191 | + | 0 | 1 | CGCTACGTGTCAC |
| FAM322 | BIHD1O | 1186 | 1190 | + | 0 | 1 | TGTCA |
| FAM002 | ASF1MOTIFCAMV | 1195 | 1207 | - | 0 | 1 | ATAGGTGACGAGA |
| FAM272 | SV40COREEN HAN | 1228 | 1235 | - | 0 | 1 | GTGGAAAG |
| FAM002 | ASF1MOTIFCAMV | 1243 | 1255 | - | 0 | 1 | CAAAGTGACGGAG |
| FAM245 | TBOXATGAPB | 1250 | 1255 | + | 0 | 1 | ACTTTG |
| FAM305 | ANAERO1CONSENSUS | 1252 | 1258 | - | 0 | 1 | AAACAAA |

2) p-MAWS27

PLACE analysis results of p-MAWS27 are listed in Table 50. Multiple TATA box motifs are found in this promoter, located at nucleotide position 1-7, 278-284, 597-603, 1246-1252 of the forward strand, and 273-279, 533-539 of the reverse strand, respectively. Three CAAT Box motifs are located at nucleotide position -947-951, 968-972 and 985-989 of the forward strand.

Table 50. PLACE analysis results of the 1355bp promoter p-MAWS27

| IUPAC Family | IUPAC | Start pos. | End pos. | Strand | Mismatches | Score | Sequence |
|---|---|---|---|---|---|---|---|
| FAM241 | TATABOX2 | 1 | 7 | + | 0 | 1 | TATAAAT |
| FAM325 | MYBCOREATCYCB1 | 11 | 15 | + | 0 | 1 | AACGG |
| FAM002 | T/GBOXATPIN2 | 54 | 66 | - | 0 | 1 | TTAAACGTGATGA |
| FAM307 | ANAERO3CONSENSUS | 54 | 60 | + | 0 | 1 | TCATCAC |
| FAM305 | ANAERO1CONSENSUS | 67 | 73 | - | 0 | 1 | AAACAAA |
| FAM292 | PREATPRODH | 84 | 89 | - | 0 | 1 | ACTCAT |
| FAM008 | MYB2AT | 90 | 100 | - | 0 | 1 | GCTGTAACTGA |
| FAM012 | IBOXCORE | 105 | 111 | + | 0 | 1 | GATAATT |
| FAM107 | CGACGOSAMY3 | 112 | 116 | + | 0 | 1 | CGACG |
| FAM147 | HEXAMERATH4 | 112 | 117 | - | 0 | 1 | CCGTCG |
| FAM026 | RYREPEATBNNAPA | 123 | 133 | - | 0 | 1 | TGCATGCAAAT |
| FAM026 | RYREPEATLEGUMINBOX | 126 | 136 | + | 0 | 1 | TGCATGCACTT |
| FAM290 | GT1GMSCAM4 | 207 | 212 | - | 0 | 1 | GAAAAA |
| FAM304 | OSE2ROOTNODULE | 212 | 216 | + | 0 | 1 | CTCTT |
| FAM014 | REBETALGLHCB21 | 217 | 223 | + | 0 | 1 | CGGATAC |
| FAM014 | REBETALGLHCB21 | 223 | 229 | + | 0 | 1 | CGGATAT |
| FAM012 | IBOXCORE | 227 | 233 | - | 0 | 1 | GATAATA |
| FAM260 | CAREOSREP1 | 234 | 239 | - | 0 | 1 | CAACTC |
| FAM244 | TATABOXOSPAL | 273 | 279 | - | 0 | 1 | TATTTAA |
| FAM243 | TATABOX4 | 278 | 284 | + | 0 | 1 | TATATAA |
| FAM245 | TBOXATGAPB | 284 | 289 | + | 0 | 1 | ACTTTG |
| FAM295 | P1BS | 289 | 296 | + | 0 | 1 | GAATATAC |
| FAM295 | P1BS | 289 | 296 | - | 0 | 1 | GTATATTC |
| FAM014 | REBETALGLHCB21 | 296 | 302 | + | 0 | 1 | CGGATAC |
| FAM015 | ACGTABOX | 300 | 305 | + | 0 | 1 | TACGTA |
| FAM015 | ACGTABOX | 300 | 305 | - | 0 | 1 | TACGTA |
| FAM014 | REBETALGLHCB21 | 313 | 319 | + | 0 | 1 | CGGATAT |
| FAM262 | CIACADIANLELHC | 340 | 349 | - | 0 | 1 | CAATTTAATC |
| FAM100 | CCAATBOX1 | 346 | 350 | - | 0 | 1 | CCAAT |
| FAM280 | AGMOTIFNTMYB2 | 347 | 354 | - | 0 | 1 | AGATCCAA |
| FAM024 | PROXBBNNAPA | 368 | 376 | - | 0 | 1 | CAAACACCC |
| FAM310 | CPBCSPOR | 389 | 394 | - | 0 | 1 | TATTAG |

(continued)

| IUPAC Family | IUPAC | Start pos. | End pos. | Strand | Mismatches | Score | Sequence |
|---|---|---|---|---|---|---|---|
| FAM002 | SORLIP1AT | 397 | 409 | - | 0 | 1 | ATTTTAGCCACTA |
| FAM024 | PROXBBNNAPA | 423 | 431 | + | 0 | 1 | CAAACACCC |
| FAM310 | CPBCSPOR | 435 | 440 | - | 0 | 1 | TATTAG |
| FAM310 | CPBCSPOR | 448 | 453 | + | 0 | 1 | TATTAG |
| FAM012 | IBOXCORE | 485 | 491 | - | 0 | 1 | GATAACT |
| FAM170 | AMYBOX1 | 490 | 496 | - | 0 | 1 | TAACAGA |
| FAM156 | L1BOXATPDF1 | 528 | 535 | - | 0 | 1 | TAAATGCA |
| FAM244 | TATABOXOSPAL | 533 | 539 | - | 0 | 1 | TATTTAA |
| FAM025 | AMYBOX2 | 569 | 575 | - | 0 | 1 | TATCCAT |
| FAM273 | TATCCAOSAMY | 569 | 575 | - | 0 | 1 | TATCCAT |
| FAM014 | MYBST1 | 570 | 576 | + | 0 | 1 | TGGATAA |
| FAM014 | SREATMSD | 571 | 577 | - | 0 | 1 | ATTATCC |
| FAM012 | IBOXCORE | 572 | 578 | + | 0 | 1 | GATAATA |
| FAM014 | MYBST1 | 576 | 582 | - | 0 | 1 | TGGATAT |
| FAM025 | AMYBOX2 | 577 | 583 | + | 0 | 1 | TATCCAT |
| FAM273 | TATCCAOSAMY | 577 | 583 | + | 0 | 1 | TATCCAT |
| FAM202 | -300ELEMENT | 586 | 594 | - | 0 | 1 | TGTAAAATG |
| FAM227 | SEF1MOTIF | 597 | 605 | - | 0 | 1 | ATATTTATA |
| FAM241 | TATABOX2 | 597 | 603 | + | 0 | 1 | TATAAAT |
| FAM010 | WBOXATNPR1 | 616 | 630 | - | 0 | 1 | TTTGACATCTATATA |
| FAM322 | BIHD1OS | 624 | 628 | + | 0 | 1 | TGTCA |
| FAM171 | MYBPZM | 635 | 641 | - | 0 | 1 | CCCAACC |
| FAM270 | RAV1AAT | 644 | 648 | - | 0 | 1 | CAACA |
| FAM002 | SORLIP1AT | 655 | 667 | - | 0 | 1 | TATCGTGCCACGG |
| FAM276 | TRANSINITDICOTS | 686 | 693 | + | 0 | 1 | AACATGGC |
| FAM061 | GCCCORE | 696 | 702 | - | 0 | 1 | CGCCGCC |
| FAM290 | GT1GMSCAM4 | 742 | 747 | - | 0 | 1 | GAAAAA |
| FAM260 | CAREOSREP1 | 808 | 813 | + | 0 | 1 | CAACTC |
| FAM012 | IBOX | 863 | 869 | - | 0 | 1 | GATAAGC |
| FAM303 | OSE1ROOTNODULE | 866 | 872 | - | 0 | 1 | AAAGATA |
| FAM228 | SEF3MOTIFGM | 909 | 914 | + | 0 | 1 | AACCCA |
| FAM205 | PYRIMIDINEBOXOSRAM | 940 | 945 | + | 0 | 1 | CCTTTT |
| FAM100 | CCAATBOX1 | 947 | 951 | + | 0 | 1 | CCAAT |
| FAM002 | ABRELATERD | 954 | 966 | - | 0 | 1 | ACGGACGTGGTTT |
| FAM266 | MYB1AT | 954 | 959 | + | 0 | 1 | AAACCA |
| FAM194 | PALBOXAPC | 963 | 969 | + | 0 | 1 | CCGTCCC |
| FAM100 | CCAATBOX1 | 968 | 972 | + | 0 | 1 | CCAAT |

(continued)

| IUPAC Family | IUPAC | Start pos. | End pos. | Strand | Mismatches | Score | Sequence |
|---|---|---|---|---|---|---|---|
| FAM221 | S1FBOXSORPS1L21 | 971 | 976 | + | 0 | 1 | ATGGTA |
| FAM228 | SEF3MOTIFGM | 976 | 981 | + | 0 | 1 | AACCCA |
| FAM100 | CCAATBOX1 | 985 | 989 | + | 0 | 1 | CCAAT |
| FAM263 | DPBFCOREDCDC3 | 990 | 996 | - | 0 | 1 | ACACGAG |
| FAM024 | CANBNNAPA | 991 | 999 | - | 0 | 1 | CGAACACGA |
| FAM069 | ARFAT | 1003 | 1009 | - | 0 | 1 | CTGTCTC |
| FAM069 | SURECOREATSULTR11 | 1003 | 1009 | + | 0 | 1 | GAGACAG |
| FAM271 | SEBFCONSSTPR10A | 1003 | 1009 | - | 0 | 1 | CTGTCTC |
| FAM026 | RYREPEATBNNAPA | 1010 | 1020 | + | 0 | 1 | AGCATGCAAAC |
| FAM305 | ANAERO1CONSENSUS | 1017 | 1023 | + | 0 | 1 | AAACAAA |
| FAM039 | AACACOREOSGLUB1 | 1018 | 1024 | + | 0 | 1 | AACAAAC |
| FAM026 | RYREPEATVFLEB4 | 1025 | 1035 | + | 0 | 1 | AGCATGCATGC |
| FAM026 | RYREPEATVFLEB4 | 1026 | 1036 | - | 0 | 1 | CGCATGCATGC |
| FAM324 | CGCGBOXAT | 1053 | 1058 | + | 0 | 1 | GCGCGC |
| FAM324 | CGCGBOXAT | 1053 | 1058 | - | 0 | 1 | GCGCGC |
| FAM324 | CGCGBOXAT | 1055 | 1060 | + | 0 | 1 | GCGCGG |
| FAM324 | CGCGBOXAT | 1055 | 1060 | - | 0 | 1 | CCGCGC |
| FAM002 | ABRELATERD | 1058 | 1070 | + | 0 | 1 | CGGGACGTGAACC |
| FAM324 | CGCGBOXAT | 1069 | 1074 | + | 0 | 1 | CCGCGC |
| FAM324 | CGCGBOXAT | 1069 | 1074 | - | 0 | 1 | GCGCGG |
| FAM324 | CGCGBOXAT | 1071 | 1076 | + | 0 | 1 | GCGCGC |
| FAM324 | CGCGBOXAT | 1071 | 1076 | - | 0 | 1 | GCGCGC |
| FAM061 | GCCCORE | 1083 | 1089 | + | 0 | 1 | TGCCGCC |
| FAM194 | PALBOXAPC | 1089 | 1095 | + | 0 | 1 | CCGTCCG |
| FAM069 | SURECOREATSULTR11 | 1094 | 1100 | - | 0 | 1 | GAGACCG |
| FAM002 | ASF1MOTIFCAMV | 1139 | 1151 | + | 0 | 1 | CATCCTGACGCGC |
| FAM324 | CGCGBOXAT | 1146 | 1151 | + | 0 | 1 | ACGCGC |
| FAM324 | CGCGBOXAT | 1146 | 1151 | - | 0 | 1 | GCGCGT |
| FAM324 | CGCGBOXAT | 1148 | 1153 | + | 0 | 1 | GCGCGT |
| FAM324 | CGCGBOXAT | 1148 | 1153 | - | 0 | 1 | ACGCGC |
| FAM302 | SORLIP2AT | 1157 | 1167 | - | 0 | 1 | GGGGCCCAGAC |
| FAM302 | SITEIIATCYTC | 1160 | 1170 | + | 0 | 1 | TGGGCCCCAAA |
| FAM002 | ABRELATERD | 1169 | 1181 | - | 0 | 1 | GCGGACGTGGTTT |
| FAM266 | MYB1AT | 1169 | 1174 | + | 0 | 1 | AAACCA |
| FAM002 | ABREOSRAB21 | 1170 | 1182 | + | 0 | 1 | AACCACGTCCGCC |
| FAM324 | CGCGBOXAT | 1181 | 1186 | + | 0 | 1 | CCGCGG |
| FAM324 | CGCGBOXAT | 1181 | 1186 | - | 0 | 1 | CCGCGG |

(continued)

| IUPAC Family | IUPAC | Start pos. | End pos. | Strand | Mismatches | Score | Sequence |
|---|---|---|---|---|---|---|---|
| FAM061 | GCCCORE | 1185 | 1191 | - | 0 | 1 | CGCCGCC |
| FAM061 | GCCCORE | 1188 | 1194 | - | 0 | 1 | CGCCGCC |
| FAM324 | CGCGBOXAT | 1192 | 1197 | + | 0 | 1 | GCGCGC |
| FAM324 | CGCGBOXAT | 1192 | 1197 | - | 0 | 1 | GCGCGC |
| FAM061 | GCCCORE | 1236 | 1242 | + | 0 | 1 | TGCCGCC |
| FAM241 | TATABOX2 | 1246 | 1252 | + | 0 | 1 | TATAAAT |
| FAM156 | L1BOXATPDF1 | 1248 | 1255 | + | 0 | 1 | TAAATGCA |
| FAM069 | SURECOREATSULTR11 | 1269 | 1275 | - | 0 | 1 | GAGACGC |
| FAM246 | TCA1MOTIF | 1283 | 1292 | + | 0 | 1 | TCATCTTCTT |

3) p-MAWS30

PLACE analysis results of p-MAWS30 are listed in Table 51. One TATA box motif is found in this promoter, located at nucleotide position290-296 of the forward strand. No CAAT Box motifs are found in this promoter.

Table 51. PLACE analysis results of the 623bp promoter p-MAWS30

| IUPAC Family | IUPAC | Start pos. | End pos. | Strand | Mismat ches | Score | Sequence |
|---|---|---|---|---|---|---|---|
| FAM234 | SP8BFIBSP8BIB | 15 | 21 | - | 0 | 1 | TACTATT |
| FAM267 | TAAAGSTKST1 | 18 | 24 | + | 0 | 1 | AGTAAAG |
| FAM267 | NTBBF1ARROLB | 19 | 25 | - | 0 | 1 | ACTTTAC |
| FAM266 | MYB1AT | 31 | 36 | - | 0 | 1 | TAACCA |
| FAM322 | BIHD1O | 56 | 60 | + | 0 | 1 | TGTCA |
| FAM027 | -10PEHVPSBD | 72 | 77 | - | 0 | 1 | TATTCT |
| FAM010 | WBOXHVISO1 | 73 | 87 | - | 0 | 1 | CGTGACTACATATTC |
| FAM270 | RAV1AAT | 90 | 94 | + | 0 | 1 | CAACA |
| FAM290 | GT1GMSCAM4 | 123 | 128 | - | 0 | 1 | GAAAAA |
| FAM171 | MYBPZM | 132 | 138 | + | 0 | 1 | TCCAACC |
| FAM027 | -10PEHVPSBD | 152 | 157 | + | 0 | 1 | TATTCT |
| FAM156 | L1BOXATPDF1 | 167 | 174 | + | 0 | 1 | TAAATGTA |
| FAM014 | MYBST1 | 186 | 192 | - | 0 | 1 | AGGATAG |
| FAM205 | PYRIMIDINEBOXOSRAM | 190 | 195 | + | 0 | 1 | CCTTTT |
| FAM290 | GT1GMSCAM4 | 192 | 197 | - | 0 | 1 | GAAAAA |
| FAM008 | MYB2AT | 211 | 221 | + | 0 | 1 | GCATTAACTGA |
| FAM304 | OSE2ROOTNODULE | 255 | 259 | - | 0 | 1 | CTCTT |
| FAM162 | LTRE1HVBLT49 | 273 | 278 | + | 0 | 1 | CCGAAA |
| FAM170 | MYBGAHV | 280 | 286 | - | 0 | 1 | TAACAAA |
| FAM241 | TATABOX2 | 290 | 296 | + | 0 | 1 | TATAAAT |
| FAM066 | AMMORESIVDCRNIA1 | 313 | 319 |  | 0 | 1 | CGAACTT |
| FAM266 | MYB1AT | 343 | 348 | + | 0 | 1 | TAACCA |
| FAM010 | WBOXNTCHN48 | 358 | 372 | + | 0 | 1 | GCTGACTCGACCACC |
| FAM026 | RYREPEATLEGUMINBOX | 391 | 401 | + | 0 | 1 | TCCATGCACAT |
| FAM172 | MYCATERD | 396 | 402 | - | 0 | 1 | CATGTGC |

(continued)

| IUPAC Family | IUPAC | Start pos. | End pos. | Strand | Mismat ches | Score | Sequence |
|---|---|---|---|---|---|---|---|
| FAM172 | MYCATRD2 | 397 | 403 | + | 0 | 1 | CACATGT |
| FAM010 | WBOXHVISO1 | 443 | 457 | + | 0 | 1 | CATGACTCTGACAGC |
| FAM322 | BIHD1OS | 451 | 455 | - | 0 | 1 | TGTCA |
| FAM322 | BIHD1OS | 482 | 486 | + | 0 | 1 | TGTCA |
| FAM315 | SORLIP5AT | 489 | 495 | - | 0 | 1 | GAGTGAG |
| FAM026 | RYREPEATBNNAPA | 507 | 517 | + | 0 | 1 | TCCATGCAAGC |
| FAM002 | SORLIP1AT | 522 | 534 | + | 0 | 1 | ACCTCGGCCACGT |
| FAM002 | ABREATCONSENSUS | 526 | 538 | - | 0 | 1 | GGGTACGTGGCCG |
| FAM002 | ABREMOTIFAOSOSEM | 548 | 560 | + | 0 | 1 | CCTTACGTGTCAC |
| FAM322 | BIHD1OS | 555 | 559 | + | 0 | 1 | TGTCA |
| FAM272 | SV40COREEN HAN | 597 | 604 | - | 0 | 1 | GTGGAAAG |
| FAM294 | CTRMCAMV35S | 613 | 621 | + | 0 | 1 | TCTCTCTCT |
| FAM294 | CTRMCAMV35S | 615 | 623 | + | 0 | 1 | TCTCTCTCT |

4) p-MAWS57

PLACE analysis results of p-MAWS57 are listed in Table 52. No TATA box motifs are found in this promoter. Four CAAT box motifs are located at nucleotide position 217-221, 423-427, 501-505 of the forward strand and 340-344 of the reverse strand, respectively.

Table 52. PLACE analysis results of the 1950bp promoter p-MAWS57

| IUPAC Family | IUPAC | Start pos. | End pos. | Strand | Mismatc hes | Score | Sequence |
|---|---|---|---|---|---|---|---|
| FAM307 | ANAERO3CONSENSUS | 17 | 23 | - | 0 | 1 | TCATCAC |
| FAM266 | MYB1AT | 46 | 51 | - | 0 | 1 | AAACCA |
| FAM304 | OSE2ROOTNODULE | 118 | 122 | - | 0 | 1 | CTCTT |
| FAM069 | ARFAT | 120 | 126 | - | 0 | 1 | ATGTCTC |
| FAM069 | SURECOREATSULTR11 | 120 | 126 | + | 0 | 1 | GAGACAT |
| FAM010 | WBOXATNPR1 | 159 | 173 | + | 0 | 1 | GTTGACTGGTTGTCT |
| FAM100 | CCAATBOX1 | 217 | 221 | + | 0 | 1 | CCAAT |
| FAM172 | MYCATRD2 | 230 | 236 | - | 0 | 1 | CACATGT |
| FAM172 | MYCATERD | 231 | 237 | + | 0 | 1 | CATGTGT |
| FAM263 | DPBFCOREDCDC3 | 231 | 237 | - | 0 | 1 | ACACATG |
| FAM026 | RYREPEATGMGY2 | 242 | 252 | + | 0 | 1 | AACATGCATTT |
| FAM026 | RYREPEATBNNAPA | 318 | 328 | - | 0 | 1 | AACATGCAAAT |
| FAM270 | RAV1AAT | 325 | 329 | - | 0 | 1 | CAACA |
| FAM273 | TATCCAOSAMY | 327 | 333 | - | 0 | 1 | TATCCAA |
| FAM014 | MYBST1 | 328 | 334 | + | 0 | 1 | TGGATAT |
| FAM100 | CCAATBOX1 | 340 | 344 | - | 0 | 1 | CCAAT |
| FAM304 | OSE2ROOTNODULE | 374 | 378 | - | 0 | 1 | CTCTT |
| FAM302 | SITEIIATCYTC | 415 | 425 | - | 0 | 1 | TGGGCTCTTTC |
| FAM304 | OSE2ROOTNODULE | 417 | 421 | - | 0 | 1 | CTCTT |
| FAM100 | CCAATBOX1 | 423 | 427 | + | 0 | 1 | CCAAT |
| FAM002 | SORLIP1AT | 448 | 460 | - | 0 | 1 | CACCCTGCCACCC |
| FAM304 | OSE2ROOTNODULE | 468 | 472 | + | 0 | 1 | CTCTT |
| FAM100 | CCAATBOX1 | 501 | 505 | + | 0 | 1 | CCAAT |
| FAM228 | SEF3MOTIFGM | 514 | 519 | + | 0 | 1 | AACCCA |
| FAM002 | RAV1BAT | 525 | 537 | - | 0 | 1 | TATCACCTGTGAA |

(continued)

| IUPAC Family | IUPAC | Start pos. | End pos. | Strand | Mismatc hes | Score | Sequence |
|---|---|---|---|---|---|---|---|
| FAM304 | OSE2ROOTNODULE | 620 | 624 | - | 0 | 1 | CTCTT |
| FAM292 | PREATPRODH | 637 | 642 | - | 0 | 1 | ACTCAT |
| FAM173 | NAPINMOTIFBN | 642 | 648 | + | 0 | 1 | TACACAT |
| FAM292 | PREATPRODH | 682 | 687 | - | 0 | 1 | ACTCAT |
| FAM260 | CAREOSREP1 | 684 | 689 | - | 0 | 1 | CAACTC |
| FAM263 | DPBFCOREDCDC3 | 691 | 697 | + | 0 | 1 | ACACAGG |
| FAM270 | RAV1AAT | 701 | 705 | + | 0 | 1 | CAACA |
| FAM273 | TATCCAOSAMY | 724 | 730 | - | 0 | 1 | TATCCAG |
| FAM014 | MYBST1 | 725 | 731 | + | 0 | 1 | TGGATAC |
| FAM304 | OSE2ROOTNODULE | 752 | 756 | - | 0 | 1 | CTCTT |
| FAM263 | DPBFCOREDCDC3 | 768 | 774 | - | 0 | 1 | ACACTGG |
| FAM010 | WBOXATNPR1 | 769 | 783 | - | 0 | 1 | CTTGACACCACACTG |
| FAM322 | BIHD1OS | 777 | 781 | + | 0 | 1 | TGTCA |
| FAM295 | P1BS | 790 | 797 | + | 0 | 1 | GTATATGC |
| FAM295 | P1BS | 790 | 797 | - | 0 | 1 | GCATATAC |
| FAM304 | OSE2ROOTNODULE | 799 | 803 | - | 0 | 1 | CTCTT |
| FAM010 | WBOXHVISO1 | 802 | 816 | - | 0 | 1 | GATGACTTGTATTCT |
| FAM027 | -10PEHVPSBD | 802 | 807 | - | 0 | 1 | TATTCT |
| FAM026 | RYREPEATGMGY2 | 866 | 876 | + | 0 | 1 | TTCATGCATAT |
| FAM263 | DPBFCOREDCDC3 | 890 | 896 | - | 0 | 1 | ACACTTG |
| FAM202 | -300ELEMENT | 899 | 907 | - | 0 | 1 | TGAAAAGGT |
| FAM205 | PYRIMIDINEBOXOSRAM | 900 | 905 | + | 0 | 1 | CCTTTT |
| FAM267 | TAAAGSTKST1 | 909 | 915 | + | 0 | 1 | TTTAAAG |
| FAM008 | MYB2AT | 916 | 926 | + | 0 | 1 | GCTGTAACTGT |
| FAM270 | RAV1AAT | 968 | 972 | - | 0 | 1 | CAACA |

(continued)

| IUPAC Family | IUPAC | Start pos. | End pos. | Strand | Mismatc hes | Score | Sequence |
|---|---|---|---|---|---|---|---|
| FAM295 | P1BS | 977 | 984 | + | 0 | 1 | GCATATAC |
| FAM295 | P1BS | 977 | 984 | - | 0 | 1 | GTATATGC |
| FAM310 | CPBCSPOR | 987 | 992 | - | 0 | 1 | TATTAG |
| FAM010 | WBOXATNPR1 | 988 | 1002 | - | 0 | 1 | TTTGACATTTTATTA |
| FAM322 | BIHD1OS | 996 | 1000 | + | 0 | 1 | TGTCA |
| FAM012 | IBOXCORE | 1035 | 1041 | + | 0 | 1 | GATAATT |
| FAM170 | MYBGAHV | 1064 | 1070 | - | 0 | 1 | TAACAAA |
| FAM290 | GT1GMSCAM4 | 1074 | 1079 | + | 0 | 1 | GAAAAA |
| FAM245 | TBOXATGAPB | 1086 | 1091 | + | 0 | 1 | ACTTTG |
| FAM321 | WRECSAA01 | 1105 | 1114 | - | 0 | 1 | AAAGTATCGA |
| FAM245 | TBOXATGAPB | 1110 | 1115 | + | 0 | 1 | ACTTTG |
| FAM010 | ELRECOREPCRP1 | 1121 | 1135 | + | 0 | 1 | ATTGACCCGTTACCA |
| FAM325 | MYBCOREATCYCB1 | 1127 | 1131 | - | 0 | 1 | AACGG |
| FAM008 | MYB2AT | 1133 | 1143 | - | 0 | 1 | GTGGTAACTGG |
| FAM010 | WBOXNTCHN48 | 1173 | 1187 | + | 0 | 1 | TCTGACTTGAAGAAG |
| FAM002 | RAV1BAT | 1205 | 1217 | + | 0 | 1 | GTCCACCTGAACG |
| FAM325 | MYBCOREATCYCB1 | 1214 | 1218 | + | 0 | 1 | AACGG |
| FAM069 | ARFAT | 1218 | 1224 | - | 0 | 1 | CTGTCTC |
| FAM069 | SURECOREATSULTR11 | 1218 | 1224 | + | 0 | 1 | GAGACAG |
| FAM271 | SEBFCONSSTPR10A | 1218 | 1224 | - | 0 | 1 | CTGTCTC |
| FAM002 | SORLIP1AT | 1231 | 1243 | - | 0 | 1 | CTCTCCGCCACAA |
| FAM002 | RAV1BAT | 1244 | 1256 | + | 0 | 1 | CTCCACCTGAACG |
| FAM171 | MYBPZM | 1283 | 1289 | - | 0 | 1 | GCCAACC |
| FAM002 | SORLIP1AT | 1289 | 1301 | - | 0 | 1 | CAGCTCGCCACGG |
| FAM002 | SORLIP1AT | 1296 | 1308 | + | 0 | 1 | GAGCTGGCCACCT |

(continued)

| IUPAC Family | IUPAC | Start pos. | End pos. | Strand | Mismatc hes | Score | Sequence |
|---|---|---|---|---|---|---|---|
| FAM069 | SURECOREATSULTR11 | 1311 | 1317 | - | 0 | 1 | GAGACTA |
| FAM324 | CGCGBOXAT | 1348 | 1353 | + | 0 | 1 | GCGCGG |
| FAM324 | CGCGBOXAT | 1348 | 1353 | - | 0 | 1 | CCGCGC |
| FAM263 | DPBFCOREDCDC3 | 1356 | 1362 | - | 0 | 1 | ACACTGG |
| FAM147 | HEXAMERATH4 | 1365 | 1370 | + | 0 | 1 | CCGTCG |
| FAM107 | CGACGOSAMY3 | 1366 | 1370 | - | 0 | 1 | CGACG |
| FAM228 | SEF3MOTIFGM | 1373 | 1378 | - | 0 | 1 | AACCCA |
| FAM061 | GCCCORE | 1387 | 1393 | + | 0 | 1 | GGCCGCC |
| FAM061 | GCCCORE | 1390 | 1396 | + | 0 | 1 | CGCCGCC |
| FAM209 | RBCSCONSENSUS | 1408 | 1414 | + | 0 | 1 | AATCCAA |
| FAM325 | MYBCOREATCYCB1 | 1414 | 1418 | + | 0 | 1 | AACGG |
| FAM302 | SITEIIATCYTC | 1455 | 1465 | + | 0 | 1 | TGGGCCTTATC |
| FAM012 | IBOXCORENT | 1459 | 1465 | - | 0 | 1 | GATAAGG |
| FAM002 | SORLIP1AT | 1463 | 1475 | + | 0 | 1 | ATCTAGGCCACAA |
| FAM059 | ACGTTBOX | 1474 | 1479 | + | 0 | 1 | AACGTT |
| FAM059 | ACGTTBOX | 1474 | 1479 | - | 0 | 1 | AACGTT |
| FAM010 | WBOXHVISO1 | 1480 | 1494 | + | 0 | 1 | TGTGACTCTGTGAGC |
| FAM302 | SITEIIATCYTC | 1500 | 1510 | - | 0 | 1 | TGGGCCCAAAC |
| FAM302 | SITEIIATCYTC | 1503 | 1513 | + | 0 | 1 | TGGGCCCATCT |
| FAM304 | OSE2ROOTNODULE | 1512 | 1516 | + | 0 | 1 | CTCTT |
| FAM012 | IBOXCORE | 1536 | 1542 | - | 0 | 1 | GATAAAA |
| FAM302 | SITEIIATCYTC | 1542 | 1552 | - | 0 | 1 | TGGGCTTGATG |
| FAM266 | MYB1AT | 1560 | 1565 | + | 0 | 1 | AAACCA |
| FAM171 | MYBPZM | 1569 | 1575 | + | 0 | 1 | TCCTACC |
| FAM305 | ANAERO1CONSENSUS | 1603 | 1609 | + | 0 | 1 | AAACAAA |

(continued)

| IUPAC Family | IUPAC | Start pos. | End pos. | Strand | Mismatc hes | Score | Sequence |
|---|---|---|---|---|---|---|---|
| FAM245 | TBOXATGAPB | 1606 | 1611 | - | 0 | 1 | ACTTTG |
| FAM172 | MYCATERD | 1628 | 1634 | - | 0 | 1 | CATGTGA |
| FAM172 | MYCATRD2 | 1629 | 1635 | + | 0 | 1 | CACATGC |
| FAM324 | CGCGBOXAT | 1634 | 1639 | + | 0 | 1 | GCGCGT |
| FAM324 | CGCGBOXAT | 1634 | 1639 | - | 0 | 1 | ACGCGC |
| FAM261 | CDTDREHVCBF2 | 1641 | 1646 | + | 0 | 1 | GTCGAC |
| FAM261 | CDTDREHVCBF2 | 1641 | 1646 | - | 0 | 1 | GTCGAC |
| FAM304 | OSE2ROOTNODULE | 1692 | 1696 | - | 0 | 1 | CTCTT |
| FAM276 | TRANSINITDICOTS | 1696 | 1703 | - | 0 | 1 | AACATGGC |
| FAM304 | OSE2ROOTNODULE | 1734 | 1738 | - | 0 | 1 | CTCTT |
| FAM324 | CGCGBOXAT | 1738 | 1743 | + | 0 | 1 | GCGCGT |
| FAM324 | CGCGBOXAT | 1738 | 1743 | - | 0 | 1 | ACGCGC |
| FAM061 | GCCCORE | 1765 | 1771 | + | 0 | 1 | GGCCGCC |
| FAM306 | ANAERO2CONSENSUS | 1772 | 1777 | + | 0 | 1 | AGCAGC |
| FAM324 | CGCGBOXAT | 1780 | 1785 | + | 0 | 1 | CCGCGG |
| FAM324 | CGCGBOXAT | 1780 | 1785 | - | 0 | 1 | CCGCGG |
| FAM002 | ABRELATERD | 1793 | 1805 | - | 0 | 1 | ACGGACGTGCTGC |
| FAM325 | MYBCOREATCYCB1 | 1802 | 1806 | - | 0 | 1 | AACGG |
| FAM302 | SORLIP2AT | 1806 | 1816 | - | 0 | 1 | CGGGCCGACCA |
| FAM013 | DRECRTCOREAT | 1807 | 1813 | - | 0 | 1 | GCCGACC |
| FAM002 | ABRELATERD | 1828 | 1840 | - | 0 | 1 | CGCGACGTGTGCC |
| FAM107 | CGACGOSAMY3 | 1834 | 1838 | - | 0 | 1 | CGACG |
| FAM026 | RYREPEATLEGUMINBOX | 1839 | 1849 | + | 0 | 1 | CGCATGCACGC |
| FAM324 | CGCGBOXAT | 1846 | 1851 | + | 0 | 1 | ACGCGC |
| FAM324 | CGCGBOXAT | 1846 | 1851 | - | 0 | 1 | GCGCGT |

(continued)

| IUPAC Family | IUPAC | Start pos. | End pos. | Strand | Mismatc hes | Score | Sequence |
|---|---|---|---|---|---|---|---|
| FAM324 | CGCGBOXAT | 1848 | 1853 | + | 0 | 1 | GCGCGG |
| FAM324 | CGCGBOXAT | 1848 | 1853 | - | 0 | 1 | CCGCGC |
| FAM002 | GADOWNAT | 1858 | 1870 | - | 0 | 1 | CGGCACGTGTCCG |
| FAM002 | CACGTGMOTIF | 1859 | 1871 | + | 0 | 1 | GGACACGTGCCGG |
| FAM263 | DPBFCOREDCDC3 | 1861 | 1867 | + | 0 | 1 | ACACGTG |
| FAM324 | CGCGBOXAT | 1871 | 1876 | + | 0 | 1 | GCGCGG |
| FAM324 | CGCGBOXAT | 1871 | 1876 | - | 0 | 1 | CCGCGC |
| FAM002 | ABREOSRAB21 | 1874 | 1886 | - | 0 | 1 | AGGGACGTGCCCG |
| FAM324 | CGCGBOXAT | 1889 | 1894 | + | 0 | 1 | CCGCGC |
| FAM324 | CGCGBOXAT | 1889 | 1894 | - | 0 | 1 | GCGCGG |
| FAM002 | SORLIP1AT | 1918 | 1930 | + | 0 | 1 | CCAGCAGCCACAA |
| FAM306 | ANAERO2CONSENSUS | 1920 | 1925 | + | 0 | 1 | AGCAGC |
| FAM270 | RAV1AAT | 1928 | 1932 | + | 0 | 1 | CAACA |

5) p-MAWS60

PLACE analysis results of p-MAWS60 are listed in Table 53. One TATA box motif is found at nucleotide position 156-162 of the forward strand. One CAAT box motif is located at nucleotide position 547-551 of the reverse strand.

Table 53. PLACE analysis results of the 1106bp promoter p-MAWS60

| IUPAC Family | IUPAC | Start pos. | End pos. | Strand | Mismatches | Score | Sequence |
|---|---|---|---|---|---|---|---|
| FAM266 | MYB1AT | 2 | 7 | - | 0 | 1 | AAACCA |
| FAM012 | IBOXCORE | 11 | 17 | + | 0 | 1 | GATAATT |
| FAM305 | ANAERO1CONSENSUS | 16 | 22 | - | 0 | 1 | AAACAAA |
| FAM012 | IBOXCORE | 19 | 25 | - | 0 | 1 | GATAAAC |
| FAM172 | MYCATERD | 26 | 32 | - | 0 | 1 | CATGTGA |
| FAM 172 | MYCATRD2 | 27 | 33 | + | 0 | 1 | CACATGA |
| FAM290 | GT1GMSCAM4 | 77 | 82 | - | 0 | 1 | GAAAAA |
| FAM303 | OSE1ROOTNODULE | 95 | 101 | + | 0 | 1 | AAAGATA |
| FAM322 | BIHD1OS | 132 | 136 | + | 0 | 1 | TGTCA |
| FAM010 | ELRECOREPCRP1 | 140 | 154 | - | 0 | 1 | TTTGACCATTTCATT |
| FAM241 | TATABOX2 | 156 | 162 | + | 0 | 1 | TATAAAT |
| FAM292 | PREATPRODH | 174 | 179 | - | 0 | 1 | ACTCAT |
| FAM303 | OSE1ROOTNODULE | 200 | 206 | - | 0 | 1 | AAAGATT |
| FAM267 | TAAAGSTKST1 | 227 | 233 | - | 0 | 1 | TTTAAAG |
| FAM263 | DPBFCOREDCDC3 | 257 | 263 | - | 0 | 1 | ACACTAG |
| FAM270 | RAV1AAT | 261 | 265 | - | 0 | 1 | CAACA |
| FAM290 | GT1GMSCAM4 | 265 | 270 | + | 0 | 1 | GAAAAA |
| FAM311 | EECCRCAH1 | 285 | 291 | - | 0 | 1 | GAGTTTC |
| FAM304 | OSE2ROOTNODULE | 289 | 293 | + | 0 | 1 | CTCTT |
| FAM263 | DPBFCOREDCDC3 | 297 | 303 | - | 0 | 1 | ACACTCG |
| FAM263 | DPBFCOREDCDC3 | 311 | 317 | + | 0 | 1 | ACACTCG |
| FAM304 | OSE2ROOTNODULE | 327 | 331 | + | 0 | 1 | CTCTT |
| FAM010 | WBOXATNPR1 | 331 | 345 | - | 0 | 1 | TTTGACACTCGGCAA |
| FAM263 | DPBFCOREDCDC3 | 335 | 341 | - | 0 | 1 | ACACTCG |
| FAM322 | BIHD1OS | 339 | 343 | + | 0 | 1 | TGTCA |

(continued)

| IUPAC Family | IUPAC | Start pos. | End pos. | Strand | Mismatches | Score | Sequence |
|---|---|---|---|---|---|---|---|
| FAM300 | LECPLEACS2 | 348 | 355 | + | 0 | 1 | TAAAATAT |
| FAM267 | TAAAGSTKST1 | 400 | 406 | + | 0 | 1 | GGTAAAG |
| FAM263 | DPBFCOREDCDC3 | 418 | 424 | - | 0 | 1 | ACACTCG |
| FAM162 | LTRE1HVBLT49 | 425 | 430 | + | 0 | 1 | CCGAAA |
| FAM290 | GT1GMSCAM4 | 427 | 432 | + | 0 | 1 | GAAAAA |
| FAM012 | IBOXCORE | 439 | 445 | + | 0 | 1 | GATAAAA |
| FAM300 | LECPLEACS2 | 441 | 448 | + | 0 | 1 | TAAAATAT |
| FAM311 | EECCRCAH1 | 469 | 475 | + | 0 | 1 | GAATTCC |
| FAM010 | WBOXNTCHN48 | 477 | 491 | - | 0 | 1 | TCTGACTCACGCTAC |
| FAM022 | GCN4OSGLUB1 | 482 | 490 | + | 0 | 1 | GTGAGTCAG |
| FAM124 | ERELEE4 | 509 | 516 | - | 0 | 1 | ATTTCAAA |
| FAM262 | CIACADIANLELHC | 520 | 529 | + | 0 | 1 | CAAACAAATC |
| FAM305 | ANAERO1CONSENSUS | 521 | 527 | + | 0 | 1 | AAACAAA |
| FAM098 | CATATGGMSAUR | 540 | 545 | + | 0 | 1 | CATATG |
| FAM098 | CATATGGMSAUR | 540 | 545 | - | 0 | 1 | CATATG |
| FAM100 | CCAATBOX1 | 547 | 551 | - | 0 | 1 | CCAAT |
| FAM002 | SORLIP1AT | 550 | 562 | + | 0 | 1 | GGCTTTGCCACAT |
| FAM172 | MYCATERD | 557 | 563 | - | 0 | 1 | CATGTGG |
| FAM172 | MYCATRD2 | 558 | 564 | + | 0 | 1 | CACATGG |
| FAM221 | S1FBOXSORPS1L21 | 561 | 566 | + | 0 | 1 | ATGGTA |
| FAM263 | DPBFCOREDCDC3 | 578 | 584 | - | 0 | 1 | ACACTCG |
| FAM281 | MYB1LEPR | 603 | 609 | - | 0 | 1 | GTTAGTT |
| FAM228 | SEF3MOTIFGM | 607 | 612 | + | 0 | 1 | AACCCA |
| FAM266 | MYB1AT | 613 | 618 | - | 0 | 1 | AAACCA |
| FAM170 | AMYBOX1 | 622 | 628 | + | 0 | 1 | TAACAGA |

(continued)

| IUPAC Family | IUPAC | Start pos. | End pos. | Strand | Mismatches | Score | Sequence |
|---|---|---|---|---|---|---|---|
| FAM263 | DPBFCOREDCDC3 | 633 | 639 | + | 0 | 1 | ACACCAG |
| FAM010 | WBOXHVISO1 | 638 | 652 | + | 0 | 1 | AGTGACTCCATCGTT |
| FAM003 | MYB26PS | 739 | 749 | - | 0 | 1 | TGTTAGGTTGA |
| FAM003 | MYBPLANT | 741 | 751 | + | 0 | 1 | AACCTAACACA |
| FAM024 | CANBNNAPA | 744 | 752 | + | 0 | 1 | CTAACACAG |
| FAM026 | RYREPEATVFLEB4 | 762 | 772 | + | 0 | 1 | TACATGCATGC |
| FAM026 | RYREPEATVFLEB4 | 763 | 773 | - | 0 | 1 | CGCATGCATGT |
| FAM304 | OSE2ROOTNODULE | 789 | 793 | - | 0 | 1 | CTCTT |
| FAM002 | RAV1BAT | 794 | 806 | - | 0 | 1 | CATCACCTGCCTC |
| FAM307 | ANAERO3CONSENSUS | 801 | 807 | - | 0 | 1 | TCATCAC |
| FAM069 | SURECOREATSULTR11 | 811 | 817 | - | 0 | 1 | GAGACCT |
| FAM263 | DPBFCOREDCDC3 | 827 | 833 | - | 0 | 1 | ACACCAG |
| FAM002 | SORLIP1AT | 831 | 843 | + | 0 | 1 | TGTGCAGCCACGT |
| FAM002 | ABREATCONSENSUS | 835 | 847 | - | 0 | 1 | GGGTACGTGGCTG |
| FAM324 | CGCGBOXAT | 852 | 857 | + | 0 | 1 | ACGCGT |
| FAM324 | CGCGBOXAT | 852 | 857 | - | 0 | 1 | ACGCGT |
| FAM107 | CGACGOSAMY3 | 855 | 859 | - | 0 | 1 | CGACG |
| FAM324 | CGCGBOXAT | 860 | 865 | + | 0 | 1 | CCGCGG |
| FAM324 | CGCGBOXAT | 860 | 865 | - | 0 | 1 | CCGCGG |
| FAM107 | CGACGOSAMY3 | 867 | 871 | + | 0 | 1 | CGACG |
| FAM002 | RAV1BAT | 869 | 881 | - | 0 | 1 | CAACACCTGTCGT |
| FAM263 | DPBFCOREDCDC3 | 873 | 879 | - | 0 | 1 | ACACCTG |
| FAM024 | CANBNNAPA | 874 | 882 | - | 0 | 1 | CCAACACCT |
| FAM270 | RAV1AAT | 877 | 881 | - | 0 | 1 | CAACA |
| FAM171 | MYBPZM | 882 | 888 | + | 0 | 1 | GCCAACC |

(continued)

| IUPAC Family | IUPAC | Start pos. | End pos. | Strand | Mismatches | Score | Sequence |
|---|---|---|---|---|---|---|---|
| FAM322 | BIHD1OS | 912 | 916 | - | 0 | 1 | TGTCA |
| FAM015 | ACGTABOX | 932 | 937 | + | 0 | 1 | TACGTA |
| FAM015 | ACGTABOX | 932 | 937 | - | 0 | 1 | TACGTA |
| FAM069 | SURECOREATSULTR11 | 941 | 947 | + | 0 | 1 | GAGACGA |
| FAM024 | CANBNNAPA | 945 | 953 | + | 0 | 1 | CGAACACGA |
| FAM194 | PALBOXAPC | 963 | 969 | + | 0 | 1 | CCGTCCT |
| FAM002 | ASF1MOTIFCAMV | 978 | 990 | - | 0 | 1 | GAGCATGACGGGC |
| FAM026 | RYREPEATVFLEB4 | 988 | 998 | + | 0 | 1 | CTCATGCATGC |
| FAM026 | RYREPEATVFLEB4 | 989 | 999 | - | 0 | 1 | TGCATGCATGA |
| FAM026 | RYREPEATVFLEB4 | 992 | 1002 | + | 0 | 1 | TGCATGCATGC |
| FAM026 | RYREPEATVFLEB4 | 993 | 1003 | - | 0 | 1 | TGCATGCATGC |
| FAM026 | RYREPEATVFLEB4 | 996 | 1006 | + | 0 | 1 | TGCATGCATGC |
| FAM026 | RYREPEATVFLEB4 | 997 | 1007 | - | 0 | 1 | AGCATGCATGC |
| FAM026 | RYREPEATGMGY2 | 1009 | 1019 | + | 0 | 1 | ATCATGCATAC |
| FAM012 | IBOXCORE | 1022 | 1028 | + | 0 | 1 | GATAAAT |
| FAM015 | ACGTABOX | 1048 | 1053 | + | 0 | 1 | TACGTA |
| FAM015 | ACGTABOX | 1048 | 1053 | - | 0 | 1 | TACGTA |
| FAM151 | INTRONLOWER | 1092 | 1097 | + | 0 | 1 | TGCAGG |

6) p-MAWS63

PLACE analysis results of p-MAWS63 are listed in Table 54. Three TATA box motifs are found at nucleotide position 1555-1561, 1577-1583 and 1628-1634 of the forward strand, respectively. One CAAT box motif is located at nucleotide position 987-991 of the forward strand, and three CAAT box motifs are located at nucleotide position 156-160, 199-203, 249-253 of the reverse strand.

Table 54. PLACE analysis results of the 1941 bp promoter p-MAWS63

| IUPAC Family | IUPAC | Start pos. | End pos. | Strand | Mismatches | Score | Sequence |
|---|---|---|---|---|---|---|---|
| FAM002 | SORLIP1AT | 8 | 20 | - | 0 | 1 | GTGTAGGCCACGA |
| FAM263 | DPBFCOREDCDC3 | 17 | 23 | + | 0 | 1 | ACACACG |
| FAM263 | DPBFCOREDCDC3 | 19 | 25 | + | 0 | 1 | ACACGCG |
| FAM324 | CGCGBOXAT | 21 | 26 | + | 0 | 1 | ACGCGC |
| FAM324 | CGCGBOXAT | 21 | 26 | - | 0 | 1 | GCGCGT |
| FAM008 | MYB2AT | 34 | 44 | - | 0 | 1 | ATGGTAACTGA |
| FAM221 | S1FBOXSORPS1L21 | 39 | 44 | - | 0 | 1 | ATGGTA |
| FAM173 | NAPINMOTIFBN | 73 | 79 | + | 0 | 1 | TACACAT |
| FAM172 | MYCATERD | 74 | 80 | - | 0 | 1 | CATGTGT |
| FAM263 | DPBFCOREDCDC3 | 74 | 80 | + | 0 | 1 | ACACATG |
| FAM172 | MYCATRD2 | 75 | 81 | + | 0 | 1 | CACATGA |
| FAM124 | ERELEE4 | 92 | 99 | - | 0 | 1 | AATTCAAA |
| FAM311 | EECCRCAH1 | 95 | 101 | + | 0 | 1 | GAATTTC |
| FAM026 | RYREPEATBNNAPA | 105 | 115 | + | 0 | 1 | AGCATGCAAAA |
| FAM202 | -300ELEMENT | 109 | 117 | + | 0 | 1 | TGCAAAATT |
| FAM012 | IBOXCORE | 136 | 142 | + | 0 | 1 | GATAAAA |
| FAM325 | MYBCOREATCYCB1 | 142 | 146 | + | 0 | 1 | AACGG |
| FAM002 | SORLIP1AT | 145 | 157 | - | 0 | 1 | ATTTTGGCCACCC |
| FAM100 | CCAATBOX1 | 156 | 160 | - | 0 | 1 | CCAAT |
| FAM061 | GCCCORE | 161 | 167 | - | 0 | 1 | TGCCGCC |
| FAM012 | IBOX | 177 | 183 | + | 0 | 1 | GATAAGC |
| FAM014 | MYBST1 | 184 | 190 | - | 0 | 1 | TGGATAG |
| FAM025 | AMYBOX2 | 185 | 191 | + | 0 | 1 | TATCCAT |
| FAM273 | TATCCAOSAMY | 185 | 191 | + | 0 | 1 | TATCCAT |
| FAM100 | CCAATBOX1 | 199 | 203 | - | 0 | 1 | CCAAT |
| FAM322 | BIHD1OS | 207 | 211 | + | 0 | 1 | TGTCA |
| FAM100 | CCAATBOX1 | 249 | 253 | - | 0 | 1 | CCAAT |
| FAM014 | MYBST1 | 258 | 264 | - | 0 | 1 | TGGATAG |
| FAM273 | TATCCAOSAMY | 259 | 265 | + | 0 | 1 | TATCCAG |
| FAM306 | ANAERO2CONSENSUS | 264 | 269 | + | 0 | 1 | AGCAGC |
| FAM008 | MYB2AT | 304 | 314 | + | 0 | 1 | TCCCTAACTGC |
| FAM002 | SORLIP1AT | 316 | 328 | + | 0 | 1 | CCGGCCGCCACAC |

(continued)

| IUPAC Family | IUPAC | Start pos. | End pos. | Strand | Mismat ches | Score | Sequence |
|---|---|---|---|---|---|---|---|
| FAM061 | GCCCORE | 318 | 324 | + | 0 | 1 | GGCCGCC |
| FAM013 | LTRECOREATCOR15 | 337 | 343 | + | 0 | 1 | CCCGACC |
| FAM270 | RAV1AAT | 350 | 354 | + | 0 | 1 | CAACA |
| FAM002 | SORLIP1AT | 352 | 364 | + | 0 | 1 | ACAATGGCCACCG |
| FAM276 | TRANSINITDICOTS | 352 | 359 | + | 0 | 1 | ACAATGGC |
| FAM194 | PALBOXAPC | 362 | 368 | + | 0 | 1 | CCGTCCT |
| FAM324 | CGCGBOXAT | 378 | 383 | + | 0 | 1 | CCGCGC |
| FAM324 | CGCGBOXAT | 378 | 383 | - | 0 | 1 | GCGCGG |
| FAM324 | CGCGBOXAT | 380 | 385 | + | 0 | 1 | GCGCGC |
| FAM324 | CGCGBOXAT | 380 | 385 | - | 0 | 1 | GCGCGC |
| FAM194 | PALBOXAPC | 403 | 409 | + | 0 | 1 | CCGTCCT |
| FAM324 | CGCGBOXAT | 419 | 424 | + | 0 | 1 | CCGCGC |
| FAM324 | CGCGBOXAT | 419 | 424 | - | 0 | 1 | GCGCGG |
| FAM002 | SORLIP1AT | 437 | 449 | - | 0 | 1 | GGCAGCGCCACGG |
| FAM302 | SITEIIATCYTC | 470 | 480 | + | 0 | 1 | TGGGCCGTAGC |
| FAM306 | ANAERO2CONSENSUS | 484 | 489 | + | 0 | 1 | AGCAGC |
| FAM324 | CGCGBOXAT | 501 | 506 | + | 0 | 1 | GCGCGC |
| FAM324 | CGCGBOXAT | 501 | 506 | - | 0 | 1 | GCGCGC |
| FAM324 | CGCGBOXAT | 503 | 508 | + | 0 | 1 | GCGCGC |
| FAM324 | CGCGBOXAT | 503 | 508 | - | 0 | 1 | GCGCGC |
| FAM002 | SORLIP1AT | 505 | 517 | + | 0 | 1 | GCGCAGGCCACCT |
| FAM002 | BP5OSWX | 517 | 529 | + | 0 | 1 | TACAACGTGAAGC |
| FAM002 | RAV1BAT | 555 | 567 | - | 0 | 1 | GGGCACCTGCAGC |
| FAM151 | INTRONLOWER | 557 | 562 | + | 0 | 1 | TGCAGG |
| FAM013 | LTRECOREATCOR15 | 565 | 571 | + | 0 | 1 | CCCGACG |
| FAM107 | CGACGOSAMY3 | 567 | 571 | + | 0 | 1 | CGACG |
| FAM002 | ABRELATERD | 568 | 580 | + | 0 | 1 | GACGACGTGTACA |
| FAM107 | CGACGOSAMY3 | 570 | 574 | + | 0 | 1 | CGACG |
| FAM194 | PALBOXAPC | 599 | 605 | - | 0 | 1 | CCGTCCT |
| FAM324 | CGCGBOXAT | 627 | 632 | + | 0 | 1 | CCGCGC |
| FAM324 | CGCGBOXAT | 627 | 632 | - | 0 | 1 | GCGCGG |
| FAM324 | CGCGBOXAT | 629 | 634 | + | 0 | 1 | GCGCGG |
| FAM324 | CGCGBOXAT | 629 | 634 | - | 0 | 1 | CCGCGC |
| FAM069 | SURECOREATSULTR11 | 662 | 668 | - | 0 | 1 | GAGACGA |
| FAM013 | LTRECOREATCOR15 | 685 | 691 | + | 0 | 1 | TCCGACC |
| FAM107 | CGACGOSAMY3 | 702 | 706 | + | 0 | 1 | CGACG |
| FAM107 | CGACGOSAMY3 | 705 | 709 | + | 0 | 1 | CGACG |

(continued)

| IUPAC Family | IUPAC | Start pos. | End pos. | Strand | Mismatches | Score | Sequence |
|---|---|---|---|---|---|---|---|
| FAM147 | HEXAMERATH4 | 705 | 710 | - | 0 | 1 | CCGTCG |
| FAM061 | GCCCORE | 717 | 723 | + | 0 | 1 | CGCCGCC |
| FAM002 | RAV1BAT | 732 | 744 | + | 0 | 1 | GCTCACCTGCCAC |
| FAM002 | SORLIP1AT | 734 | 746 | + | 0 | 1 | TCACCTGCCACGC |
| FAM171 | MYBPZM | 745 | 751 | + | 0 | 1 | GCCTACC |
| FAM002 | TGACGTVMAMY | 754 | 766 | + | 0 | 1 | ACCTCTGACGTCG |
| FAM002 | HEXMOTIFTAH3H4 | 756 | 768 | - | 0 | 1 | GACGACGTCAGAG |
| FAM057 | ACGTCBOX | 760 | 765 | + | 0 | 1 | GACGTC |
| FAM057 | ACGTCBOX | 760 | 765 | - | 0 | 1 | GACGTC |
| FAM002 | ASF1MOTIFCAMV | 762 | 774 | - | 0 | 1 | CTCGATGACGACG |
| FAM107 | CGACGOSAMY3 | 762 | 766 | - | 0 | 1 | CGACG |
| FAM069 | SURECOREATSULTR11 | 772 | 778 | + | 0 | 1 | GAGACGC |
| FAM107 | CGACGOSAMY3 | 842 | 846 | - | 0 | 1 | CGACG |
| FAM324 | CGCGBOXAT | 864 | 869 | + | 0 | 1 | ACGCGC |
| FAM324 | CGCGBOXAT | 864 | 869 | - | 0 | 1 | GCGCGT |
| FAM324 | CGCGBOXAT | 866 | 871 | + | 0 | 1 | GCGCGT |
| FAM324 | CGCGBOXAT | 866 | 871 | - | 0 | 1 | ACGCGC |
| FAM002 | ACGTOSGLUB1 | 869 | 881 | - | 0 | 1 | CAGTACGTGTACG |
| FAM305 | ANAERO1CONSENSUS | 884 | 890 | - | 0 | 1 | AAACAAA |
| FAM107 | CGACGOSAMY3 | 914 | 918 | - | 0 | 1 | CGACG |
| FAM260 | CAREOSREP1 | 918 | 923 | - | 0 | 1 | CAACTC |
| FAM311 | EECCRCAH1 | 918 | 924 | + | 0 | 1 | GAGTTGC |
| FAM276 | TRANSINITDICOTS | 928 | 935 | - | 0 | 1 | ACGATGGC |
| FAM069 | SURECOREATSULTR11 | 932 | 938 | - | 0 | 1 | GAGACGA |
| FAM294 | CTRMCAMV35S | 935 | 943 | + | 0 | 1 | TCTCTCTCT |
| FAM294 | CTRMCAMV35S | 937 | 945 | + | 0 | 1 | TCTCTCTCT |
| FAM294 | CTRMCAMV35S | 939 | 947 | + | 0 | 1 | TCTCTCTCT |
| FAM234 | SP8BFIBSP8BIB | 955 | 961 | + | 0 | 1 | TACTATT |
| FAM324 | CGCGBOXAT | 971 | 976 | + | 0 | 1 | GCGCGC |
| FAM324 | CGCGBOXAT | 971 | 976 | - | 0 | 1 | GCGCGC |
| FAM087 | BOXIINTPATPB | 977 | 982 | - | 0 | 1 | ATAGAA |
| FAM290 | GT1GMSCAM4 | 982 | 987 | - | 0 | 1 | GAAAAA |
| FAM100 | CCAATBOX1 | 987 | 991 | + | 0 | 1 | CCAAT |
| FAM289 | LEAFYATAG | 987 | 993 | + | 0 | 1 | CCAATGT |
| FAM270 | RAV1AAT | 991 | 995 | - | 0 | 1 | CAACA |
| FAM311 | EECCRCAH1 | 995 | 1001 | + | 0 | 1 | GAGTTAC |
| FAM002 | ASF1MOTIFCAMV | 1064 | 1076 | + | 0 | 1 | TGTGGTGACGGTT |

(continued)

| IUPAC Family | IUPAC | Start pos. | End pos. | Strand | Mismatches | Score | Sequence |
|---|---|---|---|---|---|---|---|
| FAM003 | MYBPLANT | 1070 | 1080 | - | 0 | 1 | AACCAACCGTC |
| FAM171 | BOXLCOREDCPAL | 1073 | 1079 | - | 0 | 1 | ACCAACC |
| FAM002 | SORLIP1AT | 1086 | 1098 | - | 0 | 1 | GTCGCCGCCACAC |
| FAM061 | GCCCORE | 1090 | 1096 | - | 0 | 1 | CGCCGCC |
| FAM002 | ABREOSRAB21 | 1092 | 1104 | - | 0 | 1 | ACTGACGTCGCCG |
| FAM002 | HEXMOTIFTAH3H4 | 1093 | 1105 | + | 0 | 1 | GGCGACGTCAGTC |
| FAM010 | WBOXHVISO1 | 1094 | 1108 | - | 0 | 1 | CATGACTGACGTCGC |
| FAM002 | TGACGTVMAMY | 1095 | 1107 | - | 0 | 1 | ATGACTGACGTCG |
| FAM107 | CGACGOSAMY3 | 1095 | 1099 | + | 0 | 1 | CGACG |
| FAM057 | ACGTCBOX | 1096 | 1101 | + | 0 | 1 | GACGTC |
| FAM057 | ACGTCBOX | 1096 | 1101 | - | 0 | 1 | GACGTC |
| FAM304 | OSE2ROOTNODULE | 1113 | 1117 | - | 0 | 1 | CTCTT |
| FAM026 | RYREPEATGMGY2 | 1122 | 1132 | - | 0 | 1 | CCCATGCATTC |
| FAM024 | CANBNNAPA | 1132 | 1140 | - | 0 | 1 | CCAACACCC |
| FAM270 | RAV1AAT | 1135 | 1139 | - | 0 | 1 | CAACA |
| FAM324 | CGCGBOXAT | 1177 | 1182 | + | 0 | 1 | GCGCGC |
| FAM324 | CGCGBOXAT | 1177 | 1182 | - | 0 | 1 | GCGCGC |
| FAM147 | HEXAMERATH4 | 1186 | 1191 | + | 0 | 1 | CCGTCG |
| FAM002 | ASF1MOTIFCAMV | 1187 | 1199 | - | 0 | 1 | AGCCATGACGACG |
| FAM107 | CGACGOSAMY3 | 1187 | 1191 | - | 0 | 1 | CGACG |
| FAM107 | CGACGOSAMY3 | 1200 | 1204 | + | 0 | 1 | CGACG |
| FAM324 | CGCGBOXAT | 1226 | 1231 | + | 0 | 1 | CCGCGC |
| FAM324 | CGCGBOXAT | 1226 | 1231 | - | 0 | 1 | GCGCGG |
| FAM324 | CGCGBOXAT | 1228 | 1233 | + | 0 | 1 | GCGCGC |
| FAM324 | CGCGBOXAT | 1228 | 1233 | - | 0 | 1 | GCGCGC |
| FAM015 | ACGTABOX | 1253 | 1258 | + | 0 | 1 | TACGTA |
| FAM015 | ACGTABOX | 1253 | 1258 | - | 0 | 1 | TACGTA |
| FAM002 | SORLIP1AT | 1294 | 1306 | - | 0 | 1 | CTCTTCGCCACCC |
| FAM002 | SORLIP1AT | 1301 | 1313 | + | 0 | 1 | GAAGAGGCCACGG |
| FAM304 | OSE2ROOTNODULE | 1302 | 1306 | - | 0 | 1 | CTCTT |
| FAM302 | SORLIP2AT | 1307 | 1317 | - | 0 | 1 | CGGGCCGTGGC |
| FAM013 | LTRECOREATCOR15 | 1314 | 1320 | + | 0 | 1 | CCCGACC |
| FAM002 | SORLIP1AT | 1339 | 1351 | + | 0 | 1 | CATCTCGCCACCA |
| FAM089 | BS1EGCCR | 1359 | 1364 | - | 0 | 1 | AGCGGG |
| FAM002 | SORLIP1AT | 1366 | 1378 | + | 0 | 1 | GCCGCTGCCACCG |
| FAM270 | RAV1AAT | 1381 | 1385 | - | 0 | 1 | CAACA |

(continued)

| IUPAC Family | IUPAC | Start pos. | End pos. | Strand | Mismatches | Score | Sequence |
|---|---|---|---|---|---|---|---|
| FAM228 | SEF3MOTIFGM | 1384 | 1389 | - | 0 | 1 | AACCCA |
| FAM171 | MYBPZM | 1386 | 1392 | - | 0 | 1 | CCCAACC |
| FAM302 | SITEIIATCYTC | 1389 | 1399 | + | 0 | 1 | TGGGCTGAAGC |
| FAM010 | QELEMENTZMZM13 | 1403 | 1417 | - | 0 | 1 | AAAGGTCACGGGC TT |
| FAM205 | PYRIMIDINEBOXOSRAM | 1413 | 1418 | + | 0 | 1 | CCTTTT |
| FAM290 | GT1GMSCAM4 | 1415 | 1420 | - | 0 | 1 | GAAAAA |
| FAM290 | GT1GMSCAM4 | 1421 | 1426 | - | 0 | 1 | GAAAAA |
| FAM267 | TAAAGSTKST1 | 1426 | 1432 | - | 0 | 1 | AATAAAG |
| FAM027 | -10PEHVPSBD | 1429 | 1434 | + | 0 | 1 | TATTCT |
| FAM205 | PYRIMIDINEBOXOSRAM | 1437 | 1442 | + | 0 | 1 | CCTTTT |
| FAM270 | RAV1AAT | 1466 | 1470 | - | 0 | 1 | CAACA |
| FAM202 | -300ELEMENT | 1469 | 1477 | + | 0 | 1 | TGCAAAATC |
| FAM267 | TAAAGSTKST1 | 1499 | 1505 | + | 0 | 1 | TATAAAG |
| FAM267 | NTBBF1ARROLB | 1500 | 1506 | - | 0 | 1 | ACTTTAT |
| FAM270 | RAV1AAT | 1514 | 1518 | - | 0 | 1 | CAACA |
| FAM267 | TAAAGSTKST1 | 1542 | 1548 | - | 0 | 1 | ATTAAAG |
| FAM221 | S1FBOXSORPS1L21 | 1551 | 1556 | + | 0 | 1 | ATGGTA |
| FAM243 | TATABOX4 | 1555 | 1561 | + | 0 | 1 | TATATAA |
| FAM172 | MYCATERD | 1564 | 1570 | - | 0 | 1 | CATGTGA |
| FAM172 | MYCATRD2 | 1565 | 1571 | + | 0 | 1 | CACATGT |
| FAM241 | TATABOX2 | 1577 | 1583 | + | 0 | 1 | TATAAAT |
| FAM087 | BOXIINTPATPB | 1596 | 1601 | - | 0 | 1 | ATAGAA |
| FAM304 | OSE2ROOTNODULE | 1611 | 1615 | + | 0 | 1 | CTCTT |
| FAM099 | CCA1ATLHCB1 | 1620 | 1627 | - | 0 | 1 | AACAATCT |
| FAM241 | TATABOX2 | 1628 | 1634 | + | 0 | 1 | TATAAAT |
| FAM010 | WBOXHVISO1 | 1647 | 1661 | - | 0 | 1 | TATGACTTTTAAGA T |
| FAM087 | BOXIINTPATPB | 1667 | 1672 | + | 0 | 1 | ATAGAA |
| FAM290 | GT1GMSCAM4 | 1670 | 1675 | + | 0 | 1 | GAAAAA |
| FAM305 | ANAERO1CONSENSUS | 1698 | 1704 | + | 0 | 1 | AAACAAA |
| FAM202 | -300ELEMENT | 1711 | 1719 | - | 0 | 1 | TGAAAAGTT |
| FAM267 | TAAAGSTKST1 | 1740 | 1746 | - | 0 | 1 | CATAAAG |
| FAM026 | RYREPEATGMGY2 | 1742 | 1752 | - | 0 | 1 | TGCATGCATAA |
| FAM026 | RYREPEATVFLEB4 | 1745 | 1755 | + | 0 | 1 | TGCATGCATGC |
| FAM026 | RYREPEATVFLEB4 | 1746 | 1756 | - | 0 | 1 | TGCATGCATGC |
| FAM026 | RYREPEATBNNAPA | 1749 | 1759 | + | 0 | 1 | TGCATGCAACT |
| FAM325 | MYBCOREATCYCB1 | 1772 | 1776 | + | 0 | 1 | AACGG |

(continued)

| IUPAC Family | IUPAC | Start pos. | End pos. | Strand | Mismatches | Score | Sequence |
|---|---|---|---|---|---|---|---|
| FAM002 | SORLIP1AT | 1802 | 1814 | + | 0 | 1 | TGGGCGGCCACGT |
| FAM061 | GCCCORE | 1804 | 1810 | - | 0 | 1 | GGCCGCC |
| FAM002 | ABREOSRAB21 | 1806 | 1818 | - | 0 | 1 | GGCGACGTGGCCG |
| FAM002 | ABREOSRAB21 | 1807 | 1819 | + | 0 | 1 | GGCCACGTCGCCG |
| FAM107 | CGACGOSAMY3 | 1812 | 1816 | - | 0 | 1 | CGACG |
| FAM061 | GCCCORE | 1815 | 1821 | + | 0 | 1 | CGCCGCC |
| FAM002 | TGACGTVMAMY | 1827 | 1839 | + | 0 | 1 | GGAACTGACGTGT |
| FAM002 | HEXMOTIFTAH3H4 | 1829 | 1841 | - | 0 | 1 | GGACACGTCAGTT |
| FAM002 | GADOWNAT | 1830 | 1842 | + | 0 | 1 | ACTGACGTGTCCC |
| FAM302 | SORLIP2AT | 1838 | 1848 | - | 0 | 1 | CGGGCCGGGAC |
| FAM013 | LTRECOREATCOR15 | 1845 | 1851 | + | 0 | 1 | CCCGACG |
| FAM107 | CGACGOSAMY3 | 1847 | 1851 | + | 0 | 1 | CGACG |
| FAM107 | CGACGOSAMY3 | 1850 | 1854 | + | 0 | 1 | CGACG |
| FAM107 | CGACGOSAMY3 | 1853 | 1857 | + | 0 | 1 | CGACG |
| FAM107 | CGACGOSAMY3 | 1856 | 1860 | + | 0 | 1 | CGACG |
| FAM324 | CGCGBOXAT | 1871 | 1876 | + | 0 | 1 | CCGCGC |
| FAM324 | CGCGBOXAT | 1871 | 1876 | - | 0 | 1 | GCGCGG |
| FAM324 | CGCGBOXAT | 1876 | 1881 | + | 0 | 1 | CCGCGC |
| FAM324 | CGCGBOXAT | 1876 | 1881 | - | 0 | 1 | GCGCGG |
| FAM304 | OSE2ROOTNODULE | 1889 | 1893 | + | 0 | 1 | CTCTT |
| FAM151 | INTRONLOWER | 1910 | 1915 | + | 0 | 1 | TGCAGG |

7) p-MAEM1

PLACE analysis results of p-MAEM1 are listed in Table 55. No TATA box motifs are found in this promoter. One CAAT box motif is located at nucleotide position 655-659 of the forward strand.

Table 55. PLACE analysis results of the 922bp promoter p-MAEM1

| IUPAC Family | IUPAC | Start pos. | End pos. | Strand | Mismatches | Score | Sequence |
|---|---|---|---|---|---|---|---|
| FAM027 | -10PEHVPSBD | 8 | 13 | - | 0 | 1 | TATTCT |
| FAM304 | OSE2ROOTNODULE | 52 | 56 | - | 0 | 1 | CTCTT |
| FAM310 | CPBCSPOR | 105 | 110 | + | 0 | 1 | TATTAG |
| FAM087 | BOXIINTPATPB | 160 | 165 | + | 0 | 1 | ATAGAA |
| FAM271 | SEBFCONSSTPR10A | 168 | 174 | + | 0 | 1 | TTGTCAC |
| FAM322 | BIHD1OS | 169 | 173 | + | 0 | 1 | TGTCA |
| FAM012 | IBOXCORE | 208 | 214 | + | 0 | 1 | GATAAAT |
| FAM273 | TATCCAOSAMY | 216 | 222 | - | 0 | 1 | TATCCAA |
| FAM014 | MYBST1 | 217 | 223 | + | 0 | 1 | TGGATAC |
| FAM292 | PREATPRODH | 236 | 241 | + | 0 | 1 | ACTCAT |
| FAM027 | -10PEHVPSBD | 244 | 249 | - | 0 | 1 | TATTCT |
| FAM107 | CGACGOSAMY3 | 253 | 257 | + | 0 | 1 | CGACG |
| FAM267 | TAAAGSTKST1 | 298 | 304 | + | 0 | 1 | ACTAAAG |
| FAM263 | DPBFCOREDCDC3 | 313 | 319 | + | 0 | 1 | ACACACG |
| FAM014 | MYBST1 | 362 | 368 | - | 0 | 1 | TGGATAT |
| FAM025 | AMYBOX2 | 363 | 369 | + | 0 | 1 | TATCCAT |
| FAM273 | TATCCAOSAMY | 363 | 369 | + | 0 | 1 | TATCCAT |
| FAM015 | ACGTABOX | 393 | 398 | + | 0 | 1 | TACGTA |
| FAM015 | ACGTABOX | 393 | 398 | - | 0 | 1 | TACGTA |
| FAM202 | -300ELEMENT | 422 | 430 | + | 0 | 1 | TGAAAAATT |
| FAM290 | GT1GMSCAM4 | 423 | 428 | + | 0 | 1 | GAAAAA |
| FAM305 | ANAERO1CONSENSUS | 433 | 439 | + | 0 | 1 | AAACAAA |
| FAM039 | AACACOREOSGLUB1 | 434 | 440 | + | 0 | 1 | AACAAAC |
| FAM026 | RYREPEATGMGY2 | 493 | 503 | - | 0 | 1 | CCCATGCATCG |
| FAM002 | T/GBOXATPIN2 | 505 | 517 | + | 0 | 1 | GGAAACGTGGACA |

(continued)

| IUPAC Family | IUPAC | Start pos. | End pos. | Strand | Mismatches | Score | Sequence |
|---|---|---|---|---|---|---|---|
| FAM002 | SITEIOSPCNA | 540 | 552 | - | 0 | 1 | GACCAGGTGGGTT |
| FAM228 | SEF3MOTIFGM | 540 | 545 | + | 0 | 1 | AACCCA |
| FAM002 | RAV1BAT | 541 | 553 | + | 0 | 1 | ACCCACCTGGTCC |
| FAM002 | CACGTGMOTIF | 572 | 584 | - | 0 | 1 | TGCCACGTGTATC |
| FAM002 | ABREATRD2 | 573 | 585 | + | 0 | 1 | ATACACGTGGCAC |
| FAM263 | DPBFCOREDCDC3 | 575 | 581 | + | 0 | 1 | ACACGTG |
| FAM002 | SORLIP1AT | 577 | 589 | - | 0 | 1 | CAGCGTGCCACGT |
| FAM010 | WBOXATNPR1 | 613 | 627 | - | 0 | 1 | CTTGACACGTTAGCT |
| FAM002 | GADOWNAT | 614 | 626 | + | 0 | 1 | GCTAACGTGTCAA |
| FAM322 | BIHD1OS | 621 | 625 | + | 0 | 1 | TGTCA |
| FAM263 | DPBFCOREDCDC3 | 624 | 630 | - | 0 | 1 | ACACTTG |
| FAM002 | SORLIP1AT | 627 | 639 | - | 0 | 1 | GGGCCGGCCACAC |
| FAM302 | SORLIP2AT | 630 | 640 | - | 0 | 1 | GGGGCCGGCCA |
| FAM151 | INTRONLOWER | 639 | 644 | - | 0 | 1 | TGCAGG |
| FAM107 | CGACGOSAMY3 | 648 | 652 | - | 0 | 1 | CGACG |
| FAM100 | CCAATBOX1 | 655 | 659 | + | 0 | 1 | CCAAT |
| FAM228 | SEF3MOTIFGM | 680 | 685 | + | 0 | 1 | AACCCA |
| FAM061 | GCCCORE | 694 | 700 | + | 0 | 1 | TGCCGCC |
| FAM061 | GCCCORE | 697 | 703 | + | 0 | 1 | CGCCGCC |
| FAM194 | PALBOXAPC | 702 | 708 | + | 0 | 1 | CCGTCCG |
| FAM302 | SORLIP2AT | 706 | 716 | - | 0 | 1 | GGGGCCGGCGG |
| FAM002 | ACGTOSGLUB1 | 724 | 736 | + | 0 | 1 | TTGTACGTGCACC |
| FAM002 | ASF1MOTIFCAMV | 743 | 755 | - | 0 | 1 | ATCGATGACGATG |
| FAM307 | ANAERO3CONSENSUS | 755 | 761 | + | 0 | 1 | TCATCAC |
| FAM094 | CACGCAATGMGH3 | 761 | 768 | + | 0 | 1 | CACGCAAT |

(continued)

| IUPAC Family | IUPAC | Start pos. | End pos. | Strand | Mismatches | Score | Sequence |
|---|---|---|---|---|---|---|---|
| FAM263 | DPBFCOREDCDC3 | 771 | 777 | + | 0 | 1 | ACACAAG |
| FAM302 | SITEIIATCYTC | 785 | 795 | - | 0 | 1 | TGGGCTGTTTA |
| FAM002 | ASF1MOTIFCAMV | 846 | 858 | - | 0 | 1 | GCATGTGACGACA |
| FAM172 | MYCATERD | 851 | 857 | - | 0 | 1 | CATGTGA |
| FAM026 | RYREPEATLEGUMINBOX | 852 | 862 | + | 0 | 1 | CACATGCACAT |
| FAM172 | MYCATRD2 | 852 | 858 | + | 0 | 1 | CACATGC |
| FAM267 | TAAAGSTKST1 | 870 | 876 | + | 0 | 1 | CATAAAG |
| FAM304 | OSE2ROOTNODULE | 874 | 878 | - | 0 | 1 | CTCTT |

8) p-MAEM20

PLACE analysis results of p-MAEM20 are listed in Table 56. No TATA box motifs are found in this promoter. One CAAT box motif is located at nucleotide position 668-672 of the reverse strand.

Table 56. PLACE analysis results of the 698bp promoter p-MAEM20

| IUPAC Family | IUPAC | Start pos. | End pos. | Strand | Mismatches | Score | Sequence |
|---|---|---|---|---|---|---|---|
| FAM262 | CIACADIANLELHC | 3 | 12 | - | 0 | 1 | CAACTTAATC |
| FAM010 | WBOXATNPR1 | 9 | 23 | + | 0 | 1 | GTTGACTGGCAAATT |
| FAM012 | IBOXCORE | 32 | 38 | + | 0 | 1 | GATAATA |
| FAM012 | IBOXCORE | 55 | 61 | + | 0 | 1 | GATAACC |
| FAM266 | MYB1AT | 57 | 62 | + | 0 | 1 | TAACCA |
| FAM003 | MYBPLANT | 68 | 78 | + | 0 | 1 | CACCAACCGAC |
| FAM 171 | BOXLCOREDCPAL | 69 | 75 | + | 0 | 1 | ACCAACC |
| FAM013 | DRE2COREZMRAB17 | 73 | 79 | + | 0 | 1 | ACCGACT |
| FAM270 | RAV1AAT | 80 | 84 | - | 0 | 1 | CAACA |
| FAM303 | OSE1ROOTNODULE | 95 | 101 | - | 0 | 1 | AAAGATC |
| FAM021 | GT1CORE | 96 | 106 | - | 0 | 1 | TGGTTAAAGAT |
| FAM267 | TAAAGSTKST1 | 98 | 104 | - | 0 | 1 | GTTAAAG |
| FAM266 | MYB1AT | 101 | 106 | + | 0 | 1 | TAACCA |
| FAM008 | MYB2AT | 116 | 126 | + | 0 | 1 | TAACTAACTGT |
| FAM281 | MYB1LEPR | 117 | 123 | - | 0 | 1 | GTTAGTT |
| FAM270 | RAV1AAT | 124 | 128 | - | 0 | 1 | CAACA |
| FAM270 | RAV1AAT | 127 | 131 | - | 0 | 1 | CAACA |
| FAM069 | SURECOREATSULTR11 | 154 | 160 | + | 0 | 1 | GAGACTT |
| FAM245 | TBOXATGAPB | 157 | 162 | + | 0 | 1 | ACTTTG |
| FAM234 | SP8BFIBSP8BIB | 186 | 192 | + | 0 | 1 | TACTATT |
| FAM015 | ACGTABOX | 205 | 210 | + | 0 | 1 | TACGTA |
| FAM015 | ACGTABOX | 205 | 210 | - | 0 | 1 | TACGTA |
| FAM116 | DRE1COREZMRAB17 | 217 | 223 | - | 0 | 1 | ACCGAGA |
| FAM010 | WBOXHVISO1 | 253 | 267 | + | 0 | 1 | GGTGACTGACAGACT |
| FAM322 | BIHD1OS | 259 | 263 | - | 0 | 1 | TGTCA |

(continued)

| IUPAC Family | IUPAC | Start pos. | End pos. | Strand | Mismatches | Score | Sequence |
|---|---|---|---|---|---|---|---|
| FAM010 | WBBOXPCWRKY1 | 290 | 304 | - | 0 | 1 | TTTGACTAGAACAAG |
| FAM324 | CGCGBOXAT | 336 | 341 | + | 0 | 1 | GCGCGC |
| FAM324 | CGCGBOXAT | 336 | 341 | - | 0 | 1 | GCGCGC |
| FAM324 | CGCGBOXAT | 338 | 343 | + | 0 | 1 | GCGCGC |
| FAM324 | CGCGBOXAT | 338 | 343 | - | 0 | 1 | GCGCGC |
| FAM304 | OSE2ROOTNODULE | 348 | 352 | + | 0 | 1 | CTCTT |
| FAM012 | IBOXCORE | 351 | 357 | - | 0 | 1 | GATAAAA |
| FAM014 | SREATMSD | 352 | 358 | + | 0 | 1 | TTTATCC |
| FAM014 | MYBST1 | 353 | 359 | - | 0 | 1 | AGGATAA |
| FAM263 | DPBFCOREDCDC3 | 357 | 363 | - | 0 | 1 | ACACAGG |
| FAM278 | UPRMOTIFIIAT | 357 | 375 | + | 0 | 1 | CCTGTGTGTCTCCTCC ACG |
| FAM069 | ARFAT | 362 | 368 | + | 0 | 1 | GTGTCTC |
| FAM069 | SURECOREATSULTR11 | 362 | 368 | - | 0 | 1 | GAGACAC |
| FAM002 | ASF1MOTIFCAMV | 381 | 393 | - | 0 | 1 | TCTCATGACGCCT |
| FAM107 | CGACGOSAMY3 | 402 | 406 | + | 0 | 1 | CGACG |
| FAM026 | RYREPEATBNNAPA | 407 | 417 | + | 0 | 1 | ACCATGCAGTG |
| FAM324 | CGCGBOXAT | 426 | 431 | + | 0 | 1 | CCGCGC |
| FAM324 | CGCGBOXAT | 426 | 431 | - | 0 | 1 | GCGCGG |
| FAM324 | CGCGBOXAT | 428 | 433 | + | 0 | 1 | GCGCGC |
| FAM324 | CGCGBOXAT | 428 | 433 | - | 0 | 1 | GCGCGC |
| FAM324 | CGCGBOXAT | 430 | 435 | + | 0 | 1 | GCGCGT |
| FAM324 | CGCGBOXAT | 430 | 435 | - | 0 | 1 | ACGCGC |
| FAM061 | GCCCORE | 457 | 463 | - | 0 | 1 | GGCCGCC |
| FAM002 | ABRELATERD | 475 | 487 | - | 0 | 1 | GGCGACGTGGTAA |
| FAM002 | ABREOSRAB21 | 476 | 488 | + | 0 | 1 | TACCACGTCGCCC |

(continued)

| IUPAC Family | IUPAC | Start pos. | End pos. | Strand | Mismatches | Score | Sequence |
|---|---|---|---|---|---|---|---|
| FAM107 | CGACGOSAMY3 | 481 | 485 | - | 0 | 1 | CGACG |
| FAM002 | ABRE3HVA1 | 507 | 519 | + | 0 | 1 | AGCAACGTGTCGA |
| FAM261 | CDTDREHVCBF2 | 515 | 520 | + | 0 | 1 | GTCGAC |
| FAM261 | CDTDREHVCBF2 | 515 | 520 | - | 0 | 1 | GTCGAC |
| FAM002 | TGACGTVMAMY | 527 | 539 | + | 0 | 1 | GCCTCTGACGTGT |
| FAM002 | HEXMOTIFTAH3H4 | 529 | 541 | - | 0 | 1 | GGACACGTCAGAG |
| FAM002 | GADOWNAT | 530 | 542 | + | 0 | 1 | TCTGACGTGTCCC |
| FAM 194 | PALBOXAPC | 545 | 551 | + | 0 | 1 | CCGTCCT |
| FAM205 | PYRIMIDINEBOXOSRAM | 558 | 563 | - | 0 | 1 | CCTTTT |
| FAM107 | CGACGOSAMY3 | 595 | 599 | - | 0 | 1 | CGACG |
| FAM304 | OSE2ROOTNODULE | 660 | 664 | - | 0 | 1 | CTCTT |
| FAM100 | CCAATBOX1 | 668 | 672 | - | 0 | 1 | CCAAT |

EXAMPLE 11

BINARY VECTOR CONSTRUCTION FOR MAIZE TRANSFORMATION TO EVALUATE THE FUNCTION OF THE PROMOTERS

To facilitate subcloning, the promoter fragments of MAWS23, 27, 30, 57, 60, 63, MAEM1 and MAEM20 were modified by the addition of a *SwaI* restriction enzyme site at its 5' end and a a *Bsi*WI site at its 3'end. The *SwaI*-p-MA promoter-BsiWI fragment was digested and ligated into a *SwaI* and *BsiWI* digested BPS basic binary vector RCB1006 that comprises a plant selectable marker expression cassette (p-Ubi::AHAS::t-XI12), as well as a promoter evaluation cassette that consists of a multiple cloning site (MCS) for insertion of promoter and the rice MET1-1 intron to supply intron-mediated enhancement in monocot cells, GUS reporter gene, and NOS terminator. Diagram of RCB1006 is shown in Figure 13.

Table 57 lists the resulting binary vectors of the MA promoters, Sequences of the promoter cassettes in the binary vectors are shown in SEQ ID NOs: 55, 56, 59-61, 69-71.

Table57. Binary vectors of the MA promoters for corn transformation

| Promoter ID | Vector ID | Description | SEQ ID NO |
|---|---|---|---|
| p-MAWS23 | RTP1060 | p_MAWS23::iMET1::GUS::t-NOS | 69 |
| p-MAWS27 | RTP1059 | p_MAWS27::iMET1::GUS::t-NOS | 60 |
| p-MAWS30 | RTP1053 | p_MAWS30::iMET1::GUS::t-NOS | 70 |
| p-MAWS57 | RTP1049 | p_MAWS57::iMET1::GUS::t-NOS | 71 |
| p-MAWS60 | RTP1056 | p_MAWS60::iMET1::GUS::t-NOS | 55 |
| p-MAWS63 | RTP1048 | p_MAWS63::iMET1::GUS::t-NOS | 61 |
| p-MAEM1 | RTP1061 | p_MAEM1::iMET1::GUS::t-NOS | 56 |
| p-MAEM20 | RTP1064 | p_MAEM20::iMET1::GUS::t-NOS | 59 |

EXAMPLE 12

PROMOTER EVALUATION IN TRANSGENIC MAIZE WITH THE MA PROMOTERS

Expression patterns and levels driven by the MA promoters were measured using GUS histochemical analysis following the protocol in the art (Jefferson 1987). Maize transformation was conducted using an *Agrobacterium*-mediated transformation system. Ten and five single copy events for T0 and T1 plants were chosen for the promoter analysis. GUS expression was measured at various developmental stages:

1) Roots and leaves at 5-leaf stage
2) Stem at V-7 stage
2) Leaves, husk and silk at flowering stage (first emergence of silk)
3) Spikelets/Tassel (at pollination)
5) Ear or Kernels at 5, 10, 15, 20, and 25 days after pollination (DAP)

The results indicated that all these 9 promoters expressed specifically in pollen and in embryo (Figure 14 to 21).

Example 13

Identification of maize promoter pZmNP28

Based on an Affymetrix GeneChip® Wheat Genome Arrays experiment carried out using methods well-known to the persons skilled in the art, a transcript, *Ta.4874.1.S1_at* was selected as drought inducible expression. In brief, Affymetrix GeneChip® Wheat Genome Arrays were interrogated with probes derived from different RNA samples (stems, leaves, roots, drought-stressed roots and drought-stressed leaves) and candidate genes exhibiting drought inducible expression profile were identified. Stems, leaves and roots at normal growth condition and drought-stressed conditions were harvested, RNA was extracted and further purified, and the quality and yield of RNA was confirmed by techniques known in the art. The RNA was labeled and hybridized to GeneChip® Wheat Genome Arrays and the data analyzed to derive lists of genes in rank order. Microarray expression was analyzed using AVADIS™ software (Strand Genomics Pvt. Ltd. Bangalore). The raw data for all microarray analysis were imported into AVADIS and the RMA algorithm (Irazarry et al., Biostatistics 4(2): 249-264, 2003) was applied for background correction, normalization and probe aggregation. Absolute calls and p-values were generated for each gene and all probe sets that did not hybridize to nucleic acid in a sample, i.e., were absent (absolute call), across all arrays were removed from the analysis. For determination of transcripts preferentially or selectively expressed in drought-stressed roots and leaves, differential expression analyses were conducted where normal grown stem, leaves and roots were compared to drought-stressed roots and leaves. *Ta.4874.1.S1_at* showing 10-fold greater expression in drought-stressed leaves than in other tissues was selected as a drought inducible transcript.

The sequence of Ta.14617.1.S1_at was aligned to the sequences of the Affymetrix maize chip. The maize Zm.8705.1.S1_at was identified as an ortholog based on nucleotide sequence identity at 76% to part of the Ta.4874.1.S1_at. The sequence of Zm.8705.1.S1_at is shown in SEQ ID NO: 108.

Example 14

The expression patterns of Zm.8705.1.S1_at in maize

Analysis of 36 Affymetrix maize chips including immature embryo (6), leaf (8), ear (11), and kernel (11) indicated that Zm.8705.1.S1_at expressed specifically in immature embryo and in kernel (Figure 22).

Example 15

Validation of the expression pattern of Zm.8705.1.S1_at at the mRNA levels

Quantitative RT-PCR (qRT-PCR) was performed to validate expression of Zm.8705.1.S1_at gene in various types of tissues. To find mRNA sequence with better quality for designing qRT-PCR primers, the sequence of Zm.8705.1.S1_at was blasted against the BPS in-house Hyseq database. One Hyseq maize EST ZM06MC34918_62096753 (846 bp) was identified to be the same gene as Zm.8705.1.S1_at. The sequence of ZM06MC34918_62096753 is shown in SEQ ID NO: 108.

Primers for qRT-PCR were designed using the Vector NTI software package (Invitrogen, Carlsbad, CA, USA). Two sets of primers were used for PCR amplification. The sequences of primers are in Table 58.

Table 58. Primer sequences for RT-QPCR

| Primer | Sequence |
|---|---|
| ZM06MC34918_62096753_Forward_1 | CTCAAGGACGAGCTGACGAGCAT |
| ZM06MC34918_62096753_Reverse_1 | TAGCCCGGACGAGTCTCCTGAA |
| ZM06MC34918_62096753_Forward_2 | CAAGGACGAGCTGACGAGCAT |
| ZM06MC34918_62096753_Reverse_2 | CCCGGACGAGTCTCCTGAAA |
| GAPDH_Forward | GTAAAGTTCTTCCTGATCTGAAT |
| GAPDH_Reverse | TCGGAAGCAGCCTTAATA |

qRT-PCR was performed using SuperScript III Reverse Transcriptase (Invitrogen, Carlsbad, CA, USA) and SYBR Green QPCR Master Mix (Eurogentec, San Diego, CA, USA) in an ABI Prism 7000 sequence detection system. cDNA was synthesized using 2-3 $\mu$g of total RNA and 1 $\mu$L reverse transcriptase in a 20 $\mu$L volume. The cDNA was diluted to a range of concentrations (15-20 ng/$\mu$L). Thirty to forty ng of cDNA was used for QPCR in a 30 $\mu$L volume with SYBR Green QPCR Master Mix following the manufacturer's instruction. The thermocycling conditions were as follows: hold at 50°C for 2 minutes and at 95°C for 10 minutes, 40 cycles of 95 °C for 15 seconds and 60°C for 1 minute for amplification. After the final cycle of the amplification, the dissociation curve analysis was carried out to verify that the amplification occurred specifically and no primer dimer was produced during the amplification process. The housekeeping gene glyceraldehyde-3-phosphate-dehydrogenase (GAPDH, Table 58 for primer sequences) was used as an endogenous reference gene to normalize the calculation using Comparative Ct (Cycle of threshold) value method. The $\Delta C_T$ value was obtained by subtracting the GAPDH Ct value from the candidate gene (ZM06MC34918_62096753) Ct value of the same samples. The relative mRNA expression level of the gene candidate was given by $2^{-\Delta CT}$. The qRT-

PCR results are summarized in Figure 22. Both primer sets gave embryo-specifical expression patterns that are validated to the expression patterns obtained from the maize Affymetrix chip analysis.

Example 16

Annotation of the Zm.8705.1.S1_at

The sequence of the maize EST ZM06MC34918_62096753 was searched via BlastX. The EST did not hit to any known maize gene. The 20 homologues of EST ZM06MC34918_62096753 with the highest score are listed in Table 59.

Table 59. Zm.8705.1.S1_at gene annotation

| Accession | Description | Species | Score | E-value |
|---|---|---|---|---|
| AAL23749.1 | stress-inducible membrane pore protein | Bromus inermis | 259 | 2.00E-67 |
| NP_001042833. 1 | Os01g0303300 | Oryza sativa | 265 | 7.00E-65 |
| ABE83193.1 | Mitochondrial import inner membrane translocase, subunit Tim17/22 | Medicago truncatula | 200 | 9.00E-50 |
| AAV84280.1 | dehydration up-regulated putative membrane pore protein | | 199 | 1.00E-49 |
| NP_001049884. 1 | Os03g0305600 | Oryza sativa | 190 | 7.00E-47 |
| ABD32318.1 | mitochondrial import inner membrane translocase subunit Tim17/Tim22/Tim23 family protein | Oryza sativa | 190 | 9.00E-47 |
| EAY89687.1 | hypothetical protein OsI_010920 | Oryza sativa | 188 | 3.00E-46 |
| NP_849394.1 | protein translocase/ protein transporter | Arabidopsis thaliana | 178 | 3.00E-43 |
| AAM65873.1 | protein translocase/ protein transporter | Arabidopsis thaliana | 178 | 3.00E-43 |
| NP_567488.1 | hypothetical protein OsI_010920 | Oryza sativa | 178 | 3.00E-43 |
| EAZ26649.1 | hypothetical protein OsI_010920 | Oryza sativa | 164 | 4.00E-39 |
| CAB10395.1 | pore protein homolog | Arabidopsis thaliana | 148 | 3.00E-34 |
| AAT45008.1 | stress-inducible membrane pore-like protein | Xerophyta humilis | 136 | 2.00E-30 |
| CAK26794.1 | hypothetical protein | Sporobolus stapfianus | 134 | 5.00E-30 |
| NP_001054446.1 | Os05g0111200 | Oryza sativa | 91 | 7.00E-17 |
| CAA09867.1 | amino acid selective channel protein | Hordeum vulgare subsp.vulgure | 89 | 4.00E-16 |
| EAY96269.1 | hypothetical protein OsI_017502 | Oryza sativa | 87 | 8.00E-16 |
| CAA97910.1 | core protein | Pisum sativum | 84 | 7.00E-15 |
| CAA63967.1 | pom14 | Solanum tuberosum | 84 | 7.00E-15 |
| NP_180456.1 | protein translocase/ protein transporter | Arabidopsis thaliana | 80 | 1.00E-13 |

Example 17

Identification and isolation of the promoter region

For our promoter identification purposes, the sequence upstream of the start codon of Zm.8705.1.S1_at gene was defined as the promoter pZmNP28_655. To isolate this predicted promoter region, the sequence of EST ZM06MC34918_62096753 was mapped to the BPS in-house maize genomic DNA sequence database. One maize genomic DNA sequence of 1697bp, ZmGSStuc11-12-04.119561.1 was identified to harbour the EST ZM06MC34918_62096753 in an antisense direction. The 1697 bp sequence of the ZmGSStuc11-12-04.119561.1 shown in SEQ ID NO: 196 contains a complete coding sequence (CDS) of the gene and a 655 bp upstream sequence of the start codon ATG including a 140 bp putative 5"UTR based on the sequence alignment result to the Zea mays mRNA clone EL01 N0448C02.c sequence (GenBank accession: BTO17732.1). This 656 bp was designated as pZmNP28_655 and cloned by PCR using the following specific primers:

Forward primer: AAAAGTAGCAATTGGGATAAC (SEQ ID NO: 188)
Reverse primer: GCTCGTCAGCTCGTCCTTGAG (SEQ ID NO: 189)

The CDS sequence shown in SEQ ID NO: 36 was identified by Vector NTI software package as a gene encoding a protein that is homologous to the stress-inducible membrane pore protein of Bromus inermis (GenBank accession: AAL23749.1, Table 59). The translated amino acid sequence of the CDS is shown in SEQ ID NO: 54 and sequence of pZmNP28_655 is shown as part of SEQ ID NO: 18 (nucleotide 1459 to nucleotide 2112).

To obtain more sequence information of further upstream of pZmNP28_655,, GenomeWalk was conducted. Maize genomic DNA was extracted from *Zea mays* B73 and digested with the blunt end restriction enzymes *Ssp*I, *Sca*I, *Eco*RV, *Stu*I, *Dra*I, to generate Genome Walker™ maize DNA libraries. Digested DNA was purified with phenol/chloroform and re-dissolved in TE buffer (10mM Tris HCl, 0.1 mM EDTA, pH 8.0) prior to ligation to the Genome Walker™ adapters following the manufacturer's instruction (Clontech Laboratories, Inc, Mountain View, CA, USA). Nested PCR was performed using Genome Walker™ library template with adapter and sequence specific primers. The GenomeWalk reactions produced a 2771 bp fragment containing 270 bp overlap with the 5' end of pZmNP28_655. The agarose gel showing this fragment was imaged as in Figure 23. The fragment of Lane 6 of Figure 23 was purified, cloned into a TOPO TA vector, pCR2.1-TOPO (Invitrogen, Carlsbad, CA 92008, USA) and sequenced. A contig sequence containing this 2771 bp fragment combined with pZmNP28_655 was assembled and the resulted 3177 bp contig sequence is shown in SEQ ID NO: 90.

Example 18

Identification of the longer promoter regions

To determine the promoter region, we isolated 3 more fragment with different lengths based on above contig sequence information for evaluation of their function as a promoter:

1). A 2070 bp fragment designated as pZmNP28_2070 and isolated by PCR using the following specific primers:

Forward primer: CTAGGTTGGTGAGATCCTTAG (SEQ ID NO: 190)
Reverse primer: CATCTTCTTCGACGCCTGTTC (SEQ ID NO: 191)

Sequence of pZmNP28_2070 is shown in SEQ ID NO:18

2). A 1706 bp fragment designated as pZmNP28_1706 and isolated by PCR using the following specific primers:

Forward primer: GTGGCAGCTCTGAAGACTCCAAC (SEQ ID NO: 192)
Reverse primer: TGAGGCCGAGGCACTACGTCATG (SEQ ID NO: 193)

Compared to pZmNP28_2070, pZmNP28_1706 has a deletion of 326 bp at its 3' end of the pZmNP28_2070. Sequence of pZmNP28_1706 is shown as part of SEQ ID NO: 18.

3). A 507 bp fragment designated as pZmNP28_507 and isolated by PCR using the following specific primers:

Forward primer: TGACGTTTGTGTAATTGGGCTTG (SEQ ID NO: 194)
Reverse primer: GCTCGTCAGCTCGTCCTTGAG (SEQ ID NO: 195)

Example 19

BlastN results of the longest promoter region pZmNP28_2070

The 2113 bp region from the 5'end of pZmNP28_2070 to immediate upstream of the ATG was searched via BlastN. A few homologues to the 3' end of this region were found and listed in Table 60.

Table 60. BlastN results of the 2113 bp region including ZmNP28_2070

| | | | |
|---|---|---|---|
| NM_001159134.1 | Zea mays LOC100286246 (gpm462), mRNA >gb\|EU976384.1\|Zea | 202 | 7,00E-48 |
| EU968359.1 | Zea mays clone 319482 stress-inducible membrane pore protein mRNA, complete cds | 202 | 7,00E-48 |
| EU953175.1 | Zea mays clone 1389131 mRNA sequence | 202 | 7,00E-48 |
| AY111174.1 | Zea mays CL27726_1 mRNA sequence | 196 | 3,00E-46 |
| DQ245984.1 | Zea mays clone 93911 mRNA sequence | 195 | 1,00E-45 |

Example 20

PLACE Analysis of the longest promoter region pZmNP28_2070

*Cis*-acting motifs in the 2113 bp region from the 5'end of pZmNP28_2070 to immediate upstream of the ATG were identified using PLACE (a database of Plant Cis-acting Regulatory DNA elements) via Genomatix. The Results are listed in Table 61.

Table 61. PLACE analysis results of the 656 bp ZmNP19 promoter

| IUPAC | Start pos. | End pos. | Strand | Sequence |
|---|---|---|---|---|
| MYBPLANT | 1 | 11 | - | CACCAACCTAG |
| PALBOXLPC | 4 | 14 | - | TCTCACCAACC |
| BOXLCOREDCPAL | 4 | 10 | - | ACCAACC |
| TAAAGSTKST1 | 33 | 39 | - | CATAAAG |
| SORLIP1AT | 37 | 49 | - | AGAGCTGCCACAT |
| RAV1AAT | 58 | 62 | + | CAACA |
| CATATGGMSAUR | 86 | 91 | + | CATATG |
| CATATGGMSAUR | 86 | 91 | - | CATATG |
| GT1GMSCAM4 | 105 | 110 | + | GAAAAA |
| OSE1ROOTNODULE | 152 | 158 | - | AAAGATG |
| TAAAGSTKST1 | 159 | 165 | - | CCTAAAG |
| TBOXATGAPB | 194 | 199 | + | ACTTTG |
| CCAATBOX1 | 203 | 207 | + | CCAAT |
| OSE2ROOTNODULE | 227 | 231 | - | CTCTT |
| AMMORESIIUDCRNIA1 | 231 | 238 | + | GGTAGGGT |
| MYBPZM | 231 | 237 | - | CCCTACC |
| CCAATBOX1 | 247 | 251 | + | CCAAT |
| CIACADIANLELHC | 268 | 277 | + | CAATAAAATC |
| IBOXCORENT | 276 | 282 | - | GATAAGA |
| P1BS | 283 | 290 | + | GAATATCC |

(continued)

| IUPAC | Start pos. | End pos. | Strand | Sequence |
|---|---|---|---|---|
| P1BS | 283 | 290 | - | GGATATTC |
| MYBST1 | 285 | 291 | - | GGGATAT |
| OSE2ROOTNODULE | 310 | 314 | - | CTCTT |
| -10PEHVPSBD | 322 | 327 | + | TATTCT |
| BOXIINTPATPB | 324 | 329 | - | ATAGAA |
| PYRIMIDINEBOXOSRAM | 333 | 338 | - | CCTTTT |
| -10PEHVPSBD | 361 | 366 | + | TATTCT |
| MYB1LEPR | 372 | 378 | + | GTTAGTT |
| -300CORE | 386 | 394 | + | TGTAAAGAC |
| TAAAGSTKST1 | 386 | 392 | + | TGTAAAG |
| IBOXCORE | 427 | 433 | + | GATAAAG |
| TAAAGSTKST1 | 427 | 433 | + | GATAAAG |
| BIHD1OS | 448 | 452 | - | TGTCA |
| RAV1AAT | 451 | 455 | + | CAACA |
| GT1GMSCAM4 | 456 | 461 | - | GAAAAA |
| TBOXATGAPB | 462 | 467 | - | ACTTTG |
| TAAAGSTKST1 | 468 | 474 | + | ATTAAAG |
| OSE2ROOTNODULE | 472 | 476 | - | CTCTT |
| CCAATBOX1 | 487 | 491 | + | CCAAT |
| DPBFCOREDCDC3 | 511 | 517 | + | ACACAAG |
| MYBST1 | 516 | 522 | + | AGGATAT |
| SEF3MOTIFGM | 562 | 567 | - | AACCCA |
| AACACOREOSGLUB1 | 565 | 571 | - | AACAAAC |
| SORLIP1AT | 570 | 582 | + | TTCCTCGCCACTC |
| DPBFCOREDCDC3 | 630 | 636 | + | ACACTAG |
| ELRECOREPCRP1 | 661 | 675 | - | TTTGACCTAAATAAG |
| QELEMENTZMZM13 | 666 | 680 | + | TTAGGTCAAACTATC |
| SORLIP2AT | 682 | 692 | - | GGGGCCATGAA |
| TAAAGSTKST1 | 692 | 698 | - | TATAAAG |
| WBBOXPCWRKY1 | 695 | 709 | - | TTTGACTATACTATA |
| PYRIMIDINEBOXHVEPB | 710 | 717 | - | TTTTTTCC |
| GT1GMSCAM4 | 711 | 716 | + | GAAAAA |
| ANAERO1CONSENSUS | 715 | 721 | + | AAACAAA |
| CAREOSREP1 | 749 | 754 | + | CAACTC |
| CPBCSPOR | 762 | 767 | + | TATTAG |
| GT1CORE | 777 | 787 | - | AGGTTAAGGAC |
| PYRIMIDINEBOXOSRAM | 785 | 790 | + | CCTTTT |
| ATHB1ATCONSENSUS | 809 | 817 | + | CAATAATTG |

(continued)

| IUPAC | Start pos. | End pos. | Strand | Sequence |
|---|---|---|---|---|
| ATHB1ATCONSENSUS | 809 | 817 | - | CAATTATTG |
| S1FSORPL21 | 818 | 825 | - | ATGGTATT |
| S1FBOXSORPS1L21 | 820 | 825 | - | ATGGTA |
| CCAATBOX1 | 851 | 855 | + | CCAAT |
| ATHB6COREAT | 852 | 860 | + | CAATTATTA |
| PREATPRODH | 863 | 868 | + | ACTCAT |
| CCAATBOX1 | 871 | 875 | + | CCAAT |
| RAV1AAT | 883 | 887 | - | CAACA |
| WBOXHVISO1 | 885 | 899 | - | AGTGACTAATGACAA |
| BIHD1OS | 886 | 890 | + | TGTCA |
| DPBFCOREDCDC3 | 928 | 934 | - | ACACGAG |
| 2SSEEDPROTBANAPA | 929 | 937 | - | CAAACACGA |
| CCAATBOX1 | 944 | 948 | + | CCAAT |
| QELEMENTZMZM13 | 951 | 965 | + | CTAGGTCATGTTTGG |
| SITEIIATCYTC | 963 | 973 | + | TGGGCTCCACT |
| CIACADIANLELHC | 994 | 1003 | + | CAACATGATC |
| RAV1AAT | 994 | 998 | + | CAACA |
| RAV1AAT | 1016 | 1020 | - | CAACA |
| WBOXATNPR1 | 1017 | 1031 | + | GTTGACTAAAGACCT |
| TAAAGSTKST1 | 1021 | 1027 | + | ACTAAAG |
| WBOXHVISO1 | 1053 | 1067 | + | CATGACTTCGCTCAA |
| SURECOREATSULTR11 | 1079 | 1085 | - | GAGACTA |
| MYCATERD | 1127 | 1133 | - | CATGTGG |
| MYCATRD2 | 1128 | 1134 | + | CACATGT |
| -300ELEMENT | 1138 | 1146 | + | TGCAAAGGG |
| CGACGOSAMY3 | 1169 | 1173 | - | CGACG |
| TRANSINITDICOTS | 1173 | 1180 | - | AAGATGGC |
| OSE1ROOTNODULE | 1175 | 1181 | - | AAAGATG |
| TAAAGSTKST1 | 1185 | 1191 | - | GGTAAAG |
| IBOXCORE | 1218 | 1224 | - | GATAATA |
| SREATMSD | 1219 | 1225 | + | ATTATCC |
| MYBST1 | 1220 | 1226 | - | GGGATAA |
| CGCGBOXAT | 1226 | 1231 | + | CCGCGT |
| CGCGBOXAT | 1226 | 1231 | - | ACGCGG |
| MYBPZM | 1240 | 1246 | + | CCCTACC |
| HBOXCONSENSUSPVCHS | 1241 | 1261 | + | CCTACCCTAAACACTATGGGC |
| RAV1AAT | 1261 | 1265 | + | CAACA |
| CGACGOSAMY3 | 1267 | 1271 | - | CGACG |

(continued)

| IUPAC | Start pos. | End pos. | Strand | Sequence |
|---|---|---|---|---|
| SURECOREATSULTR11 | 1303 | 1309 | - | GAGACCT |
| MYBCOREATCYCB1 | 1314 | 1318 | - | AACGG |
| RAV1AAT | 1326 | 1330 | - | CAACA |
| CGACGOSAMY3 | 1373 | 1377 | - | CGACG |
| LTRECOREATCOR15 | 1425 | 1431 | - | TCCGACC |
| SORLIP5AT | 1440 | 1446 | + | GAGTGAG |
| INTRONLOWER | 1447 | 1452 | + | TGCAGG |
| CCAATBOX1 | 1469 | 1473 | - | CCAAT |
| MYBST1 | 1472 | 1478 | + | GGGATAA |
| SREATMSD | 1473 | 1479 | - | GTTATCC |
| IBOXCORE | 1474 | 1480 | + | GATAACA |
| GT1GMSCAM4 | 1485 | 1490 | + | GAAAAA |
| GT1CORE | 1513 | 1523 | + | GGGTTAAATAA |
| TATABOXOSPAL | 1516 | 1522 | - | TATTTAA |
| EECCRCAH1 | 1560 | 1566 | - | GATTTCC |
| IBOXCORE | 1568 | 1574 | - | GATAAAT |
| OSE1ROOTNODULE | 1571 | 1577 | - | AAAGATA |
| CCAATBOX1 | 1590 | 1594 | - | CCAAT |
| RBCSCONSENSUS | 1591 | 1597 | - | AATCCAA |
| TGACGTVMAMY | 1602 | 1614 | + | GATTTTGACGTTT |
| HEXMOTIFTAH3H4 | 1604 | 1616 | - | ACAAACGTCAAAA |
| WBOXATNPR1 | 1605 | 1619 | + | TTTGACGTTTGTGTA |
| CCAATBOX1 | 1620 | 1624 | - | CCAAT |
| SITEIIATCYTC | 1622 | 1632 | + | TGGGCTTGACA |
| WBOXATNPR1 | 1626 | 1640 | + | CTTGACAGCCCCATC |
| BIHD1OS | 1628 | 1632 | - | TGTCA |
| LTRE1HVBLT49 | 1661 | 1666 | - | CCGAAA |
| SITEIIATCYTC | 1675 | 1685 | - | TGGGCCGAATC |
| MYBST1 | 1689 | 1695 | + | AGGATAG |
| RAV1AAT | 1707 | 1711 | + | CAACA |
| TGACGTVMAMY | 1720 | 1732 | + | GTCCATGACGTAG |
| HEXMOTIFTAH3H4 | 1722 | 1734 | - | CACTACGTCATGG |
| SITEIIATCYTC | 1742 | 1752 | - | TGGGCTTTGAG |
| TATABOX3 | 1757 | 1763 | - | TATTAAT |
| MYBST1 | 1761 | 1767 | - | TGGATAT |
| TATCCAYMOTIFOSRAMY | 1762 | 1768 | + | TATCCAC |
| TATCCACHVAL21 | 1762 | 1768 | + | TATCCAC |
| ACGTABOX | 1772 | 1777 | + | TACGTA |

(continued)

| IUPAC | Start pos. | End pos. | Strand | Sequence |
|---|---|---|---|---|
| ACGTABOX | 1772 | 1777 | - | TACGTA |
| WBOXHVISO1 | 1785 | 1799 | - | AGTGACTCCCTCGGC |
| WBOXNTCHN48 | 1793 | 1807 | - | ACTGACTCAGTGACT |
| GCN4OSGLUB1 | 1798 | 1806 | + | CTGAGTCAG |
| MYBCOREATCYCB1 | 1812 | 1816 | + | AACGG |
| UPRMOTIFIIAT | 1832 | 1850 | + | CCGTGTGCCGGTGTCCACG |
| DPBFCOREDCDC3 | 1839 | 1845 | - | ACACCGG |
| CGCGBOXAT | 1848 | 1853 | + | ACGCGC |
| CGCGBOXAT | 1848 | 1853 | - | GCGCGT |
| CGCGBOXAT | 1850 | 1855 | + | GCGCGC |
| CGCGBOXAT | 1850 | 1855 | - | GCGCGC |
| UPRMOTIFIIAT | 1855 | 1873 | + | CCCCGGTGCGGCCGCCACG |
| SORLIP1AT | 1862 | 1874 | + | GCGGCCGCCACGA |
| GCCCORE | 1864 | 1870 | + | GGCCGCC |
| CGCGBOXAT | 1875 | 1880 | + | CCGCGG |
| CGCGBOXAT | 1875 | 1880 | - | CCGCGG |
| GADOWNAT | 1878 | 1890 | - | CGGCACGTGTCCG |
| CACGTGMOTIF | 1879 | 1891 | + | GGACACGTGCCGG |
| DPBFCOREDCDC3 | 1881 | 1887 | + | ACACGTG |
| SORLIP2AT | 1889 | 1899 | + | CGGGCCTCGCA |
| DPBFCOREDCDC3 | 1899 | 1905 | + | ACACGCG |
| CGCGBOXAT | 1901 | 1906 | + | ACGCGT |
| CGCGBOXAT | 1901 | 1906 | - | ACGCGT |
| SORLIP2AT | 1909 | 1919 | - | GGGGCCGTGGG |
| UPRMOTIFIIAT | 1935 | 1953 | + | CCGCGGTGCCCGCGCCACG |
| CGCGBOXAT | 1935 | 1940 | + | CCGCGG |
| CGCGBOXAT | 1935 | 1940 | - | CCGCGG |
| SORLIP1AT | 1942 | 1954 | + | GCCCGCGCCACGG |
| CGCGBOXAT | 1944 | 1949 | + | CCGCGC |
| CGCGBOXAT | 1944 | 1949 | - | GCGCGG |
| REBETALGLHCB21 | 1980 | 1986 | + | CGGATAG |
| CGACGOSAMY3 | 1999 | 2003 | + | CGACG |
| HEXAMERATH4 | 1999 | 2004 | - | CCGTCG |
| DRE2COREZMRAB17 | 2003 | 2009 | - | ACCGACC |
| MYBCOREATCYCB1 | 2013 | 2017 | - | AACGG |
| SORLIP1AT | 2021 | 2033 | - | TGTCTCGCCACTC |
| ARFAT | 2028 | 2034 | - | CTGTCTC |
| SURECOREATSULTR11 | 2028 | 2034 | + | GAGACAG |

(continued)

| IUPAC | Start pos. | End pos. | Strand | Sequence |
|---|---|---|---|---|
| SEBFCONSSTPR10A | 2028 | 2034 | - | CTGTCTC |
| BS1EGCCR | 2033 | 2038 | + | AGCGGG |
| CGACGOSAMY3 | 2058 | 2062 | + | CGACG |
| TCA1MOTIF | 2063 | 2072 | - | TCATCTTCTT |
| DPBFCOREDCDC3 | 2082 | 2088 | + | ACACGCG |
| CGCGBOXAT | 2084 | 2089 | + | ACGCGG |
| CGCGBOXAT | 2084 | 2089 | - | CCGCGT |
| ASF1MOTIFCAMV | 2101 | 2113 | + | CGAGCTGACGAGC |

Example 21

Binary vector construction for maize transformation

For pZmNP28_655 and pZmNP28_507, the promoter fragments obtained from PCR were cloned into pENTR™ 5'-TOPO TA Cloning vector (Invitrogen, Carlsbad, CA, USA). An intron-mediated enhancement (IME)-intron (BPSI.1) was inserted into the restriction enzyme *BsrG*I site that is 24 bp downstream of the 3' end of the pZmNP28_655 and pZ_mNP28_507. The resulting vector was used as a Gateway entry vector in order to produce the final binary vector RLN 90 and RLN 93 for maize transformation, which comprises a plant selectable marker expression cassette (p-Ubi::AHAS::t-NOS) as well as a promoter evaluation cassette that consists testing promoter, MET1-1 intron to supply intron-mediated enhancement in monocot cells, GUS reporter gene, and NOS terminator (Figure 24 A and B). For pZmNP28_2070 and pZmNP28_1706, the 2070 bp and the 1706 bp fragments were modified by the addition of a *Pac*I restriction enzyme site at its 5' end and a *Bsi*WI site at its 3'end. The *PacI*-pZmNP28_2070-*Bsi*WI and *Pac*I-pZmNP28_1706-*Bsi*WI fragments were digested and ligated into a *Pac*I and *Bsi*WI digested BPS basic binary vector HF84. HF84 comprises a plant selectable marker expression cassette (p-Ubi::c-EcEsdA::t-NOS) as well as a promoter evaluation cassette that consists of a multiple cloning site for insertion of putative promoters via *Pac*I and *Bsi*WI, rice MET1-1 intron to supply intron-mediated enhancement in monocot cells, GUS reporter gene, and NOS terminator. The resulting binary vectors comprising the pZmNP28_2070::i-MET1::GUS::t-NOS or pZmNP28_1706::i-MET1::GUS::t-NOS expression cassette was named as RHF160 or RHF158 and were used maize transformation to evaluate the expression pattern driven by pZmNP28_2070 or pZmNP1706. Figure 24 C is a diagram of RHF160 and Figure 24 D is a diagram of RHF158.

Example 22

Promoter evaluation in transgenic maize with the binary vectrs RLN90, RLN93, RHF158 and RHF160

Expression patterns and levels driven by the promoter were measured using GUS histochemical analysis following the protocol in the art (Jefferson 1987). Maize transformation was conducted using an *Agrobacterium*-mediated transformation system. Ten and five single copy events for T0 and T1 plants were used for the promoter analysis. GUS expression was measured at various developmental stages:

1) Roots and leaves at 5-leaf stage
2) Stem at V-7 stage
2) Leaves, husk and silk at flowering stage (first emergence of silk)
3) Spikelets/Tassel (at pollination)
5) Ear or Kernels at 5, 10, 15, 20, and 25 days after pollination (DAP)

The results indicated that pZmNP28_655, pZmNP28_507 and pZmNP28_2070 expressed specifically in pollen and in embryo, and pZmNP28_1706 did not express in any tested tissues (Figure 25 A to D)).

Example 23

Core sequences driving the embryo-specific expression of promoter pZmNP28

The experiment results of expression evaluation driven by several fragments of this promoter region in different length as described as above showed that a 326 bp core sequence is critical to the embryo specific expression of this promoter. The promoter fragments, pZmNP28_655, pZmNP28-507 and pZmNP28_2070, which contain this core sequence, showed embryo specific expression (Figures 25 A, C and D). The promoter fragment pZmNP28_1706, which does not contain this core sequence, showed no expression at all. The core sequence is shown in SEQ ID NO: 18 (in particular nucleotides 1745 to 2070 of SEQ ID NO:18)

## Claims

1. An expression cassette for regulating seed-specific expression of a polynucleotide of interest, said expression cassette comprising a transcription regulating nucleotide sequence selected from the group of sequences consisting of:

   (a) a nucleic acid sequence of SEQ ID NO: SEQ ID NO: 7, 8, 9, 10, 11, 12, 13, 14, 15, 16, 17, 2, 3, 4, 5, 6, or 18;
   (b) a nucleic acid sequence which is at least 80% identical to a nucleic acid sequence shown in any one of SEQ ID NO: SEQ ID NO: 7, 8, 9, 10, 11, 12, 13, 14, 15, 16, 17, 2, 3, 4, 5, 6, or 18;
   (c) a nucleic acid sequence which hybridizes under stringent conditions to a nucleic acid sequence of SEQ ID NO: SEQ ID NO: 7, 8, 9, 10, 11, 12, 13, 14, 15, 16, 17, 2, 3, 4, 5, 6, or 18.

2. The expression cassette of claim 1, wherein said expression cassette further comprises at least one polynucleotide of interest being operatively linked to the transcription regulating nucleotide sequence.

3. The expression cassette of claim 1 or 2, wherein said polynucleotide of interest is heterologous with respect to the transcription regulating nucleotide sequence.

4. A vector comprising the expression cassette of any one of claims 1 to 3.

5. The vector of claim 4, wherein said vector is an expression vector.

6. A host cell comprising the expression cassette of any one of claims 1 to 3 or the vector of claim 4 or 5.

7. The host cell of claim 6, wherein said host cell is a plant cell.

8. A transgenic plant tissue, plant organ, plant or seed comprising the expression cassette of any one of claims 1 to 3 or the vector of claim 4 or 5.

9. The transgenic plant tissue, plant organ, plant or seed of claim 8, wherein said transgenic plant tissue, plant organ, plant or seed is derived from monocotyledones.

10. A method for producing a transgenic plant tissue, plant organ, plant or seed comprising

    (a) introducing the expression cassette of any one of claims 1 to 3 or the vector of claim 4 or 5 into a plant cell; and
    (b) regenerating said plant cell to form a plant tissue, plant organ, plant or seed.

11. A method for producing a transgenic plant tissue, plant organ, plant or seed comprising

    (a) integrating the expression cassette of any one of claims 1 to 3 or the vector of claim 4 or 5 into the genome of a plant cell;
    (b) regenerating said plant cell to form a plant tissue, plant organ, plant or seed, and
    (c) selecting said plant cell to form a plant tissue, plant organ, plant or seed for the presence of the expression cassette of any of claims 1 to 3 or the vector of claim 4 or 5.

Figure 1

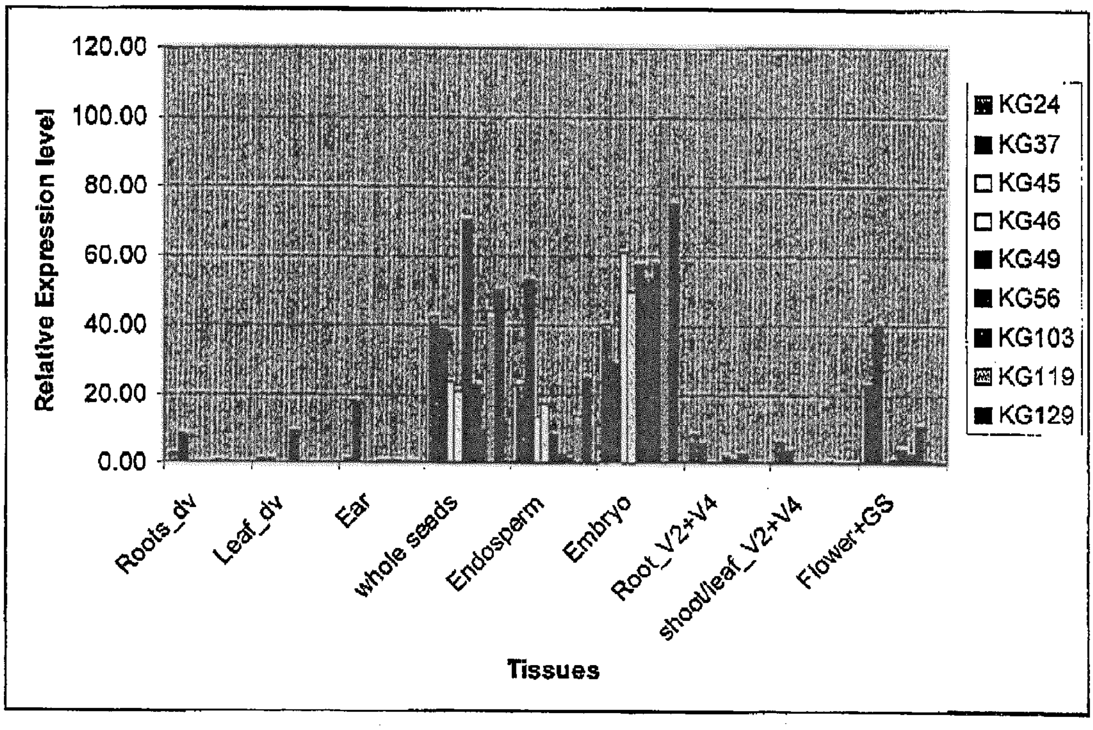

120.00
100.00
80.00
60.00
40.00
20.00
0.00
Relative Expression level
KG24
KG37
KG45
KG46
KG49
KG56
KG103
KG119
KG129
Roots_dv
Leaf_dv
Ear
whole seeds
Endosperm
Embryo
Root_V2+V4
shoot/leaf_V2+V4
Flower+GS
Tissues

Figure 2

(A)

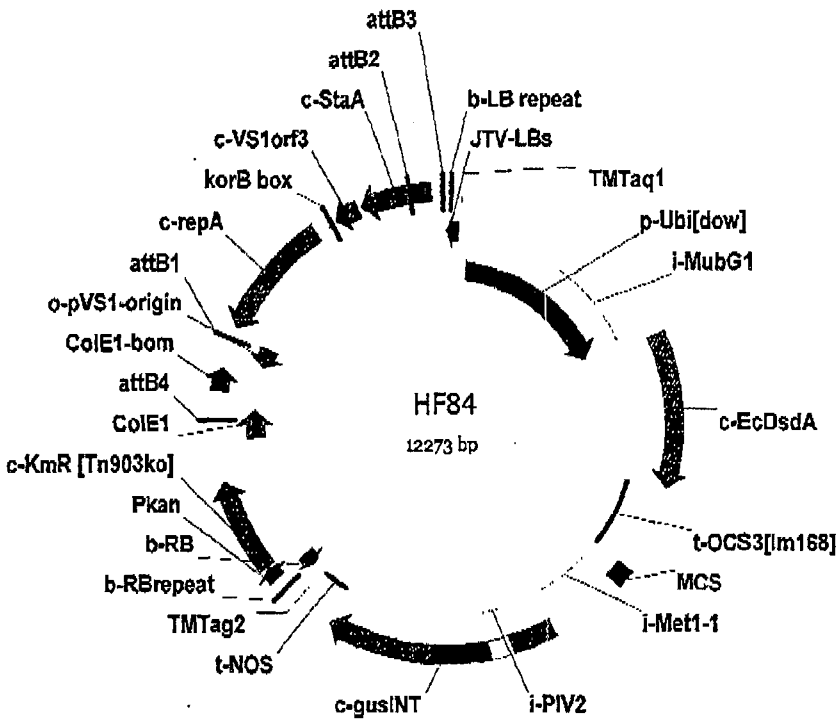
attB3
attB2
c-StaA
c-VS1orf3
korB box
c-repA
attB1
o-pVS1-origin
ColE1-bom
attB4
ColE1
c-KmR [Tn903ko]
Pkan
b-RB
b-RBrepeat
TMTag2
t-NOS
c-gusINT
i-PIV2
i-Met1-1
MCS
t-OCS3[lm168]
c-EcDsdA
i-MubG1
p-Ubi[dow]
TMTaq1
JTV-LBs
b-LB repeat
HF84
12273 bp

Figure 2

(B)

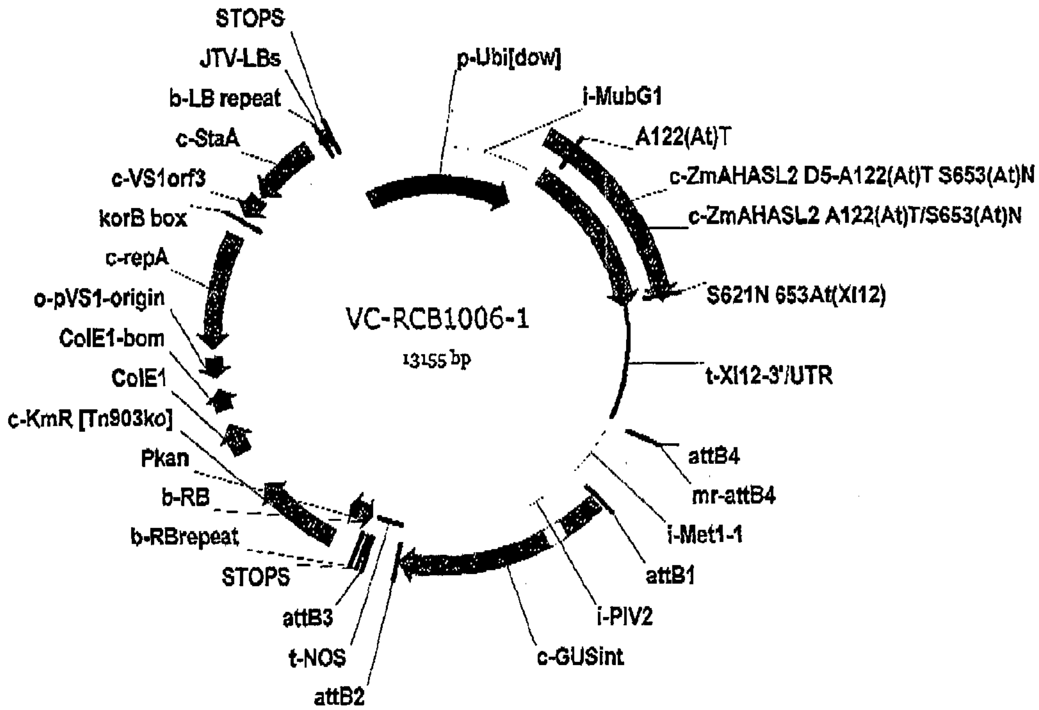
STOPS
JTV-LBs
b-LB repeat
c-StaA
c-VS1orf3
korB box
c-repA
o-pVS1-origin
ColE1-bom
ColE1
c-KmR [Tn903ko]
Pkan
b-RB
b-RBrepeat
STOPS
attB3
t-NOS
attB2
p-Ubi[dow]
i-MubG1
A122(At)T
c-ZmAHASL2 D5-A122(At)T S653(At)N
c-ZmAHASL2 A122(At)T/S653(At)N
S621N 653At(XI12)
t-XI12-3'/UTR
attB4
mr-attB4
i-Met1-1
attB1
i-PIV2
c-GUSint
VC-RCB1006-1
13155 bp

Figure 3

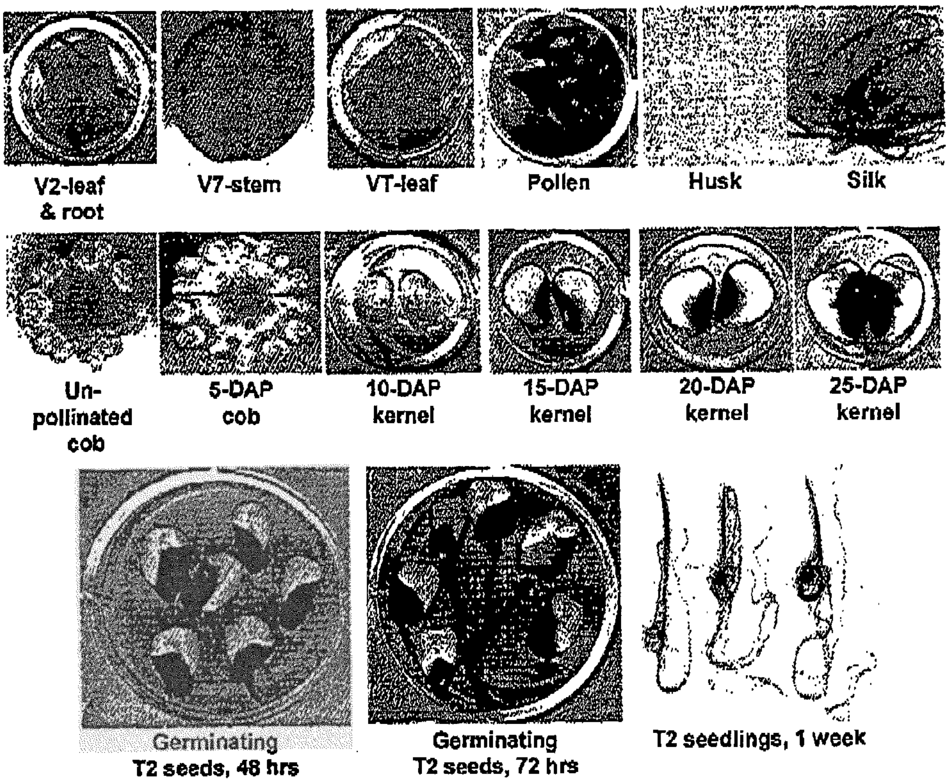


Figure 4

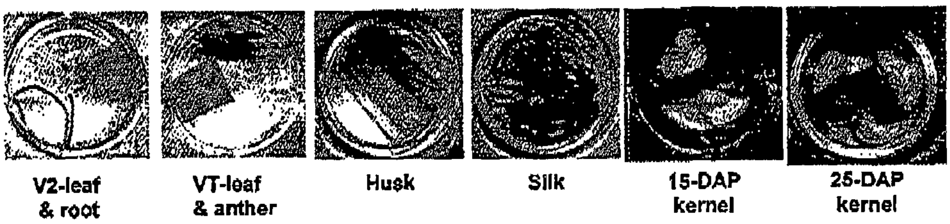

Figure 5

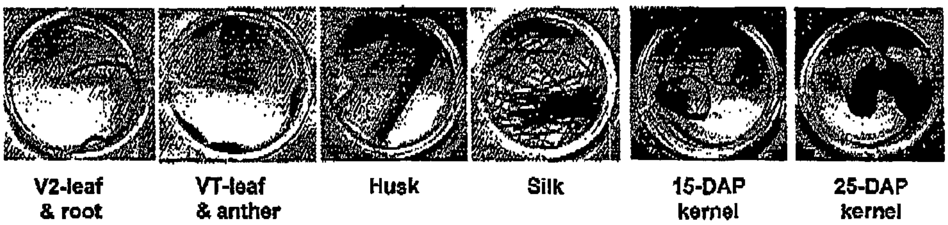


Figure 6

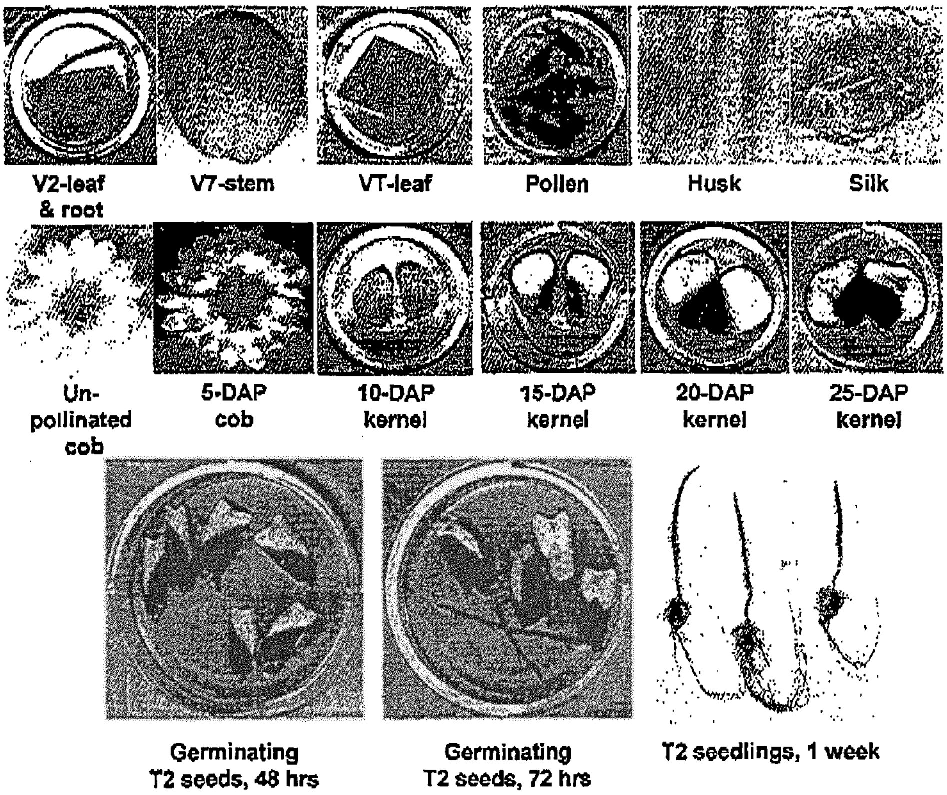

Figure 7

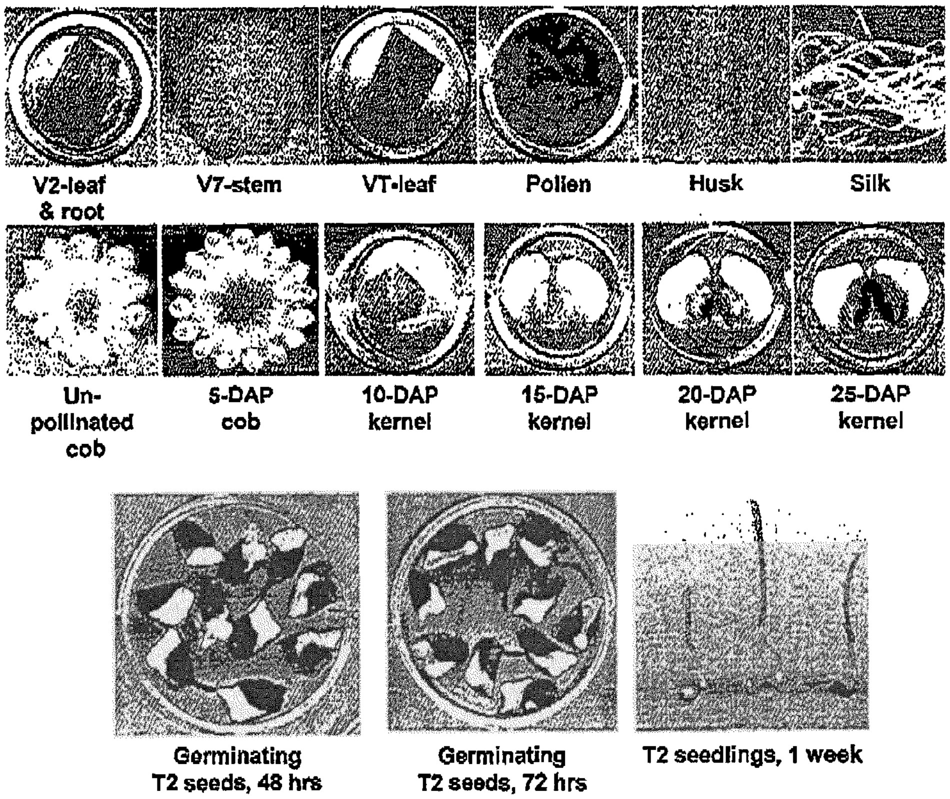
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 8

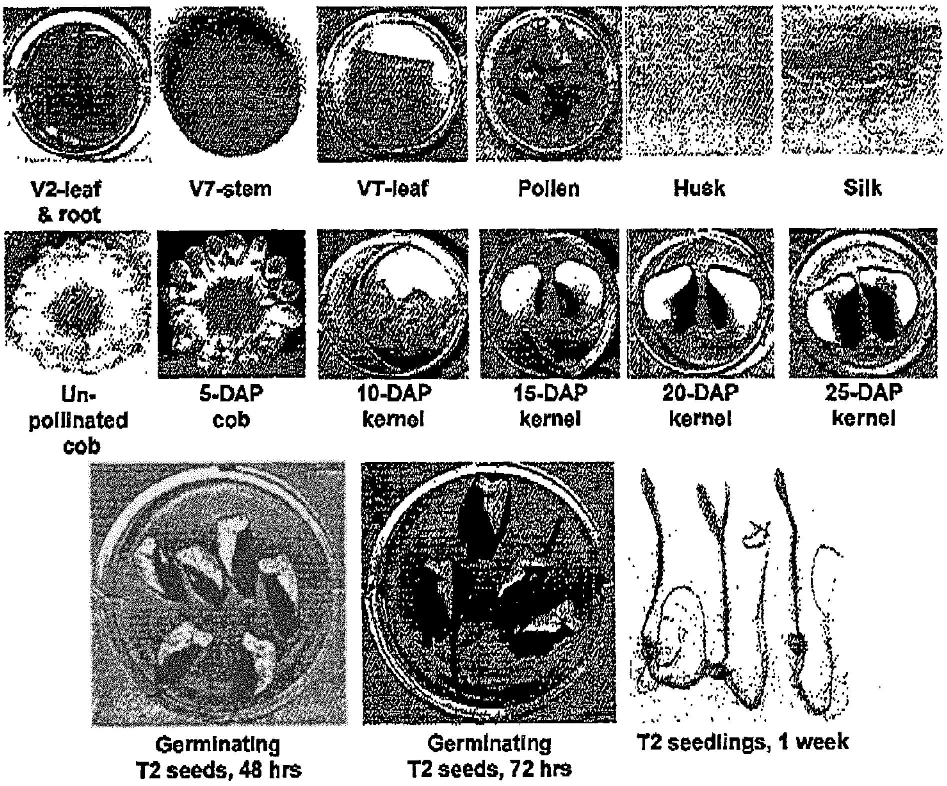
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 9

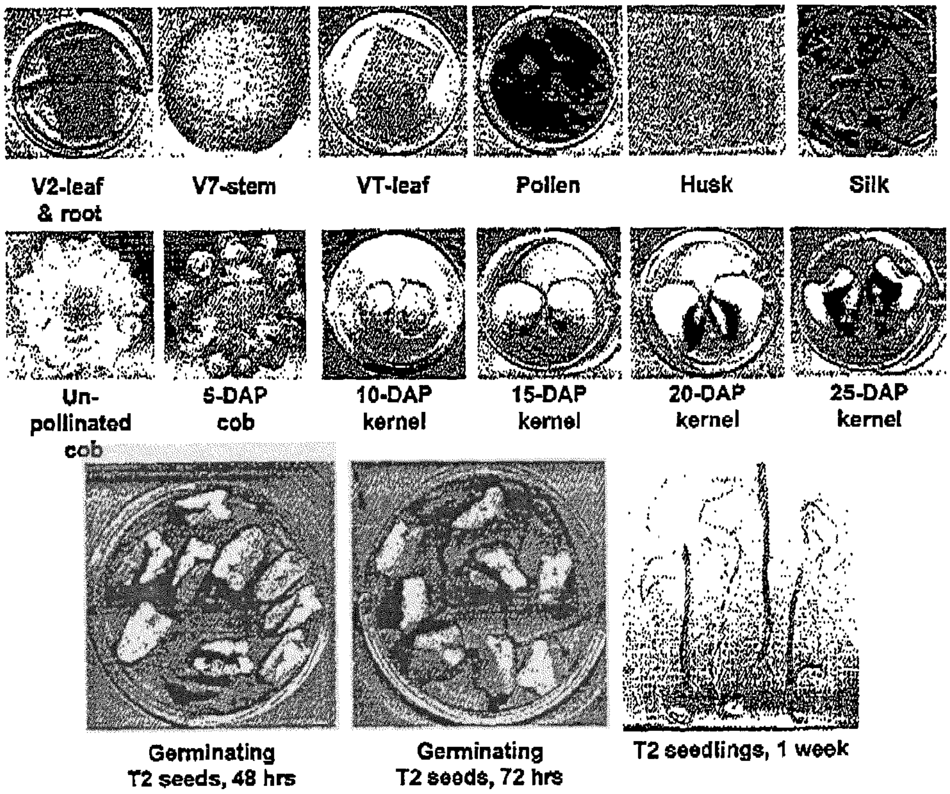
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 10

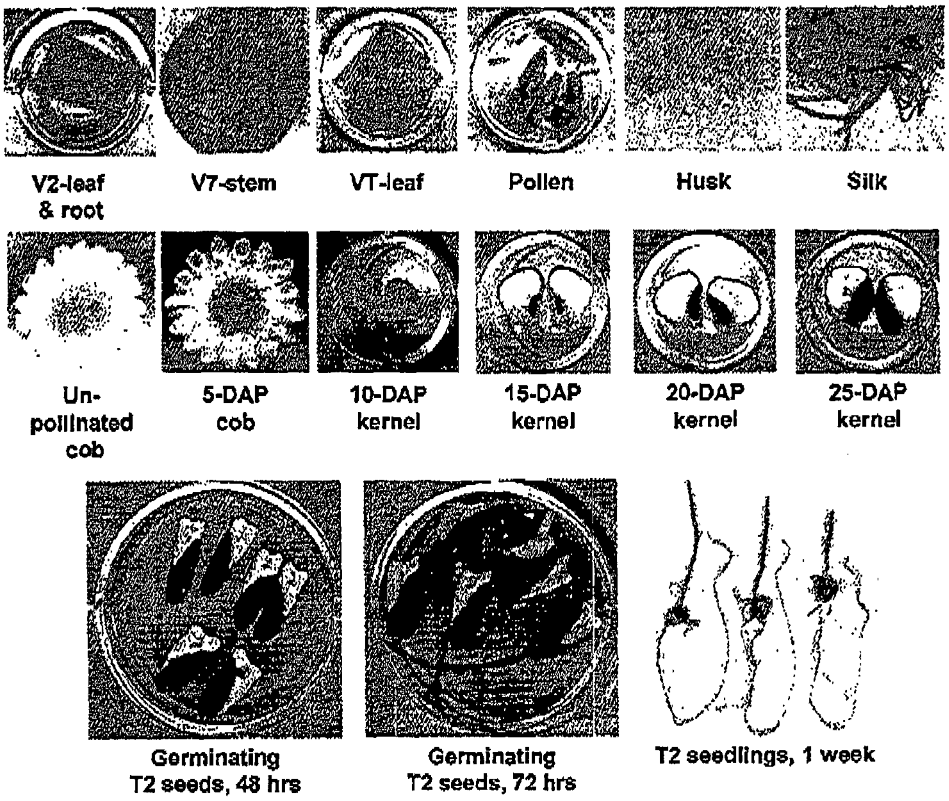
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 11

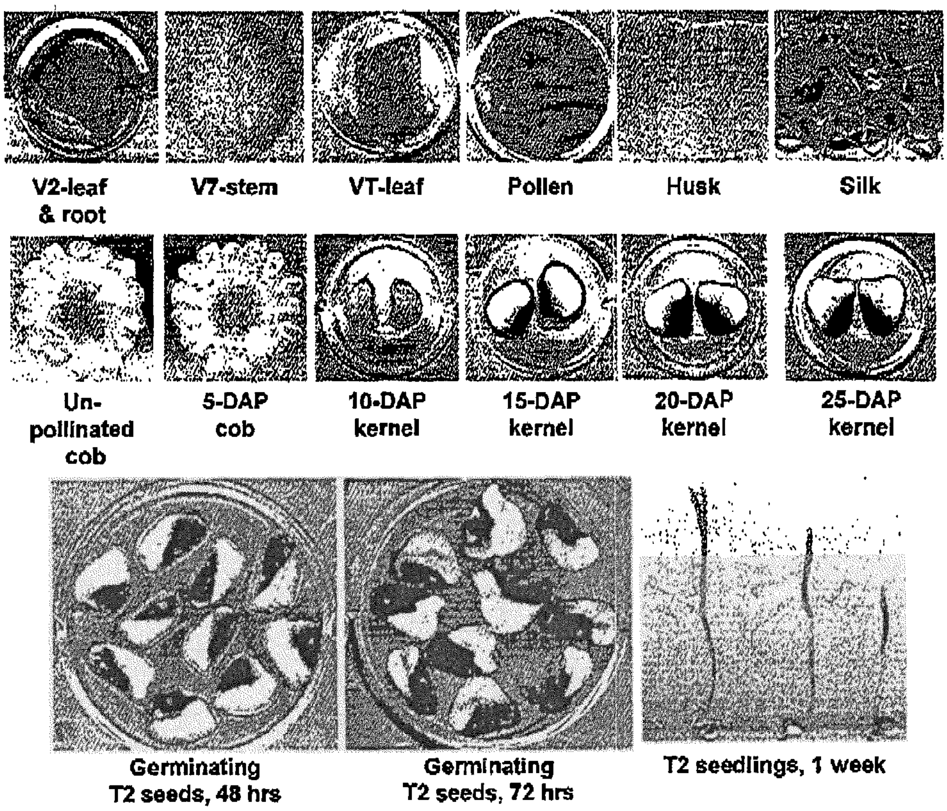
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 12

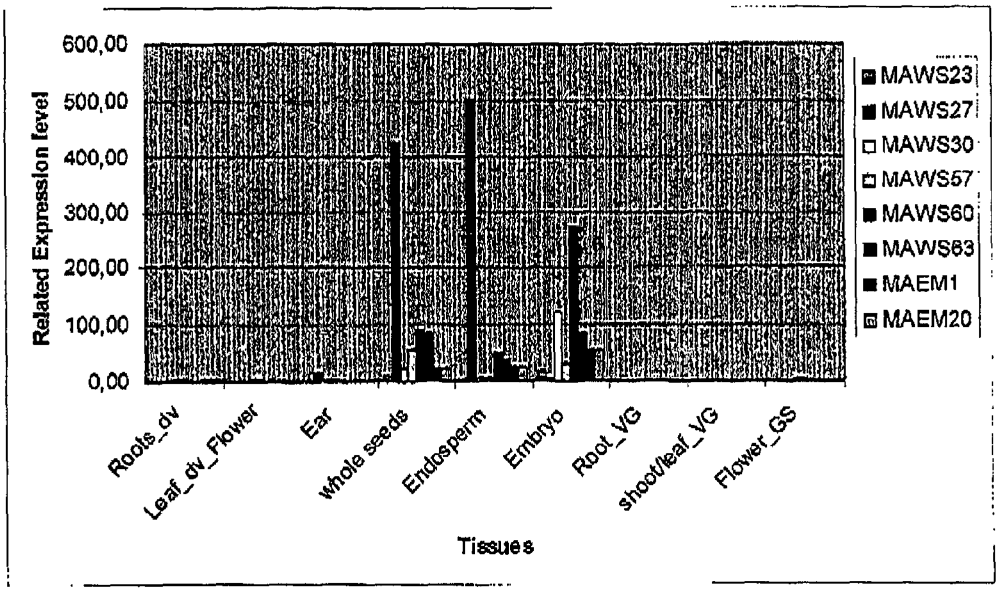
600,00
500,00
400,00
300,00
200,00
100,00
0,00
Related Expression level
MAWS23
MAWS27
MAWS30
MAWS57
MAWS60
MAWS63
MAEM1
MAEM20
Roots_dv
Leaf_dv_Flower
Ear
whole seeds
Endosperm
Embryo
Root_VG
shoot/leaf_VG
Flower_GS
Tissues

Figure 13

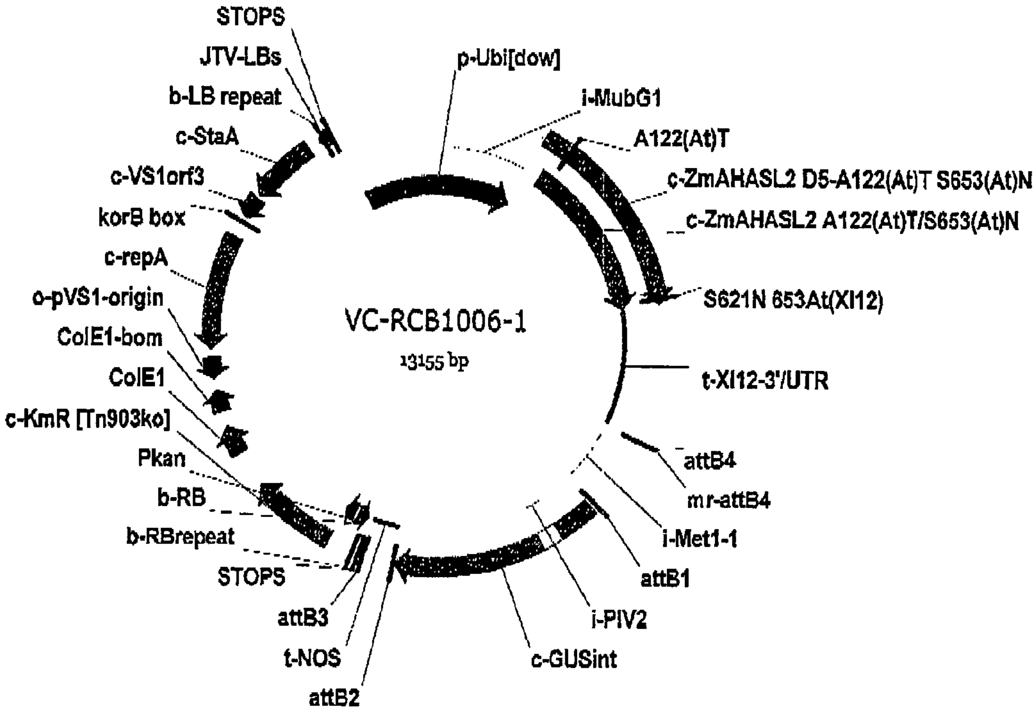
STOPS
JTV-LBs
b-LB repeat
c-StaA
c-VS1orf3
korB box
c-repA
o-pVS1-origin
ColE1-bom
ColE1
c-KmR [Tn903ko]
Pkan
b-RB
b-RBrepeat
STOPS
attB3
t-NOS
attB2
p-Ubi[dow]
i-MubG1
A122(At)T
c-ZmAHASL2 D5-A122(At)T S653(At)N
c-ZmAHASL2 A122(At)T/S653(At)N
S621N 653At(Xl12)
VC-RCB1006-1
13155 bp
t-Xl12-3'/UTR
attB4
mr-attB4
i-Met1-1
attB1
i-PIV2
c-GUSint

Figure 14

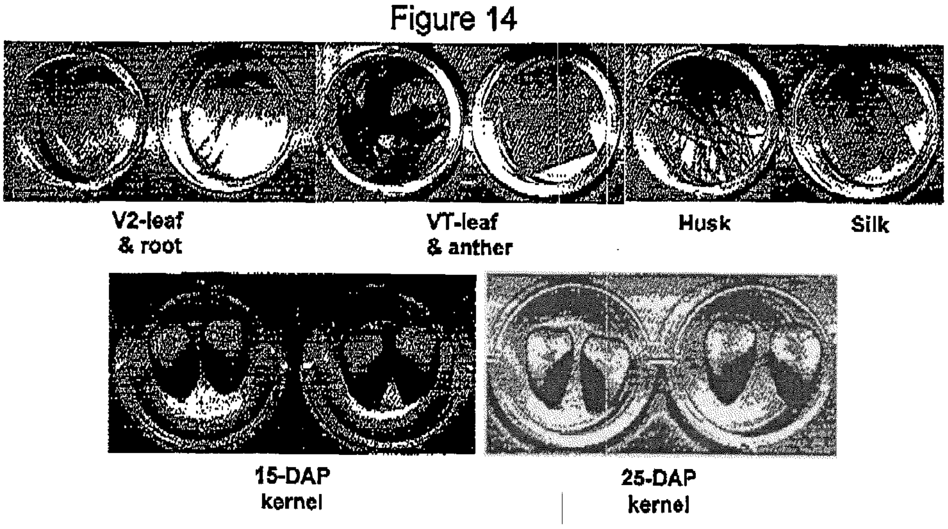


Figure 15

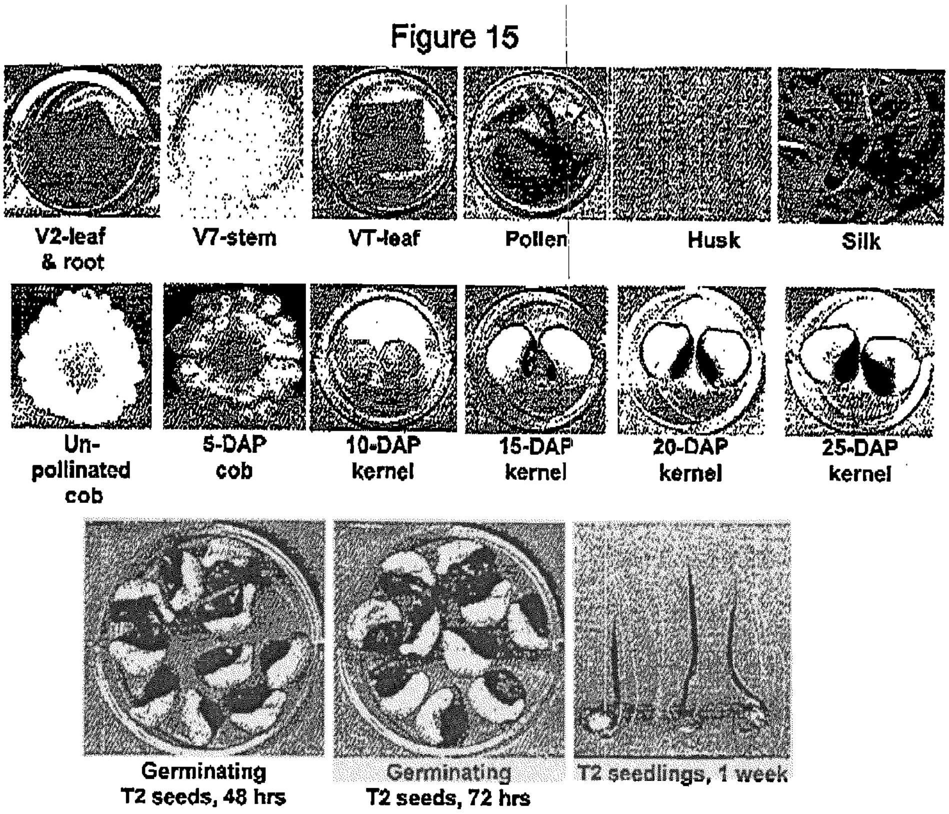

Figure 16

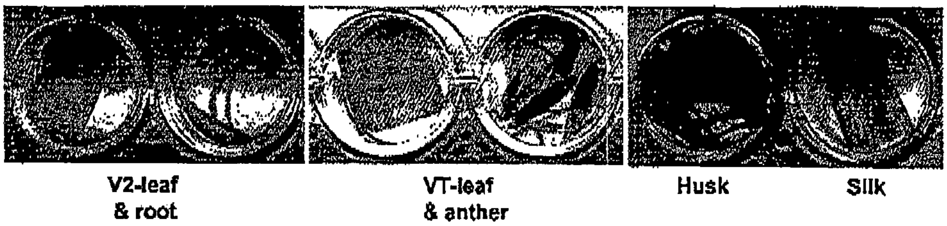

V2-leaf & root
VT-leaf & anther
Husk
Silk

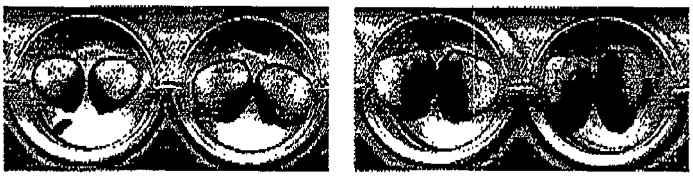

15-DAP kernel
25-DAP kernel

Figure 17

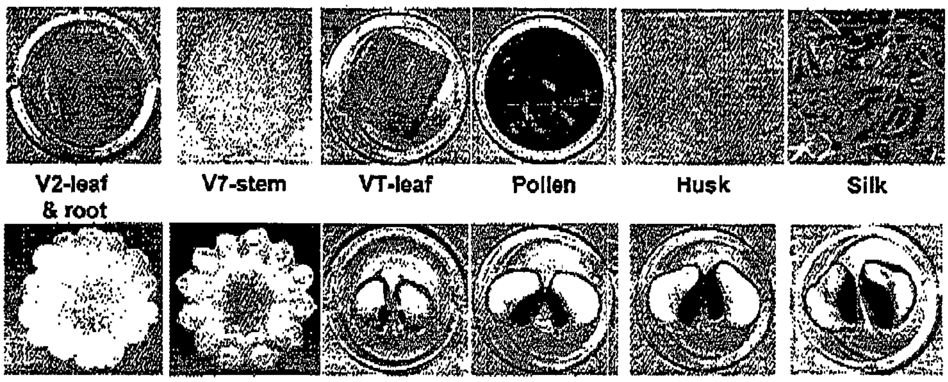

V2-leaf & root
V7-stem
VT-leaf
Pollen
Husk
Silk

Un-pollinated cob
5-DAP cob
10-DAP kernel
15-DAP kernel
20-DAP kernel
25-DAP kernel

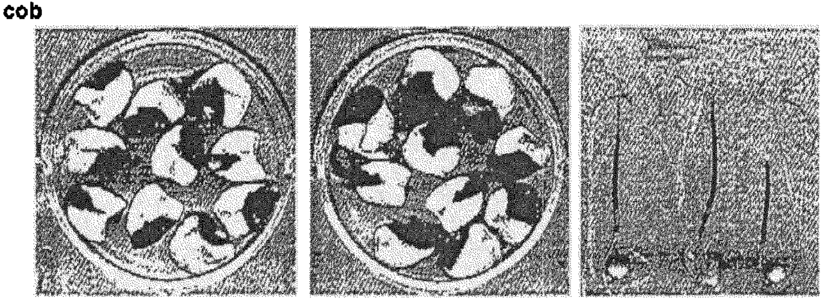

Germinating T2 seeds, 48 hrs
Germinating T2 seeds, 72 hrs
T2 seedlings, 1 week

Figure 18

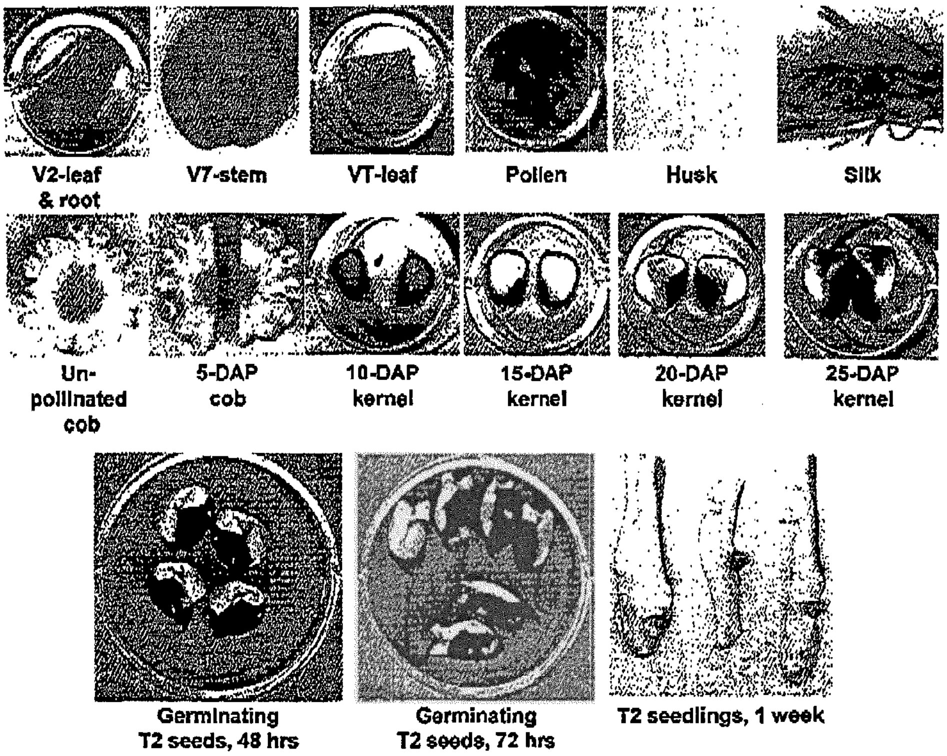
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 19

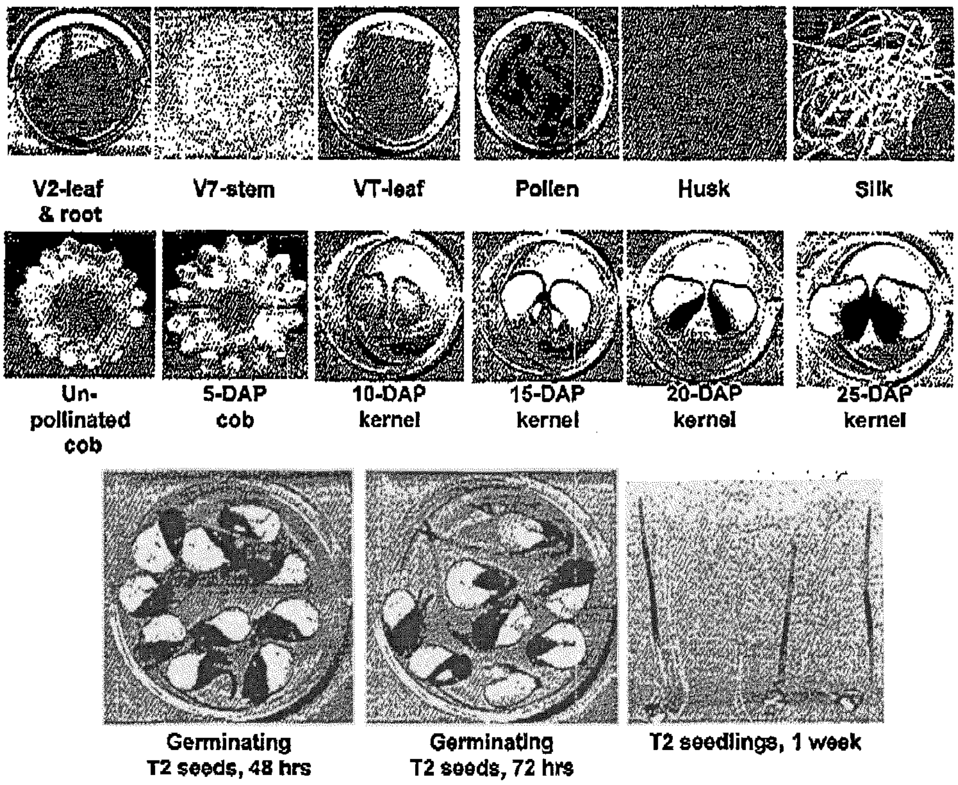
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 20

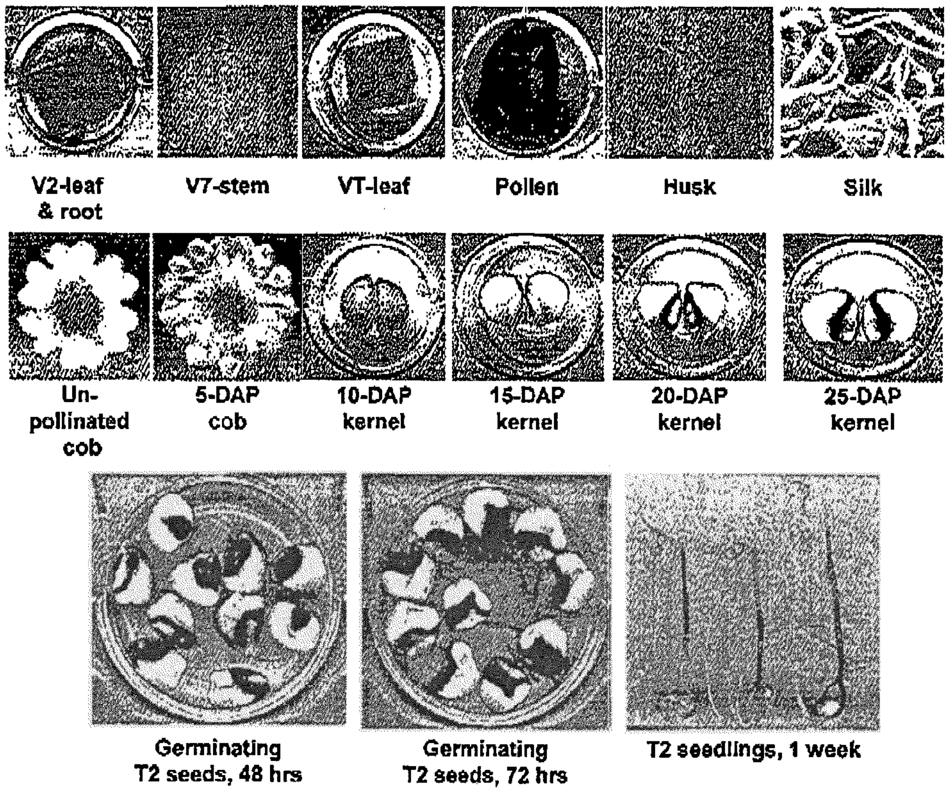
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 21

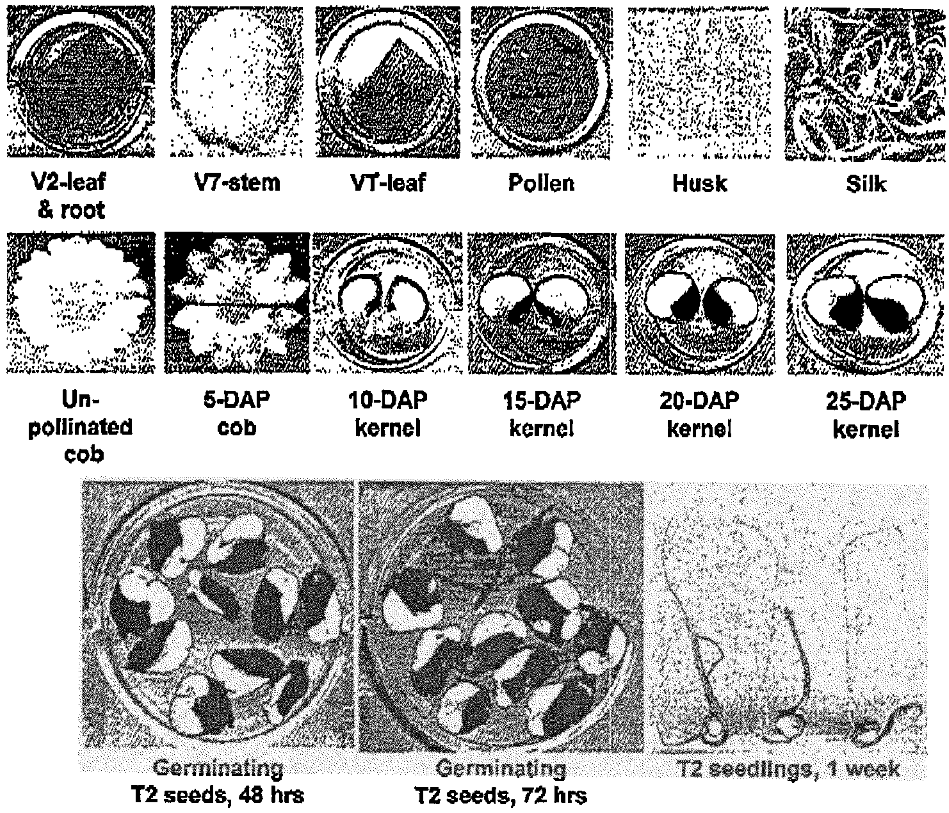
V2-leaf & root
V7-stem
VT-leaf
Pollen
Husk
Silk
Un-pollinated cob
5-DAP cob
10-DAP kernel
15-DAP kernel
20-DAP kernel
25-DAP kernel
Germinating T2 seeds, 48 hrs
Germinating T2 seeds, 72 hrs
T2 seedlings, 1 week

Figure 22

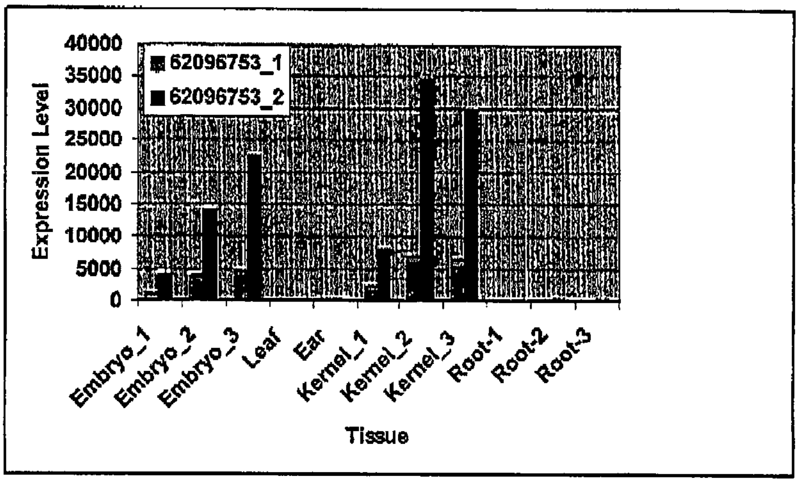


Figure 23

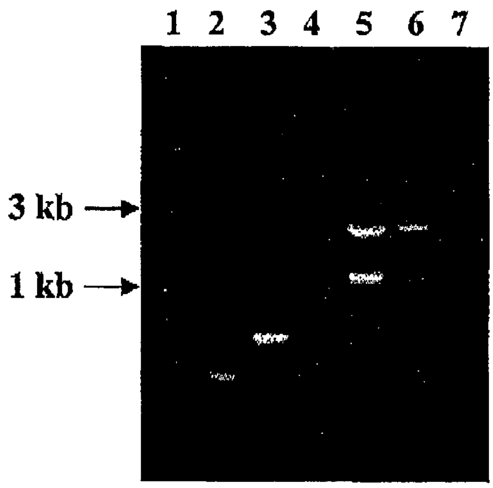

Figure 24

(A)

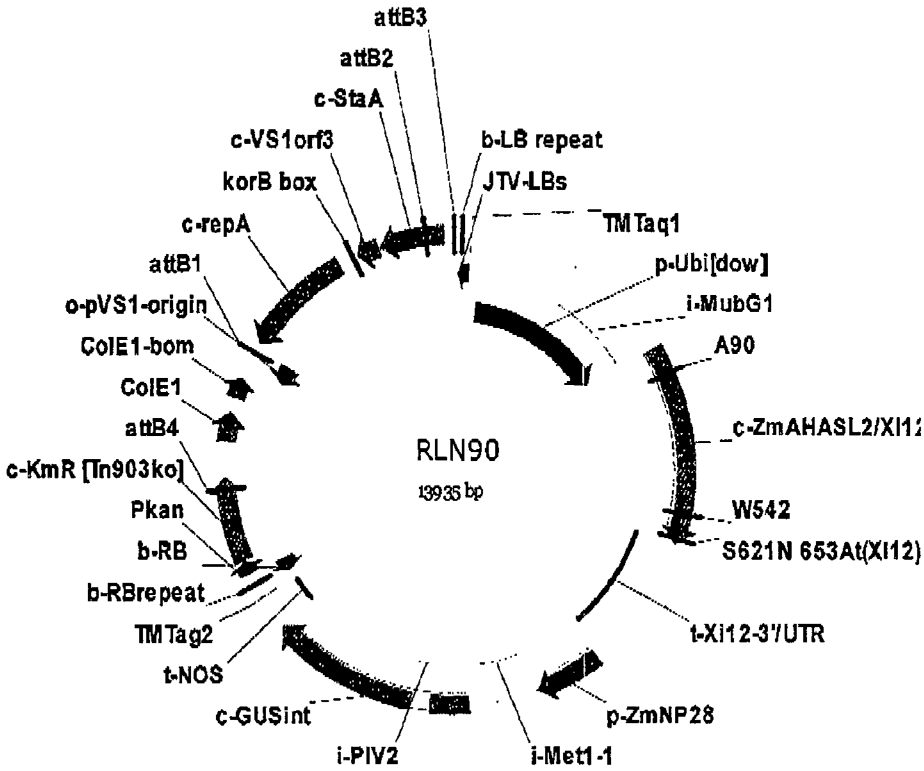
attB3
attB2
c-StaA
c-VS1orf3
b-LB repeat
korB box
JTV-LBs
c-repA
TMTaq1
attB1
p-Ubi[dow]
o-pVS1-origin
i-MubG1
ColE1-bom
A90
ColE1
attB4
c-ZmAHASL2/Xl1
RLN90
13935 bp
c-KmR [Tn903ko]
Pkan
W542
b-RB
S621N 653At(Xl12)
b-RBrepeat
TMTag2
t-Xl12-3'/UTR
t-NOS
c-GUSint
p-ZmNP28
i-PIV2
i-Met1-1

Figure 24

(B)

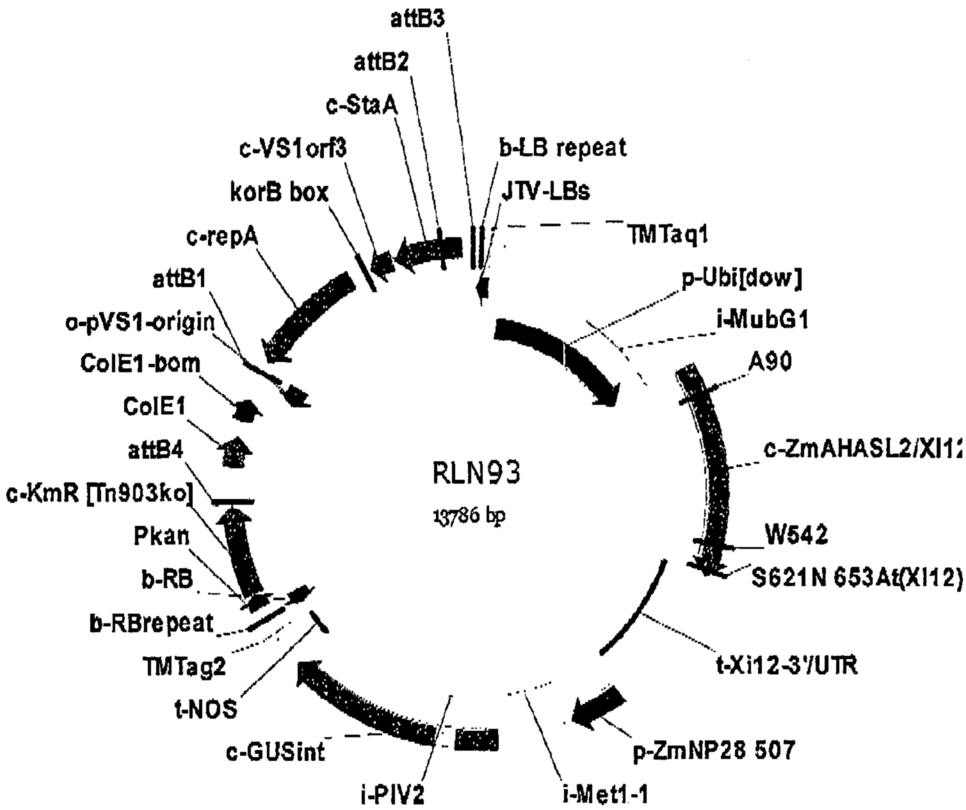
attB3
attB2
c-StaA
c-VS1orf3
korB box
c-repA
attB1
o-pVS1-origin
ColE1-bom
ColE1
attB4
c-KmR [Tn903ko]
Pkan
b-RB
b-RBrepeat
TMTag2
t-NOS
c-GUSint
i-PIV2
i-Met1-1
p-ZmNP28 507
t-Xi12-3'/UTR
S621N 653At(XI12)
W542
c-ZmAHASL2/XI1:
A90
i-MubG1
p-Ubi[dow]
TMTaq1
JTV-LBs
b-LB repeat
RLN93
13786 bp

Figure 24

(C)

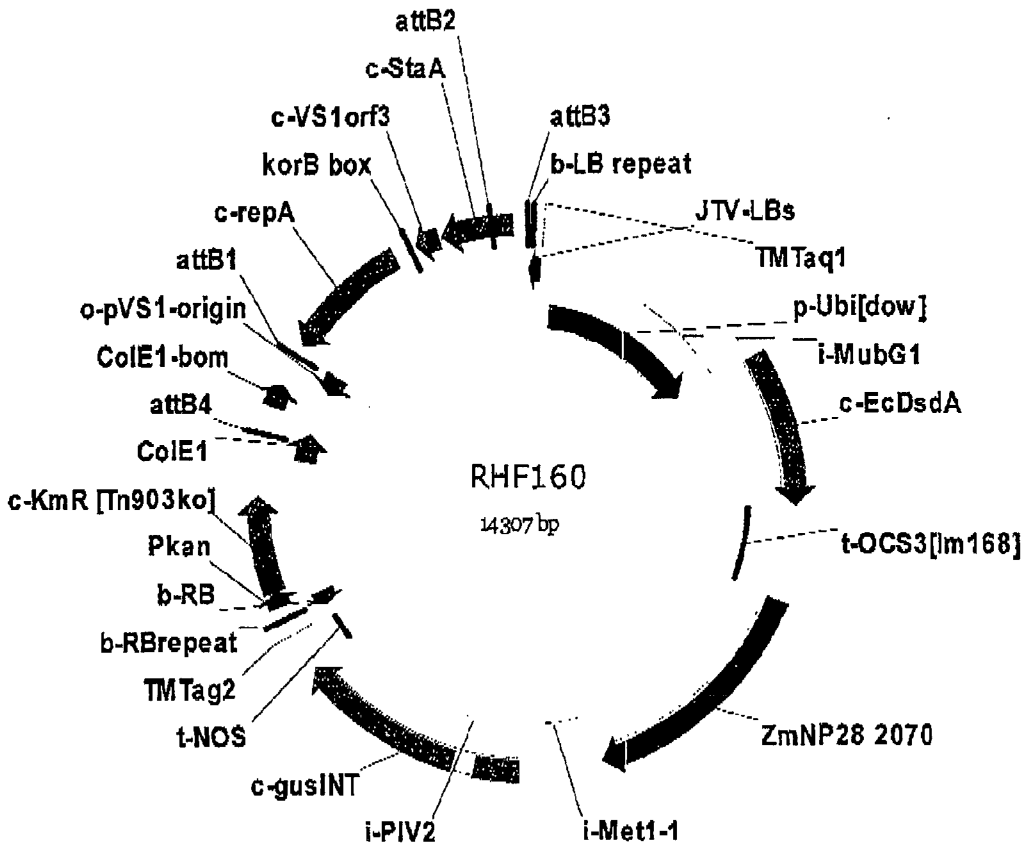
attB2
c-StaA
c-VS1orf3
korB box
c-repA
attB1
o-pVS1-origin
ColE1-bom
attB4
ColE1
c-KmR [Tn903ko]
Pkan
b-RB
b-RBrepeat
TMTag2
t-NOS
c-gusINT
i-PIV2
i-Met1-1
ZmNP28 2070
t-OCS3[lm168]
c-EcDsdA
i-MubG1
p-Ubi[dow]
TMTaq1
JTV-LBs
b-LB repeat
attB3
RHF160
14307 bp

Figure 24

(D)

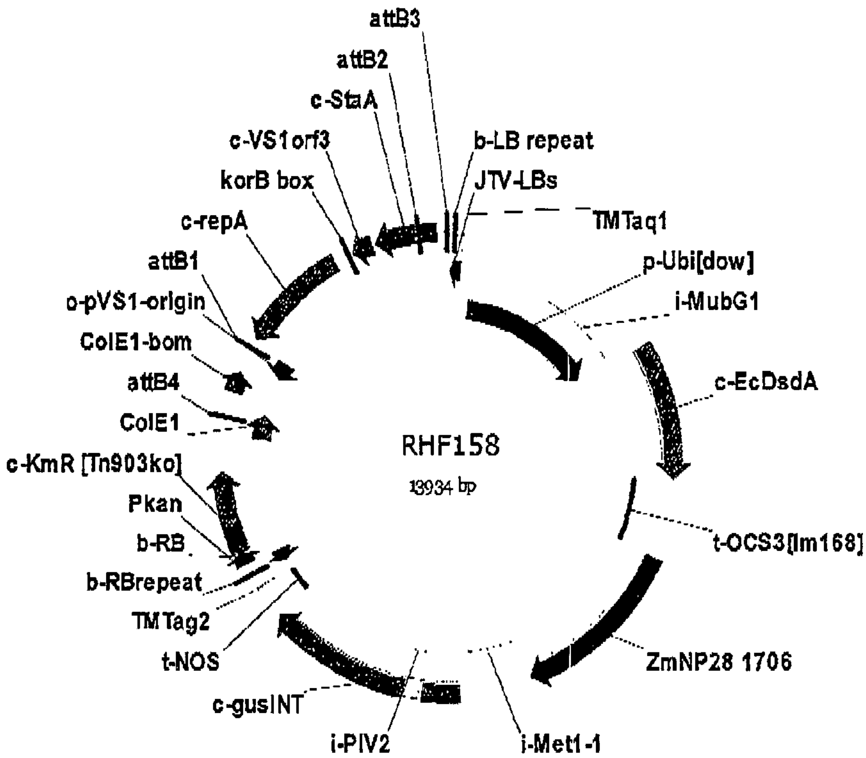
attB3
attB2
c-StaA
c-VS1orf3
korB box
c-repA
attB1
o-pVS1-origin
ColE1-bom
attB4
ColE1
c-KmR [Tn903ko]
Pkan
b-RB
b-RBrepeat
TMTag2
t-NOS
c-gusINT
i-PIV2
i-Met1-1
ZmNP28 1706
t-OCS3[lm168]
c-EcDsdA
i-MubG1
p-Ubi[dow]
TMTaq1
JTV-LBs
b-LB repeat
RHF158
13934 bp

Figure 25

(A)

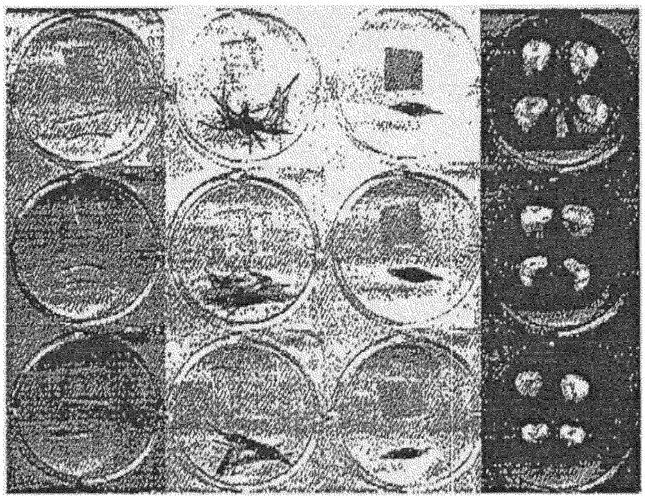

Leaf & root 5-leaf | Leaf & silk Flowering | Leaf & anther Flowering | Seeds 25 DAP

(B)

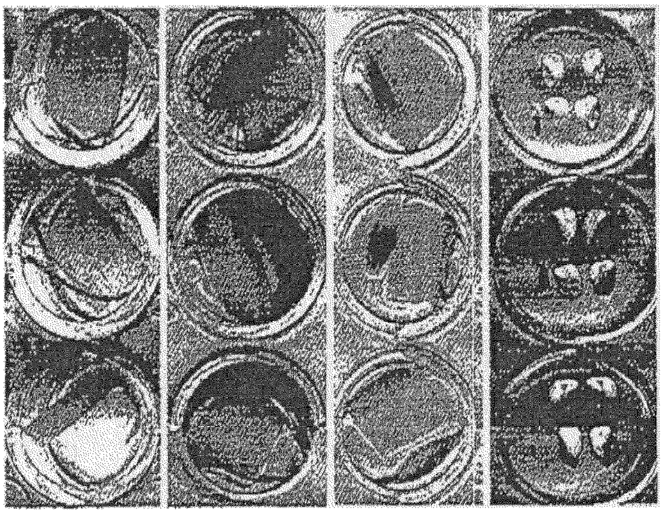

Leaf & root 5-leaf | Leaf & silk Flowering | Leaf & anther Flowering | Seeds 25 DAP

Figure 25

(C)

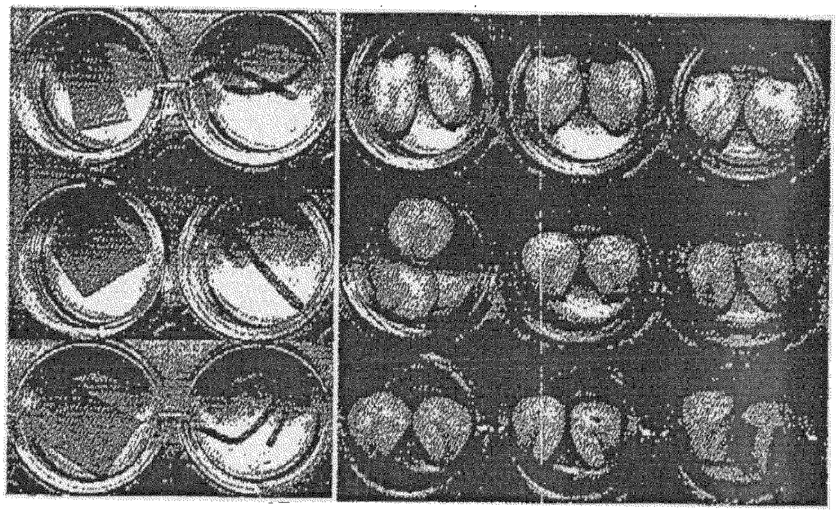

Leaf & root
5-leaf stage

Seeds
25 DAP

(D)

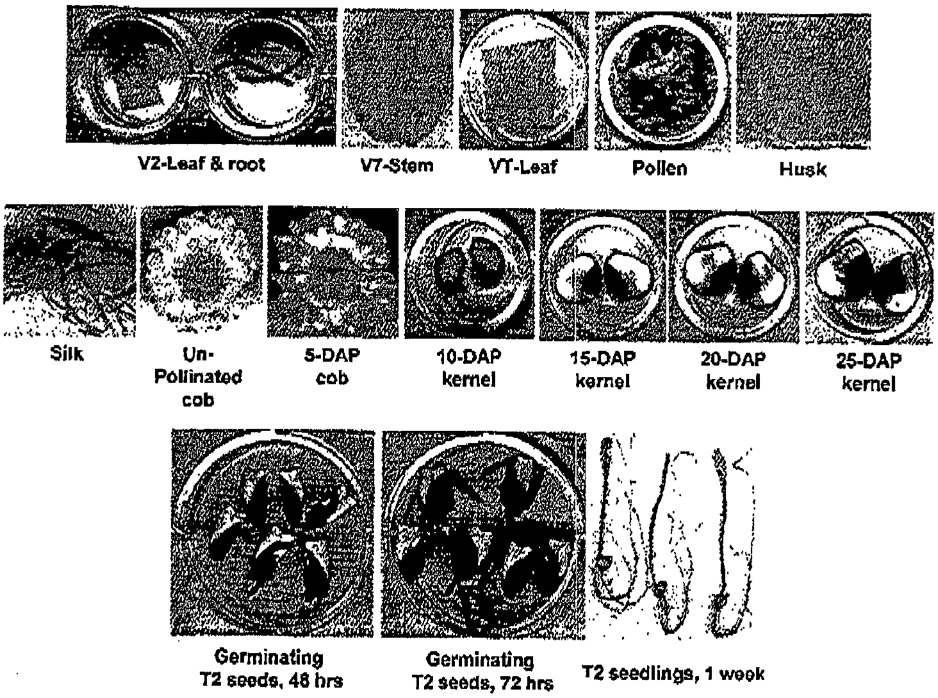

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5504200 A
- US 5608152 A
- US 5912414 A
- WO 02102970 A
- WO 9116432 A
- US 5605794 A
- US 12837458 B
- US 4945050 A
- US 4237224 A
- EP 295959 A
- EP 138341 A
- EP 120516 A
- EP 301749 A
- EP 0332581 A
- US 5451513 A
- US 5545817 A
- US 5545818 A
- WO 9516783 A, McBride
- WO 0200900 A
- WO 0000512 A
- WO 9712983 A
- WO 9811240 A
- WO 9826045 A
- WO 9905902 A
- WO 02052012 A
- WO 9906580 A
- WO 9904013 A
- WO 9742326 A
- WO 9750561 A
- US 6063597 A
- US 6063756 A
- US 6093695 A
- US 5942664 A
- US 6110464 A
- US 5516671 A
- US 5773696 A
- US 6121436 A
- US 6316407 B
- US 6506962 B
- US 5304730 A
- US 6013864 A
- US 6228992 B
- US 6194636 B
- US 6207879 B
- US 6232526 B
- US 6426446 B
- US 6429357 B
- US 6433252 B
- US 6437217 B
- US 6515201 B
- US 6583338 B
- WO 02057471 A
- WO 0032757 A
- WO 0010380 A
- US 5750876 A
- US 6476295 B
- US 6380466 B
- US 5512466 A
- US 5985605 A
- US 6171640 B
- US 5958745 A
- US 20030028917 A
- US 6072103 A
- US 6080560 A
- US 6531648 B
- US 6166292 A
- US 5543576 A
- US 6011199 A
- US 5229114 A
- US 5689041 A
- US 5998700 A
- US 6232122 B
- US 6147279 A
- WO 9722703 A
- US 6444876 B
- US 5608149 A
- US 6537750 B
- US 5512482 A
- US 5530186 A
- US 5945585 A
- US 5639790 A
- US 5807893 A
- US 5955650 A
- US 5955329 A
- US 5759829 A
- US 5147792 A
- US 5304481 A
- US 5298421 A
- US 5344771 A
- US 5760206 A
- US 20030115632 A1
- US 90030028923 A1
- US 5689050 A
- US 5663068 A
- US 5614393 A
- US 5856157 A
- US 6117677 A
- US 6043411 A

- US 6194167 B
- US 5705391 A
- US 5552306 A
- US 6075183 A
- US 6051754 A
- US 5789220 A
- US 5057419 A
- US 5654402 A
- US 5659645 A
- US 6100091 A
- US 6172106 B
- US 5952544 A
- US 5866789 A
- US 5443974 A
- US 5093249 A
- US 5965727 A
- WO 9726366 A
- WO 9911800 A
- WO 9949058 A
- WO 9906581 A
- US 5534421 A
- US 5942660 A
- WO 9519442 A
- WO 9902656 A
- WO 9855601 A
- US 5258300 A
- US 5367110 A
- US 5858749 A
- US 6040160 A
- US 4886878 A
- US 4885357 A
- US 5215912 A
- US 5589616 A
- US 5508468 A
- US 5939599 A
- US 5633436 A
- US 5990384 A
- WO 9001869 A
- WO 9113993 A
- WO 9214822 A
- WO 9308682 A
- WO 9420628 A
- WO 9728247 A
- WO 9826064 A
- WO 9940209 A
- US 5003045 A
- US 5576203 A
- US 5850024 A
- WO 9617064 A
- WO 9735023 A
- WO 0019839 A
- US 6107051 A
- US 5885802 A
- US 5885801 A
- US 5270200 A
- US 6110891 A
- US 5990389 A
- US 5914450 A
- US 5998701 A
- WO 9932619 A
- WO 9953050 A
- WO 0068374 A
- WO 0044914 A
- WO 0044895 A
- WO 0049035 A
- WO 0063364 A
- WO 9845456 A
- EP 0333033 A
- US 4975374 A
- EP 0218571 A1
- EP 154204 A1
- EP 0807836 A
- WO 03060133 A, Erikson
- EP 0601092 A
- US 20040034888 A1

**Non-patent literature cited in the description**


- **WU et al.** *Plant Cell Physiol,* 1998, vol. 39 (8), 885-889
- **CHEN et al.** *Dev. Genet.,* 1989, vol. 10 (2), 112-122
- **KEDDIE et al.** *Plant Mol. Biol.,* 1992, vol. 19 (3), 443-53
- **SJODAHL et al.** *Planta,* 1995, vol. 197 (2), 264-71
- **REIDT et al.** *Plant J.,* 2000, vol. 21 (5), 401-8
- **AHM, V et al.** *Plant Phys,* 1995, vol. 109, 1151-1158
- *Gene,* 2005, vol. 356, 146-152
- **LIU ; HUANG.** *Plant Molecular Biology Reporter,* 1998, vol. 16, 175-181
- **LIU et al.** *The Plant Journal,* 1995, vol. 8, 457-463
- **HIGO K et al.** *Nucleic Acids Res,* 1999, vol. 27 (1), 297-300
- **KUNKEL et al.** *Methods Enzymol,* 1987, vol. 154, 367-382
- **TOMIC et al.** *Nucl Acids Res,* 1990, vol. 12, 1656
- **UPENDER et al.** *Biotechniques,* 1995, vol. 18 (1), 29-30
- **SAMBROOK et al.** Molecular Cloning: A Laboratory Manual. Cold Spring Harbor Laboratory Press, 1989
- Methods in Molecular Biology. Agrobacterium protocols. Humana Press, 1995, vol. 44
- **HELLENS et al.** *Trends in Plant Science,* 2000, vol. 5, 446-451

- Plant Molecular Biology and Biotechnology. CRC Press, 1993, 71-119
- Vectors for Gene Transfer in Higher Plants. **F.F. WHITE.** Transgenic Plants, vol. 1, Engineering and Utilization. Academic Press, 1993, vol. 1, 15-38
- Techniques for Gene Transfer. **B. JENES et al.** Transgenic Plants, vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **SAMBROOK.** Molecular Cloning A Laboratory Manual. Cold Spring Harbor Laboratory, 1989
- **AUSUBEL.** Current Protocols in Molecular Biology. Green Publishing Associates and Wiley Interscience, 1994
- **SMITH, D.B. ; JOHNSON, K.S.** *Gene,* 1988, vol. 67, 31-40
- **BALDARI et al.** *Embo J.,* 1987, vol. 6, 229-234
- **KURJAN ; HERSKOWITZ.** *Cell,* 1982, vol. 30, 933-943
- **SCHULTZ et al.** *Gene,* 1987, vol. 54, 113-123
- Gene transfer systems and vector development for filamentous fungi. **VAN DEN HONDEL, C.A.M.J.J. ; PUNT, P.J. et al.** Applied Molecular Genetics of fungi. Cambridge University Press, 1991, 1-28
- More Gene Manipulations in Fungi. Academic Press, 396-428
- Brock Biology of Microorganisms. A-8, , A-9, , A10, , A11
- **PEI ZM et al.** *Science,* 1998, vol. 282, 287-290
- **DEAK M et al.** *Nature Biotechnology,* 1999, vol. 17, 192-196
- **DUNWELL JM.** *Biotechn Genet Eng Rev,* 1998, vol. 15, 1-32
- **KASUGA M et al.** *Nature Biotech,* 1999, vol. 17, 276-286
- **RASK L et al.** *Plant Mol Biol,* 2000, vol. 42, 93-113
- **MENARD R et al.** *Phytochemistry,* 1999, vol. 52, 29-35
- **VAECK et al.** *Nature,* 1987, vol. 328, 33-37
- **BROGLIE et al.** *Science,* 1991, vol. 254, 1194-1197
- **RAO et al.** *Plant J,* 1998, vol. 15 (4), 469-77
- **GOYAL RK et al.** *Crop Protection,* 2000, vol. 19 (5), 307-312
- **LEE TJ et al.** *J Amer Soc Horticult Sci,* 2002, vol. 127 (2), 158-164
- **KRIDL et al.** *Seed Sci. Res,* 1991, vol. 1 (209), 219
- **KEEGSTRA.** *Cell,* 1989, vol. 56 (2), 247-53
- **NAWRATH et al.** *Proc. Natl. Acad. Sci. USA,* 1994, vol. 91, 12760-12764
- **XIA et al.** *J. Gen. Microbiol.,* 1992, vol. 138, 1309-1316
- **LOIS et al.** *Proc. Natl. Acad. Sci. USA,* 1998, vol. 95 (5), 2105-2110
- **TAKAHASHI et al.** *Proc. Natl. Acad. Sci. USA,* 1998, vol. 95 (17), 9879-9884
- **NORRIS et al.** *Plant Physiol.,* 1998, vol. 117, 1317-1323
- **BARTLEY ; SCOLNIK.** *Plant Physiol.,* 1994, vol. 104, 1469-1470
- **SMITH et al.** *Plant J.,* 1997, vol. 11, 83-92
- **SAINT GUILY et al.** *Plant Physiol.,* 1992, vol. 100 (2), 1069-1071
- **SATO et al.** *J. DNA Res.,* 2000, vol. 7 (1), 31-63
- **GIRKE et al.** *Plant J,* 1998, vol. 15, 39-48
- **SAKURADANI et al.** *Gene,* 1999, vol. 238, 445-453
- **MICHAELSON et al.** *FEBS Letters,* 1998, vol. 439, 215-218
- **MICHAELSON et al.** *JBC,* vol. 273, 19055-19059
- **BEAUDOIN et al.** *PNAS,* 2000, vol. 97, 6421-6426
- **ZANK et al.** *Biochemical Society Transactions,* 2000, vol. 28, 654-657
- **HOOD EE ; JILKA JM.** *Curr Opin Biotechnol,* 1999, vol. 10 (4), 382-6
- **MA JK ; VINE ND.** *Curr Top Microbiol Immunol,* 1999, vol. 236, 275-92
- **HOOD et al.** *Adv Exp Med Biol,* 1999, vol. 464, 127-47
- **DUNWELL JM.** Transgenic approaches to crop improvement. *J Exp Bot.,* 2000, 487-96
- **IRAZARRY et al.** *Biostatistics,* 2003, vol. 4 (2), 249-264